(19) Europäisches Patentamt
European Patent Office
Office européen des brevets

(11) EP 4 775 577 A1

(12) EUROPEAN PATENT APPLICATION
published in accordance with Art. 153(4) EPC

(43) Date of publication:
**15.07.2026 Bulletin 2026/29**

(21) Application number: **24862080.9**

(22) Date of filing: **06.09.2024**

(51) International Patent Classification (IPC):
***C07D 409/14*** *(2006.01)* ***A61K 31/513*** *(2006.01)*
***A61P 35/00*** *(2006.01)*

(52) Cooperative Patent Classification (CPC):
**A61K 31/513; A61K 31/517; A61K 31/551; A61K 31/553; A61P 35/00; A61P 35/02; C07D 409/14; C07D 413/14; C07D 487/04; C07D 493/10; C07D 498/04; C07D 519/00**

(86) International application number:
**PCT/CN2024/117451**

(87) International publication number:
**WO 2025/051242 (13.03.2025 Gazette 2025/11)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority:
**08.09.2023 CN 202311164113**
**27.02.2024 CN 202410217579**
**21.05.2024 CN 202410636632**
**14.08.2024 CN 202411117853**
**05.09.2024 CN 202411239838**

(71) Applicant: **Tyligand Bioscience (Shanghai) Limited**
**Shanghai 201203 (CN)**

(72) Inventors:
- **SHANG, Erchang**
  **Shanghai 201112 (CN)**
- **ZHONG, Boyu**
  **Irving, TX 75038 (US)**
- **ZHANG, Tony Yantao**
  **Fishers, IN 46037 (US)**
- **SONG, Guanglin**
  **Shanghai 200125 (CN)**
- **JIANG, Jun**
  **Shanghai 201203 (CN)**
- **WANG, Longfei**
  **Shanghai 201210 (CN)**
- **HOU, Fuliang**
  **Shanghai 201203 (CN)**
- **WANG, Jifa**
  **Shanghai 201203 (CN)**
- **WANG, Heng**
  **Shanghai 201203 (CN)**
- **ZHENG, Aijun**
  **Shanghai 201203 (CN)**
- **DONG, Chunlan**
  **Shanghai 201203 (CN)**

(74) Representative: **Vossius & Partner Patentanwälte Rechtsanwälte mbB**
**Siebertstraße 3**
**81675 München (DE)**

(54) **RAS INHIBITOR**

(57) The present invention provides a compound having a structure as represented by formula (I) which can be used as a RAS inhibitor, a pharmaceutical composition comprising the compound, a method for preparing the compound, and a use of the compound in the treatment of cancer.

EP 4 775 577 A1

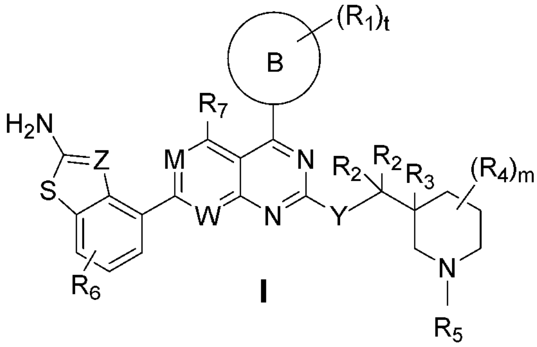

(I)

## Description

### Cross-references

This application claims priority to Chinese patent application 2023111641130 filed on September 8, 2023, Chinese patent application 2024102175790 filed on February 27, 2024, Chinese patent application 2024106366320 filed on May 21, 2024, Chinese patent application 2024111178533 filed on August 14, 2024, and Chinese patent application 2024112398386 filed on September 5, 2024.

### Technical Field

The present invention relates to the field of pharmaceutical chemistry. More specifically, the present invention relates to a class of compounds with novel structures that can be used as RAS inhibitors, pharmaceutical compositions containing such compounds, methods for preparing such compounds, and the use of these compounds in the treatment of cancer or tumors.

### Background Technology

RAS, the rat sarcoma oncogene homolog, represents a group of closely related monomeric globular proteins belonging to the GTPase protein family. Specifically, under normal physiological conditions, RAS is activated by growth factors and various other extracellular signals and is responsible for regulating functions such as cell growth, survival, migration, and differentiation. These regulatory functions of RAS are carried out through the transition between the GDP-bound state and GTP-bound state, i.e., "molecular switch" (Alamgeer et al., Current Opin Pharmacol. 2013, 13:394-401). RAS bound to GDP is an inactive form and is in a dormant or off state, at which point the signaling system is turned off and activated when exposed to some probiotic stimuli pro-growth stimuli, for example, it can be induced by guanine nucleotide exchange factor (GEF) to release GDP and bind GTP, resulting in RAS being "switched on", thereby converting to RAS active form, which recruits and activates various downstream effectors for signal transmission and is able to transmit signals from the cell surface to the cytoplasm. This controls numerous critical cellular processes such as differentiation, survival, and proliferation (Zhi Tan et al., Mini-Reviews in Medicinal Chemistry, 2016, 16, 345-357).

Ras possesses GTPase activity, which can cleave the terminal phosphate of GTP and convert it to GDP, that is, convert itself to an inactive state. However, the endogenous GTPase activity of RAS is very low, and the exogenous protein GAP (GTPase activating protein) is required to convert GTP-RAS to GDP-RAS. GAP interacts with RAS and promotes the conversion of GTP to GDP. Therefore, any RAS gene mutation that impacts RAS interaction with GAP or influences the conversion of GTP to GDP will result in RAS to be in an activated state for a long time, thereby continuously conveying signals for growth and division to cells, stimulating continuous cell proliferation, and ultimately leading to tumor formation and development.

Among the genes associated with human tumors, there are three ubiquitously expressed Ras genes H-RAS, K-RAS, and N-RAS, which encode highly homologous HRAS, NRAS, and KRAS proteins of approximately 21 KDa, respectively. In 1982, researchers first discovered that RAS was mutationally activated in cancer cell lines (Chang, E.H. et al., Proceedings of the National Academy of Sciences of the United States of America, 1982, 79(16), 4848-4852). Subsequent large genome sequencing studies in different cancer types have revealed that RAS protein is mutated in more than 30% of cancer types, with the highest mutation rates especially in pancreatic cancer (>90%), colon cancer (45%), and lung cancer (35%). Transgenic and genetically engineered mouse models have also revealed that mutated RAS proteins are sufficient to drive and trigger multiple types of cancer, and RAS oncogenes are also crucial for the maintenance and progression of tumors in multiple cancer types, such as RAS mutant cancer cell lines and animal models of cancer, where RNA intervention has been shown to slow tumor growth. These studies have made RAS tumor proteins as a very attractive anticancer drug target that is widely accepted in the pharmaceutical field.

Studies have shown that RAS mutations are most commonly seen in KRAS, and KRAS mutations can be observed in approximately 85% of RAS mutation-driven cancers; the vast majority of RAS mutations occur at codons G12, G13, and Q61, where about 80% of these KRAS mutations also occur at glycine at codon 12, such as G12C mutation, G12D mutation, G12V mutation, G12A mutation, G12R mutation, G12S mutation, G13D mutation, and Q61H mutation, etc. KRAS mutations are frequently found in pancreatic cancer, lung adenocarcinoma, colorectal cancer, gallbladder cancer, thyroid cancer, and cholangiocarcinoma, and can also be found in 25% of patients with non-small cell lung cancer (McCormick, F. et al., Clinical Cancer Research 21(8), 1797-1801, 2015). Therefore, KRAS mutant proteins have become the most important branch in RAS drug target research, and the development of their inhibitors is also regarded as a very promising direction for research in anticancer/tumor drug development.

However, drug development targeting RAS over the past few decades has revealed that due to the smooth surface of RAS protein, the lack of obvious groove or pocket-like structure for binding small molecule inhibitors, and its very

high affinity for guanine substrates (picomolar level), the development of its small-molecule inhibitors has fallen into an intractable predicament. For this reason, RAS has long been regarded as an "undruggable" target in the industry. At the same time, there remains a great need for compounds that have more structural types or patterns of KRAS inhibitors, provide more therapeutic options, or provide further improved inhibitory activity relative to existing KRAS inhibitors, thereby providing more potent therapeutic agents in the clinic.

The present invention addresses these and other needs. The present invention provides novel structural inhibitor compounds with RAS protein inhibitory activity. These compounds of the present invention have an improved structural pattern, and compared with the existing RAS protein inhibitors in the prior art, they have enhanced RAS protein inhibitory activity and related tumor inhibitory activity, and have good pharmacokinetic properties, thus having good drugability, such as being easier to absorb in the body after administration in a convenient manner, and having reduced toxic and side effects, improved drug resistance and safety, and reduced risk of drug interactions.

**Summary of invention**

The present invention provides a compound having structural formula (X) as defined below, a stereoisomer, tautomer, stable isotope variant, pharmaceutically acceptable salt or solvate thereof.

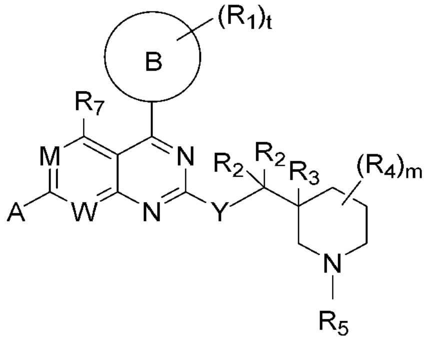

(X)

wherein A is selected from substituted $C_{6-10}$ aryl or substituted 6- to 10-membered heteroaryl containing 1 to 4 heteroatoms independently selected from N, O, S and Se, specifically, A is selected from the following fragments:

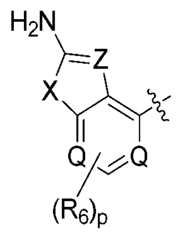

,

more specifically

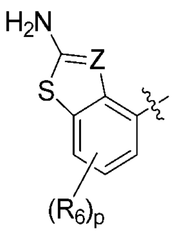

;

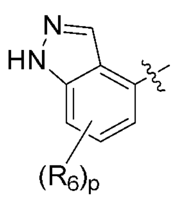

; or

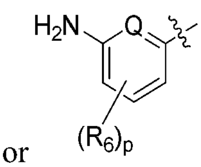

;

the definitions of the remaining group are as defined in the detailed description of the present invention.

The first aspect of the present invention provides a compound having structural formula (I) as defined below, a stereoisomer, tautomer, stable isotope variant, pharmaceutically acceptable salt or solvate thereof:

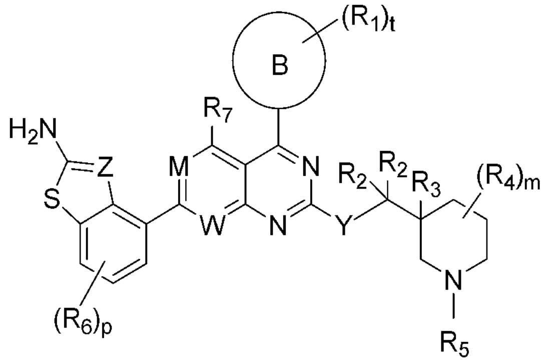

(I)

wherein the definitions of each group are as defined in the detailed description of the present invention.

The second aspect of the present invention provides a compound having structural formula (II) below, a stereoisomer, tautomer, stable isotopic variant, pharmaceutically acceptable salt or solvate thereof:

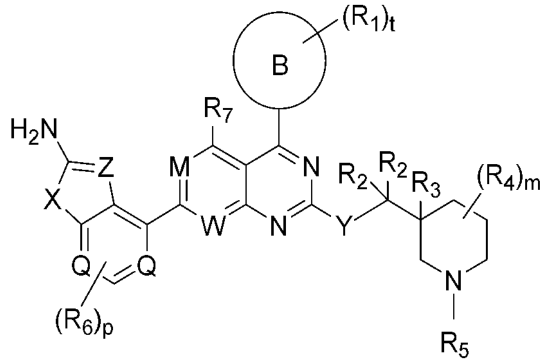

(II)

wherein the definitions of each group are as defined in the detailed description of the present invention.

The present invention also provides a compound of Formula (III) below, a stereoisomer, tautomer, stable isotope variant, pharmaceutically acceptable salt or solvate thereof:

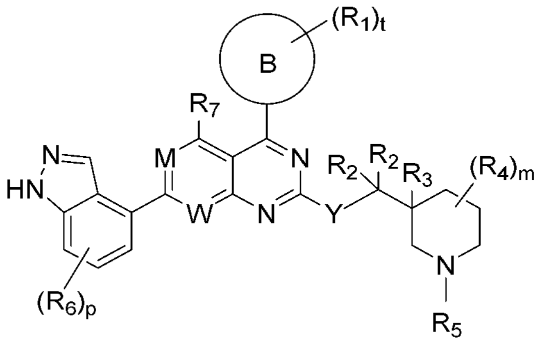

(III)

wherein the definitions of each group are as defined in the detailed description of the present invention.

The present invention also provides a compound of Formula (IV) below, a stereoisomer, tautomer, stable isotope variant, pharmaceutically acceptable salt or solvate thereof:

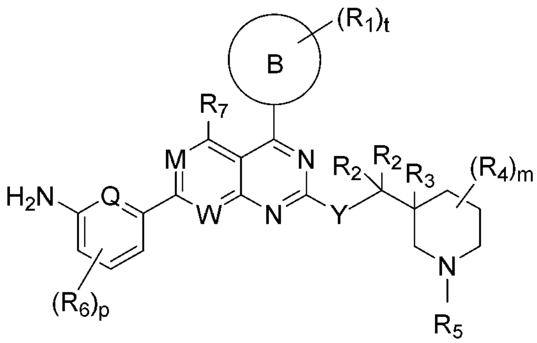

(IV)

wherein the definitions of each group are as defined in the detailed description of the present invention.

The present invention also provides a pharmaceutical composition comprising a compound of the present invention, or a stereoisomer, tautomer, stable isotopic variant, pharmaceutically acceptable salt or solvate thereof, and optionally a pharmaceutically acceptable excipient or carrier.

The present invention also provides a compound of the present invention, or a stereoisomer, tautomer, stable isotopic variant, pharmaceutically acceptable salt or solvate thereof, for use as a medicament.

The present invention also provides a compound of the present invention, or a stereoisomer, tautomer, stable isotopic variant, pharmaceutically acceptable salt or solvate thereof, for use as an inhibitor of RAS proteins, especially KRAS mutant proteins (e.g., G12C mutation, G12D mutation, G12V mutation, G12A mutation, G12R mutation, G12S mutation, G13D mutation and Q61H mutation proteins) and KRAS-amplified cells.

The present invention also provides a compound of the present invention, or a stereoisomer, tautomer, stable isotopic variant, pharmaceutically acceptable salt or solvate thereof, or a pharmaceutical composition comprising the same, for use in the treatment and/or prevention of diseases mediated by RAS proteins, especially KRAS mutant proteins (e.g., G12C mutation, G12D mutation, G12V mutation, G12A mutation, G12R mutation, G12S mutation, G13D mutation and Q61H mutation proteins) and KRAS amplification.

The present invention also provides the use of a compound of the present invention, or a stereoisomer, tautomer, stable isotopic variant, pharmaceutically acceptable salt or solvate thereof, or a pharmaceutical composition comprising the same, for the treatment and/or prevention of diseases mediated by RAS proteins, especially KRAS mutant proteins (e.g., G12C mutation, G12D mutation, G12V mutation, G12A mutation, G12R mutation, G12S mutation, G13D mutation and Q61H mutation proteins) and KRAS amplification.

The present invention also provides the use of a compound of the present invention, or a stereoisomer, tautomer, stable isotopic variant, pharmaceutically acceptable salt or solvate thereof, or a pharmaceutical composition comprising the same, in the preparation of a medicament for the treatment and/or prevention of diseases mediated by RAS proteins, especially KRAS mutant proteins (e.g., G12C mutation, G12D mutation, G12V mutation, G12A mutation, G12R mutation, G12S mutation, G13D mutation and Q61H mutation proteins) and KRAS amplification.

The present invention also provides a method for treating and/or preventing diseases mediated by RAS proteins, especially KRAS mutant proteins (e.g., G12C mutation, G12D mutation, G12V mutation, G12A mutation, G12R mutation, G12S mutation, G13D mutation and Q61H mutation proteins) and KRAS amplification, comprising administering a therapeutically effective amount of a compound of the present invention, or a stereoisomer, tautomer, stable isotopic variant, pharmaceutically acceptable salt or solvate thereof, or a pharmaceutical composition comprising the same, to a subject in need thereof.

The present invention also provides a method for treating tumors or cancers, comprising administering a compound of the present invention, or a stereoisomer, tautomer, stable isotopic variant, pharmaceutically acceptable salt or solvate thereof, or a pharmaceutical composition comprising the same, to a patient in need thereof.

The present invention also provides the use of a compound of the present invention, or a pharmaceutically acceptable salt or solvate thereof, as a research tool compound for inhibiting RAS, particularly for inhibiting KRAS mutant proteins (e.g., G12C mutation, G12D mutation, G12V mutation, G12A mutation, G12R mutation, G12S mutation, G13D mutation and Q61H mutation proteins) and KRAS amplification in research.

The present invention also provides a pharmaceutical combination comprising a compound of the present invention, a stereoisomer, tautomer, stable isotopic variant, pharmaceutically acceptable salt or solvate thereof, and one or more other pharmaceutically active agents.

The present invention also provides a method for preparing the compound of the present invention.

## Detailed description of invention

## Definitions

Unless otherwise indicated, each term used in the specification and the claims shall have the meanings set forth below. In the absence of a specific definition for a particular term or phrase, it shall be construed in accordance with its ordinary meaning in the art. In the event of a conflict, the present specification, including the definitions herein, shall govern.

In the event of a conflict between the chemical structure and the name of a compound disclosed herein, the chemical structure shall govern.

As used herein, the terms "RAS mutation" or "RAS mutant protein" refer to a protein encoded and expressed by a RAS gene with a mutation at one or more codons, typically including, but not limited to, a RAS protein with a mutation at glycine at codon 12, glycine at codon 13 or glutamine at codon 61 of RAS, such as a mutant HRAS, NRAS or KRAS. These residues are located at the active site of RAS, and their mutations can impair the intrinsic or GAP-catalyzed GTPase activity of RAS, leading to the persistent presence of GTP-bound RAS.

For the purposes of the present invention, "RAS mutation" or "RAS mutant protein" and "Ras" targeted at describing inhibitory activity are used interchangeably and generally refer to mutated HRAS, NRAS, or KRAS, for example, but not limited to, KRAS-G12C (mutation of glycine to cysteine at codon G12), KRAS-G12D (mutation of glycine to aspartic acid at codon G12), HRAS-G12D, NRAS-G12D, KRAS-G12V (mutation of glycine to valine at codon G12), KRAS-G13D (mutation of glycine to aspartic acid at codon G13); specifically refers to KRAS mutant protein, i.e., more specifically refers to KRAS-G12C mutant protein, KRAS-G12D mutant protein, KRAS-G12V mutant protein, G12A mutant protein, G12R mutant protein, G12S mutant protein, KRAS-G13D mutant protein, and Q61H mutant protein.

The term "treatment" as used herein refers to the administration of one or more of the compounds of the present invention or pharmaceutically acceptable salts or solvents thereof to cure, relieve, mitigate or affect the disease or its symptoms to subjects suffering from or having symptoms of the disease described, such as mammals, such as humans. Preferably, treatment is curative or improving.

The term "prevention" used herein is well-known in the art and refers to subjects suspected of developing or predisposing to RAS mutation-mediated disease as defined herein, especially cancer or tumors, such as mammals, or humans administered one or more of the compounds described herein or their pharmaceutically acceptable salts or solvents to reduce the risk of developing the disease as defined, or to prevent the onset of the disease. The term "prevention" includes compounds of the present invention used prior to diagnosis or identification of any clinical and/or pathological symptoms.

The terms "inhibit" and "reduce" used herein, or any variant of these terms, refer to the ability of a bioactive agent to reduce the signaling activity of the target by interacting directly or indirectly with the target, and refer to any measurable reduction or complete inhibition of target activity. For example, compared to normal conditions, the reduction in activity (e.g., KRas activity) can be about, at most or at least about 5%, 10%, 15%, 20%, 25%, 30%, 35%, 40%, 45%, 50%, 55%, 60%, 65%, 70%, 75%, 80%, 85%, 90%, 95%, 99% or more, or any range derivable therefrom.

The term "RAS mutation-mediated disease" used herein refers to a disease in which RAS mutations contribute to the development and progression of the described disease, or inhibition of RAS will reduce the incidence of the disease and reduce or eliminate disease symptoms. For the purposes of the present invention, "RAS-mediated disease" refers preferably to KRAS-mediated disease, and further preferably KRAS-mutation mediated cancer or tumor.

The term "cancer" or "tumor" used herein refers to abnormal cell growth and proliferation, whether malignant or benign, and all premalignant cells and cancer cells and tissues. For all aspects of the present invention, said cancers or tumors include, but are not limited to, lung adenocarcinoma, lung cancer, bone cancer, pancreatic cancer, skin cancer, head and neck cancer, skin or intraocular melanoma, uterine cancer, ovarian cancer, rectal cancer, anal region cancer, gastric cancer, colon cancer, breast cancer, fallopian tube cancer, endometrial cancer, cervical cancer, vaginal cancer, vulvar cancer, Hodgkin's disease, esophageal cancer, small bowel cancer, endocrine system cancer, thyroid cancer, parathyroid carcinoma, adrenal carcinoma, soft tissue sarcoma, urethral cancer, penile cancer, prostate cancer, chronic or acute leukemia, lymphocytic lymphoma, bladder cancer, renal or ureteral cancer, renal cell carcinoma, renal pelvis cancer, central nervous system tumor (CNS), primary CNS lymphoma, spinal tumor, brain stem glioma, or pituitary adenoma.

For all aspects of the present invention, the cancer or tumor described is preferably associated with RAS, especially KRAS mutation and amplification, including but not limited to, the tumor types described above and their preferred ranges. The particularly preferred tumors of the present invention include lung cancer, lung adenocarcinoma, colon cancer, rectal cancer, pancreatic cancer, endometrial cancer, cholangiocarcinoma, leukemia, and ovarian cancer.

The terms "subject", "individual", or "patient" used herein refer to vertebrates. In some embodiments vertebrates are mammals. Mammals include, but are not limited to, farm animals (e.g., cattle), locomotor animals, pets (e.g., guinea pigs, cats, dogs, rabbits, and horses), primates, mice, and rats. In certain embodiments, mammals are humans.

The term "therapeutically effective amount" as used herein refers to an amount or dose sufficient to elicit a beneficial therapeutic response in a patient afflicted with a "Ras-mediated disease", such as cancer or tumors in need of treatment. A person skilled in the art may determine the effective amount or dose of the active ingredient of the present

invention using conventional methodologies, in combination with routine influencing factors.

The term "pharmaceutical combination" as used herein refers to a combination of the compound of the present invention with other active agents to achieve the objectives of the present invention. The other active agent(s) may be one or more additional compounds of the present invention, or may be a second or further (e.g., third) compound that is compatible with the compound of the present invention, meaning that they do not adversely affect each other or exhibit complementary activities. For example, such active agents may be known to modulate other biological pathways, or regulate different components of the biological pathway involved in the compound of the present invention, or even overlap with the biological target of the compound of the present invention. These active agents are suitably combined in effective amounts to achieve the intended purpose. The other active agent(s) may be co-administered with the compound of the present invention in a single pharmaceutical composition or administered separately with the compounds of the present invention in distinct discrete units. When administered separately, the administration may be simultaneous or sequential. The sequential administration may be closely spaced or separated by a longer interval.

The term "pharmaceutically acceptable" as used herein refers to molecular entities and compositions that, when administered in appropriate amounts to an animal, such as a human, do not produce adverse, allergic, or other undesirable reactions.

The term "pharmaceutically acceptable salts" used herein refers to those salts that retain the biological validity and properties of the parent compound and are biologically or otherwise not biologically desirable, and include acid and base addition salts. "Pharmaceutically acceptable acid adducts" can be formed from compounds with basic groups with inorganic or organic acids, inorganic acids, such as hydrochloric acid, hydrobromic acid, sulfuric acid, nitric acid, carbonic acid, phosphoric acid, etc. Organic acids can be selected from aliphatic, alicyclic, aromatic, araliphatic, heterocyclic, carboxylic, and sulfonic organic acids, such as formic acid, acetic acid, propionic acid, glycolic acid, gluconic acid, lactic acid, pyruvate, oxalic acid, apple acid, maleic acid, malonic acid, succinic acid, fumaric acid, tartaric acid, citric acid, aspartic acid, ascorbic acid, glutamic acid, anthranilic acid, benzoic acid, cinnamic acid, mandelic acid, pamoate, phenylacetic acid, methanesulfonic acid, ethanesulfonic acid, benzenesulfonic acid, p-toluenesulfonic acid, salicylic acid, etc. "Pharmaceutically acceptable base addition salts" include those derived from inorganic bases, such as salts of sodium, potassium, lithium, ammonium, calcium, magnesium, iron, zinc, copper, manganese, and aluminum, etc, and salts derived from pharmaceutically acceptable organic non-toxic bases, including but not limited to, primary, secondary and tertiary amines, substituted ammonium, including naturally occurring substituted amines, cyclic amines and basic ion exchange resins, such as ammonia, isopropylamine, trimethylamine, diethylamine, triethylamine, tripropylamine, ethanolamine, diethanolamine, 2-bis methyl aminoethanol, 2-diethylaminoethanol, tromethamine, dicyclohexylamine, lysine, arginine, histidine, caffeine, procaine, hydrabamine, choline, betaine, ethylenediamine, glucosamine, methylglucosamine, triethanolamine, theobromine, purine, piperazine, piperidine, N-ethylpiperidine, polyamine resin, etc.

The term "isomer" used herein refers to any stereoisomer, enantiomeric mixture, including racemate, diastereomeric mixture, geometric isomer, atropisomer, and/or tautomer that may be structurally present in a compound. The methods used to identify and separate stereochemistry of the isomers described are well-known by technicians in the art (S. P. Parker, Ed., McGraw-Hill Dictionary of Chemical Chemistry Terms (1984) McGraw-Hill Book Company, New York; and Eliel, E. and Wilen, S.,"Stereochemistry of Organic Compounds", John Wiley & Sons, Inc., New York, 1994).

Certain compounds of the present invention contain **at least** one center of asymmetry and thus give rise to stereoisomers, so the present invention covers all possible isomer forms of compounds defined herein, and pharmaceutically acceptable salts or solvates thereof, unless otherwise indicated.

"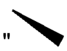" or "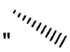" used in the structural formula or structural fragment of a compound herein means the absolute configuration of the stereocenter, that is, the chiral center, and correspondingly R or S is used in the nomenclature of the compound or intermediate provided in the present invention to indicate the absolute configuration about the chiral center; "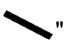" or "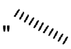" means the relative configuration of the stereocenter, for example, OH and CN in

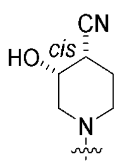

are in cis-configuration, and OH and CN in

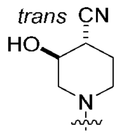

are in trans-configuration; Axial chirality can also be used to indicate the configuration of the compounds of the present invention in certain definitions thereof, where a solid bond denotes the corresponding structural fragment oriented out of the plane of the paper and a dashed bond denotes the corresponding structural fragment oriented into the plane of the paper. For example,

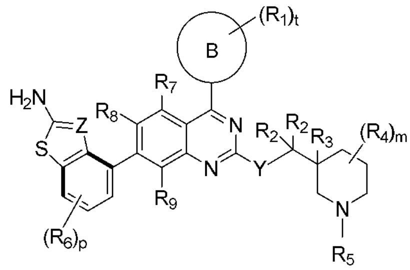

indicates that the benzoheterocyclic side is oriented outwards,

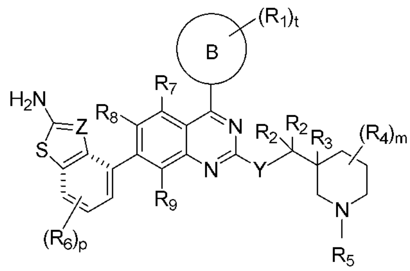

indicates that the benzoheterocyclic side is oriented inwards; the naming of these configurations follows the Cahn-Ingold-Prelog rules well known to those skilled in the art.

An asterisk "*" is also used in the general formulas or examples of the compounds of the present invention to indicate a single axis-chiral compound whose absolute configuration has not yet been determined, for example, the asterisk-labeled position of

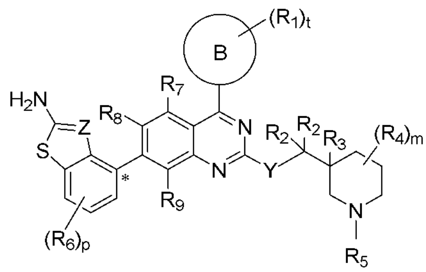

may optionally have axial chirality (e.g., depending on the values of R8 and R9) including

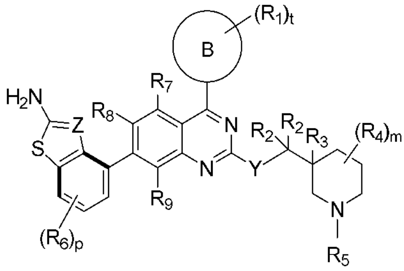

or

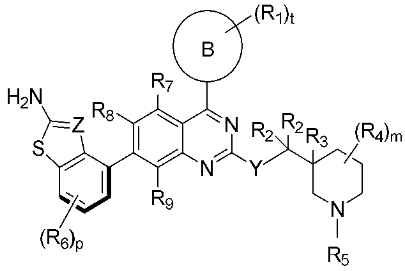

.

It should be understood that when those skilled in the art can determine based on the structure of the compound shown herein, that the compound exists and only exists as a pair of chiral isomers and determine be easily resolved by conventional methods in the art, and determine that it can be easily resolved based on conventional methods in the art, the disclosure (either structural formula or chemical name) of the racemate of the compound in the present invention should be regarded as having disclosed each isomer of the compound separately.

The "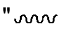" used in the structural fragments referred to herein indicates that the bond it intersects is the bond linking the structural fragment to the rest of the molecule.

Unless otherwise defined, substituents shown as cross-linking chemical bonds in the ring structural fragments referred to herein, for example -$(R_4)_m$ in

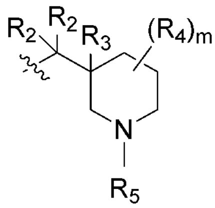

,

means that this defined number of substituents can substitute one or more feasible sites in a ring, wherein m is 0 meaning that the ring does not carry a non-H substituent, but the ring atom still carries a stoichiometric hydrogen atom.

The compound comprises an unlabeled form of the compound and an isotopically labeled form thereof. The isotopically labeled form of a compound is the replacement of a different compound by the corresponding isotopically enriched atom at only one or more atoms. Examples of isotopes that can be incorporated into the compounds of the present invention include isotopes such as hydrogen, carbon, nitrogen, oxygen, fluorine, chlorine and iodine, e.g., $^{2}H$, $^{3}H$, $^{11}C$, $^{13}C$, $^{14}C$, $^{15}N$, $^{18}O$, $^{17}O$, $^{35}S$, $^{18}F$, $^{37}Cl$, and $^{125}I$. Such isotopically labeled compounds may be used as probes, analytical tools, or as therapeutic agents in, for example, bioassays. In certain embodiments, the compound of the present invention is provided in an unlabeled form.

In some embodiments, the compound of the present invention is provided in an unlabeled form. In other embodiments, the compound of the present invention is provided in isotopically labelled form, for example, a compound in which one or more H atoms are replaced by deuterium atoms (D), for example in

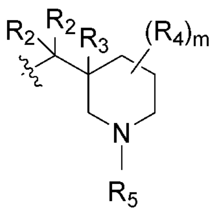

as $R_B$, two $R_2$ each independently can be D, or each defined group substituted by D; $R_5$ can be each defined group substituted by D.

The term "solvate" used herein refers to the solvent addition form of a compound containing stoichiometric or non-stoichiometric solvents, including any solvated form of the compound of the present invention, including, for example, a solvate with water, such as hydrate, or a solvate with an organic solvent, such as methanol, ethanol, or acetonitrile, that is, as a methanolate, ethanolate, or acetonitrile, respectively; or in the form of any polymorph. It should be understood that such solvates of the compounds of the present invention also include solvates of pharmaceutically acceptable salts of the compounds of the present invention.

The term "metabolite" used herein refers to a product generated from the metabolism of a compound *in vivo.*

Such products may be derived, for example, from oxidation, reduction, hydrolysis, amidation, deamidation, esterification, de-esterification, enzymatic cleavage, etc. of the administered compound. Identification and analysis of metabolites are performed in a manner well-known to those skilled in the art.

The term "pharmaceutically acceptable excipient" or "pharmaceutically acceptable carrier" used herein refers to one or more compatible solid or liquid fillers or gel substances that are suitable for human use and have sufficient purity and sufficient low toxicity, in fact examples include, but are not limited, to cellulose and its derivatives (e.g., sodium carboxymethylcellulose, cellulose acetate, etc.), gelatin, talc, solid lubricants (e.g., magnesium stearate), calcium sulfate, vegetable oil, polyols (e.g., propylene glycol, glycerol, mannitol, sorbitol, etc.), emulsifiers (e.g., Tween), humectants (e.g., sodium lauryl sulfate), colorants, flavorants, stabilizers, antioxidants, preservatives, etc.

The term "halogen" or "halogenated" used herein refers to F, Cl, Br, or I. In addition, the term "halogen-substituted" used in the definition of groups herein is intended to include mono- or poly halogenated groups, in which one or more identical or different halogens replace one or more hydrogens in the corresponding group.

The term "alkyl" used herein refers to a straight or branched monovalent saturated hydrocarbon groups consisting of carbon atoms and hydrogen atoms. Specifically, the alkyl has 1-10, examples include 1 to 8, 1 to 6, 1 to 5, 1 to 4, 1 to 3, or 1 to 2 carbon atoms. For example, as used herein, the term "$C_{1-6}$ alkyl" refers to saturated hydrocarbon groups having straight or branched chains of 1 to 6 carbon atoms, examples include methyl, ethyl, propyl (including n-propyl and isopropyl), butyl (including n-butyl, isobutyl, sec-butyl or tert-butyl), pentyl (including n-pentyl, isopentyl, neo-pentyl), n-hexyl, 2-methylpentyl, etc.

The term "alkylene" used herein refers to a straight or branched divalent saturated hydrocarbon group consisting of carbon atoms and hydrogen atoms, preferably a straight divalent saturated hydrocarbon group. Generally, the alkylene has 1 to 10, such as 1 to 8, 1 to 6, 1 to 5, 1 to 4, 1 to 3, or 1 to 2 carbon atoms. For example, as used herein, the terms "-$C_{1-3}$ alkylene-" or "$-(CH_2)_n-$, where n is an integer of 1 to 3" refer to divalent saturated hydrocarbon groups having straight or branched chains of 1 to 3 carbon atoms.

The term "alkoxy" used herein refers to an alkyl defined herein that is connected by an oxygen atom to the rest of the molecule. Specifically, the alkoxy has 1-10, such as 1 to 8, 1 to 6, 1 to 5, 1 to 4, 1 to 3, or 1 to 2 carbon atoms. For example, as used herein, the term "-$C_{1-6}$ alkoxy" or "-O-$C_{1-6}$ alkyl" refers to a straight or branched saturated hydrocarbon group having cha 1 to 6 carbon atoms connected to the rest of the molecule by an oxygen atom, further preferred "-$C_{1-3}$ alkoxy" or "-O-$C_{1-3}$ alkyl", examples of which include -O-methyl, -O-ethyl, -O-propyl (including -O-n-propyl and -O-isopropyl), -O-butyl (including -O-n-butyl, -O-isobutyl, -O-sec-butyl or -O-tert-butyl), -O-pentyl (including -O-n-pentyl, -O-isopentyl, -O-neo-pentyl), -O-n-hexyl, 2-methylpentyl-O-, etc.

As used herein, the term "$C_{1-6}$ alkyl optionally substituted by halogen" refers to the $C_{1-6}$ alkyl described above, wherein one or more (e.g., 1, 2, 3, 4, or 5) hydrogen atoms are optionally replaced by halogen. It will be understood by those skilled in the art that when there is more than one halogen substituent, the halogens may be the same or different, and can be located on the same or different C atoms. Examples of "$C_{1-6}$ alkyl substituted by halogen" include, for example, $-CH_2F$, $-CHF_2$, $-CF_3$, - $CCl_3$, $-C_2F_5$, $-C_2Cl_5$, $-CH_2CF_3$, $-CH_2Cl$, $-CH_2CH_2CF_3$ or $-CF(CF_3)_2$, etc.

The term "alkenyl" used herein refers to a straight or branched unsaturated hydrocarbon group consisting of carbon atoms and hydrogen atoms and containing at least one double bond. Specifically, the alkenyl has 2-8, such as 2 to 6, 2 to 5, 2 to 4, or 2 to 3 carbon atoms. For example, as used herein, the term "$C_{2-6}$ alkenyl" refers to a straight or branched alkenyl with 2 to 6 carbon atoms, and further preferably "$C_{2-4}$ alkenyl", such as vinyl, propenyl, allyl, butenyl, pentenyl, etc., and the carbon atoms in the alkenyl that are connected to the rest of the molecule can be saturated or alkenyl carbon atoms.

The term "alkynyl" used herein refers to a straight or branched unsaturated hydrocarbon group consisting of carbon atoms and hydrogen atoms and containing at least one triple bond. Specifically, the alkynyl has 2-8, such as 2 to 6, 2 to 5, 2 to 4, or 2 to 3 carbon atoms. For example, as used herein, the term "$C_{2-6}$ alkynyl" refers to a straight or branched alkynyl with 2 to 6 carbon atoms, and further preferably "$C_{2-4}$ alkynyl", such as ethynyl, propynyl, butynyl, etc., and carbon atoms in the alkynyl that are connected to the rest of the molecule can be saturated or alkynyl bond carbon atoms.

As used herein, the term "cycloalkyl" refers to a monocyclic, fused polycyclic, bridged polycyclic, or spiro non-aromatic saturated monovalent hydrocarbon ring structure having a specified number of ring carbon atoms. The cycloalkyl groups can have 3 to 12 carbon atoms (i.e., $C_{3-12}$ cycloalkyl), examples include 3 to 10, 3 to 8, 3 to 7, 3 to 6, 5 to 6 carbon atoms. Examples of suitable cycloalkyl groups include, but are not limited to, monocyclic structures such as cyclopropyl, cyclobutyl, cyclopentyl, cyclohexyl, cycloheptyl, or cyclooctyl; or polycyclic (e.g., bicyclic) structures including spiro, fused, or bridged systems, such as bicyclic[1.1.1]pentyl, bicyclic[2.2.1]heptyl, spiro[3.4]octyl, bicyclic[3.1.1]hexyl, bicyclic[3.1.1] heptyl or bicyclic[3.2.1]octyl, etc. For example, the term "$C_{3-6}$ cycloalkyl" or "$C_{3-4}$ cycloalkyl", as used herein refers to monocyclic cyclopropyl, cyclobutyl, cyclopentyl, or cyclohexyl.

As used herein, the term "heterocycloalkyl" refers to a monocyclic, fused polycyclic, spirocyclic or bridged polycyclic non-aromatic saturated or unsaturated ring structure containing one or more (e.g., 1, 2, 3 or 4) heteroatoms independently selected from O, N, P, Se and S and having a specified number of ring atoms, or an N-oxide, S-oxide or S-dioxide thereof. In the case of a polycyclic heterocycloalkyl, it is sufficient that the ring structure connected to the rest of the

molecule is a non-aromatic ring; even if the ring further fused, spiro-fused or bridged to the non-aromatic ring is an aromatic ring, the heterocyclic system is still defined as a heterocycloalkyl herein. The heterocycloalkyl may have 3 to 12 ring members (referred to as a 3- to 12-membered heterocycloalkyl), for example 3 to 10 ring members, 3 to 8 ring members, 3 to 7 ring members, 4 to 7 ring members, 4 to 6 ring members, 5 to 7 ring members, 5 to 6 ring members, 6 to 10 ring members, 6 to 12 ring members, such as a 5- to 7-membered monocyclic heterocycloalkyl (e.g., a 5- to 7-membered monocyclic saturated heterocycloalkyl), a 4- to 7-membered monocyclic heterocycloalkyl; or a 6- to 12-membered polycyclic heterocycloalkyl, such as an 8- to 10-membered spiroheterocycloalkyl, an 8- to 10-membered fused heterocycloalkyl, a 9- to 10-membered fused heterocycloalkyl, a 9- to 11-membered fused heterocycloalkyl, a 9- to 12-membered fused heterocycloalkyl, a 6- to 10-membered spiroheterocycloalkyl, a 6- to 10-membered fused heterocycloalkyl and a 6- to 10-membered bridged heterocycloalkyl. A heterocycloalkyl typically contains at least 1 and up to 4 (e.g., 1, 2, 3 or 4) heteroatoms, for example a 5- to 7-membered monocyclic heterocycloalkyl containing 1 to 3 heteroatoms independently selected from N, O and S (e.g., a 5- to 7-membered monocyclic saturated heterocycloalkyl), a 4- to 7-membered monocyclic heterocycloalkyl (e.g., a 4- to 7-membered monocyclic saturated heterocycloalkyl), such as a 5- to 7-membered monocyclic saturated heterocycloalkyl or a 4- to 7-membered monocyclic saturated heterocycloalkyl containing 1 or 2 N atoms, a 5- to 7-membered monocyclic saturated heterocycloalkyl or a 4- to 7-membered monocyclic saturated heterocycloalkyl containing 1 or 2 heteroatoms selected from N and O atoms, or a 6- to 12-membered polycyclic heterocycloalkyl containing 1 to 4 heteroatoms independently selected from N, O, P, Se and S, for example an 8- to 10-membered spiroheterocycloalkyl, fused heterocycloalkyl or bridged heterocycloalkyl containing 1 to 3 N atoms and 0 to 1 O atom. Examples of suitable heterocycloalkyl groups include, but are not limited to, azetidinyl, oxetanyl, thietanyl, pyrrolidinyl (e.g., 1-pyrrolidinyl, 2-pyrrolidinyl and 3-pyrrolidinyl), tetrahydrofuranyl (e.g., 1-tetrahydrofuranyl, 2-tetrahydrofuranyl and 3-tetrahydrofuranyl), tetrahydrothienyl (e.g., 1-tetrahydrothienyl, 2-tetrahydrothienyl and 3-tetrahydrothienyl), piperidinyl (e.g., 1-piperidinyl, 2-piperidinyl, 3-piperidinyl and 4-piperidinyl), tetrahydropyranyl (e.g., 4-tetrahydropyranyl), tetrahydrothiopyranyl (e.g., 4-tetrahydrothiopyranyl), morpholinyl (e.g., morpholino), thiomorpholinyl, dioxanyl, piperazinyl, azepanyl, diazepanyl (e.g., 1,4-diazepanyl), 3,6-diaza-bicyclo[3.1.1]heptyl and 3-aza-bicyclo[3.2.1]octyl,

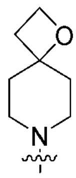 , 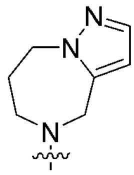 ,

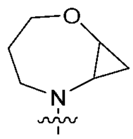 .

The atom in the heterocycloalkyl group connected to the rest of the compound can be a carbon atom or a heteroatom, provided that it is chemically feasible.

The term "heteroaryl" used herein means a monocyclic, fused polycyclic, spiro or bridged polycyclic non-aromatic saturated or unsaturated ring structure consisting of one or more (e.g., 1, 2, 3 or 4) heteroatoms independently selected from O, N, P, Se and S, and a specified number of ring atoms, such as 5-14 membered heteroaryl, 6-10 membered heteroaryl, and the ring connected to the rest of the molecule is aromatic ring. Specifically, examples of heteroaryl groups suitable for the compound of the present invention include, but are not limited to, 6-10 membered heteroaryl containing 1 to 4 heteroatoms independently selected from O, N, Se and S; 5-7 membered monocyclic heteroaryl containing 1 or 3 heteroatoms independently selected from O, N and S, such as 5-7 membered monocyclic heteroaryl containing 1 or 2 heteroatoms independently selected from O, N and S (e.g., 6-membered monocyclic heteroaryl); 9-10 membered fused bicyclic heteroaryl containing 1 to 4 heteroatoms independently selected from O, N, Se and S, such as 9-membered fused bicyclic heterocyclic heteroaryl containing 1 to 4 independently selected from heteroatoms of O, N, Se and S.

The term "aryl" used herein refers to a monocyclic or fused polycyclic aromatic ring structure that include a specified number of ring carbon atoms, such as $C_{6-14}$ aryl, $C_{6-10}$ aryl, with specific examples of aryl are phenyl or naphthyl.

The term "hydroxy" used herein refers to the -OH group.

The term "cyano" used herein refers to the -CN group.

The term "amino" used herein refers to the $-NH_2$.

Substituted amino groups, are also present in the compound definition herein, such as $-NHC_{1-6}$ alkyl, $-N(C_{1-6}$ alkyl$)_2$, wherein with the $-C_{1-6}$ alkyl can be further optionally substituted as shown below each group definition. In some embodiments, the substituted amino group is $-N(R_b)_2$ in - $CON(R_b)_2$, wherein $R_b$ is $-C_{1-6}$ alkyl optionally substituted by

halogen or deuterium. Examples of substituted amino groups include, but are not limited to, $-NH_2$, $-NH-CH_3$, $-NH-CH_2CH_3$, $-NH-CF_3$, $-NH-CH_2CF_3$, $-NH-CH_2CN$, $-NH-CH_2CH_2CN$, $-N(CH_3)_2$, $-N(CD_3)_2$, $-N(CH_3)(CH_2CH_3)$, $-N(CH_3)(CF_3)$, $-N(CH_3)(CH_2CF_3)$, $-N(CH_3)(CH_2CH_2CN)$, $-NHCH_2-OCH_3$, $N(CH_3)(CH_2CH_2-OCH_3)$.

The term "optionally substituted" used herein means, unless otherwise stated, that a group may be unsubstituted or substituted by one or more (e.g., 1, 2, 3, 4, or 5 or more, or any range derived therein) for the substituents listed in that group, wherein each of the substituents may be the same or different when multiple substituents are present. In one embodiment, the optionally substituted group has 1 substituent. In another embodiment, the optionally substituted group has 2 identical or different substituents. In another embodiment, the optionally substituted group has 3 identical or different substituents. In another embodiment, the optionally substituted group has 4 identical or different substituents. In another embodiment, the optionally substituted group has 5 identical or different substituents.

Many of the groups defined herein are optionally substituted, and the list of substituents given in this definition section is only exemplary and does not intend to limit the substituents defined elsewhere in this specification and claims.

In the compound definition herein, H carried by saturated carbon atoms can be unrepresented in the compound definition, and those skilled in the art can easily determine the number of H or non-H substituents carried by atoms, for example, in the structural fragment

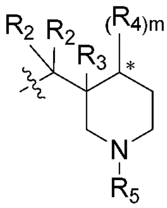

,

when m is 1 and $R_4$ is non-H, there is an unrepresented H in the ring carbon atom to which it is connected; when m is 2 and $R_4$ is non-H, the ring carbon atom connected to it does not carry H atoms, and the two $R_4$ carried by it can be respectively to connected to the ring carbon atoms shown by a single bond, or together form a double bond connected to the ring carbon atoms shown, or together form a spiro ring with the carbon atom shown together.

Unless otherwise specified, $C_{n-n+m}$ or $C_n$-$C_m$ in the definition of the compound of the present invention includes various cases of n to n+m carbons, for example, $C_{1-6}$ includes $C_1$, $C_2$, $C_3$, $C_4$, $C_5$ and $C_6$, and includes any range from n to n+m, for example, $C_{0-6}$ includes $C_1$, $C_2$, $C_3$, $C_4$, $C_5$, $C_6$, $C_{0-1}$, $C_{0-2}$, $C_{0-3}$, $C_{0-4}$, $C_{0-5}$, $C_{1-2}$, $C_{1-3}$, $C_{1-4}$, $C_{2-3}$, etc., $C_{1-6}$ includes $C_{1-2}$, $C_{1-3}$, $C_{1-4}$, $C_{2-6}$, $C_{3-6}$, etc. Similarly, the n-membered to n+m-membered in the definition of the compounds of the present invention represents the number of ring atoms from n to n+m, for example, 3-12 membered ring includes 3 membered ring, 4 membered ring, 5 membered ring, 6 membered ring, 12 membered ring, etc., and also includes any range of n to n+m-membered, for example, 3-12 membered ring includes 3-6 membered ring, 3-8 membered ring, 3-9 membered ring, 4-10 membered ring, 4-7 membered ring, 4-5 membered ring, 5-6 membered ring, 5-7 membered ring, 5-8 membered ring, 5-9 membered ring, 6-7 membered ring, 6-8 membered ring, 6-10 membered ring, and 6-12 membered ring, etc.

It will be understood by those skilled in the art of organic synthesis that the various groups carried in the structure of the compounds of the present invention, whether unsubstituted or substituted by various defined substituents, are subject to the premise that the compound molecule is chemically feasible and stable, wherein the type and number of substituents are determined by the number of atoms and valency in the group.

As used in this specification and the claims that follow, the term "comprise" and its variants such as "include" and "contain" mean "including but not limited to" and are not intended to exclude, for example, other additives, components, integers or steps. When an element is described as comprising a plurality of components, steps or conditions, it is to be understood that the element may also be described as comprising any combination of the plurality of components, steps or conditions, or as "consisting of" or "consisting essentially of" the plurality or combination of components, steps or conditions.

It is to be understood that the dosages referred to herein in the specification of the compounds of the present invention, the pharmaceutical compositions, pharmaceutical combinations and kits containing the same, as well as the related uses and methods, are based on the weight of the free form, excluding any salts, hydrates or solvates thereof, unless the specification indicates that the dosage is based on the weight of the salt, hydrate or solvate.

## Problem solved by the present invention

As described above, compounds capable of inhibiting RAS proteins, especially KRAS mutant proteins (e.g., G12C, G12D, G12V, G12A, G12R, G12S, G13D and Q61H mutant proteins), and KRAS wild-type amplified cells are useful for the treatment or prevention of diseases mediated by said proteins (e.g., cancers or tumors). Accordingly, a

variety of structural types of RAS inhibitors have been developed in the art. However, existing KRAS inhibitors still have problems to be addressed, including, for example, unsatisfactory anti-tumor activity of many inhibitors, or poor drug resistance due to toxic and side effects, or insufficient pharmacokinetic properties to allow administration via a convenient route i.e., poor "drugability", or undesirable drug interactions caused by the inhibition of the cytochrome P450 enzyme system, and the like. Furthermore, even for inhibitors with favorable anti-tumor activity, it is still desirable to further improve their selective inhibitory activity against the target protein *in vivo,* further enhance their drug resistance (fewer toxic and side effects or better safety) and further optimize their pharmacokinetic properties through structural optimization, so as to provide more and better therapeutic options for clinical practice.

## Problem solving method

Through extensive and intensive research, a group of compounds was developed with significant inhibitory activity against RAS mutant proteins, especially KRAS mutant proteins (e.g., G12C, G12D, G12V, G12A, G12R, G12S, G13D and Q61H mutant proteins) and KRAS amplified cells. Through structural modification and activity verification, the inventor finds that at several specific sites of the benzopyrimidine ring and quinazoline of the KRAS inhibitor structure, a specific type of substituent modification was performed, and the specific combination of several substitution sites and substituent types implemented obtained a further improved inhibitory activity against KRAS mutant proteins compared to the prior art inhibitors, and the compounds obtained by such modification have good safety, reduced risk of drug interactions, and good, or even further improved, pharmacokinetic properties, which allows for convenient administration.

The present invention primarily provides potent RAS inhibitors, specifically KRAS (e.g., G12C mutation, G12D mutation, G12V mutation, G12A mutation, G12R mutation, G12S mutation, G13D mutation, Q61H mutation and KRAS amplification) inhibitor compounds; pharmaceutical compositions containing such compounds as active ingredients; the compound as a medicant, used for treating or preventing tumor or cancer mediated by RAS, specifically KRAS (e.g., G12C mutation, G12D mutation, G12V mutation, G12A mutation, G12R mutation, G12S mutation, G13D mutation, Q61H mutation, and KRAS amplification) or benefited from the inhibition of RAS, specifically KRAS (e.g., G12C mutation, G12D mutation, G12V mutation, G12A mutation, G12R mutation, G12S mutation, G13D mutation, Q61H mutation and KRAS amplification); and the method of the use of the compound for treating or preventing a disease (such as tumor or cancer) mediated by RAS, specifically KRAS (e.g., G12C mutation, G12D mutation, G12V mutation, G12A mutation, G12R mutation, G12S mutation, G13D mutation, Q61H mutation, and KRAS amplification) or benefited from the inhibition of RAS, specifically KRAS (e.g., G12C mutation, G12D mutation, G12V mutation, G12A mutation, G12R mutation, G12S mutation, G13D mutation, Q61H mutation and KRAS amplification); and the use of the compound in the preparation of a medicant for treating or preventing a disease (such as tumor or cancer) mediated by RAS, specifically KRAS (e.g., G12C mutation, G12D mutation, G12V mutation, G12A mutation, G12R mutation, G12S mutation, G13D mutation, Q61H mutation, and KRAS amplification) or benefited from the inhibition of RAS, specifically KRAS (e.g., G12C mutation, G12D mutation, G12V mutation, G12A mutation, G12R mutation, G12S mutation, G13D mutation, Q61H mutation and KRAS amplification).

It should be understood that the various types of KRAS mutations mentioned herein also include combinations of two or more of the listed KRAS mutations. The present invention thus provides the following technical solutions.

## Compounds of the present invention

As used throughout this application, the terms "compounds of the invention" and "compounds of the present invention" and the like, unless otherwise defined, encompass the compounds defined in the various embodiments and preferred embodiments herein and their various specific embodiments, including isomers thereof, including atropisomers, mixtures of enantiomers (in particular racemates), mixtures of diastereomers, geometric isomers, tautomers, solvates, metabolites, isotopic variants and salts (e.g., pharmaceutically acceptable salts) thereof.

Accordingly, the aforementioned various isomers and derivatives of the compounds of the present invention are all thus encompassed within the scope of the present invention, the respective meanings, preparation and specific examples of which are as defined in the Definitions section above or are well known in the art. Preferably, however, they are the compounds of the present invention and/or pharmaceutically acceptable salts or solvates thereof.

The present invention also encompasses N-oxides of the compounds of the present invention, provided that such compounds contain a basic nitrogen atom (e.g., a nitrogen atom present in a nitrogen-containing heterocycle) and are chemically and biologically feasible. Certain compounds of the present invention may exist in polymorphic or amorphous forms, and as such they also fall within the scope of the present invention.

In the first aspect, the present invention provides the following embodiments of the compounds:

**Embodiment 1:** A compound of formula (I), a stereoisomer, tautomer, stable isotopic variant, pharmaceutically acceptable salt or solvate thereof,

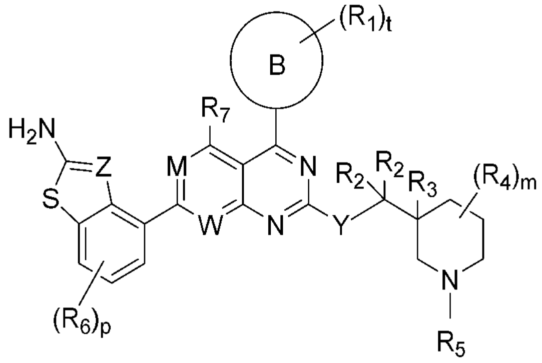

(I)

wherein:

M is selected from N or C-$R_8$;

W is selected from N or C-$R_9$;

Z is selected from N or C-$R_{10}$;

Y is selected from O, S, Se and N-$R_a$;

B is selected from 5-7 membered monocyclic heterocycloalkyl containing 1 to 3 heteroatoms independently selected from N, O, P, S and Se, and 6-12 membered polycyclic heterocycloalkyl containing 1 to 4 heteroatoms independently selected from N, O, P, S and Se, provided that the ring atom attached to the pyrimidine ring portion of the molecule is nitrogen heteroatom;

$R_1$ is selected from H, halogen, -CN, -OH, -$B(OH)_2$, -$C_{1-6}$ alkyl, -O-$C_{1-6}$ alkyl, -C(O)N($R_b$)$_2$), wherein the -$C_{1-6}$ alkyl present is optionally substituted by halogen;

$R_2$ is each independently selected from H, D, -$C_{1-6}$ alkyl and -$(CH_2)_n$-$C_{3-6}$ cycloalkyl, or two $R_2$ together with the carbon atom to which they are attached form a $C_{3-4}$ cycloalkyl, wherein the $C_{1-6}$ alkyl, $C_{3-6}$ cycloalkyl or $C_{3-4}$ cycloalkyl is each independently and optionally substituted by halogen, D, $C_{1-6}$ alkyl optionally substituted by halogen or D, or -O-$C_{1-6}$ alkyl optionally substituted by halogen or D;

$R_3$ is selected from -$C_{1-6}$ alkyl and -$(CH_2)_n$-$C_{3-6}$ cycloalkyl, each independently and optionally substituted by halogen, D, $C_{1-6}$ alkyl optionally substituted by halogen or D, or -O-$C_{1-6}$ alkyl optionally substituted by halogen or D.

$R_4$ is selected from H, D, halogen, -CN, -OH, -$C_{1-6}$ alkyl, -$C_{2-6}$ alkenyl, -$C_{2-6}$ alkynyl, -$NH_2$, - NH-$C_{1-6}$ alkyl, -N($C_{1-6}$ alkyl)$_2$, -O-$C_{1-6}$ alkyl, -O-$C_{3-6}$ cycloalkyl and -$(CH_2)_n$-$C_{3-6}$ cycloalkyl; wherein the -$C_{1-6}$ alkyl, -$C_{2-6}$ alkenyl, -$C_{2-6}$ alkynyl or -$C_{3-6}$ cycloalkyl present is each independently and optionally substituted by D, halogen, CN, or -O-$C_{1-6}$ alkyl; or

two $R_4$ attached to the same ring carbon atom form =C($R_c$)$_2$, or two $R_4$ attached to the same ring carbon atom together with the ring carbon atom to which they are attached form a spiro $C_{3-6}$ cycloalkyl or 4-7 membered heterocycloalkyl, wherein each $R_c$ is independently selected from H, halogen and - $C_{1-6}$ alkyl optionally substituted by halogen, and the spiro $C_{3-6}$ cycloalkyl or spiro 4-7 membered heterocycloalkyl is optionally substituted by halogen and -$C_{1-6}$ alkyl optionally substituted by halogen;

$R_5$ is selected from H, -$C_{1-6}$ alkyl, -$C_{2-6}$ alkenyl, -$C_{2-6}$ alkynyl and -$(CH_2)_n$-$C_{3-6}$ cycloalkyl, wherein the -$C_{1-6}$ alkyl, -$C_{2-6}$ alkenyl, -$C_{2-6}$ alkynyl or $C_{3-6}$ cycloalkyl is each independently and optionally substituted by D, halogen, CN, or -O-$C_{1-6}$ alkyl or -O-CON($R_b$)$_2$;

$R_6$ and $R_7$ are each independently selected from H, D, halogen, CN, -$C_{1-6}$ alkyl, -$C_{2-6}$ alkenyl, - $C_{2-6}$ alkynyl, -O-$C_{1-6}$ alkyl, -S-$C_{1-6}$ alkyl, and -Se-$C_{1-6}$ alkyl, wherein the -$C_{1-6}$ alkyl, -$C_{2-6}$ alkenyl, and -$C_{2-6}$ alkynyl present are each independently and optionally substituted by halogen;

$R_8$ is selected from H, D, halogen, CN, -$C_{1-6}$ alkyl, -$C_{2-6}$ alkenyl, and -$C_{2-6}$ alkynyl, and -$OR_b$, wherein the -$C_{1-6}$ alkyl, -$C_{2-6}$ alkenyl, and -$C_{2-6}$ alkynyl are each independently and optionally substituted by halogen, CN or -O-$C_{1-6}$ alky; or $R_8$ is -$(CH_2)_n$-$C_{3-6}$ cycloalkyl, wherein the $C_{3-6}$ cycloalkyl is optionally substituted by halogen or CN;

$R_{10}$ is selected from H, D, halogen, CN, -$C_{1-6}$ alkyl, -$C_{2-6}$ alkenyl, -$C_{2-6}$ alkynyl, and -$OR_b$, wherein the -$C_{1-6}$ alkyl, -$C_{2-6}$ alkenyl, and -$C_{2-6}$ alkynyl are each independently and optionally substituted by halogen or CN;

$R_9$ is selected from H, halogen, CN, -$C_{1-6}$ alkyl, -$C_{2-6}$ alkenyl, -$C_{2-6}$ alkynyl, -$OR_b$, and -O-CON$(R_b)_2$, wherein the -$C_{1-6}$ alkyl, -$C_{2-6}$ alkenyl, and -$C_{2-6}$ alkynyl are each independently and optionally substituted by halogen or CN;

$R_a$ and $R_b$ are each independently selected from H and -$C_{1-6}$ alkyl optionally substituted by halogen; or two $R_b$ attached to the same N atom together with the N atom to which they are attached form a 4-7 membered heterocycloalkyl;

n and p are each independently an integer from 0 to 3; and

m and t are each independently an integer from 0 to 8;

In a specific embodiment, $R_8$ and $R_{10}$ are each independently selected from H, D, halogen, CN, -$C_{1-6}$ alkyl, -$C_{2-6}$ alkenyl, -$C_{2-6}$ alkynyl, and -$OR_b$, wherein the -$C_{1-6}$ alkyl, -$C_{2-6}$ alkenyl, and -$C_{2-6}$ alkynyl are each independently and optionally substituted by halogen or CN; $R_a$ and $R_b$ are each independently selected from H and -$C_{1-6}$ alkyl optionally substituted by halogen; and each remaining group is as defined in Formula (I) above.

**Embodiment 1.1:** The compound of Formula (I) according to Embodiment 1, a stereoisomer, tautomer, stable isotopic variant, pharmaceutically acceptable salt or solvate thereof, wherein Z is N.

**Embodiment 1.2:** The compound of Formula (I) according to Embodiment 1, a stereoisomer, tautomer, stable isotopic variant, pharmaceutically acceptable salt or solvate thereof, wherein Z is C-$R_{10}$.

**Embodiment 1.2.1:** The compound of Formula (I) according to Embodiment 1.2, a stereoisomer, tautomer, stable isotopic variant, pharmaceutically acceptable salt or solvate thereof, wherein $R_{10}$ is H; or $R_{10}$ is D.

**Embodiment 1.2.2:** The compound of Formula (I) according to Embodiment 1.2, a stereoisomer, tautomer, stable isotopic variant, pharmaceutically acceptable salt or solvate thereof, wherein $R_{10}$ is halogen, e.g., F, Cl, Br, I.

**Embodiment 1.2.3:** The compound of Formula (I) according to Embodiment 1.2, a stereoisomer, tautomer, stable isotopic variant, pharmaceutically acceptable salt or solvate thereof, wherein $R_{10}$ is CN.

**Embodiment 1.2.4:** The compound of Formula (I) according to Embodiment 1.2, a stereoisomer, tautomer, stable isotopic variant, pharmaceutically acceptable salt or solvate thereof, wherein $R_{10}$ is - $C_{1-6}$ alkyl, preferably -$C_{1-3}$ alkyl, optionally substituted by halogen or CN; for example, but not limited to, -$CH_3$, -$CH_2CH_3$, -$CH_2CH_2CH_3$, -$CH(CH_3)(CH_3)$, -$CH_2CH_2CH_2CH_3$, -$CH_2CH(CH_3)CH_3$, - $C(CH_3)_3$, -$CH_2Cl$, -$CH_2F$, -$CHF_2$, -$CF_3$, -$CCl_3$, -$CH_2CN$, -$CH_2CH_2F$, -$CH_2CH_2CN$, -$CH_2CHF_2$, - $CH_2CF_3$, -$CH_2CH_2CH_2F$, -$CH_2CH_2CHF_2$, -$CH_2CH_2CF_3$, -$C(CH_3)_2CF_3$, -$C_2F_5$, -$C_2Cl_5$.

**Embodiment 1.2.5:** The compound of Formula (I) according to Embodiment 1.2, a stereoisomer, tautomer, stable isotopic variant, pharmaceutically acceptable salt or solvate thereof, wherein $R_{10}$ is - $C_{2-6}$ alkenyl, preferably -$C_{2-4}$ alkenyl, optionally substituted by halogen or CN; for example, but not limited to, -$CH=CH_2$, -CH=CHF, -$CH=CF_2$, -$CF=CF_2$, -CH=CHCN, -$CH_2CH=CH_2$, -$CH_2$CH=CHCN, -$CH_2CH=CF_2$, -$CH_2CF=CF_2$, -$C(CH_3)=CH_2$, -$C(CF_3)=CH_2$, -$C(CH_3)=CF_2$, -$CH=CHCF_3$, - $C(CH_3)=CHCF_3$, -$CH_2CH=CHCF_3$.

**Embodiment 1.2.6:** The compound of Formula (I) according to Embodiment 1.2, a stereoisomer, tautomer, stable isotopic variant, pharmaceutically acceptable salt or solvate thereof, wherein $R_{10}$ is - $C_{2-6}$ alkynyl, preferably -$C_{2-4}$ alkynyl, optionally substituted by halogen or CN; for example, but not limited to, -$C\equiv C(CF_3)$, -$CH_2C\equiv C(CF_3)$,

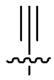, 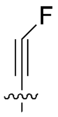, 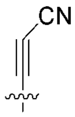, 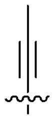, 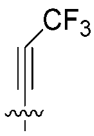, 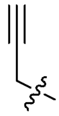, 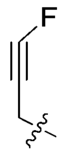.

**Embodiment 1.2.7:** The compound of Formula (I) according to Embodiment 1.2, a stereoisomer, tautomer, stable isotopic variant, pharmaceutically acceptable salt or solvate thereof, wherein $R_{10}$ is - $OR_b$; or $R_b$ is H or -$C_{1-6}$ alkyl optionally substituted by halogen, preferably -$C_{1-3}$ alkyl; for example, but not limited to, -O-$CH_3$, -O-$CH_2CH_3$, -O-$CH_2CH_2CH_3$, -O-CH($CH_3$)($CH_3$), -$OCH_2Cl$, -$OCH_2F$, - $OCHF_2$, -$OCF_3$, -$OCH_2CH_2F$, -$OCH_2CHF_2$, -$OCH_2CF_3$, -$OCH_2CH_2CH_2F$, -$OCH_2CH_2CHF_2$, - $OCH_2CH_2CF_3$.

**Embodiment 1.3:** The compound of Formula (I) according to Embodiment 1, a stereoisomer, tautomer, stable isotopic variant, pharmaceutically acceptable salt or solvate thereof, wherein Z is C-$R_{10}$, preferably Z is C-CN.

**Embodiment 2.1:** The compound of Formula (I) according to any one of Embodiments 1 to 1.3, a stereoisomer, tautomer, stable isotopic variant, pharmaceutically acceptable salt or solvate thereof, wherein p is 0.

**Embodiment 2.2:** The compound of Formula (I) according to any one of Embodiments 1 to 1.3, a stereoisomer, tautomer, stable isotopic variant, pharmaceutically acceptable salt or solvate thereof, wherein p is 1; or p is 2; or p is 3.

**Embodiment 2.2.1:** The compound of Formula (I) according to any one of Embodiments 1 to 1.3, a stereoisomer, tautomer, stable isotopic variant, pharmaceutically acceptable salt or solvate thereof, wherein $R_6$ is H; or $R_6$ is D.

**Embodiment 2.2.2:** The compound of Formula (I) according to Embodiment 2.2, a stereoisomer, tautomer, stable isotopic variant, pharmaceutically acceptable salt or solvate thereof, wherein $R_6$ is halogen, e.g., F, Cl, Br, I, preferably F;

preferably, there is one $R_6$ that is halogen, and the $R_6$ is located at the para, ortho or meta position relative to the junction point of the benzo-fused ring where it is present and the rest of the molecule, more preferably the para position, as shown

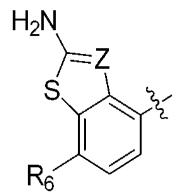;

or

preferably, there are two $R_6$ that are halogen, and the two $R_6$ are respectively located at the para and ortho positions, the para and meta positions, or the ortho and meta positions relative to the junction point of the benzo-fused ring where they are present and the rest of the molecule, more preferably the para and ortho positions, as shown

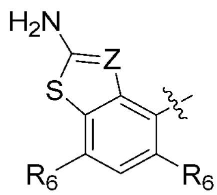.

**Embodiment 2.2.3:** The compound of Formula (I) according to Embodiment 2.2, a stereoisomer, tautomer, stable isotopic variant, pharmaceutically acceptable salt or solvate thereof, wherein there is one $R_6$, which is selected from CN, -$C_{1-6}$ alkyl, -$C_{2-6}$ alkenyl, and -$C_{2-6}$ alkynyl, -O-$C_{1-6}$ alkyl, -S-$C_{1-6}$ alkyl, and -Se-$C_{1-6}$ alkyl; wherein the -$C_{1-6}$ alkyl, -$C_{2-6}$ alkenyl, and -$C_{2-6}$ alkynyl present are preferably -$C_{1-3}$ alkyl, -$C_{2-4}$ alkenyl, -$C_{2-4}$ alkynyl, each independently and

optionally substituted by halogen, and the $R_6$ is located at the para, ortho or meta position relative to the junction point of the benzo-fused ring where it is present and the rest of the molecule; wherein the optionally halogen-substituted $-C_{1-6}$ alkyl, $-C_{2-6}$ alkenyl and $-C_{2-6}$ alkynyl present are for example, but not limited to, those respectively set forth in Embodiment 1.2.4 to 1.2.6.

**Embodiment 2.2.4:** The compound of Formula (I) according to Embodiment 2.2, a stereoisomer, tautomer, stable isotopic variant, pharmaceutically acceptable salt or solvate thereof, wherein there are two $R_6$; one of which is selected from halogen (preferably F) or CN, and the other is selected from CN, $C_{1-6}$ alkyl, $C_{2-6}$ alkenyl, $C_{2-6}$ alkynyl, O-$C_{1-6}$ alkyl, S-$C_{1-6}$ alkyl and Se-$C_{1-6}$ alkyl; wherein the $C_{1-6}$ alkyl, $C_{2-6}$ alkenyl and $C_{2-6}$ alkynyl present are preferably $C_{1-3}$ alkyl, $C_{2-4}$ alkenyl and $C_{2-4}$ alkynyl, each independently and optionally substituted by halogen, and the two $R_6$ are respectively located at the para and ortho positions, the para and meta positions, or the ortho and meta positions relative to the junction point of the benzo-fused ring where they are present and the rest of the molecule, more preferably the para and ortho positions; wherein the optionally halogen-substituted $C_{1-6}$ alkyl, $C_{2-6}$ alkenyl and $C_{2-6}$ alkynyl present include, but are not limited to, those respectively set forth in Embodiments 1.2.4 to 1.2.6.

**Embodiment 2.2.5:** The compound of Formula (I) according to Embodiment 2.2, a stereoisomer, tautomer, stable isotopic variant, pharmaceutically acceptable salt or solvate thereof, wherein there are two $R_6$, each independently selected from $C_{1-6}$ alkyl, $C_{2-6}$ alkenyl, $C_{2-6}$ alkynyl, O-$C_{1-6}$ alkyl, S-$C_{1-6}$ alkyl and Se-$C_{1-6}$ alkyl; wherein the $C_{1-6}$ alkyl, $C_{2-6}$ alkenyl and $C_{2-6}$ alkynyl present are preferably $C_{1-3}$ alkyl, $C_{2-4}$ alkenyl and $C_{2-4}$ alkynyl, each independently and optionally substituted by halogen, and the two $R_6$ are respectively located at the para and ortho positions, or the para and meta positions, or the ortho and meta positions relative to the junction point of the benzo-fused ring where they are present and the rest of the molecule, more preferably the para and ortho positions; wherein the optionally halogen-substituted $C_{1-6}$ alkyl, $C_{2-6}$ alkenyl and $C_{2-6}$ alkynyl present for example, but are not limited to, those respectively set forth in Embodiments 1.2.4 to 1.2.6..

**Embodiment 2.2.6:** The compound of Formula (I) according to Embodiment 2.2, a stereoisomer, tautomer, stable isotopic variant, pharmaceutically acceptable salt or solvate thereof, wherein the benzo-fused ring fragment containing Z is for example, but not limited to

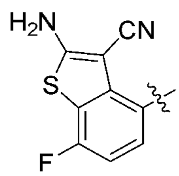,

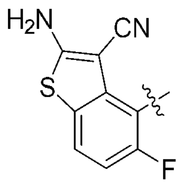, 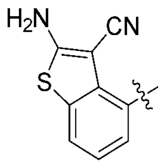, 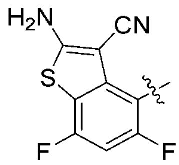, 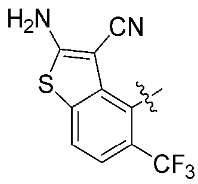, 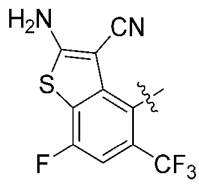, 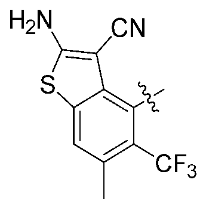,

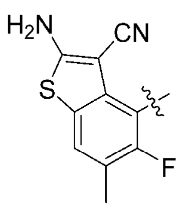, 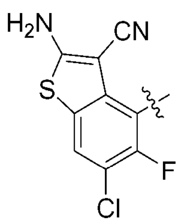, 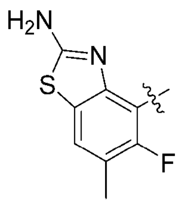, 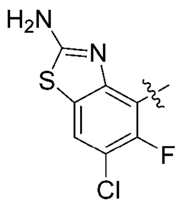, 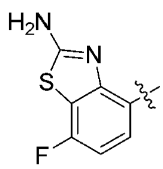, 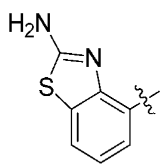,

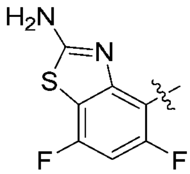, 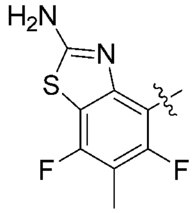, 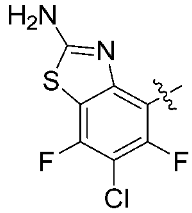.

**Embodiment 2.3:** The compound of Formula (I) according to any one of Embodiments 1 to 1.3, a stereoisomer, tautomer, stable isotopic variant, pharmaceutically acceptable salt or solvate thereof, wherein the benzo-fused ring fragment containing Z is

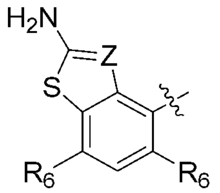,

more specifically

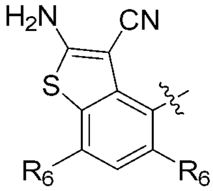,

wherein each $R_6$ is independently selected from H and halogen, preferably F; e.g.,

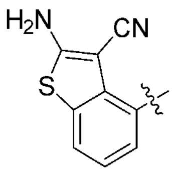, 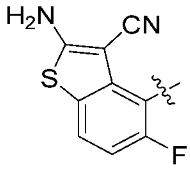, 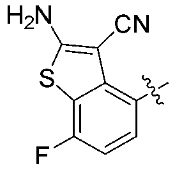, 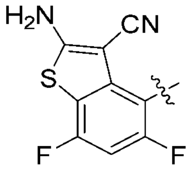.

**Embodiment 3.1:** The compound of Formula (I) according to any one of Embodiments 1 to 2.3, a stereoisomer, tautomer, stable isotopic variant, pharmaceutically acceptable salt or solvate thereof, wherein M is N.

**Embodiment 3.2:** The compound of Formula (I) according to any one of Embodiments 1 to 2.3, a stereoisomer, tautomer, stable isotopic variant, pharmaceutically acceptable salt or solvate thereof, wherein M is C-$R_8$.

**Embodiment 3.2.1:** The compound of Formula (I) according to Embodiment 3.2, a stereoisomer, tautomer, stable isotopic variant, pharmaceutically acceptable salt or solvate thereof, wherein $R_8$ is H; or $R_8$ is D.

**Embodiment 3.2.2:** The compound of Formula (I) according to Embodiment 3.2, a stereoisomer, tautomer, stable isotopic variant, pharmaceutically acceptable salt or solvate thereof, wherein $R_8$ is halogen, e.g., F, Cl, Br, I.

**Embodiment 3.2.3:** The compound of Formula (I) according to Embodiment 3.2, a stereoisomer, tautomer, stable isotopic variant, pharmaceutically acceptable salt or solvate thereof, wherein $R_8$ is CN.

**Embodiment 3.2.4:** The compound of Formula (I) according to Embodiment 3.2, a stereoisomer, tautomer, stable isotopic variant, pharmaceutically acceptable salt or solvate thereof, wherein $R_8$ is - $C_{1-6}$ alkyl, preferably -$C_{1-3}$ alkyl, optionally substituted by halogen or CN, or optionally substituted by -O-$C_{1-6}$ alkyl; for example, but not limited to the groups exemplified in Embodiment 1.2.4; preferably $R_8$ is -$CF_3$, or $R_8$ is -$CH_2$-$CH_3$, or $R_8$ is -$CH_2$-O-$CH_3$.

**Embodiment 3.2.5:** The compound of Formula (I) according to Embodiment 3.2, a stereoisomer, tautomer, stable isotopic variant, pharmaceutically acceptable salt or solvate thereof, wherein $R_8$ is - $C_{2-6}$ alkenyl, preferably -$C_{2-4}$ alkenyl, optionally substituted by halogen or CN; for example, but not limited to the groups exemplified in Embodiment 1.2.5.

**Embodiment 3.2.6:** The compound of Formula (I) according to Embodiment 3.2, a stereoisomer, tautomer, stable isotopic variant, pharmaceutically acceptable salt or solvate thereof, wherein $R_8$ is - $C_{2-6}$ alkynyl, preferably -$C_{2-4}$ alkynyl, optionally substituted by halogen or CN; for example, but not limited to the groups exemplified in Embodiment 1.2.6.

**Embodiment 3.2.7:** the compound of Formula (I) according to Embodiment 3.2, a stereoisomer, tautomer, stable isotopic variant, pharmaceutically acceptable salt or solvate thereof, wherein $R_8$ is - $OR_b$, wherein $R_b$ is H or $C_{1-6}$ alkyl optionally substituted by halogen, preferably -$C_{1-3}$ alkyl optionally substituted by halogen; for example, but not limited to the groups exemplified in Embodiment 1.2.7.

**Embodiment 3.2.8:** The compound of Formula (I) according to Embodiment 3.2, a stereoisomer, tautomer, stable isotopic variant, pharmaceutically acceptable salt or solvate thereof, wherein $R_8$ is - $(CH_2)_n$-$C_{3-6}$ cycloalkyl, wherein n is integer of 0 to 3, preferably -$C_{3-6}$ alkyl, for example, but not limited to the groups exemplified in Embodiment 8.4; in this case, the fused parent ring containing M and W can be, for example:

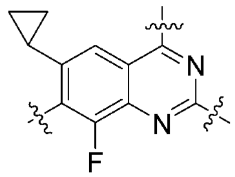

.

**Embodiment 3.3:** The compound of Formula (I) according to any one of Embodiments 1 to 2.3, a stereoisomer, tautomer, stable isotopic variant, pharmaceutically acceptable salt or solvate thereof, wherein M is -$C_{1-6}$ alkyl optionally substituted by halogen, preferably -$C_{1-3}$ alkyl optionally substituted by halogen, preferably C-$CF_3$; or M is C-$C_{1-6}$ alkyl, preferably C-$C_{1-3}$ alkyl, e.g., C-$CH_2$-$CH_3$; or M is C-$C_{1-6}$ alkyl-O-$C_{1-6}$ alkyl, preferably C-$C_{1-3}$ alkyl-O-$C_{1-3}$ alkyl, e.g., C-$CH_2$-O-$CH_3$.

**Embodiment 4.1:** The compound of Formula (I) according to any one of Embodiments 1 to 3.3, a stereoisomer, tautomer, stable isotopic variant, pharmaceutically acceptable salt or solvate thereof, wherein W is N.

**Embodiment 4.2:** The compound of Formula (I) according to any one of Embodiments 1 to 3.3, a stereoisomer, tautomer, stable isotopic variant, pharmaceutically acceptable salt or solvate thereof, wherein W is C-$R_9$.

**Embodiment 4.2.1:** The compound of Formula (I) according to Embodiment 4.2, a stereoisomer, tautomer, stable isotopic variant, pharmaceutically acceptable salt or solvate thereof, wherein $R_9$ is H.

**Embodiment 4.2.2:** The compound of Formula (I) according to Embodiment 4.2, a stereoisomer, tautomer, stable isotopic variant, pharmaceutically acceptable salt or solvate thereof, wherein $R_9$ is halogen, e.g., F, Cl, Br, I, preferably F.

**Embodiment 4.2.3:** The compound of Formula (I) according to Embodiment 4.2, a stereoisomer, tautomer, stable isotopic variant, pharmaceutically acceptable salt or solvate thereof, wherein $R_9$ is CN.

**Embodiment 4.2.4:** The compound of Formula (I) according to Embodiment 4.2, a stereoisomer, tautomer, stable isotopic variant, pharmaceutically acceptable salt or solvate thereof, wherein $R_9$ is selected from -$C_{1-6}$ alkyl, -$C_{2-6}$ alkenyl, and -$C_{2-6}$ alkynyl, preferably -$C_{1-3}$ alkyl, -$C_{2-4}$ alkenyl, -$C_{2-4}$ alkynyl, each independently and optionally substituted by halogen or CN, for example, but not limited to the groups exemplified in Embodiments 1.2.4 to 1.2.6, respectively.

**Embodiment 4.2.5:** The compound of Formula (I) according to Embodiment 4.2, a stereoisomer, tautomer, stable isotopic variant, pharmaceutically acceptable salt or solvate thereof, wherein $R_9$ is - $OR_b$, wherein $R_b$ is H or -$C_{1-6}$ alkyl optionally substituted by halogen, preferably -$C_{1-3}$ alkyl optionally substituted by halogen, for example, but not limited

to the groups exemplified in Embodiment 1.2.7.

**Embodiment 4.2.6:** The compound of Formula (I) according to Embodiment 4.2, a stereoisomer, tautomer, stable isotopic variant, pharmaceutically acceptable salt or solvate thereof, wherein $R_9$ is - O-CON$(R_b)_2$, wherein $R_b$ is H or -$C_{1-6}$ alkyl optionally substituted by halogen, preferably -$C_{1-3}$ alkyl optionally substituted by halogen, for example, but not limited to -OCON$H_2$, -O-CONHC$H_3$, -O-CONHC$F_3$, -OCON$(CH_3)_2$, -OCONHC$H_2$C$H_3$.

**Embodiment 4.3:** The compound of Formula (I) according to any one of Embodiments 1 to 3.3, a stereoisomer, tautomer, stable isotopic variant, pharmaceutically acceptable salt or solvate thereof, wherein W is selected from N, C-halogen or C-O-optionally halogen-substituted $C_{1-3}$ alkyl, preferably N, C-F, C-Cl, C-O$CH_3$.

**Embodiment 5.1:** The compound of Formula (I) according to any one of Embodiments 1 to 4.3, a stereoisomer, tautomer, stable isotopic variant, pharmaceutically acceptable salt or solvate thereof, wherein $R_7$ is H; or is D.

**Embodiment 5.2:** The compound of Formula (I) according to any one of Embodiments 1 to 4.3, a stereoisomer, tautomer, stable isotopic variant, pharmaceutically acceptable salt or solvate thereof, wherein $R_7$ is halogen, preferably F or Cl.

**Embodiment 5.3:** The compound of Formula (I) according to any one of Embodiments 1 to 4.3, a stereoisomer, tautomer, stable isotopic variant, pharmaceutically acceptable salt or solvate thereof, wherein $R_7$ is CN.

**Embodiment 5.4:** The compound of Formula (I) according to any one of Embodiments 1 to 4.3, a stereoisomer, tautomer, stable isotopic variant, pharmaceutically acceptable salt or solvate thereof, wherein $R_7$ is -$C_{1-6}$ alkyl optionally substituted by halogen, preferably -$C_{1-3}$ alkyl optionally substituted by halogen, for example, but not limited to the groups exemplified in Embodiment 1.2.4.

**Embodiment 5.5:** The compound of Formula (I) according to any one of Embodiments 1 to 4.3, a stereoisomer, tautomer, stable isotopic variant, pharmaceutically acceptable salt or solvate thereof, wherein $R_7$ is -$C_{2-6}$ alkenyl optionally substituted by halogen, preferably -$C_{2-4}$ alkenyl optionally substituted by halogen, for example, but not limited to the groups exemplified in Embodiment 1.2.5.

**Embodiment 5.6:** The compound of Formula (I) according to any one of Embodiments 1 to 4.3, a stereoisomer, tautomer, stable isotopic variant, pharmaceutically acceptable salt or solvate thereof, wherein $R_7$ is -$C_{2-6}$ alkynyl optionally substituted by halogen, preferably -$C_{2-4}$ alkynyl optionally substituted by halogen; for example, but not limited to the groups exemplified in Embodiment 1.2.6.

**Embodiment 5.7:** The compound of Formula (I) according to any one of Embodiments 1 to 4.3, a stereoisomer, tautomer, stable isotopic variant, pharmaceutically acceptable salt or solvate thereof, wherein $R_7$ is selected from -O-$C_{1-6}$ alkyl, -S-$C_{1-6}$ alkyl, and -Se-$C_{1-6}$ alkyl, wherein the -$C_{1-6}$ alkyl, preferably -$C_{1-3}$ alkyl, is optionally substituted by halogen; for example, but not limited to the groups exemplified in Embodiment 1.2.7.

**Embodiment 5.8:** The compound of Formula (I) according to any one of Embodiments 1 to 4.3, a stereoisomer, tautomer, stable isotopic variant, pharmaceutically acceptable salt or solvate thereof, wherein M is C-optionally halogen-substituted $C_{1-6}$ alkyl, preferably C-optionally halogen-substituted $C_{1-3}$ alkyl, more preferably C-$CF_3$; W is selected from N, C-halogen or C-O-optionally halogen-substituted $C_{1-3}$ alkyl, preferably N, C-F, C-Cl, C-O$CH_3$, more preferably C-F; $R_7$ is H; the fused parent ring containing M and W can be, for example, but not limited to

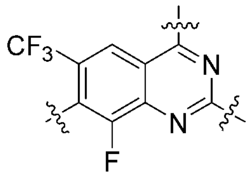

,

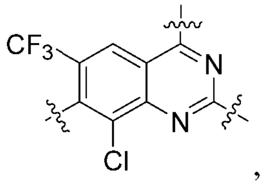, 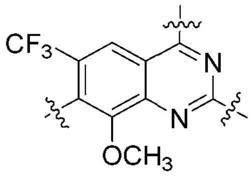.

**Embodiment 6.1:** The compound of Formula (I) according to any one of Embodiments 1 to 5.8, a stereoisomer, tautomer, stable isotopic variant, pharmaceutically acceptable salt or solvate thereof, wherein B is a 5-7-membered monocyclic heterocycloalkyl containing 1 to 3 heteroatoms independently selected from N, O, P, Se, and S, attached to the pyrimidine ring portion of the molecule by a nitrogen heteroatom, for example, but not limited to

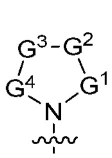, 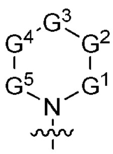, 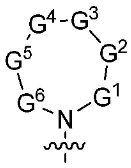,

wherein each G in each ring can be C, or at most three of them, preferably at most two of them are heteroatoms selected from N, O, P, Se, and S, for example, 5-7 membered monocyclic heterocycloalkyl containing 1-3 nitrogen atoms, 5-7 membered monocyclic heterocycloalkyl containing 1 nitrogen atom and 1-2 heteroatoms selected from O, P, Se and S, 5-7 membered monocyclic heterocycloalkyl containing 2 nitrogen atoms and 1 heteroatom selected from O, P, Se and S, 5-7 membered monocyclic heterocycloalkyl containing 1 nitrogen atom and 1 heteroatom selected from O, P, Se and S (e.g., 5-7 membered monocyclic heterocycloalkyl containing 1 nitrogen atom and 1 O atom), for example, but not limited to

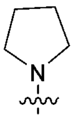, 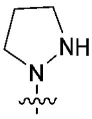, 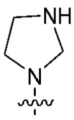, 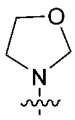, 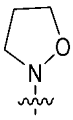, 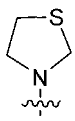, 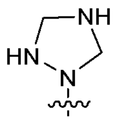, 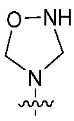, 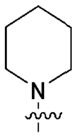, 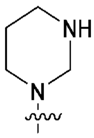,

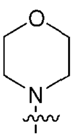, 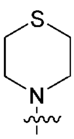, 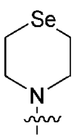, 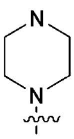, 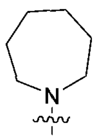, 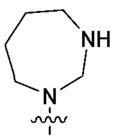, 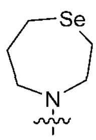, 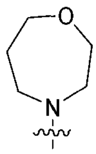, 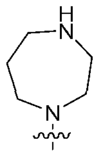,

each substituted by 1-8, preferably 1-4 of $R_1$, more preferably 1-2 of $R_1$, more preferably $R_1$ substitution occurs at the ring carbon atom.

**Embodiment 6.1.1:** The compound of Formula (I) according to Embodiment 6.1, a stereoisomer, tautomer, stable isotopic variant, pharmaceutically acceptable salt or solvate thereof, wherein B is

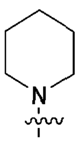,

substituted by 1-2 of $R_1$, preferably substituted by $R_1$ at the meta position of N, for example, but not limited to

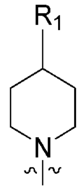, 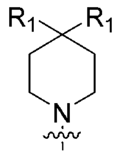, 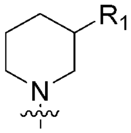, 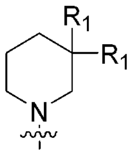, 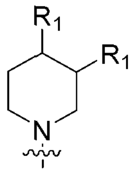.

**Embodiment 6.1.1.1:** The compound of Formula (I) according to Embodiment 6.1.1, a stereoisomer, tautomer, stable isotopic variant, pharmaceutically acceptable salt or solvate thereof, wherein the substituent $R_1$ on B is stereoisomeric as appropriate, e.g., R or S configuration, as long as chemically feasible.

**Embodiment 6.1.1.2:** The compound of Formula (I) according to Embodiments 6.1 to 6.1.1.1, a stereoisomer, tautomer, stable isotopic variant, pharmaceutically acceptable salt or solvate thereof, wherein the substituents $R_1$ carried on B are each independently selected from halogen, CN, -OH, - $B(OH)_2$, $-C_{1-6}$ alkyl and $-OC_{1-6}$ alkyl, wherein the $-C_{1-6}$ alkyl, preferably $-C_{1-3}$ alkyl, is optionally substituted by halogen; preferably $R_1$ is selected from -OH or $-C_{1-6}$ alkyl, wherein the $-C_{1-6}$ alkyl, preferably $-C_{1-3}$ alkyl, is optionally substituted by halogen; more preferably, two $R_1$ are attached to the same ring carbon atom, one of which is -OH and the other is $-C_{1-6}$ alkyl optionally substituted by halogen, preferably $-C_{1-3}$ alkyl, more preferably methyl; further preferably two $R_1$ are attached to the meta ring carbon atom of the N atom attached to the rest of the molecule (e.g., pyrimidine ring), one of which is -OH and the other is $-C_{1-6}$ alkyl, preferably $C_{1-3}$ alkyl, more preferably methyl, e.g., B is

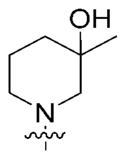,

preferably

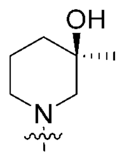.

**Embodiment 6.1.1.3:** The compound of Formula (I) according to any one of Embodiments 6.1 to 6.1.1.1, a stereoisomer, tautomer, stable isotopic variant, pharmaceutically acceptable salt or solvate thereof, wherein the substituent $R_1$ carried on B is each independently selected from $-CONH_2$, $-CONH(C_{1-6}$ alkyl), $-CON(C_{1-6}$ alkyl$)_2$, wherein the $-C_{1-6}$ alkyl, preferably $-C_{1-3}$ alkyl, is optionally substituted by halogen; preferably $-CON(C_{1-6}$ alkyl$)_2$, more preferably $-CON(C_{1-3}$ alkyl$)_2$, for example $-CON(CH_3)_2$, further preferably, one $R_1$ is attached to the meta ring carbon atom of the N atom attached to the rest of the molecule (e.g., pyrimidine ring), e.g., B is

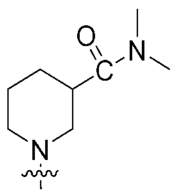.

**Embodiment 6.1.2:** The compound of Formula (I) according to Embodiment 6.1, a stereoisomer, tautomer, stable isotopic variant, pharmaceutically acceptable salt or solvate thereof, wherein B is

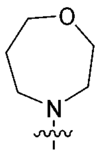,

substituted by 1-2 of $R_1$, preferably substituted by $R_1$ at the meta position of N, for example, but not limited to

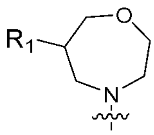, 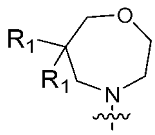, 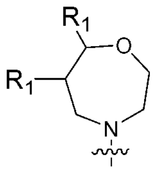, 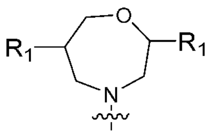.

**Embodiment 6.1.2.1:** The compound of Formula (I) according to Embodiment 6.1.2, a stereoisomer, tautomer, stable isotopic variant, pharmaceutically acceptable salt or solvate thereof, wherein the substituent $R_1$ on B is stereoisomeric as appropriate, e.g., R or S configuration, as long as chemically feasible.

**Embodiment 6.1.2.2:** The compound of Formula (I) according to any one of Embodiments 6.1.2 or 6.1.2.1, a stereoisomer, tautomer, stable isotopic variant, pharmaceutically acceptable salt or solvate thereof, wherein the substituent $R_1$ carried on B is each independently selected from halogen, CN, -OH, $-B(OH)_2$, $-C_{1-6}$ alkyl and $-OC_{1-6}$ alkyl, wherein the $-C_{1-6}$ alkyl, preferably $-C_{1-3}$ alkyl, is optionally substituted by halogen; preferably $R_1$ is selected from -OH or $-C_{1-6}$ alkyl, wherein the $-C_{1-6}$ alkyl, preferably $-C_{1-3}$ alkyl, is optionally substituted by halogen; more preferably, two $R_1$ are attached to the same ring carbon atom, one of which is -OH and the other is $-C_{1-6}$ alkyl optionally substituted by halogen, preferably $-C_{1-3}$ alkyl, more preferably methyl; further preferably two $R_1$ are attached to the meta ring carbon atom of the N atom attached to the rest of the molecule (e.g., pyrimidine ring), one of which is -OH and the other is $-C_{1-6}$ alkyl, preferably $C_{1-3}$ alkyl, more preferably methyl, e.g., B is

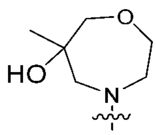,

preferably

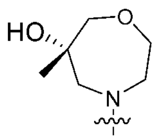.

**Embodiment 6.1.2.3:** The compound of Formula (I) according to Embodiment 6.1.2 or 6.1.2.1, a stereoisomer, tautomer, stable isotopic variant, pharmaceutically acceptable salt or solvate thereof, wherein the substituent $R_1$ carried on B is each independently selected from $-CONH_2$, $-CONH(C_{1-6}$ alkyl), $-CON(C_{1-6}$ alkyl$)_2$, wherein the $-C_{1-6}$ alkyl, preferably $-C_{1-3}$ alkyl, is optionally substituted by halogen; preferably $-CON(C_{1-6}$ alkyl$)_2$, more preferably $-CON(C_{1-3}$ alkyl$)_2$, for example $-CON(CH_3)_2$, further preferably, one $R_1$ is attached to the meta ring carbon atom of the N atom attached to the rest of the molecule (e.g., pyrimidine ring), e.g., B is

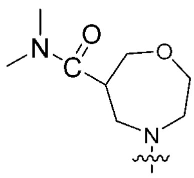.

**Embodiment 6.2:** The compound of Formula (I) according to any one of Embodiments 1 to 5.8, a stereoisomer,

tautomer, stable isotopic variant, pharmaceutically acceptable salt or solvate thereof, wherein B is a 6-12-membered polycyclic heterocycloalkyl containing 1 to 4 heteroatoms independently selected from N, O, P, Se, and S, for example 6-12 membered spiro heterocycloalkyl, for example 6-12 membered fused heterocycloalkyl, such as 6-10 membered, 6-9 membered, 6-8 membered, 6-7 membered, 7-10 membered, 7-9 membered, 7-8 membered, 8-10 membered spiro heterocycloalkyl or fused heterocycloalkyl, are connected to the pyrimidine ring portion of the molecule through a nitrogen heteroatom; wherein the heteroatom can be for example, 1-4 nitrogen atoms (e.g., 1, 2, 3, 4 nitrogen atoms), 1 nitrogen atom and 1-3 heteroatoms selected from O, S, P and Se (e.g., 1 nitrogen atom and 1 heteroatom of O, S, P or Se), 2 nitrogen atoms and 1-2 heteroatoms selected from O, S, P and Se, 3 nitrogen atoms and 1 heteroatom selected from O, S, P and Se, 1-3 nitrogen atoms and 0-1 heteroatoms selected from O, S, P or Se (e.g., 1-3 nitrogen atoms and 0-1 O atoms), for example, but not limited to

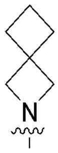 , 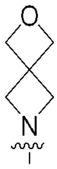 , 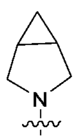 , 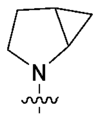 , 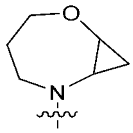 , 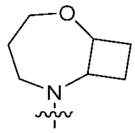 ,

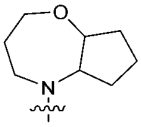 , 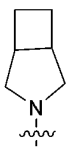 , 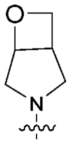 , 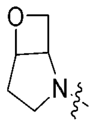 , 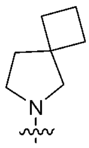 , 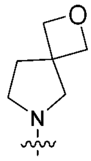 , 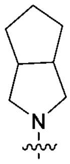 , 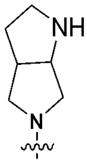 , 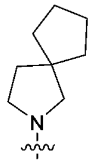 , 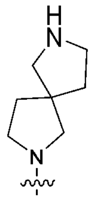 , 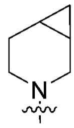 ,

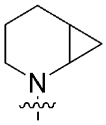 , 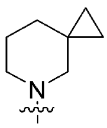 , 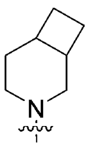 , 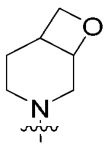 , 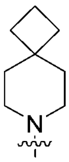 , 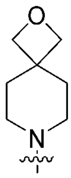 , 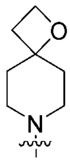 , 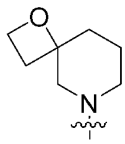 , 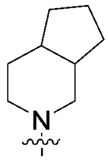 , 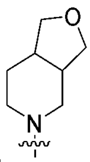 , 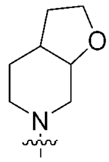 ,

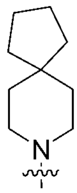 , 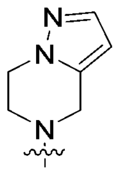 , 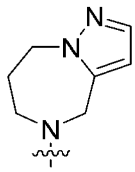 ,

is each substituted by 0-8 of $R_1$, preferably 0-4 of $R_1$, preferably 0-2 of $R_1$, and preferably $R_1$ substitution occurs at the ring carbon atom.

**Embodiment 6.2.1:** The compound of Formula (I) according to Embodiment 6.2, a stereoisomer, tautomer, stable isotopic variant, pharmaceutically acceptable salt or solvate thereof, wherein B is

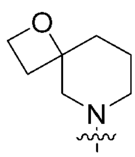 , 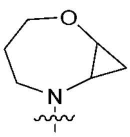 , or 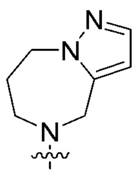 ;

substituted by 0-8 of $R_1$, preferably 0-2 of $R_1$, for example, but not limited to

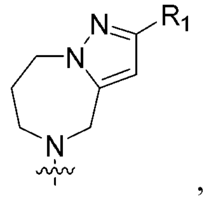 , 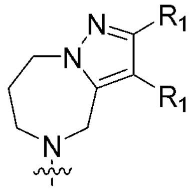 ;

or substituted by 1-3 of $R_1$, preferably 1-2 of $R_1$, for example, but not limited to

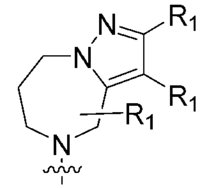 .

**Embodiment 6.2.2:** The compound of Formula (I) according to Embodiment 6.2 or 6.2.1, a stereoisomer, tautomer, stable isotopic variant, pharmaceutically acceptable salt or solvate thereof, wherein the substituent $R_1$ carried on B is each independently selected from H, halogen, -CN, - $CONH_2$, $-CONH(C_{1-6}$ alkyl), $-CON(C_{1-6}$ alkyl$)_2$, wherein the $-C_{1-6}$ alkyl, preferably $-C_{1-3}$ alkyl, is optionally substituted by halogen; preferably $R_1$ adjacent to the nitrogen atom of the five-membered ring is $-CON(C_{1-6}$ alkyl$)_2$, more preferably $-CON(C_{1-3}$ alkyl$)_2$, e.g., $-CON(CH_3)_2$, e.g., B is

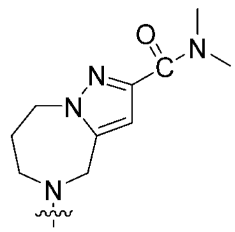 ;

or
B is

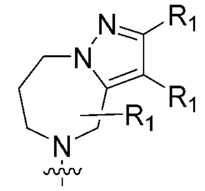 ,

the substituents $R_1$ carried on B are each independently selected from H, halogen, -CN, $-C_{1-6}$ alkyl, $-CONH_2$, $-CONHR_b$, $-CON(R_b)_2$, of which $-C_{1-6}$ alkyl is preferably $-C_{1-3}$ alkyl, optionally substituted by halogen, wherein $R_b$ connecting the N atom is $-C_{1-6}$ alkyl, or both $R_b$ together with the N atom to which they are attached can form a 4-7 membered heterocycloalkyl, e.g., 4-7 membered heterocycloalkyl containing 1 or 2 heteroatoms selected from N and O; preferably $R_1$ adjacent to the nitrogen atom of the five-membered ring is selected from $-CON(R_b)_2$, e.g., $-CON(C_{1-3}$ alkyl$)_2$, -CO-nitrogen linked 4-7 membered monocyclic heterocycloalkyl, e.g., $-CON(CH_3)_2$,

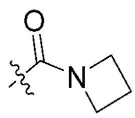 , 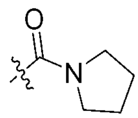 , 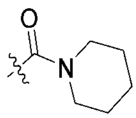 , 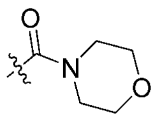 ,

the remaining $R_1$ is each independently selected from H, halogen, and $C_{1-6}$ alkyl, for example

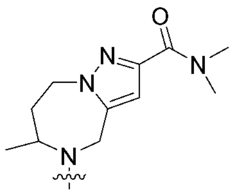 , 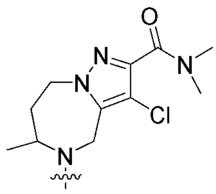 , 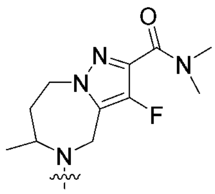 ,

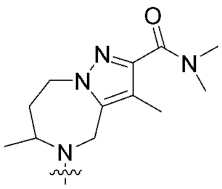 , 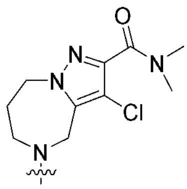 , 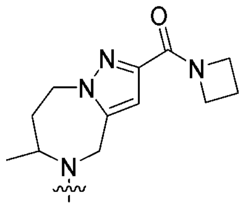 , 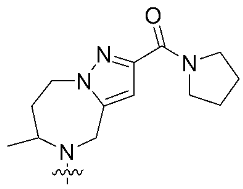 , 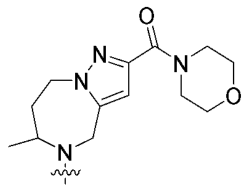 .

**Embodiment 6.3:** The compound of Formula (I) according to any one of Embodiments 1 to 5.8, a stereoisomer, tautomer, stable isotopic variant, pharmaceutically acceptable salt or solvate thereof, wherein B is a 5-7 membered monocyclic heterocycloalkyl containing 1 nitrogen atom or 1 nitrogen atom and 1 heteroatom selected from O, P, Se and S (preferably a 5-7 membered monocyclic heterocycloalkyl containing 1 nitrogen atom or 1 nitrogen atom and 1 O atom), or 8-10 membered spiro-heterocycloalkyl or fused heterocycloalkyl containing 1-3 nitrogen atoms and 0-1 heteroatom selected from O, S, P or Se (preferably 1-3 nitrogen atoms and 0-1 O atom), each of which is optionally substituted by 0-2 -OH, $-C_{1-6}$ alkyl and $-CON(C_{1-6}$ alkyl$)_2$, wherein $-C_{1-6}$ alkyl are preferably $-C_{1-3}$ alkyl, optionally substituted by halogen;
preferably, B is selected from

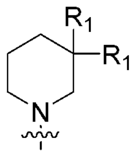 , 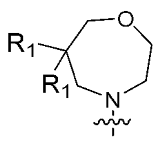 ,

wherein $R_1$ is each independently selected from -OH and $-C_{1-6}$ alkyl, wherein the $-C_{1-6}$ alkyl is preferably $-C_{1-3}$ alkyl, optionally substituted by halogen; preferably one of which is -OH and the other is $-C_{1-6}$ alkyl, preferably $-C_{1-3}$ alkyl, more preferably methyl, most preferably

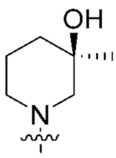 or 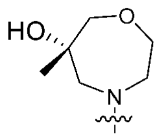 ;

or B is selected from

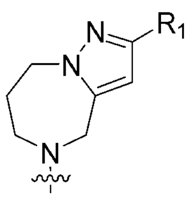 , 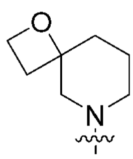 , 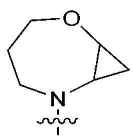 ,

wherein $R_1$ is $-CON(C_{1-6}$ alkyl$)_2$, preferably $-CON(C_{1-3}$ alkyl$)_2$, more preferably $-CON(CH_3)_2$.

**Embodiment 7.1:** The compound of Formula (I) according to Embodiments 1 to 6.3, a stereoisomer, tautomer, stable isotopic variant, pharmaceutically acceptable salt or solvate thereof, wherein Y is O.

**Embodiment 7.2:** The compound of Formula (I) according to Embodiments 1 to 6.3, a stereoisomer, tautomer, stable

isotopic variant, pharmaceutically acceptable salt or solvate thereof, wherein Y is S.

**Embodiment 7.3:** The compound of Formula (I) according to Embodiments 1 to 6.3, a stereoisomer, tautomer, stable isotopic variant, pharmaceutically acceptable salt or solvate thereof, wherein Y is Se.

**Embodiment 7.4:** The compound of Formula (I) according to Embodiments 1 to 6.3, a stereoisomer, tautomer, stable isotopic variant, pharmaceutically acceptable salt or solvate thereof, wherein Y is N-$R_a$, $R_a$ is H; or Y is N-$R_a$, $R_a$ is -$C_{1-6}$ alkyl optionally substituted by halogen, preferably -$C_{1-3}$ alkyl optionally substituted by halogen, for example, but not limited to -$CH_3$, -$CH_2CH_3$.

**Embodiment 8.1:** The compound of Formula (I) according to Embodiments 1 to 7.4, a stereoisomer, tautomer, stable isotopic variant, pharmaceutically acceptable salt or solvate thereof, wherein each of $R_2$ is H.

**Embodiment 8.2:** The compound of Formula (I) according to Embodiments 1 to 7.4, a stereoisomer, tautomer, stable isotopic variant, pharmaceutically acceptable salt or solvate thereof, wherein one $R_2$ is deuterium (D), the other is H, or both $R_2$ are deuterium (D).

**Embodiment 8.2:** The compound of Formula (I) according to Embodiments 1 to 7.4, a stereoisomer, tautomer, stable isotopic variant, pharmaceutically acceptable salt or solvate thereof, wherein one $R_2$ is deuterium (D), the other is H, or both $R_2$ are deuterium (D).

**Embodiment 8.3:** The compound of Formula (I) according to Embodiments 1 to 7.4, a stereoisomer, tautomer, stable isotopic variant, pharmaceutically acceptable salt or solvate thereof, wherein one $R_2$ is H or D, the other $R_2$ is -$C_{1-6}$ alkyl, preferably -$C_{1-3}$ alkyl, optionally substituted by halogen, D, or $C_{1-6}$ alkyl optionally substituted by halogen or D, or -O-$C_{1-6}$ alkyl optionally substituted by halogen or D, or two $R_2$ are each independently -$C_{1-6}$ alkyl, preferably -$C_{1-3}$ alkyl, optionally substituted by halogen, D, or $C_{1-6}$ alkyl optionally substituted by halogen or D, or -O-$C_{1-6}$ alkyl optionally substituted by halogen or D; -$C_{1-6}$ alkyl of $R_2$ is for example, but not limited to -$CH_3$, - $CH_2D$, -$CD_3$, -$CH_2CH_3$, -$CH_2CD_3$, -$CH_2CH_2CH_3$, -$CH(CH_3)(CH_3)$, -$CH_2CH_2CH_2CH_3$, - $CH_2CH(CH_3)CH_3$, -$C(CH_3)_3$, -$CH_2$-$OCH_3$, -$CH_2$-$OCF_3$, -$CH_2$-$OCD_3$, -$CH_2$-O-$CH_2CH_3$, -$CH_2CH_2$-O-$CH_3$, -$CH_2CH_2$-O-$CH_2CH_3$, -$CH_2F$, -$CH_2Cl$, -$CHF_2$, -$CF_3$, -$CCl_3$, -$CH_2CH_2F$, -$CH_2CHF_2$, -$CH_2CF_3$, - $CH_2CH_2CH_2F$, -$CH_2CH_2CHF_2$, -$CH_2CH_2CF_3$, -$C_2F_5$, -$C_2Cl_5$, -$CF(CF_3)_2$.

**Embodiment 8.4:** The compound of Formula (I) according to any one of Embodiments 1 to 7.4, a stereoisomer, tautomer, stable isotopic variant, pharmaceutically acceptable salt or solvate thereof, wherein one $R_2$ is H or D and the other $R_2$ is -$(CH_2)_{0-3}$-$C_{3-6}$ cycloalkyl, preferably -$(CH_2)_{0-3}$-$C_{3-4}$ cycloalkyl, wherein the cycloalkyl is substituted by halogen, D, $C_{1-6}$ alkyl optionally substituted by halogen or D, or -O-$C_{1-6}$ alkyl optionally substituted by halogen or D; for example, but not limited to

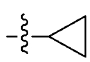, 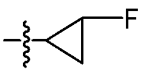, 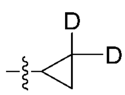, 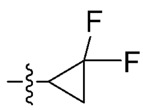, 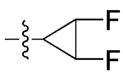, 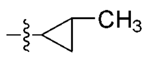, 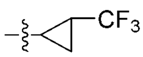, 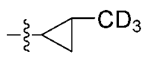,

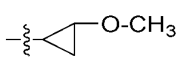, 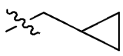, 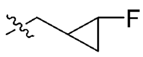, 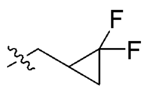, 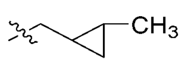, 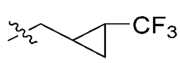,

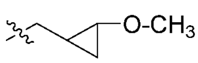, 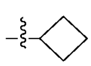, 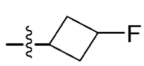, 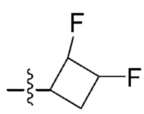, 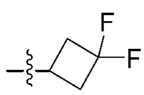, 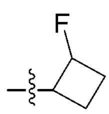, 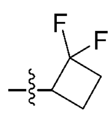,

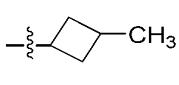, 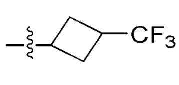, 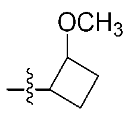, 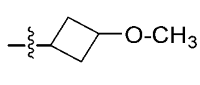, 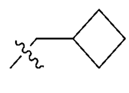, 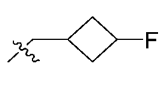,

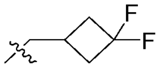, 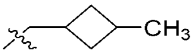, 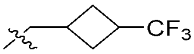, 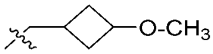, 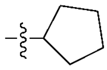, 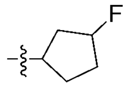,

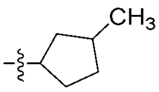, 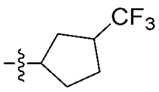, 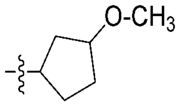, 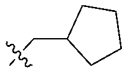, 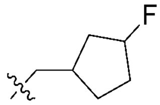, 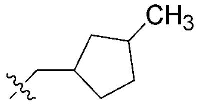,

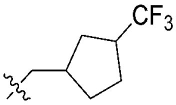, 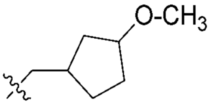, 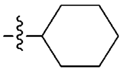, 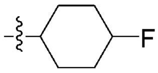, 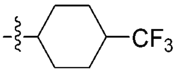, 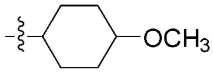,

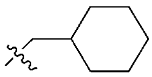.

**Embodiment 8.5:** The compound of Formula (I) according to any one of Embodiments 1 to 7.4, a stereoisomer, tautomer, stable isotopic variant, pharmaceutically acceptable salt or solvate thereof, wherein two $R_2$, together with the carbon atom to which they are attached, form a spiro $C_{3-4}$ cycloalkyl, wherein the $C_{3-4}$ cycloalkyl is optionally substituted by halogen, D, $C_{1-6}$ alkyl optionally substituted by halogen or D, and -O-$C_{1-6}$ alkyl optionally substituted by halogen or D; for example, but not limited to

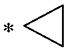, 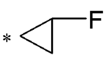, 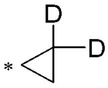, 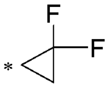, 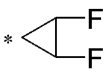, 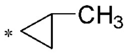, 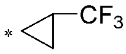, 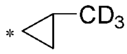, 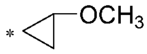,

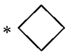, 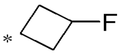, 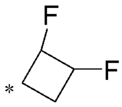, 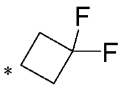, 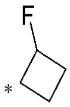, 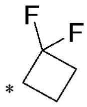, 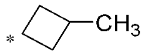, 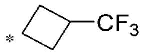, 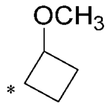,

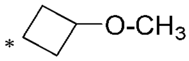,

wherein * indicates an atom attached to the rest of the molecule.

**Embodiment 9.1:** The compound of Formula (I) according to any one of Embodiments 1 to 8.5, a stereoisomer, tautomer, stable isotopic variant, pharmaceutically acceptable salt or solvate thereof, wherein $R_3$ is -$C_{1-6}$ alkyl, preferably -$C_{1-3}$ alkyl, optionally substituted by halogen, D, $C_{1-6}$ alkyl optionally substituted by halogen or D, or -O-$C_{1-6}$ alkyl optionally substituted by halogen or D, for example, but not limited to -$CH_3$, -$CH_2CH_3$, -$CH_2CH_2CH_3$, -$CH(CH_3)(CH_3)$, -$CH_2CH_2CH_2CH_3$, - $CH_2CH(CH_3)CH_3$, -$C(CH_3)_3$, -$CH_2$-$OCH_3$, -$CH_2$-O-$CH_2CH_3$, -$CH_2CH_2$-O-$CH_3$, -$CH_2CH_2$-O-$CH_2CH_3$, -$CH_2F$, -$CH_2Cl$, -$CHF_2$, -$CF_3$, -$CCl_3$, -$CH_2CH_2F$, -$CH_2CHF_2$, -$CH_2CF_3$, -$CH_2CH_2CH_2F$, -$CH_2CH_2CHF_2$, -$CH_2CH_2CF_3$, -$C_2F_5$, -$C_2Cl_5$, -$CF(CF_3)_2$; preferably -$CH_3$ or -$CH_2CH_3$.

**Embodiment 9.1.1**:The compound of Formula (I) according to Embodiment 9,1, a stereoisomer, tautomer, stable isotopic variant, pharmaceutically acceptable salt or solvate thereof, wherein $R_3$ is - deuterated $C_{1-6}$ alkyl, preferably -deuterated $C_{1-3}$ alkyl, for example, but not limited to -$CH_2D$, -$CHD_2$, -$CD_3$.

**Embodiment 9.1.2:** The compound of Formula (I) according to Embodiment 9,1 or 9.1.1, a stereoisomer, tautomer, stable isotopic variant, pharmaceutically acceptable salt or solvate thereof, wherein $R_3$ is stereoisomeric as appropriate, e.g., R or S configuration, preferably S configuration.

**Embodiment 9.2:** The compound of Formula (I) according to any one of Embodiments 1 to 8.5, a stereoisomer, tautomer, stable isotopic variant, pharmaceutically acceptable salt or solvate thereof, wherein $R_3$ is -$(CH_2)_{0-3}$-$C_{3-6}$

cycloalkyl, preferably $-(CH_2)_{0-3}-C_{3-4}$ cycloalkyl, wherein the cycloalkyl is substituted by halogen, D, $C_{1-6}$ alkyl optionally substituted by halogen or D, $-O-C_{1-6}$ alkyl optionally substituted by halogen or D; for example, but not limited to the groups exemplified in Embodiment 8.4.

**Embodiment 9.3:** The compound of Formula (I) according to any one of Embodiments 1 to 9.2, a stereoisomer, tautomer, stable isotopic variant, pharmaceutically acceptable salt or solvate thereof, wherein the stereochemical configuration of the structural fragment

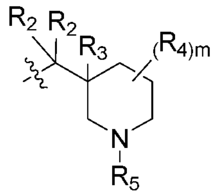

is shown in

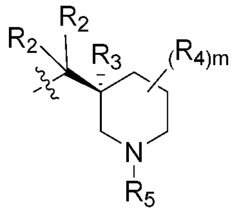

.

**Embodiment 10.1:** The compound of Formula (I) according to any one of Embodiments 1 to 9.3, a stereoisomer, tautomer, stable isotopic variant, pharmaceutically acceptable salt or solvate thereof, wherein $R_4$ is H.

**Embodiment 10.2:**The compound of Formula (I) according to any one of Embodiments 1 to 9.3, a stereoisomer, tautomer, stable isotopic variant, pharmaceutically acceptable salt or solvate thereof, wherein $R_4$ is 1 to 8 of D.

**Embodiment 10.3:** The compound of Formula (I) according to any one of Embodiments 1 to 9.3, a stereoisomer, tautomer, stable isotopic variant, pharmaceutically acceptable salt or solvate thereof, wherein $R_4$ is halogen, e.g., F, Cl, Br, I, preferably F; or $R_1$ is CN.

**Embodiment 10.4:** The compound of Formula (I) according to any one of Embodiments 1 to 9.3, a stereoisomer, tautomer, stable isotopic variant, pharmaceutically acceptable salt or solvate thereof, wherein $R_4$ is $-NH_2$, $-$ $NH-C_{1-6}$ alkyl, $-N(C_{1-6}$ alkyl$)_2$, wherein the $-C_{1-6}$ alkyl is optionally substituted by halogen, CN or $-OC_{1-6}$ alkyl; for example, but not limited to, $-NH_2$, $-NH-CH_3$, $-NH-CH_2CH_3$, $-NH-CF_3$, $-NH-CH_2CF_3$, $-NH-CH_2CN$, $-NH-CH_2CH_2CN$, $-N(CH_3)_2$, $-N(CH_3)(CH_2CH_3)$, $-$ $N(CH_3)(CF_3)$, $-N(CH_3)(CH_2CF_3)$, $-N(CH_3)(CH_2CH_2CN)$, $-NHCH_2-OCH_3$, $N(CH_3)(CH_2CH_2-OCH_3)$, and a group formed by the substitution of one or more H of the alkyl in the above example group by D.

**Embodiment 10.5:** The compound of Formula (I) according to any one of Embodiments 1 to 9.3, a stereoisomer, tautomer, stable isotopic variant, pharmaceutically acceptable salt or solvate thereof, wherein $R_4$ is -OH.

**Embodiment 10.6:** The compound of Formula (I) according to any one of Embodiments 1 to 9.3, a stereoisomer, tautomer, stable isotopic variant, pharmaceutically acceptable salt or solvate thereof, wherein $R_4$ is $-O-C_{1-6}$ alkyl, preferably $-O-C_{1-3}$ alkyl, wherein the alkyl are optionally substituted by halogen, D, CN or $C_{1-6}$ alkoxy; for example, but not limited to, $-O-CH_3$, $-O-CH_2CH_3$, $-O-CH_2CH_2CH_3$, $-O-CH(CH_3)(CH_3)$, $-O-CH_2CH_2CH_2CH_3$, $-O-CH_2CH(CH_3)CH_3$, $-O-C(CH_3)_3$, $-O-CH_2Cl$, $-O-CH_2CN$, $-O-CH_2F$, $-O-CHF_2$, $-O-CF_3$, $-O-CCl_3$, $-O-CH_2CH_2F$, $-O-CH_2CH_2CN$, $-O-CH_2CHF_2$, $-O-CH_2CF_3$, $-O-CH_2CH_2CH_2F$, $-O-CH_2CH_2CHF_2$, $-O-CH_2CH_2CF_3$, $-O-C(CH_3)_2CF_3$, $-O-C_2F_5$, $-O-C_2Cl_5$, $-O-CH_2-OCH_3$, $-O-CH_2-O-CH_2CH_3$, $-O-CH_2CH_2-O-CH_3$, $-O-CH_2CH_2-O-CH_2CH_3$, and a group formed by the substitution of one or more H of the alkyl in the above example group by D.

**Embodiment 10.7:**The compound of Formula (I) according to any one of Embodiments 1 to 9.3, a stereoisomer, tautomer, stable isotopic variant, pharmaceutically acceptable salt or solvate thereof, wherein $R_4$ is $-C_{1-6}$ alkyl,

preferably $-C_{1-3}$ alkyl, optionally substituted by halogen, D, CN, or $-OC_{1-6}$ alkyl, for example, but not limited to $-CH_3$, $-CH_2CH_3$, $-CH_2CH_2CH_3$, $-CH(CH_3)(CH_3)$, $-CH_2CH_2CH_2CH_3$, $-CH_2CH(CH_3)CH_3$, $-C(CH_3)_3$, $-CH_2-OCH_3$, $-CH_2-O-CH_2CH_3$, $-CH_2CH_2-O-CH_3$, $-CH_2CH_2-O-CH_2CH_3$, $-CH_2F$, $-CH_2Cl$, $-CH_2CN$, $-CHF_2$, $-CF_3$, $-CCl_3$, $-CH_2CH_2F$, $-CH_2CH_2CN$, $-CH_2CHF_2$, $-CH_2CF_3$, $-CH_2CH_2CH_2F$, $-CH_2CH_2CHF_2$, $-CH_2CH_2CF_3$, $-C_2F_5$, $-C_2Cl_5$, $-CF(CF_3)_2$, and a group formed by the substitution of one or more H of the alkyl in the above example group by D.

**Embodiment 10.8:** The compound of Formula (I) according to any one of Embodiments 1 to 9.3, a stereoisomer, tautomer, stable isotopic variant, pharmaceutically acceptable salt or solvate thereof, wherein $R_4$ is $-O-C_{3-6}$ cycloalkyl, wherein the $C_{3-6}$ cycloalkyl optionally substituted by halogen, D, CN or $-OC_{1-6}$ alkyl; for example, but not limited to

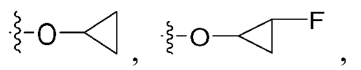

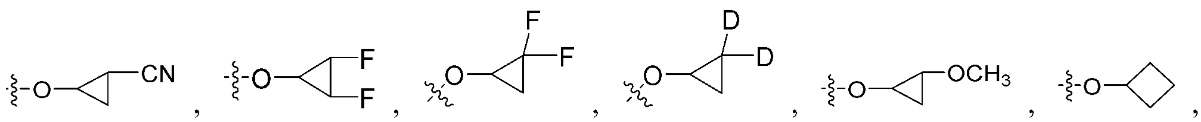

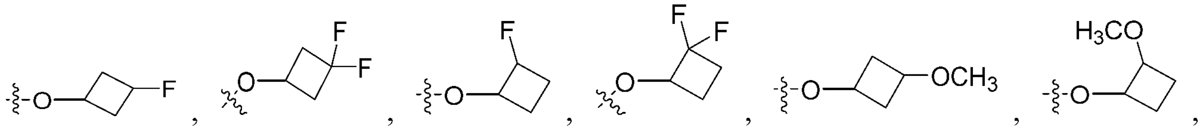

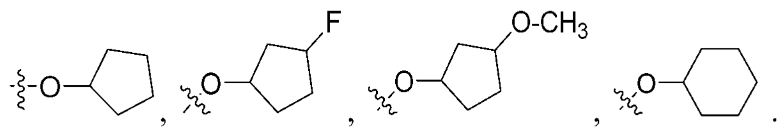

**Embodiment 10.9:** The compound of Formula (I) according to any one of Embodiments 1 to 9.3, a stereoisomer, tautomer, stable isotopic variant, pharmaceutically acceptable salt or solvate thereof, wherein $R_4$ is $-(CH_2)_{0-3}-C_{3-6}$ cycloalkyl, preferably $-(CH_2)_{0-3}-C_{3-4}$ cycloalkyl, more preferably $-C_{3-4}$ cycloalkyl, optionally substituted by halogen, D, CN, or $-OC_{1-6}$ alkyl, preferably optionally substituted by halogen; for example, but not limited to

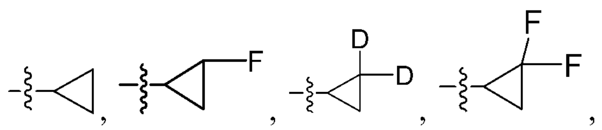

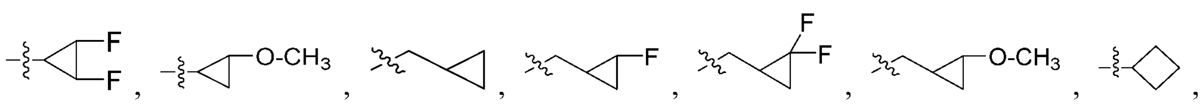

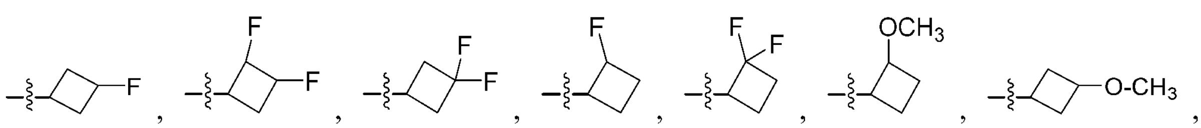

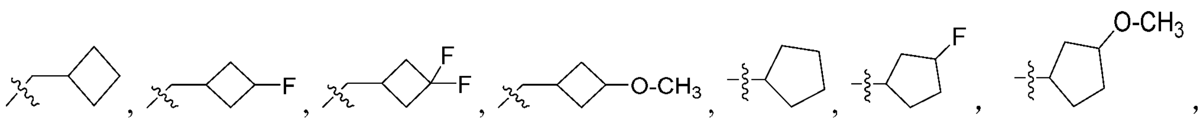

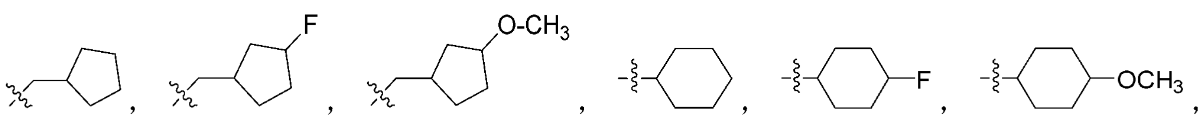

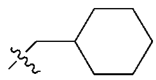, 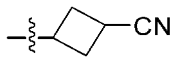, 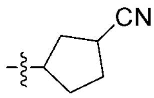.

**Embodiment 10.10:** The compound of Formula (I) according to any one of Embodiments 1 to 9.3, a stereoisomer, tautomer, stable isotopic variant, pharmaceutically acceptable salt or solvate thereof, wherein $R_4$ is $-C_{2-6}$ alkenyl or $-C_{2-6}$ alkynyl, optionally substituted by halogen, D, CN or $-C_{1-6}$ alkoxy; for example, but not limited to vinyl, propyl, ethynyl each optionally substituted by halogen, D, CN, or $-C_{1-6}$ alkoxy.

**Embodiment 10.11:** The compound of Formula (I) according to any one of Embodiments 1 to 9.3, a stereoisomer, tautomer, stable isotopic variant, pharmaceutically acceptable salt or solvate thereof, wherein two $R_4$ attached to the same carbon atom form $=C(R_c)_2$, wherein each $R_c$ is independently selected from H, F, Cl, Br, I, and $-C_{1-6}$ alkyl optionally substituted by halogen; for example, but not limited to $= CH_2$, $=CHF$, $=CF_2$, $=CCl_2$, $=C(CH_3)_2$, $=C(CF_3)_2$.

**Embodiment 10.11:** The compound of Formula (I) according to any one of Embodiments 1 to 9.3, a stereoisomer, tautomer, stable isotopic variant, pharmaceutically acceptable salt or solvate thereof, two $R_4$ attached to the same ring carbon atom together with the ring carbon atom to which they are attached form a spiro $C_{3-6}$ cycloalkyl or spiro 4-7 membered membered heterocycloalkyl, and the spiro $C_{3-6}$ cycloalkyl or spiro 4-7 membered membered heterocycloalkyl is optionally substituted by halogen and $-C_{1-6}$ alkyl optionally substituted by halogen for example, but not limited to, spirocyclopropyl, spirocyclobutyl, spirocyclopentyl, spiroazetidine, spiroazetidine, spiroazacyclopentane, each optionally substituted by halogen (preferably F) or optionally substituted by halogen substituted $C_{1-6}$ alkyl (preferably $-CF_3$), for example, but not limited to

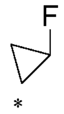, 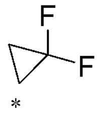,

wherein * indicates an atom attached to the rest of the molecule.

**Embodiment 10.12:** The compound of Formula (I) according to any one of Embodiments 1 to 10.11, a stereoisomer, tautomer, stable isotopic variant, pharmaceutically acceptable salt or solvate thereof, wherein $R_4$ substitution occurs at the para position of the ring N atom, i.e.,

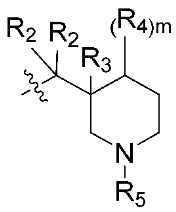

and wherein m is 1 or 2, $R_4$ is selected from each substituent which is not H in the $R_4$ of Embodiments described above.

**Embodiment 10.13:** The compound of Formula (I) according to any one of Embodiments 1 to 9.3, a stereoisomer, tautomer, stable isotopic variant, pharmaceutically acceptable salt or solvate thereof, wherein $R_4$ is selected from halogen, $-O-C_{1-6}$ alkyl and $-C_{1-6}$ alkyl, wherein the $-C_{1-6}$ alkyl present is preferably $-C_{1-3}$ alkyl, each independently and optionally substituted by halogen.

Or two $R_4$ attached to the same carbon atom form $=C(R_c)_2$, wherein $R_c$ is each independently selected from H, F, Cl, Br, I, and $-C_{1-6}$ alkyl optionally substituted by halogen , preferably $-C_{1-3}$ alkyl.
Or two $R_4$ attached to the same ring carbon atom together with the ring carbon atom to which they are attached form a spiro $C_{3-6}$ cycloalkyl, and the spiro $C_{3-6}$ cycloalkyl is preferably $C_{3-4}$ cycloalkyl, optionally substituted by halogen and $-C_{1-6}$ alkyl (preferably $-C_{1-3}$ alkyl) optionally substituted by halogen;
preferably, substitution of $R_4$ occurs in the para-position of the ring N atom.

**Embodiment 10.14:** The compound of Formula (I) according to any one of Embodiments 1 to 9.3, a stereoisomer,

tautomer, stable isotopic variant, pharmaceutically acceptable salt or solvate thereof, wherein m is an integer of 1 to 8; preferably an integer of 1-4; and more preferably an integer of 1 to 2.

**Embodiment 11.1:** The compound of Formula (I) according to any one of Embodiments 1 to 10.14, a stereoisomer, tautomer, stable isotopic variant, pharmaceutically acceptable salt or solvate thereof, wherein $R_5$ is H.

**Embodiment 11.2:** The compound of Formula (I) according to any one of Embodiments 1 to 10.14, a stereoisomer, tautomer, stable isotopic variant, pharmaceutically acceptable salt or solvate thereof, wherein $R_5$ is $-C_{1-6}$ alkyl, preferably $-C_{1-3}$ alkyl, optionally substituted by D, halogen, CN, - $O-C_{1-6}$, or $-O-CON(R_b)_2$, preferably optionally substituted by halogen or D; for example, but not limited to $-CH_3$, $-CD_3$, $-CH_2CH_3$, $-CH_2CH_2CH_3$, $-CH(CH_3)(CH_3)$, $-CH_2CH_2CH_2CH_3$, - $CH_2CH(CH_3)CH_3$, $-C(CH_3)_3$, $-CH_2-OCH_3$, $-CH_2-O-CH_2CH_3$, $-CH_2CH_2-O-CH_3$, $-CH_2CH_2-O-CH_2CH_3$, $-CH_2F$, $-CH_2Cl$, $-CH_2CN$, $-CHF_2$, $-CF_3$, $-CCl_3$, $-CH_2CH_2F$, $-CH_2CH_2CN$, $-CH_2CHF_2$, - $CH_2CF_3$, $-CH_2CH_2CH_2F$, $-CH_2CH_2CHF_2$, $-CH_2CH_2CF_3$, $-C_2F_5$, $-C_2Cl_5$, $-CF(CF_3)_2$, $-CH_2-OCONH_2$, - $CH_2-OCONH(CH_3)$, $-CH_2-OCON(CH_3)_2$; preferably $-CH_3$ or $-CD_3$.

**Embodiment 11.2.1:** The compound of Formula (I) according to Embodiment 11.2, a stereoisomer, tautomer, stable isotopic variant, pharmaceutically acceptable salt or solvate thereof, $R_5$ is -deuterated $C_{1-6}$ alkyl, i.e., the hydrogen atom in it is replaced by one or more isotopic D, e.g., - $CD_3$.

**Embodiment 11.3:** The compound of Formula (I) according to any one of Embodiments 1 to 10.14, a stereoisomer, tautomer, stable isotopic variant, pharmaceutically acceptable salt or solvate thereof, wherein $R_5$ is $-C_{2-6}$ alkynyl or $-C_{2-6}$ alkynyl, optionally substituted by D, halogen, CN, $-O-C_{1-6}$ alkyl, or $-O-CON(R_b)_2$; for example, but not limited to vinyl, propyl, allyl, ethynyl optionally substituted by D, halogen, CN, or $-O-C_{1-6}$, or $-O-CON(R_b)_2$;

**Embodiment 11.4:** The compound of Formula (I) compound of any one of Embodiments 1 to 10.14, a stereoisomer, tautomer, stable isotopic variant, pharmaceutically acceptable salt or solvate thereof, wherein $R_5$ is $-(CH_2)_{0-3}-C_{3-6}$ cycloalkyl, the $-C_{3-6}$ cycloalkyl are optionally substituted by D, halogen, CN, $-O-C_{1-6}$ alkyl, or $-O-CON(R_b)_2$; for example, but not limited to

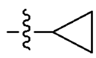 , 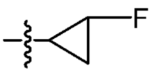 ,

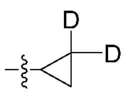 , 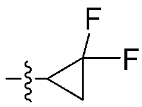 , 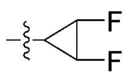 , 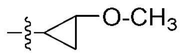 , 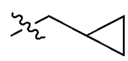 , 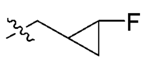 , 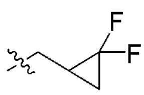 ,

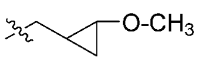 , 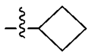 , 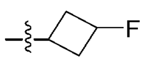 , 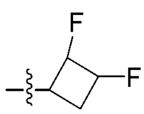 , 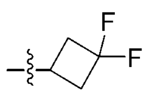 , 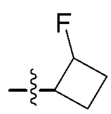 , 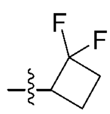 , 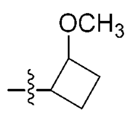 ,

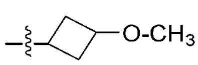 , 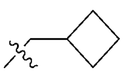 , 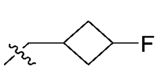 , 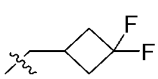 , 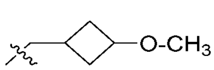 , 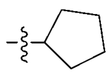 , 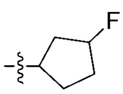 ,

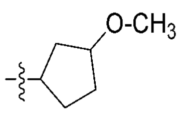 , 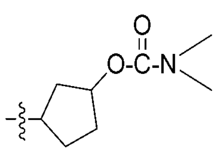 , 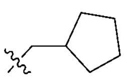 , 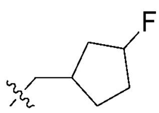 , 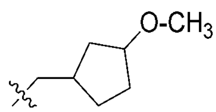 , 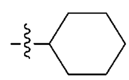 ,

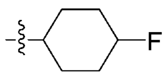 , 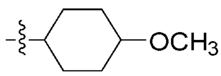 , 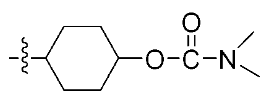 , 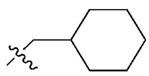 , 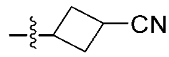 , 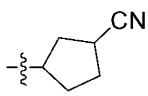 .

**Embodiment 11.5:** The compound of Formula (I) according to any one of Embodiments 1 to 10.14, a stereoisomer, tautomer, stable isotopic variant, pharmaceutically acceptable salt or solvate thereof, wherein $R_5$ is selected from $-C_{1-6}$ alkyl, -deuterated $C_{1-6}$ alkyl, and $-C_{3-6}$ cycloalkyl, preferably selected from $-CH_3$ and -deuterated $C_{1-3}$ alkyl, for example, but not limited to $-CH_3$, $-CD_3$, $-CH_2CH_3$, and cyclopropyl.

**Embodiment 12.1:** The compound of Formula (I) according to any one of Embodiments 1 to 7.4, a stereoisomer, tautomer, stable isotopic variant, pharmaceutically acceptable salt or solvate thereof , wherein in the structural fragment

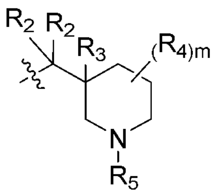

,

specifically in

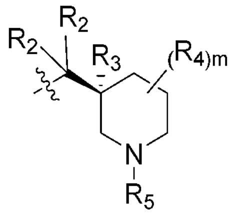

,

more specifically in

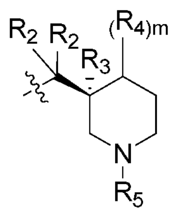

,

$R_2$ is selected from halogen and D, $R_3$ is selected from $-C_{1-6}$ alkyl and - deuterated $C_{1-6}$ alkyl; m is 1 or 2; $R_4$ is selected from halogen, $-O-C_{1-6}$ alkyl and $-C_{1-6}$ alkyl, wherein the $C_{1-6}$ alkyl present is each independently and optionally substituted by halogen, or two $R_4$ attached to the same carbon atom form $=C(R_c)_2$, wherein each $R_c$ is independently selected from H, halogen, - $C_{1-6}$ alkyl optionally substituted by halogen, or two $R_4$ attached to the same ring carbon atom together with the ring carbon atoms to which they are attached form a spiro $C_{3-6}$ cycloalkyl, and the spiro $C_{3-6}$ cycloalkyl is optionally substituted by halogen and $-C_{1-6}$ alkyl optionally substituted by halogen, preferably $R_4$ substitution occurs at para position of ring N atom; specific examples of $R_4$ include, but are not limited to fluoro, difluoro, fluoromethyl, methyl, dimethyl, difluoromethyl, methoxy, fluoromethylene, difluoromethylene, methylene, spiro-cyclopropyl, spiro-cyclopropyl substituted by one or two F; $R_5$ is selected from $-C_{1-6}$ alkyl, -deuterated $C_{1-6}$ alkyl and $-C_{3-6}$ cycloalkyl;
preferably, $R_2$ is selected from H and D; $R_3$ is selected from $-C_{1-3}$ alkyl; m is 1 or 2; $R_4$ is halogen, $-C_{1-3}$ alkyl optionally substituted by halogen, or two $R_4$ attached to the same carbon atom form $=C(R_c)_2$, where each $R_c$ is independently selected from H, halogen, $-C_{1-3}$ alkyl optionally substituted by halogen, or two $R_4$ attached to the same ring carbon atom together with the ring carbon atoms to which they are attached form a spiro $C_{3-4}$ cycloalkyl, and the spiro $C_{3-4}$ cycloalkyl is optionally substituted by halogen, for example, but not limited to F, $=CH_2$, $=CHF$, $=CF_2$, $=CCl_2$, $-CH_3$, $-CHF_2$, spiro-cyclopropyl, spiro-cyclopropyl substituted by F; $R_5$ is $-C_{1-3}$ alkyl, or $-C_{1-3}$ alkyl substituted by one or more deuterium, for example $-CH_3$, $-CH_2CH_3$, $-CD_3$.

**Embodiment 12.2:** The compound of Formula (I) according to any one of Embodiments 1 to 7.4 and 12.1, a stereoisomer, tautomer, stable isotopic variant, pharmaceutically acceptable salt or solvate thereof, wherein examples of the structural fragment

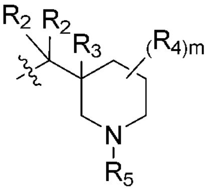,

specifically

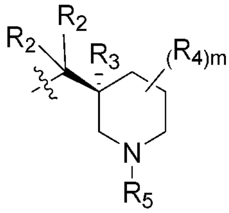,

more specifically

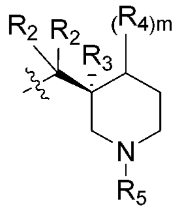

include, but are not limited to

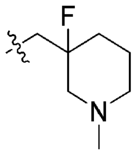,

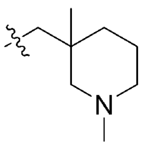, 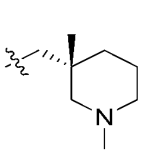, 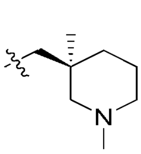, 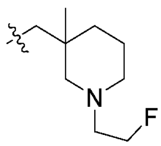, 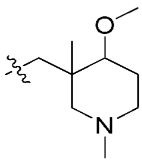, 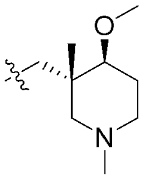, 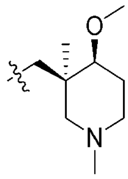,

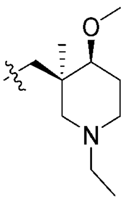, 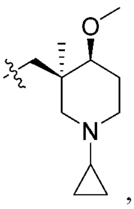, 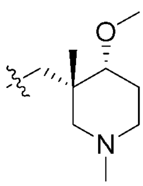, 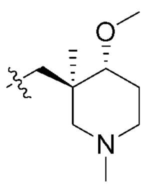, 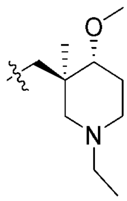, 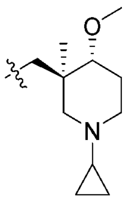, 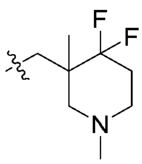, 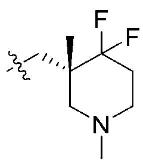,

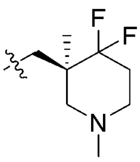, 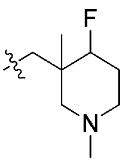, 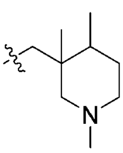, 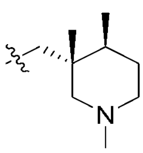, 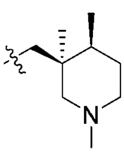, 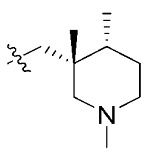, 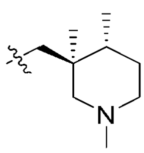, 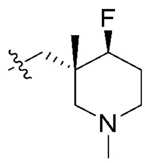,

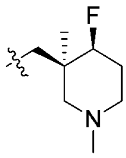, 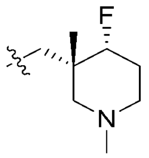, 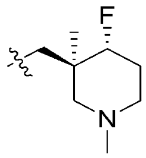, 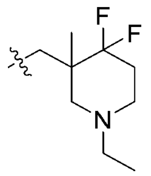, 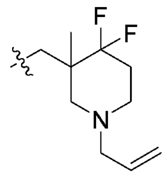, 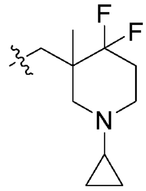 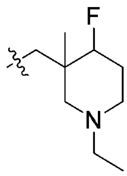,

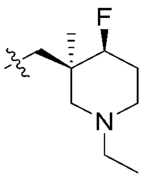, 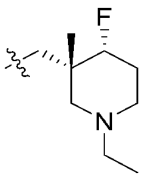, 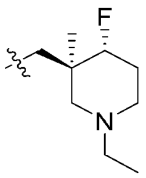, 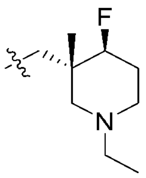, 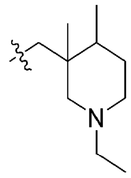, 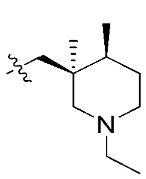, 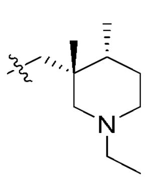,

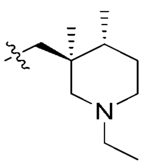, 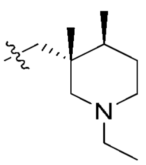, 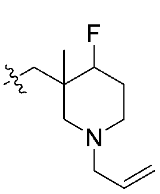, 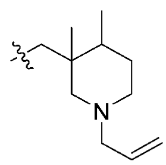, 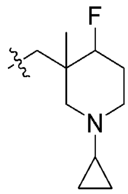, 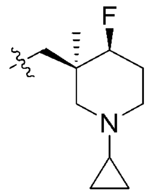, 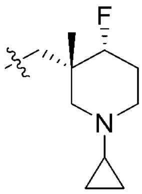,

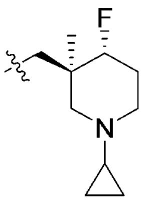, 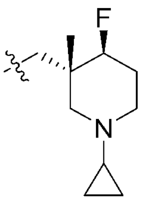, 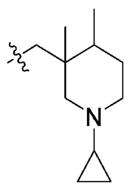, 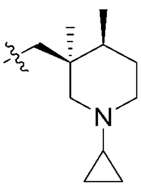, 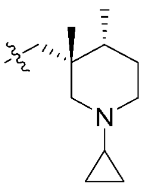, 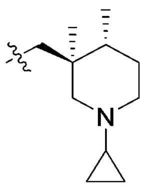, 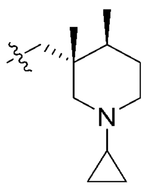,

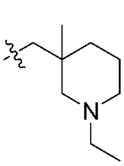, 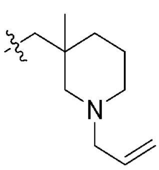, 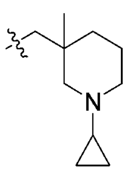, 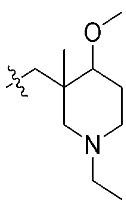, 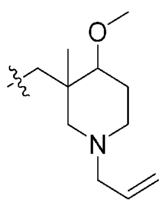, 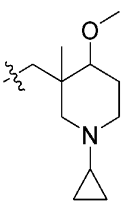 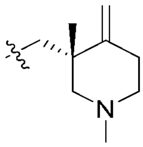, 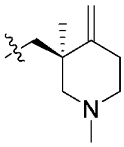,

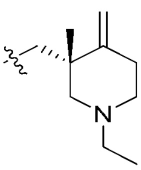, 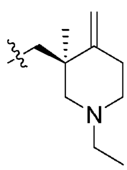, 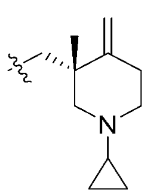, 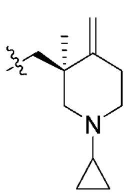, , , , ,

, , , , , , ,

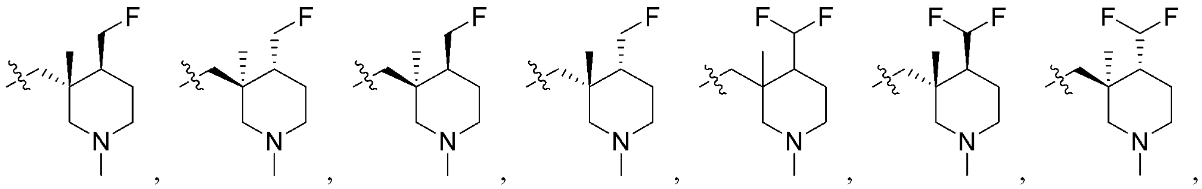

, , , , , , ,

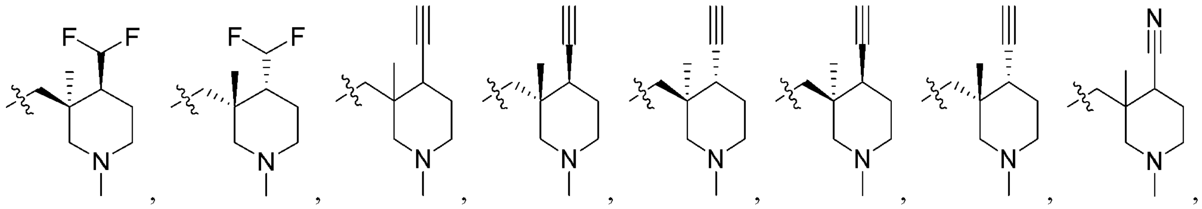

, , , , , , , ,

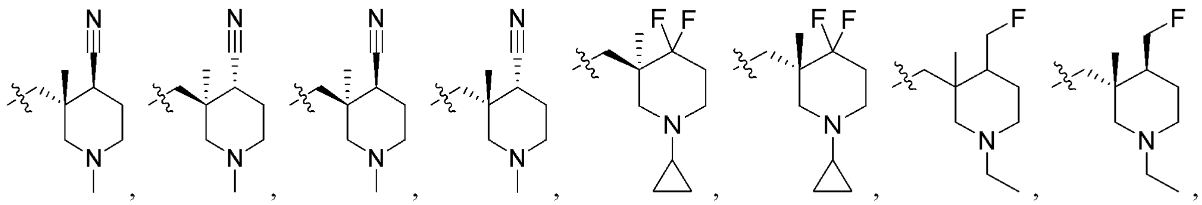

, , , , , , , ,

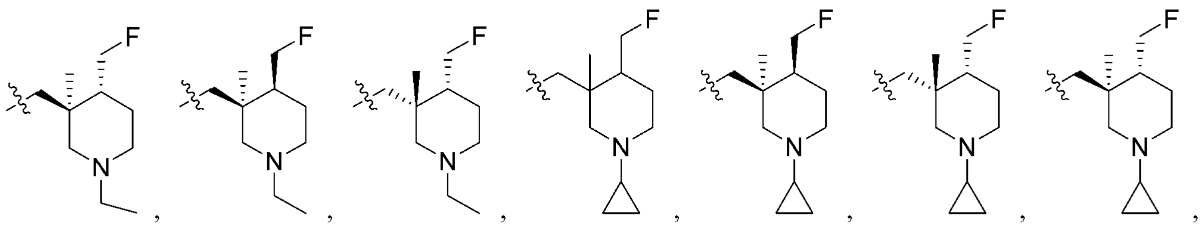

, , , , , , ,

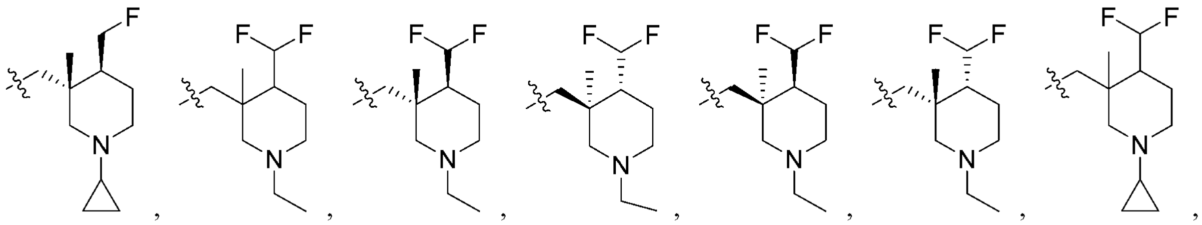

, , , , , , ,

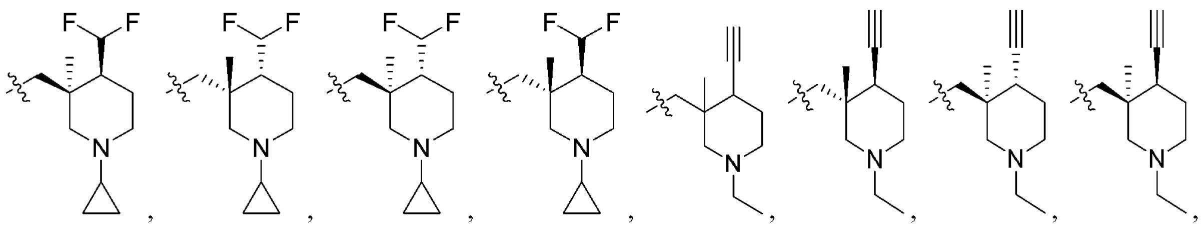

, , , , , , , ,

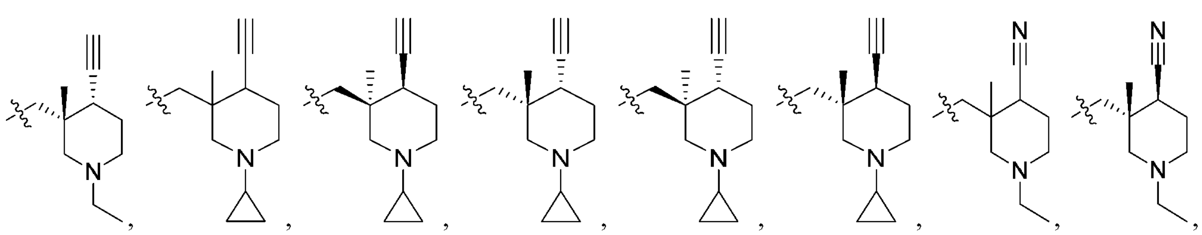

, , , , , , , ,

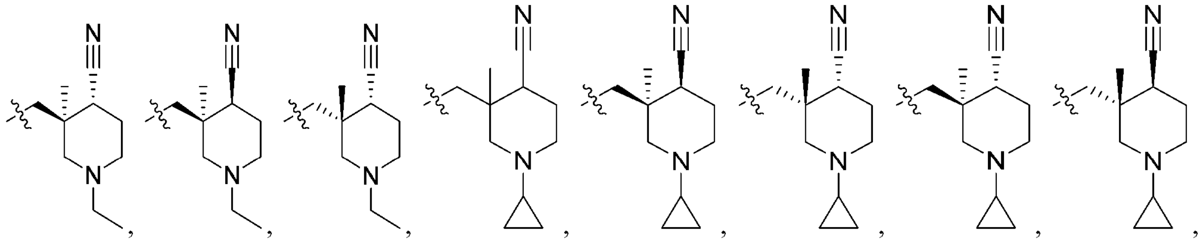

, , , , , , , ,

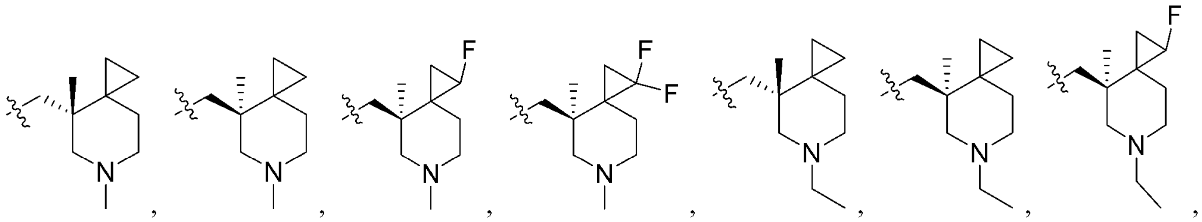

, , , , , , ,

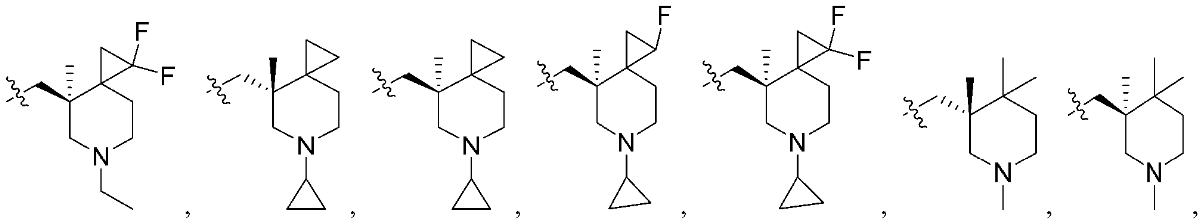

, , , , , , ,

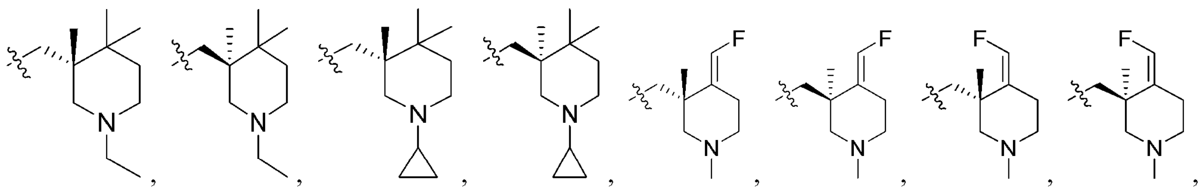

, , , , , , , ,

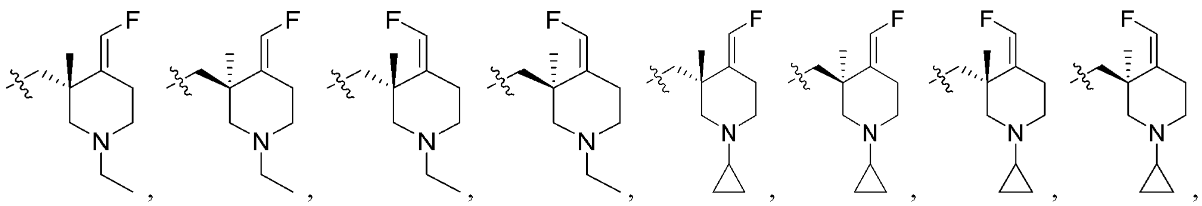

, , , , , , , ,

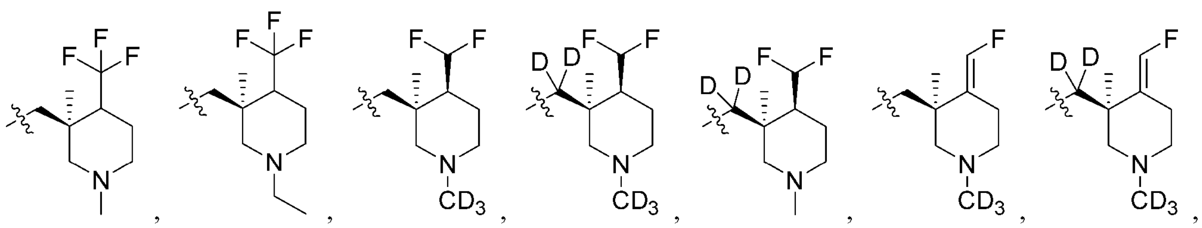

, , , , , , ,

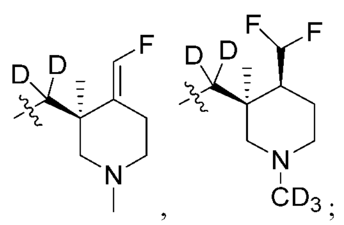

, ;

preferably

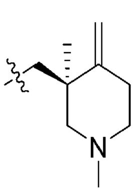 , 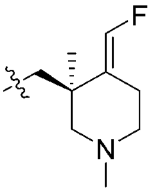 , 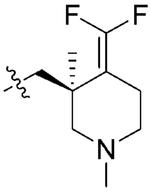 , 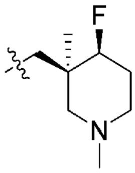 , 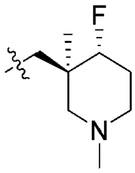 , 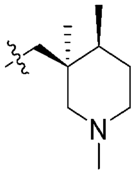 , 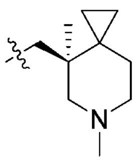 ,

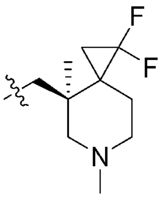 , 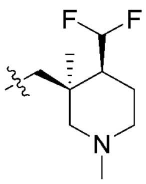 , 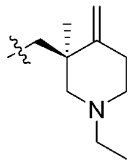 , 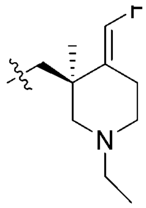 , 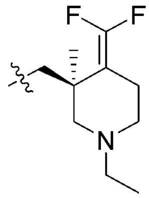 , 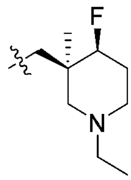 , 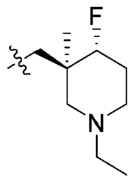 ,

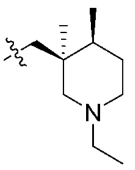 , 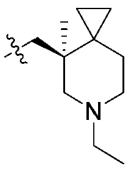 , 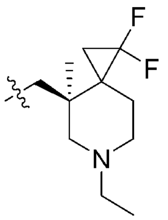 , 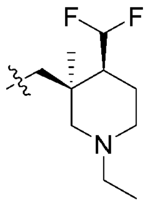 , 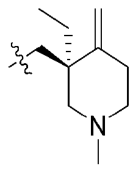 , 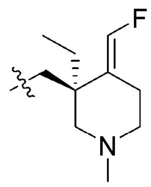 , 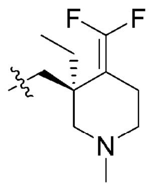 ,

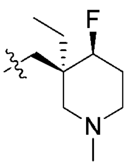 , 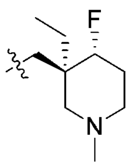 , 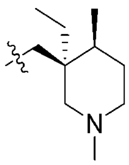 , 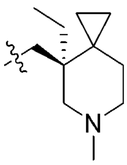 , 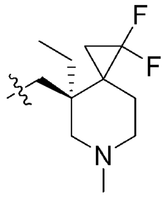 , 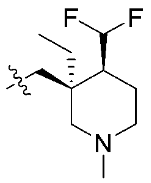 , 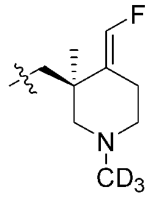 ,

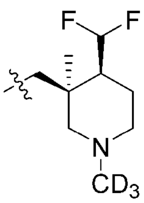 , 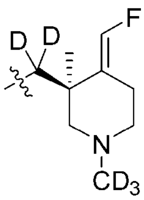 , 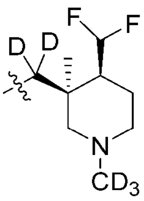 , 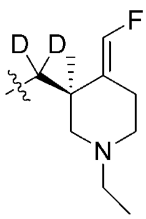 , 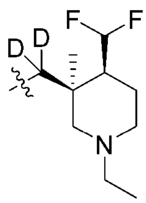 , 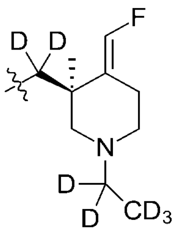 , 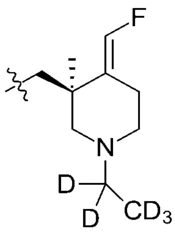 ,

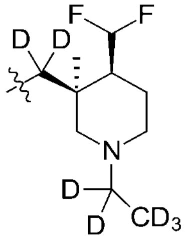 , 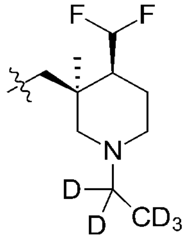 ,

more preferably

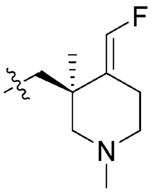, 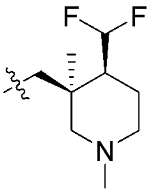, 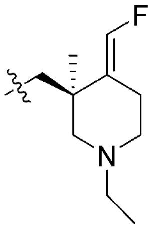, 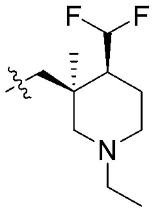, 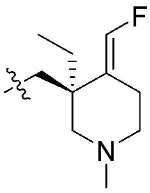, 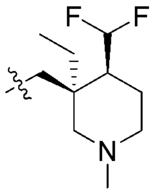,

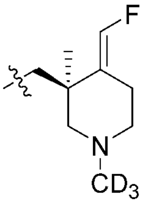, 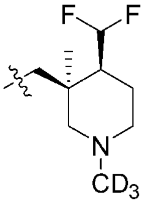, 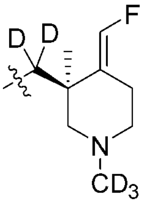, 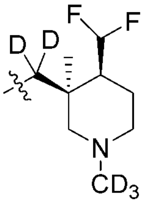, 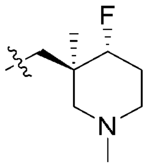, 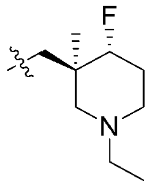, 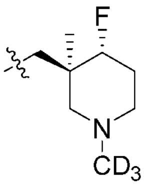,

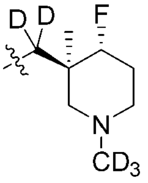, 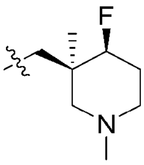, 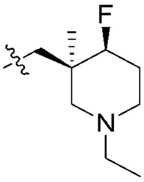, 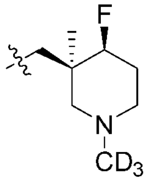, 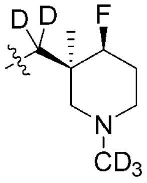, 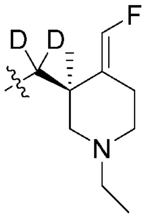, 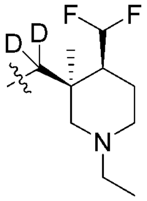,

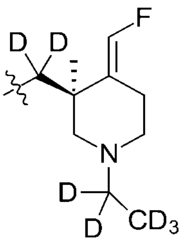, 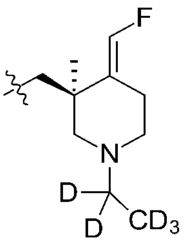, 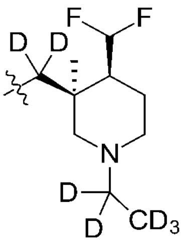, 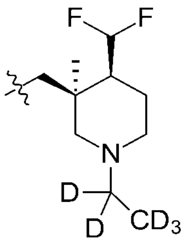.

**Embodiment 13.1:** The compound of Formula (I) according to Embodiment 1, a stereoisomer, tautomer, stable isotopic variant, pharmaceutically acceptable salt or solvate thereof, wherein: M is C-$R_8$ and W is C-$R_9$, wherein $R_8$, $R_9$ and each of the remaining substituents in general formula (I) is generally and preferably defined in the corresponding Embodiments described above.

**Embodiment 13.2:** The compound of Formula (I) according to Embodiment 1, a stereoisomer, tautomer, stable isotopic variant, pharmaceutically acceptable salt or solvate thereof, wherein: M is C-$R_8$ and W is N, wherein $R_8$ and each of the remaining substituents in general formula (I) is generally and preferably defined in the corresponding Embodiments described above.

**Embodiment 13.3:** The compound of Formula (I) according to Embodiment 1, a stereoisomer, tautomer, stable isotopic variant, pharmaceutically acceptable salt or solvate thereof, wherein: M is N and W is C-$R_9$, wherein $R_9$ and each of the remaining substituents in general formula (I) is generally and preferably defined in the corresponding Embodiments described above.

**Embodiment 13.4:** The compound of Formula (I) according to Embodiment 1, a stereoisomer, tautomer, stable isotopic variant, pharmaceutically acceptable salt or solvate thereof, wherein: M is N and W is N, where each of the remaining substituents in general formula (I) is generally and preferably defined in the corresponding Embodiments described above.

**Embodiment** 13.5: The compound of Formula (I) according to any one of Embodiments 13.1 to 13.4, a stereoisomer, tautomer, stable isotopic variant, pharmaceutically acceptable salt or solvate thereof, wherein: Z is C-CN, and Y is O.

**Embodiment 14.1:** The compound of Formula (I) according to Embodiment 1, a stereoisomer, tautomer, stable isotopic variant, pharmaceutically acceptable salt or solvate thereof, having the following sub-general formula:

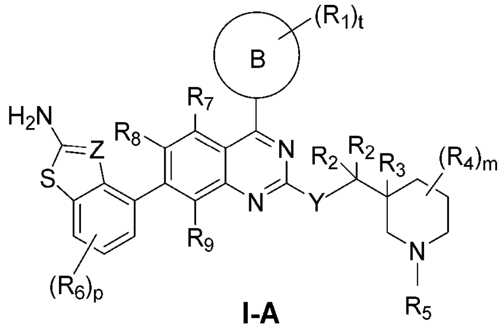

**I-A**

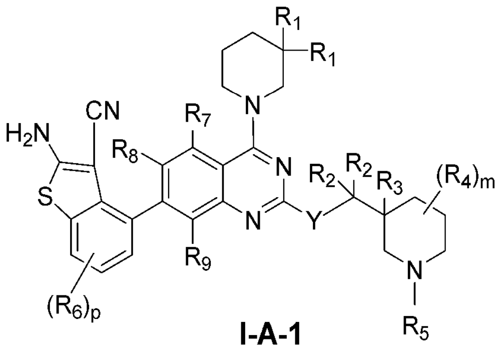

**I-A-1**

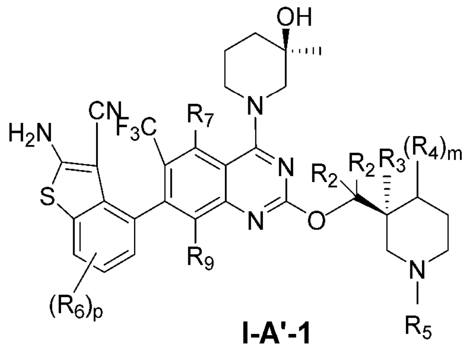

**I-A'-1**

| | |
|---|---|
| 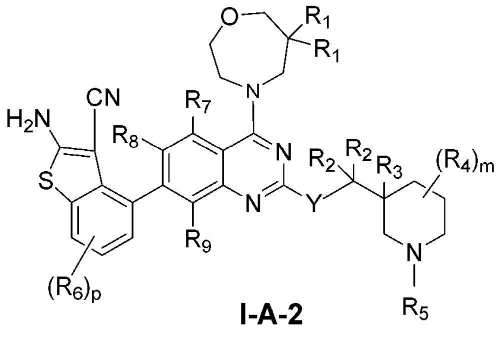 I-A-2 | 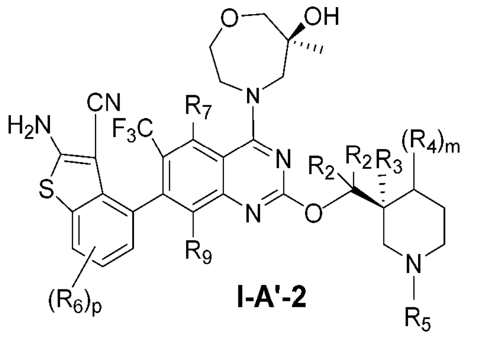 I-A'-2 |
| 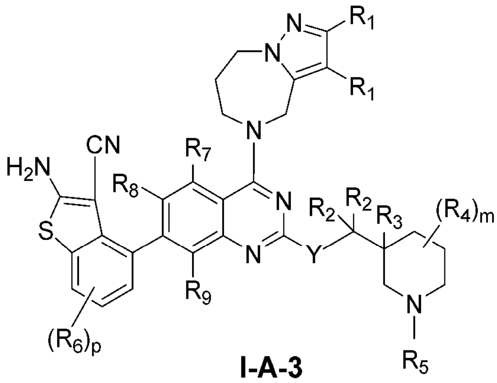 I-A-3 | 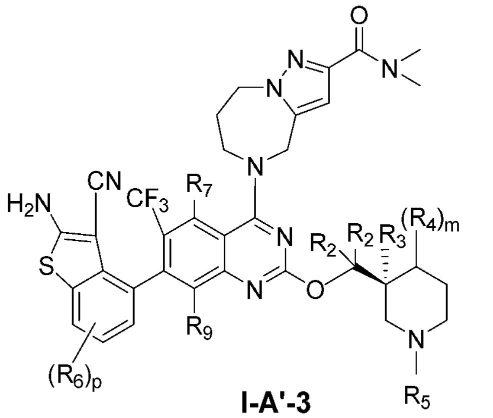 I-A'-3 |
| 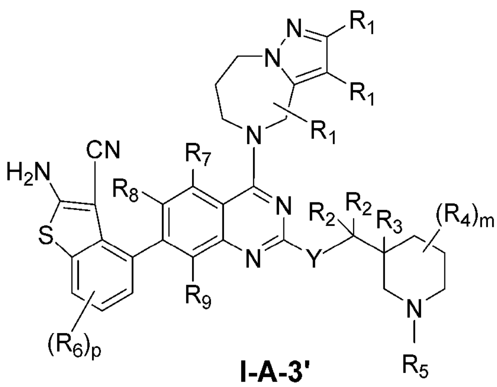 I-A-3' 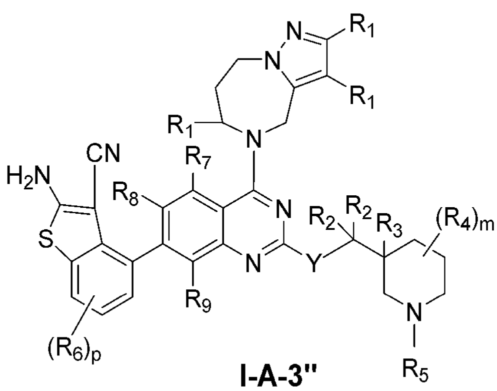 I-A-3'' | 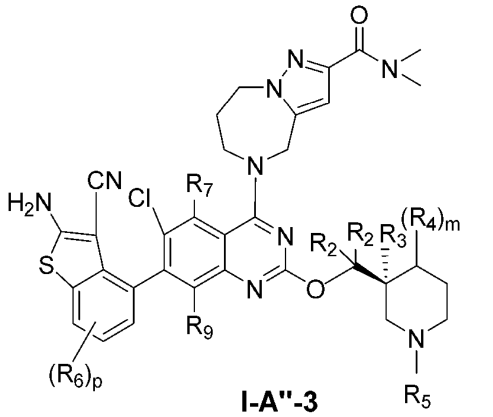 I-A''-3 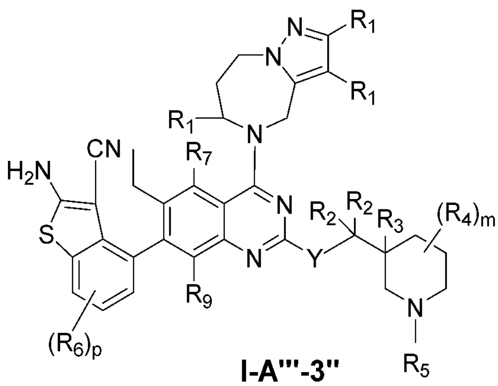 I-A'''-3'' |

| | |
|---|---|
| 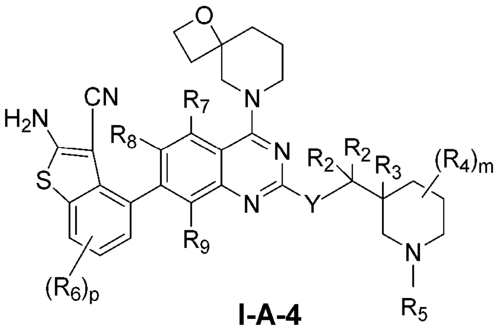 I-A-4 | 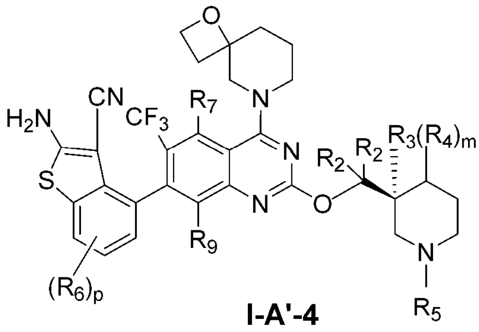 I-A'-4 |
| 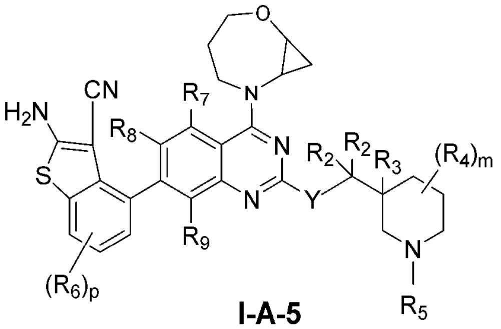 I-A-5 | 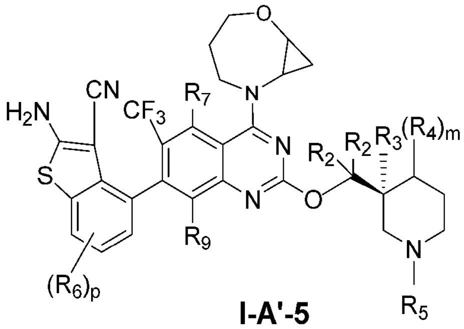 I-A'-5 |
| 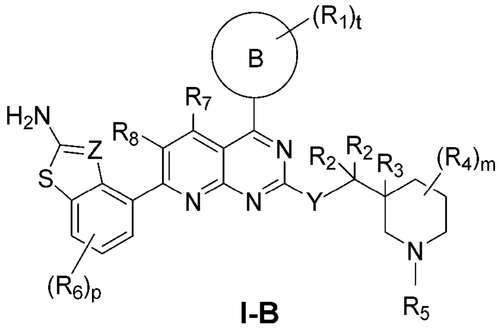 I-B | |
| 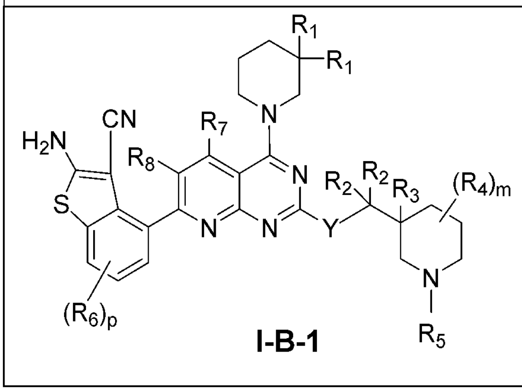 I-B-1 | 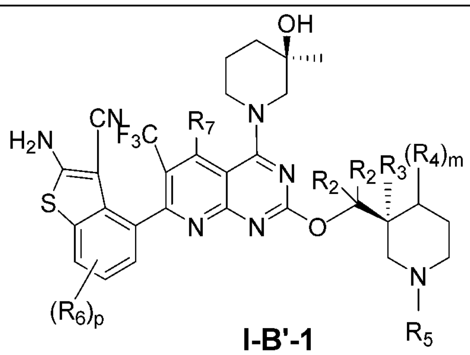 I-B'-1 |

| | |
|---|---|
| 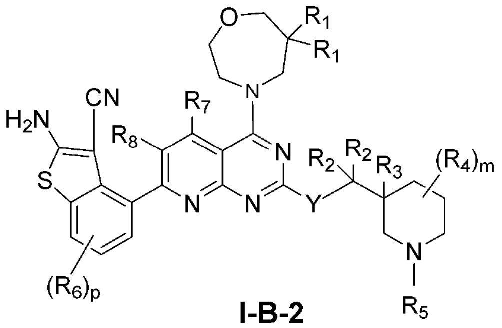 I-B-2 | 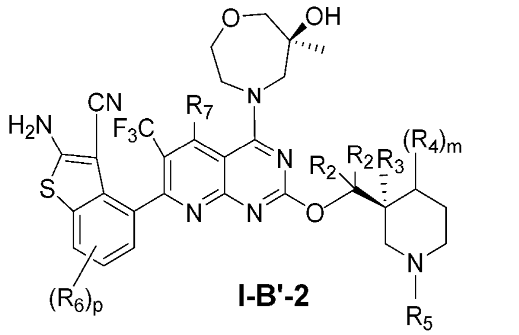 I-B'-2 |
| 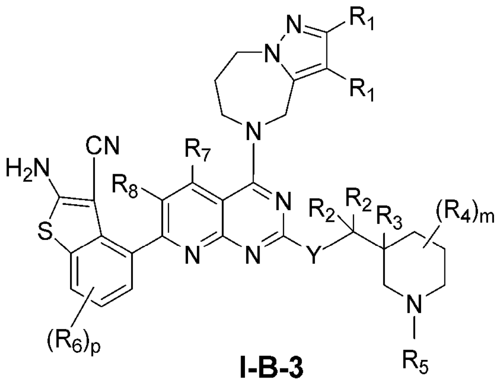 I-B-3 | 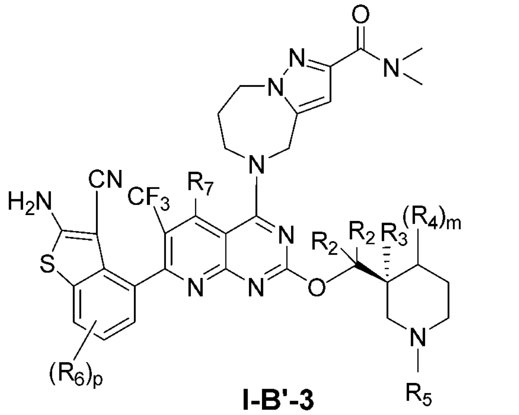 I-B'-3 |
| 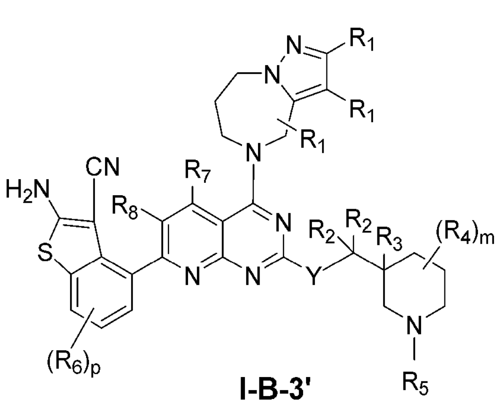 I-B-3' | 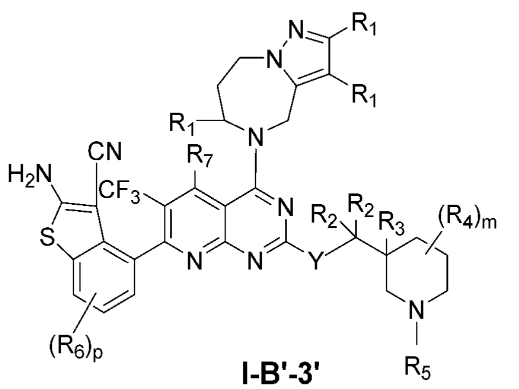 I-B'-3' |
| 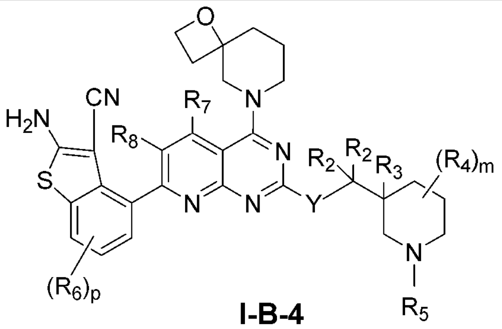 I-B-4 | 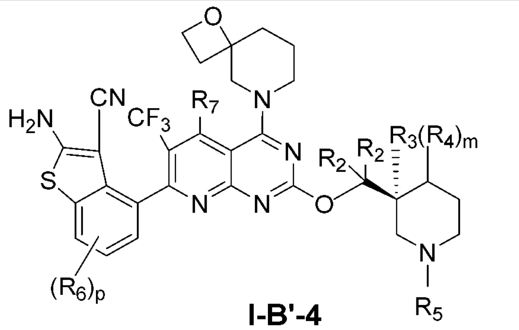 I-B'-4 |

| | |
|---|---|
| 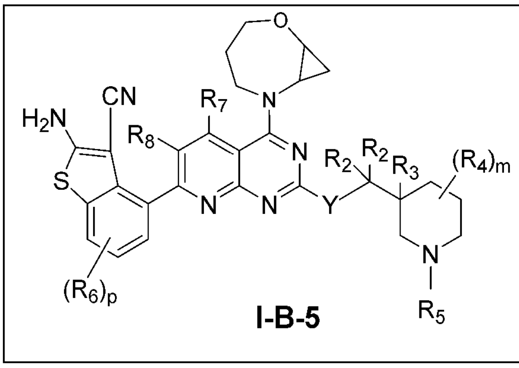 I-B-5 | 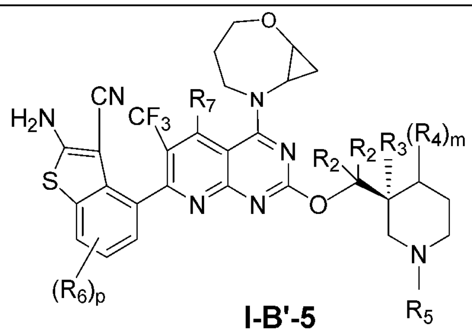 I-B'-5 |

wherein each substituent has the general or preferred meaning as defined in each corresponding Embodiment mentioned above.

**Embodiment** 14.2: The compound of Formula (I) according to Embodiment 14.1, a stereoisomer, tautomer, stable isotopic variant, pharmaceutically acceptable salt or solvate thereof, wherein:

Y is O;

the benzo-fused ring fragment containing Z is

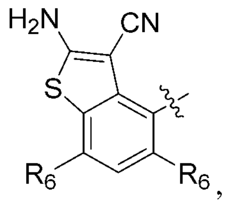

wherein each $R_6$ is independently selected from H and halogen, preferably H and F;

$R_7$ is H

$R_8$ is halogen; preferably Cl; or

$R_8$ is -$C_{1-6}$ alkyl optionally substituted by halogen, preferably C-halogen substituted -$C_{1-3}$ alkyl, more preferably C-$CF_3$; or

$R_8$ is -$C_{1-6}$ alkyl, preferably $C_{1-3}$ alkyl; or -$C_{1-6}$ alkyl-O-$C_{1-6}$ alkyl, preferably $C_{1-3}$ alkyl-O-$C_{1-3}$ alkyl;

$R_9$ is selected from halogen and -O-$C_{1-6}$ alkyl optionally substituted by halogen, preferably selected from F, Cl and -O-$C_{1-3}$ alkyl optionally substituted by halogen;

For Formulas I-A-1, I-A-2, I-B-1 and I-B-2 and their respective sub-general formulas, $R_1$ is selected from -OH and -$C_{1-6}$ alkyl, wherein the -$C_{1-6}$ alkyl preferably -$C_{1-3}$ alkyl, optionally substituted by halogen; preferably one of which is -OH and the other is -$C_{1-3}$ alkyl, preferably -$C_{1-3}$ alkyl, and more preferably methyl;

For Formulas I-A-3 and I-B-3 and their respective sub-general formulas, $R_1$ at the ortho position of the five-membered ring nitrogen atom is -CON($C_{1-6}$ alkyl)$_2$, preferably -CON($C_{1-3}$ alkyl)$_2$, more preferably -CON($CH_3$)$_2$, and the remaining $R_1$ is each independently selected from H, halogen and - $C_{1-6}$ alkyl;

$R_2$ is each independently selected from H and D;

$R_3$ is -$C_{1-6}$ alkyl, preferably -$C_{1-3}$ alkyl,

$R_4$ is selected from halogen, -O-$C_{1-6}$ alkyl and -$C_{1-6}$ alkyl, wherein the $C_{1-6}$ alkyl present is each independently and optionally substituted by halogen, or

two $R_4$ attached to the same carbon atom form $=C(R_c)_2$, wherein each $R_c$ is independently selected from H, F, Cl, Br, I, $-C_{1-6}$ alkyl optionally substituted by halogen (preferably $-C_{1-3}$ alkyl),
or two $R_4$ attached to the same ring carbon atom together with the ring carbon atom to which they are attached form a spiro $C_{3-6}$ cycloalkyl, and the spiro $C_{3-6}$ cycloalkyl, preferably $C_{3-4}$ cycloalkyl, are optionally substituted by halogen and $-C_{1-6}$ alkyl optionally substituted by halogen (preferably - $C_{1-3}$ alkyl);
$R_5$ is selected from $-C_{1-6}$ alkyl, -deuterated $C_{1-6}$ alkyl and $-C_{3-6}$ cycloalkyl, preferably selected from $-C_{1-3}$ alkyl and -deuterated $C_{1-3}$ alkyl;
m and p are each independently an integer of 0 to 2.

Embodiment 14.3: The compound of Formula (I) according to any one of Embodiments 14.1 to 14.2, a stereoisomer, tautomer, stable isotopic variant, pharmaceutically acceptable salt or solvate thereof, wherein in the structural fragment

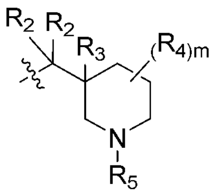
,

specifically

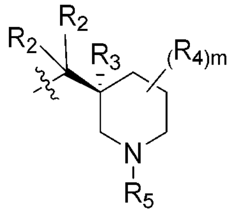
,

more specifically

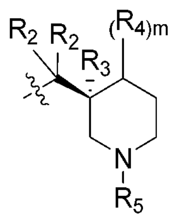
,

$R_2$ is selected from H and D, $R_3$ is selected from $-C_{1-3}$ alkyl; m is 1 or 2; $R_4$ is halogen, $-C_{1-3}$ alkyl optionally substituted by halogen, or two $R_4$ attached to the same carbon atom form $=C(R_c)_2$, wherein each $R_c$ independently selected from H, halogen, $-C_{1-3}$ alkyl optionally substituted by halogen, or two $R_4$ attached to the same ring carbon atom together with the ring carbon atoms to which they are attached form a spiro $C_{3-4}$ cycloalkyl, and the spiro $C_{3-4}$ cycloalkyl is optionally substituted by halogen, for example, but not limited to F, $=CH_2$, $=CHF$, $=CF_2$, $=CCl_2$, - $CH_3$, $-CHF_2$, spiro-cyclopropyl, spiro-cyclopropyl substituted by F; $R_5$ is $-C_{1-3}$ alkyl, or $-C_{1-3}$ alkyl substituted by one or more deuterium, for example $-CH_3$, $-CH_2CH_3$, $-CD_3$.

**Embodiment 15:** A compound selected from the compound of the Examples below, or a pharmaceutically acceptable salt or solvate thereof.

In a second aspect, the present invention provides the following compound embodiments:

**Embodiment 11-1:** A compound of Formula (II), a stereoisomer, tautomer, stable isotopic variant, pharmaceutically acceptable salt or solvate thereof,

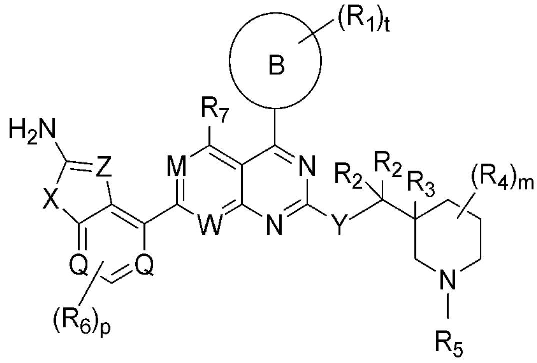

(II)

wherein:

M is selected from N and C-$R_8$;

W is selected from N and C-$R_9$;

Z is selected from N and C-$R_{10}$;

X is selected from S or Se;

each Q is independently selected from C-$R_6$ and N;

Y is selected from O, S, Se and N-$R_a$;

B is selected from a 4-7-membered monocyclic heterocycloalkyl containing 1 to 3 heteroatoms independently selected from N, O, P, S and Se, and a 6-12-membered polycyclic heterocycloalkyl containing 1 to 4 heteroatoms independently selected from N, O, P, S and Se, provided that the ring atom attached to the pyrimidine ring portion of the molecule is a nitrogen heteroatom;

$R_1$ is selected from H, halogen, -CN, -OH, -$NH_2$, -$B(OH)_2$, oxo, -$C_{1-6}$ alkyl, -$C_{2-6}$ alkenyl, -$C_{2-6}$ alkynyl, -O-$C_{1-6}$ alkyl, -C(O)-$C_{1-6}$ alkyl, -C(O)N$(R_b)_2$, wherein the $C_{1-6}$ alkyl present is optionally substituted by halogen, deuterium, -OH, or -O-$C_{1-6}$ alkyl;

$R_2$ is each independently selected from H, D, -$C_{1-6}$ alkyl, and -$(CH_2)_n$-$C_{3-6}$ cycloalkyl, or two $R_2$ together with the carbon atom to which they are attached form $C_{3-4}$ cycloalkyl, wherein the $C_{1-6}$ alkyl, $C_{3-6}$ cycloalkyl, or $C_{3-4}$ cycloalkyl is each independently and optionally substituted by halogen, D, or $C_{1-6}$ alkyl substituted by halogen or D, or -O-$C_{1-6}$ alkyl substituted by halogen or D;

$R_3$ is selected from -$C_{1-6}$ alkyl and -$(CH_2)_n$-$C_{3-6}$ cycloalkyl, each independently and optionally substituted by halogen, D, $C_{1-6}$ alkyl substituted by halogen or D, or -O-$C_{1-6}$ alkyl substituted by halogen or D;

$R_4$ is selected from H, D, halogen, -CN, -OH, -$C_{1-6}$ alkyl, -$C_{2-6}$ alkenyl, -$C_{2-6}$ alkynyl, -$NH_2$, -NH-$C_{1-6}$ alkyl, -N($C_{1-6}$ alkyl$)_2$, -O-$C_{1-6}$ alkyl, -O-$C_{3-6}$ cycloalkyl, and -$(CH_2)_n$-$C_{3-6}$ cycloalkyl, wherein the -$C_{1-6}$ alkyl, -$C_{2-6}$ alkenyl, -$C_{2-6}$ alkynyl, or -$C_{3-6}$ cycloalkyl present is each independently and optionally substituted by D, halogen, CN, or -$OC_{1-6}$ alkyl, or

two $R_4$ attached to the same ring carbon atom to form =$C(R_c)_2$, or two $R_4$ attached to the same ring carbon atom together with the ring carbon atom to which they are attached form a spiro $C_{3-6}$ cycloalkyl or a spiro 4-7 membered heterocycloalkyl, wherein each $R_c$ is independently selected from H, halogen, and -$C_{1-6}$ alkyl optionally substituted by halogen, and the spiro $C_{3-6}$ cycloalkyl or spiro 4-7 membered heterocycloalkyl is optionally substituted by halogen and -$C_{1-6}$ alkyl optionally substituted by halogen;

$R_5$ is selected from H, -$C_{1-6}$ alkyl, -$C_{2-6}$ alkenyl, -$C_{2-6}$ alkynyl, and -$(CH_2)_n$-$C_{3-6}$ cycloalkyl, wherein -$C_{1-6}$ alkyl, -$C_{2-6}$ alkenyl, -$C_{2-6}$ alkynyl, or -$C_{3-6}$ cycloalkyl is each independently and optionally substituted by D, halogen, CN, -$OC_{1-6}$ alkyl or -O-CON$(R_b)_2$;

$R_6$ and $R_7$ are each independently selected from H, D, halogen, CN, $-C_{1-6}$ alkyl, $-C_{2-6}$ alkenyl, - $C_{2-6}$ alkynyl, $-OC_{1-6}$ alkyl, $-S-C_{1-6}$ alkyl and $-Se-C_{1-6}$ alkyl, wherein the $-C_{1-6}$ alkyl, $-C_{2-6}$ alkenyl, and $-C_{2-6}$ alkynyl present are each independently and optionally substituted by halogen, $-OC_{1-6}$ alkyl or D;

$R_8$ is selected from H, D, halogen, CN, $NO_2$, $-C_{1-6}$ alkyl, $-C_{2-6}$ alkenyl, $-C_{2-6}$ alkynyl, or $-OR_b$, wherein $-C_{1-6}$ alkyl, $-C_{2-6}$ alkenyl, and $-C_{2-6}$ alkynyl are each independently and optionally substituted by halogen, CN, or $-OC_{1-6}$ alkyl; or $R_8$ is $-(CH_2)_n-C_{3-6}$ cycloalkyl, wherein the $C_{3-6}$ cycloalkyl is optionally substituted by halogen or CN;

$R_{10}$ is selected from H, D, halogen, CN, $NO_2$, $-C_{1-6}$ alkyl, $-C_{2-6}$ alkenyl, $-C_{2-6}$ alkynyl, or $-OR_b$, wherein the $-C_{1-6}$ alkyl, $-C_{2-6}$ alkenyl, and $-C_{2-6}$ alkynyl are each independently and optionally substituted by halogen or CN;

$R_9$ is selected from H, halogen, -CN, $-C_{1-6}$ alkyl, $-C_{2-6}$ alkenyl, $-C_{2-6}$ alkynyl, $-OR_b$, and $-O-CON(R_b)_2$, wherein the $-C_{1-6}$ alkyl, $-C_{2-6}$ alkenyl, and $-C_{2-6}$ alkynyl are each independently and optionally substituted by halogen or CN;

$R_a$ is selected from H, $-C_{1-6}$ alkyl optionally substituted by halogen;

$R_b$ is selected from H, $-C_{1-6}$ alkyl optionally substituted by halogen, -OH, $-OC_{1-6}$ alkyl or deuterium, and $-C_{3-6}$ cycloalkyl, or wherein two $R_b$ attached to the same N atom together with the N atom to which they are attached form a 4-7 membered heterocycloalkyl optionally substituted by halogen or $-C_{1-6}$ alkyl;

n and p are each independently an integer from 0 to 3; and

m and t are each independently an integer from 0 to 8;

in a specific embodiment, $R_1$ is selected from H, halogen, -CN, -OH, $-NH_2$, $-B(OH)_2$, oxo, $-C_{1-6}$ alkyl, $-O-C_{1-6}$ alkyl, $-C(O)-C_{1-6}$ alkyl, $-C(O)N(R_b)_2$, wherein the $C_{1-6}$ alkyl present is optionally substituted by halogen, $R_8$ and $R_{10}$ are each independently selected from H, D, halogen, CN, $NO_2$, - $C_{1-6}$ alkyl, $-C_{2-6}$ alkenyl, $-C_{2-6}$ alkynyl, and $-OR_b$, wherein the $-C_{1-6}$ alkyl, $-C_{2-6}$ alkenyl, and $-C_{2-6}$ alkynyl are each independently and optionally substituted by halogen or CN, $R_a$ and $R_b$ are each independently selected from H and $-C_{1-6}$ alkyl optionally substituted by halogen, and the remaining groups are defined under Formula II above;

in a specific embodiment, $R_1$ is selected from H, halogen, -CN, -OH, $-NH_2$, $-B(OH)_2$, oxo, $-C_{1-6}$ alkyl, $-O-C_{1-6}$ alkyl, $-C(O)-C_{1-6}$ alkyl, $-C(O)N(R_b)_2$, wherein the $C_{1-6}$ alkyl present is optionally substituted by halogen, $R_a$ and $R_b$ are each independently selected from H and $-C_{1-6}$ alkyl optionally substituted by halogen, or the two $R_b$ attached to the same N atom together with the N atom to which they are attached form a 4-7 heterocycloalkyl, and the remaining groups are defined under Formula II above;

in a specific embodiment, $R_1$ is selected from H, halogen, -CN, -OH, $-NH_2$, $-B(OH)_2$, oxo, $-C_{1-6}$ alkyl, $-C_{2-6}$ alkenyl, $-C_{2-6}$ alkynyl, $-O-C_{1-6}$ alkyl, $-C(O)-C_{1-6}$ alkyl, $-C(O)N(R_b)_2$, wherein the $C_{1-6}$ alkyl present is optionally substituted by halogen, deuterium, or $-O-C_{1-6}$ alkyl; $R_a$ and $R_b$ are each independently selected from H and $-C_{1-6}$ alkyl optionally substituted by halogen or deuterium, or two $R_b$ attached to the same N atom together with the N atom to which they are attached form a 4-7 membered heterocycloalkyl; and the remaining groups are defined under Formula II above;

**Embodiment II-2:** The compound of Formula (II) according to Embodiment II-1, a stereoisomer, tautomer, stable isotope variant, pharmaceutically acceptable salt or solvate thereof, which is the compound of Formula (I) of any one of Embodiment 1 to Embodiment 15 of the first aspect of the present invention, a stereoisomer, tautomer, stable isotopic variant, pharmaceutically acceptable salt or solvate thereof.

**Embodiment II-3:** The compound of Formula (II) according to Embodiment II-1 or Embodiment II-2, a stereoisomer, tautomer, stable isotope variant, pharmaceutically acceptable salt or solvate thereof, wherein Z is selected from N and $C-R_{10}$, wherein $R_{10}$ is defined as in any one of Embodiment 1.2.1 to Embodiment 1.3 of the first aspect of the present invention; preferably Z is C-CN.

**Embodiment II-4:** The compound of Formula (II) accroding to Embodiment II-1 to Embodiment II-3, a stereoisomer, tautomer, stable isotope variant, pharmaceutically acceptable salt or solvate thereof, wherein p is 0, or p is 1, or p is 2, or p is 3, preferably p is selected from an integer of 0 to 2.

**Embodiment II-5:** The compound of Formula (II) according to any one of Embodiments II-1 to II-4, a stereoisomer, tautomer, stable isotope variant, pharmaceutically acceptable salt or solvate thereof, wherein the fused ring fragment containing X, Z, and Q is selected from

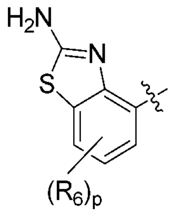
,

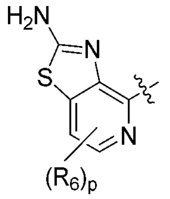
,
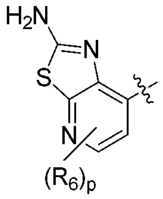
,
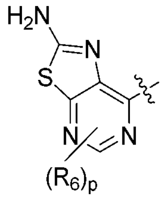
,
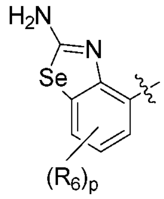
,
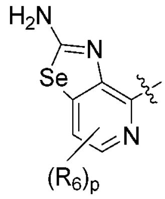
,
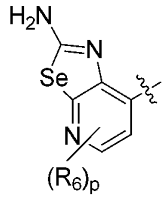
,

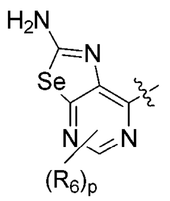
,
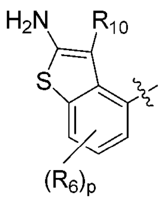
,
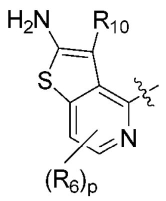
,
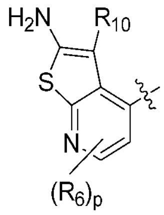
,
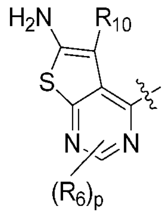
,
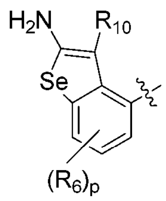
,

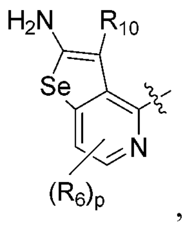
,
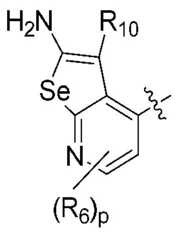
or
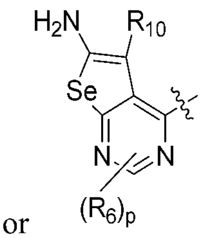
;

in a specific embodiment, the fused ring fragment containing X, Z, and Q is selected from

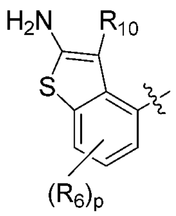
or
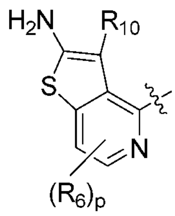
,

for example

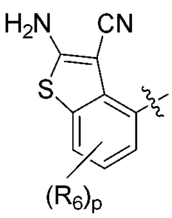
or
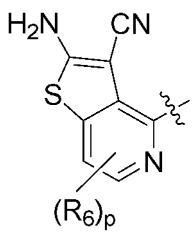
;

preferably

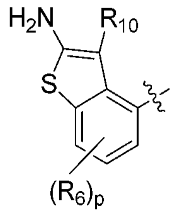
,

more preferably

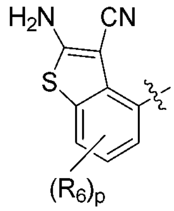
.

**Embodiment II-6:** The compound of Formula (II) according to any one of Embodiments II-1 to II-5, a stereoisomer, tautomer, stable isotope variant, pharmaceutically acceptable salt or solvate thereof, wherein $R_6$ is defined as in any one of Embodiments 2.2.1 to 2.2.5 of the first aspect of the present invention;

preferably, $R_6$ is selected from H, halogen, CN, and -$C_{1-6}$ alkyl optionally substituted by halogen, and p is selected from an integer of 0 to 2;

more preferably, there is a $R_6$ selected from halogen, CN, and -$C_{1-6}$ alkyl optionally substituted by halogen, preferably selected from halogen, CN, and -$C_{1-6}$ alkyl optionally substituted by halogen, and more preferably halogen, most preferably F, and this $R_6$ is located at the para, ortho or meta position relative to the junction point of the fused ring where it is present and the rest of the molecule, preferably para or ortho, i.e.

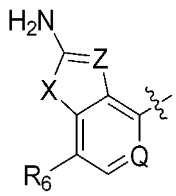
,

for example

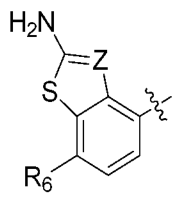
or
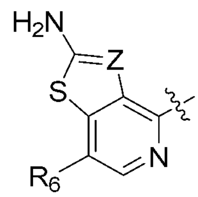
,

more specifically

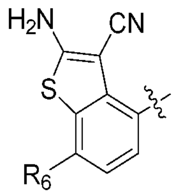
or
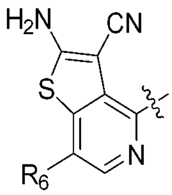
, or
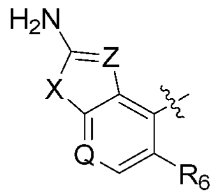
,

for example

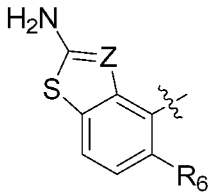

or

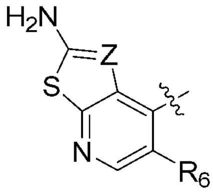

,

more specifically

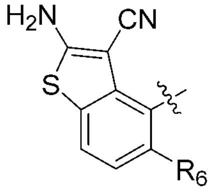 or 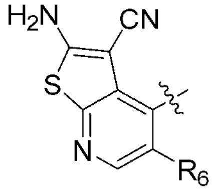, or

there are two $R_6$, each independently selected from halogen, CN, and $C_{1-6}$ alkyl optionally substituted by halogen, preferably selected from halogen, CN, and $C_{1-6}$ alkyl substituted by halogen, more preferably halogen, most preferably F; and said two $R_6$ are each located at the para and ortho positions, or the para and meta positions, or the ortho and meta positions relative to the junction point of the fused ring where they are present and the rest of the molecule, more preferably the para and ortho positions, i.e.,

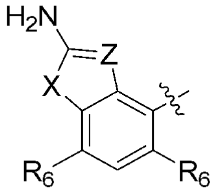

,

preferably

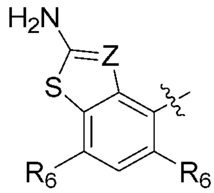

,

more preferably

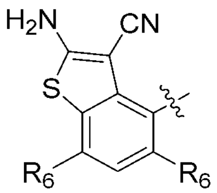

.

**Embodiment II-7:** The compound of Formula (II) according to any one of Embodiments II-1 to II-5, a stereoisomer, tautomer, stable isotope variant, pharmaceutically acceptable salt or solvate thereof, wherein examples of the fused ring fragment containing X, Z, and Q include, but are not limited to:

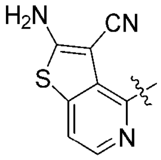 , 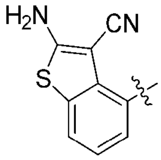 , 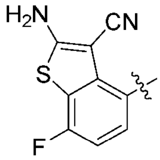 , 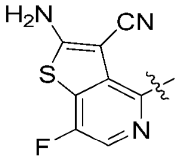 , 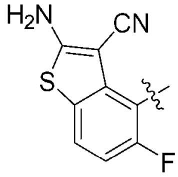 , 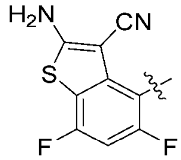 ,

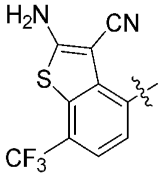 , 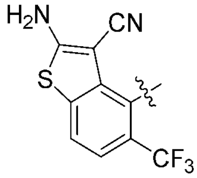 , 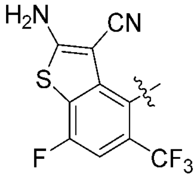 , 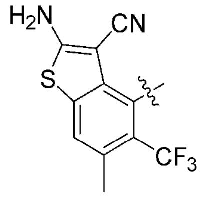 , 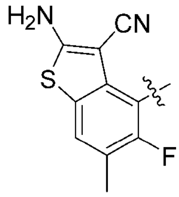 , 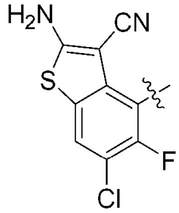 ,

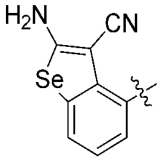 , 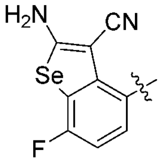 , 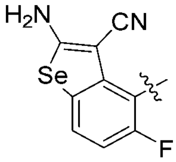 , 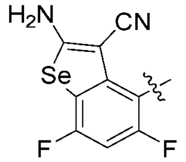 , 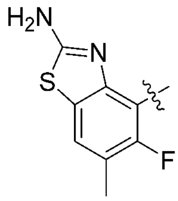 , 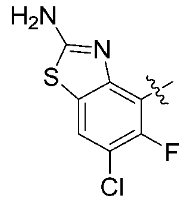 ,

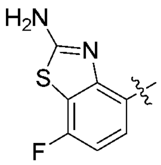 , 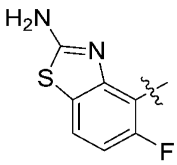 , 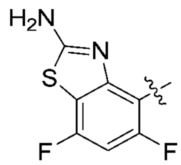 , 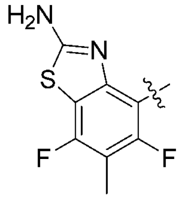 , 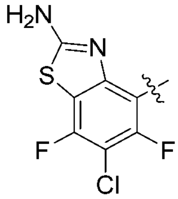 , 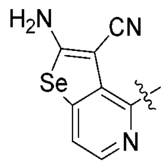 ,

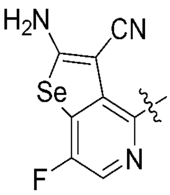 , 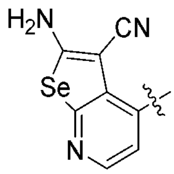 , 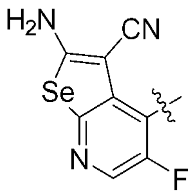 .

**Embodiment II-8:** The compound of Formula (II) according to any one of Embodiments II-1 to II-7, a stereoisomer, tautomer, stable isotope variant, pharmaceutically acceptable salt or solvate thereof, wherein M is defined as in any one of the Embodiments 3.1 to 3.3 of the first aspect of the present invention.

**Embodiment II-8-1:** The compound of Formula (II) according to any one of Embodiments II-1 to II-7, a stereoisomer, tautomer, stable isotope variant, pharmaceutically acceptable salt or solvate thereof, wherein M is $NO_2$.

**Embodiment II-8-2:** The compound of Formula (II) according to any one of Embodiments II-1 to II-7, a stereoisomer, tautomer, stable isotope variant, pharmaceutically acceptable salt or solvate thereof, wherein M is selected from N, C-H, C-halogen, C-CN, C-$NO_2$, C-halogen-substituted -$C_{1-6}$ alkyl and C-halogen-substituted -$C_{2-6}$ alkenyl; preferably M is selected from N, C-halogen, C-CN, C-$NO_2$, C-halogen-substituted -$C_{1-3}$ alkyl and C-halogen-substituted -$C_{2-4}$ alkenyl; more preferably M is selected from N, C-Cl, C-F, C-CN, C-$NO_2$, C-$CF_3$, C-$CHF_2$, C-CH=CHF; and most preferably M is selected from C-Cl and C-$CF_3$; or

M is selected from C-$C_{1-6}$ alkyl, preferably C-$C_{1-3}$ alkyl, e.g., C-$CH_2$-$CH_3$; or

M is selected from C-$C_{3-6}$ cycloalkyl, e.g., C-cyclopropyl; or

M is selected from $C-C_{1-6}$ alkyl-$O-C_{1-6}$ alkyl, preferably $C-C_{1-3}$ alkyl-$O-C_{1-3}$ alkyl, e.g., $C-CH_2-O-CH_3$;

in one embodiment, M is selected from C-halogen and C-halogen-substituted $-C_{1-6}$ alkyl, preferably C-halogen and C-halogen-substituted $-C_{1-3}$ alkyl, e.g., C-Cl and $C-CF_3$.

**Embodiment II-9:** The compound of Formula (II) according to any one of Embodiments II-1 to II-8-2, a stereoisomer, tautomer, stable isotope variant, pharmaceutically acceptable salt or solvate thereof, wherein W is defined as in any one of Embodiments 4.1 to 4.3 of the first aspect of the present invention; preferably W is $C-R_9$, wherein $R_9$ is halogen and preferably F;
in one embodiment, W is $C-R_9$, wherein $R_9$ is selected from halogen, $-C_{1-6}$ alkyl optionally substituted by halogen, -OH, $-OC_{1-6}$ alkyl, preferably selected from halogen, $-C_{1-3}$ alkyl optionally substituted by halogen, -OH, $-OC_{1-3}$ alkyl, specifically F, Cl, $-CH_3$, $-CHF_2$, -OH, and $-OCH_3$, preferably selected from halogen and $-C_{1-3}$ alkyl, e.g., F or $-CH_3$.
**Embodiment II-10:** The compound of Formula (II) according to any one of Embodiments II-1 to II-9, a stereoisomer, tautomer, stable isotope variant, pharmaceutically acceptable salt or solvate thereof, wherein $R_7$ is defined as in any one of the Embodiments 5.1 to 5.7 of the first aspect of the present invention.
**Embodiment II-10-1:** The compound of Formula (II) according to any one of Embodiments II-1 to II-9, a stereoisomer, tautomer, stable isotope variant, pharmaceutically acceptable salt or solvate thereof, wherein $R_7$ is selected from $-C_{1-6}$ alkyl, $-C_{2-6}$ alkenyl, $-C_{2-6}$ alkynyl, $-OC_{1-6}$ alkyl, wherein the $-C_{1-6}$ alkyl, $-C_{2-6}$ alkenyl, and $-C_{2-6}$ alkynyl present are each independently and optionally substituted by $-O-C_{1-6}$ alkyl or D, include, but are not limited to $-CD_3$, $-O-CD_3$, $-CH_2-O-CH_3$, $-CH_2-O-CD_3$.
**Embodiment II-10-2:** The compound of Formula (II) according to any one of Embodiments II-1 to II-9, a stereoisomer, tautomer, stable isotope variant, pharmaceutically acceptable salt or solvate thereof, wherein $R_7$ is H; or $R_7$ is selected from $-C_{1-6}$ alkyl, $-C_{2-6}$ alkenyl, $-C_{2-6}$ alkynyl, and $-O-C_{1-6}$ alkyl, wherein the $-C_{1-6}$ alkyl, $-C_{2-6}$ alkenyl, and $-C_{2-6}$ alkynyl present are each preferably $-C_{1-3}$ alkyl, $-C_{2-4}$ alkenyl, $-C_{2-4}$ alkynyl, and are each independently and optionally substituted by halogen, or are each independently substituted by $-O-C_{1-3}$ alkyl or D.
**Embodiment II-11:** The compound of Formula (II) according to any one of Embodiments II-1 to II-7, a stereoisomer, tautomer, stable isotope variant, pharmaceutically acceptable salt or solvate thereof, wherein

M is selected from N, C-H, C-halogen, C-CN, $C-NO_2$, C-halogen-substituted $-C_{1-3}$ alkyl and C-halogen-substituted $-C_{2-6}$ alkenyl; preferably M is selected from N, C-halogen, C-CN, $C-NO_2$, C-halogen-substituted $-C_{1-3}$ alkyl and C-halogen-substituted $-C_{2-4}$ alkenyl; more preferably M is selected from N, C-Cl, C-F, C-CN, $C-NO_2$, $C-CF_3$, $C-CHF_2$, C-CH=CHF;
Or M is selected from $C-C_{1-6}$ alkyl, preferably $C-C_{1-3}$ alkyl, e.g., $C-CH_2-CH_3$; or M is selected from $C-C_{3-6}$ cycloalkyl, e.g., C-cyclopropyl; or M is selected from $C-C_{1-6}$ alkyl-$O-C_{1-6}$ alkyl, preferably $C-C_{1-3}$ alkyl-$O-C_{1-3}$ alkyl, e.g., $C-CH_2-O-CH_3$;
Or M is selected from C-halogen and C-halogen-substituted $-C_{1-6}$ alkyl, preferably C-halogen and C-halogen-substituted $-C_{1-3}$ alkyl, more preferably is C-Cl and $C-CF_3$;
W is $C-R_9$, wherein $R_9$ is halogen, preferably F; or $R_9$ is $C_{1-6}$ alkyl, preferably $-C_{1-3}$ alkyl, optionally substituted by halogen; or $R_9$ is $-OC_{1-6}$ alkyl, preferably $-OC_{1-3}$ alkyl; or $-R_9$ is OH;
$R_7$ is H; or $R_7$ is selected from $-C_{1-6}$ alkyl, $-C_{2-6}$ alkenyl, $-C_{2-6}$ alkynyl, $-OC_{1-6}$ alkyl, wherein the $-C_{1-6}$ alkyl, $-C_{2-6}$ alkenyl, and $-C_{2-6}$ alkynyl present are each preferably $-C_{1-3}$ alkyl, $-C_{2-4}$ alkenyl, $-C_{2-4}$ alkynyl, and are each independently and optionally substituted by halogen, or are each independently substituted by $-O-C_{1-3}$ alkyl or D;
in the compound according to any one of Embodiments II-1 to II-7, the fused parent nuclei containing M and W for example, but not limited to:

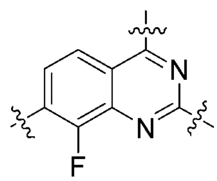
,
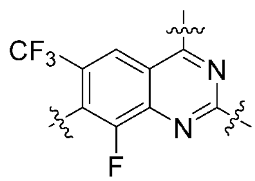
,

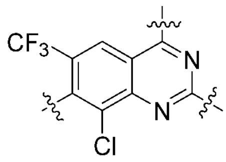, 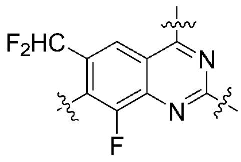, 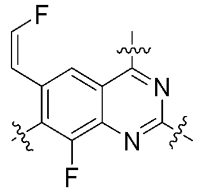, 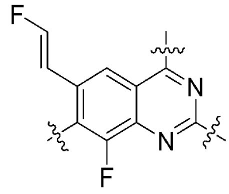, 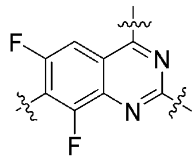,

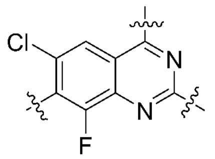, 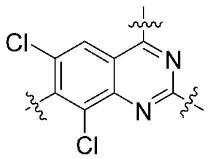, 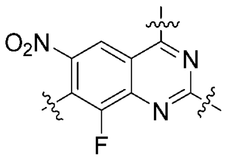, 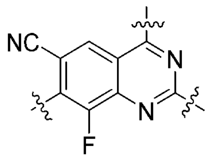, 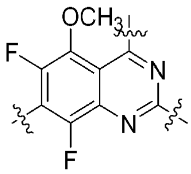,

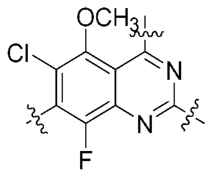, 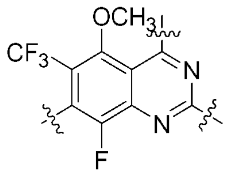, 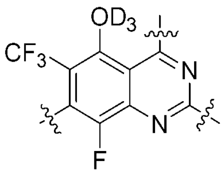, 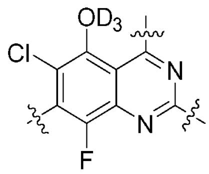, 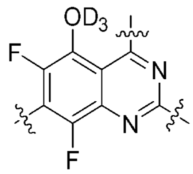,

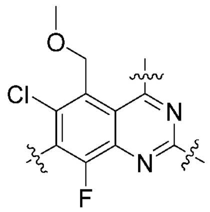, 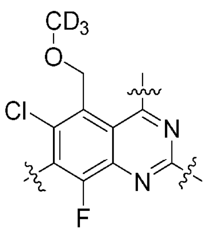, 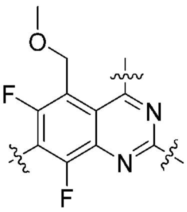, 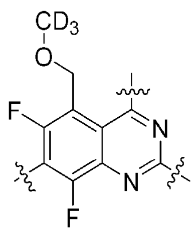, 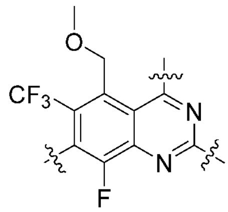,

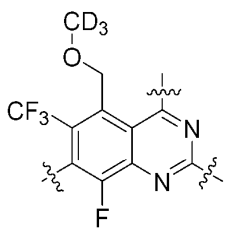, 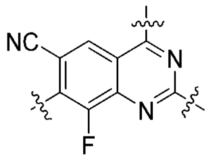, 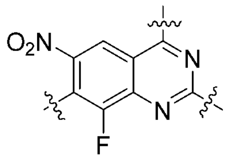, 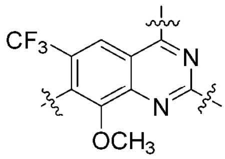, 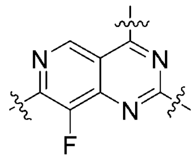,

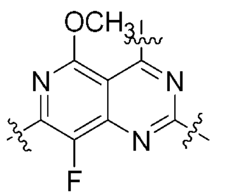, 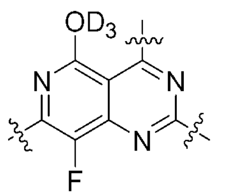, 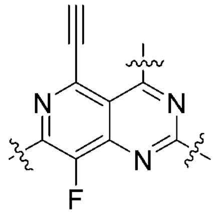;

preferably

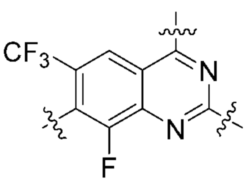,

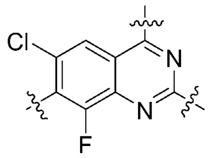, 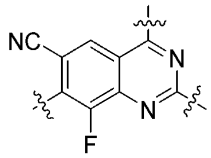, 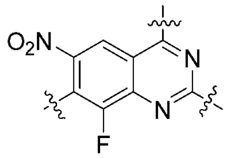, 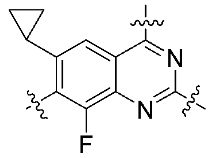, 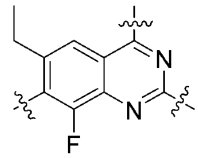,

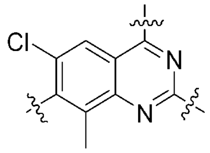, 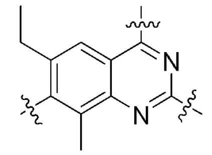, 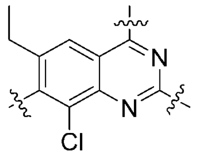, 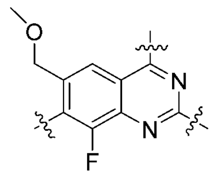, 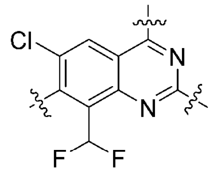,

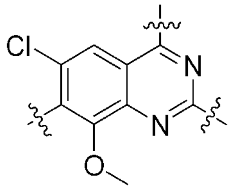, 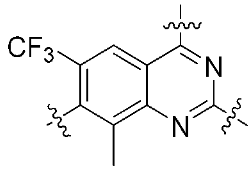, 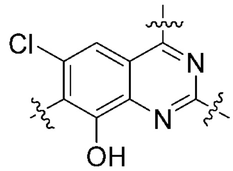;

in some specific embodiments, the fused parent nuclei containing M and W is

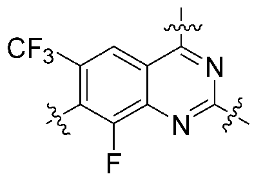;

in some specific embodiments, the fused parent nuclei containing M and W is

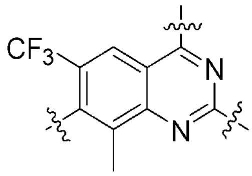;

in some specific embodiments, the fused parent nuclei containing M and W is

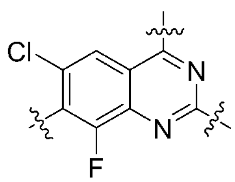;

in some specific embodiments, the fused parent nuclei containing M and W is

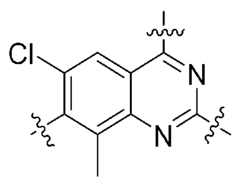;

in some specific embodiments, the fused parent nuclei containing M and W is

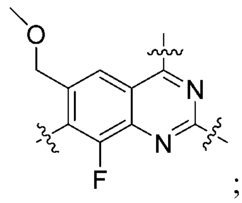

;

in some specific embodiments, the fused parent nuclei containing M and W is

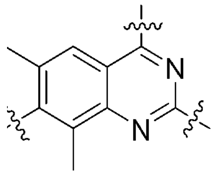

;

in some specific embodiments, the fused parent nuclei containing M and W is

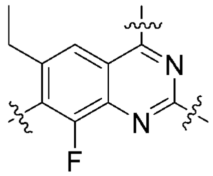

;

in some specific embodiments, the fused parent nuclei containing M and W is

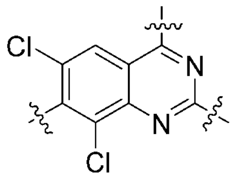

;

in some specific embodiments, the fused parent nuclei containing M and W is

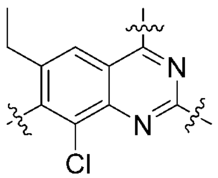

;

in some specific embodiments, the fused parent nuclei containing M and W is

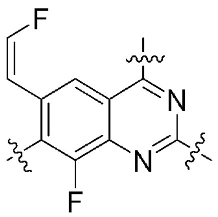

,

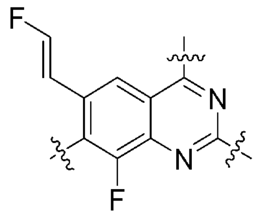

;

in some specific embodiments, the fused parent nuclei containing M and W is

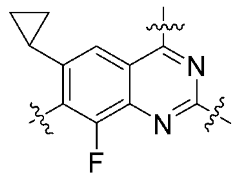

;

in some specific embodiments, the fused parent nuclei containing M and W is

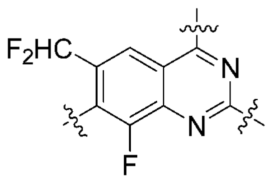

;

in some specific embodiments, the fused parent nuclei containing M and W is

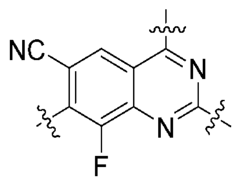

;

in some specific embodiments, the fused parent nuclei containing M and W is

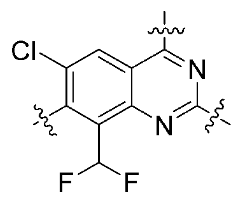

;

in some specific embodiments, the fused parent nuclei containing M and W is

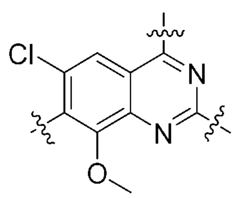

;

in some specific embodiments, the fused parent nuclei containing M and W is

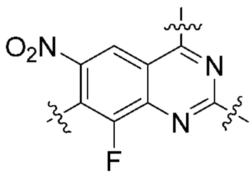

.

**Embodiment II-12:** The compound of Formula (II) according to any one of Embodiments II-1 to II-11, a stereoisomer, tautomer, stable isotope variant, pharmaceutically acceptable salt or solvate thereof, wherein B is defined as in any one of Embodiments 6.1 to 6.3 of the first aspect of the present invention;

In further embodiments, B is a 4-7 membered monocyclic alkyl containing 1 nitrogen atom or containing 1 nitrogen atom and 1 heteroatom selected from O, P, Se, and S (preferably 4-7 membered monocyclic alkyl containing 1 nitrogen atom or containing 1 nitrogen atom and 1 O atom), or a 8-10 membered spiro heterocycloalkyl or fused heteroalkyl containing 1-3 nitrogen atoms and 0-1 heteroatom selected from O, S, P, or Se (preferably 1-3 nitrogen atoms and 0-1 O atom), each optionally substituted by 0-3 groups independently selected from -OH, $NH_2$, CN, halogen, $-C_{1-6}$ alkyl, $-COC_{1-6}$ alkyl, $-CON(R_b)_2$;

In a specific embodiment, B is a 4-7 membered monocyclic heterocycloalkyl containing 1 to 2 (preferably 1) heteroatoms independently selected from N, O, P, Se, and S (preferably N), such as a 4-membered heterocycloalkyl, which is connected to the pyrimidine ring portion of the molecule by a nitrogen heteroatom, for example

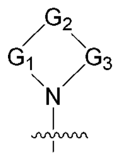

,

each G in the ring can be C, or at most one of them is a heteroatom selected from N, O, P, Se, and S, for example, a 4-membered monocyclic heterocycloalkyl containing 1 nitrogen atom, a 4-membered monocyclic heterocycloalkyl containing 1 nitrogen atom and 1 heteroatom selected from O, P, Se and S (e.g., a 4-membered monocyclic heterocycloalkyl containing 1 nitrogen atom and 1 O atom), and a 5-7-membered monocyclic heterocycloalkyl containing 2 nitrogen atoms; for example, B is

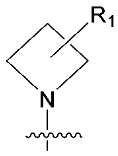

,

for example

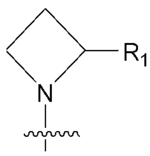

,

wherein $R_1$ is defined as in embodiment II, in one embodiment, $R_1$ is selected from $-C(O)-C_{1-6}$ alkyl and $-C(O)N(R_b)_2$, e.g., $R_1$ is selected from $-C(O)-C_{1-3}$ alkyl, $-C(O)NH_2$, $-C(O)NH(C_{1-3}$ alkyl), $- C(O)N(C_{1-3}$ alkyl$)_2$.

**Embodiment II-12-1:** The compound of Formula (II) according to any one of Embodiments II-1 to II-11, a stereoisomer, tautomer, stable isotope variant, pharmaceutically acceptable salt or solvate thereof, where B is

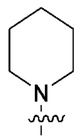,

substituted by 1-2 $R_1$, for example, but not limited to

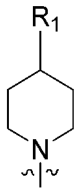, 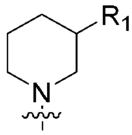,

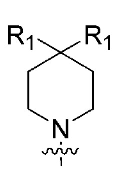, 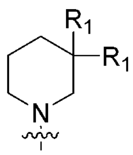, 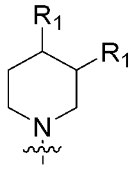, 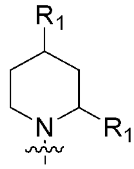,

wherein each $R_1$ is independently selected from H, halogen, -CN, -OH, $-NH_2$, $-B(OH)_2$, oxo, $-C_{1-6}$ alkyl, $-O-C_{1-6}$ alkyl, $-C(O)-C_{1-6}$ alkyl, $C(O)N(R_b)_2$, wherein the $C_{1-6}$ alkyl present is optionally substituted by halogen; preferably selected from halogen, CN, -OH, $-C_{1-6}$ alkyl, and $-OC_{1-6}$ alkyl, wherein the $-C_{1-6}$ alkyl, preferably $-C_{1-3}$ alkyl, is optionally substituted by halogen;

In a specific embodiment, B is

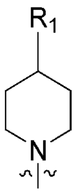 or 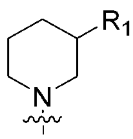,

wherein $R_1$ is selected from halogen, CN, halogen-substituted $-C_{1-6}$ alkyl;

In a specific embodiment, B is

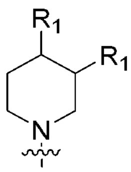 or 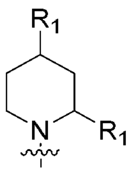,

can also be expressed as

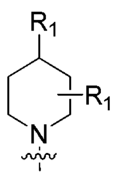,

wherein one $R_1$ is selected from halogen, CN, $-C_{1-6}$ alkyl optionally substituted by halogen, preferably located at

the para position of the ring N atom, and the other is selected from H, -OH, $-C_{1-6}$ alkyl optionally substituted by halogen or $-O-C_{1-6}$ alkyl; for example, but not limited to

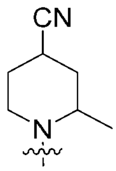,
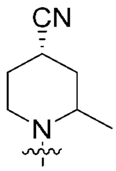,

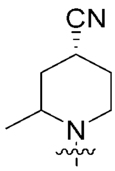,
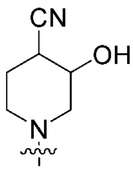,
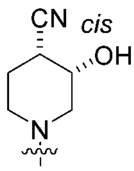;

In a specific embodiment, B is

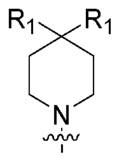 or 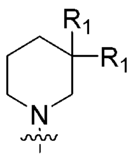,

wherein one $R_1$ is -OH and the other is - $C_{1-6}$ alkyl optionally substituted by halogen, preferably $-C_{1-3}$ alkyl, and more preferably methyl; more preferably

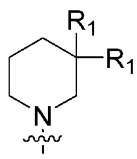,

wherein one $R_1$ is -OH and the other is $-C_{1-6}$ alkyl, preferably $C_{1-3}$ alkyl, preferably methyl, e.g., B is

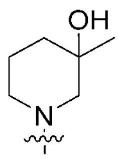,

preferably

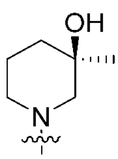.

**Embodiment 11-12-2:** The compound of Formula (II) according to any one of Embodiments II-1 to II-11, a stereoisomer, tautomer, stable isotope variant, pharmaceutically acceptable salt or solvate thereof, wherein B is a 7-membered monocyclic saturated heterocycloalkyl containing 1-2 heteroatoms selected from N, O, and S;
In a specific embodiment, B is

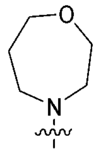,

substituted by 1-2 $R_1$, for example, but not limited to

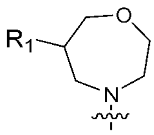, 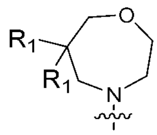, 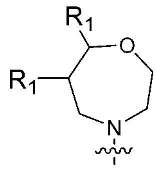, 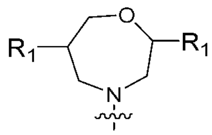,

preferably

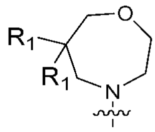,

as defined in any one of the Embodiments 6.1.2 to 6.1.2.3 of the first aspect of this present invention.

**Embodiment II-12-3:** The compound of Formula (II) according to any one of Embodiments II-1 to II-11, a stereoisomer, tautomer, stable isotope variant, pharmaceutically acceptable salt or solvate thereof, wherein B is a 6-12 membered polycyclic heterocycloalkyl containing 1 to 4 heteroatoms independently selected from N, O, P, Se, and S, for example 6-12 membered spiro heterocycloalkyl, for example 6-12 membered fused heterocycloalkyl, such as the groups as specifically defined and/or exemplified in Embodiment 6.2 of the first aspect of the present invention.

In a specific embodiment, B is a piperidine spiro 4-5 membered heterocycloalkyl, the heterocycloalkyl contains 1 or 2 heteroatoms selected from N or O, for example, but not limited to,

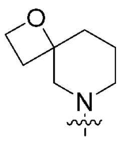, 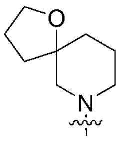, 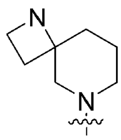, 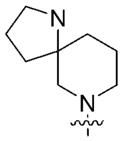, 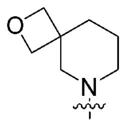, 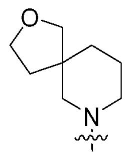, 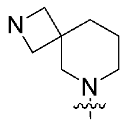, 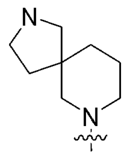, 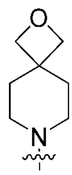,

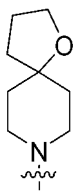, 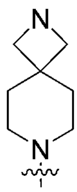, 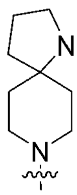, 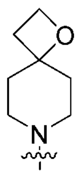, 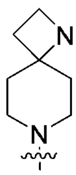;

preferably

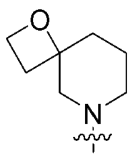;

each independently substituted by 0 to 8, preferably 0 to 2 $R_1$;

In a specific embodiment, B is an oxazacycloheptanocyclopropane or diazacycloheptanocyclopropane, for

example, but not limited to

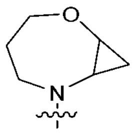 , 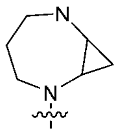 ,

preferably

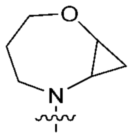 .

**Embodiment II-12-3-1:** The compound of Formula (II) according to any one of Embodiments II-1to II-11, a stereoisomer, tautomer, stable isotope variant, pharmaceutically acceptable salt or solvate thereof, wherein B in a specific embodiment, B is a 9-10 membered dicyclic heterocycloalkyl connected by nitrogen atoms, and contains 1 to 3 heteroatoms selected from N and O, such as a six-membered and five-membered fused heterocycloalkyl, a seven-membered and five-membered fused heterocycloalkyl, which can be specifically expressed as

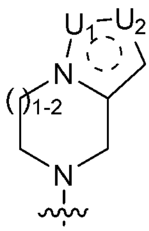 ,

where

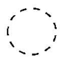

indicates that the ring can be saturated, partially unsaturated, or aromatic, $U_1$ selected from C or N, $U_2$ from N, C, or O, for example, but not limited to

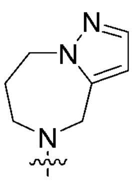 , 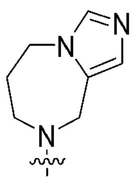 , 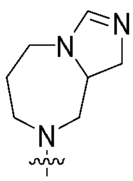 , 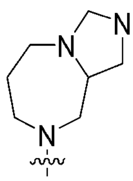 , 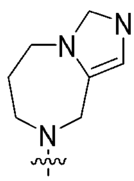 ,

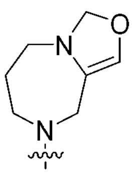 , 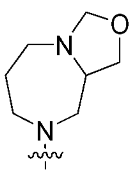 , 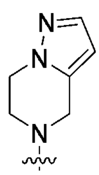 ,

substituted by 0 to 8, preferably 1 to 3, and preferably 1 to 2 $R_1$, wherein $R_1$ is independently selected from H, halogen, -CN, -OH, $-NH_2$, $-B(OH)_2$, oxo, $-C_{1-6}$ alkyl, - O-$C_{1-6}$ alkyl, -C(O)-$C_{1-6}$ alkyl, $-C(O)N(R_b)_2$, wherein the $C_{1-6}$ alkyl present is optionally substituted by halogen; preferably selected from H, halogen, CN, $-NH_2$, oxo, $-C_{1-6}$ alkyl, -C(O)-$C_{1-6}$ alkyl, -$C(O)N(R_b)_2$, wherein the $-C_{1-6}$ alkyl, preferably $-C_{1-3}$ alkyl, is optionally substituted by halogen; Rb is preferably H or $-C_{1-3}$ alkyl, or two $R_b$ together with the N atom to which they are attached can form a 4-7 heterocycloalkyl , for example, it also contains 4-7 membered heterocycloalkyl containing 1 or 2 heteroatoms selected from N and O;

preferably, B is selected from

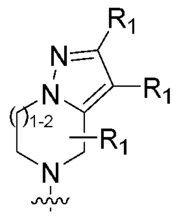

,

for example

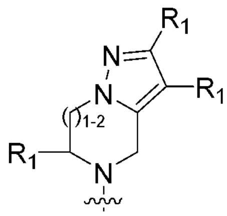

,

wherein $R_1$ is selected from H, halogen, -CN, $NH_2$, -$C_{1-6}$ alkyl, -C(O)-$C_{1-6}$ alkyl, and -C(O)N$(R_b)_2$, wherein the -$C_{1-6}$ alkyl is preferably -$C_{1-3}$ alkyl, or two of the $R_b$ together with the N atom to which they are attached can form a 4-7 membered heterocycloalkyl, for example, it also contains 4-7 membered heterocycloalkyl containing 1 or 2 heteroatoms selected from N and O; more preferably, $R_1$ at the ortho position of the nitrogen heteroatom of the five-membered ring is selected from -C(O)-$C_{1-6}$ alkyl and -C(O)N$(R_p)_2$, for example -CON$(C_{1-3}$ alkyl$)_2$, -CO-4-7 monocyclic heterocycloalkyl, e.g., - CON$(CH_3)_2$,

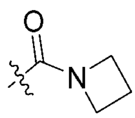 , 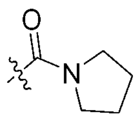 , 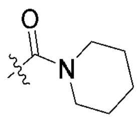 , 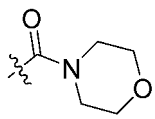 ,

and the remaining $R_1$ is each independently selected from H, halogen, -CN, -$NH_2$ and -$C_{1-6}$ alkyl, and preferably from H, halogen, and -$C_{1-6}$ alkyl;

**Embodiment II-12-3-2:** The compound of Formula (II) according to any one of Embodiment II-1 to 11-11, a stereoisomer, tautomer, stable isotope variant, pharmaceutically acceptable salt or solvate thereof, wherein B is a 9-10 membered fused bicyclic heterocycloalkyl connected by a nitrogen atom and also contains one to two heteroatoms selected from N and O, specifically,

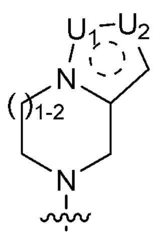

,

wherein

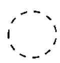

indicates that the ring can be saturated, partially unsaturated, or aromatic, $U_1$ is selected from C or N, and $U_2$ is selected from N, C, or O, for example, but not limited to

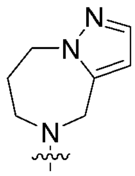, 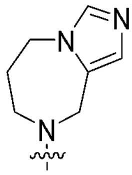, 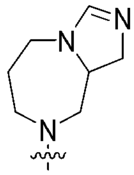, 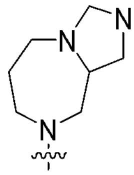, 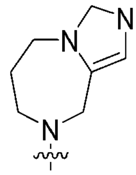, 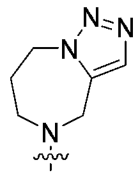, 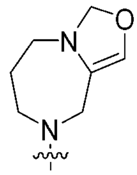,

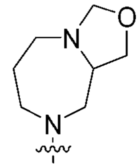, 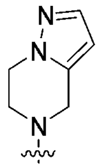,

substituted by 1 to 3, e.g., 1,2 or 3 $R_1$, wherein each $R_1$ is independently selected from H, halogen -CN, -OH, $-NH_2$, $-C_{1-6}$ alkyl, $-C_{2-6}$ alkenyl, $-C_{2-6}$ alkynyl, $-O-C_{1-6}$ alkyl, - $C_{1-6}$ alkyl-O-$C_{1-6}$ alkyl, $-C(O)N(R_b)_2$, preferably selected from H, halogen, -CN, -OH, $-NH_2$, $-C_{1-6}$ alkyl, $-C_{1-6}$ alkyl-O-$C_{1-6}$ alkyl, $-C(O)N(R_b)_2$, wherein each $R_b$ is independently H or $C_{1-6}$ alkyl optionally substituted by one or more deuterium, wherein the $-C_{1-6}$ alkyl, preferably $-C_{1-3}$ alkyl, present in $R_1$ is optionally substituted by one or more halogen or deuterium; or, wherein each $R_b$ is independently selected from H, $-C_{1-6}$ alkyl or $-C_{3-6}$ cycloalkyl optionally substituted by one or more halogen, deuterium, $-O-C_{1-6}$ alkyl, or two $R_b$ together with the N atom to which they are attached form a 4-7-membered heterocycloalkyl optionally substituted by halogen or $-C_{1-6}$ alkyl;
preferably, B is selected from

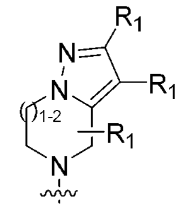,

for example

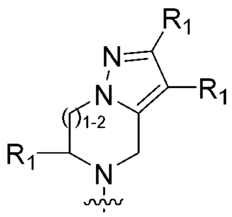,

wherein

$R_1$ at the ortho position of the nitrogen heteroatom on the five-membered ring is selected from $-C(O)-C_{1-6}$ alkyl and $-C(O)N(R_b)_2$, preferably $-C(O)N(R_b)_2$, each $R_b$ is independently H or $-C_{1-6}$ alkyl optionally substituted by one or more deuterium, preferably each is independently H or $-C_{1-3}$ alkyl optionally substituted by one or more deuterium, for example, $-C(O)N(CH_3)_2$, $-C(O)N(CD_3)_2$,
$R_1$ at the meta position of the nitrogen heteroatom on the five-membered ring is selected from H, halogen, -CN, -OH, $-NH_2$, $-C_{1-6}$ alkyl, $-C_{2-6}$ alkenyl, $-C_{2-6}$ alkynyl, $-O-C_{1-6}$ alkyl, and $-C_{1-6}$ alkyl-O-$C_{1-6}$ alkyl, preferably selected from H, halogen, -CN, -OH, $-NH_2$, $-C_{1-3}$ alkyl, $-C_{2-4}$ alkenyl, $-C_{2-4}$ alkynyl, $-O-C_{1-3}$ alkyl, and $-C_{1-3}$ alkyl-O-$C_{1-3}$ alkyl, wherein the alkyl present is optionally substituted by one or more halogen or deuterium, i.e., $R_1$ at this position is, for example, but not limited to H, F, Cl, -CN, -OH, $-NH_2$, $-CH_3$, $-C\equiv CH$, $-O-CH_3$, $-O-CD_3$, $-O-CH_2-CH_3$, $-CH_2-O-CH_3$, $-CD_2-O-CD_3$,
B and $R_1$ at the ortho position wherein nitrogen is attached to the rest of the molecule is selected from H or $-C_{1-6}$ alkyl, preferably H or $-C_{1-3}$ alkyl, e.g., H, $-CH_3$, $-CH_2CH_3$; or
$R_1$ at the ortho position of the nitrogen heteroatom on the five-membered ring is selected from $-C(O)-C_{1-6}$ alkyl and $-C(O)N(R_b)_2$, preferably $-C(O)N(R_b)_2$, each $R_b$ is independently H, $-C_{1-6}$ alkyl optionally substituted by one or

more halogen, deuterium, -O-$C_{1-6}$ alkyl, or -$C_{3-6}$ cycloalkyl, preferably each independently H or -$C_{1-3}$ alkyl optionally substituted by one or more deuterium, most preferably each independently -$C_{1-3}$ alkyl optionally substituted by one or more deuterium, such as - $C(O)CH_3$, -$C(O)N(CH_3)(CH_2CH_2F)$, -$C(O)N(CH_3)_2$, -C(O) $N(CH_3)$(cyclopropyl), -$C(O)NH(CH_3)$, - $C(O)N(CD_3)_2$, -$C(O)N(CH_3)(CH_2CH_2CH_2OCH_3)$; or two $R_b$ form a 4-7 membered heterocycloalkyl optionally substituted by halogen or -$C_{1-6}$ alkyl, such as azetidinyl, 3-F-azetidinyl, morpholinyl, pyrrolidinyl, piperazinyl, 4-methylpiperazinyl,

$R_1$ at the meta position of the nitrogen heteroatom on the five-membered ring is selected from H, halogen, -CN, -OH, -$NH_2$, -$C_{1-6}$ alkyl, -$C_{2-6}$ alkenyl, -$C_{2-6}$ alkynyl, -O-$C_{1-6}$ alkyl, and -$C_{1-6}$ alkyl-O-$C_{1-6}$ alkyl, preferably selected from H, halogen, -CN, -OH, -$NH_2$, -$C_{1-3}$ alkyl, -$C_{2-4}$ alkenyl, -$C_{2-4}$ alkynyl, -O-$C_{1-3}$ alkyl, and -$C_{1-3}$ alkyl-O-$C_{1-3}$ alkyl, more preferably selected from -$C_{1-3}$ alkyl-O-$C_{1-3}$ alkyl, wherein the alkyl present is optionally substituted by one or more halogen, OH, or deuterium, preferably substituted by one or more deuterium, $R_1$ at this position is for example, but not limited to H, F, Cl, -OH, -$NH_2$, CN, -O-$CH_3$, -$CH_3$, ethynyl, -$CH_2F$, -$CHF_2$, -O-$CD_3$, -$CH_2OH$, -$CH_2$-$CH_3$, -O-$CH_2$-$CH_3$, -$CH_2$-O-$CH_3$, -$CD_2$-O-$CD_3$, -$CH_2$-O-$CD_3$, -$CH_2$-O-$CH_2CH_3$,

B and $R_1$ at the ortho position wherein nitrogen is attached to the rest of the molecule is selected from H or -$C_{1-6}$ alkyl, preferably H or -$C_{1-3}$ alkyl, such as H, -$CH_3$ or -$CH_2CH_3$;

As the specific embodiment of this preferred embodiment, B can be,

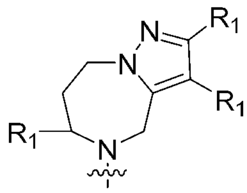

,

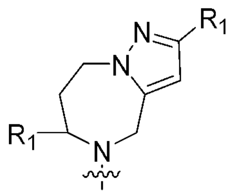

,

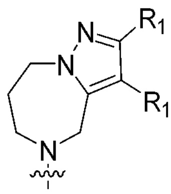

,

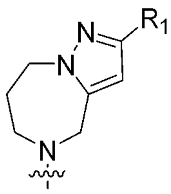

,

wherein each $R_1$ is as defined by this embodiment, and the conditions are that each is not H;

in some embodiments, B is a 9-11 membered fused bicyclic heterocycloalkyl connected by nitrogen, specifically

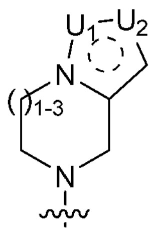

,

wherein

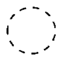

indicates that the ring can be saturated, partially unsaturated, and $U_1$ is selected from C or N, $U_2$ is selected from N, C, or O, for example, but not limited to,

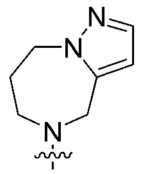, 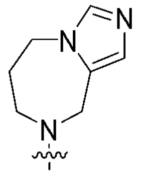, 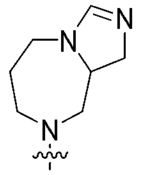, 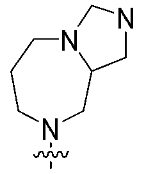, 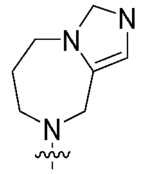, 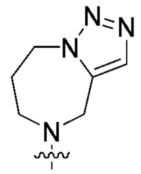, 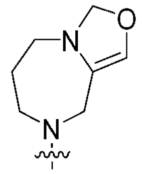,

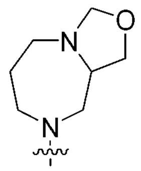, 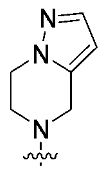,

substituted by 1 to 3, e.g., 1, 2, or 3 $R_1$, wherein each $R_1$ is independently selected from H, halogen, -CN, -OH, $-NH_2$, $-C_{1-6}$ alkyl, $-C_{2-6}$ alkenyl, $-C_{2-6}$ alkynyl, $-O-C_{1-6}$ alkyl, $-C_{1-6}$ alkyl-O-$C_{1-6}$ alkyl, -C(O)-$C_{1-6}$ alkyl, $-C(O)N(R_b)_2$, preferably selected from H, halogen, -CN, -OH, $-NH_2$, - $C_{1-3}$ alkyl, $-C_{2-4}$ alkenyl, $-C_{2-4}$ alkynyl, $-O-C_{1-3}$ alkyl, $-C_{1-3}$ alkyl-O-$C_{1-3}$ alkyl, -C(O)-$C_{1-3}$ alkyl, $-C_{1-3}$ alkyl-O-$C_{1-3}$ alkyl, $-C(O)N(R_b)_2$, wherein each $R_b$ is independently H, $-C_{1-6}$ alkyl optionally substituted by one or more halogen, deuterium, $-O-C_{1-6}$ alkyl (preferably $-C_{1-3}$ alkyl substituted by - $O-C_{1-3}$ alkyl) or $-C_{3-6}$ cycloalkyl; or two $R_b$, together with the N atom to which they are attached form a 4-7 membered heterocycloalkyl optionally substituted by halogen or $-C_{1-6}$ alkyl (preferably $-C_{1-3}$ alkyl);

preferably, B is

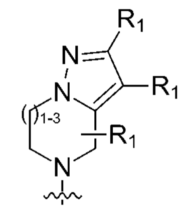,

for example

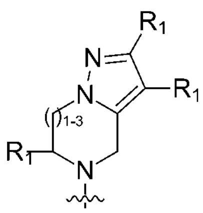,

wherein

$R_1$ at the ortho position of the nitrogen heteroatom on the five-membered ring is selected from - C(O)-$C_{1-6}$ alkyl and $-C(O)N(R_b)_2$, preferably $-C(O)N(R_b)_2$, each $R_b$ is independently H, $-C_{1-6}$ alkyl optionally substituted by one or more halogen, deuterium, $-O-C_{1-6}$ alkyl, or $-C_{3-6}$ cycloalkyl, preferably each is independently H or $-C_{1-3}$ alkyl optionally substituted by one or more deuterium, most preferably each is independently $-C_{1-3}$ alkyl optionally substituted by one or more deuterium, such as $-C(O)CH_3$, $-C(O)N(CH_3)(CH_2CH_2F)$, $-C(O)N(CH_3)_2$, $-C(O)N(CH_3)$(cyclopropyl), - C(O) $NH(CH_3)$, $-C(O)N(CD_3)_2$, $-C(O)N(CH_3)(CH_2CH_2OCH_3)$; or two $R_b$ together with the nitrogen atom to which they are attached form a 4-7 membered heterocycloalkyl optionally substituted by halogen or $-C_{1-6}$ alkyl, for example azetidinyl, 3-F-azetidinyl, morpholinyl, pyrrolidinyl, piperazinyl, 4-methylpiperazinyl,

$R_1$ at the meta position of the nitrogen heteroatom on the five-membered ring is selected from H, halogen, -CN, -OH, $-NH_2$, $-C_{1-6}$ alkyl, $-C_{2-6}$ alkenyl, $-C_{2-6}$ alkynyl, $-O-C_{1-6}$ alkyl, and $-C_{1-6}$ alkyl-O-$C_{1-6}$ alkyl, preferably selected from H, halogen, -CN, -OH, $-NH_2$, $-C_{1-3}$ alkyl, $-C_{2-4}$ alkenyl, $-C_{2-4}$ alkynyl, $-O-C_{1-3}$ alkyl, and $-C_{1-3}$ alkyl-O-$C_{1-3}$ alkyl, more preferably selected from $-C_{1-3}$ alkyl-O-$C_{1-3}$ alkyl, wherein the alkyl present is optionally substituted by one or more halogen, OH, or deuterium, preferably substituted by one or more deuterium, $R_1$ at this position is for example, but not

limited to H, F, Cl, -OH, $-NH_2$, CN, $-O-CH_3$, $-CH_3$, ethynyl, $-CH_2F$, $-CHF_2$, $-O-CD_3$, $-CH_2OH$, $-CH_2-CH_3$, $-O-CH_2-CH_3$, $-CH_2-O-CH_3$, $-CD_2-O-CD_3$, $-CH_2-O-CD_3$, $-CH_2-O-CH_2CH_3$,

B and $R_1$ at the ortho position wherein nitrogen is attached to the rest of the molecule is selected from H or $-C_{1-6}$ alkyl, preferably H or $-C_{1-3}$ alkyl, such as H, $-CH_3$ or $-CH_2CH_3$;

in a specific embodiment of this preferred embodiment, B may be

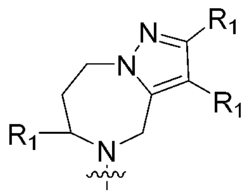
,

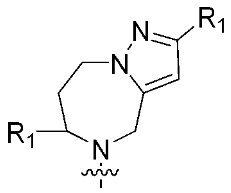
,
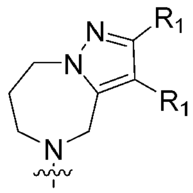
,
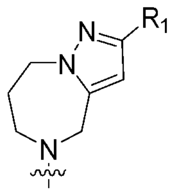
,

wherein $R_1$ is each defined in this embodiment, provided that each is not H.

**Embodiment II-12-3-3:** The compound of Formula (II) according to any one of Embodiments II-1to II-11, a stereoisomer, tautomer, stable isotope variant, pharmaceutically acceptable salt or solvate thereof, wherein specific examples of B include but are not limited to

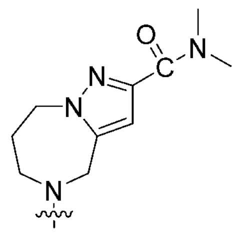
,

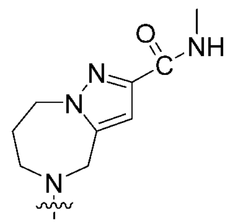
,
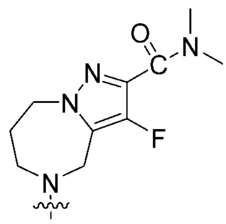
,
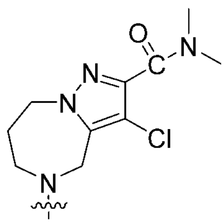
,
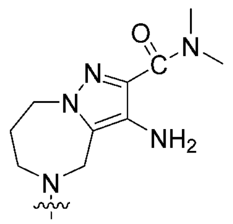
,
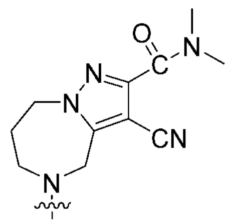
,

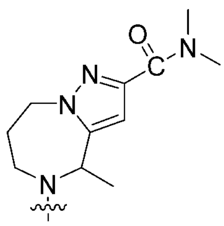
,
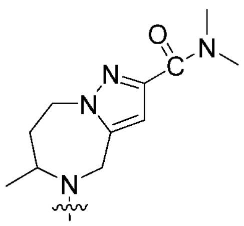
,
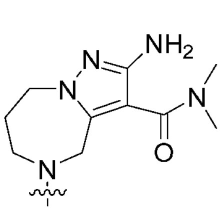
,
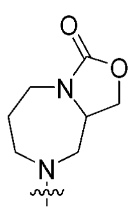
,
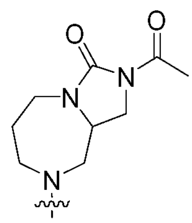
,
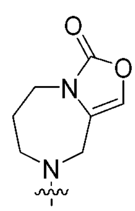
,

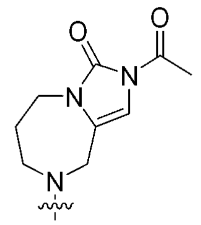 , 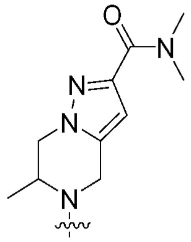 , 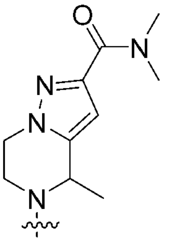 , 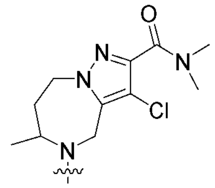 , 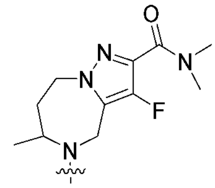 ,

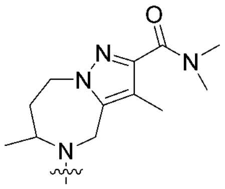 , 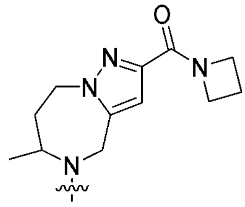 , 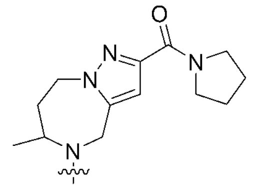 , 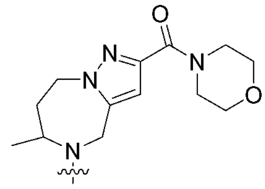 ,

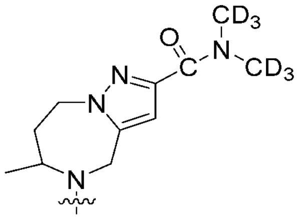 , 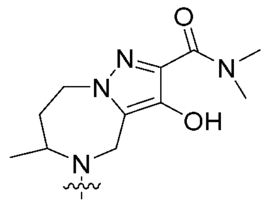 , 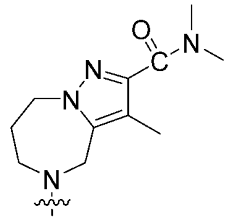 , 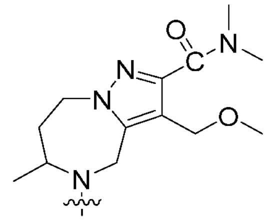 ,

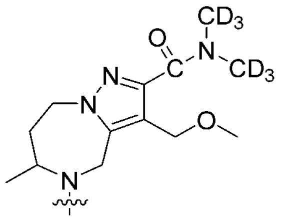 , 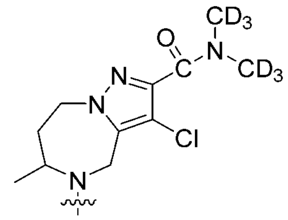 , 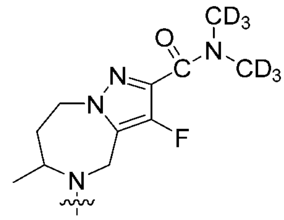 , 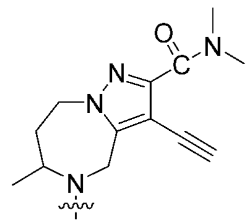 ,

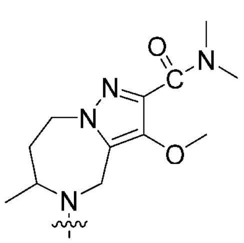 , 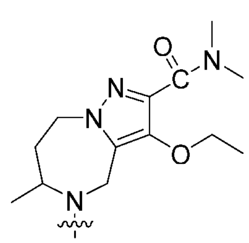 , 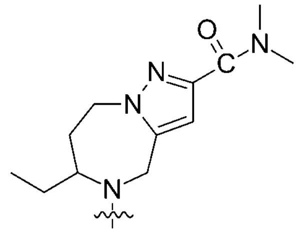 , 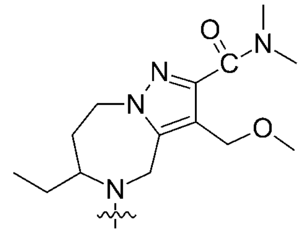 ,

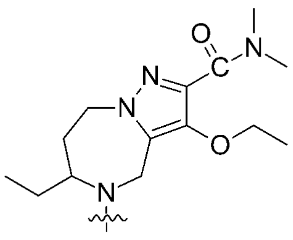 , 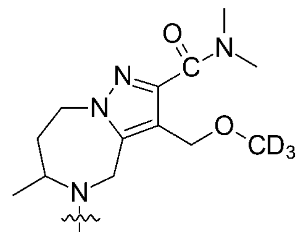 , 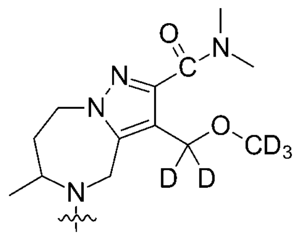 , 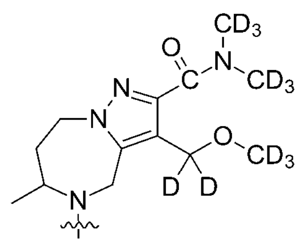 ,

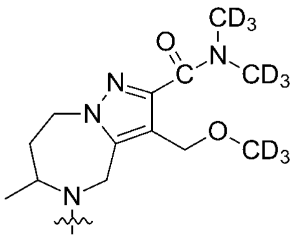 , 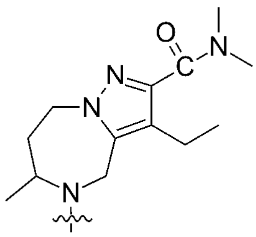 , 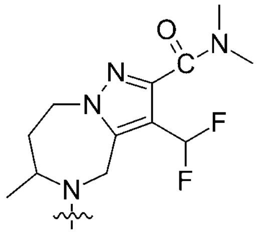 , 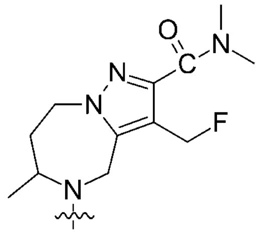 ,

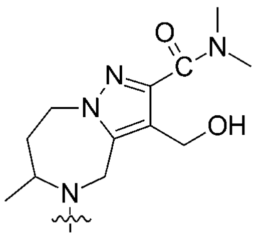 , 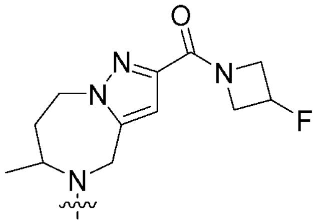 , 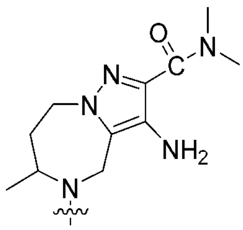 , 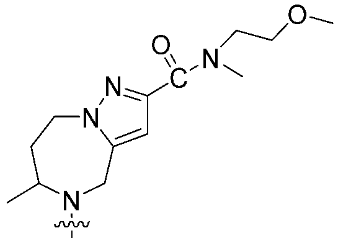 ,

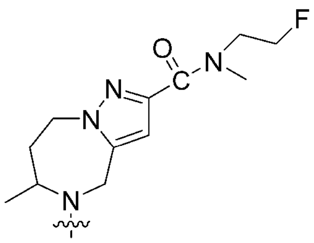 , 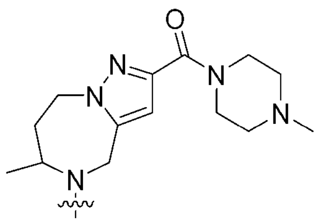 , 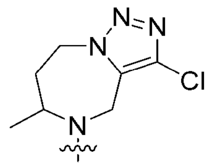 , 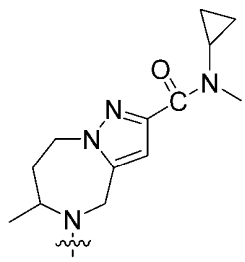 ,

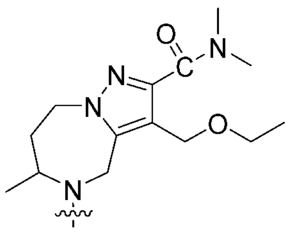 ;

in some embodiments, B is

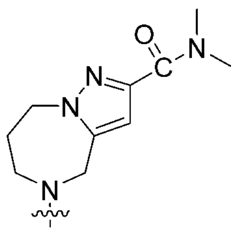 ;

in other embodiments, B is

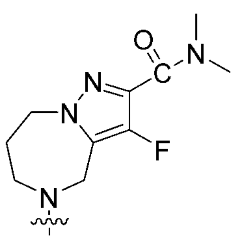

or

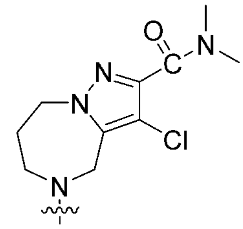

;

in other embodiments, B is

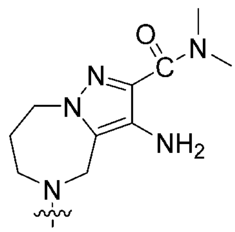

or

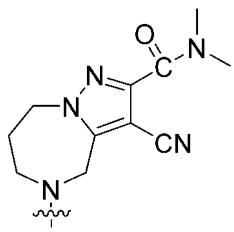

;

in other embodiments, B is

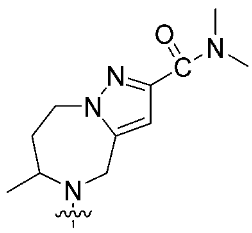

, or

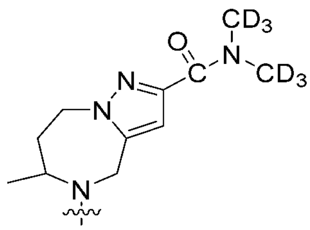

;

in other embodiments, B is

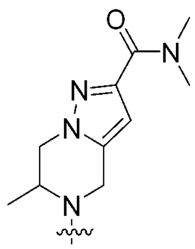

or

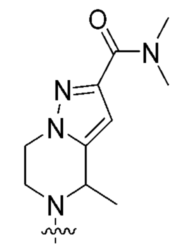

;

in other embodiments, B is

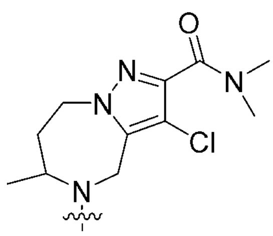

,

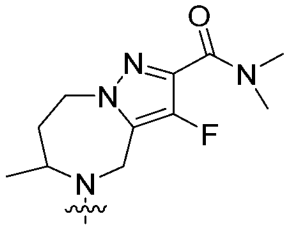 or 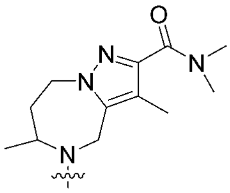 ;

in other embodiments, B is

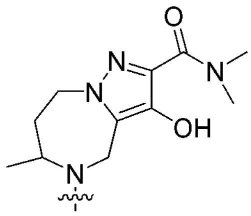 ,

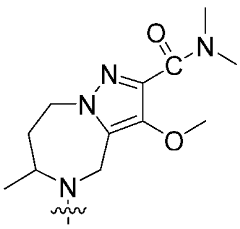 , 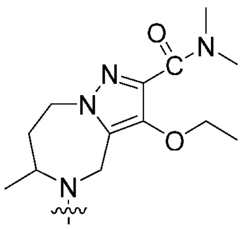 , 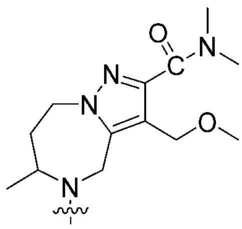 , 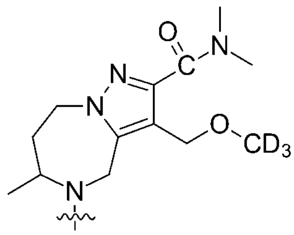 ,

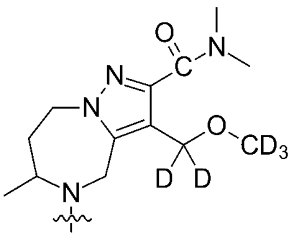 , 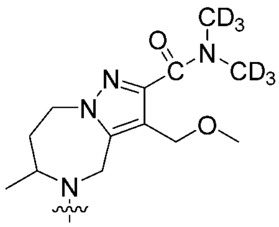 , 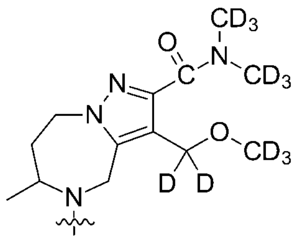 , 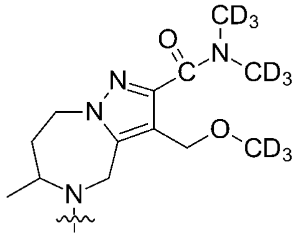 ;

in other embodiments, B is

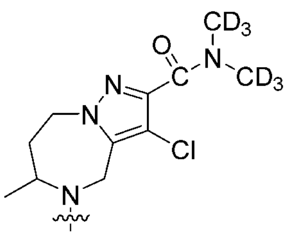 or 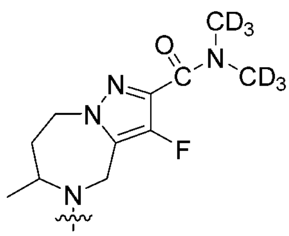 ;

in other embodiments, B is

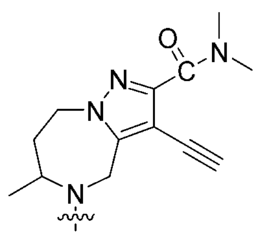

;

in other embodiments, B is

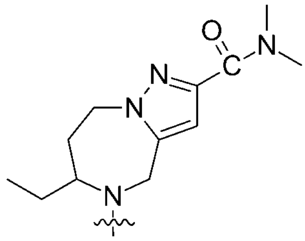

,

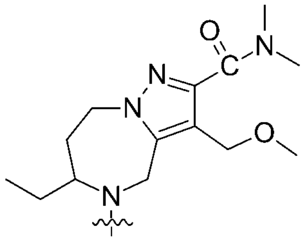

,

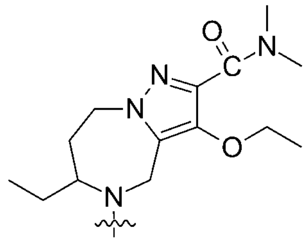

.

**Embodiment II-12-3-4:** The compound of Formula (II) according to any one of Embodiments II-12-3-1 to II-12-3-3, a stereoisomer, tautomer, stable isotope variant, pharmaceutically acceptable salt or solvate thereof, wherein in each embodiment of the above B, $R_1$ located at the ortho position of the ring nitrogen atom attached to the rest of the molecule can exist in stereoisomeric form, such as the R or S configuration, for example

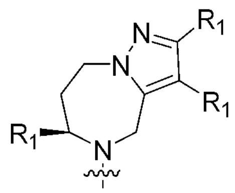

.

**Embodiment II-12-4:** The compound of Formula (II) according to any one of Embodiments II-1 to II-11, a stereoisomer, tautomer, stable isotope variant, pharmaceutically acceptable salt or solvate thereof, wherein B is selected from the structural fragments generally or specifically defined in Embodiments II-12-1, II-12-2, and 11-12-3; preferably, B is selected from

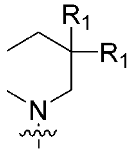

,

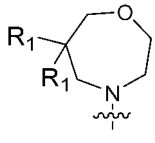

,

wherein each $R_1$ is independently selected from -OH and $-C_{1-6}$ alkyl, wherein the $-C_{1-6}$ alkyl is preferably $-C_{1-3}$ alkyl and is optionally substituted by halogen; preferably one of them is -OH and the other is $-C_{1-6}$ alkyl, preferably $-C_{1-3}$ alkyl, more preferably methyl, most preferably

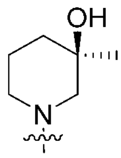

or

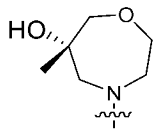

;

or B is selected from

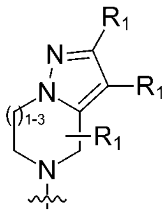

,

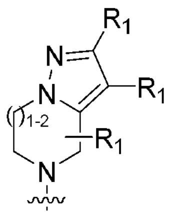

,

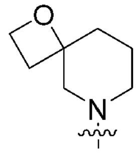

,

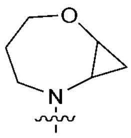

,

wherein one $R_1$ (preferably $R_1$ at the ortho position of the nitrogen heteroatom) is selected from -C(O)-$C_{1-6}$ alkyl and -C(O) $N(R_b)_2$, the remaining $R_1$ is selected from H, halogen, -CN, $NH_2$, -$C_{1-6}$ alkyl, wherein the - $C_{1-6}$ alkyl preferably -$C_{1-3}$ alkyl; for example, one $R_1$ (preferably $R_1$ at the ortho position of the nitrogen heteroatom) is selected from -CON($C_{1-6}$ alkyl)$_2$, -CONH$C_{1-6}$ alkyl, $CONH_2$, -COC$_{1-6}$ alkyl, preferably -CON($C_{1-3}$ alkyl)$_2$, -CONH$C_{1-3}$ alkyl, -COC$_{1-3}$ alkyl, more preferably -CON($CH_3$)$_2$, - $CONHCH_3$, $COCH_3$, and the remaining $R_1$ is selected from H, F, Cl, -CN, -$NH_2$, and -$C_{1-3}$ alkyl (e.g., methyl); or B is

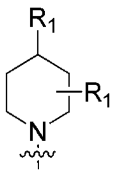

,

wherein one $R_1$ is selected from halogen, CN, halogen-substituted -$C_{1-6}$ alkyl, preferably located at the para position of the ring N atom, and the other is selected from H, - OH, optionally halogen-substituted -$C_{1-6}$ alkyl or -O-$C_{1-6}$ alkyl.

**Embodiment II-12-5:** The compound of Formula (II) according to any one of Embodiments II-1 to II-12-4, a stereoisomer, tautomer, stable isotope variant, pharmaceutically acceptable salt or solvate thereof, wherein the substituent $R_1$ on B is stereoisomeric as appropriate, e.g., the R or S configuration.

**Embodiment II-13:** The compound of Formula (II) according to any one of Embodiments II-1 to II-12-5, a stereoisomer, tautomer, stable isotope variant, pharmaceutically acceptable salt or solvate thereof, wherein Y is defined and/or exemplified as specifically defined in any one of Embodiments 7.1 to 7.4 of the first aspect of the present invention; preferably Y is O.

**Embodiment 11-14:** The compound of Formula (II) according to any one of Embodiments II-1 to 11-13, a stereoisomer, tautomer, stable isotope variant, pharmaceutically acceptable salt or solvate thereof, wherein $R_2$ is defined and/or exemplified in any one of the specific definitions and/or examples of the Embodiments 8.1 to 8.4 of the first aspect of the present invention; preferably $R_2$ is each independently selected from H and D; e.g., both are H, one is H and the other is D, or both are D.

**Embodiment 11-15:** The compound of Formula (II) according to any one of Embodiments II-1 to II-14, a stereoisomer, tautomer, stable isotope variant, pharmaceutically acceptable salt or solvate thereof, wherein $R_3$ is defined and/or exemplified as in any one of the Embodiments 9.1 to 9.4 of the first aspect of the present invention; preferably $R_3$ is -$C_{1-6}$ alkyl, preferably -$C_{1-3}$ alkyl, most preferably methyl, optionally substituted by one or more D; the stereochemical configuration of the structural fragment

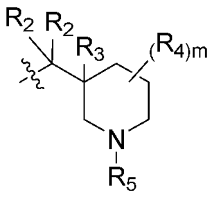

is preferred as shown in

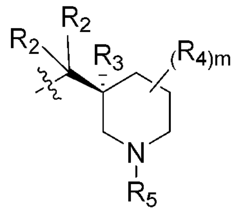

.

**Embodiment II-16:** The compound of Formula (II) according to any one of Embodiments II-1 to II-15, a stereoisomer, tautomer, stable isotope variant, pharmaceutically acceptable salt or solvate thereof, wherein $R_4$ is defined and/or exemplified as in any one of the specific definitions and/or examples from Embodiments 10.1 to 10.13 of the first aspect of the present invention; more preferably,

$R_4$ is selected from halogen and -$C_{1-6}$ alkyl, wherein the -$C_{1-6}$ alkyl preferably -$C_{1-3}$ alkyl, substituted by halogen (preferably F), e.g., -$CHF_2$; or two $R_4$ attached to the same carbon atom form =$C(R_c)_2$, wherein each $R_c$ is independently selected from H, halogen (preferably F), -$C_{1-6}$ alkyl optionally substituted by halogen (preferably -$C_{1-3}$ alkyl optionally substituted by halogen, preferably F), e.g., =$CH_2$, CHF, =$CHCF_3$;
preferably, $R_4$ substitution occurs at the para position of the ring N atom, i.e.,

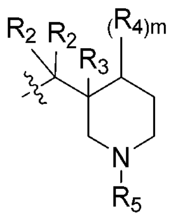

,

wherein m is 1 or 2;
wherein $R_4$ is stereoisomeric as appropriate, e.g., R or S configuration.

**Embodiment II-17:** The compound of Formula (II) according to any one of Embodiments II-1 to II-16, a stereoisomer, tautomer, stable isotope variant, pharmaceutically acceptable salt or solvate thereof, wherein $R_5$ is defined and/or exemplified as in any one of the specific definitions and/or examples of Embodiments 11.1 to 11.5 of the first aspect of the present invention; more preferably, $R_5$ is selected from -$C_{1-6}$ alkyl and -deuterated $C_{1-6}$ alkyl, preferably selected from -$C_{1-3}$ alkyl and - deuterated $C_{1-3}$ alkyl, such as, but not limited to -$CH_3$, -$CD_3$, and -$CH_2CH_3$.

**Embodiment II-18:** The compound of Formula (II) according to any one of Embodiments II-1 to II-12-5, a stereoisomer, tautomer, stable isotope variant, pharmaceutically acceptable salt or solvate thereof, wherein the structural fragment

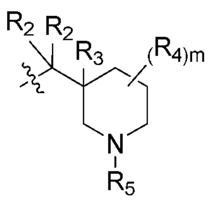

is specifically defined and/or exemplified as in Embodiments 12.1 or 12.2 of the first aspect of the present invention; more preferably,

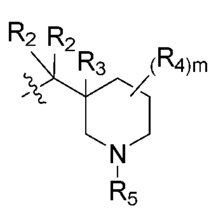 is 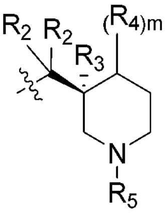 ,

$R_2$ is each independently selected from H and deuterium; $R_3$ is selected from $-C_{1-3}$ alkyl optionally substituted by one or more deuterium; m is 1 or 2; $R_4$ selected from halogen and $-C_{1-3}$ alkyl substituted by halogen (preferably F), or two $R_4$ attached to the same carbon atom form $=C(R_c)_2$, wherein $R_c$ is independently selected from H, halogen, $-C_{1-3}$ alkyl substituted by halogen, preferably, $R_4$ substitution occurs at the para position of the ring N atom, and specific examples of $R_4$ include, but are not limited to fluoro, difluoro, difluoromethyl, fluoromethyl, difluoromethyl, fluoromethylene, difluoromethylene, preferably fluoro, difluoromethyl, fluoromethylene; $R_5$ is selected from $-C_{1-3}$ alkyl optionally substituted by one or more deuterium, e.g., $-CH_3$, $-CH_2CH_3$, $-CD_3$;
Examples of preferred fragments are

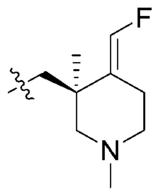 , 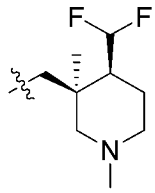 , 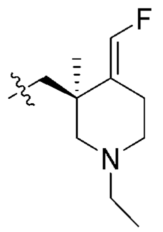 , 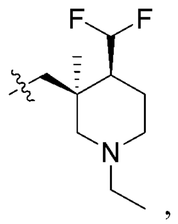 ,

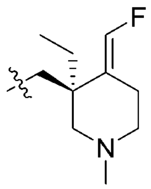 , 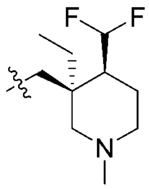 , 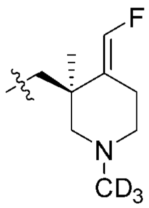 , 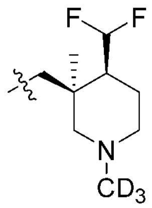 , 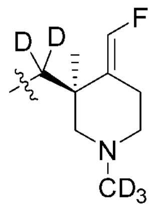 , 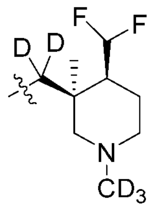 , 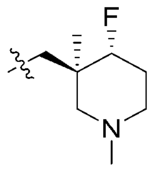 ,

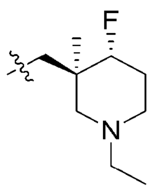 , 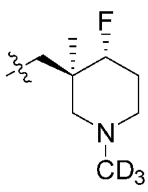 , 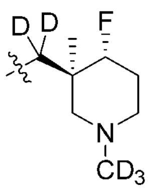 , 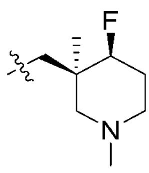 , 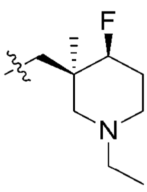 , 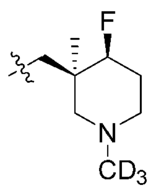 , 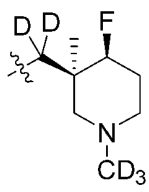 ,

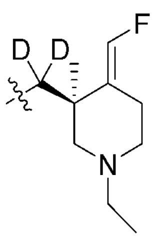 , 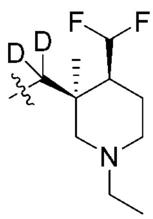 , 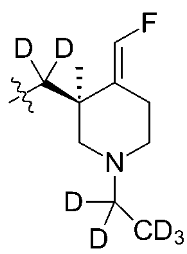 , 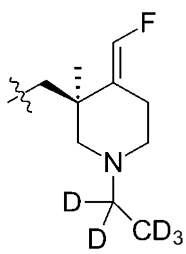 , 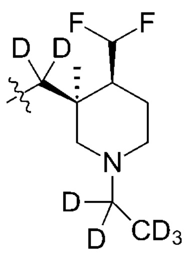 , 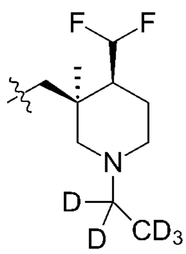 .

**Embodiment II-19:** The compound of Formula (II) according to any one of Embodiments II-1 or II-2, a stereoisomer, tautomer, stable isotope variant, pharmaceutically acceptable salt or solvate thereof having sub-general formulae as defined in any one of the Embodiments 14.1 to 14.3, of the first aspect of the present invention.

**Embodiment II-19-1:** The compound of Formula (II) according to any one of Embodiments II-1 or II-2, a stereoisomer, tautomer, stable isotope variant, pharmaceutically acceptable salt or solvate thereof, which can have the following sub-general formula:

| Sub-general formula | Example of sub-general formula |
|---|---|
| 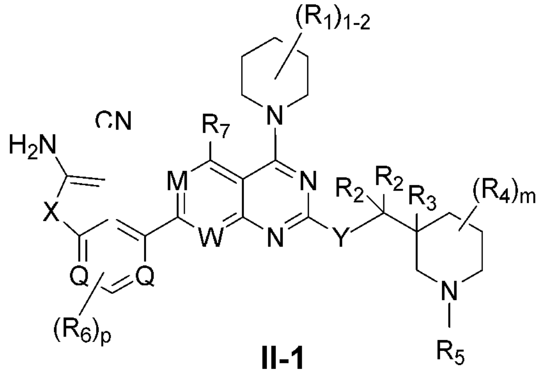 II-1 | 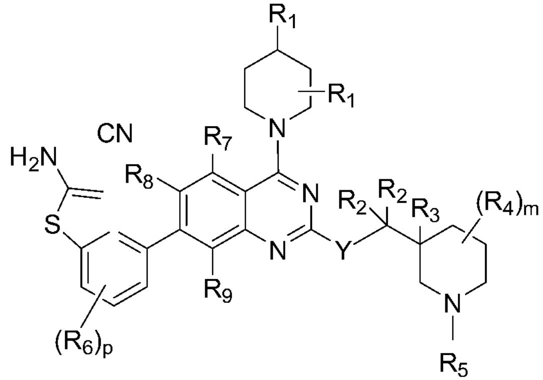 |
| 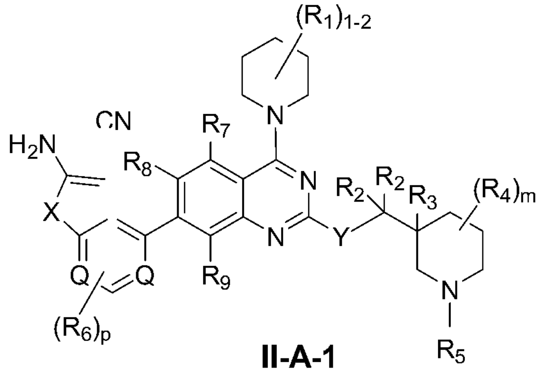 II-A-1 | 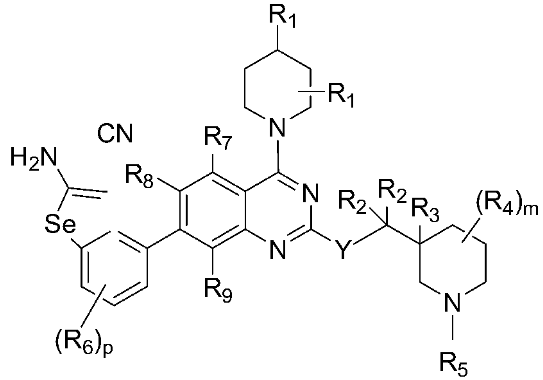 |
| 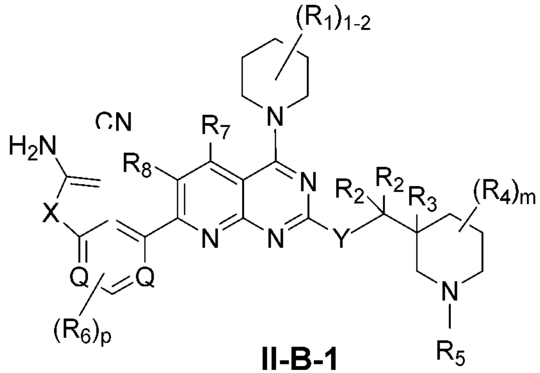 II-B-1 | 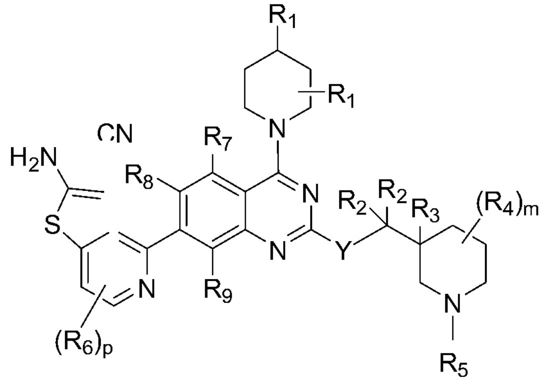 |

(continued)

| Sub-general formula | Example of sub-general formula |
|---|---|
| 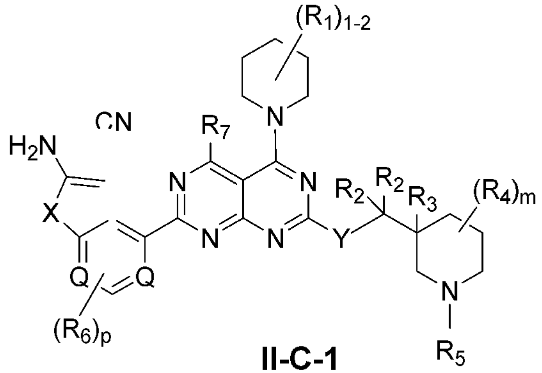 II-C-1 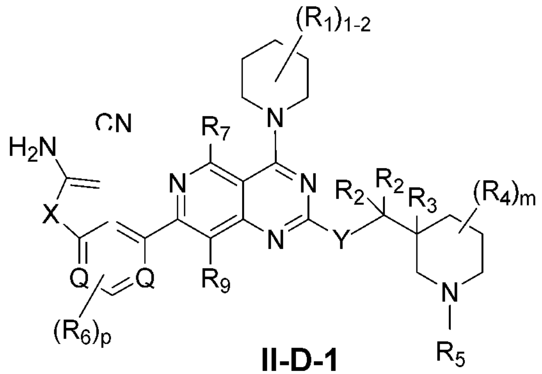 II-D-1 | 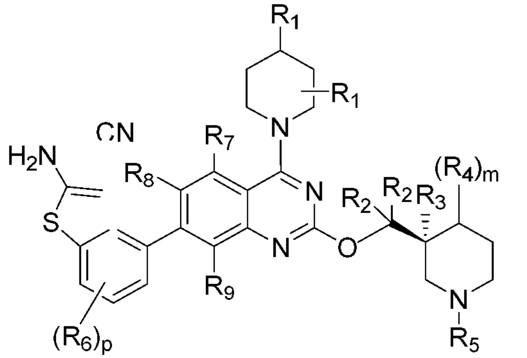 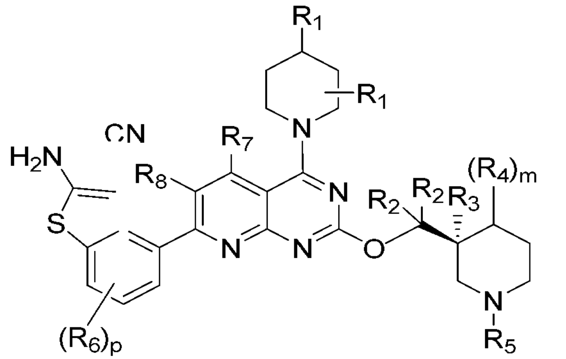 |
| 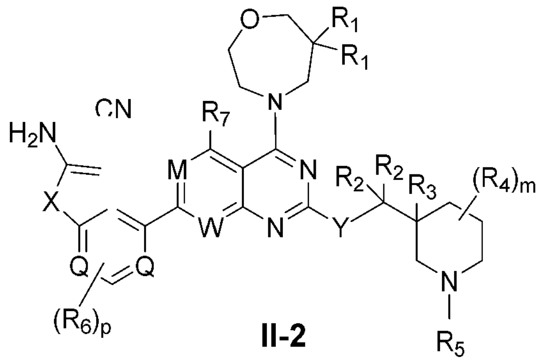 II-2 | 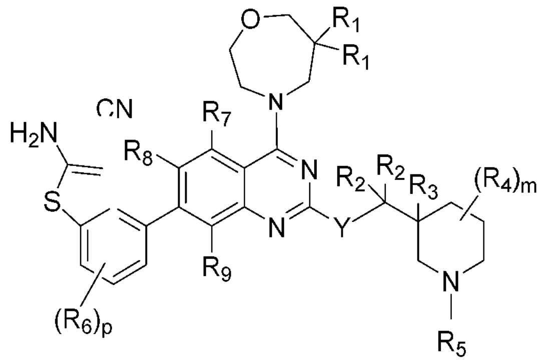 |

(continued)

| Sub-general formula | Example of sub-general formula |
|---|---|
| 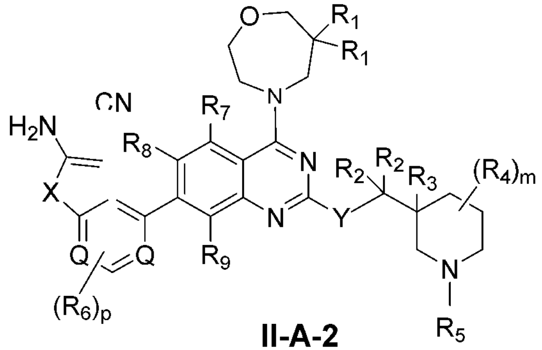 II-A-2 | 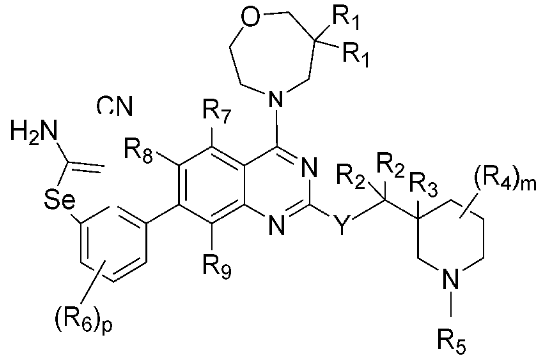 |
| 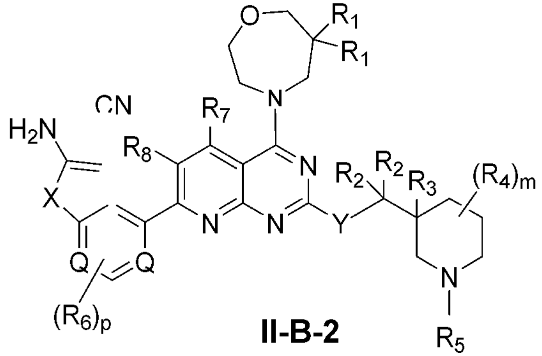 II-B-2 | 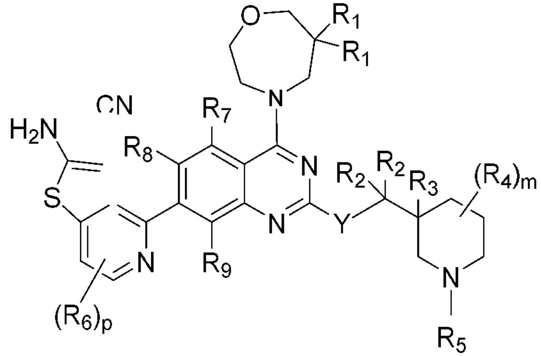 |
| 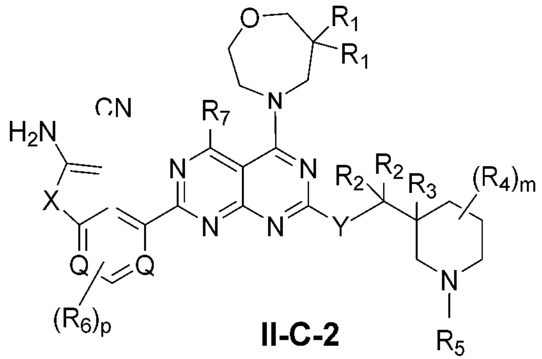 II-C-2 | 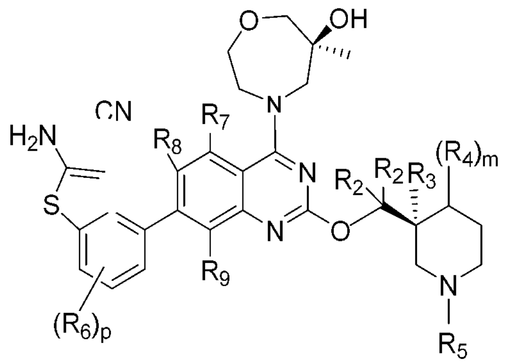 |

(continued)

| Sub-general formula | Example of sub-general formula |
|---|---|
| 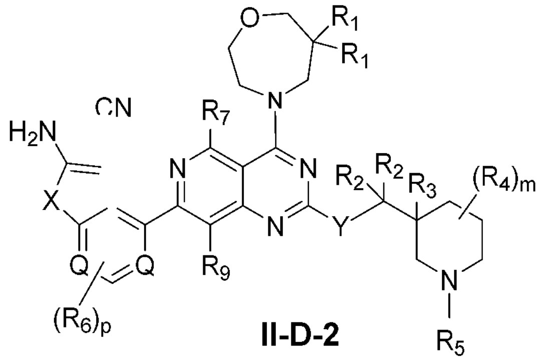 II-D-2 | 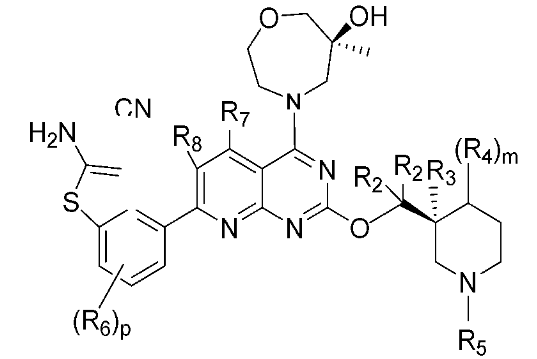 |
| 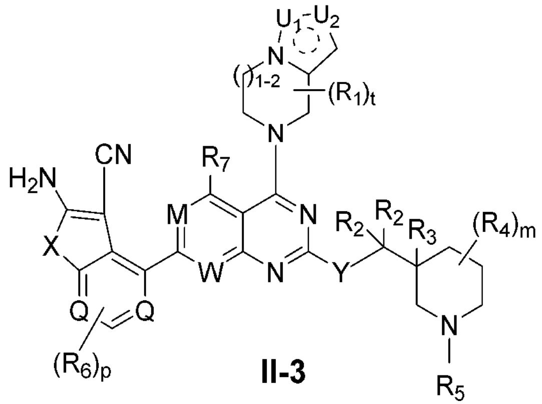 II-3 | 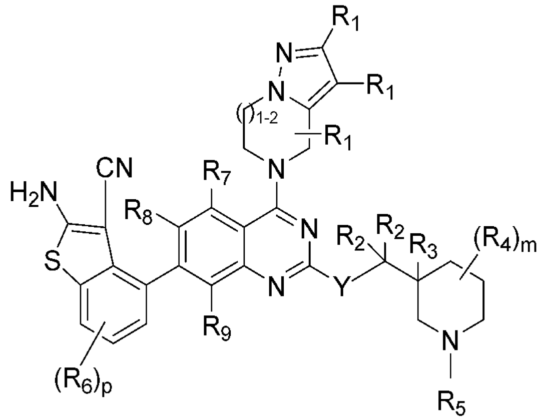 |

(continued)

| Sub-general formula | Example of sub-general formula |
|---|---|
| 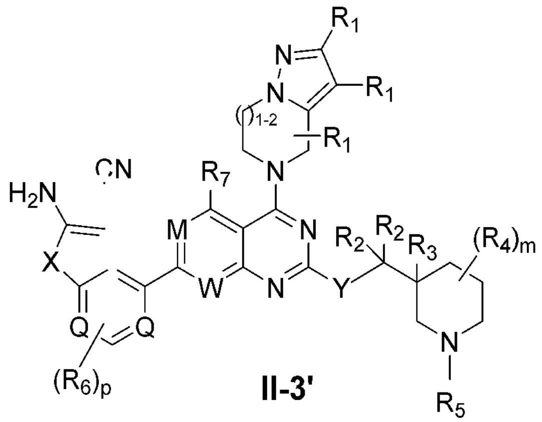 II-3' | 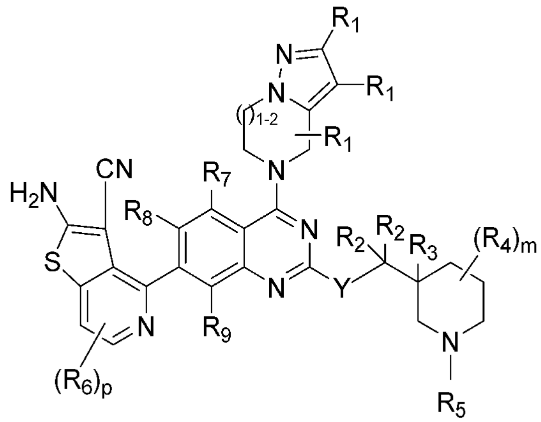 |
| 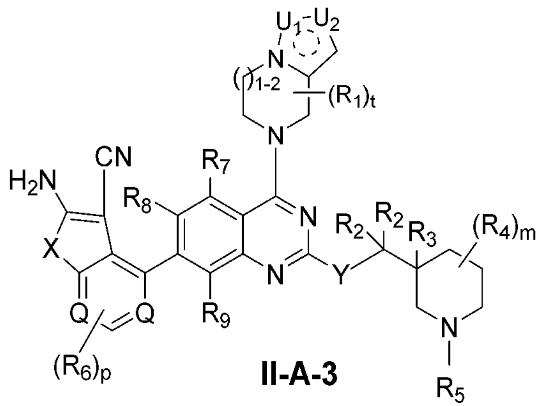 II-A-3 | 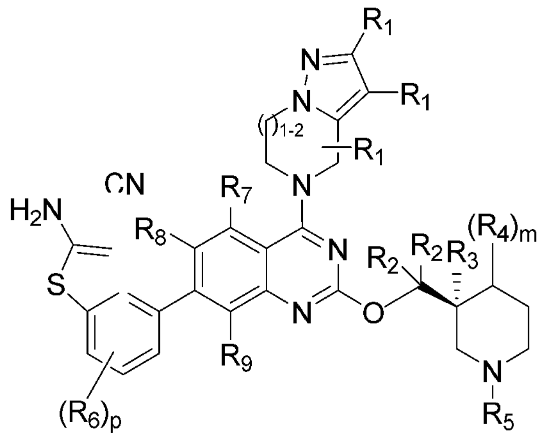 |

(continued)

| Sub-general formula | Example of sub-general formula |
|---|---|
| 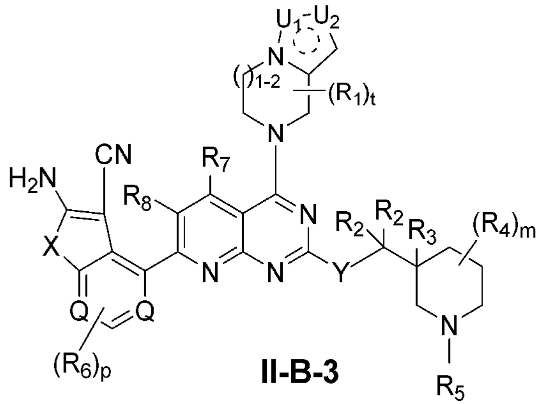 II-B-3 | 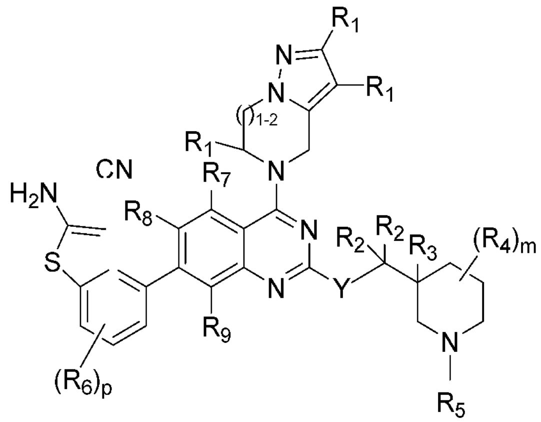 |
| 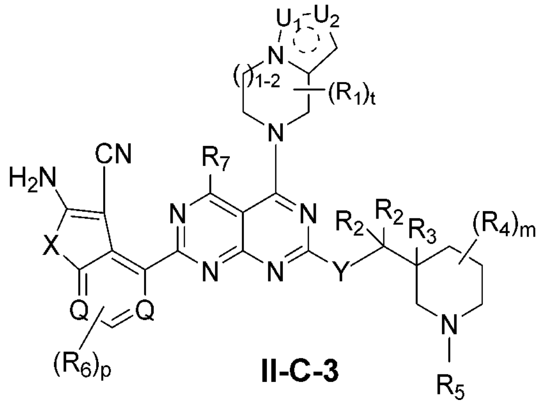 II-C-3 | 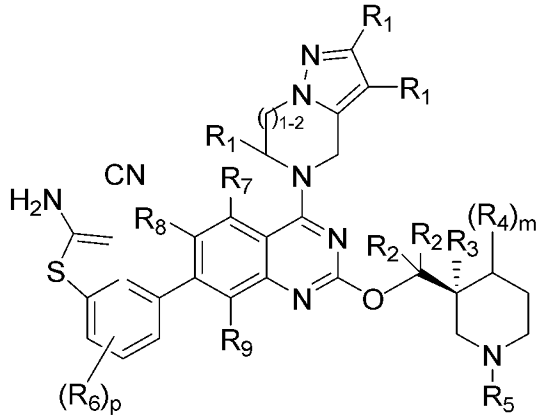 |

(continued)

| Sub-general formula | Example of sub-general formula |
|---|---|
| 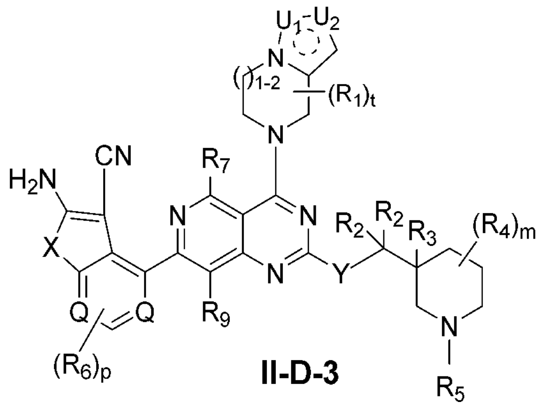 II-D-3 | 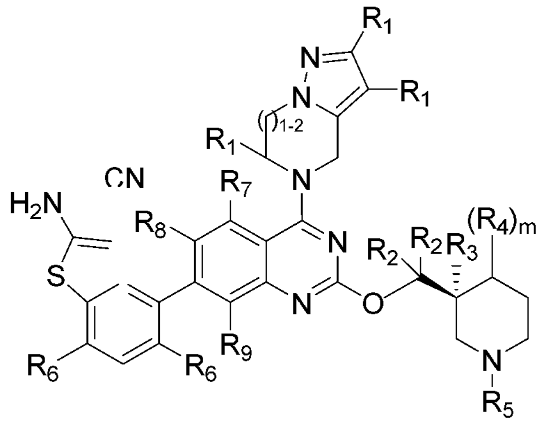 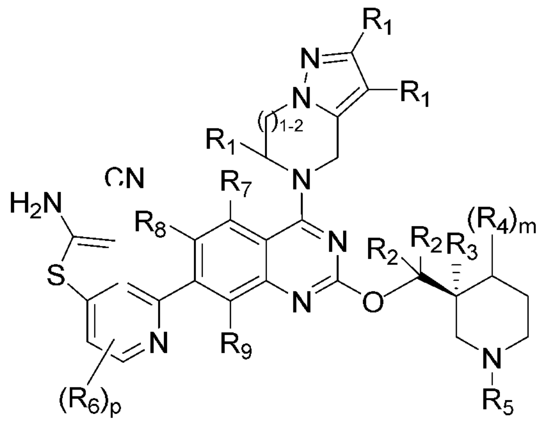 |

(continued)

| Sub-general formula | Example of sub-general formula |
|---|---|
| | 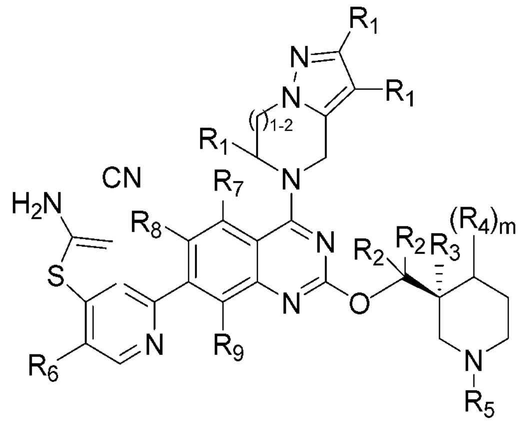<br>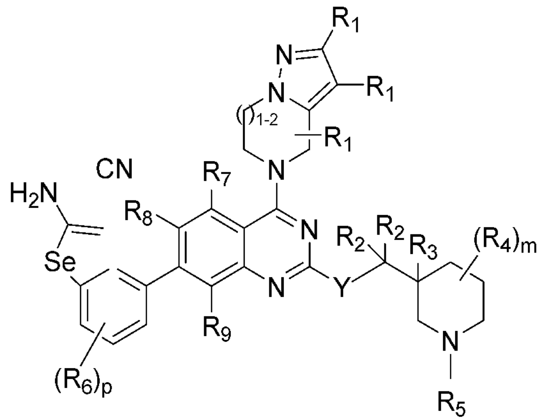 |

(continued)

| Sub-general formula | Example of sub-general formula |
| --- | --- |
| | 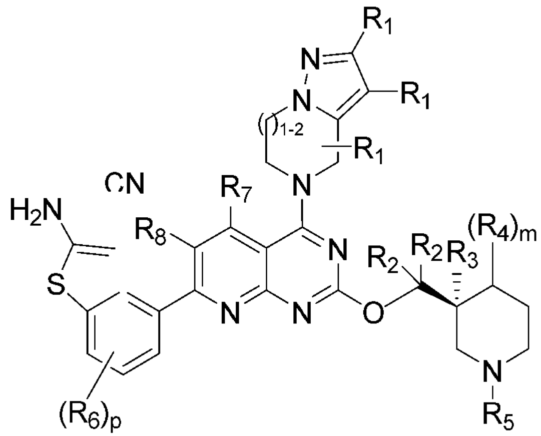 |
| and wherein the structural fragment 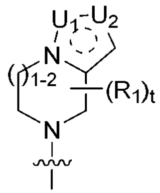 and its example fragment are correspondingly replaced by 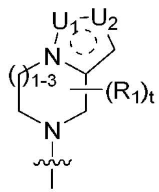 and each sub-general formula of the corresponding example fragment, e.g., | |

(continued)

| Sub-general formula | Example of sub-general formula |
|---|---|
| 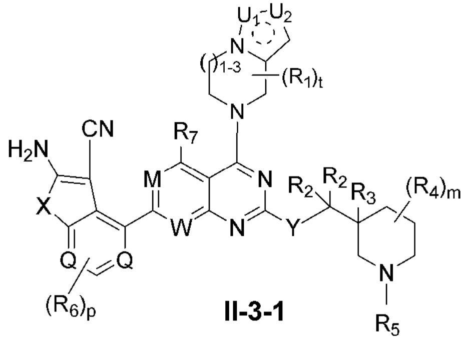 II-3-1 , | 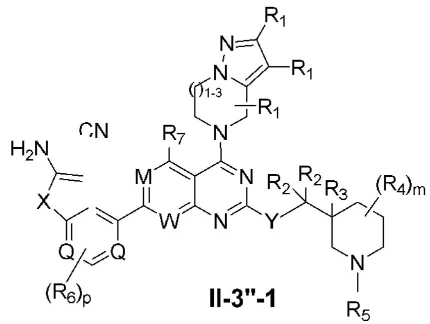 II-3"-1 , |
| 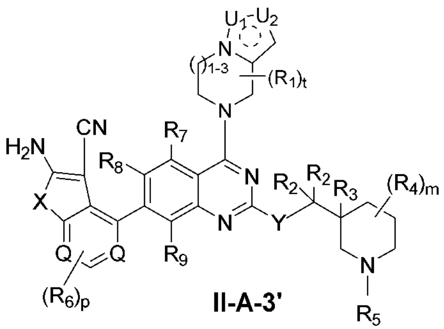 II-A-3' , specifically | 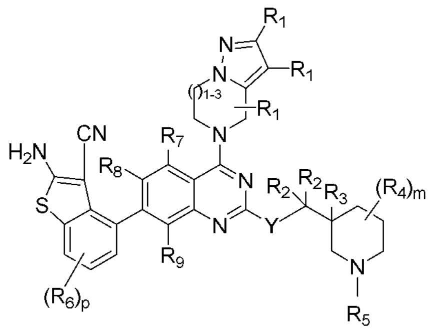 , |

(continued)

| Sub-general formula | Example of sub-general formula |
| --- | --- |
| 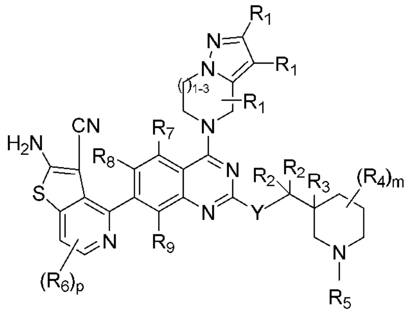, 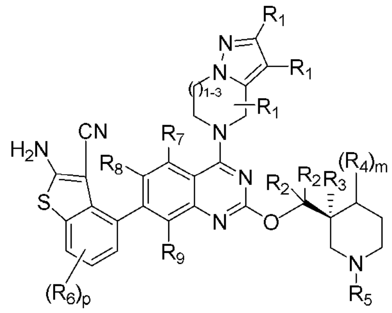, 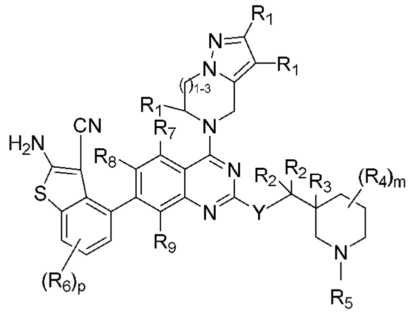, 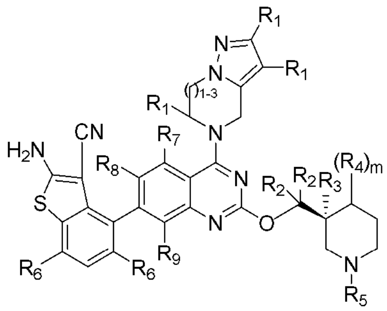, 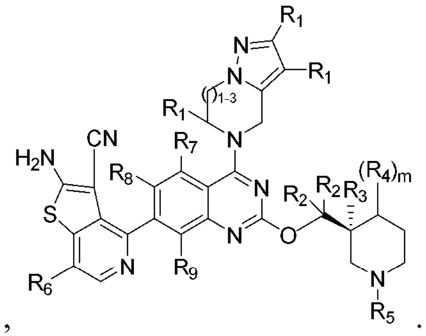. | |

wherein each group or structural fragment has the general, specific or preferred definitions given in the corresponding Embodiments II-1 to 11-18 of the second aspect of the present invention as described above;
in the above described sub-general formulae and examples of some of the specific embodiments,

X is selected from S and Se;
each Q is independently selected from C-$R_6$ and N;
$R_6$ is independently selected from H and halogen;
the fused ring fragment containing X is preferably

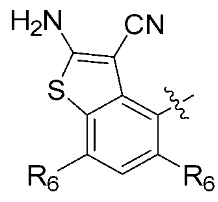
,

wherein each $R_6$ is independently selected from H and halogen, wherein halogen is preferably F;
M is selected from N and C-$R_8$;
W is selected from N and C-$R_9$;
$R_7$ is H;
$R_8$ is $C_{1-3}$ alkyl optionally substituted by halogen (preferably F), $C_{2-4}$ alkenyl optionally substituted by halogen (preferably F), CN, $NO_2$, halogen (preferably Cl); or $R_8$ is -$C_{1-6}$ alkyl-O-$C_{1-6}$ alkyl, preferably -$C_{1-3}$ alkyl-O-$C_{1-3}$ alkyl, for example -$CH_2$-O-$CH_3$;
$R_9$ is selected from F, Cl, and -O-optionally halogen-substituted $C_{1-3}$ alkyl;
for Formula II-1and each of its sub-general formulas, one of $R_1$ is selected from halogen, CN, halogen-substituted -$C_{1-6}$ alkyl preferably located at the para position of the ring N atom, and the other is selected from H, -OH, optionally halogen-substituted -$C_{1-6}$ alkyl or -O-$C_{1-6}$ alkyl;
for Formula II-2 and each of its sub-general formulas, one of $R_1$ is -OH and the other is -$C_{1-3}$ alkyl; for Formula II-3 and each of its sub-general formulas, $R_1$ at the ortho position of the nitrogen heteroatom on the five-membered ring is selected from -C(O)-$C_{1-6}$ alkyl, -$CONH_2$, -CONH$C_{1-6}$ alkyl and -CON($C_{1-6}$ alkyl)$_2$, or is 4-7 membered monocyclic heterocycloalkyl attached to -C(O)-nitrogen atom and containing 1 or 2 heteroatoms selected from N and O, and the remaining $R_1$ is independently selected from H, $NH_2$, CN, halogen, and -$C_{1-6}$ alkyl;
Y is O;
$R_2$ is each independently selected from H and D;
$R_3$ is -$C_{1-3}$ alkyl;
$R_4$ is selected from halogen, -O-$C_{1-3}$ alkyl, and -$C_{1-3}$ alkyl, wherein the $C_{1-3}$ alkyl present is each independently substituted by halogen, or
two $R_4$ attached to the same carbon atom form =C($R_c$)$_2$, wherein each $R_c$ is independently selected from H, halogen, and the halogen-substituted -$C_{1-3}$ alkyl, or
two $R_4$ attached to the same ring carbon atom together with the ring carbon atom to which they are attached form a spiro $C_{3-4}$ cycloalkyl, and the spiro $C_{3-4}$ cycloalkyl is optionally substituted by halogen and optionally halogen-substituted -$C_{1-3}$ alkyl;
$R_5$ is selected from -$C_{1-3}$ alkyl and -deuterated $C_{1-3}$ alkyl;
m is an integer of 1 to 2 and p is an integer of 0 to 2;
preferably, $R_4$ is located at the para position of the ring nitrogen atom of the attached ring; preferably, the structural fragment

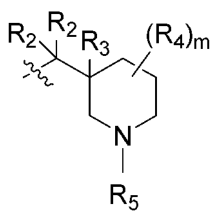
is
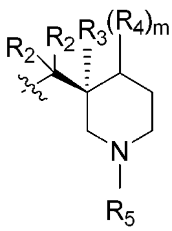
.

**Embodiment II-19-2:** The compound of Formula (II) according to Embodiment II-19-1, a stereoisomer, tautomer, stable isotope variant, pharmaceutically acceptable salt or solvate thereof, wherein for formula II-3 and each of its

subgeneral formulae, the fragment B is

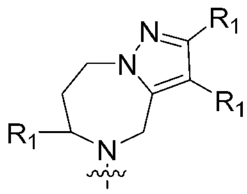

,

wherein $R_1$ at the ortho position of the nitrogen heteroatom on the five-membered ring is selected from -C(O)-$C_{1-6}$ alkyl and -C(O)N($R_b$)$_2$, preferably -C(O)N($R_b$)$_2$, each $R_b$ is independently H or -$C_{1-6}$ alkyl optionally substituted by one or more deuterium, preferably each is independently H or -$C_{1-3}$ alkyl optionally substituted by one or more deuterium, e.g., -C(O)N($CH_3$)$_2$, -C(O)N($CD_3$)$_2$;

$R_1$ at the meta position of the nitrogen heteroatom on the five-membered ring is selected from H, halogen, -CN, -OH, -$NH_2$, -$C_{1-6}$ alkyl, -$C_{2-6}$ alkenyl, -$C_{2-6}$ alkynyl, -O-$C_{1-6}$ alkyl, and -$C_{1-6}$ alkyl-O-$C_{1-6}$ alkyl, preferably selected from H, halogen, -CN, -OH, -$NH_2$, -$C_{1-3}$ alkyl, -$C_{2-4}$ alkenyl, -$C_{2-4}$ alkynyl, -O-$C_{1-3}$ alkyl, and -$C_{1-3}$ alkyl-O-$C_{1-3}$ alkyl, wherein the alkyl present is optionally substituted by one or more halogen or deuterium, $R_1$ at this position is for example, but not limited to H, F, Cl, CN, -OH, -$NH_2$, -$CH_3$, -C≡CH, -O-$CH_3$, -O-$CD_3$, -O-$CH_2$-$CH_3$, -$CH_2$-O-$CH_3$, -$CD_2$-O-$CD_3$.

B and $R_1$ at the ortho position wherein nitrogen is attached to the rest of the molecule is selected from H or -$C_{1-6}$ alkyl, preferably H or -$C_{1-3}$ alkyl, such as H or -$CH_3$;

for example, B is selected from

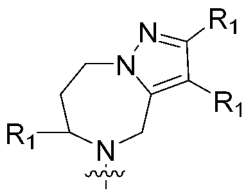

,

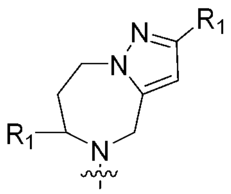

,

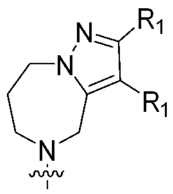

,

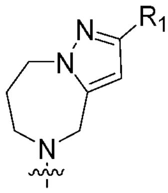

,

wherein each $R_1$ is defined as above, but each is not H;

The remaining groups in Formula II-3 and each of its sub-general formulae are generally or specifically defined as in Embodiment II-19-1.

**Embodiment II-19-3:** The compound of Formula (II) according to Embodiment II-19-1, a stereoisomer, tautomer, stable isotope variant, pharmaceutically acceptable salt or solvate thereof, wherein for Formula II-3 and each of its sub-general formulae (including examples of sub-general formulae),

The fragment B is

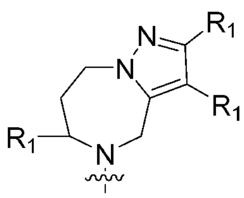

,

wherein

$R_1$ at the ortho position of the nitrogen heteroatom on the five-membered ring is selected from -C(O)-$C_{1-6}$ alkyl and

$-C(O)N(R_b)_2$, preferably $-C(O)N(R_b)_2$, each $R_b$ is independently H, $-C_{1-6}$ alkyl optionally substituted by one or more halogen, deuterium, $-O-C_{1-6}$ alkyl, or $-C_{3-6}$ cycloalkyl, preferably each is independently H or $-C_{1-3}$ alkyl optionally substituted by one or more deuterium, most preferably each is independently $-C_{1-3}$ alkyl optionally substituted by one or more deuterium, such as $-C(O)CH_3$, $-C(O)N(CH_3)(CH_2CH_2F)$, $-C(O)N(CH_3)_2$, $-C(O)N(CH_3)$(cyclopropyl), - C(O) $NH(CH_3)$, $-C(O)N(CD_3)_2$, $-C(O)N(CH_3)(CH_2CH_2OCH_3)$; or two $R_b$ together with the nitrogen atom to which they are attached form a 4-7 membered heterocycloalkyl optionally substituted by halogen or $-C_{1-6}$ alkyl, for example azetidinyl, 3-F-azetidinyl, morpholinyl, pyrrolidinyl, piperazinyl, 4-methylpiperazinyl,

$R_1$ at the meta position of the nitrogen heteroatom on the five-membered ring is selected from H, halogen, -CN, -OH, $-NH_2$, $-C_{1-6}$ alkyl, $-C_{2-6}$ alkenyl, $-C_{2-6}$ alkynyl, $-O-C_{1-6}$ alkyl, and $-C_{1-6}$ alkyl-O-$C_{1-6}$ alkyl, preferably selected from H, halogen, -CN, -OH, $-NH_2$, $-C_{1-3}$ alkyl, $-C_{2-4}$ alkenyl, $-C_{2-4}$ alkynyl, $-O-C_{1-3}$ alkyl, and $-C_{1-3}$ alkyl-O-$C_{1-3}$ alkyl, more preferably selected from $-C_{1-3}$ alkyl-O-$C_{1-3}$ alkyl, wherein the alkyl present is optionally substituted by one or more halogen, OH or deuterium, preferably substituted by one or more deuterium, $R_1$ at this position is for example, but not limited to H, F, Cl, -OH, $-NH_2$, -CN, $-O-CH_3$, $-CH_3$, ethynyl, $-CH_2F$, $-CHF_2$, $-O-CD_3$, $-CH_2OH$, $-CH_2-CH_3$, $-O-CH_2-CH_3$, $-CH_2-O-CH_3$, $-CD_2-O-CD_3$, $-CH_2-O-CD_3$, $-CH_2-O-CH_2CH_3$,

B and $R_1$ at the ortho position wherein nitrogen is attached to the rest of the molecule is selected from H or $-C_{1-6}$ alkyl, preferably H or $-C_{1-3}$ alkyl, such as H, $-CH_3$, or $-CH_2CH_3$; specifically, B can be

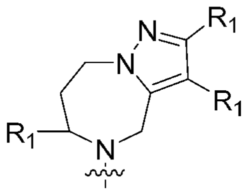 , 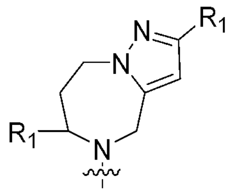 , 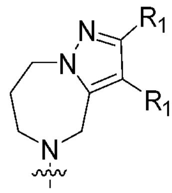 , 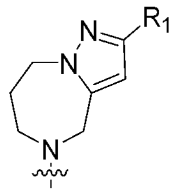 ,

wherein each $R_1$ is defined as above, but each is not H;

the fused ring fragment containing X is

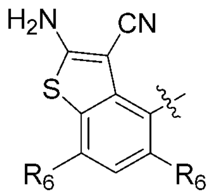

wherein $R_6$ is independently selected from

H and halogen (preferably F); preferably

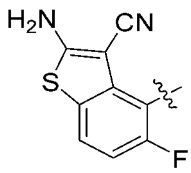

M is C-$R_8$;

W is C-$R_9$;

$R_7$ is H;

$R_8$ is halogen (preferably Cl), $C_{1-3}$ alkyl (e.g. $CF_3$) optionally substituted by halogen (preferably F), $C_{2-4}$ alkenyl optionally substituted by halogen (preferably F), CN, $NO_2$, $-C_{1-6}$ alkyl-O-$C_{1-6}$ alkyl (preferably $-C_{1-3}$ alkyl-O-$C_{1-3}$ alkyl, e.g., $-CH_2-O-CH_3$); preferably halogen or $C_{1-3}$ alkyl substituted by halogen, e.g., Cl and $CF_3$;

$R_9$ is selected from halogen, -$C_{1-3}$ alkyl, -OH, and -O-optionally halogen-substituted $C_{1-3}$ alkyl; preferably halogen and -$C_{1-3}$ alkyl; e.g., F, Cl, $CH_3$, -O-$CH_3$;

Y is O;

each $R_2$ is independently selected from H and D;

$R_3$ is -$C_{1-3}$ alkyl;

$R_4$ is -$C_{1-3}$ alkyl, which is substituted by halogen, or two $R_4$ attached to the same carbon atom form =$C(R_c)_2$, wherein each $R_c$ is independently selected from H and halogen;

$R_5$ is selected from -$C_{1-3}$ alkyl and -deuterated $C_{1-3}$ alkyl;

m is an integer from 1 to 2;

preferably, $R_4$ is located at the para position of the ring nitrogen atom of the attached ring; and

preferably, the structural fragment

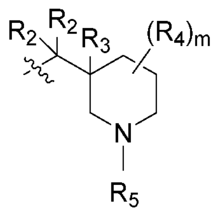

is

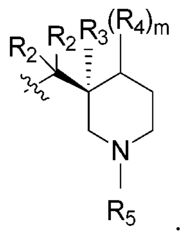

**Embodiment II-20:** The compound of Formula (II) according to Embodiment II, a stereoisomer, tautomer, stable isotope variant, pharmaceutically acceptable salt or solvate thereof, wherein said compound is the compound specifically synthesized and characterized in the examples.

In the third aspect, the present invention provides the following compound embodiments:

**Embodiment III-1:** The compound of Formula (III), a stereoisomer, tautomer, stable isotope variant, pharmaceutically acceptable salt or solvate thereof,

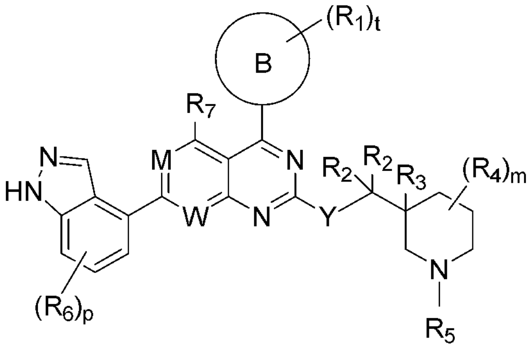

(III)

wherein each of or combinations of B, Y, $R_1$, $R_2$, $R_3$, $R_4$, $R_5$, $R_6$, $R_7$, M, W, t, m, p has the general or specific defined meaning in Embodiments II-1 to II-20 for the compound of formula (II) of the second aspect of the present invention.

**Embodiment III-2:** The compound of Formula (III) according to Embodiment III-1, a stereoisomer, tautomer, stable isotope variant, pharmaceutically acceptable salt or solvate thereof, wherein p is 1, or p is 2, or p is 3, preferably p is selected from an integer of 1 to 2.

**Embodiment III-3:** The compmound of Formula (III) according to Embodiment III-1 or III-2, a stereoisomer, tautomer, stable isotope variant, pharmaceutically acceptable salt or solvate thereof, wherein in the structural fragment

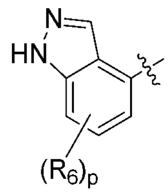

,

p is 1 or 2, for example

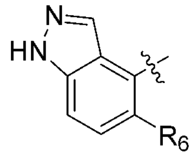

or

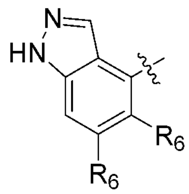

,

each $R_6$ is independently selected from H, D, halogen, CN, -$C_{1-6}$ alkyl, -$C_{2-6}$ alkenyl, -$C_{2-6}$ alkynyl, -O-$C_{1-6}$ alkyl, -S-$C_{1-6}$ alkyl, and -Se-$C_{1-6}$ alkyl, wherein the -$C_{1-6}$ alkyl, -$C_{2-6}$ alkenyl, and -$C_{2-6}$ alkynyl present are each independently and optionally substituted by halogen, -O-$C_{1-6}$ alkyl, or D; preferably selected from H, D, halogen, CN, -$C_{1-6}$ alkyl, -$C_{2-6}$ alkenyl, -$C_{2-6}$ alkynyl, wherein the -$C_{1-6}$ alkyl, -$C_{2-6}$ alkenyl and -$C_{2-6}$ alkynyl are each independently and optionally substituted by halogen;

in a specific Embodiment, $R_6$ in this fragment is each independently selected from halogen, -CN and -$C_{1-6}$ alkyl optionally substituted by halogen; preferably $R_6$ at the ortho position of this fragment attached to the rest of the molecule is -$C_{1-6}$ alkyl (preferably -$C_{1-3}$ alkyl), and $R_6$ at the meta position, if present, is selected from halogen and CN (preferably F or Cl); examples include, but not limited to

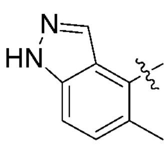, 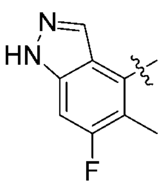, 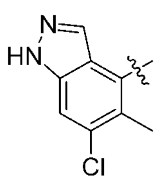, 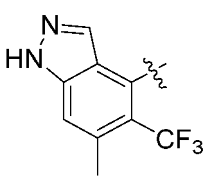, 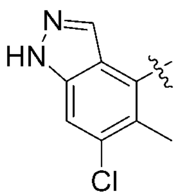, 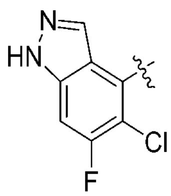.

**Embodiment III-4:** The compound of Formula (III) according to any one of Embodiments III-1 to III-3, a stereoisomer, tautomer, stable isotope variant, pharmaceutically acceptable salt or solvate thereof, wherein M is generally or specifically defined as in any one of the Embodiments II-8 to II-8-2.

**Embodiment III-5:** The compound of Formula (III) according to any one of Embodiments III-1 to III-4, a stereoisomer, tautomer, stable isotope variant, pharmaceutically acceptable salt or solvate thereof, wherein W is generally or specifically defined as in Embodiment II-9.

**Embodiment III-6:** The compound of Formula (III) according to any one of Embodiments III-1 to III-5, a stereoisomer,

tautomer, stable isotope variant, pharmaceutically acceptable salt or solvate thereof, wherein $R_7$ is generally or specifically defined as in any one of the Embodiments II-10 to II-10-2.

**Embodiment III-7:** The compound of Formula (III) according to any one of Embodiments III-1 to III-6, a stereoisomer, tautomer, stable isotope variant, pharmaceutically acceptable salt or solvate thereof, wherein the fused parent nuclei containing M and W is generally or specifically defined as in Embodiment II-11.

**Embodiment III-8:** The compound of Formula (III) according to any one of Embodiments III-1 to III-7, a stereoisomer, tautomer, stable isotope variant, pharmaceutically acceptable salt or solvate thereof, wherein in the structural fragment

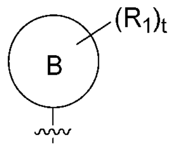

,

t is selected from an integer 0 to 6, an integer 0 to 4, an integer 0 to 3, e.g., t is 0, or t is 1, or t is 2, or t is 3;

in a specific embodiment, the structural fragment

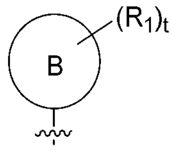

is generally or specifically defined as in any one of the Embodiments 11-12 to II-12-5;
preferably, this structural fragment is B, selected from

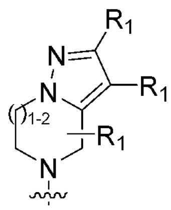

,

e.g.,

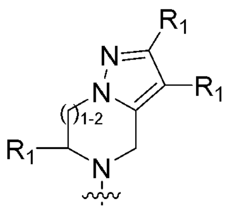

,

wherein $R_1$ is selected from H, halogen, -CN, $-NH_2$, $-C_{1-6}$ alkyl, $-C(O)-C_{1-6}$ alkyl, and $-C(O)N(R_b)_2$, wherein the $-C_{1-6}$ alkyl is preferably $-C_{1-3}$ alkyl, or whererin two $R_b$ together with the attached N atom can form a 4-7 membered heterocycloalkyl, e.g., a 4-7 membered heterocycloalkyl containing 1 or 2 heteroatoms selected from N and O; more preferably, $R_1$ at the ortho position of the nitrogen heteroatom on the five-membered ring is selected from $-C(O)-C_{1-6}$ alkyl and $-C(O)N(R_b)_2$, e.g., $-CON(C_{1-3}\ alkyl)_2$, -CO-4-7 membered monocyclic heterocycloalkyl, e.g., $-CON\ (CH_3)_2$,

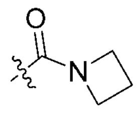

,

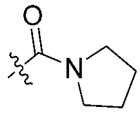, 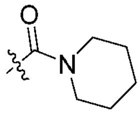, 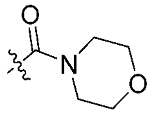,

and the remaining $R_1$ is each independently selected from H, halogen, -CN, $-NH_2$, and $-C_{1-6}$ alkyl, preferably selected from H, halogen, and $-C_{1-6}$ alkyl;
wherein $R_1$ located at the ortho position of the ring nitrogen atom attached to the rest of the molecule can exist in stereoisomeric form, such as R or S configuration, e.g.,

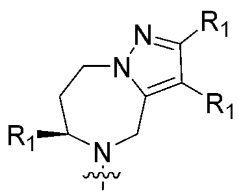.

**Embodiment III-9:** The compound of Formula (III) according to any one of Embodiments III-1 to III-8-1, a stereoisomer, tautomer, stable isotope variant, pharmaceutically acceptable salt or solvate thereof, wherein the structural fragment

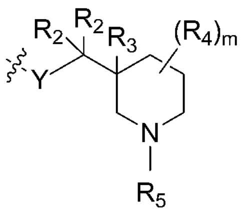

is as defined generally or specifically in any one of the Embodiments II-13 to II-18.
**Embodiment III-10:** The compound of Formula (III) according to Embodiment III-1, a stereoisomer, tautomer, stable isotope variant, pharmaceutically acceptable salt or solvate thereof, which may have definitions of each sub-general formula and each group as shown in Embodiment II-19-1, wherein the difference is that the left fragment is

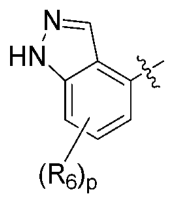

as defined in any one of Embodiments III-1 to III-3.

In the fourth aspect, the present invention provides the following compound Embodiment:

**Embodiment IV-1:** The compound of Formula (IV), a stereoisomer, tautomer, stable isotope variant, pharmaceutically acceptable salt or solvate thereof,

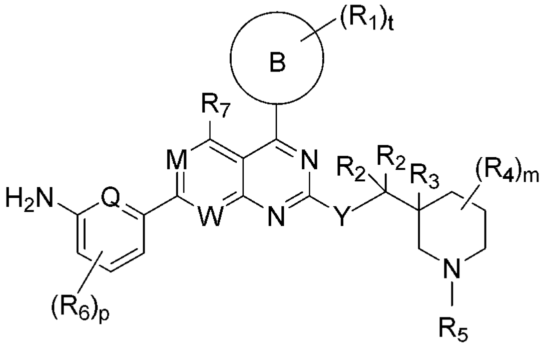

(IV-1)

wherein each of or combinations of B, Y, $R_1$, $R_2$, $R_3$, $R_4$, $R_5$, $R_6$, $R_7$, M, W, t, m, p has the general or specific defined meaning in Embodiments II-1 to II-20 for formula (II) compounds of the second aspect of the present invention.

**Embodiment IV-2:** The compound of Formula (IV)according to Embodiment IV-1, a stereoisomer, tautomer, stable isotope variant, pharmaceutically acceptable salt or solvate thereof, wherein p is 1, or p is 2, or p is 3, preferably p is selected from an integer of 1 to 2.

**Embodiment IV-3:** The compound of Formula (IV) according to Embodiment IV-1 or Embodiment IV-2, a stereoisomer, tautomer, stable isotope variant, pharmaceutically acceptable salt or solvate thereof, wherein the structural fragment

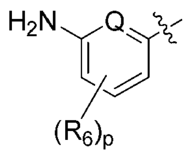

is

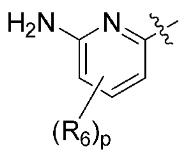

or

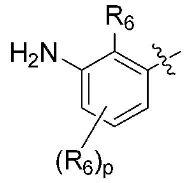

,

e.g.,

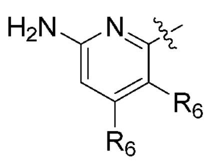

,

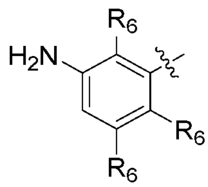

;

wherein each $R_6$ is independently selected from H, D, halogen, CN, -$C_{1-6}$ alkyl, -$C_{2-6}$ alkenyl, -$C_{2-6}$ alkynyl, -O-$C_{1-6}$ alkyl, -S-$C_{1-6}$ alkyl, and -Se-$C_{1-6}$ alkyl, wherein the -$C_{1-6}$ alkyl, -$C_{2-6}$ alkenyl, -$C_{2-6}$ alkynyl present are each independently and optionally substituted by halogen, -O-$C_{1-6}$ alkyl, or D; preferably each is independently selected from halogen, CN, and - $C_{1-6}$ alkyl optionally substituted by halogen;

in a specific embodiment, $R_6$ carried by the Q position in this fragment is selected from halogen and CN, $R_6$ at the para position of -$NH_2$ is $C_{1-6}$ alkyl optionally substituted by halogen, preferably - $C_{1-3}$ alkyl optionally substituted by halogen, e.g., -$CF_3$, and $R_6$ at the meta position is a -$C_{1-6}$ alkyl, preferably -$C_{1-3}$ alkyl, e.g. -$CH_3$.

**Embodiment IV-4:** The compound of Formula (IV) according to any one of Embodiments IV-1 to IV-3, a stereoisomer, tautomer, stable isotope variant, pharmaceutically acceptable salt or solvate thereof, wherein M is generally or specifically defined as in any one of the Embodiments II-8 to II-8-2.

**Embodiment IV-5:** The compound of Formula (IV) according to any one of Embodiments IV-1 to IV-4, a stereoisomer, tautomer, stable isotope variant, pharmaceutically acceptable salt or solvate thereof, wherein W is generally or

specifically defined as in the Embodiment II-9.

**Embodiment IV-6:** The compound of Formula (IV) according to any one of Embodiments IV-1 to IV-5, a stereoisomer, tautomer, stable isotope variant, pharmaceutically acceptable salt or solvate thereof, wherein $R_7$ is generally or specifically defined as in any one of the Embodiments 11-10 to II-10-2.

**Embodiment IV-7:** The compound of Formula (IV) according to any one of Embodiments IV-1 to IV-6, a stereoisomer, tautomer, stable isotope variant, pharmaceutically acceptable salt or solvate thereof, wherein the fused parent nuclei containing M and W is generally or specifically defined as in Embodiment II-11.

**Embodiment IV-8:** The compound of Formula (IV) according to any one of Embodiments IV-1 to IV-7, a stereoisomer, tautomer, stable isotope variant, pharmaceutically acceptable salt or solvate thereof, wherein the structural fragment

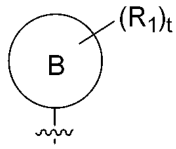

is generally or specifically defined as in Embodiment III-8.

**Embodiment IV-9:** The compound of Formula (IV) according any one of Embodiments IV-1 to IV-8, a stereoisomer, tautomer, stable isotope variant, pharmaceutically acceptable salt or solvate thereof, wherein the structural fragment

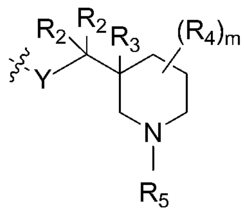

is generally or specifically defined as in any one of the Embodiments II-13 to II-18.

**Embodiment IV-10:** The compound of Formula (IV) of Embodiment IV-1, a stereoisomer, tautomer, stable isotope variant, pharmaceutically acceptable salt or solvate thereof, which may have definitions of each sub-general formula and each group as shown in Embodiment II-19-1, wherein the difference is that the left fragment is

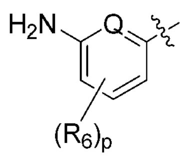

as defined in any one of Embodiments IV-1 to IV-3.

It is to be noted that the compounds of the present invention encompass the above independent embodiments or specific embodiments, and further encompass embodiments consisting of any combination or sub-combination of the above embodiments or specific embodiments, as well as embodiments consisting of any combination of any preferred or exemplary embodiments above.

**Beneficial effects of the present invention**

As described previously, it is known that Ras proteins, especially KRas mutant proteins, play a role in tumorigenesis and a variety of other diseases. We have surprisingly found that the compounds of the present invention with the above structural features can potently inhibit cell proliferation in cell lines carrying KRas mutant proteins (e.g., G12C, G12D, G12V, G12A, G12R, G12S, G13D and Q61H mutant proteins), and KRAS wild type amplification, thereby shows potential value as an antiproliferative, pro-apoptotic, and/or anti-invasive agent in the prevention, containment, and/or treatment of associated neoplastic diseases. In particular, the compounds of the present invention are expected to be useful for prevention or treatment of diseases or conditions inhibited by RAS proteins, especially KRAS mutant proteins (e.g., G12C, G12D, G12V, G12A, G12R, G12S, G13D and Q61H mutant proteins), or KRAS wild type amplification, such as cancer or tumor as defined herein.

Specifically, through research, it has been found that the compound of the present invention achieves one or more of the following technical effects:

- High mutant protein inhibitory activity: The compounds of the present invention, especially the compounds specifically exemplified in the context herein, have shown inhibitory activity against cell proliferation in RAS associated cells, especially KRAS mutant cells (e.g., G12C mutation, G12D mutation, G12V mutation, G12A mutation, G12R mutation, G12S mutation, G13D mutation, and Q61H mutation) and KRAS amplified cell proliferation inhibition assays, IC50 values are in the range of 0.001nM-10μM, for example 0.001nM-1μM, 0.01nM-0.5μM, 0.01nM-0.1μM, 0.01nM-0.05μM, as shown in the activity examples 1, 2, 5, 6,and 8;

- Having favorable pharmacokinetic properties such as having a longer $t_{1/2}$ such that the dosing interval can be increased and the half-life longer to allow better patient compliance; having $AUC_{0-}t$ data with the best combined effect of safety/activity, having better druggability and higher bioavailability, and can achieve convenient oral administration, as shown in the activity example 3; and/or

- With clear satisfactory safety, a reduced risk of drug-drug interactions and no significant inhibition of CYP isoforms critical for drug metabolism, as shown in the activity example 4

- Oral administration has a good tumor suppression effect, as demonstrated by activity example 7 or 9.

Based on the beneficial effects of the compounds of the present invention described above, the present invention also provides technical solutions for the following aspects.

**Compounds of the present invention for use in the treatment or as medicants**

In one aspect, the present invention provides compounds of the present invention, preferably pharmaceutically acceptable salts or solvates thereof for use as medicants.

In another aspect, the present invention provides compounds of the present invention, preferably pharmaceutically acceptable salts or solvates thereof, for use as inhibitors of RAS, especially KRAS mutant proteins (e.g., G12C mutation, G12D mutation, G12V mutation, G12A mutation, G12R mutation, G12S mutation, G13D mutation, G12C-Q99L, G12C-R68M, G12C-Y96S, G12C-95H, G12C-A59S and Q61H mutation or their combination) and KRAS wild type amplified cell inhibitors.

In another aspect, the present invention provides compounds of the present invention, preferably pharmaceutically acceptable salts or solvates thereof, for the treatment and/or prevention diseases or conditions mediated by RAS mutant proteins, specifically KRAS mutant proteins (e.g., G12C, G12D, G12V, G12A, G12R, G12S, G13D, and Q61H mutant proteins) and KRAS amplification, or benefitted from the inhibition of RAS mutations, specifically KRAS mutant proteins (e.g., G12C, G12D, G12V, G12A, G12R, G12S, G13D, and Q61H mutant proteins) and KRAS amplification.

In specific embodiments, the present invention provides compounds of the present invention for the treatment and/or prevention of RAS mutations, specifically KRAS mutant proteins (e.g., G12C, G12D, G12V, G12A, G12R, G12S, G13D, and Q61H mutant proteins) and KRAS amplification, which promote the development and progression of the described disease, or the inhibition of RAS mutant proteins, specifically KRAS mutant proteins (e.g., G12C, G12D, G12V, G12A, G12R, G12S, G13D, and Q61H mutant proteins) and KRAS amplification will reduce the incidence of the diseases and decrease or eliminate the symptoms of the diseases, which including but not limited to: lung cancer, lung adenocarcinoma, bone cancer, pancreatic cancer, skin cancer, head and neck cancer, skin or intraocular melanoma, uterine cancer, ovarian cancer, rectal cancer, anal region cancer, gastric cancer, colon cancer, breast cancer, fallopian tube cancer, endometrial cancer, cervical cancer, vaginal cancer, vulvar cancer, Hodgkin's disease, esophageal cancer, small intestinal cancer, endocrine system cancer, thyroid cancer, parathyroid carcinoma, adrenal carcinoma, soft tissue sarcoma, urethral cancer, penile cancer, prostate cancer, chronic or acute leukemia, lymphocytic lymphoma, bladder cancer, renal or ureteral cancer, renal pelvis cancer, central nervous system tumor (CNS), primary CNS lymphoma, spinal tumor, brainstem glioma, or pituitary adenoma.

The present invention especially provides compounds of Formula (I) or isomers, pharmaceutically acceptable salts or solvates thereof that can be used for the treatment of patients suffering from pancreatic cancer, colon cancer, rectal cancer, lung adenocarcinoma, lung carcinoma, cholangiocarcinoma, endometrial carcinoma, ovarian cancer, leukemia; and most preferably selected from patients with pancreatic cancer, colon cancer, rectal cancer, lung adenocarcinoma, cholangiocarcinoma.

**Pharmaceutical compositions and their administration**

In another aspect, the present invention provides pharmaceutical compositions comprising compounds of Formula (I) as defined above, preferably pharmaceutically acceptable salts or solvates thereof, and pharmaceutical carriers or excipients. The pharmaceutical compositions of the present invention can be used for treating or preventing

diseases mediated by RAS mutations, especially KRAS mutations, e.g., diseases mediated by KRAS G12C, KRAS G12D, KRAS G12V, G12A, G12R, G12S or KRAS G13D mutations or KRAS Q61H mutations, and KRAS amplification, such as tumor or cancer.

The pharmaceutical compositions of the present invention described above can be formulated by techniques known to those skilled in the art, such as techniques disclosed in Remington's Pharmaceutical Sciences Edition 20. For example, it can be formulated as tablets, powders, capsules, lozenges, granules, solutions, dispersions, suspensions, syrups, sprays, suppositories, gels, emulsions, patches, etc. The compositions may contain conventional components in pharmaceutical formulations such as diluents (e.g., glucose, lactose, or mannitol), carriers, pH modifiers, buffers, sweeteners, fillers, stabilizers, surfactants, wetting agents, lubricants, emulsifiers, suspending agents, preservatives, antioxidants, opacifiers, glidants, processing aids, colorants, fragrance adding agents, flavoring agents, other known additives, and other active agents. Appropriate carriers and vehicles are well-known to those skilled in the art and are described in detail, for example in Ansel, Howard C., et al, Ansel's Pharmaceutical Dosage Forms and Drug Delivery Systems. Philadelphia: Lippincott, Williams & Wilkins, 2004.

The administration and application of the pharmaceutical compositions of the present invention are in accordance with good medical practice. Factors to consider in this context include the specific disorder treated, the specific mammals treated, the clinical situation of the individual patient, the cause of the disorder, the location of the agent delivery, the method of application, the arrangement of application, and other factors well-known to the physician practitioner. The optimal dose level and frequency of administration of the compound or pharmaceutical composition of the present invention may be determined by those skilled in the art through standard tests in the field of pharmaceutical research.

The compositions of the present invention may be administered in any suitable manner, including oral, topical (including buccal and sublingual), rectal, vaginal, transdermal, parenteral, subcutaneous, intraperitoneal, intrapulmonary, intradermal, intrathecal, inhalation, and epidural and intranasal, and if local therapy is desired, it can also be administered intralesionally. Parenteral infusion includes intramuscular, intravenous, intra-arterial, intraperitoneal, or subcutaneous administration. In some embodiments, the pharmaceutical compositions of the present invention are administered orally.

For a 70 kg human subject, the appropriate dose range of the compound of the present invention may be routinely determined by those skilled in the field, e.g., it can be 1-1000 mg/day.

When the dosage of a drug or its pharmaceutically acceptable salt is described herein, it should be understood that the dosage is based on the weight of the free base and does not include any hydrates or solvate unless it is indicated in the specification that the dosage is based on the weight of the salt, hydrate, or solvate.

## Methods of treatment and uses

As described above, the compounds of the present invention and the compounds of various specific embodiments thereof, especially the compounds specifically prepared and characterized in the examples, exhibit inhibitory effects on RAS mutations, especially KRAS mutations, e.g., KRAS G12C, KRAS G12D, KRAS G12V, G12A, G12R, G12S or KRAS G13D mutations, or KRAS Q61H mutated, and KRAS amplified cells.

Therefore, in another aspect, the present invention provides a method for inhibiting RAS mutations, especially KRAS mutations, and preferably KRAS G12D mutations in cells, which comprises contacting the cells with a compound of the present invention, preferably a pharmaceutically acceptable salt or solvate thereof, to inhibit the activity of RAS mutations, especially KRAS mutations (e.g., G12C mutation, G12D mutation, G12V mutation, G12A mutation, G12R mutation, G12S mutation, G13D mutation and Q61H mutation), and KRAS amplification.

Based on the same properties, the present invention correspondingly provides a method for inhibiting abnormal cell growth in a mammal, which comprises administering to the mammal a therapeutically effective amount of a compound of the present invention, preferably a pharmaceutically acceptable salt or solvate thereof, or a pharmaceutical composition comprising a compound of the present invention, preferably a pharmaceutically acceptable salt or solvate thereof.

In another aspect, the present invention provides a method for treating and/or preventing diseases mediated by RAS mutations, especially KRAS mutations (e.g., G12C mutation, G12D mutation, G12V mutation, G12A mutation, G12R mutation, G12S mutation, G13D mutation and Q61H mutation), and KRAS amplification, which comprises administering to a subject in need thereof a therapeutically effective amount of a compound of the present invention, preferably a pharmaceutically acceptable salt or solvate thereof, or a pharmaceutical composition comprising a compound of the present invention, preferably a pharmaceutically acceptable salt or solvate thereof.

In another aspect, the present invention provides the use of a compound of the present invention, preferably a pharmaceutically acceptable salt or solvate thereof, or a pharmaceutical composition comprising a compound of the present invention, preferably a pharmaceutically acceptable salt or solvate thereof, for inhibiting diseases medicated by RAS mutations, especially KRAS mutations, preferably KRAS G12C, KRAS G12D, KRAS G12V, KRAS G12A, KRAS G12R, KRAS G12S or KRAS G13D, or KRAS Q61H mutation, and KRAS amplification in cells.

In another aspect, the present invention provides the use of a compound of the present invention, preferably a pharmaceutically acceptable salt or solvate thereof, or a pharmaceutical composition comprising a compound of the

present invention, preferably a pharmaceutically acceptable salt or solvate thereof, in the preparation of a medicament for treating and/or preventing diseases mediated by RAS mutations, especially KRAS mutations (e.g., G12C mutation, G12D mutation, G12V mutation, G12A mutation, G12R mutation, G12S mutation, G13D mutation, and Q61H mutation), and KRAS amplification.

For each of the technical solutions of the methods and uses provided by the present invention described above, the abnormal cell growth or the disease mediated by RAS mutations, especially KRAS mutations, preferably KRAS G12C, KRAS G12D, KRAS G12V, KRAS G12A, KRAS G12R, KRAS G12S or KRAS G13D, or KRAS Q61H mutation, and KRAS amplification especially refer to cancer or tumors. Exemplary cancers or tumors include, but are not limited to lung cancer, lung adenocarcinoma, bone cancer, pancreatic cancer, skin cancer, head and neck cancer, cutaneous or intraocular melanoma, uterine cancer, ovarian cancer, rectal cancer, anal region cancer, gastric cancer, colon cancer, breast cancer, fallopian tube cancer, endometrial cancer, cervical cancer, vaginal cancer, vulvar cancer, Hodgkin's disease, esophageal cancer, small intestine cancer, endocrine system cancer, thyroid cancer, parathyroid cancer, adrenal cancer, soft tissue sarcoma, urethral cancer, penile cancer, prostate cancer, chronic or acute leukemia, lymphocytic lymphoma, bladder cancer, kidney or ureteral cancer, renal cell carcinoma, renal pelvic cancer, central nervous system (CNS) tumors, primary CNS lymphoma, spinal tumor, brainstem glioma or pituitary adenoma.

For each of the technical solutions of the methods and uses provided by the present invention described above, the abnormal cell growth or the disease mediated by RAS mutation, especially KRAS mutation (e.g., G12C mutation, G12D mutation, G12V mutation, G12A mutation, G12R mutation, G12S mutation, G13D mutation, and Q61H mutation), and KRAS amplification are preferably selected from pancreatic cancer, colon cancer, rectal cancer, lung adenocarcinoma, lung cancer, cholangiocarcinoma, endometrial cancer, ovarian cancer, leukemia; and most preferably selected from pancreatic cancer, colon cancer, rectal cancer, lung adenocarcinoma, and cholangiocarcinoma.

Therefore, in a preferred embodiment in this aspect, the present invention provides each of the above technical solutions of the methods and uses for treating or preventing cancers or tumors by inhibiting RAS mutation or amplification. In a further preferred embodiment, the present invention provides each of the above technical solutions of the methods and uses for treating or preventing pancreatic cancer, colon cancer, rectal cancer, lung adenocarcinoma and cholangiocarcinoma by inhibiting RAS mutation or amplification.

The present invention also provides the use of a compound of the present invention, preferably a pharmaceutically acceptable salt or solvate thereof, as a research tool compound for a RAS inhibitor, especially a KRAS inhibitor (e.g., G12C mutation, G12D mutation, G12V mutation, G12A mutation, G12R mutation, G12S mutation, G13D mutation, Q61H mutation and KRAS amplification inhibitor) in research. Therefore, the present invention relates to the in vitro use of a compound of the present invention, preferably a pharmaceutically acceptable salt or solvate thereof, as a RAS inhibitor, and particularly relates to the in vitro use of a compound of the present invention, preferably a pharmaceutically acceptable salt or solvate thereof, as a research tool compound that acts as a RAS inhibitor. The present invention also relates to a method for inhibiting RAS, especially KRAS (e.g., G12C mutation, G12D mutation, G12V mutation, G12A mutation, G12R mutation, G12S mutation, G13D mutation, Q61H mutation and KRAS amplification), particularly an in vitro method, the method comprises administering a compound of the present invention, preferably a pharmaceutically acceptable salt or solvate thereof, to a sample (such as a biological sample). It should be understood that the term "in vitro" is used in this specific context with the meaning of "outside a living human body or animal body", which specifically includes experiments performed with cells, cell or subcellular extracts and/or biomolecules in an artificial environment, for example, in an aqueous solution or culture medium that can be provided in a flask, test tube, culture dish, microtiter plate, etc.

## Pharmaceutical combination

The compound of the present invention may be administered as the sole active ingredient or in combination with other drugs or therapies.

Thus, in another aspect, the present invention provides a pharmaceutical combination comprising, or consisting of, a compound of the present invention, preferably a pharmaceutically acceptable salt or solvate thereof, and other active agents. This pharmaceutical combination is used for inhibiting abnormal cell growth in mammals, or for treating and/or preventing diseases mediated by RAS mutation, preferably KRAS mutation (e.g., G12C mutation, G12D mutation, G12V mutation, G12A mutation, G12R mutation, G12S mutation, G13D mutation, and Q61H mutation), and KRAS amplification.

The other active agents described can be one or more additional compounds of the present invention, or can be a second or additional (e.g., third) compound that is compatible with the compounds of the present invention, i.e., does not adversely affect each other, or has complementary activities. For example, these active agents can be compounds known to regulate other biological activity pathways or can be compounds that regulate different components in the biological activity pathway involved in the compounds of the present invention, or even compounds that overlap with the biological targets of the compounds of the present invention.

In a specific embodiment, other active agents that can be used in combination with the compounds of the present invention include, but are not limited to, chemotherapeutic agents, therapeutic antibodies, and radiotherapy, such as

alkylating agents, antimetabolites, cell cycle inhibitors, mitotic inhibitors, topoisomerase inhibitors, antihormones, angiogenesis inhibitors, cytotoxic agents.

The other active agents used in combination with the present invention can be administered simultaneously, separately, or sequentially with the compounds of the present invention via the same or different administration routes. The other active agents can be co-administered with the compounds of the present invention in a single pharmaceutical composition or administered separately from the compounds of the present invention in different discrete units, such as a combination product, preferably in the form of a kit. When administered separately, they can be administered simultaneously or successively, and the successive administration can be close or far apart in time. They can be prepared and/or formulated by the same or different manufacturers. Moreover, the compounds of the present invention and other active agents can be added to the combination therapy (i) before the combination product is sent to the physician (e.g., in the case of a kit containing the compound of the present invention and another drug); (ii) by the physician himself/herself (or under the physician's guidance) immediately before administration; (iii) by the patient himself/herself, for example, during the sequential administration of the compound of the present invention and other active agents.

The compounds of the present invention can also be combined with antitumor therapies, which include but are not limited to surgery, radiation therapy, transplantation (e.g., stem cell transplantation, bone marrow transplantation), tumor immunotherapy and chemotherapy, etc.

Therefore, in another aspect, the present invention also provides a kit, which comprises two or more separate pharmaceutical compositions, at least one of which contains a compound of the present invention or a pharmaceutically acceptable salt or solvate thereof, and a device for separately containing the compositions, such as containers, dispensing bottles, or discrete foil packages, for example, blister packs for packaging tablets, capsules, etc., and also includes instructions for use. The kit of the present invention is particularly suitable for administering different dosage forms, such as oral and parenteral dosage forms, or for administering different compositions at different dosage intervals.

For the technical solutions of the pharmaceutical compositions, pharmaceutical combinations, or kits of the present invention described above, the abnormal cell growth or the diseases mediated by RAS mutations, especially KRAS mutations, preferably KRAS G12C, KRAS G12D, KRAS G12V, KRAS G12A, KRAS G12R, KRAS G12S or KRas G13D, or KRAS Q61H mutations, and KRAS amplification are as defined above for the methods and uses of the present invention.

For the compounds, pharmaceutical compositions, methods, uses, pharmaceutical combinations, and kits of the present invention described above, the compounds in the examples herein are preferred.

## Preparation method of compound of the present invention

In another aspect, the present invention also provides a preparation method for a compound defined in the present invention.

The compounds of the present invention can be prepared by a variety of methods, including the general methods given below, the methods disclosed in the examples, or methods similar thereto.

Standard synthetic methods and operations for preparing organic compounds and for the transformation and manipulation of functional groups are known in the art and can be found in standard textbooks, for example, Smith M.B., "March's Advanced Organic Chemistry: Reactions, Mechanisms, and Structure", 7th edition, Wiley, 2013). For each reaction step of each general synthetic scheme, appropriate reaction conditions are known to those skilled in the art or can be routinely determined. The method steps for synthesizing the compounds of the present invention can be carried out under reaction conditions known per se (including those specifically mentioned), in the absence or usually in the presence of a solvent or diluent (including, for example, a solvent or diluent that is inert to the reagents used and can dissolve the reagents used), in the absence or presence of a catalyst, a condensing agent or a neutralizing agent (such as an ion exchanger, such as a cation exchanger, for example, in the H+ form), according to the nature of the reaction and/or the reactants, at a reduced, normal or elevated temperature (for example, about -100 °C to about 190 °C, including, for example, about -78 °C to about 150 °C, such as about 0 °C to about 125 °C, room temperature, - 20 to 40 °C or the reflux temperature), under atmospheric pressure or in a closed container, when appropriate, under pressure, and/or in an inert atmosphere such as an argon or nitrogen atmosphere.

Unless otherwise specified, the starting materials and reagents used in the preparation of the compounds are commercially available, or are compounds known in the literature, or can be prepared by those skilled in the art by the methods described below, by methods similar to those given below, or by standard methods known in the art. Unless otherwise stated in the method description, the applicable solvents are those conventional solvents well known to those skilled in the art that are suitable for the specific type of reaction involved, such as water, esters, ethers, liquid aromatic hydrocarbons, alcohols, nitriles, halogenated hydrocarbons, amides, bases, carboxylic anhydrides, cyclic, straight or branched hydrocarbons, or mixtures of these solvents. Such solvent mixtures can also be used for work-up, for example, work-up by chromatography or partition.

If necessary, the starting materials and intermediates in the synthetic reaction process can be separated and

purified by conventional techniques, which include but are not limited to filtration, distillation, crystallization, chromatography, etc. If the intermediates and the final products are obtained in solid form, purification can also be carried out by recrystallization or aging. The materials can be characterized by conventional methods including physical constants and spectroscopic data. The reaction mixture is worked up in a conventional manner, for example, by mixing with water, separating the phases, and purifying the crude product by chromatography when appropriate.

Those skilled in the art can recognize whether there are stereocenters in the compounds of the present invention. At all stages of the reaction, a mixture of the formed isomers can be separated into individual isomers, such as diastereoisomers or enantiomers, or into any desired mixture of isomers, such as a racemate or a mixture of diastereoisomers, see, for example, E. L. Eliel, S. H. Wilen, and L. N. Mander's "Stereochemistry of Organic Compounds" (Wiley-Interscience, 1994).

In the case where a mixture of stereoisomers is generated during the preparation of the compounds of the present invention, the individual stereoisomers of the compounds of the present invention can be obtained by resolution. For example, starting from the compounds of the present invention obtained as a mixture of stereoisomers, using well-known methods, such as forming diastereomeric pairs, forming salts with optically active acids, followed by fractional crystallization and regeneration of the free base, or by chiral preparative chromatography; alternatively, starting materials or intermediates with established stereochemistry can be used, or any known chiral resolution method can be used to obtain an optically pure or enantiomerically enriched synthetic intermediate, which can then be used as such in the subsequent steps at various stages of the above synthetic process.

In certain specific cases, it may be necessary to protect specific reactive groups using appropriate protecting groups to avoid interference with the reaction of other reactive groups. Suitable protecting groups and methods for protection and deprotection using such suitable protecting groups are well known to those skilled in the art; examples thereof can be found in T. Greene and P. Wuts, Protective Organic Groups in Synthesis (3rd Edition), John Wiley & Sons, NY (1999).

Only the general synthetic schemes for synthesizing the compounds of the present invention are illustrated below. Other routes known to those of ordinary skill in the art, as well as other reactants and intermediates, can also be used to obtain the compounds of the present invention.

For clarity, in the exemplary synthesis scheme described below, $R_1$, $R_2$, $R_3$, $R_4$, $R_5$, $R_6$, $R_7$, $R_8$, $R_9$, $R_{10}$, X, Y, Z, n, m and t appearing in the structural formulas of each intermediate compound are defined as above for the compound of the present invention, wherein PG represents a suitable protecting group that can be determined by those skilled in the art based on organic chemistry knowledge, unless otherwise specified.

**Synthesis scheme A**

The synthesis of the compounds of the present invention can be prepared according to the following schemes or suitable variations thereof. Unless otherwise specified, each group is as defined above.

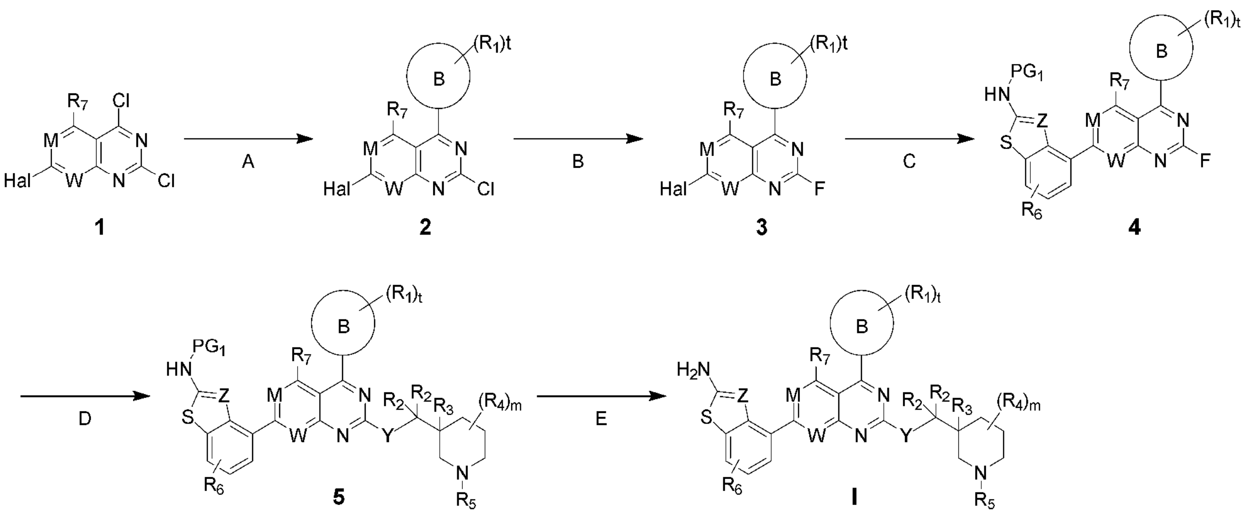

1 2 3 4 5 I

Compound 1 is commercially available, can be obtained according to known methods reported in the literature, or can be prepared by the methods used in the examples herein or methods analogous thereto, wherein Hal represents a halogen atom, such as Cl, Br, or I. In Step A, fragment B-$(R_1)_t$ is introduced into Compound 1 via an aromatic nucleophilic substitution reaction to afford Compound 2. Typical conditions for aromatic nucleophilic substitution are well known in the art, for example, DIEA/THF and the like. In Step B, Compound 2 is fluorinated via a halogen exchange reaction under

conditions such as KF/DMSO to afford Compound 3. In Step C, a benzothiophene or benzothiazole fragment is introduced into Compound 3 via a metal-catalyzed coupling reaction to afford Compound 4. Typical metal-catalyzed coupling reactions include the Suzuki-Miyaura coupling reaction, with typical conditions such as RuPhos Pd G4/K3PO4/dioxane/-water and the like. In Step D, a piperidine derivative fragment is introduced into Compound 4 via an aromatic nucleophilic substitution reaction to afford Compound 5. The latter is deprotected in Step E to remove the protecting group that may be present to afford the compound of Formula I.

**Synthesis scheme B**

Some compounds of the present invention (where Z = C-CN) can also be prepared according to the following schemes or suitable variations thereof. Unless otherwise specified, each group is as defined above.

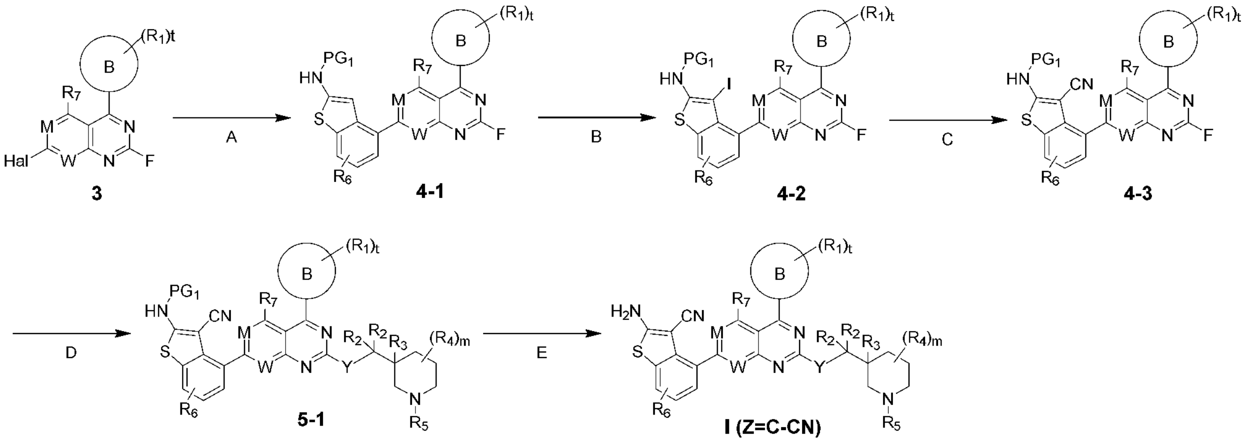

3, 4-1, 4-2, 4-3, 5-1, I (Z=C-CN)

In Step A, a benzothiophene fragment is introduced into Compound 3 via a metal-catalyzed coupling reaction to afford Compound 4-1; in Step B, Compound 4-1 undergoes iodination with NIS to give Compound 4-2; in Step C, Compound 4-2 undergoes a metal-catalyzed cyanation reaction to afford Compound 4-3, with typical cyanation conditions such as $Zn(CN)_2/Pd(PPh_3)_4$ and the like; in Step D, a piperidine derivative fragment is introduced into Compound 4-3 via an aromatic nucleophilic substitution reaction to afford Compound 5-1; the latter is deprotected in Step E to remove the protecting group that may be present to afford the compound of Formula I (Z = C-CN).

**Synthesis scheme C**

The synthesis of the compounds of Formula II of the present invention can be prepared according to the following schemes or suitable variations thereof. Unless otherwise specified, each group is as defined above.

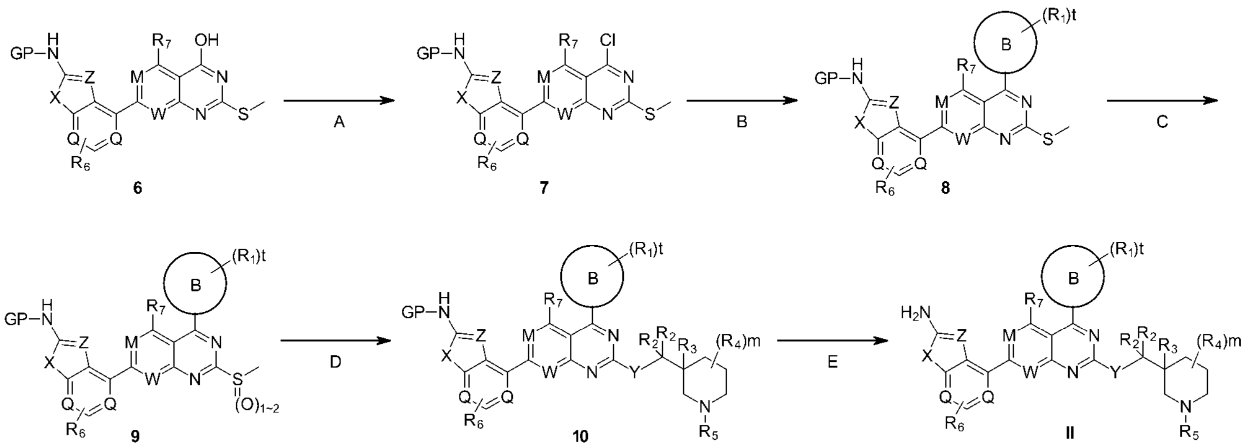

6, 7, 8, 9, 10, II

Intermediate 6 can be prepared with reference to the synthesis of intermediates in the Examples section of the present invention; in Step A, Intermediate 6 undergoes a chlorination reaction under conditions such as oxalyl chloride/DMF/DCM to afford Intermediate 7; in Step B, Intermediate 7 undergoes a substitution reaction via an aromatic

nucleophilic substitution reaction under conditions such as DIPEA/DMF to afford Compound 8; in Step C, Compound 8 undergoes an oxidation reaction under conditions such as magnesium monoperoxyphthalate hexahydrate/DCM/water to afford Compound 9, which may be sulfone, sulfoxide, or a mixture thereof depending on the reaction conditions; in Step D, Compound 9 undergoes a reaction via an aromatic nucleophilic substitution reaction under conditions such as tBuONa/THF to afford Compound 10; in Step E, Compound 10 is deprotected using appropriate reaction conditions to afford the compound of Formula II. It should be noted that the finally obtained compound of Formula II can be resolved into diastereomers by Pre-HPLC or SFC to obtain a single chiral compound of Formula II; alternatively, Intermediate 6 can be subjected to SFC chiral resolution to obtain axially chiral single Intermediate 6A, which is then converted into a single chiral compound of Formula II according to the above scheme.

It should be noted that the typical metal-catalyzed coupling reactions involved in the present invention are well known in the art, for example but not limited to Suzuki-Miyaura coupling, Negishi coupling, Stille coupling and the like. Suitable catalysts include, for example, XantPhos Pd G2, cataCXium® A Pd G3, bis(triphenylphosphine)palladium(II) chloride, Pd(dppf)Cl2, Pd2dba3, tetrakis(triphenylphosphine)palladium and palladium(II) acetate and the like; if necessary, suitable ligands may include tricyclohexylphosphine, triphenylphosphine and the like; suitable bases also include potassium fluoride, cesium carbonate, sodium carbonate, potassium tert-butoxide and potassium phosphate monohydrate and the like.

It should be noted that the removal of the protecting group in Step E can be adjusted according to the protecting group carried by the molecule, and can be a one-step reaction or a multi-step reaction.

## Synthesis examples

The present invention is further illustrated below with reference to Examples. It should be noted that the following Examples are illustrative and should not be construed as limiting the protection scope of the present invention.

The following abbreviations are used in the description of the embodiments and subsequent specific examples: ACN (Acetonitrile); Boc (tert-butoxycarbonyl); $CDCl_3$ (deuterated chloroform); CSI (chlorosulfonyl isocyanate); DCM (dichloromethane); $DephosPdCl_2$ or $DPEphosPdCl_2$ (Bis(diphenylphosphophenyl ether) palladium (II) chloride); DIEA or DIPEA (*N,N*-diisopropylethylamine); DMF (*N,N*-dimethylformamide); DMSO (dimethyl sulfoxide); DMSO-$d_6$ (hexadeuterated dimethyl sulfoxide); EA or EtOAc (ethyl acetate); EDTA-K2 (ethylenediaminetetraacetic acid dipotassium salt); EtOH (Ethanol); FCC (flash column chromatography); g (gram); h (hour); HCl (hydrogen chloride); HCl-MeOH or HCl/MeOH (hydrogen chloride in methanol); HLM (human liver microsomes); $H_2O$ (water); $H_2SO_4$ (sulfuric acid); IV (intravenous); $K_2CO_3$ (potassium carbonate); LCMS (liquid chromatography mass spectrometry); LC-MS/MS (liquid chromatography-mass spectrometry-mass spectrometry); MeOH (methanol); Methanol-$d_4$ (tetradeuterated methanol); mg (milligram); MHz (megahertz); min (minute); mL (milliliter); mmol (millimolar); MMPP (magnesium monoperoxyphthalate hexahydrate); MOM (methoxymethyl ether); MTBE (methyl tert-butyl ether); m/z (mass-to-charge ratio); $N_2$ (nitrogen); NaCl (sodium chloride); NaH (sodium hydride); $NaHCO_3$ (sodium bicarbonate); $Na_2SO_3$ (sodium sulfite); $Na_2SO_4$ (sodium sulfate); NCS (chlorobutanediimide); $NH_4Cl$ (ammonium chloride); nM (nanomolar concentration); NMR (nuclear magnetic resonance); $PdCl_2$(dtbpf) or Pd(dtbpf)$Cl_2$ (1,1'-bis(di-tert-butylphosphine)ferrocene palladium dichloride); $PdCl_2$(dppf) or Pd(dppf)$Cl_2$ (1,1'-diphenylphosphine ferrocene palladium dichloride); $Pd(OAc)_2$ (palladium acetate); $Pd(PPh_3)_4$ (palladium tetraphylphosphonium); PE (petroleum ether); PO (oral); $POCl_3$ (phosphorus oxychloride); r.t. (room temperature); Selectfluor (1-Chloromethyl-4-fluoro-1,4-diazotized bicyclo [2.2.2]octane bis(tetrafluoroborate)); $SiO_2$ (silica gel); TCDI (*N,N'*-thiocarbonyldiimidazole); TEA (triethylamine); THF (tetrahydrofuran); TIPS (triisopropylsilyl); TLC (thin-layer chromatography); TsOH (p-toluenesulfonic acid); TsOH·$H_2O$ (p-toluenesulfonic acid monohydrate); μL (microliter); μM (micromolar concentration); μmol (micromolar).

In the following examples, the names and structures of the synthesized target compounds are given. Any deviation between the name and the structure is not intentional, and in such a case, the structure shall prevail.

The experimental methods for which specific conditions are not specified in the following examples are generally carried out according to the conventional conditions of such reactions or according to the conditions recommended by the manufacturer. Unless otherwise stated, percentages and parts are weight percentages and weight parts. Unless otherwise stated, the ratios of liquids are volume ratios.

The experimental materials and reagents used in the following examples can be obtained from commercial sources, prepared according to the methods of the prior art, or prepared according to methods similar to those disclosed in this application, unless otherwise specified.

In the following examples, $^1$H-NMR spectra were recorded with Bruker (400 MHz) and chemical shifts were relative to the deuterated solvent peak ($CDCl_3$: $\delta$ = 7.26 ppm; $CD_3OD$: $\delta$ = 3.31 ppm; DMSO-$d_6$: $\delta$ = 2.50 ppm); mass spectra were recorded with Agilent 1260 liquid chromatography + Agilent G6125B mass spectrometry LCMS liquid chromatography mass spectrometry. Gas chromatography mass spectrometry was performed using Shimadzu GCMS-QP2010SE.

## Intermediate A

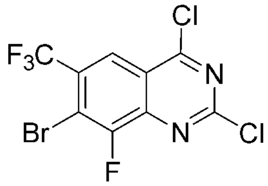

7-Bromo-2,4-difluoro-8-fluoro-6-(trifluoromethyl)quinazoline

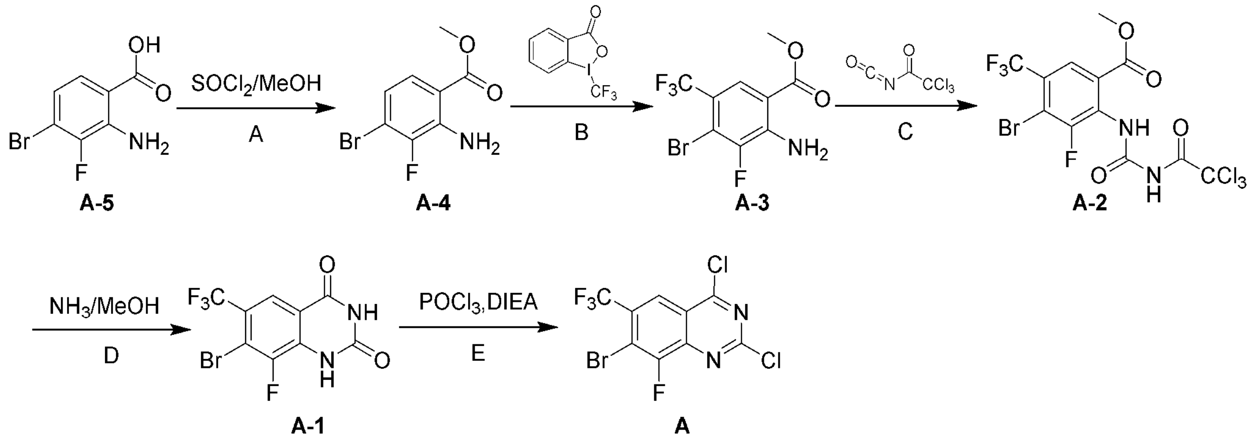


A-5 A-4 A-3 A-2 A-1 A

**Step A:** Methyl 2-amino-4-bromo-3-fluorobenzoate

Under 0 °C conditions, $SOCl_2$ (156 mL, 210 mmol) was added to a mixture of 2-amino-4-bromo-3-fluorobenzoic acid (50 g, 214 mmol) and methanol (300 mL). The reaction system was heated to 100 °C and stirred for 16 h. The reaction completion was monitored by TLC, the reaction solution was returned to room temperature, concentrated under reduced pressure, diluted with EA (1 L), washed with saturated aqueous $NaHCO_3$ (500 mL), and the collected organic phase was dried with anhydrous $Na_2SO_4$ and filtered, and the organic solution was concentrated and purified by FCC ($SiO_2$, EA/PE = 0-20%) to obtain methyl 2-amino-4-bromo-3-fluorobenzoate (40 g) as a white solid. LCMS (m/z): 249.9 (M+H).

**Step B:** Methyl 2-amino-4-bromo-3-fluoro-5-(trifluoromethyl)benzoate

At room temperature, the 1-(trifluoromehyl)-1,2-benzoyl-3(1*H*)-one (19g, 60 mmol) was added to a mixture of methyl 2-amino-4-bromo-3-fluorobenzoate (5 g, 20 mmol) and ACN (20 mL), and $N_2$ was degassed three times and heated to 100 °C for 72 h. After the reaction completion was monitored by LCMS, the reaction solution was returned to room temperature, diluted with EA (100 mL) after concentration, washed with water (50 mL × 2), and the collected organic phase was dried with anhydrous $Na_2SO_4$ and filtered, and the organic solution was concentrated and purified by FCC ($SiO_2$, EA/PE=0-10%) to obtain a light yellow solid methyl 2-amino-4-bromo-3-fluoro-5-(trifluoromethyl)benzoate (1.2 g). LCMS (m/z): 317.1 (M+H).

**Step C:** Methyl 4-bromo-3-fluoro-2-(3-(2,2,2-trichloroacetyl)ureido)-5-(trifluoromethyl)benzoate

At room temperature, 2,2,2-trichloroacetyl isocyanate (0.7 mL, 5.7 mmol) was added to a mixture of methyl 2-amino-4-bromo-3-fluoro-5-(trifluoromethyl)benzoate (1.2 g, 3.8 mmol) and THF (10 mL). The reaction system was stirred for 0.5 h at room temperature. The reaction completion was monitored by TLC, the reaction solution was directly concentrated to obtain methyl 4-bromo-3-fluoro-2-(3-(2,2,2-trichloroacetyl)ureido)-5-(trifluoromethyl)benzoate (1.6 g), a white solid, which was directly used in the next step reaction.

**Step D:** 7-Bromo-8-fluoro-6-(trifluoromethyl)quinazoline-2,4-diol

At room temperature, ammonia methanol solution (7M, 20 mL) was added to methyl 4-bromo-3-fluoro-2-(3-(2,2,2-trichloroacetyl)ureido)-5-(trifluoromethyl)benzoate (1.6 g), stirred at room temperature for 1 h. The reaction completion was monitored by TLC, the reaction solution was concentrated to obtain a white solid 7-bromo-8-fluoro-6-(trifluoromethyl)quinazoline-2,4-diol (1.0 g), which was directly used for the next step reaction. LCMS (m/z): 328.8 (M+H).

**Step E:** 7-Bromo-2,4-dichloro-8-fluoro-6-(trifluoromethyl)quinazoline

Under 0 °C conditions, DIEA (1.8 g, 14 mmol) was added to a mixture of 7-bromo-8-fluoro-6-(trifluoromethyl) quinazoline-2,4-diol (1.0 g, 3.0 mmol) and $POCl_3$ (10 mL). The reaction system was heated to 110 °C and stirred for 2 h. After the reaction completion was monitored by LCMS, the reaction solution was returned to room temperature, concentrated and diluted with acetonitrile (0.5 mL), then the resulting mixture was dropped into water (10 mL), the solid was precipitated, filtered and the filter cake was dried to obtain a brown-yellow solid 7-bromo-2,4-dichloro-8-fluoro-6-(trifluoromethyl)quinazoline (1.5 g) LCMS (m/z): 365.8 (M+H).

## Intermediate B

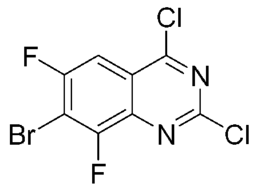

**B**

7-Bromo-2,4-dichloro-6,8-difluoroquinazoline

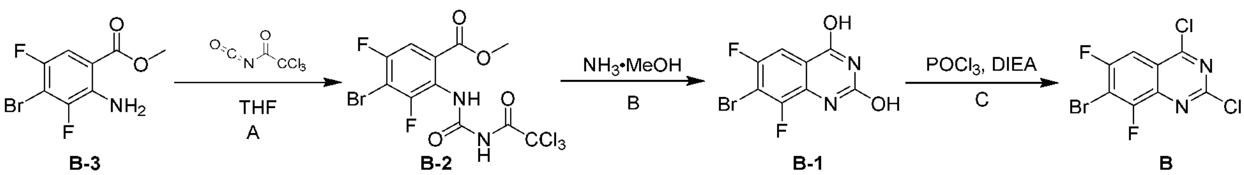


B-3 B-2 B-1 B

**Step A:** Methyl 4-bromo-3,5-difluoro-2-(3-(2,2,2-trichloroacetyl)ureido)benzoate

At room temperature, methyl 2-amino-4-bromo-3,5-difluorobenzoate (10g, 37.6 mmol) and THF (100 mL) were added to a round bottom flask with a stir bar. Then, 2,2,2-trichloroacetyl isocyanate (8.5 g, 45.1 mmol) was added dropwise to the system under stirring at room temperature. The resulting mixture was stirred at room temperature for 2 h and concentrated under reduced pressure to give methyl 4-bromo-3,5-difluoro-2-(3-(2,2,2-trichloroacetyl)ureido)benzoate (crude) as a brown solid, which was used directly in the next step. LCMS (ESI, m/z): 452.8 (M+H).

**Step B:** 7-Bromo-6,8-difluoroquinazoline-2,4-diol

At room temperature, methyl 4-bromo-3,5-difluoro-2-(3-(2,2,2-trichloroacetyl)ureido)benzoate obtained in the previous step was added to a round-bottom flask with a stir bar, and then $NH_3$ (100 mL, 7 M MeOH solution) was added. The resulting mixture was stirred at room temperature for 2 h and the reaction completion was monitored by LCMS. Concentrated under reduced pressure, the resulting solid was slurried with methyl tert-butyl ether and filtered to yield a light yellow solid 7-bromo-6,8-difluoroquinazoline-2,4-diol (14 g, crude), which was used directly in the next step without further purification. LCMS (ESI, m/z): 277.0 (M+H).

**Step C:** 7-Bromo-2,4-dichloro-6,8-difluoroquinazoline

7-Bromo-6,8-difluoroquinazoline-2,4-diol (14 g, crude) and $POCl_3$ (112 mL) was added to a round-bottom flask with a stir bar. DIEA (28 mL) was added dropwise to the system under stirring at room temperature. After dropwise addition, the system was heated to 110 °C and the reaction was stirred overnight. The reaction solution was concentrated under reduced pressure to about 30 mL, poured into water (600 mL), precipitated and collected by filtration, and after drying, a yellow solid 7-bromo-2,4-dichloro-6,8-difluoroquinazoline (11 g, crude) was obtained and directly used for subsequent reactions. LCMS (ESI, m/z): 312.9 (M+H).

## Intermediate C

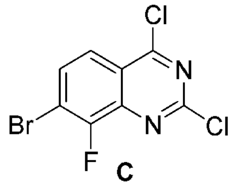

C

7-Bromo-2,4-dichloro-8-fluoroquinazoline

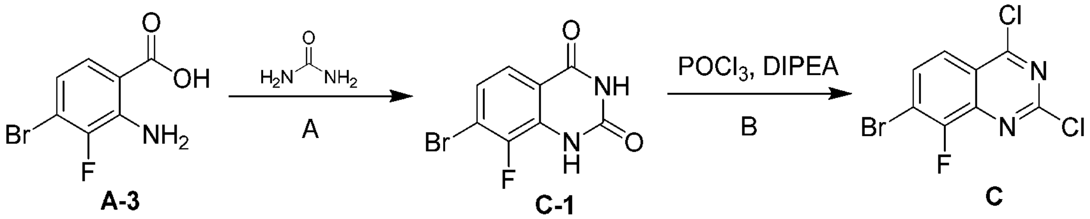


A-3 C-1 C

**Step A:** 7-Bromo-8-fluoroquinazoline-2,4 (1*H*, 3*H*)-dione

At room temperature, 2-amino-4-bromo-3-fluorobenzoic acid (10.0 g, 42.7 mmol) and urea (25.7 g, 427.3 mmol) were mixed together, warmed to 200 °C, and stirred for 2 h. The reaction gradually changed from solid state to liquid state, and then continued to change to solid state. After the reaction completion was monitored by LCMS, the solid was washed with hot water (250 mL) and filtered and collected to obtain 7-bromo-8-fluoroquinazoline-2,4 (1*H*, 3*H*)-dione (12 g, crude). LCMS (ESI, m/z): 258.9 (M+H).

**Step B:** 7-Bromo-2,4-dichloro-8-fluoroquinazoline

At room temperature, DIPEA (13.5 mL, 77.2 mmol) was added to a mixture of 7-bromo-8-fluoroquinazoline-2,4 (1*H*, 3*H*)-dione (4.0 g, 15.4 mmol) and $POCl_3$ (35.9 mL, 386.0 mmol) and warmed to 100°C, and stirred for 5 h. After the reaction completion was monitored by LCMS, most of the solvent and base were removed by concentration and diluted with ACN (10 mL). Under stirring at room temperature, the resulting dilution was slowly dropped into water to precipitate a solid, filtered, and dried to obtain a yellow solid 7-bromo-2, 4-dichloro-8-fluoroquinazoline (3.8 g, yield 83%). LCMS (ESI, m/z): 296.8 (M+H).

**The following intermediates were prepared according to the synthesis method described above:**

| Number | Structural Formula | Number | Structural Formula |
|---|---|---|---|
| Intermediate D | 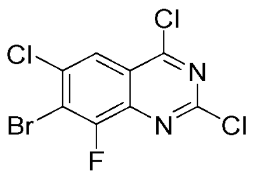 LCMS (m/z): 328.8 (M+H) | Intermediate E | 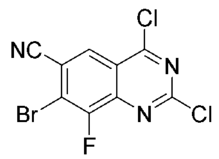 LCMS (m/z): 319.9 (M+H) |

## Intermediate F

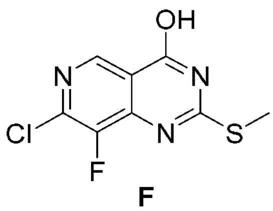

F

7-Bromo-8-fluoro-2(methylthio)pyrido[4,3-*d*]pyrimidine-4-ol

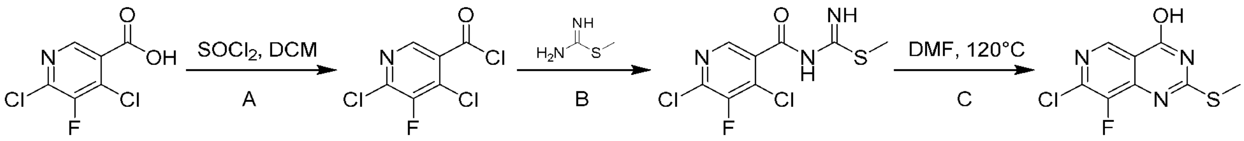


F-3 F-2 F-1 F

**Step A:** 4,6-Dichloro-5-fluoronicotinyl chloride

Under stirring at room temperature, sulfoxide chloride (2.25 mL, 31 mmol) was slowly added to a solution of 4,6-dichloro-5-fluoronicotinic acid (5.0 g, 23.4 mmol) in DCM (100 mL), followed by DMF (175 mg, 2.4 mmol). The resulting reaction solution was stirred at 50 °C for 2 h. The reaction completion was monitored by TLC, concentrated, added a small amount of toluene with steaming, a yellow solid 4,6-dichloro-5-fluoronicotinyl chloride (4.5 g, yield 83%) was obtained and directly used for subsequent reactions.

**Step B:** Methyl (4,6-Dichloro-5-fluoronicotinoyl)carbamimidothioate

Under stirring at 0 °C, a mixture of 4,6-dichloro-5-fluoronicotinyl chloride (4.5 g, 19.8 mmol) and ethylene glycol dimethyl ether (20 ml) was slowly added dropwise to a mixture of 2-methylisothiourea sulfuric acid (15 g, 49.5 mmol) and 1 M NaOH aqueous solution (70 ml). The temperature was maintained and stirred for 1 h. The precipitated solid was filtered and dried to obtain a product methyl (4,6-dichloro-5-fluoronicotinoyl)carbamimidothioate (5.0 g, yield 90%). LCMS (m/z): 282.1 (M+H).

**Step C:** 7-Chloro-8-fluoro-2-(methylthio)pyrido[4,3-d]pyrimidine-4-ol

Compound methyl (4,6-dichloro-5-fluoronicotinyl)carbamimidothioate (5.0 g, 17.8 mmol) was dissolved in DMF (40 mL) and heated to 120°C, stirred for 3 h. After the reaction completion was monitored by LCMS, it was cooled to room temperature and water (200 mL) was added. The precipitated solid was filtered and dried to give the product 7-chloro-8-fluoro-2-(methylthio)pyrido[4,3-*d*]pyrimidine-4-ol (3.6 g, yield 82%). LCMS (m/z): 245.6 (M+H).

## Intermediate G

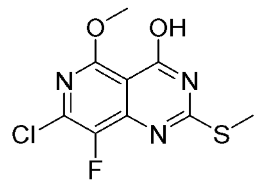

**G**

7-Chloro-8-fluoro-5-methoxy-2-(methylthio)pyrido[4,3-*d*]pyrimidine-4-ol

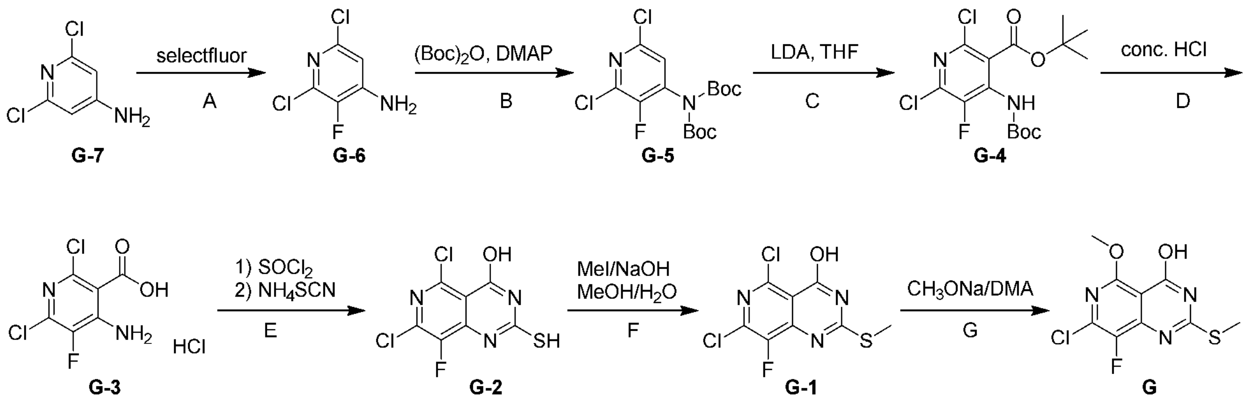


G-7 G-6 G-5 G-4

G-3 G-2 G-1 G

**Step A:** 2,6-Dichloro-3-fluoropyridine-4-amine

At room temperature, selectfluor (68 g, 180 mmol) was added to a solution of 2,6-dichloropyridine-4-amine (25 g,

154 mmol) in methanol/water (V/V = 5:1, 300 mL). The resulting mixture was stirred at 50°C for 48 h, concentrated under reduced pressure, diluted with ethyl acetate, washed sequentially with water and saturated brine, and dried with anhydrous sodium sulfate. The crude product was filtered and concentrated, and purified by FCC ($SiO_2$, EA/PE=0-10%) to obtain 2,6-dichloro-3-fluoropyridine-4-amine (10 g) as a white solid. LCMS (m/z): 180.9 (M+H).

**Step B:** tert-Butyl (tert-butoxycarbonyl) (2,6-dichloro-3-fluoropyridine-4-yl)carbamate

Under stirring at room temperature, 4-dimethylaminopyridine (307 mg, 2.75 mmol) and di-tert-butyl dicarbonate (30 g, 138 mmol) were added to a solution of 2,6-dichloro-3-fluoropyridine-4-amine (10 g, 55 mmol) in tetrahydrofuran (100 mL). The resulting mixture was heated to 60°C and stirred for 16 h. The reaction completion was monitored by TLC, concentrated, and the crude product was obtained and slurried with methanol to give tert-butyl (tert-butoxycarbonyl)(2,6-dichloro-3-fluoropyridine-4-yl)carbamate (16 g) as a white solid. LCMS (m/z): 381.2 (M+H).

**Step C:** tert-Butyl 4-((tert-butoxycarbonyl)amino)-2,6-dichloro-5-fluoronicotinate

Under a dry ice ethanol bath, LDA (2.0 M, 63 mL, 126 mmol) was slowly added to a solution of tert-butyl (tert-butoxycarbonyl) (2,6-dichloro-3-fluoropyridine-4-yl)carbamate (16 g, 42 mmol) in THF (200 mL), and the resulting mixture was stirred at a holding temperature for 1 h. The reaction completion was monitored by TLC, quenched by adding an appropriate amount of acetic acid, diluted with EA, washed with water, and dried over anhydrous sodium sulfate. After filtration, the crude product was purified by FCC ($SiO_2$, EA/PE=0-20%) to obtain tert-butyl 4-((tert-butoxycarbonyl) amino)-2,6-dichloro-5-fluoronicotinate (13g).

**Step D:** 4-Amino-2,6-dichloro-5-fluoronicotinic acid-hydrochloride

At room temperature, concentrated hydrochloric acid (30ml) was added to a solution of tert-butyl 4-((tert-butoxycarbonyl)amino)-2,6-dichloro-5-fluoronicotinate (13g, 34 mmol) in dioxane (90ml). The resulting mixture was stirred for 3 h at room temperature. The reaction completion was monitored by LCMS, concentrated, and obtained 4-amino-2,6-dichloro-5-fluoronicotinic acid-hydrochloride (8 g). LCMS (m/z): 224.9 (M+H).

**Step E:** 5,7-Dichloro-8-fluoro-2-mercaptopyrido[4,3-*d*]pyrimidine-4-ol

A mixture of 4-amino-2,6-dichloro-5-fluoronicotinic acid (8 g, 30.8 mmol) and thionyl chloride (200 mL) was stirred at 50°C for 3 h. The residue was then concentrated and dissolved in acetone (50 mL) to give solution 1. A mixture of ammonium thiocyanate (7 g, 92 mmol) and acetone solution (160 mL) was dropped into solution 1 at room temperature, and the resulting reaction solution was stirred for an additional 1 h at room temperature. After the reaction completion was monitored by LCMS, the reaction solution was poured into water, filtered, and the filter cake was dried to obtain 5,7-dichloro-8-fluoro-2-mercaptopyrido[4,3-*d*]pyrimidine-4(3*H*)-one (5 g). LCMS (m/z): 265.9 (M+H)

**Step F:** 5,7-Dichloro-8-fluoro-2-(methylthio)pyrido[4,3-*d*]pyrimidine-4-ol

At room temperature, a mixture of 5,7-dichloro-8-fluoro-2-mercaptopyrido[4,3-d]pyrimidine-4-ol (5 g, 18.8 mmol), methanol (380 mL), aqueous sodium hydroxide (0.1 M, 380 mL, 380 mmol), and methyl iodide (5.3 g, 380 mmol) was stirred for 2 hrs. After the reaction completion was monitored by LCMS, the reaction solution was poured into 1000 ml water and acidified with concentrated hydrochloric acid to pH ~ 6. The solution was filtered and the filter cake was dried to give the product 5,7-dichloro-8-fluoro-2-(methylthio)pyrido[4,3-*d*]pyrimidine-4-ol (4 g). LCMS (m/z): 279.9 (M+H).

**Step G:** 7-Chloro-8-fluoro-5-methoxy-2-(methylthio)pyrido[4,3-*d*]pyrimidine-4-ol

A mixture of 5,7-dichloro-8-fluoro-2-(methylthio)pyrido[4,3-d]pyrimidine-4-ol (6.0 g, 22 mmol), sodium methoxide (10.3 g, 330 mmol), DMA (100 mL) and methanol (10 mL) was stirred at 50°C for 16 h. After the reaction completion was monitored by LCMS, it was diluted with water, adjusted to pH ~ 3 with concentrated hydrochloric acid, filtered, and the filter cake was collected and dried to give the product 7-chloro-8-fluoro-5-methoxy-2-(methylthio)pyrido[4,3-*d*]pyrimidine-4-ol (5.5 g). LCMS (m/z): 276.0 (M+H).

## Intermediate H

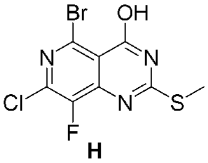
H

5-Bromo-7-chloro-8-fluoro-2-(methylthio)pyrido[4,3-*d*]pyrimidine-4-ol

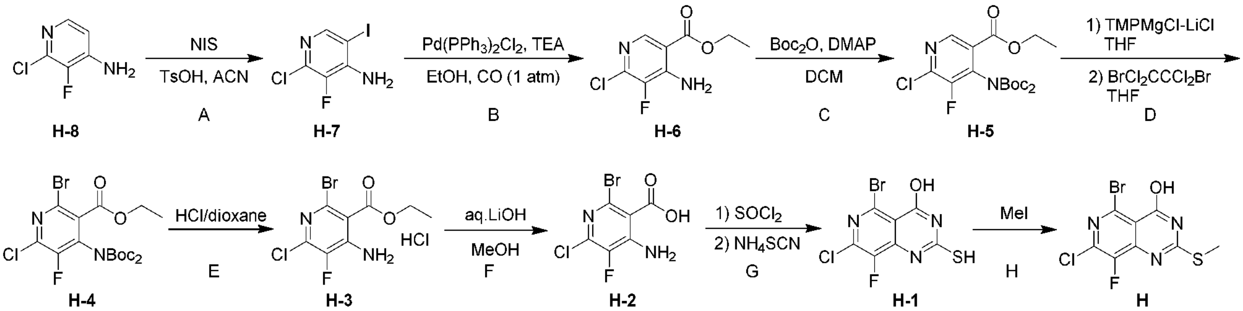

H-8 H-7 H-6 H-5 H-4 H-3 H-2 H-1 H

**Step A:** 2-Chloro-3-fluoro-5-iodopyridine-4-amine

At room temperature, 2-chloro-3-fluoro-4-aminopyridine (25.0 g, 171 mmol) was dissolved into 250 mL acetonitrile. Then NIS (35.4 g, 205 mmol) and p-toluenesulfonic acid monohydrate (3.24 g, 17.1 mmol) were added to the above system, respectively. The resulting mixture was heated to 70°C and the reaction was stirred overnight. After the reaction completion was monitored by LCMS, cooled to room temperature, quenched by pouring the reaction solution into water (500 mL), and extracted with ethyl acetate (800 mL×3). The organic phase was combined and washed with saturated $NaHCO_3$, saturated $Na_2S_2O_3$, salt water, dried with anhydrous sodium sulfate, filtered and concentrated. The crude product was purified by FCC ($SiO_2$, EA/PE = 0-50%) to obtain 2-chloro-3-fluoro-5-iodopyridine-4-amine (42 g, yield 90%) as a white solid. LCMS (m/z): 272.9 (M+H). $^1$H NMR (400 MHz, DMSO-$d_6$) δ 8.10 (s, 1H), 6.69 (s, 2H). $^{19}$F NMR (376 MHz, DMSO-$d_6$) δ -138.90.

**Step B:** Ethyl 4-amino-6-chloro-5-fluoronicotinate

At room temperature, 2-chloro-3-fluoro-5-iodopyridine-4-amine (40g, 147 mmol) in 400 mL of anhydrous ethanol was dissolved. The system was replaced with CO and repeated three times. Later, $Pd(PPh_3)_2Cl_2$ (10.3 g, 14.7 mmol) and TEA (95.0 g, 735 mmol) were added to the reaction flask and replaced with CO gas three times. The resulting mixture was heated to 80°C overnight in a CO gas atmosphere. After the reaction completion was monitored by LCMS, cooled to room temperature, and filtered the reaction solution with diatomite. The filtrate was concentrated to dryness, and the crude product was purified by FCC ($SiO_2$, EA/PE=0-50%) to give ethyl 4-amino-6-chloro-5-fluoronicotinate (30 g, yield 93%) as a white solid. LCMS (m/z): 219.0 (M+H).

**Step C:** Ethyl 4-(bis(tert-butoxycarbonyl)amino)-6-chloro-5-fluoronicotinate

Under stirring at room temperature, $Boc_2O$ (65.9g, 302mmol) was added dropwise to ethyl 4-amino-6-chloro-5-fluoronicotinate (30.0 g, 137 mmol) and DMAP (3.4 g, 27.5 mmol) in anhydrous dichloromethane (600 mL). After completion of dropping, heated to 45°C for reaction overnight. Imidazole (9.33 g, 137 mmol) was added to the system, stirred for half an hour, washed with saturated ammonium chloride solution (300 mL×3), divided the solutions, and the organic phase was then washed with saturated brine (300 mL×2), and the organic phase was dried with anhydrous sodium sulfate, filtered, and concentrated to dryness to obtain a yellow solid ethyl 4-(bis(tert-butoxycarbonyl)amino)-6-chloro-5-fluoronicotinate (45.9 g, yield 80%). LCMS (m/z): 419.1 (M+H).

**Step D:** Ethyl 4-(bis(tert-butoxycarbonyl)amino)-2-bromo-6-chloro-5-fluoronicotinate

Ethyl 4-(bis(tert-butoxycarbonyl)amino)-6-chloro-5-fluoronicotinate (45.0 g, 107 mmol) in anhydrous THF (450 mL) solution in a dry ice acetonitrile bath was cooled to -40°C. A solution of TMPMgCl-LiCl in THF (161 mL, 161 mmol, 1 M)

was added dropwise with stirring at this temperature. After addition, the temperature was maintained and stirred for 4 h, followed by dropwise addition of dibromotetrachloroethane (42.0 g, 129 mmol) in THF (100 mL). Stirring was continued for 4 h at -40°C. 500 mL of saturated ammonium chloride solution was added to quench and extracted with EtOAc (500 mL×3). The organic phase was combined, washed with saturated salt water, dried with anhydrous sodium sulfate, filtered and concentrated, and the crude product was purified by FCC ($SiO_2$, EA/DCM=0-50%) to obtain a white solid ethyl 4-(bis(tert-butoxycarbonyl)amino)-2-bromo-6-chloro-5-fluoronicotinate (17.0 g, yield 32%). LCMS (m/z): 497.0 (M+H).

**Step E:** Ethyl 4-amino-2-bromo-6-chloro-5-fluoronicotinate• HCl

At room temperature, ethyl 4-(bis(tert-butoxycarbonyl)amino)-2-bromo-6-chloro-5-fluoronicotinate (28.0g, 56.3 mmol) was dissolved into 4M hydrochloric acid-dioxane (500 mL). After stirring for 1 h at room temperature, the reaction completion was monitored by LCMS, and the system was concentrated and dried to obtain a crude product ethyl 4-amino-2-bromo-6-chloro-5-fluoronicotinate • HCl (19.1 g, crude product) as a white solid. LCMS (m/z): 296.9 (M+H).

**Step F:** 4-Amino-2-bromo-6-chloro-5-fluoronicotinic acid

At room temperature, 1N NaOH (45.0 mL, 45.0 mmol) was added to a solution of ethyl 4-amino-2-bromo-6-chloro-5-fluoronicotinate • HCl (5.00 g, 15.0 mmol) in MeOH/THF (V:V=1:1, 100 mL). The resulting mixture was stirred at room temperature for 1 h. The reaction completion was monitored by TLC, and the organic solvent was removed by concentration of the reaction solution, and the residue was adjusted to pH = 5-6 with 1 M HCl and extracted with EA (80 mL×3). The organic phase was combined and washed with saturated brine, dried with anhydrous sodium sulfate, filtered and concentrated to obtain 4-amino-2-bromo-6-chloro-5-fluoronicotinic acid (1.90 g, 47% yield) as a white solid product. LCMS (m/z): 269.0 (M+H).

**Step G:** 5-Bromo-7-chloro-8-fluoro-2-mercaptopyrido[4,3-*d*]pyrimidine-4-ol

At room temperature, 4-amino-2-bromo-6-chloro-5-fluoronicotinic acid (1.90 g, 7.05 mmol) was dissolved into dichlorosulfoxide (40 mL, 551 mmol). After the catalytic amount of DMF (2 drops) was added to the reaction system, the reaction was stirred at 80°C for 4 h. The reaction solution was directly concentrated and dried to obtain an intermediate acyl chloride. At room temperature, the resulting acyl chloride was dissolved with anhydrous acetone (50 mL) by stirring, and ammonium thiocyanate (2.15 g, 28.2 mmol) was slowly added. The resulting mixture was stirred at room temperature for 3 h and a large amount of solid was produced. After the reaction, the reaction solution was slowly poured into stirred water (400 mL) to precipitate the solid, stirred for 15 min and then filtered, and the filter cake was dried to obtain 5-bromo-7-chloro-8-fluoro-2-mercaptopyrido[4,3-*d*]pyrimidine-4-ol (1.55 g, yield 71%). LCMS (m/z): 309.8 (M+H).

**Step H:** 5-Bromo-7-chloro-8-fluoro-2-(methylthio)pyrido[4,3-*d*]pyrimidine-4-ol

At room temperature, 0.1M NaOH (100 mL, 10.0 mmol) and methyl iodide (1.42g, 9.98 mmol) were added sequentially to a solution of 5-bromo-7-chloro-8-fluoro-2-mercaptopyrido[4,3-d]pyrimidine-4-ol (1.55g, 4.99 mmol) in methanol (50 mL). The resulting mixture was stirred for 2 h at room temperature. After the reaction, the reaction solution was slowly poured into stirred water (400 mL). Then, 1M HCl was used to adjust pH neutrality, white solid was precipitated, stirred for about 15 min and filtered, and the filter cake was dried to obtain 5-bromo-7-chloro-8-fluoro-2-(methylthio)pyrido [4,3-*d*]pyrimidine-4-ol (1.5g, yield 93%). LCMS (m/z): 323.8 (M+H).

## Intermediate I

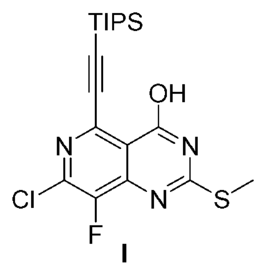

**I**

7-Chloro-8-fluoro-2-(methylthio)-5-((triisopropylsilyl)ethynyl)pyrido[4,3-*d*]pyrimidine-4-ol

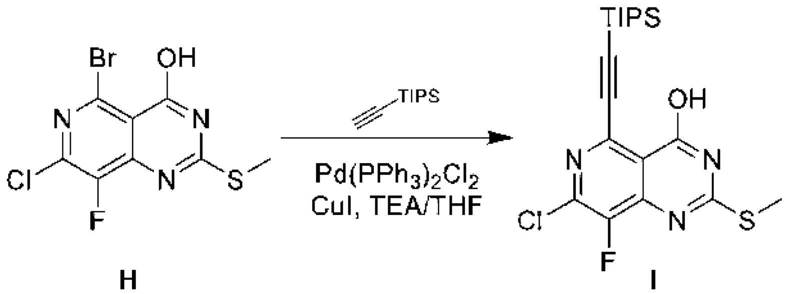


H I

**Step A:** 7-Chloro-8-fluoro-2-(methylthio)-5-((triisopropylsilyl)ethynyl)pyrido[4,3-*d*]pyrimidine-4-ol

At room temperature, 5-bromo-7-chloro-8-fluoro-2-(methylthio)pyrimidine-4-ol (500 mg, 1.54 mmol), $Pd(PPh_3)_2Cl_2$ (216 mg, 0.31 mmol) and CuI (147 mg, 0.77 mmol) were dissolved in 10-mL anhydrous THF at room temperature. The system was replaced with nitrogen three times, triisopropylsilyl acetylene (562 mg, 3.08 mmol) and TEA (779 mg, 7.70 mmol) were added, and nitrogen was degassed three times again. The resulting mixture was heated to 50°C for 16 h. After the reaction completion was monitored by LCMS, cooled to room temperature, and filtered the reaction solution with diatomite. The crude product obtained by concentration and drying of the filtrate was purified by FCC ($SiO_2$, EA/PE=- 50%) to obtain 7-chloro-8-fluoro-2-(methylthio)-5-((triisopropylsilyl)ethynyl)pyrido[4,3-*d*]pyrimidine-4-ol (330 mg, yield 46%) as a white solid. LCMS (m/z): 426.0 (M+H).

## Intermediate J

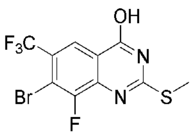

J

7-Bromo-8-fluoro-2-methylthio-6-(trifluoromethyl)quinazoline-4-ol

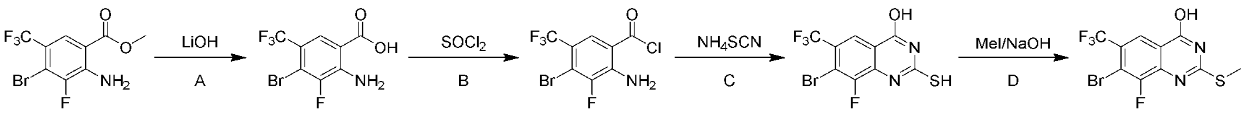


A-3 J-1 J-2 J-3 J

**Step A:** 2-Amino-4-bromo-3-fluoro-5-(trifluoromethyl)benzoic acid

At room temperature, aqueous solution of LiOH (15.17 g, 630 mmol) (100 mL) was added to a mixture of methyl 2-amino-4-bromo-3-fluoro-5-(trifluoromethyl)benzoate (20 g, 63 mmol) and THF (100 mL), MeOH (100 mL) and the resulting mixture was stirred for 16 h at room temperature. After the reaction completion was monitored by TLC and LCMS, 1 M HCl was added to adjust pH ~ 6, EA (100 mL×3) was extracted, the organic phase was collected, washed with saturated salt water, the organic phase was collected, concentrated, and a yellow solid product 2-amino-4-bromo-3-fluoro-5-(trifluoromethyl) benzoic acid (19 g, yield 99%) was obtained. LCMS (m/z): 301.9 (M+H).

**Step B:** 2-Amino-4-bromo-3-fluoro-5-(trifluoromethyl)benzoyl chloride

At room temperature, 2-amino-4-bromo-3-fluoro-5-(trifluoromethyl)benzoic acid (19 g, 63 mmol) was added to $SOCl_2$ (200 mL) and stirred at 50°C for 3 h. The reaction completion was monitored by LCMS and the temperature of the reaction solution returned to room temperature. The reaction solution was concentrated to yield a yellow oily product 2-amino-4-bromo-3-fluoro-5-(trifluoromethyl) benzoyl chloride (20 g, yield 99%). LCMS (m/z): 315.9 (M-Cl+OMe).

**Step C:** 7-Bromo-8-fluoro-2-thiol-6-(trifluoromethyl)quinazoline-4-ol

At room temperature, 2-amino-4-bromo-3-fluoro-5-(trifluoromethyl)benzoyl chloride (20 g, 62.4 mmol) was added to acetone (200 mL), ammonium thiocyanate (14.2 g, 187 mmol) was dissolved in acetone (100 mL), added to previous reaction solution, and stirred for 2 h. The reaction completion was monitored by LCMS. The reaction solution was

slowly poured into stirred water (1000 mL) to produce flocculent insoluble matter, and the filter cake was collected by filtration and dried to obtain a yellow solid 7-bromo-8-fluoro-2-thiol-6-(trifluoromethyl)quinazoline-4-ol (20 g, yield 93%). LCMS (m/z): 342.9 (M+H).

**Step D:** 7-Bromo-8-fluoro-2-methylthio-6-(trifluoromethyl)quinazoline-4-ol

At room temperature, MeI (20.68 g, 146 mmol) was added to a mixture of 7-bromo-8-fluoro-2-thiol-6-(trifluoromethyl)quinazoline-4-ol (20 g, 58.3 mmol), MeOH (200 mL), and 0.1 M NaOH (200 mL) and stirred for 2 hours. After the reaction completion was monitored by LCMS, and then slowly poured into stirred water (1000 mL) to adjust the reaction pH ~ 6 with concentrated hydrochloric acid to produce flocculent insoluble matter, and the filter cake was filtered, collected, and dried to obtain a yellow solid 7-bromo-8-fluoro-2-methylthio-6-(trifluoromethyl)quinazoline-4-ol (20 g, yield 96%). LCMS (m/z): 356.9 (M+H).

## Intermediate K

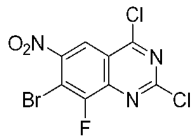

K

7-Bromo-2,4-dichloro-8-fluoro-6-nitroquinazoline

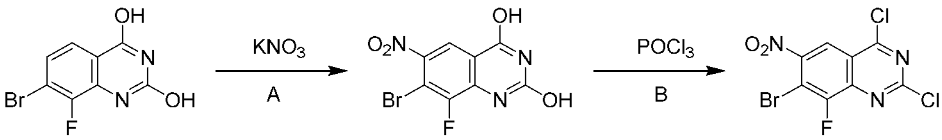


C-1 K-1 K

**Step A:** 7-Bromo-8-fluoro-6-nitroquinazoline-2,4-diol

The 7-Bromo-8-fluoroquinazoline-2,4-diol (11.0 g, 42.5 mmol) was dissolved in concentrated sulfuric acid (60 mL), and potassium nitrate (8.6 g, 84.9 mmol) was added under ice bath conditions. The mixture was stirred for 1 h. The reaction solution was poured into ice water (200 mL), and the precipitated solid was collected by filtration. The solid was washed with water (50 mL) and dried under vacuum to give a yellow solid 7-bromo-8-fluoro-6-nitroquinazoline-2,4-diol (12.0 g, 93% yield). LCMS (m/z): 303.9 (M+H).

**Step B:** 7-Bromo-2,4-dichloro-8-fluoro-6-nitroquinazoline

The 7-Bromo-8-fluoro-6-nitroquinazoline-2,4-diol (10.0 g, 32.9 mmol) was dispersed in toluene (60 mL), and phosphorus oxychloride (30 mL) and DIPEA (10 mL) were added. The mixture was stirred at 90°C for 2 h. The reaction solution was concentrated, and an appropriate amount of acetonitrile (15 mL) was added, followed by pouring into ice water (100 mL). The precipitated solid was collected by filtration, washed with water (50 mL), and dried under vacuum to obtain a yellow solid 7-bromo-2,4-dichloro-8-fluoro-6-nitroquinazoline (10.0 g, yield 89%). LCMS (m/z): 341.9 (M+H).

## Intermediate L

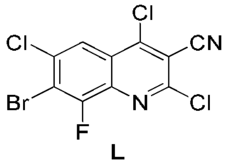

L

7-Bromo-2,4,6-trichloro-8-fluoroquinoline-3-carbonitrile
The synthesis of intermediate L was performed according to reference WO2022095960.

## Intermediate O

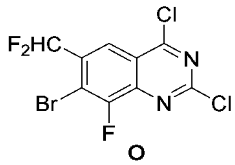

O

7-Bromo-2,4-dichloro-6-(difluoromethyl)-8-fluoroquinazoline

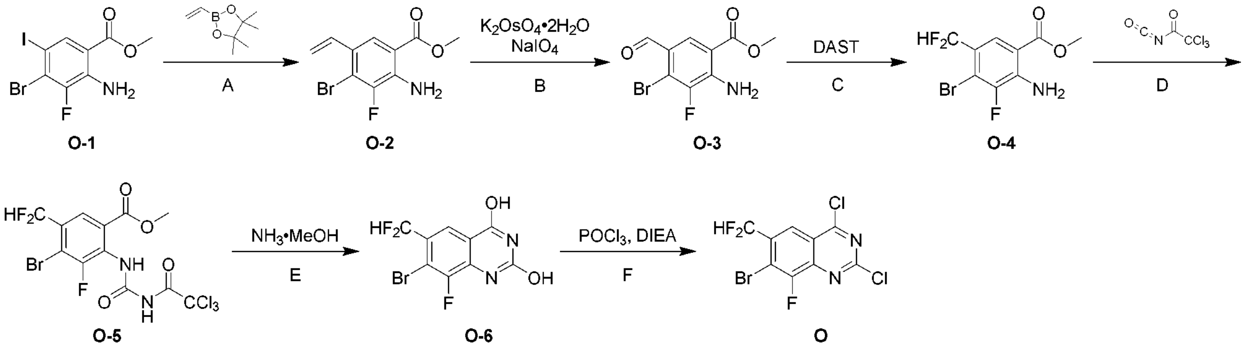


**Step A:** Methyl 2-amino-4-bromo-3-fluoro-5-vinylbenzoate

At room temperature, methyl 2-amino-4-bromo-3-fluoro-5-iodobenzoate (20 g, 53.76 mmol), vinylboronic acid pinacol ester (16.4 g, 107.52 mmol), Pd(dppf)$Cl_2$•DCM (2.2 g, 2.68 mmol), and $K_2CO_3$ (22 g, 161.28 mmol) were added sequentially to DMF (300 mL). After $N_2$ replacement three times, the mixture was warmed to 60°C and stirred for 48 h. After the reaction completion was monitored by LCMS, the reaction solution was returned to room temperature. The palladium catalyst was removed by diatomite filtration. The crude product obtained by concentrating the mother liquor was purified by FCC ($SiO_2$, EA/PE=0-8%) to obtain a yellow solid product, methyl 2-amino-4-bromo-3-fluoro-5-vinylbenzoate (8.5 g, yield 57%). LCMS (m/z): 274.0 (M+H).

**Step B:** Methyl 2-amino-4-bromo-3-fluoro-5-formylbenzoate

At room temperature, methyl 2-amino-4-bromo-3-fluoro-5-vinylbenzoate (3 g, 10.99 mmol), $K_2OsO_4 \cdot 2H_2O$ (1.2 g, 3.3 mmol), and $NaIO_4$ (9.2 g, 43.92 mmol) were added sequentially to THF/$H_2O$ (V/V=1:1, 60 mL) and stirred at room temperature for 16 h. The reaction completion was monitored by LCMS. The reaction solution was filtered and extracted with EA (50 mL×3). The organic phase was washed with saturated brine (50 mL), dried, filtered, and the crude product obtained by concentrating the reaction solution was purified by FCC ($SiO_2$, EA/PE=0-10%) to give methyl 2-amino-4-bromo-3-fluoro-5-formylbenzoate (2.3 g, yield 76%) as a gray solid. LCMS (m/z): 275.9 (M+H).

**Step C:** Methyl 2-amino-4-bromo-5-(difluoromethyl)-3-fluorobenzoate

At 0°C, DAST (1.2 g, 7.25 mmol) was slowly added dropwise to a solution of methyl 2-amino-4-bromo-3-fluoro-5-formylbenzoate (1 g, 3.62 mmol) in DCM (20 mL), and stirred at room temperature for 16 h. The reaction end was monitored by LCMS. DCM (20 mL) was added, and the organic phase was washed with saturated sodium bicarbonate solution (20 mL×2). The reaction solution was dried, and the crude product obtained by concentrating the reaction solution was purified by FCC ($SiO_2$, EA/PE=0-5%) to obtain methyl 2-amino-4-bromo-5-(difluoromethyl)-3-fluorobenzoate (500 mg, yield 46%) as a gray solid. LCMS (m/z): 298.0 (M+H).

**Step D:** Methyl 4-bromo-5-(difluoromethyl)-3-fluoro-2-(3-(2,2,2-trichloroacetyl)ureido)benzoate

At room temperature, methyl 2-amino-4-bromo-5-(difluoromethyl)-3-fluorobenzoate (450 mg, 1.5 mmol) and 2,2,2-trichloro-1-isocyanoethane-1-one (432 mg, 2.25 mmol) were added sequentially to THF (10 mL), and the mixture was stirred at room temperature for 0.5 h. The reaction completion was monitored by LCMS. The reaction solution was concentrated to obtain a yellow solid, methyl 4-bromo-5-(difluoromethyl)-3-fluoro-2-(3-(2,2,2-trichloroacetyl)ureido) benzoate (600 mg, crude product). LCMS (m/z): 484.9 (M+H).

**Step E:** 7-Bromo-6-(difluoromethyl)-8-fluoroquinazoline-2,4-diol

At room temperature, methyl 4-bromo-5-(difluoromethyl)-3-fluoro-2-(3-(2,2,2-trichloroacetyl)ureido)benzoate (600 mg) and $NH_3$/MeOH (1 mL, 7 M) were added sequentially to MeOH (10 mL), and the mixture was stirred at room temperature for 0.5 h. The reaction completion was monitored by LCMS, and the reaction solution was concentrated. The residue was slurried with MeOH (5 mL). The filter cake was collected, dried, and a white solid 7-bromo-6-(difluoromethyl)-8-fluoroquinazoline-2,4-diol (400 mg) was obtained.

**Step F:** 7-Bromo-2,4-Dichloro-6-(difluoromethyl)-8-fluoroquinazoline

Under ice bath conditions, DIEA (1.2 mL, 6.45 mmol) was slowly added dropwise to a mixture of 7-bromo-6-(difluoromethyl)-8-fluoroquinazoline-2,4-diol (400 mg, 1.29 mmol) and $POCl_3$ (3 mL, 30.32 mmol). The mixture was heated to 105°C and stirred for 1 h. The reaction completion was monitored by LCMS. The reaction solution was concentrated, and the residue was slowly poured into $H_2O$ (20 mL). Flocculent matter was formed. The mixture was filtered, and the filter cake was collected and dried in a vacuum drying oven to obtain a yellow solid, 7-bromo-2,4-dichloro-6-(difluoromethyl)-8-fluoroquinazoline (350 mg, yield 78%).

## Intermediate P

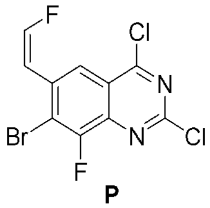

**P**

(*Z*)-7-Bromo-2,4-dichloro-8-fluoro-6-(2-fluorovinyl)quinazoline

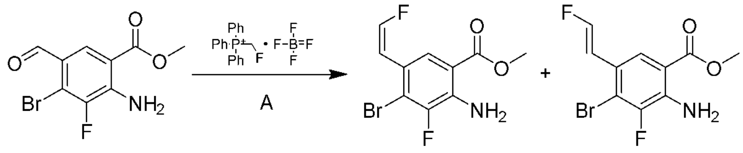


**O-3** **P-1** **Q-1**

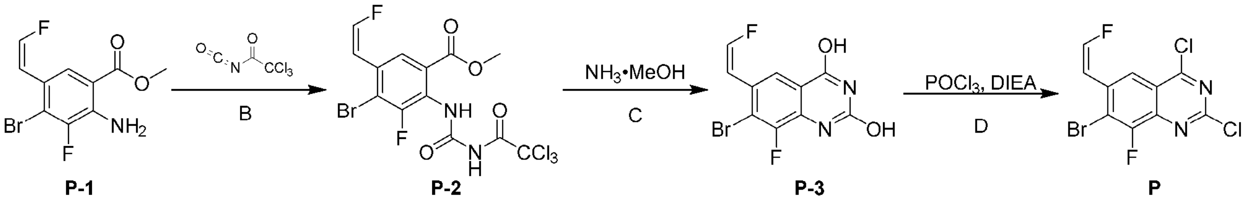


**P-1** **P-2** **P-3** **P**

**Step A:** Methyl (*Z*)-2-amino-4-bromo-3-fluoro-5-(2-fluorovinyl)benzoate (**P-1**) and Methyl (E)-2-amino-4-bromo-3-fluoro-5-(2-fluorovinyl)benzoate (**Q-1**)

(Fluoromethyl)triphenylphosphine tetrafluoroborate (2.49 g, 6.525 mmol) was dissolved in THF (15 mL), cooled to -78°C, and potassium tert-butoxide (1 M THF solution, 6.53 mL, 6.53 mmol) was added. After stirring for 1 h, methyl 2-amino-4-bromo-3-fluoro-5-formylbenzoate (1.2 g, 4.35 mmol) was added, slowly returned to room temperature, and continued stirring for 1 h. The reaction completion was monitored by TLC and LCMS. Saturated $NH_4Cl$ (200 mL) and EA (80 mL×3) were added for extraction. The organic phase was collected, washed with saturated brine, and dried over anhydrous sodium sulfate. The crude product was filtered and concentrated under reduced pressure. It was then purified by FCC ($SiO_2$, EA/PE=3%) to give a white solid methyl (*Z*)-2-amino-4-bromo-3-fluoro-5-(2-fluorovinyl)benzoate (390 mg, yield 31%). LCMS (m/z): 294.0 (M+H). $^1$H NMR (400 MHz, $CDCl_3$) δ 8.16 (t, J = 1.6 Hz, 1H), 6.70 (dd, $J_1$ = 82.8 Hz, $J_2$ = 5.6 Hz, 1H), 5.88 (dd, $J_1$ = 43.2 Hz, $J_2$ = 5.6 Hz, 1H), 3.90 (s, 3H) and a white solid methyl (*E*)-2-amino-4-bromo-3-fluoro-5-(2-fluorovinyl)benzoate (612 mg, yield 48%). LCMS (m/z): 294.0 (M+H). $^1$H NMR (400 MHz, $CDCl_3$) δ 7.64 (d, J = 2.0 Hz, 1H), 7.03 (dd, $J_1$ = 82.8 Hz, $J_2$ = 11.2 Hz, 1H), 6.55 (dd, $J_1$ = 18.0 Hz, $J_2$ = 11.2 Hz, 1H), 3.90 (s, 3H).

**Step B:** Methyl (*Z*)-4-bromo-3-fluoro-5-(2-fluorovinyl)-2-(3-(2,2,2-trichloroacetyl)ureido)benzoate

Methyl (*Z*)-2-amino-4-bromo-3-fluoro-5-(2-fluorovinyl)benzoate (390 mg, 1.335 mmol) was dissolved in THF (6.7 mL) and cooled to 0°C. Trichloroacetyl isocyanate (238 μL, 2 mmol) was added, and the mixture was stirred for 10 min, then slowly raised to room temperature and stirred for another 30 min. The reaction completion was monitored by LCMS. The reaction solution was concentrated under reduced pressure to obtain a white solid, which was directly used in the next step. LCMS (m/z): 502.9 (M+Na).

**Step C:** (*Z*)-7-bromo-8-fluoro-6-(2-fluorovinyl)quinazoline-2,4-diol

MeOH (6.7 mL) was added to the crude product obtained in **Step B,** resulting in a white suspension. $NH_3$ (7 N MeOH solution, 1.34 mL, 9.38 mmol) was added at room temperature. The resulting mixture was stirred for 30 min to obtain a pale yellow clear solution, and stirring was continued for 2 h. A large amount of white solid was precipitated, and the reaction completion was monitored by LCMS. Filtered and dried the filter cake to give a white solid product (*Z*)-7-bromo-8-fluoro-6-(2-fluorovinyl)quinazoline-2,4-diol (404 mg, yield 99%). LCMS (m/z): 304.9 (M+H).

**Step D:** (*Z*)-7-Bromo-2,4-dichloro-8-fluoro-6-(2-fluorovinyl)quinazoline

(*Z*)-7-Bromo-8-fluoro-6-(2-fluorovinyl)quinazoline-2,4-diol (404 mg, 1.33 mmol) in $POCl_3$ (3 mL, 32.16 mmol) was dissolved and cooled to 0°C. DIEA (1.9 mL, 10.72 mmol) was added, stirred for 10 min, and then refluxed in an oil bath at 110°C for 4 h. The reaction completion was monitored by LCMS. The reaction solution was concentrated under reduced pressure and extracted with saturated $NaHCO_3$ (100 mL) and EA (80 mL×3). The organic phase was collected, washed with saturated brine, dried, and concentrated under reduced pressure. The crude product was purified by FCC ($SiO_2$, EA/PE=8%) to give a white solid (*Z*)-7-bromo-2,4-dichloro-8-fluoro-6-(2-fluorovinyl)quinazoline (301 mg, yield 67%). LCMS (m/z): 333.0 (M-2Cl+2MeO+H).

## Intermediate Q

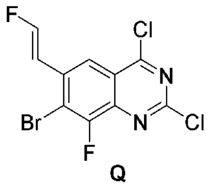

**Q**

(*Z*)-7-Bromo-2,4-dichloro-8-fluoro-6-(2-fluorovinyl)quinazoline

The synthesis of intermediate Q follows that of intermediate P, except that in step B, methyl (*E*)-2-amino-4-bromo-3-fluoro-5-(2-fluorovinyl)benzoate was used instead of methyl (*Z*)-2-amino-4-bromo-3-fluoro-5-(2-fluorovinyl)benzoate.

## Intermediate T

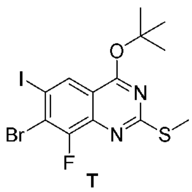

**T**

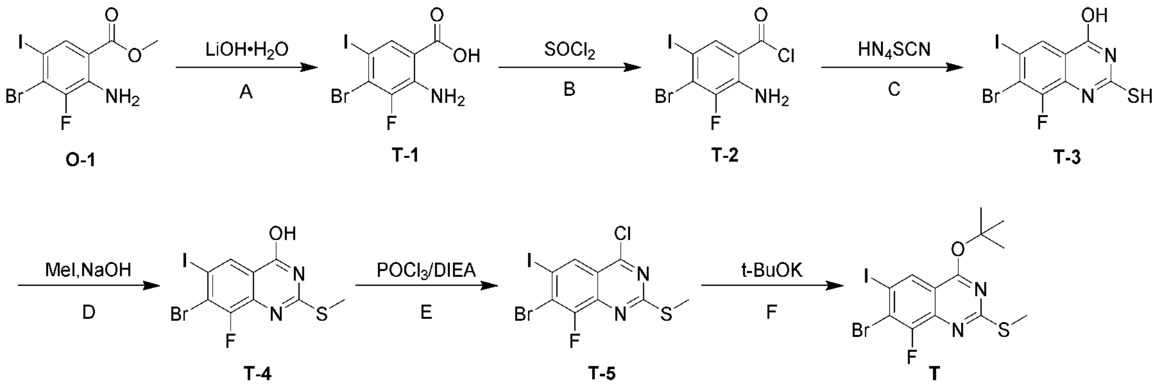


O-1 T-1 T-2 T-3

T-4 T-5 T

**Step A:** Compound **T-1**

At room temperature, LiOH·$H_2O$ (67.7 g, 1612.9 mmol) was added to a mixture of 2-amino-4-bromo-3-fluoro-5-methyl iodobenzoate (**O-1,** 60 g, 161.3 mmol) and THF (200 mL), MeOH (200 mL), and $H_2O$ (200 mL). The mixture was stirred at room temperature for 16 h. The reaction completion was monitored by TLC and LCMS. Then, 1M HCl was added to adjust the pH to 6, and the mixture was extracted with EA (300 mL×3). The organic phase was collected, washed with saturated brine, and the organic phase was collected again, and concentrated. The resulting yellow solid product **T-1** (56 g, 97% yield) was obtained. LCMS (m/z): 359.9 (M+H).

**Step B:** Compound **T-2**

At room temperature, compound **T-1** (56 g, 155.6 mmol) was added to $SOCl_2$ (200 mL), and the mixture was warmed to 70°C and stirred for 3 h. The reaction completion was monitored by LCMS, and the reaction solution was allowed to return to room temperature. The reaction solution was concentrated to obtain a yellow oily substance **T-2** (57.5 g, 98% yield). LCMS (m/z): 374.9 (M-Cl+OMe).

**Step C:** Compound **T-3**

At room temperature, compound **T-2** (57.5 g, 153.2 mmol) was added to acetone (500 mL). Ammonium thiocyanate (34.9 g, 459.7 mmol) was dissolved in acetone (500 mL) and added to the above reaction solution. The mixture was stirred for 2 h. The reaction completion was monitored by LCMS. The system was slowly poured into stirred water (1000 mL). Flocculent insoluble matter was formed. The filter cake was collected, dried, and a yellow solid compound **T-3** (60 g, 98% yield) was obtained. LCMS (m/z): 400.8 (M+H).

**Step D:** Compound **T-4**

At room temperature, MeI (63.9 g, 450 mmol) was added to a mixed solution of compound **T-3** (60 g, 150 mmol), MeOH (400 mL), and NaOH (24 g, 600 mmol), and stirred for 2 h. The reaction completion was monitored by LCMS. Then, water (1000 mL) was slowly poured into the mixture, and the pH was adjusted to 6 with concentrated hydrochloric acid. A flocculent insoluble substance was formed. The filter cake was collected, dried, and a yellow solid compound **T-4** (61 g, 98% yield) was obtained. LCMS (m/z): 414.8 (M+H).

**Step E:** Compound **T-5**

At 0°C, DIEA (57.3 g, 442.1 mmol) was slowly added dropwise to a mixed solution of $POCl_3$ (530.3 g, 3462.5 mmol) and compound **T-4** (61 g, 147.3 mmol), and the mixture was warmed to 110°C and stirred for 1 h. The reaction completion was monitored by LCMS. The reaction solution was concentrated, and the residue was slowly added to ice water. A flocculent insoluble substance was formed; the filter cake was collected, dried, and a yellow solid compound **T-5** (60 g, 94% yield) was obtained. LCMS (m/z): 434.8 (M+H).

**Step F:** Intermediate **T**

At 0°C, *t*-BuOK (152.7 mL, 1 M THF solution, 152.7 mmol) was added to a mixed solution of compound **T-5** (60 g, 138.8 mmol) and THF (1000 mL), and stirred at 0°C for 1 h, then heated to room temperature and stirred for another 1 h. The

reaction completion was monitored by LCMS and TLC. The reaction solution was poured into a semi-saturated solution of $NH_4Cl$ (200 mL), extracted with EA (500 mL×3), and the organic phase was collected, washed with saturated brine (300 mL), dried over anhydrous $Na_2SO_4$, and concentrated. The organic phase was purified by FCC ($SiO_2$, THF/PE = 0-5%) to give a yellow solid intermediate **T** (45 g, yield 69.2%). LCMS (m/z): 414.9 (M-56+H).

## Intermediate n-1

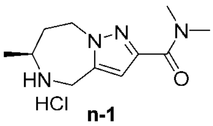

**n-1**

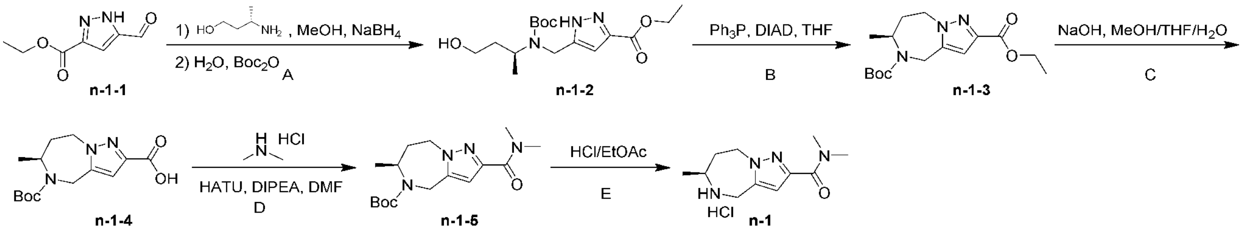


n-1-1 n-1-2 n-1-3 n-1-4 n-1-5 n-1

**Step A:** Compound **n-1-2**

At room temperature, (*S*)-3-aminobutanol (19.9 g, 223 mmol) was added to a solution of ethyl 5-formylpyrazole-3-carboxylate (**n-1-1**, 25.0 g, 149 mmol) and anhydrous methanol (250 mL), and stirred for 30 min. The reaction mixture was cooled to 0-5°C with ice water, and sodium borohydride (11.3 g, 297 mmol) was slowly added to the reaction system while maintaining the internal temperature below 20°C. After the addition was complete, the mixture was stirred at room temperature for 2 h. The reaction completion was monitored by LCMS. An equal volume of water (250 mL) was added, and $Boc_2O$ (64.9 g, 297 mmol) was added to the reaction system in one portion at room temperature, and the mixture was stirred overnight at room temperature. After the reaction completion was monitored by LCMS, the reaction solution was poured into $H_2O$ (500 mL) and extracted with EtOAc (500 mL×3). The organic phases were combined, dried over anhydrous $Na_2SO_4$, and filtered. The filtrate was concentrated to dryness, and the crude product was purified by FCC ($SiO_2$, EA/PE=0-100%) to give a colorless oily product **n-1-2** (26.2 g, yield 52%). LCMS (m/z): 342.4 (M+H).

**Step B:** Compound **n-1-3**

Under an ice-water bath, DIAD (31.0 g, 153 mmol) was added dropwise to a mixture of compound **n-1-2** (26.2 g, 76.4 mmol), triphenylphosphine (38.1 g, 153 mmol), and anhydrous THF (500 mL). The mixture was allowed to warm naturally to room temperature and stirred overnight. After the reaction completion was monitored by LCMS, the reaction solution was poured into $H_2O$ (400 mL) and extracted with EtOAc (400 mL×3). The organic phases were combined, dried over anhydrous $Na_2SO_4$, and filtered. The filtrate was concentrated to dryness, and the crude product was purified by FCC ($SiO_2$, EA/PE = 0-100%) to give a colorless oily product **n-1-3** (18.0 g, yield 73%). LCMS (m/z): 324.1 (M+H).

**Step C:** Compound **n-1-4**

Under ice bath conditions, NaOH (11.3 g, 278 mmol) was added to a solution of compound **n-1-3** (18.0 g, 55.7 mmol) and MeOH/THF/$H_2O$ (450 mL, 1:1:1), and the mixture was stirred at room temperature for 1 h. After the reaction completion monitored by LCMS, the organic solution in the reaction solution was concentrated and removed. The remaining aqueous phase was cooled to 0-10°C in an ice-water bath, and the pH was adjusted to 3-4 with 2N HCl, and then extracted with EtOAc (400 mL×5). The organic phases were combined, dried over anhydrous $Na_2SO_4$, and filtered. The filtrate was concentrated to dryness to give a white solid crude compound **n-1-4** (16 g, 97% yield). LCMS (m/z): 296.1 (M+H).

**Step D:** Compound **n-1-5**

At room temperature, HATU (34.7 g, 91.4 mmol) was added to a mixture of compound **n-1-4** (18.0 g, 60.9 mmol), dimethylamine hydrochloride (9.82 g, 122 mmol), DIPEA (39.4 g, 305 mmol), and DMF (250 mL). The mixture was stirred at room temperature for 1 h. After the reaction completion monitored by LCMS, the reaction solution was poured into a LiCl aqueous solution (5wt%, 500mL), and then extracted with EtOAc (400mL×3). The organic phases were combined, dried

over anhydrous $Na_2SO_4$, and filtered. The filtrate was concentrated to dryness, and the crude product was purified by FCC ($SiO_2$, EA/PE = 0-100%) to give a white product **n-1-5** (12.4g, yield 63%). LCMS (m/z): 323.2 (M+H). $^1$H NMR (400 MHz, DMSO-$d_6$) δ 6.39 (s, 1H), 4.88 - 4.53 (m, 1H), 4.51 - 4.11 (m, 3H), 4.09 - 3.93 (m, 1H), 3.23 (s, 3H), 2.94 (s, 3H), 2.18 - 1.93 (m, 2H), 1.45 - 1.24 (m, 9H), 1.20 (d, *J* = 6.6 Hz, 3H).

**Step E:** Intermediate **n-1**

At room temperature, 4M HCl-ethyl acetate (200 mL, 800 mmol) was added to compound **n-1-5** (12.4 g, 60.9 mmol), and the mixture was stirred at room temperature for 1 h. The reaction completion was monitored by LCMS. The acid solution was concentrated to obtain an oily intermediate, **n-1** (10.0 g, 100% yield). LCMS (m/z): 223.1 (M+H).

## Intermediate n-1E

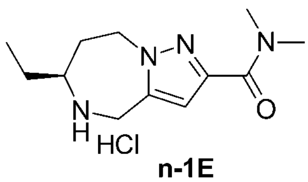

**n-1E**

Intermediate **n-1E** was synthesized according to the synthesis scheme of intermediate **n-1,** except that (S)-3-amino-1-pentanol was used instead of (S)-3-aminobutanol in step A. LCMS (m/z): 237.1 (M+H).

## Intermediate n-1-d6

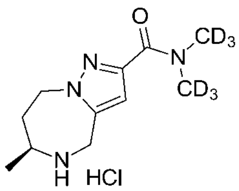

**n-1-d6**

Intermediate **n-1-d6** was prepared according to the synthesis of **n-1,** except that deuterated dimethylamine hydrochloride was used instead of dimethylamine hydrochloride in step D. LCMS (m/z): 229.3 (M+H).

## Intermediate n-2

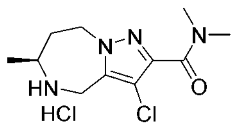

**n-2**

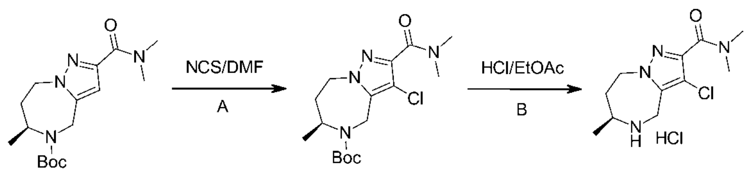


n-1-5 n-2-1 n-2

**Step A:** Compound **n-2-1**

At room temperature, NCS (311 mg, 2.33 mmol) was added to a solution of compound **n-1-5** (500 mg, 1.55 mmol) and anhydrous DMF (5 mL), and the mixture was heated to 40-50°C and stirred for 3 h. The reaction completion was monitored by LCMS. The reaction solution was poured into 5% LiCl aqueous solution (50 mL) and extracted with EtOAc (40 mLx3). The organic phases were combined, dried over anhydrous $Na_2SO_4$, and filtered. The filtrate was concentrated to

dryness, and the crude product was purified by FCC ($SiO_2$, EA/PE = 0-100%) to give a colorless oily product **n-2-1** (370 mg, yield 67%). LCMS (m/z): 357.1 (M+H).

**Step B:** Intermediate **n-2**

At room temperature, 4M HCl-ethyl acetate (20 mL, 80 mmol) was added to compound **n-2-1** (370 mg, 1.04 mmol), and the mixture was stirred at room temperature for 1 h. The reaction completion was monitored by LCMS. The acid solution was removed by concentration to yield a semi-solid oily intermediate **n-2** (300 mg). LCMS (m/z): 257.3 (M+H).

## Intermediate n-3

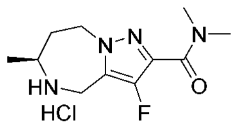

**n-3**

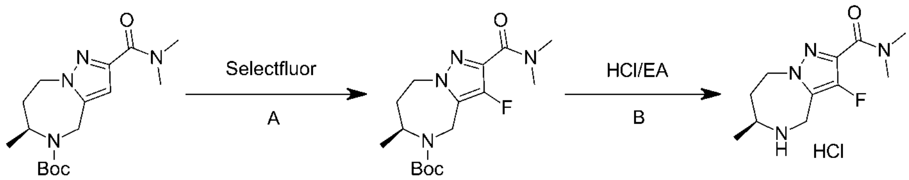


**n-1-5** **n-3-1** **n-3**

**Step A:** Compound **n-3-1**

At room temperature, 1-chloromethyl-4-fluoro-1,4-diazabicyclo[2.2.2]octane di(tetrafluoroborate) salt (2.26 g, 6.4 mmol) was added to a solution of compound **n-1-5** (270 mg, 0.8 mmol) in acetonitrile (15 mL). The mixture was warmed to 40°C and stirred for 24 h. After the reaction completion was monitored by LCMS, the reaction solution was filtered through diatomite, and the filtrate was concentrated to dryness. The crude product was purified by FCC ($SiO_2$, THF/PE=0-50%) to give a pale yellow oily product **n-3-1** (60 mg, yield 21%). LCMS (m/z): 341.2 (M+H).

**Step B:** Intermediate **n-3**

At room temperature, 2 mL of 4N HCl/EA solution was added to compound **n-3-1** (60 mg, 0.17 mmol). The resulting reaction solution was stirred for 1 h. After the reaction completion was monitored by LCMS, the reaction solution was concentrated to dryness to give a yellow oily crude product intermediate **n-3** (62 mg). LCMS (m/z): 241.1 (M+H).

## Intermediate n-4

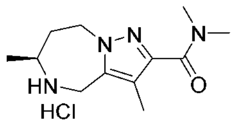

**n-4**

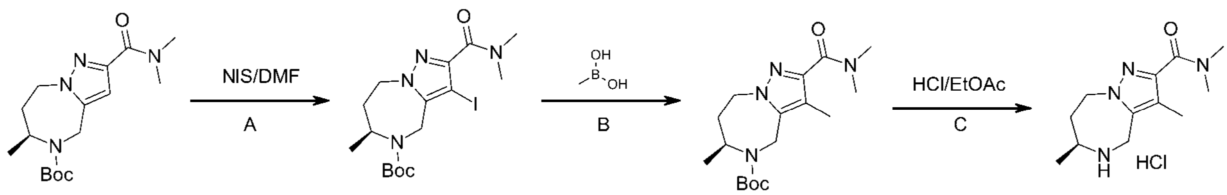


**n-1-5** **n-4-1** **n-4-2** **n-4**

**Step A:** Compound **n-4-1**

At room temperature, NIS (1.40 g, 6.20 mmol) was added to a solution of compound **n-1-5** (1.00 g, 3.10 mmol)

and anhydrous DMF (10 mL), and stirred at room temperature for 16 h. The reaction completion was monitored by LCMS. The reaction solution was poured into a 5% LiCl aqueous solution (50 mL) and extracted with EtOAc (50 mL×3). The organic phases were combined and washed with saturated sodium thiosulfate (50 mL). Finally, the mixture was dried over anhydrous $Na_2SO_4$ and filtered. The filtrate was concentrated to dryness, and the crude product was purified by FCC ($SiO_2$, EA/PE = 0-100%) to give a colorless oily product **n-4-1** (750 mg, yield 54%). LCMS (m/z): 449.0 (M+H).

**Step B:** Compound **n-4-2**

At room temperature, compound **n-4-1** (300 mg, 0.669 mmol), methylboric acid (80.1 mg, 1.34 mmol), cataCXium-A-Pd-$G_3$ (48.7 mg, 0.067 mmol), and $K_3PO_4$ (426 mg, 2.01 mmol) were added sequentially to a mixed solution of 1,4-dioxane and water (5 mL, V/V = 5:1). After $N_2$ replacement three times, the mixture was warmed to 80°C and stirred overnight. After the reaction completion was monitored by LCMS, the reaction was cooled to room temperature and concentrated to dryness. The crude product was purified by FCC ($SiO_2$, EA/PE = 0-100%) to give a white solid product **n-4-2** (185 mg, yield 82%). LCMS (m/z): 337.2 (M+H).

**Step C:** Intermediate **n-4**

At room temperature, 4M HCl-ethyl acetate (20 mL, 80 mmol) was added to compound **n-4-2** (185 mg, 0.55 mmol), and the mixture was stirred at room temperature for 1 h. The reaction completion was monitored by LCMS. The acid solution was removed by concentration to obtain a semi-solid oily intermediate, **n-4** (150 mg). LCMS (m/z): 237.1 (M+H).

## Intermediate n-5

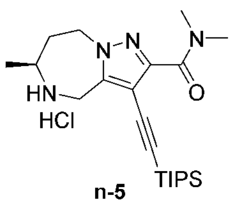

**n-5**

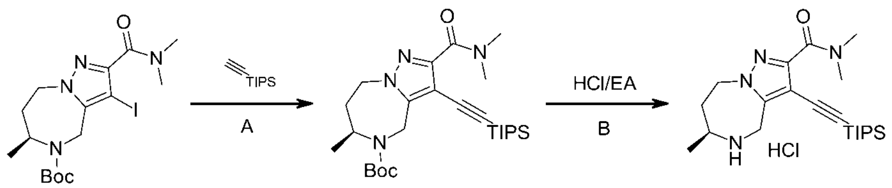


**n-4-1** **n-5-1** **n-5**

**Step A:** Compound **n-5-1**

At room temperature, compound **n-4-1** (200 mg, 0.446 mmol), triisopropylsilylacetylene (162 mg, 0.890 mmol), bis(triphenylphosphine)palladium dichloride (31 mg, 0.044 mmol), CuI (10 mg, 0.052 mmol), and TEA (225 mg, 2.28 mmol) were added sequentially to tetrahydrofuran (5 mL). After $N_2$ replacement three times, the mixture was warmed to 50°C and stirred overnight. After the reaction completion was monitored by LCMS, the reaction mixture was cooled to room temperature and concentrated to dryness. The crude product was purified by FCC ($SiO_2$, EA/PE = 0-100%) to give a white solid product **n-5-1** (50 mg, yield 22%). LCMS (m/z): 503.2 (M+H).

**Step B:** Intermediate **n-5**

At room temperature, 4M HCl-ethyl acetate (10 mL, 40 mmol) was added to compound **n-4-1** (50 mg, 0.099 mmol), and the mixture was stirred at room temperature for 1 h. The reaction completion was monitored by LCMS. The acid solution was removed by concentration to obtain a semi-solid oily intermediate, **n-5** (40 mg). LCMS (m/z): 403.2 (M+H).

## Intermediates n-6A and n-6B

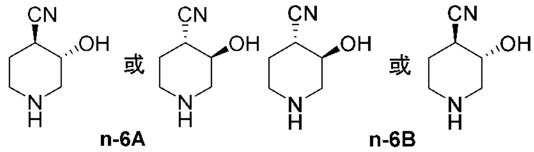
或 或

n-6A n-6B

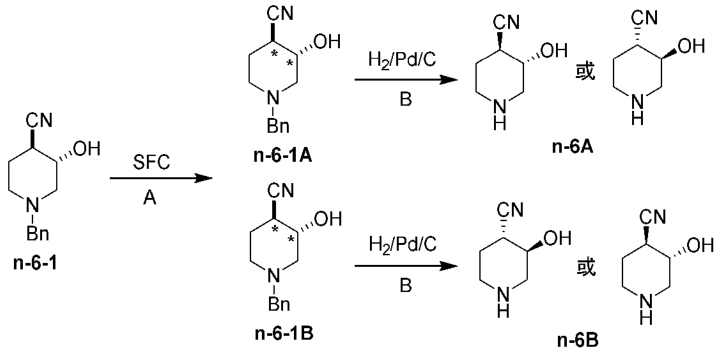


n-6-1 n-6-1A n-6A n-6-1B n-6B

**Step A:** Compounds **n-6-1A** and **n-6-1B**

Compound **n-6-1** (2.10 g) was resolved by SFC (Waters SFC 150, column: DAICELCHIRALPAK®AD, 250*30mm 10 $\mu$m; mobile phase: $CO_2$/MeOH (+0.1% 7.0mol/l $NH_3$-MeOH) = 70/30; flow rate: 120mL/min). The first eluted isomer 1 was compound **n-6-1A** (1.04 g, relatively short retention time), chiral analysis method SFC-n-6, Rt=1.184, LCMS (m/z): 217.1 (M+H); the subsequent eluted isomer 2 was compound **n-6-1B** (1.05 g, relatively long retention time). Chiral analysis method SFC-n-6, Rt=2.250, LCMS (m/z): 217.1 (M+H).

Chiral analysis method SFC-n-6: Waters UPCC, analytical column: Daicel Chiralpak®AD, 100*3mm 3$\mu$m; mobile phase A: CO2, mobile phase B: MeOH (+0.1% DEA); flow rate: 1.5 mL/min; column temperature: 35°C; back pressure: 1800 psi; gradient: 0-5.0 min A/B=80/20.

**Step B:** Intermediate **n-6A**

At room temperature, compound **n-6-1A** (1.04 g, 48.1 mmol) and Pd/C (10 wt%, 1 g, 9.6 mmol) were added sequentially to MeOH (10 mL) solution. After $H_2$ balloon replacement three times, the mixture was stirred at room temperature for 16 h. The reaction completion was monitored by LCMS. The reaction solution was filtered through diatomite to remove the palladium catalyst, and the mother liquor was collected. The organic liquid was concentrated to give intermediate **n-6A** (2.4 g, yield 20.7%). LCMS (m/z): 127.0 (M+H).

Intermediate **n-6B** was prepared from compound **n-6-1B** using the same method.

## Intermediates n-7A and n-7B

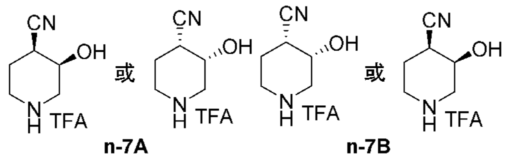
或 或

n-7A n-7B

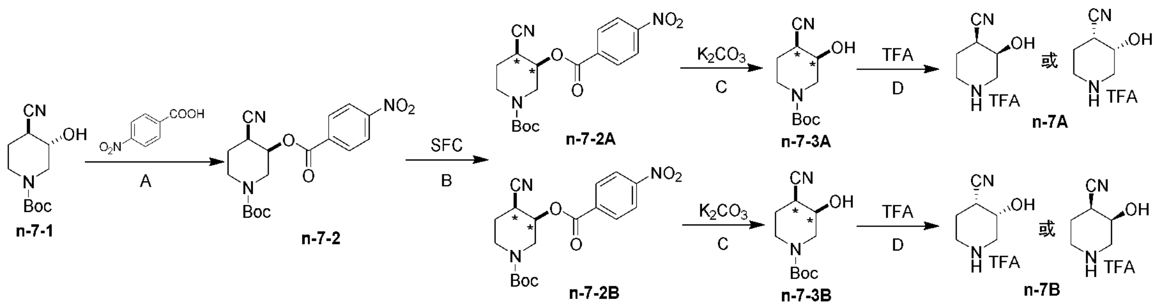


n-7-1 n-7-2 n-7-2A n-7-3A n-7A n-7-2B n-7-3B n-7B

**Step A:** Compound **n-7-2**

At room temperature, compound **n-7-1** (7.0 g, 30.9 mmol), p-nitrobenzoic acid (5.2 g, 30.9 mmol), $PPh_3$ (9.7 g, 37.1 mmol), and DIAD (7.5 g, 37.1 mmol) were added sequentially to THF (100 mL) solution. After $N_2$ replacement three times, the mixture was stirred at room temperature for 16 h. The reaction completion was monitored by LCMS and TLC. The reaction solution was added to water (200 mL), extracted with EA (100 mL×3), and the collected organic phase was washed with saturated brine (100 mL) and dried over anhydrous $Na_2SO_4$. The crude product obtained by concentrating the organic liquid was purified by FCC ($SiO_2$, DCM/PE = 0-100%) to give a white solid compound **n-7-2** (2.4 g, yield 20.7%). LCMS (m/z): 398.0 (M+Na).

**Step B:** Compounds **n-7-2A** and **n-7-2B**

Compound **n-7-2** (2.4 g) was resolved by SFC (Waters SFC 150, column: DAICELCHIRALPAK®AD, 250*30mm 10 μm; mobile phase: $CO_2$/MeOH (+0.1% 7.0mol/l $NH_3$-MeOH) = 75/25; flow rate: 120mL/min). The first eluted isomer 1 was compound **n-7-2A** (1.25 g, relatively short retention time), chiral analysis method SFC-n-7, Rt=0.861; the subsequent eluted isomer 2 was compound **n-7-2B** (1.15 g, relatively short retention time), chiral analysis method SFC-n-7, Rt=1.883.

Chiral analysis method SFC-n-7: Waters UPCC, analytical column: Daicel Chiralpak®AD, 100*3mm 3μm; mobile phase A: CO2, mobile phase B: MeOH (+0.1% DEA); flow rate: 1.5 mL/min; column temperature: 35°C; back pressure: 1800 psi; gradient: 0-5.0 min A/B=80/20.

**Step C:** Compound **n-7-3A**

At room temperature, compound **n-7-2A** (200 mg, 0.53 mmol) and $K_2CO_3$ (147 mg, 1.07 mmol) were added to MeOH (5 mL) solution and stirred for 20 h. The reaction completion was monitored by LCMS and TLC. The reaction solution was poured into $H_2O$ (20 mL), extracted with EA (20 mL×3), and the organic phase was collected, washed with saturated brine (20 mL), and dried over anhydrous $Na_2SO_4$. The crude product obtained by concentrating the organic phase was purified by FCC ($SiO_2$, EA/PE=0-50%) to give a white solid product, compound **n-7-2A** (120 mg, yield 99.5%). LCMS (m/z): 227.3 (M+H).

The same method was used to prepare **n-7-3B** from compound **n-7-2B.**

**Step D:** Intermediate **n-7A**

At room temperature, compound **n-7-3A** (120 mg, 0.53 mmol) was added to a mixed solution of DCM (3 mL) and TFA (1 mL) and stirred for 1 h. The reaction completion was monitored by LCMS. The reaction solution was concentrated and poured into saturated $NaHCO_3$ (10 mL), extracted with EA (10 mL×3), and the organic phase was collected. The mixture was washed with saturated brine (20 mL), dried over anhydrous $Na_2SO_4$, and the organic phase was concentrated to give intermediate **n-7A** (90 mg, yield 70.6%). LCMS (m/z): 127.0 (M+H).

Intermediate **n-7B** was prepared from compound **n-7-3B** using the same method.

## Intermediate n-8

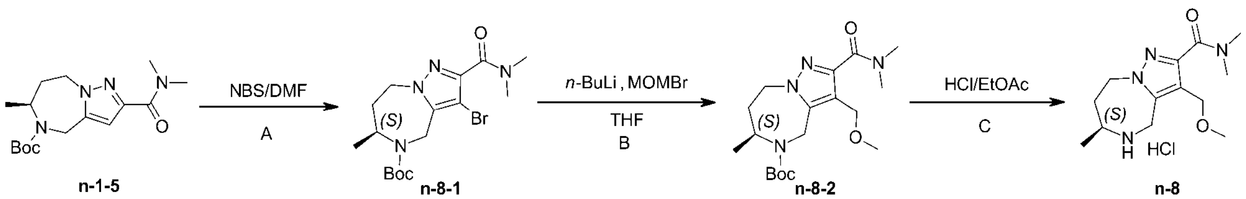


n-1-5 n-8-1 n-8-2 n-8

**Step A:** Compound **n-8-1**

At room temperature, NBS (5.0 g, 27.9 mmol) was added to a mixture of compound **n-1-5** (6.0 g, 18.6 mmol) and DMF (60 mL), and the mixture was stirred at room temperature for 3 h. The reaction completion was monitored by LCMS. The reaction solution was poured into 5% LiCl aqueous solution (200 mL) and extracted with EtOAc (200 mL×3). The organic phases were combined, dried over anhydrous $Na_2SO_4$, and filtered. The filtrate was concentrated to dryness, and the crude product was purified by FCC ($SiO_2$, EA/PE = 0-100%), lyophilized, and the white solid compound **n-8-1** (7.0 g, yield 94%) was obtained. LCMS (m/z): 401.1/403.1 (M+H). $^1$H NMR (400 MHz, DMSO-$d_6$) δ 4.68-4.58 (m, 1H), 4.45-4.39 (m, 2H), 4.31-4.20 (m, 1H), 4.09-3.90 (m, 1H), 2.96 (s, 3H), 2.95 (s, 3H), 2.18-2.05 (m, 2H), 1.33 (s, 9H), 1.19 (d, $J$ = 6.4 Hz,

3H).

**Step B:** Compound **n-8-2**

At room temperature, compound **n-8-1** (1 g, 2.5 mmol) was dissolved in anhydrous tetrahydrofuran (15 mL), cooled in a dry ice-ethanol bath for 10 min, and n-butyllithium (1.6 M, 2.4 mL, 3.75 mmol) was slowly added dropwise to the reaction system. After the addition was complete, the mixture was stirred for another 10 min in a dry ice-ethanol bath. Bromomethyl ether (468 mg, 3.75 mmol) was dissolved in anhydrous tetrahydrofuran (1.5 mL) and slowly added dropwise to the reaction system. After the addition was complete, the temperature was maintained and the mixture was stirred for another 10 min, then the mixture was warmed to room temperature and stirred for another 20 min. The reaction completion was monitored by LCMS. The reaction was quenched with an ammonium chloride aqueous solution (5 mL), 20 mL of water was added, and then extracted with EtOAc (30 mL×3). The organic phases were combined, dried over anhydrous $Na_2SO_4$, and filtered. The filtrate was concentrated to dryness, and the crude product was purified by FCC ($SiO_2$, EA/PE = 0-100%) to give a white solid compound **n-8-2** (465 mg, yield 49%). LCMS (m/z): 367.2 (M+H).

**Step C:** Intermediate **n-8**

At room temperature, 4M HCl-ethyl acetate (10 mL, 40 mmol) was added to compound **n-8-2** (465 mg, 1.27 mmol), and the mixture was stirred at room temperature for 1 h. The reaction completion was monitored by LCMS. The acid solution was removed by concentration to obtain a semi-solid oily intermediate, **n-8** (385 mg, 100%). LCMS (m/z): 267.1 (M+H).

The following intermediates were synthesized according to the above intermediates:

| Number | Structural Formula | Number | Structural Formula |
|---|---|---|---|
| **Intermediate n-8E** | 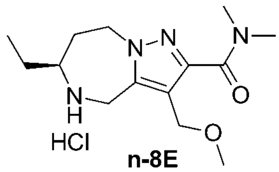 **n-8E** LCMS (m/z): 281.2 (M+H) | **Intermediate n-8-d6** | 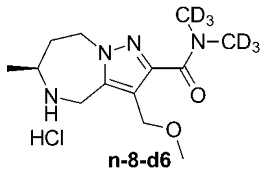 **n-8-d6** LCMS (m/z): 267.1 (M+H) |

## Intermediate n-9

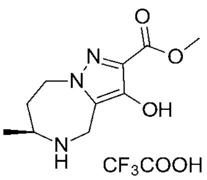

**n-9**

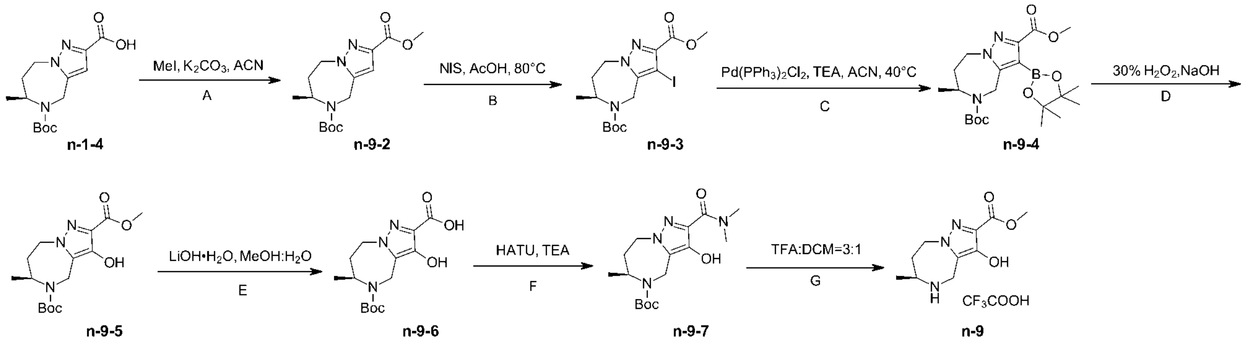


**Step A:** Compound **n-9-2**

At room temperature, $CH_3I$ (1.44 g, 10.2 mmol) was added to a mixture of compound **n-1-4** (1.00 g, 3.39 mmol),

$K_2CO_3$ (1.40 g, 10.2 mmol), and CAN (20 mL), and stirred overnight at 40°C. The reaction completion was monitored by LCMS. The reaction was concentrated using diatomite and filtered to obtain a clear organic phase. After complete concentration, a yellow solid compound **n-9-2** (1.03 g, 98% yield) was obtained. LCMS (m/z): 310.2 (M+H).

**Step B:** Compound **n-9-3**

At room temperature, NIS (1.60 g, 7.11 mmol) was added to a mixture of compound **n-9-2** (1.10 g, 3.56 mmol) and AcOH (15 mL), and stirred at 80°C for 1 h. The reaction completion was monitored by LCMS. After concentration to remove acid, the mixture was purified by FCC ($SiO_2$, EA/PE=0-60%) to obtain a deep yellow solid. This solid was then purified by slurrying (PE:EA 10:1, 15 mL) and filtered to obtain a yellow solid compound **n-9-3** (980 mg, yield 63%). LCMS (m/z): 436.1 (M+H).

**Step C:** Compound **n-9-4**

At room temperature, compound **n-9-3** (960 mg, 2.21 mmol), 4,4,5,5-tetramethyl-1,3,2-dioxaborane (2.82 g, 22.1 mmol), $Pd(PPh_3)_2Cl_2$ (155 mg, 0.221 mmol), and TEA (1.12 g, 11.0 mmol) were added sequentially to ACN (15 mL). After $N_2$ replacement three times, the mixture was heated to 40°C and stirred overnight. After the reaction completion was monitored by LCMS, the reaction solution was brought back to room temperature. The palladium catalyst was removed by diatomite filtration, the mother liquor was collected, water (50 mL) was added, and the mixture was extracted with EA (50 mL×3). The collected organic phase was concentrated, and the crude product was purified by FCC ($SiO_2$, EA/PE = 0-60%) to obtain a brownish-yellow solid compound **n-9-4** (960 mg, crude). Boron ester LCMS (m/z): 436.3 (M+H)/and boric acid LCMS (m/z): 354.2 (M+H).

**Step D:** Compound **n-9-5**

At room temperature, H2O2 (1.52 g, 30% in H2O, 13.4 mmol) was added to a mixture of compound **n-9-4** (950 mg, 2.69 mmol), NaOH (215 mg, 5.38 mmol), and THF (15 mL), and stirred at room temperature for 1 h. After the reaction completion was monitored by LCMS, water (50 mL) and EA (100 mL) were added. The pH was adjusted to ~6 with 1M HCl. Extraction with EA was performed (50 mL×3). The collected organic phase was washed with saturated brine, dried over anhydrous $Na_2SO_4$, filtered, and the organic liquid was collected and concentrated to give a yellow solid compound **n-9-5** (800 mg, 91%). LCMS (m/z): 326.1 (M+H).

**Step E:** Compound **n-9-6**

At room temperature, $LiOH•H_2O$ (774 mg, 18.4 mmol) was added to a solution of compound **n-9-5** (500 mg, 1.54 mmol) and $MeOH:H_2O$ (V/V = 1:1, 10 mL), and stirred overnight at room temperature. The reaction completion was monitored by LCMS. The reaction solution was concentrated to remove MeOH, diluted with EA (50 mL), and the pH was adjusted to ~6 with 1 M HCl. Extraction with EA was performed (50 mL×5). The collected organic phase was washed with saturated brine, dried over anhydrous $Na_2SO_4$, filtered, and the organic liquid was collected and concentrated to obtain a yellow oily product, compound **n-9-6** (400 mg, 84%). LCMS (m/z): 256 (M+H-56).

**Step F:** Compound **n-9-7**

At room temperature, TEA (650 mg, 6.42 mmol) was added to a mixture of compound **n-9-6** (400 mg, 1.28 mmol), dimethylamine hydrochloride (207 mg, 2.57 mmol), HATU (732 mg, 1.93 mmol), and DCM (10 mL). The mixture was stirred at room temperature for 2 h. The reaction completion was monitored by LCMS. After concentration, the product was purified by FCC ($SiO_2$, EA/PE=0-80%) to obtain a buttery compound **n-9-7** (250 mg, crude product). LCMS (m/z): 339.2 (M+H).

**Step G:** Intermediate **n-9**

At room temperature, TFA:DCM (V/V=3:1, 20 mL) was added to the compound **n-9-7** (150 mg, 0.443 mmol) system and stirred at room temperature for 1 h. The reaction completion was monitored by LCMS. After concentration to remove acid, a small amount of ACN and water were added for lyophilization to obtain a yellow oily intermediate **n-9** (160 mg), which was directly used in subsequent reactions. LCMS (m/z): 239.1 (M+H).

## Intermediate n-10

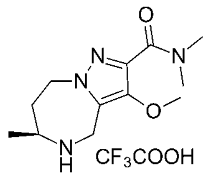

**n-10**

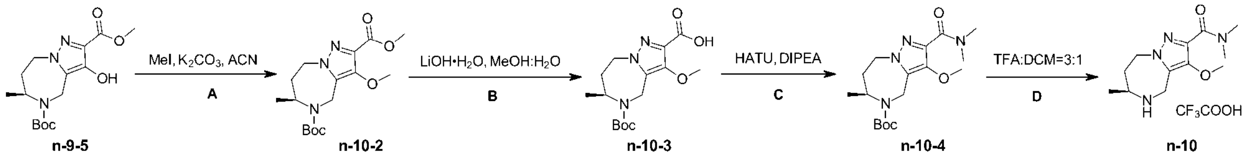


n-9-5 n-10-2 n-10-3 n-10-4 n-10

**Step A:** Compound **n-10-2**

At room temperature, $CH_3I$ (1.31 g, 9.22 mmol) was added to a mixture of compound **n-9-5** (300 mg, 0.922 mmol), $K_2CO_3$ (637 mg, 4.16 mmol), and ACN (15 mL), and stirred overnight at 40°C. The reaction completion was monitored by LCMS. The reaction solution was concentrated with diatomite to obtain a crude product, which was then purified by FCC ($SiO_2$, EA/PE=0-80%) to obtain a white solid compound **n-10-2** (160 mg, yield 51%). LCMS (m/z): 340.2 (M+H).

**Step B:** Compound **n-10-3**

At room temperature, LiOH•$H_2O$ (238 mg, 5.66 mmol) was added to a solution of compound **n-10-2** (160 mg, 0.471 mmol) and MeOH:$H_2O$ (V/V=1:1, 10 mL), and stirred overnight at room temperature. The reaction completion was monitored by LCMS. The reaction solution was concentrated to remove MeOH, diluted with EA (50 mL), and the pH was adjusted to ~6 with 1 M HCl. Extraction with EA was performed (50 mL×5). The collected organic phase was washed with saturated brine, dried over anhydrous $Na_2SO_4$, filtered, and the organic liquid was collected and concentrated to obtain a yellow oily compound **n-10-3** (150 mg, 98%). LCMS (m/z): 326.2 (M+H).

**Step C:** Compound **n-10-4**

At room temperature, DIPEA (298 mg, 2.31 mmol) was added to a mixture of compound **n-10-3** (150 mg, 0.461 mmol), dimethylamine hydrochloride (74.3 mg, 0.922 mmol), HATU (263 mg, 0.692 mmol), and DCM (10 mL), and stirred at room temperature for 2 h. The reaction completion was monitored by LCMS. After concentration, the mixture was purified by FCC ($SiO_2$, EA/PE=0-80%) to give a white solid compound **n-10-4** (140 mg, yield 86%). LCMS (m/z): 353.2 (M+H).

**Step D:** Intermediate **n-10**

At room temperature, TFA:DCM (V/V=3:1, 20 mL) was added to compound **n-10-4** (140 mg, 0.397 mmol) system, and the mixture was stirred at room temperature for 1 h. The reaction completion was monitored by LCMS. The solution was concentrated to remove acid, and a small amount of ACN and water were added. A small amount of ACN and water were added for lyophilization to obtain a white solid intermediate **n-10** (160 mg), which was used directly in subsequent reactions. LCMS (m/z): 253.2 (M+H).

## Intermediate n-11

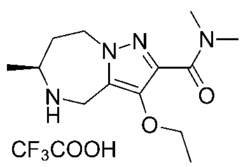

**n-11**

Intermediate n-11 was prepared according to the method of intermediate **n-10,** LCMS (m/z): 267.2 (M+H).

Referring to the above-described synthetic scheme or appropriate variants, the present invention also prepared and characterized the following intermediates:

| Number | Structural Formula | Number | Structural Formula |
|---|---|---|---|
| **Intermediate n-12** | 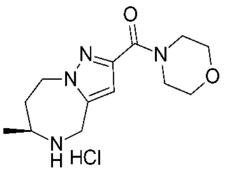 LCMS (m/z): 265.1 (M+H) | **Intermediate n-13** | 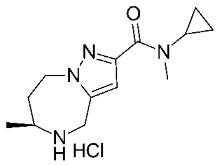 LCMS (m/z): 249.2 (M+H) |
| **Intermediate n-14** | 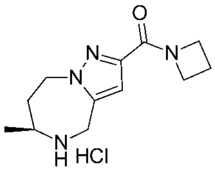 LCMS (m/z): 235.1 (M+H) | **Intermediate n-15** | 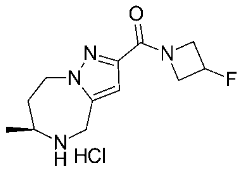 LCMS (m/z): 253.1 (M+H) |
| **Intermediate n-16** | 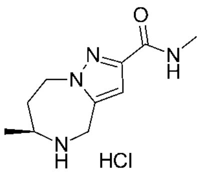 LCMS (m/z): 209.1 (M+H) | **Intermediate n-17** | 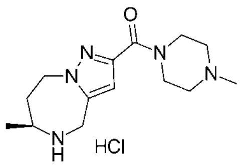 LCMS (m/z): 278.2 (M+H) |
| **Intermediate n-18** | 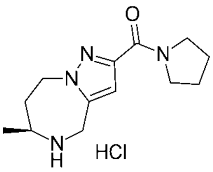 LCMS (m/z): 249.2 (M+H) | **Intermediate n-19** | 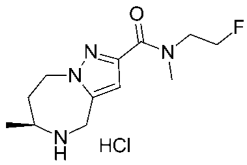 LCMS (m/z): 255.2 (M+H) |
| **Intermediate n-20** | 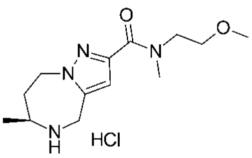 LCMS (m/z): 267.2 (M+H) | **Intermediate n-21** | 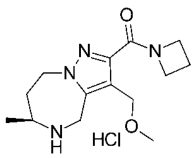 LCMS (m/z): 279.2 (M+H) |

## Intermediate n-22

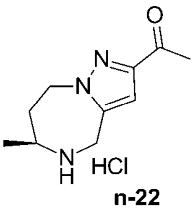

**n-22**

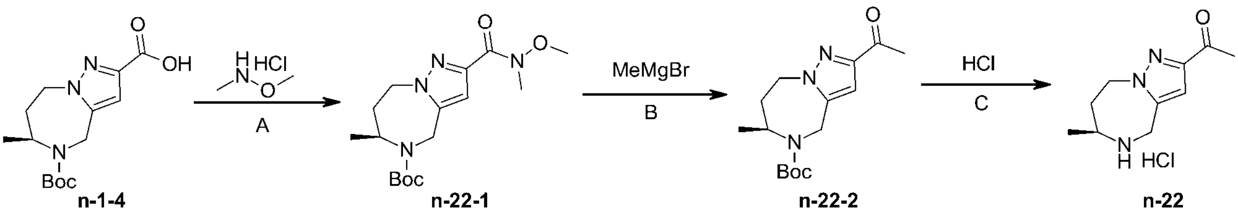


**n-1-4** **n-22-1** **n-22-2** **n-22**

**Step A:** Compound **n-22-1**

At room temperature, *N*-methyl-*O*-methylhydroxylamine hydrochloride (992 mg, 10.17 mmol) was added to a mixed solution of compound **n-1-4** (2 g, 6.78 mmol), HATU (3.86 g, 10.17 mmol), DIEA (2.04 g, 20.34 mmol), and THF (40 mL). The mixture was stirred at room temperature for 2 hours, and the reaction completion was monitored by TLC and LCMS. The reaction solution was poured into water (200 mL), extracted with EA (50 mL×3), and the collected organic phase was washed with saturated NaCl (50 mL) and dried over anhydrous $Na_2SO_4$. The crude product obtained by concentrating the organic phase was separated by FCC ($SiO_2$, EA/PE=10-40%) to give a white solid compound **n-22-1** (3.1 g). LCMS (m/z): 339.1 (M+H)

**Step B:** Compound **n-22-2**

At 0°C, a methyl magnesium bromide solution (1 M, 1.76 mL, 1.76 mmol) was added to a mixture of the compound **n-22-1** and THF (5mL), stirred at 0°C for 0.5 hours. The reaction completion was monitored by LCMS. The reaction mixture was then poured into 20 mL of $H_2O$ and extracted with EA (10 mL×3). The collected organic phase was washed with 20 mL of saturated NaCl and dried over anhydrous $Na_2SO_4$. The crude product obtained by concentrating the organic phase was separated by FCC ($SiO_2$, EA/PE=10-30%) to give a colorless oily compound **n-22-2** (150 mg, two-step yield 58%). LCMS (m/z): 294.1 (M+H).

**Step C:** Intermediate **n-22**

Compound **n-22-2** (150 mg, 0.51 mmol) was added to HCl-EA (5 mL, 4 M) solution at room temperature. The mixture was stirred at room temperature for 1 hour. The reaction completion was monitored by LCMS. The reaction solution was concentrated to give a white solid intermediate **n-22** (88 mg, 89% yield). LCMS (m/z): 194.1 (M+H).

## Intermediate n-23

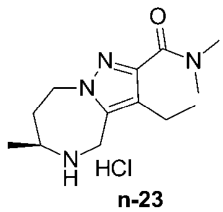

**n-23**

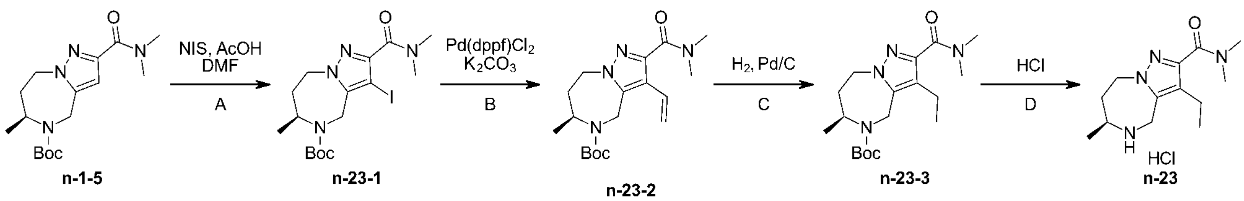


n-1-5 n-23-1 n-23-2 n-23-3 n-23

**Step A:** Compound **n-23-1**

At room temperature, N-iodosuccinimide (768 mg, 3.4 mmol) was added to a solution of compound **n-1-5** (1.0 g, 3.1 mmol) in DMF (15 mL) and acetic acid (0.5 mL). The mixture was warmed to 60°C and stirred for 12 h. After the reaction completion was monitored by LCMS, the reaction solution was slowly added to ice water and extracted with ethyl acetate. The organic phases were combined, washed with saturated brine, dried over anhydrous sodium sulfate, filtered, and concentrated to dryness. The crude product was purified by FCC ($SiO_2$, EA/PE=0-70%) to give a pale yellow solid compound **n-23-1** (1.3 g, yield 93%). LCMS (m/z): 449.1 (M+H).

**Step B:** Compound **n-23-2**

Under $N_2$ conditions, vinylboronate pinacol ester (1.4 g, 10.1 mmol), potassium carbonate (1.4 g, 10.1 mmol) and $Pd(dppf)Cl_2$ (222 mg, 0.29 mmol) was added to a solution of compound **n-23-1** (1.3 g, 2.9 mmol) in DMF (20 mL) 1.34 g (8.7 mmol). The resulting reaction solution was stirred at 90°C for 15 h. After the reaction completion was monitored by LCMS, the reaction solution was filtered through diatomite, the filtrate was concentrated to dryness, and the crude product was purified by FCC ($SiO_2$, EA/PE=0-75%) to give a yellow oily compound **n-23-2** (820 mg, yield 81%). LCMS (m/z): 349.2

(M+H).

**Step C:** Compound **n-23-3**

At room temperature, Pd/C (9.2 mg, 0.08 mmol, 10%) was added to a solution of compound **n-23-2** (300 mg, 0.86 mmol) in methanol (10 mL), and stirred at room temperature for 2 h. After the reaction completion was monitored by LCMS, the reaction solution was filtered through diatomite, the filtrate was concentrated to dryness, and the crude product was purified by FCC ($SiO_2$, EA/PE=0-75%) to give a yellow oily compound **n-23-3** (260 mg, yield 86%). LCMS (m/z): 351.2 (M+H).

**Step D:** Intermediate **n-23**

At 0°C, HCl/EA (5 mL, 4 N) was added to compound **n-23-2** (260 mg, 0.74 mmol), and the mixture was stirred for 1 h. After the reaction completion was monitored by LCMS, the reaction solution was concentrated to dryness to obtain a yellow oily intermediate **n-23** (270 mg). LCMS (m/z): 251.2 (M+H).

## Intermediate n-24

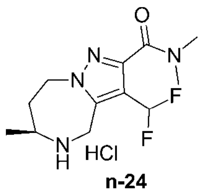

**n-24**

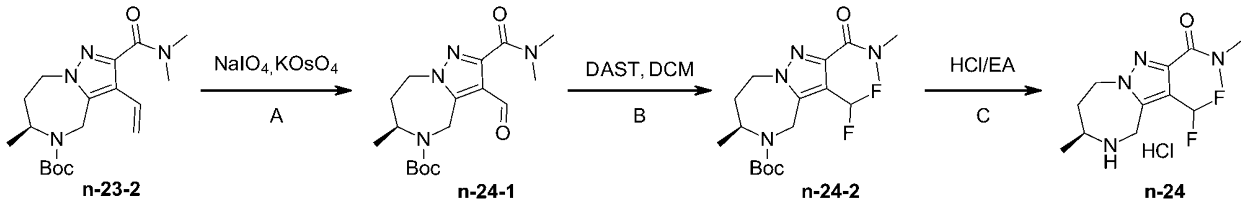


**n-23-2** **n-24-1** **n-24-2** **n-24**

**Step A:** Compound **n-24-1**

At room temperature, potassium osmium tetroxide (45 mg, 0.29 mmol) was added to a solution of compound **n-23-2** (500 mg, 2.9 mmol) in THF (10 mL) and water (5 mL) at room temperature. The resulting reaction solution was stirred at room temperature for 0.5 h. Sodium periodate (1.23 g, 5.75 mmol) was added to the above reaction solution, and the resulting reaction solution was stirred at room temperature for another 12 h. After the reaction completion was monitored by LCMS, the reaction solution was slowly added to water and extracted with ethyl acetate. The organic phases were combined, washed with saturated brine, dried over anhydrous sodium sulfate, filtered and concentrated to dryness. The crude product was purified by FCC ($SiO_2$, EA/PE=0-80%) to give a yellow oily compound **n-24-1** (120 mg, yield 24%). LCMS (m/z): 351.2 (M+H).

**Step B:** Compound **n-24-2**

At room temperature, diethylaminotrifluoride (552 mg, 3.4 mmol) was added to a solution of compound **n-24-1** (120 mg, 0.86 mmol) in dichloromethane (3 mL), and warmed to 40°C and stirred for 6 h. After the reaction completion was monitored by LCMS, the reaction solution was slowly added to a saturated aqueous sodium bicarbonate solution and extracted with dichloromethane. The organic phases were combined, washed with saturated brine, dried over anhydrous sodium sulfate, filtered, concentrated to dryness, and the crude product was purified by pre-TLC (EA) to give a yellow oily compound **n-24-2** (42 mg, yield 33%). LCMS (m/z): 373.2 (M+H).

**Step C:** Intermediate **n-24**

At 0°C, HCl/EA (2 mL, 4N) was added to compound **n-24-2** (42 mg, 0.11 mmol). The reaction mixture was stirred at the temperature for 1 h. After the reaction was completed, the reaction solution was concentrated to dryness to obtain a yellow oily intermediate **n-24** (45 mg). LCMS (m/z): 273.2 (M+H).

## Intermediate n-25

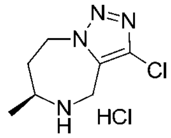

**n-25**

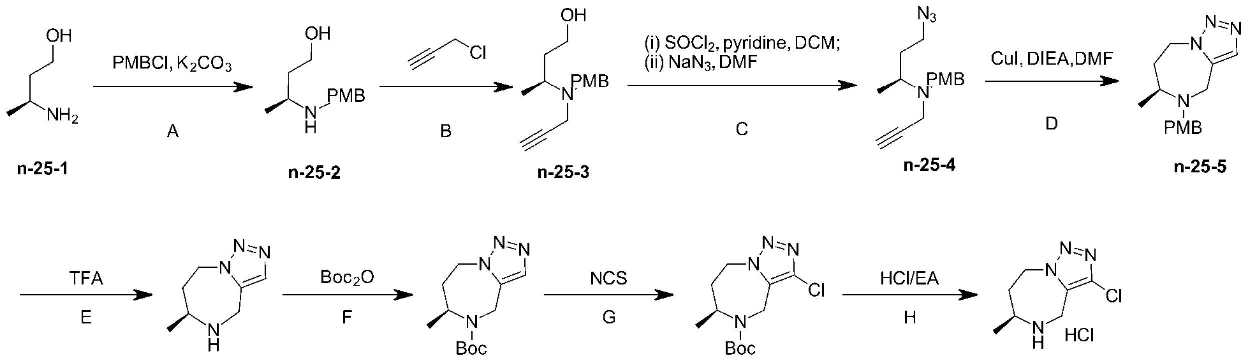


**n-25-1** **n-25-2** **n-25-3** **n-25-4** **n-25-5**

**n-25-6** **n-25-7** **n-25-8** **n-25**

**Step A:** Compound **n-25-2**

At 0°C, PMBCl (12.9 g, 82.7 mmol) was slowly added dropwise to a mixture of compound n-**25-1** (6.70 g, 75.2 mmol), $K_2CO_3$ (20.0 g, 225 mmol), and MeCN (200 mL). The mixture was stirred at room temperature for 16 h, and the reaction completion was monitored by LCMS. The crude product was filtered and concentrated, then purified by FCC ($SiO_2$, THF/PE=0-15%) to give a colorless oily compound **n-25-2** (2.3 g, 15% yield). LCMS (m/z): 210.1 (M+H).

**Step B:** Compound **n-25-3**

At room temperature, compound **n-25-2** (2.0 g, 9.56 mmol), 3-chloropropyne (848 mg, 11.4 mmol), and $K_2CO_3$ (2.64 g, 19.1 mmol) were added sequentially to a 40 mL MeCN solution. The mixture was warmed to 80°C and stirred for 16 h. The reaction completion was monitored by LCMS. The crude product was filtered and concentrated, then purified by FCC ($SiO_2$, THF /PE=0-10%) to give a colorless oily compound **n-25-3** (1.7 g, 74% yield). LCMS (m/z): 247.1 (M+H).

**Step C:** Compound **n-25-4**

At 0°C, $SOCl_2$ (1.06 mL, 14.5 mmol) was slowly added dropwise to a mixture of pyridine (0.883 mL, 10.9 mmol) and compound **n-25-3** (1.7 g, 6.88 mmol) in DCM (30 mL). The mixture was stirred at 0°C for 6 h, and the reaction completion was monitored by LCMS. The reaction was quenched with $NaHCO_3$, extracted with ethyl acetate (30 mL×3), and the organic phase was dried and concentrated. $NaN_3$ (450 mg, 6.88 mmol) was added to a solution of the crude product from the previous step in DMF (20 mL), and the mixture was stirred at 80°C for 16 h, and the reaction copmletion was monitored by LCMS. Ethyl acetate (50 mL) was added, and washed with saturated brine (15 mL×3). The organic phase was dried and concentrated to give crude compound **n-25-4** (1.8 g). LCMS (m/z): 273.1 (M + H).

**Step D:** Compound **n-25-5**

At room temperature, compound **n-25-4** (1.30 g), CuI (89.3 mg, 0.476 mmol), and DIEA (1.85 g, 14.28 mmol) were added sequentially to a solution of DMF (10 mL), and stirred at 80°C for 0.5 h. The reaction completion was monitored by LCMS. Ethyl acetate (50 mL) was added, followed by washing with saturated brine (15 mL×3). The organic phase was dried and concentrated to obtain a crude product, which was then purified by FCC ($SiO_2$, THF /PE=0-20%) to yield a colorless oily compound **n-25-4** (550 mg, two-step yield 42%). LCMS (m/z): 273.1 (M+H).

**Step E:** Compound **n-25-6**

Compound **n-25-5** (550 mg, 2.02 mmol) was added to TFA (8 mL) at room temperature and stirred at 80°C for 2 h. The reaction completion was monitored by LCMS, and the reaction solution was concentrated to obtain a brown compound

**n-25-6** (246 mg, crude product). LCMS (m/z): 153.1 (M+H).

**Step F:** Compound **n-25-7**

At room temperature, compound **n-25-6** (246 mg, 1.61 mmol), TEA (0.801 mg, 8.05 mmol), and $Boc_2O$ (702 mg, 3.22 mmol) were added sequentially to DCM (10 mL) and stirred for 2 h. The reaction completion was monitored by LCMS. The reaction solution was purified by FCC ($SiO_2$, THF/PE=0-20%) to obtain a colorless oily compound **n-25-7** (400 mg, 90% yield in two steps). LCMS (m/z): 253.1 (M+H).

**Step G:** Compound **n-25-8**

At room temperature, compound **n-25-7** (93.1 mg, 0.369 mmol) and NCS (73.3 mg, 0.551 mmol) were added sequentially to MeCN (3 mL) and stirred at 60°C for 2 h. The reaction completion was monitored by LCMS. The reaction solution was purified by C18 ($H_2O$/MeOH=0-40%) to give a white solid compound **n-25-8** (88.7 mg, yield 79%). LCMS (m/z): 287.1 (M+H).

**Step H:** Compound **n-25**

At room temperature, compound **n-25-8** (88.7 mg, 0.309 mmol) was added to EA/HCl (10 mL) and stirred for 2 h. The reaction completion was monitored by LCMS, and the mixture was concentrated to give a white solid, compound **n-25** (56 mg, 92% yield). LCMS (m/z): 187.1 (M+H).

## Intermediate n-26

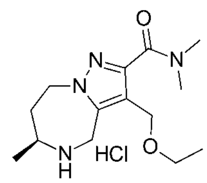

**n-26**

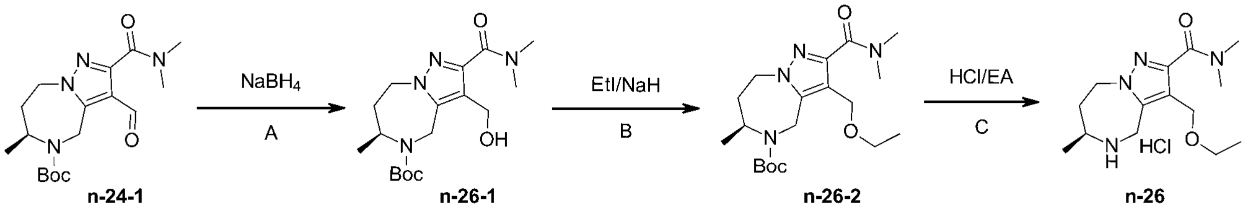


**n-24-1** **n-26-1** **n-26-2** **n-26**

Intermediate **n-26** was prepared according to the above scheme. LCMS (m/z): 281.2 (M+H).

## Intermediate n-8-d3

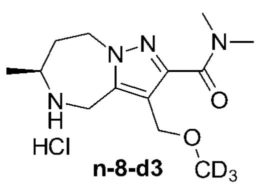

**n-8-d3**

The synthesis of intermediate n-8-d3 was carried out according to the scheme of intermediate **n-26.** LCMS (m/z): 270.2 (M+H).

## Intermediate A-I-F

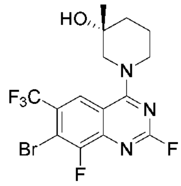

A-I-F

(*R*)-1-(7-Bromo-2,8-difluoro-6-(trifluoromethyl)quinazoline-4-yl)-3-methylpiperidine-3-ol

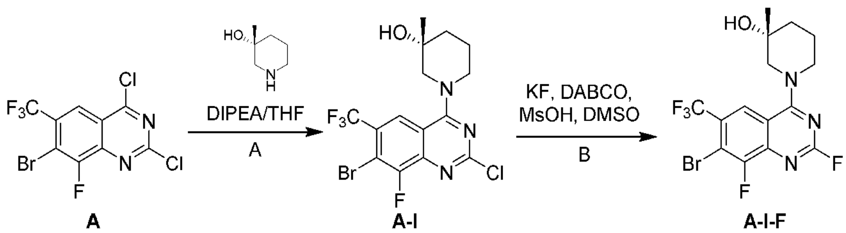


A A-I A-I-F

**Step A:** (*R*)-1-(7-bromo-2-chloro-8-fluoro-6-(trifluoromethyl)quinazoline-4-yl)-3-methylpiperidine-3-ol

At 0°C, DIEA (2.45 mL, 13.62 mmol) was added to a mixture of 7-bromo-2,4-dichloro-8-fluoro-6-(trifluoromethyl) quinazoline (1.8g, 3.79 mmol), (*R*)-3-methyl-3-piperidinol hydrochloride (600 mg, 3.79 mmol) and THF (30 mL), and was stirred at room temperature for 2 h. The reaction completion was monitored by TLC. After cooling, the reaction solution was poured into water (100 mL), extracted with EA (50 mL×3), and the collected organic phase was washed with saturated NaCl (20 mL). The crude product obtained by concentrating the organic liquid was purified by FCC ($SiO_2$, EA/PE=0~20%) to give a yellow solid (*R*)-1-(7-bromo-2-chloro-8-fluoro-6-(trifluoromethyl)quinazoline-4-yl)-3-methylpiperidine-3-ol (1.38 g, yield 79.3%).

**Step B:** (*R*)-1-(7-Bromo-2,8-difluoro-6-(trifluoromethyl)quinazoline-4-yl)-3-methylpiperidine-3-ol

At room temperature, (*R*)-1-(7-bromo-2-chloro-8-fluoro-6-(trifluoromethyl)quinazoline-4-yl)-3-methylpiperidine-3-ol (1.33 g, 3 mmol) was added to a mixed solution of 1,4-diazabicyclo[2.2.2]octane (34 mg, 301 μmol), KF (1.75 g, 30 mmol), MsOH (29 mg, 301 μmol), and DMSO (15 mL). The mixture was stirred at 65°C for 16 h, and the reaction completion was monitored by LCMS. After cooling, the reaction solution was poured into $H_2O$ (100 mL), and a brownish-yellow solid was precipitated. The filter cake was filtered, washed with $H_2O$ (100 mL), and dried to give a brown solid (*R*)-1-(7-bromo-2,8-difluoro-6-(trifluoromethyl)quinazoline-4-yl)-3-methylpiperidine-3-ol (1 g, yield 78.1%). LCMS (m/z): 427.9 (M+H).

## Intermediate A-II-F

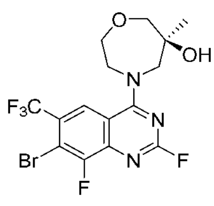

A-II-F

(S)-4-(7-Bromo-2,8-difluoro-6-(trifluoromethyl)quinazoline-4-yl)-6-methyl-1,4-oxazol-6-ol

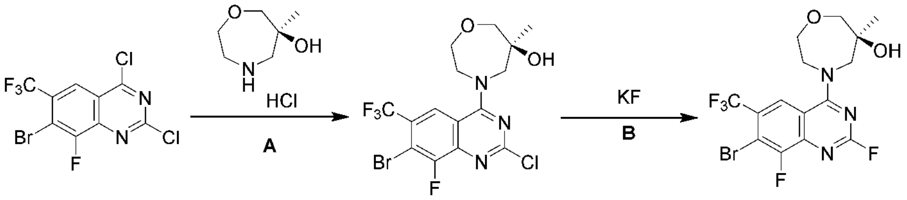


A A-II A-II-F

**Step A:** (*S*)-4-(7-bromo-2-chloro-8-fluoro-6-(trifluoromethyl)quinazoline-4-yl)-6-methyl-1,4-oxazol-6-ol

At room temperature, DIPEA (2.70 mL, 15.8 mmol) was added to a mixture of 7-bromo-2,4-dichloro-8-fluoro-6-(trifluoromethyl)quinazoline (1.80 g, 3.95 mmol), (*S*)-6-methyl-1,4-oxacyclohexane-6-ol hydrochloride (660 mg, 3.95 mmol) and THF (20 mL), and the mixture was stirred at room temperature for 1 h. The reaction completion was monitored by TLC and LCMS. $H_2O$ (100 mL) and EA (100 mL×3) were added for extraction. The organic phase was collected, washed with saturated brine, and the organic phase was collected. The crude product obtained by concentration was purified by FCC ($SiO_2$, EA/PE=0-30%) to give a yellow solid product (*S*)-4-(7-bromo-2-chloro-8-fluoro-6-(trifluoromethyl)quinazoline-4-yl)-6-methyl-1,4-oxazol-6-ol (1.4 g, yield 78%). LCMS (m/z): 459.9 (M+H).

**Step B:** (*S*)-4-(7-Bromo-2,8-difluoro-6-(trifluoromethyl)quinazoline-4-yl)-6-methyl-1,4-oxazol-6-ol

At room temperature, (S)-4-(7-bromo-2-chloro-8-fluoro-6-(trifluoromethyl)quinazoline-4-yl)-6-methyl-1,4-oxazol-6-ol (1.40 g, 3.05 mmol), KF (1.77 g, 30.5 mmol), DABCO (34 mg, 0.31 mmol), and MsOH (29 mg, 0.31 mmol) were added sequentially to DMSO (15 mL), and the mixture was heated to 65°C and stirred for 3 h. The reaction completion was monitored by LCMS, and the reaction solution was then returned to room temperature. Then, it was slowly poured into 200 mL of water under stirring. A flocculent, insoluble substance was formed. The filter cake was collected, dried, and a yellow solid (*S*)-4-(7-bromo-2,8-difluoro-6-(trifluoromethyl)quinazoline-4-yl)-6-methyl-1,4-oxazol-6-ol (1.1 g, yield 81%) was obtained. LCMS (m/z): 443.9 (M+H).

## Intermediate B-I-F

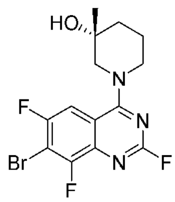

B-I-F

(*R*)-1-(7-Bromo-2,6,8-trifluoroquinazoline-4-yl)-3-methylpiperidine-3-ol

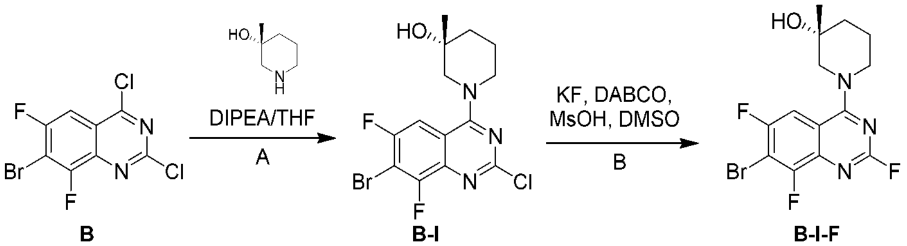


B B-I B-I-F

**Step A:** (*R*)-1-(7-Bromo-2-chloro-6,8-difluoroquinazoline-4-yl)-3-methylpiperidine-3-ol

At room temperature, DIPEA (39.9 mL, 229 mmol) was added to a mixture of 7-bromo-2,4-dichloro-6,8-difluoroquinazoline (18.0 g, 57 mmol), (*R*)-3-methylpiperidine-3-ol hydrochloride (8.69 g, 57 mmol), and THF (200 mL). The mixture was stirred at room temperature for 2 h. The reaction completion was monitored by TLC and LCMS. THF was removed by concentration, and acetonitrile (15 mL) was added. The mixture of acetonitrile and the compound was slowly poured into stirred water (600 mL), resulting in a flocculent insoluble precipitate. The precipitate was filtered, and the filter cake was dried to obtain a yellow solid (*R*)-1-(7-bromo-2-chloro-6,8-difluoroquinazoline-4-yl)-3-methylpiperidine-3-ol (21.7 g, 96% yield). LCMS (m/z): 393.9 (M+H).

**Step B:** (*R*)-1-(7-bromo-2,6,8-trifluoroquinazoline-4-yl)-3-methylpiperidine-3-ol

At room temperature, (*R*)-1-(7-bromo-2-chloro-6,8-difluoroquinazoline-4-yl)-3-methylpiperidine-3-ol (12.0 g, 30.6 mmol), KF (17.8 g, 306 mmol), DABCO (137 mg, 1.22 mmol), and MsOH (117 mg, 1.22 mmol) were added sequentially to DMSO (60 mL), and the mixture was heated to 65°C and stirred for 10 h. After the reaction completion was monitored by LCMS, the reaction solution was returned to room temperature. Then, it was slowly poured into stirred water (600 mL), producing a flocculent insoluble precipitate. The precipitate was filtered, and the filter cake was dried to obtain a yellow solid (*R*)-1-(7-bromo-2,6,8-trifluoroquinazoline-4-yl)-3-methylpiperidine-3-ol (11.3 g, 98% yield). LCMS (m/z): 378.0 (M+H).

## Intermediate B-II-F

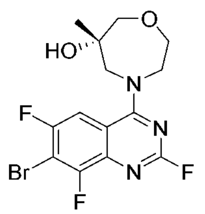

**B-II-F**

(*S*)-4-(7-Bromo -2,6,8- trifluoroquinazoline-4-yl)-6-methyl-1,4-oxazacycloheptane-6-ol

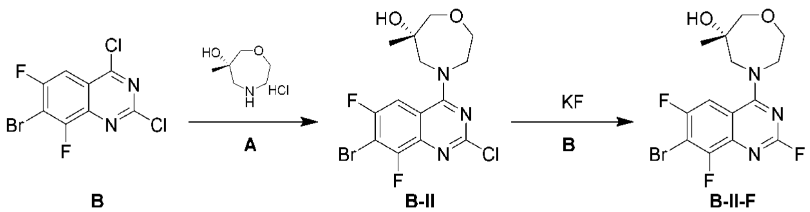


**B** **B-II** **B-II-F**

**Step A:** (*S*)-4-(7-Bromo-2-chloro-6,8-difluoroquinazoline-4-yl)-6-methyl-1,4-oxazacycloheptane-6 -ol

At room temperature, DIPEA (1.55 mL, 8.92 mmol) was added to a mixture of 7-bromo-2,4-dichloro-6,8-difluoroquinazoline (700 mg, 2.23 mmol), (*S*)-6-methyl-1,4-oxazacycloheptane-6-ol hydrochloride (374 mg, 2.23 mmol), and THF (12 mL). The mixture was stirred at room temperature for 2 h. The reaction completion was monitored by TLC and LCMS. THF was removed by concentration, and acetonitrile (2 mL) was added. The solution was then slowly poured into water (50 mL) under stirring. Flocculent insoluble matter was formed. After filtration and drying of the filter cake, a brownish-yellow solid (*S*)-4-(7-bromo-2-chloro-6,8-difluoroquinazoline-4-yl)-6-methyl-1,4-oxazacycloheptane-6-ol (720 mg, yield 79%) was obtained. LCMS (m/z): 409.9 (M+H).

**Step B:** (*S*)-4-(7-bromo-2,6,8-trifluoroquinazoline-4-yl)-6-methyl-1,4-oxazacycloheptane-6-ol

At room temperature, (*S*)-4-(7-bromo-2-chloro-6,8-difluoroquinazoline-4-yl)-6-methyl-1,4-oxazacycloheptane-6-ol (700 mg, 1.71 mmol), KF (995 mg, 17.1 mmol), MsOH (16 mg, 171 μmol), and DABCO (19 mg, 171 μmol) were added sequentially to DMSO (10 mL), and warmed to 65°C and stirred for 18 h. The reaction completion was monitored by LCMS, and the reaction solution was then returned to room temperature. Then slowly poured into stirred water (60 mL). A yellow, flocculent, insoluble substance was formed, filtered and the filter cake was dried to obtain a yellow solid (*S*)-4-(7-bromo-2,6,8-trifluoroquinazoline-4-yl)-6-methyl-1,4-oxazacycloheptane-6-ol (600 mg, yield 89%). LCMS (m/z): 392.0 (M+H).

## Intermediate D-I-F

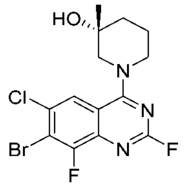

D-I-F

(*R*)-1-(7-Bromo-6-chloro-2,8-difluoro-4-quinazolinyl)-3-methyl-3-piperidinol

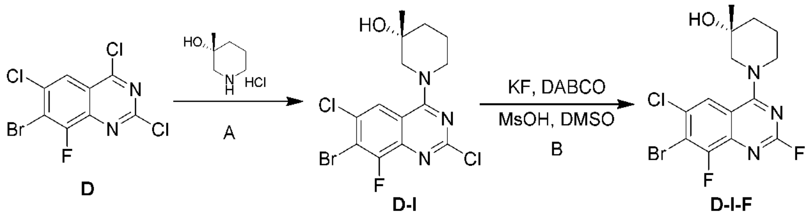


D D-I D-I-F

**Step A:** (*R*)-1-(7-Bromo-2,6-dichloro-8-fluoro-4-quinazolinyl)-3-methyl-3-piperidinol

At 0°C, DIEA (4.5 mL, 27.24 mmol) was added to a mixed solution of 7-bromo-2,4,6-trichloro-8-fluoroquinazoline (3.0 g, 3.03 mmol), (*R*)-3-methyl-3-piperidinol hydrochloride (1.38 g, 9.08 mmol), and THF (50 mL). The mixture was stirred at room temperature for 2 h. The reaction completion was monitored by TLC. The reaction solution was concentrated to obtain an oily substance, which was dissolved in acetonitrile (5 mL). This solution was poured into $H_2O$ (300 mL), and a brownish-yellow solid precipitated. The solid was filtered, and the filter cake was washed with $H_2O$ (100 mL). The filter cake was dried to give a brown solid (*R*)-1-(7-bromo-2,6-dichloro-8-fluoro-4-quinazolinyl)-3-methyl-3-piperidinol (3.7 g, 9.04 mmol, yield 99%).

**Step B:** (*R*)-1-(7-Bromo-6-chloro-2,8-difluoro-4-quinazolinyl)-3-methyl-3-piperidinol

At room temperature, (*R*)-1-(7-bromo-2,6-dichloro-8-fluoro-4-quinazolinyl)-3-methyl-3-piperidinol (3.7 g, 9.04 mmol) was added to a mixed solution of DABCO (102 mg, 904 μmol), KF (5.25 g, 90.45 mmol), MsOH (87 mg, 904 μmol), and DMSO (50 mL). The resulting mixture was heated to 65°C and stirred for 16 h. The reaction completion was monitored by LCMS. After cooling, the reaction solution was poured into $H_2O$ (400 mL), and a brownish-yellow solid precipitated. The mixture was filtered, and the filter cake was washed with $H_2O$ (100 mL). The filter cake was dried to obtain a brown solid (*R*)-*1*-(7-bromo-6-chloro-2,8-difluoro-4-quinazolinyl)-3-methyl-3-piperidinol (3.0 g, yield 84%). LCMS (m/z): 393.9 (M+H).

## Intermediate D-II-F

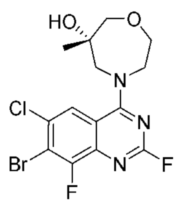

D-II-F

(*S*)-4-(7-bromo-6-chloro-2,8-difluoro-4-quinazolinyl)-6-methyl-1,4-oxazacycloheptane-6-ol

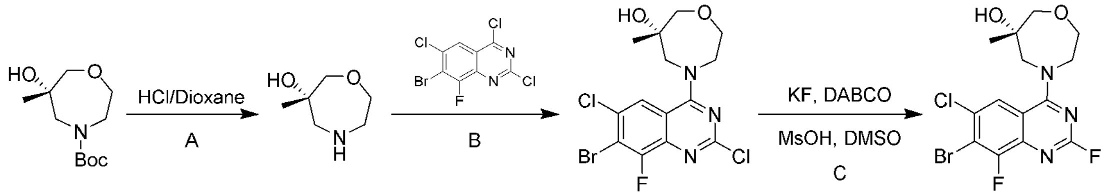


D-II-2 D-II-1 D-II D-II-F

**Step A:** (*S*)-6-Methyl-1,4-oxacyclohexane-6-ol hydrochloride

At room temperature, HCl-dioxane (4M) (10 mL, 10V) was added to tert-butyl (*S*)-6-hydroxy-6-methyl-1,4-oxacycloheptane-4-carboxylate (1.00 g, 4.32 mmol) and stirred for 1 h at room temperature. The reaction completion was monitored by LCMS. After concentration, a white solid (*S*)-6-methyl-1,4-oxacycloheptane-6-ol hydrochloride (840 mg, crude product) was obtained. LCMS (m/z): 132.1 (M+H).

**Step B:** (*S*)-4-(7-Bromo-2,6-dichloro-8-fluoro-4-quinazolinyl)-6-methyl-1,4-oxacyclohexane-6-ol

At room temperature, DIPEA (1.56 g, 12.1 mmol) was added to a mixture of 7-bromo-2,4,6-trichloro-8-fluoroquinazoline (1.00 g, 3.03 mmol), (*S*)-6-methyl-1,4-oxacyclohexane-6-ol hydrochloride (655 mg, 12.1 mmol), and THF (20 mL), and stirred for 1 h at room temperature. The reaction completion was monitored by LCMS. The reaction mixture was poured into a saturated $NH_4Cl$ aqueous solution (50 mL) and extracted with EA (30 mL×3). The organic phase was collected, washed with a saturated NaCl aqueous solution (70 mL), dried over anhydrous $Na_2SO_4$, filtered, and concentrated to give a white solid (*S*)-4-(7-bromo-2,6-dichloro-8-fluoro-4-quinazoline)-6-methyl-1,4-oxacyclohexane-6-ol (1.2 g, 93% yield). LCMS (m/z): 425.9(M+H).

**Step C:** (*S*)-4-(7-Bromo-6-chloro-2,8-difluoro-4-quinazolinyl)-6-methyl-1,4-oxacyclohexane-6-ol

At room temperature, MsOH (27.0 mg, 0.282 mmol) was added to a mixed solution of (*S*)-4-(7-bromo-2,6-dichloro-8-fluoro-4-quinazolinyl)-6-methyl-1,4-oxacyclohexane-6-ol (1.20 g, 2.82 mmol), KF (1.64 g, 28.2 mmol), DABCO (31.7 mg, 0.282 mmol), and DMSO (30 mL). The reaction solution was warmed to 100°C and stirred for 1 h. After the reaction completion was monitored by LCMS, the reaction solution was slowly added dropwise to water (150 mL) to precipitate a solid, which was filtered. The filter cake was dissolved in EA (100 mL), dried over anhydrous $Na_2SO_4$, filtered again, and the organic liquid was concentrated to give a yellow solid *(S)*-4-(7-bromo-6-chloro-2,8-difluoro-4-quinazolinyl)-6-methyl-1,4-oxacyclohexane-6-ol (1.0 g, yield 87%). LCMS (m/z): 409.9 (M+H).

## Intermediate F-I

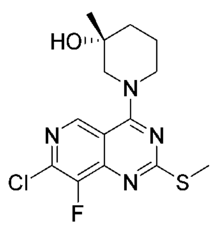

F-I

(*3R*)-1-(7-Chloro-8-fluoro-2-(methylthio)-3,4-dihydropyrido[4,3-*d*]pyrimidine-4-yl)-3-methylpiperidine-3-ol

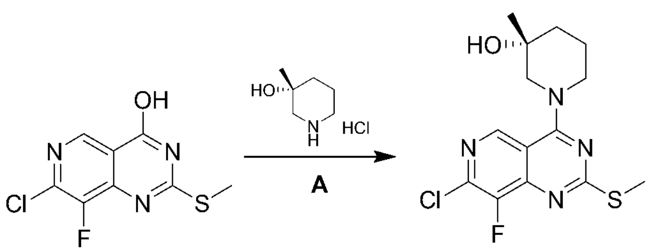


F F-I

**Step A:** (3*R*)-1-(7-Chloro-8-fluoro-2-(methylthio)-3,4-dihydropyrido[4,3-*d*]pyrimidine-4-yl)-3-methylpiperidine-3-ol

At room temperature, DIPEA (2.37 g, 18.3 mmol) was added to a mixture of 7-chloro-8-fluoro-2-(methylthio) pyrido[4,3-*d*]pyrimidine-4-ol (1.50 g, 6.11 mmol), (*R*)-3-methylpiperidine-3-ol hydrochloride (1.06 g, 7.02 mmol), BOP (5.40 g, 12.2 mmol), and DMF (20 mL). The reaction mixture was warmed to 45°C and stirred for 18 h. After the reaction completion was monitored by TLC and the reaction solution was brought back to room temperature, it was slowly poured into 200 mL of stirred $H_2O$. The solid precipitated out, and the mixture was filtered. The filter cake was washed with 100 mL of $H_2O$ and dried to obtain a brown solid (*3R*)-1-(7-chloro-8-fluoro-2-(methylthio)-3,4-dihydropyrido[4,3-*d*]pyrimidine-4-yl)-3-methylpiperidine-3-ol (2.0 g, yield 98%).

## Intermediate F-II

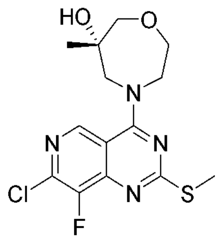

**F-II**

(*S*)-4-(7-Chloro-8-fluoro-2-(methylthio)pyrido[4,3-d]pyrimidine-4-yl)-6-methyl-1,4-oxazacycloheptane-6-ol

The synthesis of intermediate F-II was carried out according to the same scheme as intermediate F-I, in which (*S*)-6-methyl-1,4-oxazacycloheptane-6-ol-hydrochloride was used instead of (*R*)-3-methylpiperidine-3-ol·hydrochloride in step A.

## Intermediate G-II

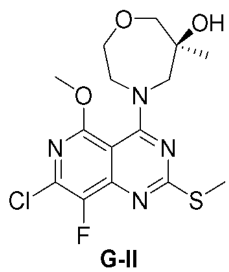

**G-II**

(*S*)-4-(7-Chloro-8-fluoro-5-methoxy-2-(methylthio)pyrido[4,3-d]pyrimidine-4-yl)-6-methyl-1,4-oxazacycloheptane-6-ol

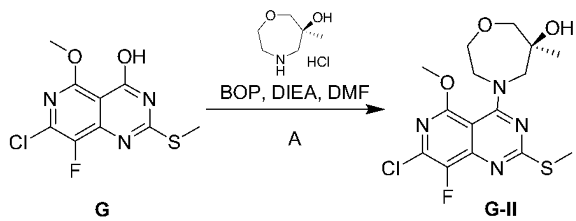


**G** **G-II**

**Step A:** (*S*)-4-(7-Chloro-8-fluoro-5-methoxy-2-(methylthio)pyrido[4,3-d]pyrimidine-4-yl)-6-methyl-1,4-oxazacycloheptane-6-ol

At room temperature, DIEA (1.4 g, 11 mmol) was added to a mixed solution of 7-chloro-8-fluoro-5-methoxy-2-(methylthio)pyrido[4,3-*d*]pyrimidine-4-ol (1.0 g, 3.6 mmol), (*S*)-6-methyl-1,4-oxazacycloheptane-6-ol·hydrochloride (770 mg, 4.64 mmol), BOP (2.4 g, 5.4 mmol), and DMF (15 mL). The reaction mixture was warmed to 50°C and stirred for 2 h. After the reaction completion was monitored by LCMS and brought to room temperature, the solution was slowly poured into 100 mL of stirred $H_2O$. A solid precipitated out, and after filtration and drying of the filter cake, a brownish-yellow solid (*S*)-4-(7-chloro-8-fluoro-5-methoxy-2-(methylthio)pyrido[4,3-*d*]pyrimidine-4-yl)-6-methyl-1,4-oxazacycloheptane-6-ol

(900 mg, yield 64%) was obtained. LCMS (m/z): 389.0 (M+H).

## Intermediate G-I

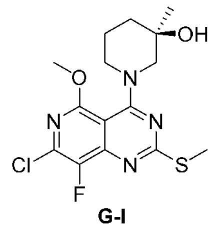

G-I

(*R*)-1-(7-Chloro-8-fluoro-5-methoxy-2-methylthiopyridino[4,3-*d*]pyrimidine-4-yl)-3-methylpiperidine-3-ol

The synthesis of intermediate G-I was carried out according to the scheme described for intermediate G-II, in which (*R*)-6-methylpiperidine-3-ol-hydrochloride was used instead of (*S*)-6-methyl-1,4-oxazacycloheptane-6-ol-hydrochloride in step A.

## Intermediate I-I

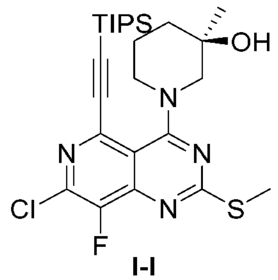

I-I

(*R*)-1-(7-Chloro-8-fluoro-2-(methylthio)-5-((triisopropylsilyl)ethynyl)pyrido[4,3-*d*]pyrimidine-4-yl)-3-methylpiperidine-3-ol

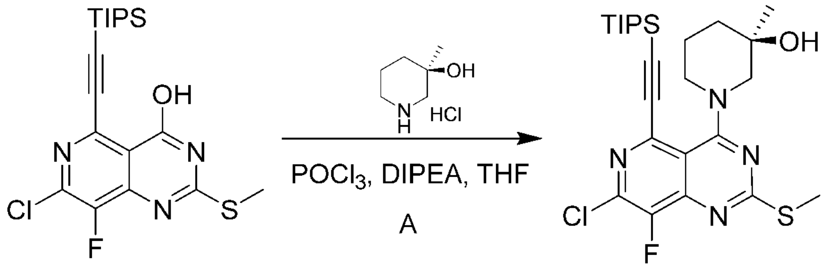


I I-I

**Step A:** (*R*)-1-(7-Chloro-8-fluoro-2-(methylthio)-5-((triisopropylsilyl)ethynyl)pyrido[4,3-*d*]pyrimidine-4-yl)-3-methylpiperidine-3-ol

At room temperature, 7-chloro-8-fluoro-2-(methylthio)-5-((triisopropylsilyl) ethynyl)pyrido[4,3-*d*]pyrimidine-4-ol (200 mg, 0.47 mmol) was dissolved in $POCl_3$ (2 mL), and DIPEA (360 mg, 2.35 mmol) was added. The resulting system was heated to 100°C and reacted for 3 h, then concentrated to dryness under reduced pressure. After cooling to room temperature, 5 mL of anhydrous THF was added, followed by (*R*)-3-methyl-3-hydroxypiperidine hydrochloride (107 mg, 0.704 mmol) and DIPEA (360 mg, 2.35 mmol). The resulting mixture was stirred at room temperature for 2 h, and the reaction completion was monitored by LCMS. The reaction solution was concentrated to dryness, and the crude product was purified by FCC ($SiO_2$, EA /PE=0-50%) to give a white solid (*R*)-1-(7-chloro-8-fluoro-2-(methylthio)-5-((triisopropylsilyl)ethynyl)pyrido[4,3-*d*]pyrimidine-4-yl)-3-methylpiperidine-3-ol (200 mg, yield 81%). LCMS (m/z): 523.2 (M+H).

## Intermediate I-II

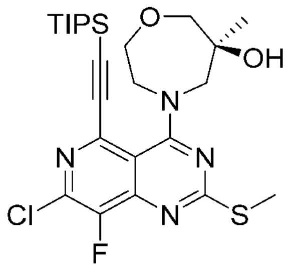

**I-II**

(*S*)-4-(7-Chloro-8-fluoro-2-(methylthio)-5-((triisopropylsilyl)ethynyl)pyrido[4,3-*d*]pyrimidine-4-yl)-6-methyl-1,4-oxazacycloheptane-6-ol

The synthesis of intermediate I-II was carried out according to the scheme described for intermediate I-I, with (*S*)-6- methyl-1,4-oxazacycloheptane-6-ol-hydrochloride used instead of (*R*)-3-methyl-3-hydroxypiperidine-hydrochloride in step A.

The following intermediates were prepared according to the above synthesis scheme:

| Number | Structural Formula | Number | Structural Formula |
|---|---|---|---|
| **Intermediate A-III-F** | 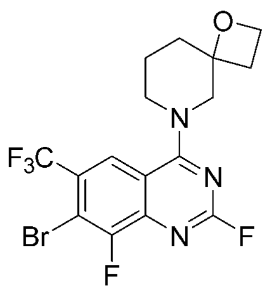 LCMS (m/z): 438.0 (M+H) | **Intermediate A-IV-F** | 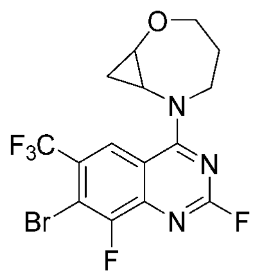 LCMS (m/z): 424.0 (M+H) |
| **Intermediate A-V-F** | 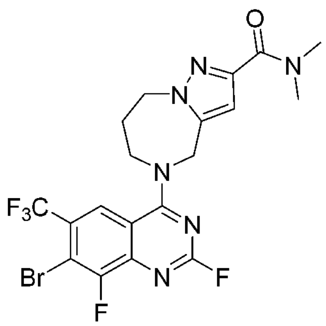 LCMS (m/z): 519.1 (M+H) | **Intermediate D-I-F** | 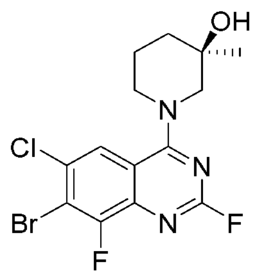 LCMS (m/z): 392.0 (M+H) |
| **Intermediate D-II-F** | 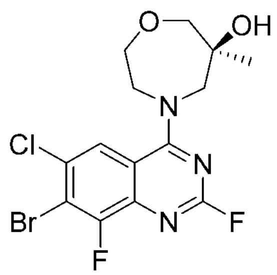 LCMS (m/z): 407.0 (M+H) | **Intermediate D-III-F** | 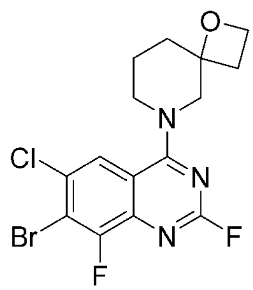 LCMS (m/z): 404.0 (M+H) |

(continued)

| Number | Structural Formula | Number | Structural Formula |
|---|---|---|---|
| **Intermediate D-IV-F** | 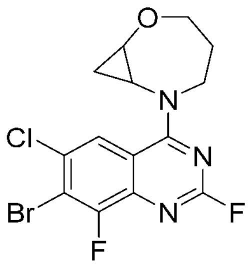 LCMS (m/z): 390.0 (M+H) | **Intermediate D-V-F** | 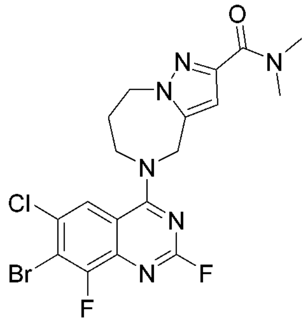 LCMS (m/z): 485.0 (M+H) |
| **Intermediate E-II-F** | 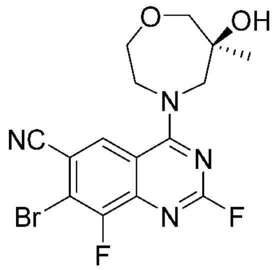 LCMS (m/z): 399.0 (M+H) | **Intermediate K-II-F** | 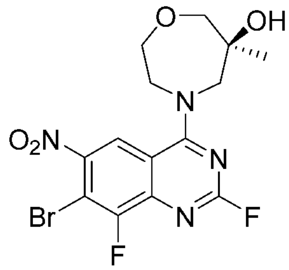 LCMS (m/z): 419.0 (M+H) |
| **Intermediate K-V-F** | 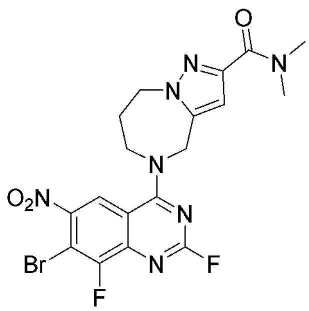 LCMS (m/z): 496.0 (M+H) | **Intermediate O-V-F** | 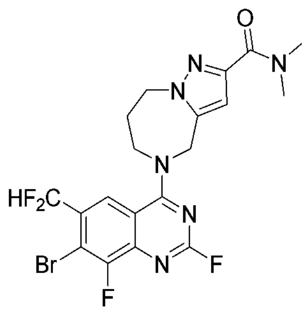 LCMS (m/z): 501.1 (M+H). |
| **Intermediate P-V-F** | 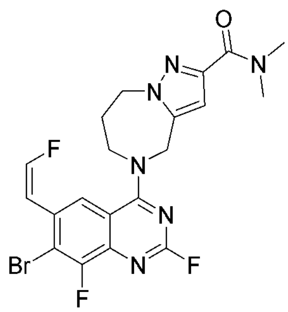 LCMS (m/z): 497.1 (M+H). | **Intermediate Q-V-F** | 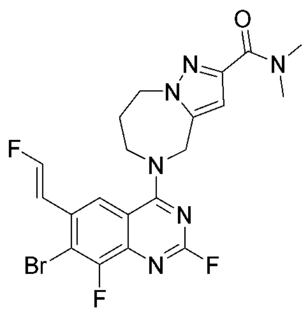 LCMS (m/z): 497.1 (M+H). |

## Intermediate X

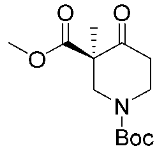

X

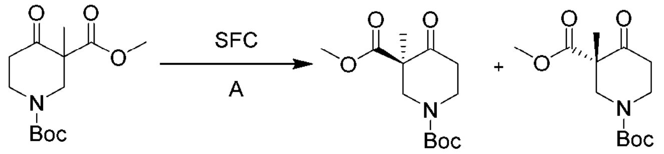


X X-1

Compound 1-(tert-butyl) 3-methyl 3-methyl-4-oxopiperidine-1,3-dicarboxylate (CAS: 193274-53-2, 120 g) was resolved by SFC (SFC150, Waters) (column: DAICEL CHIRALPAK®IG, 250*50 mm, 10 μm; mobile phase: $CO_2$/MeOH=90/10; flow rate: 120 mL/min) to yield the first eluted isomer 1, which is compound **X.** (52.8 g, relatively short retention time). Chiral analysis method-X, Rt=0.682 min. $^1$H NMR (400 MHz, Chloroform-d) δ 4.59 - 4.42 (m, 1H), 4.26 - 3.98 (m, 1H), 3.73 (s, 3H), 3.42 - 3.24 (m, 1H), 3.16 - 3.01 (m, 1H), 2.93 - 2.63 (m, 1H), 2.58 - 2.40 (m, 1H), 1.49 (s, 9H), 1.31 (s, 3H). LCMS (m/z): 216.1 (M-56+H). The subsequent eluted isomer 2 was compound **X-1** (52.4 g, relatively long retention time). Chiral analysis method-X, Rt=1.035 min. $^1$H NMR (400 MHz, Chloroform-d) δ 4.60 - 4.41 (m, 1H), 4.24 - 3.94 (m, 1H), 3.73 (s, 3H), 3.42 - 3.24 (m, 1H), 3.17 - 3.00 (m, 1H), 2.93 - 2.64 (m, 1H), 2.56 - 2.40 (m, 1H), 1.49 (s, 9H), 1.31 (s, 3H).

**Chiral analysis method-X:** Waters UPCC, analytical column: Daicel Chiralpak®IG, 100*3mm 3μm; mobile phase A: $CO_2$, mobile phase B: MeOH; flow rate: 1.5mL/min; column temperature: 35°C; back pressure: 1800psi; gradient: 0-8.0min A/B=90/10.

## Intermediate a

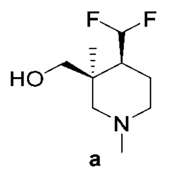

a

((3*S*,4*S*)-4-(difluoromethyl)-1,3-dimethylpiperidine-3-yl)methanol

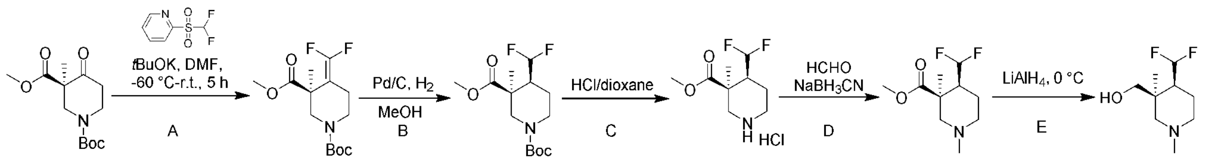


X a-4 a-3 a-2 a-1 a

**Step A:** 1-tert-Butyl 3-methyl (*S*)-4-(difluoromethylene)-3-methylpiperidine-1,3-dicarboxylate

1-tert-Butyl 3-methyl (*R*)-3-methyl-4-oxopyiridine-1,3-dicarboxylate (10.0 g, 36.86 mmol) and 2-((difluoromethyl)sulfonyl)pyridine (10.68 g, 55.29 mmol) were dissolved in anhydrous DMF (100 mL) and substituted with nitrogen three times. The system was cooled in a dry ice-ethanol bath, and 1 mol of potassium tert-butoxide tetrahydrofuran solution (66.34 mL, 66.34 mmol) was added dropwise. The resulting mixture was stirred at the same temperature for 2 h, then slowly raised to room temperature and stirred for another 3 h. After the reaction completion was monitored by LCMS, the reaction was quenched with saturated ammonium chloride aqueous solution (50 mL), and extracted five times with water (200 mL) and DCM/MeOH (100 mL, v/v=10/1). The crude product was washed three times with 100 mL of LiCl aqueous solution (4% w/w), then washed with 100 mL of saturated brine, dried over anhydrous sodium sulfate, filtered, and concentrated to dryness. The crude product was purified by FCC ($SiO_2$, EA/PE=0-20%) to give a pale yellow oily product

(5.2 g, yield 46%). LCMS (m/z): 250.1 (M-56+H).

**Step B:** 1-tert-Butyl 3-methyl (3S,4S)-4-(difluoromethyl)-3-methylpiperidine-1,3-dicarboxylate

At room temperature, 1-tert-butyl 3-methyl (*S*)-4-(difluoromethylene)-3-methylpiperidine-1,3-dicarboxylate (6.20 g, 20.31 mmol) was dissolved in methanol (150 mL) at room temperature and substituted with nitrogen three times. Palladium on carbon (2.16 g, 10% w/w) was added and substituted with hydrogen three times. The mixture was stirred at 30°C for 4 h under a hydrogen atmosphere of 15 Psi. After the reaction completion was monitored by LCMS, the reaction solution was filtered through diatomite, and the filter cake was washed three times with methanol. The filtrate was collected and concentrated to dryness to give a colorless oily product 1-tert-butyl 3-methyl (3*S*,4*S*)-4-(difluoromethyl)-3-methylpiperidine-1,3-dicarboxylate (5.53 g, yield 89%). LCMS (m/z): 252.1 (M-56+H).

**Step C:** Methyl (3*S*,4*S*)-4-(difluoromethyl)-3-methylpiperidine-3-carboxylic acid ester • hydrochloride

At room temperature, 1-tert-butyl 3-methyl (3*S*,4*S*)-4-(difluoromethyl)-3-methylpiperidine-1,3-dicarboxylate (5.53 g, 17.99 mmol) was dissolved in 4 M HCl/dioxane (60 mL). The resulting mixture was stirred at room temperature for 1 h, and the acid solution was concentrated to obtain a white solid methyl (3*S*,4*S*)-4-(difluoromethyl)-3-methylpiperidine-3-carboxylate • hydrochloride (4.38 g, 100% yield). LCMS (m/z): 208.1 (M+H).

**Step D:** methyl (3*S*,4*S*)-4-(difluoromethyl)-1,3-dimethylpiperidine-3-carboxylate

At room temperature, methyl (*3S*,*4S*)-4-(difluoromethyl)-3-methylpiperidine-3-carboxylate hydrochloride (3.58 g, 14.69 mmol) was dissolved in methanol (40 mL), and formaldehyde aqueous solution (3.58 g, 37% w/w, 44.07 mmol) was added. The resulting mixture was stirred at room temperature for 30 min. Sodium cyanoborohydride (1.11 g, 17.63 mmol) was added, and the resulting mixture was stirred at room temperature for 1.5 h. After the reaction completion was monitored by LCMS, the system was concentrated to dryness, the crude product was dissolved in ethyl acetate, and filtered through diatomite. The resulting filtrate was purified by FCC ($SiO_2$, MeOH/DCM (containing 0.3% DIEA)=0-4%) to obtain a colorless oily product, methyl (3*S*,4*S*)-4-(difluoromethyl)-1,3-dimethylpiperidine-3-carboxylate (3.0 g, yield 92%). LCMS (m/z): 222.1 (M+H).

**Step E:** (3*S*,4*S*)-(4-(difluoromethyl)-1,3-dimethylpiperidine-3-yl)methanol

Under ice bath conditions, 1 M $LiAlH_4$-THF (17.63 mL, 17.63 mmol) was added dropwise to a solution of methyl (*3S*,*4S*)-4-(difluoromethyl)-1,3-dimethylpiperidine-3-carboxylate (3.0 g, 13.56 mmol) in anhydrous THF (30 mL). The resulting mixture was stirred at 0°C for 15 min. After the reaction completion was monitored by LCMS, sodium sulfate decahydrate was added to quench the reaction until no more bubbles were generated. Approximately 8 g of anhydrous sodium sulfate was added. The mixture was filtered through diatomite, and the filter cake was washed three times with anhydrous tetrahydrofuran. The filtrate was collected, concentrated to dryness, and a colorless solid product (3*S*,4*S*)-(4-(difluoromethyl)-1,3-dimethylpiperidine-3-yl)methanol (2.6 g, 99% yield) was obtained. LCMS (m/z): 194.1 (M+H).

## Intermediate b

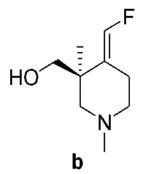

**b**

(*S*,*E*)-(4-(Fluoromethylene)-1,3-dimethylpiperidine-3-yl)methanol

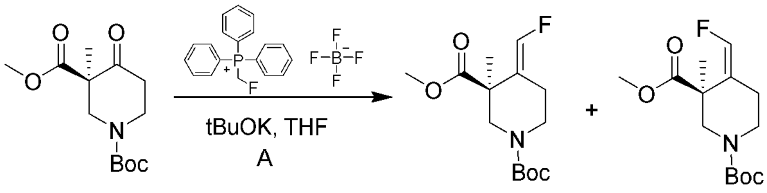


X b-3 b-3-1

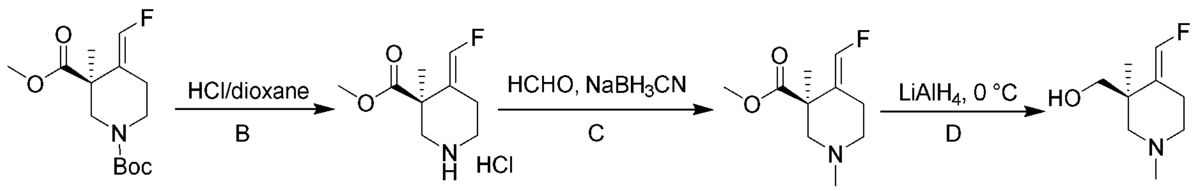


b-3 b-2 b-1 b

**Step A:** 1-tert-Butyl 3-methyl (*S,E*)-4-(fluoromethylene)-3-methylpiperidine-1,3-dicarboxylate and 1-tert-butyl 3-methyl (*S,Z*)-4-(fluoromethylene)-3-methylpiperidine-1,3-dicarboxylate

(Fluoromethylene)triphenylphosphine tetrafluoroborate (10.56 g, 27.64 mmol) was dissolved in anhydrous THF (50 mL), and the solution was substituted with nitrogen three times. Under dry ice and ethanol conditions, the reaction mixture was cooled to -70°C, and a solution of potassium tert-butoxide-tetrahydrofuran (27.64 mL, 1 M, 27.64 mmol) was added dropwise to the reaction system. The temperature was maintained and stirring continued for 1 h. Then, an anhydrous tetrahydrofuran solution(15mL) of 1-(tert-butyl) 3-methyl (*R*)-3-methyl-4-oxopyiridine-1,3-dicarboxylate (5.0 g, 18.43 mmol) was added dropwise to the reaction system. After the addition was complete, the resulting mixture was slowly warmed to room temperature and stirred overnight. After the reaction completion was monitored by TLC, the reaction mixture was slowly poured into water (100 mL) and extracted three times with ethyl acetate. After combining the organic phases, the mixture was washed with saturated brine, dried over anhydrous sodium sulfate, filtered, and concentrated to dryness to obtain a crude product. The crude product was purified by FCC ($SiO_2$, EA/PE=0-15%) to give a colorless oily product 1-tert-butyl 3-methyl (*S,E*)-4-(fluoromethylene)-3-methylpiperidine-1,3-dicarboxylate (1.98 g, yield 37%). LCMS (m/z): 232.1 (M-56+H). $^1H$ NMR (400 MHz, Chloroform-*d*) δ 6.55 (d, *J* = 84.7, 1H), 4.35 (d, *J* = 13.2 Hz, 1H), 4.10 - 3.82 (m, 1H), 3.69 (s, 3H), 3.00 - 2.85 (m, 1H), 2.76 (d, *J* = 13.1 Hz, 1H), 2.71 - 2.60 (m, 1H), 2.31 - 2.08 (m, 1H), 1.46 (s, 9H), 1.29 (s, 3H); and a colorless oily product 1-(tert-butyl) 3-methyl (*S,Z*)-4-(fluoromethylene)-3-methylpiperidine-1,3-dicarboxylate (600 mg, yield 11%). LCMS (m/z): 232.1 (M-56+H). $^1H$ NMR (400 MHz, Chloroform-*d)* δ 6.43 (d, *J* = 83.7, 1H), 3.86 - 3.75 (m, 1H), 3.71 (s, 3H), 3.62 - 3.47 (m, 1H), 3.42 - 3.29 (m, 2H), 2.24 - 2.06 (m, 2H), 1.46 (s, 9H), 1.43 - 1.39 (m, 3H).

**Step B:** Methyl (*S,E*)-4-(fluoromethylene)-3-methylpiperidine-3-carboxylate • hydrochloride

At room temperature, 10 mL of 4 M HCl- dioxane was added to 1-tert-butyl 3-methyl (*S,E*)-4-(fluoromethylene)-3-methylpiperidine-1,3-dicarboxylate (600 mg, 2.09 mmol), and the mixture was stirred at room temperature for 1 h. The acid solution was concentrated to obtain a white solid methyl (*S,E*)-4-(fluoromethylene)-3-methylpiperidine-3-carboxylate • hydrochloride (572 mg, 100% yield). LCMS (m/z): 188.1 (M+H).

**Step C:** Methyl (*S,E*)-4-(fluoromethylene)-1,3-dimethylpiperidine-3-carboxylate

At room temperature, methyl (*S,E*)-4-(fluoromethylene)-3-methylpiperidine-3-carboxylate • hydrochloride (370 mg, 1.98 mmol) was dissolved in methanol (5 mL). Triethylamine was added dropwise to bring the pH of the reaction solution to approximately 10, and the mixture was stirred for 10 minutes. Then, glacial acetic acid was added dropwise to bring the pH of the reaction solution to approximately 4. Formaldehyde aqueous solution (481.15 mg, 5.93 mmol) was added to the reaction solution, and the mixture was stirred at room temperature for 30 minutes. Sodium cyanoborohydride (136.62 mg, 2.17 mmol) was added to the reaction solution, and the mixture was stirred at room temperature for 2 hours. After the reaction completion was monitored by LCMS, the solvent was removed by concentration under reduced pressure. After distillation twice with anhydrous tetrahydrofuran, a white solid methyl (*S,E*)-4-(fluoromethylene)-1,3-dimethylpiperidine-3-carboxylate (380 mg, 96% yield) was obtained. LCMS (m/z): 202.1 (M+H).

**Step D:** (*S,E*)-(4-(fluoromethylene)-1,3-dimethylpiperidine-3-yl)methanol

Under ice bath conditions, 1 M $LiAlH_4$-THF solution (2.83 mL, 107.5 mg, 2.83 mmol) was added dropwise to an anhydrous tetrahydrofuran (5 mL) solution of methyl (*E*)-4-(fluoromethylene)-1,3-dimethylpiperidine-3-carboxylate (380 mg, 1.89 mmol). The resulting mixture was stirred at room temperature for 20 min. After the reaction completion was monitored by LCMS, the reaction solution was quenched with sodium sulfate decahydrate until no more bubbles were generated. Approximately 5 g of anhydrous sodium sulfate was added to remove water. The reaction solution was filtered through diatomite, and the filter cake was washed three times with anhydrous tetrahydrofuran. The filtrate was collected and concentrated to dryness to give a colorless oily product (*S,E*)-(4-(fluoromethylene)-1,3-dimethylpiperidine-3-yl) methanol (300 mg, yield 92%). LCMS (m/z): 174.1 (M+H).

## Intermediate c

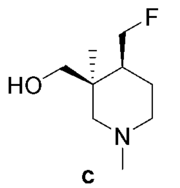

c

((3*S*,4*S*)-4-(Fluoromethyl)-1,3-dimethylpiperidine-3-yl)methanol

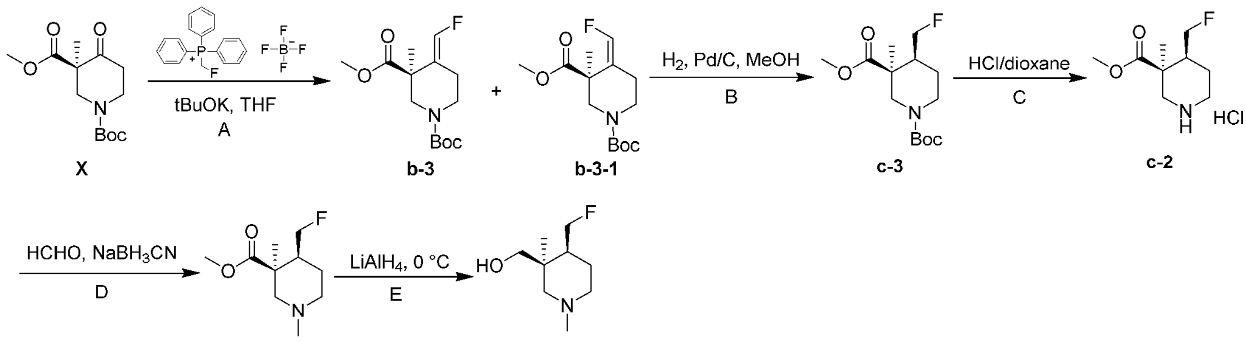


X, b-3, +, b-3-1, c-3, c-2, c-1, c

**Step A:** 1-tert-Butyl 3-methyl (*S,E*)-4-(fluoromethylene)-3-methylpiperidine-1,3-dicarboxylate and 1-tert-butyl 3-methyl (*S,Z*)-4-(fluoromethylene)-3-methylpiperidine-1,3-dicarboxylate

The synthesis in step A is carried out in accordance with the synthesis in step A for intermediate **b.**

**Step B:** 1-(tert-Butyl) 3-methyl (3*S*,4*S*)-4-(fluoromethyl)-3-methylpiperidine-1,3-dicarboxylate

Under nitrogen protection at room temperature, wet Pd/C (400 mg, 10% w/w) was added to a methanol (10 mL) solution of a mixture of 1-tert-butyl 3-methyl (*S,E*)-4-(fluoromethylene)-3-methylpiperidine-1,3-dicarboxylate and 1-tert-butyl 3-methyl (*S,Z*)-4-(fluoromethylene)-3-methylpiperidine-1,3-dicarboxylate (850 mg, 2.96 mmol). The system was replaced with a hydrogen balloon, and the reaction was stirred overnight in a hydrogen atmosphere. After the reaction completion was monitored by TLC, the solution was filtered through diatomite. The filtrate was concentrated to dryness to obtain a crude product, which was then purified by FCC ($SiO_2$, EA /PE=0-10%) to obtain a colorless oily product 1-(tert-butyl) 3-methyl (3*S*,4*S*)-4-(fluoromethyl)-3-methylpiperidine-1,3-dicarboxylate (600 mg, yield 70%). LCMS (m/z): 234.1 (M-56+H), 312.1 (M+Na). $^1H$ NMR (400 MHz, methanol-d4) δ 4.76 - 4.70 (m, 0.5 H), 4.64 - 4.52 (m, 1H), 4.46 - 4.41 (m, 0.5 H), 4.23 (d, *J* = 13.7 Hz, 1H), 4.08 - 3.99 (m, 1H), 3.67 (s, 3H), 3.06 - 2.86 (m, 1H), 2.86 - 2.70 (m, 1H), 1.95 - 1.81 (m, 1H), 1.80 - 1.68 (m, 2H), 1.46 (s, 9H), 1.27 (s, 3H). $^{19}F$ NMR (376 MHz, methanol-*d*4) δ 221.80.

**Step C:** Methyl (3*S*,4*S*)-4-(fluoromethyl)-3-methylpiperidine-3-carboxylate • hydrochloride

At room temperature, 3M dioxane hydrochloride (10 mL, 30 mmol) was added dropwise to a solution of 1-(tert-butyl) 3-methyl (3*S*,4*S*)-4-(fluoromethyl)-3-methylpiperidine-1,3-dicarboxylate (600 mg, 2.07 mmol) in 10 mL of ethyl acetate. The resulting mixture was stirred at room temperature for 1 h. The reaction completion was monitored by LCMS,

and the solution was concentrated under reduced pressure to give a white solid methyl (3*S*,4*S*)-4-(fluoromethyl)-3-methylpiperidine-3-carboxylate • hydrochloride (500 mg, crude product). LCMS (m/z): 190.1 (M+H).

**Step D:** Methyl (3*S*,4*S*)-4-(fluoromethyl)-1,3-dimethylpiperidine-3-carboxylate

At room temperature, methyl (3*S*,4*S*)-4-(fluoromethyl)-3-methylpiperidine-3-carboxylate • hydrochloride (500 mg, crude product from the previous step) was dissolved in methanol (10 mL), and formaldehyde aqueous solution (2 mL, 35-40 % w/w, ~24 mmol) was added. The mixture was stirred at room temperature for 2 h. Sodium cyanoborohydride (696 mg, 11.1 mmol) was added in portions, and the resulting mixture was stirred at room temperature for 2 h. The reaction completion was monitored by LCMS. Saturated $NH_4Cl$ aqueous solution was added to the reaction mixture, and the product was extracted three times with ethyl acetate. The organic phases were combined and concentrated to obtain a crude product, which was purified by FCC ($SiO_2$, EA/PE=0-100%) to obtain a colorless oily product methyl (3*S*,4*S*)-4-(fluoromethyl)-1,3-dimethylpiperidine-3-carboxylate (200 mg, yield 44%). LCMS (m/z): 204.1 (M+H).

**Step E:** ((3*S*,4*S*)-4-(Fluoromethyl)-1,3-dimethylpiperidine-3-yl)methanol

At room temperature, $LiAlH_4$-THF (1.7 mL, 1 M, 1.7 mmol) was slowly added dropwise to a solution of methyl (3*S*,4*S*)-4-(fluoromethyl)-1,3-dimethylpiperidine-3-carboxylate (200 mg, 0.869 mmol) in anhydrous THF (2 mL). The mixture was stirred at room temperature for 0.5 hours. After the reaction completion was monitored by TLC, the mixture was cooled in an ice bath, and sodium sulfate decahydrate was added to quench the reaction until no more bubbles were generated. A suitable amount of ethyl acetate was added, and the mixture was dried over anhydrous sodium sulfate, filtered, and concentrated to dryness to give a colorless, oily crude product ((3*S*,4*S*)-4-(fluoromethyl)-1,3-dimethylpiperidine-3-yl)methanol (100 mg, yield 58%). LCMS (m/z): 176.1 (M+H).

## Intermediate d

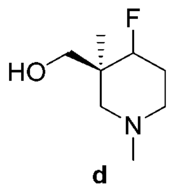

**d**

(3*S*)-(4-Fluoro-1,3-dimethylpiperidine-3-yl)methanol

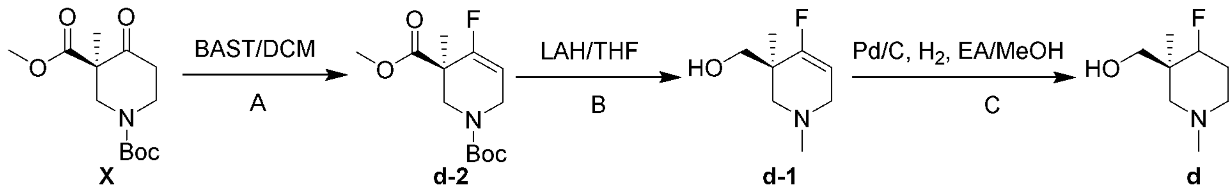


**X** **d-2** **d-1** **d**

**Step A:** 1-(tert-Butyl) 3-methyl (*S*)-4-fluoro-3-methyl-3,6-dihydropyridine-1,3(2*H*)-dicarboxylate

Under ice bath conditions, BAST (19.57 g, 16.3 mL, 88.46 mmol) was added dropwise to a mixed solution of 1-(tert-butyl) 3-methyl (*R*)-3-methyl-4-oxopiperidine-1,3-dicarboxylate (8.0 g, 29.49 mmol) and DCM (40 mL). After the addition was complete, the mixture was stirred overnight at room temperature. After the reaction completion was monitored by TLC, the reaction solution was slowly poured into a semi-saturated $NaHCO_3$ (100 mL) solution and extracted with DCM (100 mL×3). The combined organic phases were concentrated and purified by FCC ($SiO_2$, EA/PE=0-10%) to obtain a colorless oily substance 1-(tert-butyl) 3-methyl (*S*)-4-fluoro-3-methyl-3,6-dihydropyridine-1,3(2*H*)-dicarboxylate (1.0 g, yield 12%). LCMS (m/z): 218.1 (M-56+H).

**Step B:** (*S*)-(4-Fluoro-1,3-dimethyl-1,2,3,6-tetrahydropyridine-3-yl)methanol

At room temperature, $LiAH_4$ (4.02 mL, 4.02 mmol, 1 M THF solution) was added to 1-(tert-butyl) 3-methyl (*S*)-4-fluoro-3-methyl-3,6-dihydropyridine-1,3(2*H*)-dicarboxylate (500 mg, 1.83 mmol) and warmed to 70°C with stirring for 1 h. After the reaction completion monitored by LCMS, $Na_2SO_4$ • $10H_2O$ was slowly added to the reaction solution under ice bath conditions to quench $LiAH_4$ until no more gas was generated. Then, anhydrous sodium sulfate solution was added to

dry the solution, filtered, and the mother liquor was collected and concentrated to obtain a colorless oily liquid (*S*)-(4-fluoro-1,3-dimethyl-1,2,3,6-tetrahydropyridine-3-yl)methanol (284 mg, yield 98%). LCMS (m/z): 160.1 (M+H).

**Step C:** (3*S)*-(4-Fluoro-1,3-dimethylpiperidine-3-yl)methanol

Under nitrogen protection at room temperature, Pd/C (5% w/w, 374 mg, 0.176 mmol) was added to a mixed solution of (S)-(4-fluoro-1,3-dimethyl-1,2,3,6-tetrahydropyridine-3-yl)methanol (280 mg, 1.76 mmol) and EA:MeOH=1:1 (20 mL). The mixture was then purged with hydrogen. The reaction was carried out at room temperature under 60 psi $H_2$ pressure for 2 h. After the reaction completion was monitored by TLC, the reaction solution was filtered through diatomite, washed with EA (50 ml), and the organic phase was concentrated to give a colorless oily liquid (3*S)*-(4-fluoro-1,3 -dimethylpiperidine-3-yl)methanol (220 mg, yield 78%). LCMS (m/z): 162 (M+H).

The following intermediates were prepared according to the above synthetic scheme or appropriate variants:

| Number | Structural Formula | Number | Structural Formula |
|---|---|---|---|
| **Intermediate a-E** | 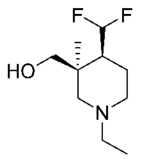 LCMS (m/z): 208.1 (M+H) | **Intermediate b-E** | 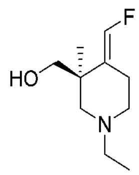 LCMS (m/z): 188.1 (M+H) |
| **Intermediate c-E** | 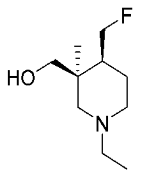 LCMS (m/z): 190.1 (M+H) | **Intermediate d-E** | 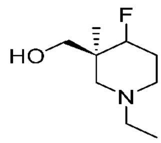 LCMS (m/z): 176.1 (M+H) |

## Intermediate b-d3

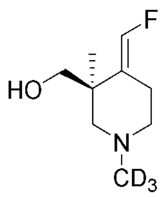

**b-d3**

(*S,E*)-(4-(Fluoromethylene)-3-methyl-1-(methyl-$d_3$)piperidine-3-yl)methanol

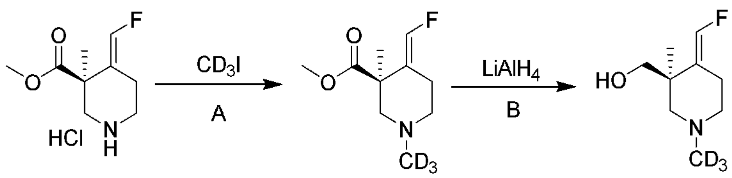


**b-2** **b-d3-1** **b-d3**

**Step A:** Methyl (*S,E*)-4-(fluoromethylene)-3-methyl-1-(methyl-$d_3$)piperidine-3-carboxylate

At room temperature, potassium carbonate (5.55 g, 40.2 mmol) was added to a mixed solution of methyl (*S,E*)-4-(fluoromethylene)-3-methylpiperidine-3-carboxylate hydrochloride (3.00 g, 13.4 mmol), deuterated iodomethane (2.33 g, 16.1 mmol), and ACN (100 mL). After the addition was complete, the mixture was heated to 90°C and stirred overnight. After the reaction completion was monitored by LCMS, the mixture was filtered, and the filter cake was washed with EA (50 mL). The filtrate was collected, concentrated, and further purified by FCC ($SiO_2$, EA /PE=0-20%) to obtain a

colorless liquid methyl (*S,E*)-4-(fluoromethylene)-3-methyl-1-(methyl-*$d_3$*)piperidine-3-carboxylate (1.9 g, yield 69%). LC-MS (m/z): 205.1 (M+H).

**Step B:** (*S,E*)-(4-(Fluoromethylene)-3-methyl-1-(methyl-*$d_3$*)piperidine-3-yl)methanol

Under ice bath conditions, $LiAlH_4$ (1M-THF, 9.3 mmol, 9.3 mL) was added dropwise to a mixed solution of methyl (*S,E*)-4-(fluoromethylene)-3-methyl-1-(methyl-*$d_3$*)piperidine-3-carboxylate (1.9 g, 9.3 mmol) and THF (50 mL). After the addition was complete, the mixture was stirred in an ice bath at 0°C for 0.5 h. After the reaction completion was monitored by LC-MS, the reaction was quenched with $Na_2SO_4 \cdot 10H_2O$ until no gas was produced. The reaction solution was filtered through diatomite, and the filtrate was concentrated at low temperature (35°C) to obtain a colorless liquid, (*S,E*)-4-(fluoromethylene)-3-methyl-1-(methyl-*$d_3$*)piperidine-3-yl)methanol (1.3 g, 79% yield). LC-MS (m/z): 177.1 (M+H).

## Intermediate b-d5

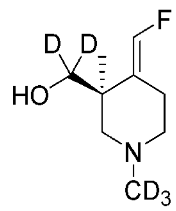

**b-d5**

(*S,E*)-(4-(Fluoromethylene)-3-methyl-1-(methyl-*$d_3$*)piperidine-3-yl)methylene-*$d_2$*-ol

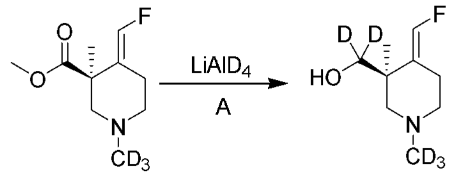


**b-d3-1** **b-d5**

**Step A:** (*S,E*)-(4-(Fluoromethylene)-3-methyl-1-(methyl-*$d_3$*)piperidine-3-yl)methylene-*$d_2$*-ol

Under ice bath conditions, $LiAlD_4$ powder (271.28 mg, 6.46 mmol) was added to a solution of methyl (*S,E*)-4-(fluoromethylene)-3-methyl-1-(methyl-*$d_3$*)piperidine-3-carboxylate (1.10 g, 5.39 mmol) in 15 mL of anhydrous tetrahydrofuran. The resulting mixture was stirred at room temperature for 15 min. After the reaction completion was monitored by LCMS, the reaction solution was quenched with sodium sulfate decahydrate until no more bubbles were generated. Approximately 5 g of anhydrous sodium sulfate was then added to the reaction solution to remove water. The reaction solution was filtered through diatomite, and the filter cake was washed three times with anhydrous tetrahydrofuran. The filtrate was collected and concentrated to dryness to give a colorless oily product (*S,E*)-(4-(fluoromethylene)-3-methyl-1-(methyl-*$d_3$*)piperidine-3-yl)methylene-*$d_2$*-ol (951 mg, 99% yield). This product was used directly in the next step reaction without purification. LCMS (m/z): 179.1 (M+H).

The following intermediates were synthesized using the method described above.

| Number | Structural Formula | Number | Structural Formula |
|---|---|---|---|
| **Intermediate a-d3** | 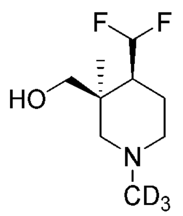 LCMS (m/z): 197.1 (M+H) | **Intermediate a-d5** | 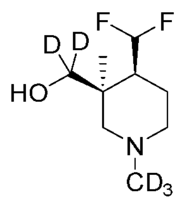 LCMS (m/z): 199.1 (M+H) |

(continued)

| Number | Structural Formula | Number | Structural Formula |
|---|---|---|---|
| **Intermediate a-E-d2** | 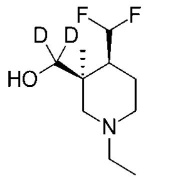 LCMS (m/z): 210.1 (M+H) | **Intermediate bE-d2** | 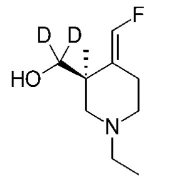 LCMS (m/z): 190.1 (M+H) |
| **Intermediate aE-d5** | 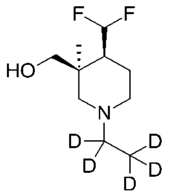 LCMS (m/z): 213.2 (M+H) | **Intermediate bE-d5** | 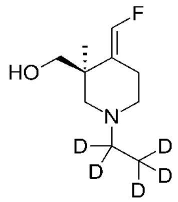 LCMS (m/z): 193.2 (M+H) |
| **Intermediate aE-d7** | 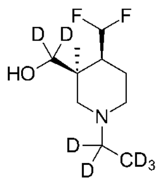 LCMS (m/z): 215.2 (M+H) | **Intermediate bE-d7** | 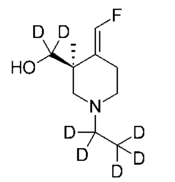 LCMS (m/z): 195.2 (M+H) |

## Intermediate w1

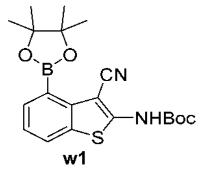

w1

tert-Butyl (3-cyano-4-(4,4,5,5-tetramethyl-1,3,2-dioxaborolan-2-yl)benzo[*b*]thiophene-2-yl) carbamate

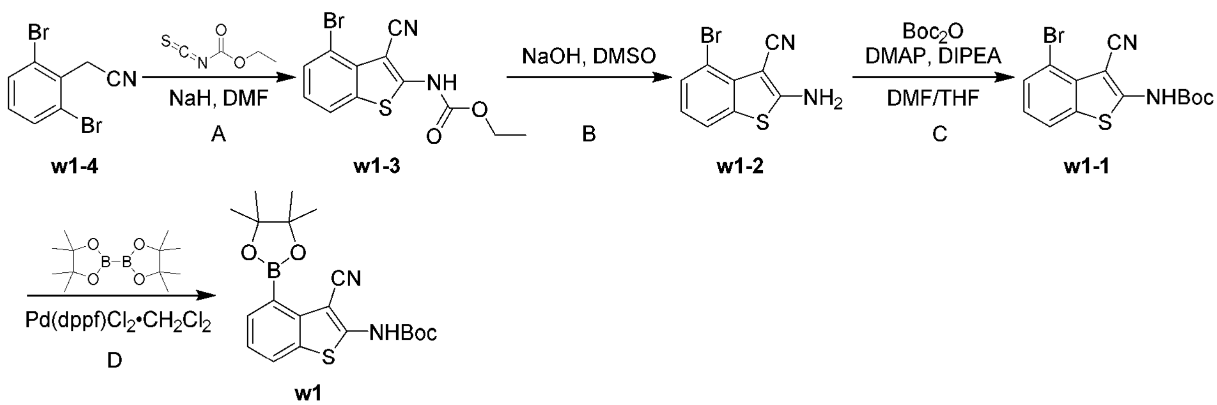


**Step A:** ethyl (4-bromo-3-cyanobenzo[*b*]thiophene-2-yl) carbamate

Under an ice bath and nitrogen atmosphere, sodium hydride (60 wt%, 0.73 g, 22 mmol) was added to a solution of 2-(2,6-dibromophenyl) acetonitrile (5.0 g, 18 mmol) in DMF (50 mL), and the reaction mixture was stirred at 0°C for 10 min. Ethyl isothiocyanate (2.14 mL, 18 mmol) was slowly added to the reaction mixture. After the addition was complete, the reaction mixture was brought to room temperature and stirred continuously for 1 h. The reaction mixture was then heated to 100°C and stirred for 1 h. The DMF was removed by vacuum concentration, and water (100 mL) and ethyl acetate (10 mL)

were added. The resulting mixture was stirred at room temperature for 15 min. After stirring, a large amount of yellow solid precipitated. The solid was filtered, washed with water, and dried to obtain 3.9 g of yellow solid ethyl (4-bromo-3-cyanobenzo[*b*]thiophene-2-yl) carbamate. $^1H$ NMR (400 MHz, DMSO-$d_6$) δ 11.69 (s, 1H), 7.93 (dd, *J* = 8.0, 1.0 Hz, 1H), 7.60 (dd, *J* = 7.8, 1.0 Hz, 1H), 7.28 - 7.14 (m, 1H), 4.24 (q, *J* = 7.1 Hz, 2H), 1.29 (t, *J* = 7.1 Hz, 3H).

**Step B:** 2-amino-4-bromobenzo[*b*]thiophene-3-carbonitrile

Sodium hydroxide (3.7 g, 93 mmol) was added to a mixture of (3.9 g, 12 mmol) of ethyl carbamate in DMSO (12 mL) and water (18 mL). The reaction mixture was stirred at 125°C for 16 h. The reaction mixture was cooled to room temperature, poured into 100 mL of ice water, filtered, washed with water, and dried to give a pale yellow solid, 2-amino-4-bromobenzo[*b*]thiophene-3-nitrile (2.2 g). $^1H$ NMR (400 MHz, DMSO-$d_6$) δ 7.96 (s, 2 H), 7.70 (dd, *J* = 7.9, 1.0 Hz, 1 H), 7.46 (dd, *J* = 7.9, 1.0 Hz, 1 H), 7.14 - 6.96 (m, 1H).

**Step C:** tert-Butyl (4-bromo-3-cyanobenzo[*b*]thiophene-2-yl)carbamate

To a mixed solution of 2-amino-4-bromobenzo[*b*]thiophene-3-carbonitrile (2.2 g, 8.7 mmol) in DMF (29 mL) and THF (4.5 mL), 4-dimethylaminopyridine (104 mg, 0.85 mmol) and diisopropylethylamine (2.3 mL, 26 mmol) were added sequentially. Then, di-tert-butyl dicarbonate (2.2 mL, 9.5 mmol) was slowly added dropwise. After the addition was complete, the reaction system was stirred at room temperature for 24 h. After the reaction was complete, water (100 mL) was added to the reaction system, and a yellow solid precipitated. The solid was collected by filtration, washed with water, and dried to obtain a pale yellow solid (1.4 g) of tert-butyl (4-bromo-3-cyanobenzo[*b*]thiophene-2-yl)carbamate.

**Step D:** tert-Butyl (3-cyano-4-(4,4,5,5-tetramethyl-1,3,2-dioxaborolan-2-yl)benzo[*b*]thiophene-2-yl)carbamate

tert-Butyl (4-bromo-3-cyanobenzo[*b*]thiophene-2-yl)carbamate (1.4 g, 3.9 mmol), bis(pinacolato)diboron (1.5 g, 5.9 mmol), potassium acetate (0.77 g, 7.8 mmol), and 1,1'-bis(diphenylphosphine) ferrocene]palladium dichloromethane dichloride complex (0.3 g, 0.35 mmol) were sequentially added to a 50 mL round-bottom flask, followed by 5 mL of 1,4-dioxane. The reaction was then substituted under a nitrogen atmosphere and heated to 105°C for 3 h. After the reaction was completed, the filtrate was collected and concentrated. The crude product was purified by FCC ($SiO_2$, petroleum ether/tetrahydrofuran~0-30%) to obtain a pale yellow solid tert-butyl (3-cyano-4-(4,4,5,5-tetramethyl-1,3,2-dioxaborolan-2-yl) benzo[*b*]thiophene-2-yl)carbamate (1.6 g). LCMS (m/z): 423.0 (M+Na).

## Intermediate w1b

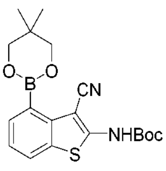

**w1b**

tert-butyl (3-cyano-4-(5,5-dimethyl-1,3,2-dioxaborolan-2-yl)benzo[*b*]thiophene-2-yl)carbamate

The synthesis of intermediate **wlb** was carried out in accordance with intermediate **w1,** with 5,5,5',5'-tetramethyl-2,2'-bis(1,3,2-dioxoboronane) used instead of bis(pinacolato)diboron in step D.

## Intermediate w2

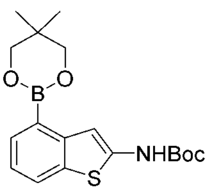

**w2**

tert-Butyl (4-(5,5-dimethyl-1,3,2-dioxaborolan-2-yl) benzo[*b*]thiophene-2-yl)carbamate

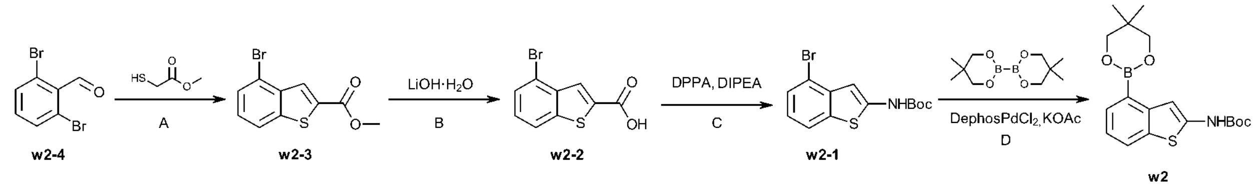

w2-4 w2-3 w2-2 w2-1 w2

**Step A:** methyl 4-bromobenzo[*b*]thiophene-2- carboxylate

At room temperature, compound 2,6-dibromobenzaldehyde (15.7 g, 59.5 mmol), methyl mercaptoacetate (5.6 mL, 65.4 mmol), $K_2CO_3$ (15.7 g, 119.0 mmol), and DMF (200 mL) were sequentially added to a reaction flask equipped with a magnetic stir bar. The resulting mixture was heated to 110°C and stirred for 10 h. The reaction completion was monitored by TLC. The reaction solution was poured into 300 mL of water, resulting in the precipitation of a large amount of yellow solid. The mixture was filtered, the filter cake was washed with water, and dried at 50°C to obtain a crude product methyl 4-bromobenzo[*b*]thiophene-2- carboxylate (18 g), LCMS (m/z): 270.9 (M+H).

**Step B:** 4-bromobenzo[*b*]thiophene-2-carboxylic acid

Added LiOH·$H_2O$ (5.4 mL, 129.7 mmol) and methyl 4-bromobenzo[b]thiophene-2- carboxylate (7.0 g, 25.9 mmol) sequentially to a mixed solution of THF (10 mL) and $H_2O$ (2 mL). The mixture was stirred at room temperature for 16 h. After the reaction completion monitored by LCMS, the pH of the system was adjusted to 3 with 1 M hydrochloric acid, extracted with EA (60 mL×3), dried over anhydrous $Na_2SO_4$, filtered, and concentrated under reduced pressure to obtain a crude product 4-bromobenzo[b]thiophene-2-carboxylic acid (8.0 g), LCMS (m/z): 256.9 (M+H).

**Step C:** tert-Butyl (4-bromobenzo[*b*]thiophene-2-yl)carbamate

4-Bromobenzo[*b*]thiophene-2-carboxylic acid (2.5 g), DPPA (3.2 mL, 14.7 mmol), DIEA (2.0 mL, 19.5 mmol), toluene (25 mL), and tert-butanol (1.4 mL, 14.7 mmol) was added sequentially to a reaction flask, and the resulting mixture was stirred at 100°C for 16 h. After the reaction completion was monitored by LCMS, the mixture was concentrated under reduced pressure, and the crude product was purified by FCC ($SiO_2$, EA/PE=0-30%) to obtain tert-butyl (4-bromobenzo[*b*]thiophene-2-yl)carbamate (3.0 g), LCMS (m/z): 271.9 (M-56).

**Step D:** tert-Butyl (4-(5,5-dimethyl-1,3,2-dioxaborolan-2-yl) benzo[*b*]thiophene-2-yl)carbamate

(4-bromobenzo[*b*]thiophene-2-yl)carbamate (2.9 g, 8.9 mmol), 5,5,5',5'- tetramethyl-2,2'-bis(1,3,2-dioxaborane) (5.0 g, 22.3 mmol), $DephosPdCl_2$ (0.64 g, 0.9 mmol), KOAc (2.6 g, 26.7 mmol), and 1,4-dioxane (30 mL) was added sequentially to the reaction flask. The mixture was substituted with nitrogen three times, and the reaction solution was stirred at 95°C for 1 h. After the reaction completion monitored by LCMS, the product was concentrated under reduced pressure. The crude product was purified by FCC ($SiO_2$, EA/PE=0-30%) to obtain tert-butyl (4-(5,5-dimethyl-1,3,2-dioxaborolan-2-yl) benzo[*b*]thiophene-2-yl)carbamate (3.1 g, yield 98%).

## Intermediate w3

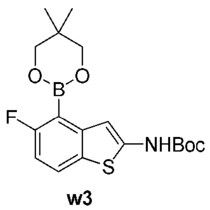
w3

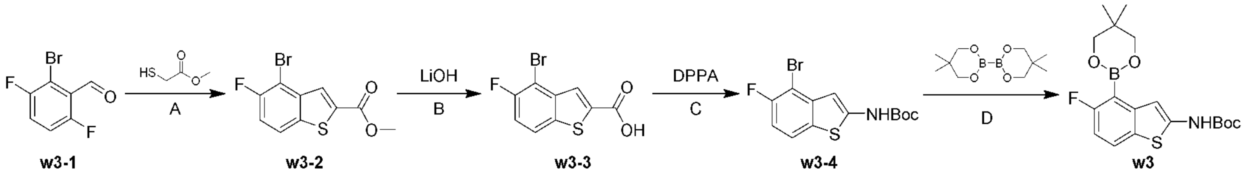

w3-1 w3-2 w3-3 w3-4 w3

**Step A:** Compound **w3-2**

Under nitrogen protection and at room temperature, potassium carbonate (37.5 g, 271.4 mmol) and methyl mercaptoacetate (17.3 g, 169.2 mmol) were added to compound **w3-1** (30 g, 135.7 mmol) in THF solution (500 mL). The resulting reaction solution was warmed to 45°C and stirred for 4 h, then warmed to 90°C and stirred for 16 h. After the reaction completion monitored by LCMS, the reaction solution was concentrated to dryness, water was added to the concentrate, and the solution was extracted with ethyl acetate. The organic phases were combined, washed with saturated brine, dried over anhydrous sodium sulfate, filtered, and concentrated to dryness. The crude product was purified by FCC ($SiO_2$, DCM/PE=0-8%) to give a white solid compound **w3-2** (24.3 g, yield 62%). LCMS (m/z): 288.9 (M+H).

**Step B:** Compound **w3-3**

Under nitrogen protection, lithium hydroxide monohydrate (10.6 g, 252.2 mmol) was added to a solution of compound **w3-2** (24.3 g, 84.1 mmol) in THF (170 mL) and water (57 mL). The resulting mixture was stirred at room temperature for 3 h. After the reaction completion was monitored by LCMS, the reaction solution was concentrated to dryness, and dilute hydrochloric acid aqueous solution (1 N) was added to the concentrated residue until pH≈5, leading to solid precipitation. The mixture was stirred at room temperature for 0.5 h and then filtered. The filter cake was washed with water and dried under vacuum to give a white solid compound **w3-3** (22.5 g, 98% yield). LCMS (m/z): 274.9 (M+H).

**Step C:** Compound **w3-4**

Under nitrogen protection, triethylamine (11.6 g, 115.4 mmol) and diphenyl azidophosphate (27.2 g, 98.9 mmol) were added to a solution of compound **w3-3** (22.5 g, 82.4 mmol) in anhydrous toluene (135 mL) and anhydrous tert-butanol (45 mL). The resulting mixture was stirred at 100°C for 16 h. After the reaction completion was monitored by LCMS, the reaction solution was poured into 100 mL of saturated sodium bicarbonate aqueous solution and extracted with ethyl acetate. The organic phases were combined, washed with saturated brine, dried over anhydrous sodium sulfate, filtered, concentrated to dryness, and the crude product was purified by slurry mixing with EA/PE=1/10 (80 mL) to give a yellow solid compound **w3-4** (28 g, 98% yield). LCMS (m/z): 289.9 (M+H).

**Step D:** Intermediate **w3**

Under nitrogen protection at room temperature, neopentyl glycol diboronate (4.88 g, 21.7 mmol), potassium acetate (2.83 g, 28.9 mmol), and DPEphosPd$Cl_2$ (1.04 g, 1.45 mmol) were added to a solution of compound **w3-4** (5 g, 14.5 mmol) in 1,4-dioxane (80 mL). The resulting reaction solution was heated to 90°C and stirred for 16 h. After the reaction completion was monitored by LCMS, the reaction solution was filtered through diatomite, the filter cake was washed with ethyl acetate, the filtrate was concentrated to dryness, and the crude product was purified by FCC ($SiO_2$, EA/PE=0-10%) to give a white solid intermediate product **w3** (3.2 g, yield 58%). LCMS (m/z): 256.0 (M+H-68-56). $^1$H NMR (400 MHz, DMSO-$d_6$) δ 10.73 (s, 1H), 7.86 - 7.77 (m, 1H), 7.14 (d, $J$ = 0.7 Hz, 1H), 6.90 (dd, $J$ = 9.8, 8.6 Hz, 1H), 3.81 (s, 4H), 1.49 (s, 9H), 1.02 (s, 6H).

## Intermediate w4

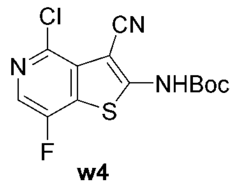

**w4**

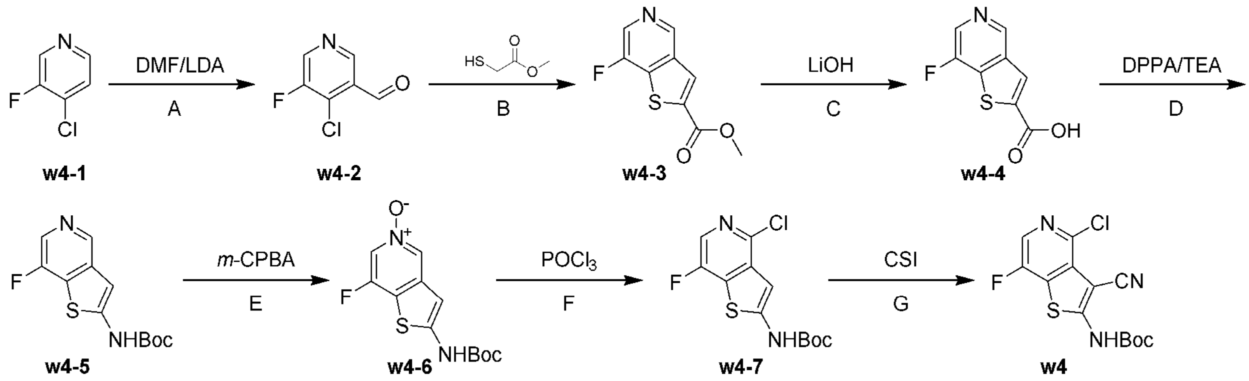


w4-1 w4-2 w4-3 w4-4 w4-5 w4-6 w4-7 w4

**Step A:** Compound **w4-2**

Under dry ice and ethanol bath conditions, LDA (143 mL, 2 M THF solution, 285 mmol) was added to a mixed solution of compound **w4-1** and DMF (25.0 g, 190 mmol) and THF (300 mL), and stirred for 5.0 h under dry ice and ethanol bath conditions. Then, under dry ice and ethanol bath conditions, DMF (41.7 g, 570 mmol) was then added to the reaction solution and stirred for 1.0 h after heating to room temperature. The reaction completion was monitored by LCMS. The reaction solution was quenched in a saturated solution of $NH_4Cl$ (300 mL), extracted with EA (200 mL×3), and the organic phase was collected. The mixture was washed with saturated brine (500 mL), dried over anhydrous $Na_2SO_4$, filtered, and the crude product obtained by concentrating the organic phase was purified by FCC ($SiO_2$, EA/PE=0-15%) to give a pale yellow oily product **w4-2** (13.9 g, yield 46%). LCMS (m/z): 177.9 (M+$H_2O$).

**Step B:** Compound **w4-3**

At room temperature, $K_2CO_3$ (18.0 g, 130 mmol) and methyl mercaptoacetate (10.7 g, 100 mmol) were added sequentially to a mixed solution of compound **w4-2** (16.0 g, 100 mmol) and DMF (250 mL), and stirred at room temperature for 2.0 h. After the reaction completion was monitored by LCMS, the reaction mixture was added dropwise to water (1000 mL), and stirred at room temperature for 0.5 h. The resulting filter cake was dried in a vacuum drying oven (50°C, 16 h) to obtain a white solid product **w4-3** (15.0 g, yield 71%). LCMS (m/z): 212.0 (M+H).

**Step C:** Compound **w4-4**

At room temperature, LiOH·$H_2O$ (8.94 g, 213 mmol) and compound **w4-3** (15.0 g, 71.0 mmol) were added sequentially to a mixed solution of MeOH (80.0 mL), $H_2O$ (80.0 mL), and THF (160 mL), and stirred at room temperature for 0.5 h. The reaction completion was monitored by LCMS. The reaction solution was concentrated to obtain a crude product, which was then added to water (150 mL). The pH was adjusted to 3 with hydrochloric acid (1M). The filter cake obtained after filtration was dried in a vacuum drying oven (50°C, 16 h) to obtain a white solid product **w4-4** (14.0 g, yield 100 %). LCMS (m/z): 198.0 (M+H).

**Step D:** Compound **w4-5**

At room temperature, DPPA (29.3 g, 107 mmol), TEA (14.4 g, 142 mmol), and compound **w4-4** (14.0 g, 71.0 mmol) were sequentially added to a mixed solution of toluene (150 mL) and tert-butanol (50 mL), and the mixture was warmed to 90°C and stirred for 16 h. The reaction completion was monitored by LCMS. The crude product obtained by concentrating the reaction solution was quenched in saturated sodium bicarbonate aqueous solution (150 mL), extracted with EA (80 mL×3), and the organic phase was collected. The mixture was washed with saturated brine (150 mL), dried over anhydrous $Na_2SO_4$, filtered, and the organic phase was concentrated and purified by FCC ($SiO_2$, EA/PE=0-50%) to give a white solid product **w4-5** (9.0 g, yield 47%). LCMS (m/z): 269.1 (M+H).

**Step E:** Compound **w4-6**

At room temperature, m-CPBA (13.6 g, 67.1 mmol, 85%) was added to a mixed solution of compound **w4-5 (9.00** g, 33.5 mmol) and DCM (800 mL), and the mixture was stirred at room temperature for 16 h. The reaction completion was monitored by LCMS. The crude product obtained by concentrating the reaction solution was purified by FCC ($SiO_2$, MeOH/DCM=0-5%) to yield a white solid product **w4-6** (8.1 g, 85% yield). LCMS (m/z): 285.0 (M+H).

**Step F:** Compound **w4-7**

Under ice-water bath conditions, $POCl_3$ (21.8 g, 142 mmol) was added dropwise to a mixed solution of compound **w4-6** (8.10 g, 28.4 mmol) and DMF (250 mL), and stirred for 1.0 h after raising to room temperature. The reaction completion was monitored by LCMS. The reaction solution was then added dropwise to water (800 mL) and stirred for 0.5 h at room temperature. The resulting filter cake was then was purified by FCC ($SiO_2$, THF/PE=0-50%) to yield a yellow solid product **w4-7** (4.2 g, yield 49%). LCMS (m/z): 303.0 (M+H).

**Step G:** Intermediate **w4**

Under ice-water bath conditions, CSI (5.42 g, 38.3 mmol) was added to a mixed solution of compound **w4-7** (2.90 g, 9.58 mmol) and ACN (300 mL), and the mixture was stirred for 1.5 h after being brought to room temperature. Under ice-water bath conditions, DMF (6.0 mL) was added dropwise to the above reaction solution, and the mixture was stirred for 1.5 h after being brought to room temperature. The reaction completion was monitored by LCMS. The reaction solution was then poured into water (300 mL) and stirred for 0.5 h at room temperature. The filter cake obtained after filtration was dissolved in EA (200 mL), dried over anhydrous $Na_2SO_4$, filtered, and the organic phase was concentrated to obtain a white solid product **w4** (2.0 g, yield 64%). LCMS (m/z): 328.0 (M+H).

## Intermediate w5

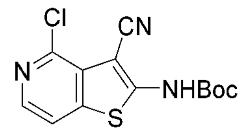

**w5**

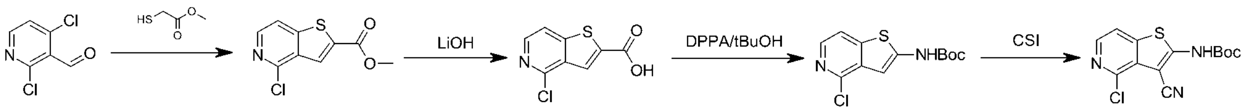


w5-1 w5-2 w5-3 w5-4 w5

**Step A:** Compound **w5-2**

Compound **w5-1** (10 g, 56.8 mmol) and methyl mercaptoacetate (6 g, 56.8 mmol) were dissolved in DMF (100 mL), and potassium carbonate (11.8 g, 85.2 mmol) was added. The mixture was stirred at 40°C for 2 h and then warmed to 60°C, and stirred for 16 h. The reaction completion was monitored by LCMS and TLC. The reaction solution was brought to room temperature and poured into water (500 mL), leading to solid precipitation. The collected solid was filtered, washed with water (200 mL), and dried under reduced pressure to give compound **w5-2** (10.5 g, yield 81%). LCMS (m/z): 227.9 (M+H).

**Step B:** Compound **w5-3**

Compound **w5-2** (10.5 g, 46.1 mmol) was dissolved in THF (100 mL), followed by the addition of methanol (50 mL) and water (50 mL). LiOH (5.8 g, 138.3 mmol) was then added with stirring, and the mixture was stirred at room temperature for 1 h. The reaction completion was monitored by LCMS and TLC. The organic solvent was removed by vacuum distillation. The remaining aqueous phase was diluted with 100 mL of water, and the pH was adjusted to ~4 with 1 N HCl to precipitate a solid. The collected solid was filtered, washed with 200 mL of water, and dried under reduced pressure to obtain compound **w5-3** (8 g, 81% yield). LCMS (m/z): 213.9 (M+H).

**Step C:** Compound **w5-4**

Compound **w5-3** (8 g, 37.4 mmol) and DPPA (13.4 g, 48.7 mmol) were dissolved in toluene (66 mL) and tert-butanol (34 mL), and triethylamine (5.68 g, 56.2 mol) was added. The resulting mixture was stirred at 90°C for 16 h. The reaction completion was monitored by LCMS and TLC. After cooling to room temperature, saturated sodium bicarbonate aqueous solution (100 mL) was added, and the mixture was extracted with EA (50 mL×3). The collected organic phase was washed with saturated brine, dried over anhydrous $Na_2SO_4$, filtered, and the crude product obtained by concentrating the organic phase was purified by FCC ($SiO_2$, EA/PE=0-100%) to give a white solid compound **w5-4** (4 g, yield 38 %). LCMS (m/z): 285.0 (M+H).

**Step D: Intermediate w5**

The synthesis of step D is performed according to the method described in step G of intermediate w4. LCMS (m/z): 310.0 (M+H).

## Intermediate w6

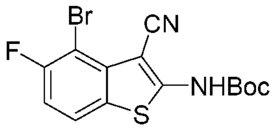

**w6**

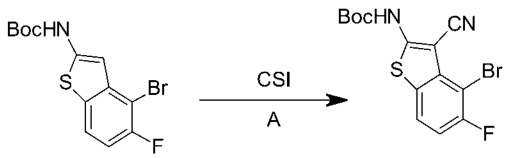


**w3-4** **w6**

**Step A: Intermediate w6**

At -10°C, CSI (3.42 g, 24.3 mmol) was added to compound **w3-4** (7.00 g, 20.2 mmol) in MeCN (175 mL) solution. The reaction was stirred for 1.0 h while maintaining the temperature, then DMF (7.28 g, 101.2 mmol) was added to the reaction solution. The resulting reaction solution was then allowed to react for another 1.0 h at -10°C. The reaction completion was monitored by LCMS. The reaction solution was poured into a water solution (300 mL), stirred at room temperature for 0.5 h, filtered, and the filter cake was dried in a vacuum drying oven to obtain a white solid **intermediate w6** (6.6 g, yield 88%). LCMS (m/z): 371.1 (M+H).

## Intermediate w7

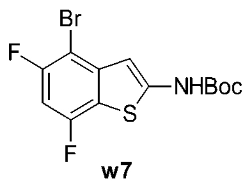

**w7**

Intermediate **w7** was prepared according to the method described in document WO2024067714.

## Intermediate J-W1

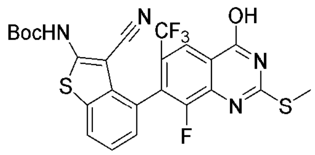

J-W1

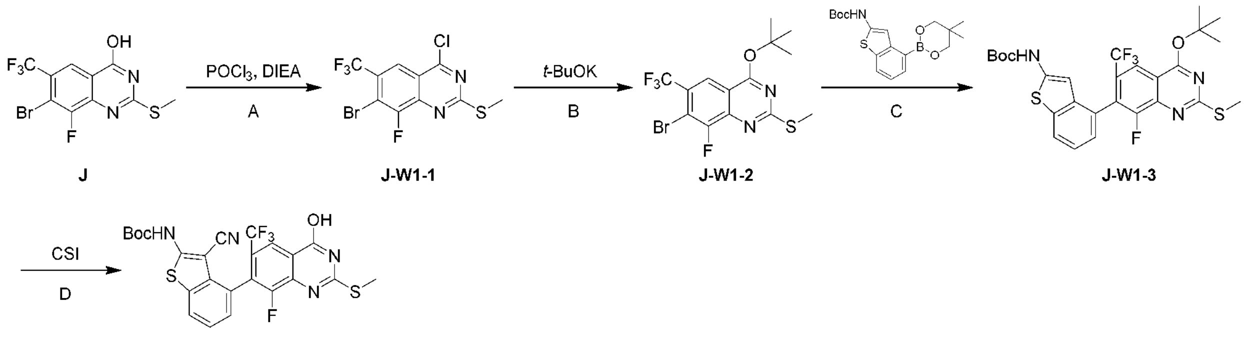


J J-W1-1 J-W1-2 J-W1-3

J-W1

**Step A:** 7-bromo-4-chloro-8-fluoro-2-(methylthio)-6-(trifluoromethyl)quinazoline

At room temperature, $POCl_3$ (20.6 g, 134 mmol) was added to a mixed solution of 7-bromo-8-fluoro-2-methylthio-6-(trifluoromethyl)quinazoline-4-ol (16 g, 44.8 mmol), DIEA (17.37 g, 134 mmol), and THF (200 mL), and the mixture was warmed to 60°C and stirred for 1 h. The reaction completion was monitored by LCMS. The reaction solution was concentrated and purified by FCC to give a yellow oily product 7-bromo-4-chloro-8-fluoro-2-(methylthio)-6-(trifluoromethyl)quinazoline (15 g, yield 89.2%). LCMS (m/z): 374.8 (M+H).

**Step B:** 7-bromo-4-tert-butoxy-8-fluoro-2-(methylthio)-6-(trifluoromethyl)quinazoline

Under dry ice and ethanol bath conditions, *t*BuOK (26.6 mL, 1 M THF solution, 26.6 mmol) was added to a mixture of 7-bromo-4-chloro-8-fluoro-2-(methylthio)-6-(trifluoromethyl)quinazoline (10 g, 26.6 mmol) and THF (200 mL), and was stirred at this temperature for 1 h, then brought to room temperature and stirred for another 1 h. The reaction completion was monitored by LCMS and TLC. The reaction solution was then poured into a semi-saturated $NH_4Cl$ (100 mL) solution and extracted with EA (100 mL×3). After the organic phase was collected, washed with saturated brine (100 mL) and dried with anhydrous $Na_2SO_4$. Filter, the organic phase was concentrated, and purified by FCC ($SiO_2$, EA/PE=0-20%) to obtain a yellow solid product 7-bromo-4-tert-butoxy-8-fluoro-2-(methylthio)-6-(trifluoromethyl)quinazoline (10 g, yield 90.1%). LCMS (m/z): 412.9 (M+H).

**Step C:** tert-Butyl (4-(4-(tert-butoxy)-8-fluoro-2-(methylthio)-6-(trifluoromethyl)quinazoline-7-yl)benzo[*b*]thiophene-2-yl)carbamate

At room temperature, tert-butyl 7-bromo-4-tert-butoxy-8-fluoro-2-(methylthio)-6-(trifluoromethyl)quinazoline (6.00 g, 14.52 mmol), (4-(5,5-dimethyl-1,3,2-dioxaborolan-2-yl)benzo[*b*]thiophene-2-yl)carbamate (7.87 g, 21.78 mmol), $DPEphosPdCl_2$ (2.07 g, 2.9 mmol), and $Cs_2CO_3$ (14.19 g, 43.56 mmol) were added sequentially to a solution of 1,4-dioxane (100 mL) and water (20 mL). After $N_2$ replacement three times, the solution was heated to 80°C and stirred for 5 h. The reaction completion was monitored by LCMS and TLC. The reaction solution was brought to room temperature, and the palladium catalyst was removed by diatomite filtration. The mother liquor was collected, and water (100 mL) was added. Extraction was performed with EA (100 mL×3). The collected organic phase was washed with saturated brine (50 mL). The crude product obtained by concentrating the organic liquid was purified by FCC ($SiO_2$, EA/PE=0-40%) to obtain a yellow solid tert-butyl (4-(4-(tert-butoxy)-8-fluoro-2-(methylthio)-6-(trifluoromethyl)quinazoline-7-yl)benzo[*b*]thiophene-2-yl)carbamate (7.2 g, yield 85.2%). LCMS (m/z): 526.0 (M+H-56).

**Step D:** tert-Butyl (3-cyano-4-(8-fluoro-4-hydroxy-2-(methylthio)-6-(trifluoromethyl)quinazoline-7-yl)benzo[*b*]thiophene-2-yl)carbamate

Under dry ice and acetonitrile bath conditions CSI (2.76 g, 19.26 mmol) was added to a mixed solution of tert-butyl (4-(4-(tert-butoxy)-8-fluoro-2-(methylthio)-6-(trifluoromethyl)quinazoline-7-yl)benzo[*b*]thiophene-2-yl)carbamate (2.8 g, 4.8 mmol) and acetonitrile (300 mL) and stirred for 1 h. The reaction completion was monitored by LCMS, and the reaction solution was poured into DMF (100 mL) in an ice-water bath and stirred for another 1 h. The reaction solution was then poured into a saturated $NaHCO_3$ (200 mL) solution and extracted with EA (100 mL×3). After the organic phase was collected, it was washed with saturated brine (100 mL) and dried over anhydrous $Na_2SO_4$. The organic phase was filtered and concentrated, then purified by FCC ($SiO_2$, EA/PE=0-50%) to obtain a yellow solid product tert-butyl (3-cyano-4-(8-fluoro-4-hydroxy-2-(methylthio)-6-(trifluoromethyl)quinazoline-7-yl)benzo[*b*]thiophene-2-yl)carbamate (2 g, yield 75.4%). LCMS (m/z): 551.0 (M+H).

## Intermediate J-W3

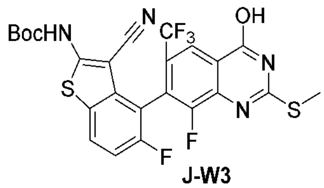

J-W3

tert-butyl (3-cyano-5-fluoro-4-(8-fluoro-4-hydroxy-2-(methylthio)-6-(trifluoromethyl)quinazoline-7-yl)benzo[*b*]thiophene-2-yl) carbamate

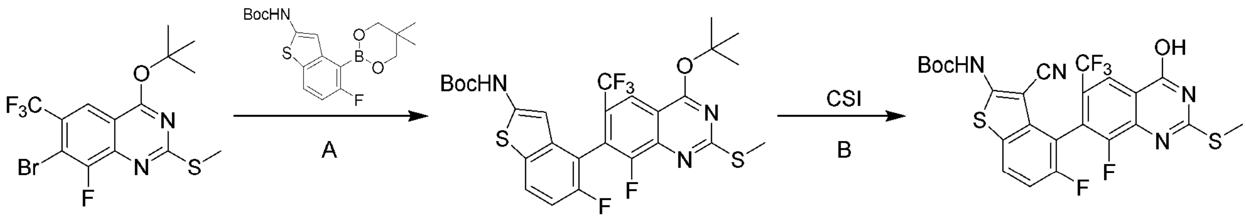


J-W1-2 J-W3-1 J-W3

**Step A:** tert-Butyl (4-(4-(tert-butoxy)-8-fluoro-2-(methylthio)-6-(trifluoromethyl)quinazoline-7-yl)-5-fluorobenzo[*b*]thiophene-2-yl)carbamate

At room temperature, 7-bromo-4-tert-butoxy-8-fluoro-2-(methylthio)-6-(trifluoromethyl)quinazoline (4.00 g, 9.68 mmol), tert-butyl (4-(5,5-dimethyl-1,3,2-dioxaborolan-2-yl)-5-fluorobenzo[*b*]thiophene-2-yl)carbamate (5.51 g, 14.52 mmol), RuphosPdG4 (1.64 g, 0.19 mmol), and DIEA (3.75 g, 29.04 mmol) were added sequentially to a mixed solution of 1,4- dioxane (80 mL) and water (16 mL). After $N_2$ replacement three times, the solution was heated to 80°C and stirred for 4 h. The reaction completion was monitored by LCMS and TLC. The reaction solution was cooled to room temperature, and the palladium catalyst was removed by diatomite filtration. The mother liquor was collected, and water (100 mL) was added. Extraction was performed with EA (100 mL×3). The collected organic phase was washed with saturated brine (50 mL). The crude product obtained by concentrating the organic liquid was purified by FCC ($SiO_2$, EA/PE=0-40%) to obtain a yellow solid tert-butyl (4-(4-(tert-butoxy)-8-fluoro-2-(methylthio)-6-(trifluoromethyl)quinazoline-7-yl)-5-fluorobenzo[*b*] thiophene-2-yl)carbamate (3 g, yield 51.6%). LCMS (m/z): 544.0 (M+H-56).

**Step B:** tert-Butyl (3-cyano-5-fluoro-4-(8-fluoro-4-hydroxy-2-(methylthio)-6-(trifluoromethyl)quinazoline-7-yl)benzo[*b*] thiophene-2-yl)carbamate

Under dry ice and acetonitrile bath conditions, CSI (2.83 g, 20 mmol) was added to a mixed solution of tert-butyl (4-(4-(tert-butoxy)-8-fluoro-2-(methylthio)-6-(trifluoromethyl)quinazoline-7-yl)-5-fluorobenzo[*b*]thiophene-2-yl)carbamate (3 g, 5 mmol) and acetonitrile (500 mL) and stirred for 1 h. After the reaction completion was monitored by LCMS, the reaction solution was poured into DMF (200 mL), cooled in an ice bath, and stirred for another 1 h. After the reaction completion was monitored by LCMS, the reaction solution was poured into a saturated $NaHCO_3$ (200 mL) solution and extracted with EA (100 mL×3). The organic phases were combined, washed with saturated brine (100 mL), and dried over anhydrous $Na_2SO_4$. The sample was filtered, concentrated, and purified by FCC ($SiO_2$, EA/PE=0-40%) to obtain a yellow

solid product tert-butyl (3-cyano-5-fluoro-4-(8-fluoro-4-hydroxy-2-(methylthio)-6-(trifluoromethyl)quinazoline-7-yl)benzo [*b*]thiophene-2-yl)carbamate (1.3 g, yield 45.7%). LCMS (m/z): 569.0 (M+H).

## Intermediate J-W3A and intermediate J-W3B

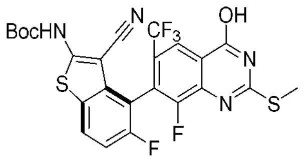

**J-W3A**

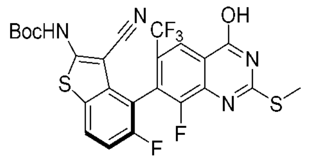

**J-W3B**

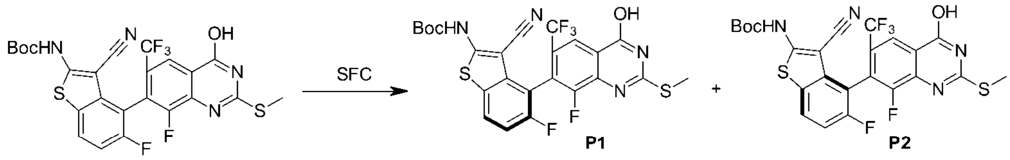


**P1** **P2**

**J-W3** **J-W3B** **J-W3A**

Intermediate **J-W3** (5.0 g) was resolved by SFC (Waters SFC 150, separation column: DAICELCHIRALCEL®IC250*25 mm 10 μm; mobile phase $CO_2$/MeOH (+0.1% 7.0 mol /1 $NH_3$-MeOH)=90/10; flow rate: 140 mL/min) to yield the first eluted isomer 1, intermediate **J-W3B** (2.1 g, with a relatively short retention time). Chiral analysis was performed using SFC-J-W3, Rt=2.692 min. LCMS (m/z): 569.0 (M+H). The subsequent eluted isomer 2 was intermediate **J-W3A** (2.0 g, relatively long retention time). Chiral analysis method SFC-J-W3, Rt=3.424 min. LCMS (m/z): 569.0 (M+H).

Chiral analysis method SFC-J-W3: Waters UPCC, analytical column: DAICELCHIRALPAK®IC, 100*3mm 3μm; mobile phase A: $CO_2$, mobile phase B: MeOH (+0.1% DEA); flow rate: 1.5 mL/min; Column temperature: 35°C; back pressure: 1800 psi; gradient: 0-8.0 min; A/B=85/15.

## Intermediate J-W3A-Cl

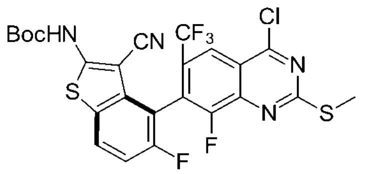

**J-W3A-Cl**

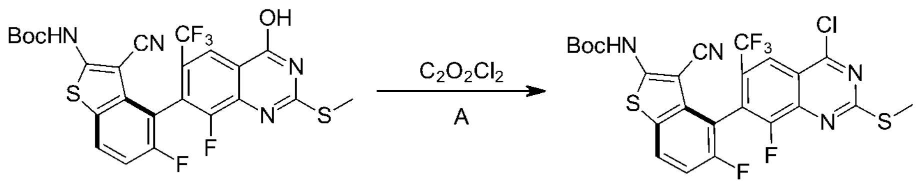


**J-W3A-OH** **J-W3A-Cl**

**Step A: Intermediate J-W3A-Cl**

Under ice-water bath conditions, DMF (25.7 mg, 0.35 mmol) was added to oxalyl chloride (178.6 mg, 1.41 mmol) in a solution of DCM (5 mL), and the mixture was stirred at room temperature for 30 min. Then, the mixture was cooled to an ice-water bath, and intermediate **J-W3A-Cl** (200 mg, 0.35 mmol) was added to the reaction solution. The mixture was stirred at room temperature for 1 h. The reaction completion was monitored by LCMS and TLC. The reaction solution was poured into saturated $NaHCO_3$ (50 mL), extracted with DCM (10 mL×3), and the organic phase was collected. The solution was washed with saturated brine (10 mL), dried over anhydrous $Na_2SO_4$, and the organic phase was concentrated to obtain a yellow solid intermediate **J-W3A-Cl** (200 mg, 97% yield). LCMS (m/z): 587.0 (M+H).

**Intermediate J-W3B-Cl** was prepared according to the above method. LCMS (m/z): 587.0 (M+H).

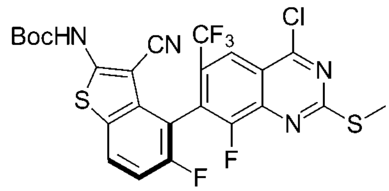

**J-W3B-Cl**

## Intermediate N1

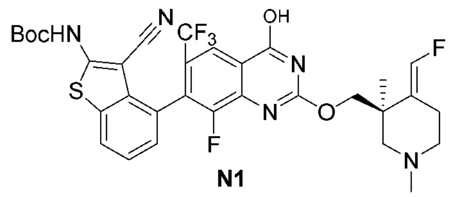

**N1**

tert-Butyl (3-cyano-4-(8-fluoro-2-(((*S,E*)-4-(fluoromethylene)-1,3-dimethylpiperidine-3-yl)methoxy)-4-hydroxy-6-(trifluoromethyl)quinazoline-7-yl)benzo[*b*]thiophene-2-yl)carbamate

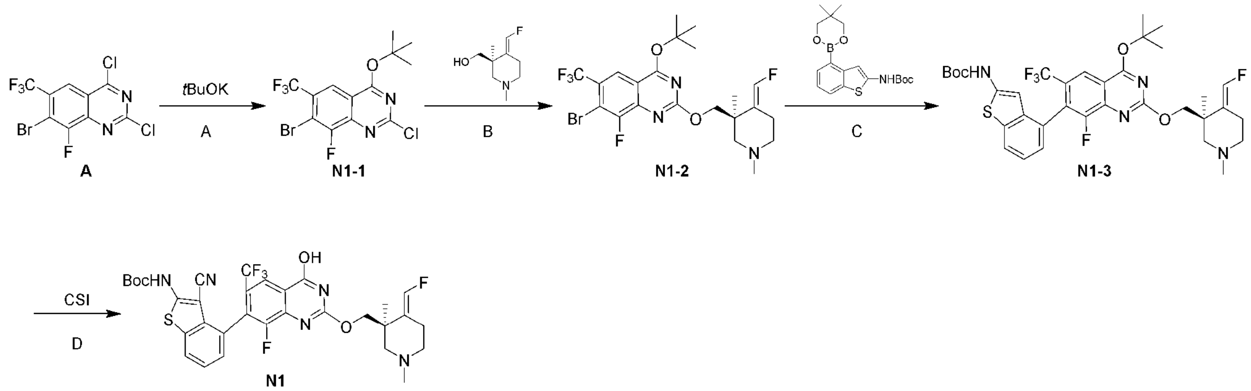


**Step A:** 7-bromo-4-tert-butoxy-2-chloro-8-fluoro-6-trifluoromethylquinazoline

Under dry ice-ethanol bath conditions, *t*-BuOK (30.2 mL, 30.2 mmol, 1 MTF solution) was added dropwise to a mixed solution of 7-bromo-2,4-dichloro-8-fluoro-6-(trifluoromethyl)quinazoline (10.0 g, 27.5 mmol) and THF (100 mL). After the addition was complete, the system was allowed to naturally recover from low temperature to room temperature and stirred for 3 h. The reaction completion was monitored by LCMS. The reaction solution was quenched in a semi-saturated solution of $NH_4Cl$ (100 mL), extracted with EA (150 mL×3), and the organic phase was washed with saturated brine (50 mL). The crude product obtained after the organic phase was collected and concentrated was purified by FCC ($SiO_2$, EA/PE=0-10%) to obtain a yellow solid 7-bromo-4-tert-butoxy-2-chloro-8-fluoro-6-trifluoromethylquinazoline (9.49 g, yield 85%). LCMS (m/z): 346.9 (M+H-56).

**Step B:** (*S,E*)-7-bromo-4-(tert-butoxy)-8-fluoro-2-((4-(fluoromethylene)-1,3-dimethylpiperidine-3-yl)methoxy)-6-(trifluoromethyl)quinazoline

At room temperature, DABCO (152 mg, 1.35 mmol) was added to a mixed solution of 7-bromo-4-tert-butoxy-2-chloro-8-fluoro-6-trifluoromethylquinazoline (2.17 g, 5.40 mmol), (*S,E*)-(4-(fluoromethylene)-1,3-dimethylpiperidine-3-yl) methanol (1.22 g, 7.02 mmol), $Cs_2CO_3$ (4.93 g, 15.1 mmol), and ACN (30 mL), and stirred overnight at 25°C. After the reaction completion was monitored by LCMS, the reaction solution was poured into $H_2O$ (100 mL) solution and extracted with EA (60 mL×3). The organic phase was washed with saturated brine (30 mL), and the crude product obtained after

collection and concentration was purified by FCC ($SiO_2$, EA/PE=0-60%) to obtain a yellow solid (*S,E*)-7-bromo-4-(tert-butoxy)-8-fluoro-2-((4-(fluoromethylene)-1,3-dimethylpiperidine-3-yl)methoxy)-6-(trifluoromethyl)quinazoline (1.03 g, yield 35%). LCMS (m/z): 540.1 (M+H).

**Step C:** tert-Butyl (4-(4-(tert-butoxy)-8-fluoro-2-(((*S,E*)-4-(fluoromethylene)-1,3-dimethylpiperidine-3-yl)methoxy)-6-(trifluoromethyl)quinazoline-7-yl)benzo[*b*]thiophene-2-yl)carbamate

At room temperature, (*S,E*)-7-bromo-4-(tert-butoxy)-8-fluoro-2-((4-(fluoromethylene)-1,3-dimethylpiperidine-3-yl)methoxy)-6-(trifluoromethyl)quinazoline (4.1 g, 7.62 mmol), tert-butyl (4-(5,5-dimethyl-1,3,2-dioxaborolan-2-yl)benzo[*b*]thiophene-2-yl)carbamate (4.13 g, 11.4 mmol), and DPEphosPdCl$_2$ (**1.09** mg, 1.52 mmol) and $Cs_2CO_3$ (7.44 g, 22.8 mmol) were added sequentially to a mixed solution of 1,4-dioxane (125 mL) and $H_2O$ (25 mL). After $N_2$ replacement three times, the mixture was heated to 80°C and stirred for 3 h. The reaction completion was monitored by LCMS until it ended, and the reaction solution was then allowed to return to room temperature. The palladium catalyst was removed by diatomite filtration. The mother liquor was collected, washed with saturated brine (20 mL), and the crude product obtained after organic phase collection and concentration was purified by FCC ($SiO_2$, EA/PE=0-60%) to obtain a yellow solid tert-butyl (4-(4-(tert-butoxy)-8-fluoro-2-(((*S,E*)-4-(fluoromethylene)-1,3-dimethylpiperidine-3-yl)methoxy)-6-(trifluoromethyl)quinazoline-7-yl)benzo[b]thiophene-2-yl)carbamate (3.8 g, yield 70%). LCMS (m/z): 707.1 (M+H).

**Step D:** tert-Butyl (3-cyano-4-(8-fluoro-2-(((*S,E*)-4-(fluoromethylene)-1,3-dimethylpiperidine-3-yl)methoxy)-4-hydroxy-6-(trifluoromethyl)quinazoline-7-yl)benzo[*b*]thiophene-2-yl)carbamate

At -15°C, a diluted solution of CSI (3.04 g, 21.5 mmol) and ACN (10 mL) was added dropwise to a mixed solution of tert-butyl (4-(4-(tert-butoxy)-8-fluoro-2-(((*S,E*)-4-(fluoromethylene)-1,3-dimethylpiperidine-3-yl)methoxy)-6-(trifluoromethyl)quinazoline-7-yl)benzo[*b*]thiophene-2-yl)carbamate (3.80 g, 5.38 mmol) and ACN (700 mL). After the addition was complete, the system was kept at low temperature and stirred for 1 h. Then, a diluted solution of DMF (8 mL) and ACN (10 mL) was added dropwise to the reaction system at -15°C and stirred for 1 h. After the reaction completion was monitored by LCMS, the reaction solution was poured into a mixture of 50 mL of semi-saturated $NaHCO_3$ solution and 50 mL of saturated saline solution, and extracted with EA (100 mL×2). The organic phase was collected, concentrated, and purified by FCC ($SiO_2$, MeOH/DCM=0-10%) to obtain a yellow oily product, which was lyophilized to obtain tert-butyl (3-cyano-4-(8-fluoro-2-(((*S,E*)-4-(fluoromethylene)-1,3-dimethylpiperidine-3-yl)methoxy)-4-hydroxy-6-(trifluoromethyl)quinazoline-7-yl)benzo[b]thiophene-2-yl)carbamate (2.8 g, 77% yield). LCMS (m/z): 676.0 (M+H).

## Intermediate N1A and Intermediate N1B

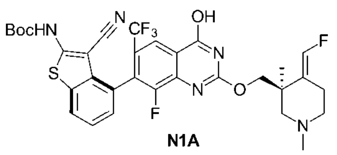
N1A

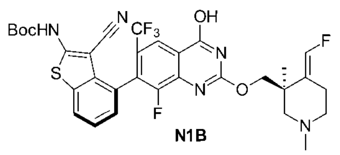
N1B

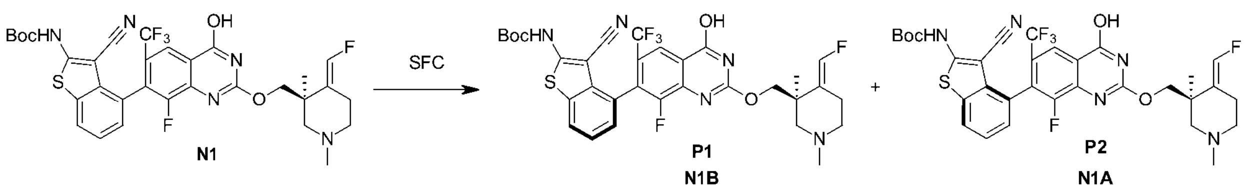

N1 P1 N1B + P2 N1A

Intermediate **N1** (4.0 g) was resolved by SFC (Waters SFC 150, separation column: REGIS(S,S)WHELK-O1 250*40 mm 10 μm; mobile phase $CO_2$/MeOH (+0.1% 7.0 mol/1 NH3-MeOH)=70/30; flow rate: 140 mL/min) to yield the first eluted isomer 1, intermediate **N1B** (1.6 g, relatively short retention time). Chiral analysis method SFC-N1, Rt=1.463 min. LCMS (m/z): 676.0 (M+H). The isomer 2 was subsequently eluted, intermediate **N1A** (1.9 g, relatively long retention time). Chiral analysis method SFC-N1, Rt=1.989 min. LCMS (m/z): 676.0 (M+H).

Chiral analysis method N1: Waters UPCC, analytical column: REGIS(S,S)WHELK-O1, 100*3mm3μm; mobile phase A: $CO_2$, mobile phase B: MeOH(+0.1% DEA); flow rate: 1.5 mL/min; column temperature: 35°C; back pressure: 1800 psi; gradient: 0-8.0 min A/B=65/35.

## Intermediate N2

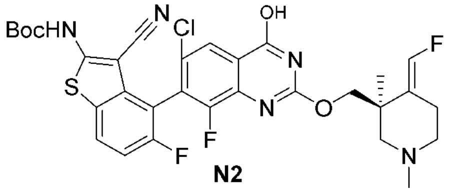

N2

tert-Butyl (4-(6-chloro-8-fluoro-2-(((*S,E*)-4-(fluoromethylene)-1,3-dimethylpiperidine-3-yl)methoxy)-4-hydroxyquinazoline-7-yl)-3-cyano-5-fluorobenzo[*b*]thiophene-2-yl)carbamate

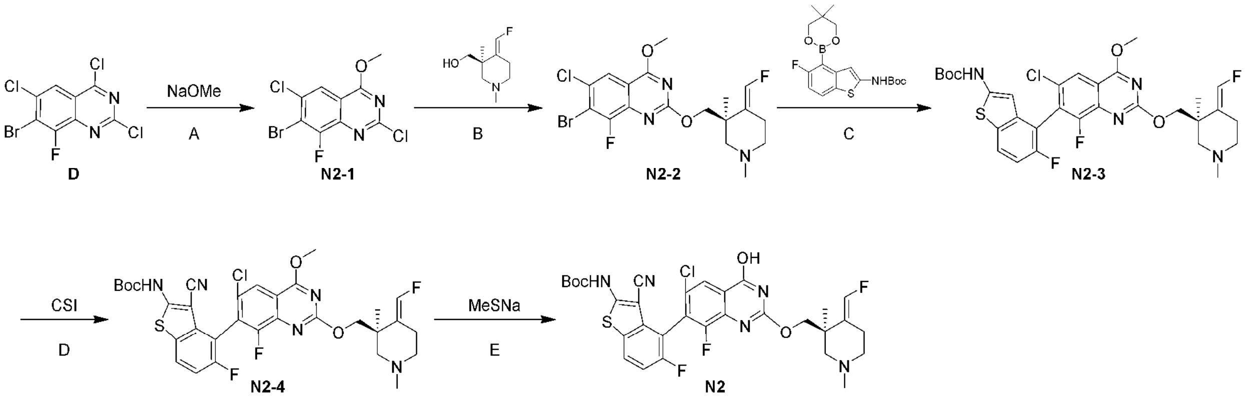


**Step A:** 7-bromo-2,6-dichloro-8-fluoro-4-methoxyquinazoline

Under dry ice and acetonitrile bath conditions, diluted solutions of NaOMe (5.45 g, 30.3 mmol, 30% MeOH solution) and MeOH (10 mL) were added dropwise to a system of 7-bromo-2,4,6-trichloro-8-fluoroquinazoline (10.0 g, 30.3 mmol) and MeOH (100 mL). After the addition was complete, the system was allowed to naturally return to room temperature and stirred for 5 h. The reaction completion was monitored by LCMS. The reaction solution was then quenched in a semi-saturated $NH_4Cl$ (100 mL) solution. The mixture was then concentrated to remove MeOH, a white solid precipitation was formed. This was thermal-filtered, the filter cake was collected, and dried to obtain a white solid, 7-bromo-2,6-dichloro-8-fluoro-4-methoxyquinazoline (9.6 g, 97% yield). LCMS (m/z): 326.8 (M+H).

**Step B:** (*S,E*)-7-bromo-6-chloro-8-fluoro-2-((4-(fluoromethylene)-1,3-dimethylpiperidine-3-yl)methoxy)-4-methoxyquinazoline

At room temperature, DABCO (172 mg, 1.53 mmol) was added to a mixed solution of 7-bromo-2,6-dichloro-8-fluoro-4-methoxyquinazoline (1.00 g, 3.07 mmol), (*S,E*)-(4-(fluoromethylene)-1,3-dimethylpiperidine-3-yl)methanol (797 mg, 4.60 mmol), $Cs_2CO_3$ (3.00 g, 9.20 mmol), and ACN (10 mL), and stirred at room temperature for 1.5 h. The reaction completion was monitored by LCMS. The reaction solution was poured into $H_2O$ (100 mL) solution, and a white solid precipitated. The mixture was filtered, and the filter cake was collected. TLC showed that the purity of the precipitated product was poor, so DCM (100 mL) was used to dissolve the filter cake, and an appropriate amount of anhydrous sodium sulfate was added for drying. The crude product obtained by concentration was purified by FCC ($SiO_2$, EA/PE=0-30%) to obtain a white solid (*S,E*)-7-bromo-6-chloro-8-fluoro-2-((4-(fluoromethylene)-1,3-dimethylpiperidine-3-yl)methoxy)-4-methoxyquinazoline (670 mg, yield 47%). LCMS (m/z): 462.0 (M+H).

**Step C:** tert-Butyl (4-(6-chloro-8-fluoro-2-(((*S,E*)-4-(fluoromethylene)-1,3-dimethylpiperidin-3-yl)methoxy)-4-methoxyquinazoline-7-yl)-5-fluorobenzo[b]thiophene-2-yl)carbamate

At room temperature, tert-butyl (*S,E*)-7-bromo-6-chloro-8-fluoro-2-((4-(fluoromethylene)-1,3-dimethylpiperidine-3-yl)methoxy)-4-methoxyquinazoline (600 mg, 1.30 mmol), (4-(5,5-dimethyl-1,3,2-dioxaborolan-2-yl)-5-fluorobenzo[*b*]thiophene-2-yl)carbamate (738 mg, 1.95 mmol), and RuPhos Pd G4 were sequentially added. Palladium catalyst (110 mg, 0.13 mmol) and DIPEA (670 mg, 5.19 mmol) were added to a mixed solution of Me-THF (16 mL) and $H_2O$ (4 mL). After $N_2$ replacement three times, the mixture was heated to 70°C and stirred for 3 h. The reaction completion was monitored by LCMS, and the reaction solution was allowed to return to room temperature. The palladium catalyst was

removed by diatomite filtration, the mother liquor was collected, and water (50 mL) was added. The mixture was then extracted with EA (50 mL×3). The combined organic phases were washed with saturated brine (20 mL), and the crude product obtained after concentration was purified by FCC ($SiO_2$, EA/PE=0-60%) to give a yellow solid (650 mg, yield 77%) of tert-butyl 4-(6-chloro-8-fluoro-2-(((*S,E*)-4-(fluoromethylene)-1,3-dimethylpiperidine-3-yl)methoxy)-4-methoxyquinazoline-7-yl)-5-fluorobenzo[*b*]thiophene-2-yl)carbamate. LCMS (m/z): 649.2 (M+H).

**Step D:** tert-Butyl (4-(6-chloro-8-fluoro-2-(((*S,E*)-4-(fluoromethylene)-1,3-dimethylpiperidine-3-yl)methoxy)-4-methoxyquinazoline-7-yl)-3-cyano-5-fluorobenzo[*b*]thiophene-2-yl)carbamate

Under -15°C and dry ice acetonitrile bath conditions, a diluted solution of CSI (394 mg, 2.77 mmol) and ACN (5 mL) was added dropwise to a system of tert-butyl (4-(6-chloro-8-fluoro-2-(((*S,E*)-4-(fluoromethylene)-1,3-dimethylpiperidine-3-yl)methoxy)-4-methoxyquinazoline-7-yl)-5-fluorobenzo[*b*]thiophene-2-yl)carbamate (600 mg, 0.924 mmol) and ACN (50 mL). After the addition was complete, the system was kept at low temperature and stirred for 1.5 h. Then, a diluted solution of DMF (5 mL) and ACN (5 mL) were added dropwise to the reaction system at -15°C and stirred for 1 h. The reaction completion was monitored by LCMS. The reaction solution was poured into a semi-saturated $NaHCO_3$ (50 mL) solution to quench the reaction, and extracted with EA (50 mL×3). The organic phase was collected, washed with saturated brine (20 mL), and concentrated to obtain a crude product. The crude product was purified by FCC ($SiO_2$, EA/PE=0-60%) to obtain a yellow oily product tert-butyl 4-(6-chloro-8-fluoro-2-(((*S,E*)-4-(fluoromethylene)-1,3-dimethylpiperidine-3-yl)methoxy)-4-methoxyquinazoline-7-yl)-3-cyano-5-fluorobenzo[*b*]thiophene-2-yl)carbamate(600 mg, yield 96%). LCMS (m/z): 674.1 (M+H).

**Step E:** tert-Butyl (4-(6-chloro-8-fluoro-2-(((*S,E*)-4-(fluoromethylene)-1,3-dimethylpiperidine-3-yl)methoxy)-4-hydroxyquinazoline-7-yl)-3-cyano-5-fluorobenzo[*b*]thiophene-2-yl)carbamate

At room temperature, MeSNa (1.2 g, 17.8 mmol) was added to a mixed solution of tert-butyl (4-(6-chloro-8-fluoro-2-(((*S,E*)-4-(fluoromethylene)-1,3-dimethylpiperidine-3-yl)methoxy)-4-methoxyquinazoline-7-yl)-3-cyano-5-fluorobenzo[*b*]thiophene-2-yl)carbamate (600 mg, 0.890 mmol) and DMF (20 mL), warmed to 90°C and stirred for 2 hours. The reaction completion was monitored by LCMS. The reaction solution was poured into water (100 mL), the pH was adjusted to 6 with 1 M HCl, and then adjusted to alkalinity with $NaHCO_3$ aqueous solution. Extraction was performed with EA (50 mL×3). The organic phase was collected, washed with saturated brine (20 mL), and the organic phase was concentrated to obtain a crude product. The crude product was purified by FCC ($SiO_2$, EA/EtOH=3:1)/PE=0-50%) to obtain a yellow solid tert-butyl (4-(6-chloro-8-fluoro-2-(((*S,E*)-4-(fluoromethylene)-1,3-dimethylpiperidine-3-yl)methoxy)-4-hydroxyquinazoline-7-yl)-3-cyano-5-fluorobenzo[*b*]thiophene-2-yl)carbamate (490 mg, yield 83%). LCMS (m/z): 660.0 (M+H).

## Intermediate N3

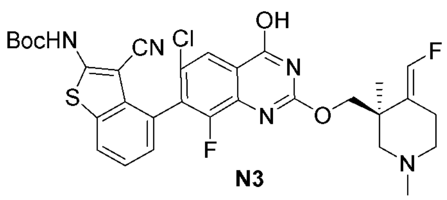

N3

tert-Butyl (4-(6-chloro-8-fluoro-2-(((*S,E*)-4-(fluoromethylene)-1,3-dimethylpiperidine-3-yl)methoxy)-4-hydroxyquinazoline-7-yl)-3-cyanobenzo[*b*]thiophene-2-yl)carbamate

The synthesis of intermediate N3 was carried out according to the scheme described for intermediate N2, in which step C was performed using tert-butyl (4-(5,5-dimethyl-1,3,2-dioxaborolan-2-yl)benzo[*b*]thiophene-2-yl)carbamate instead of tert-butyl (4-(5,5-dimethyl-1,3,2-dioxaborolan-2-yl)-5-fluorobenzo[*b*]thiophene-2-yl)carbamate. LCM S (m/z): 642.2 (M+H).

# Intermediate N4 and N4-OH

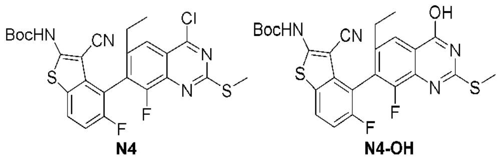

N4 N4-OH

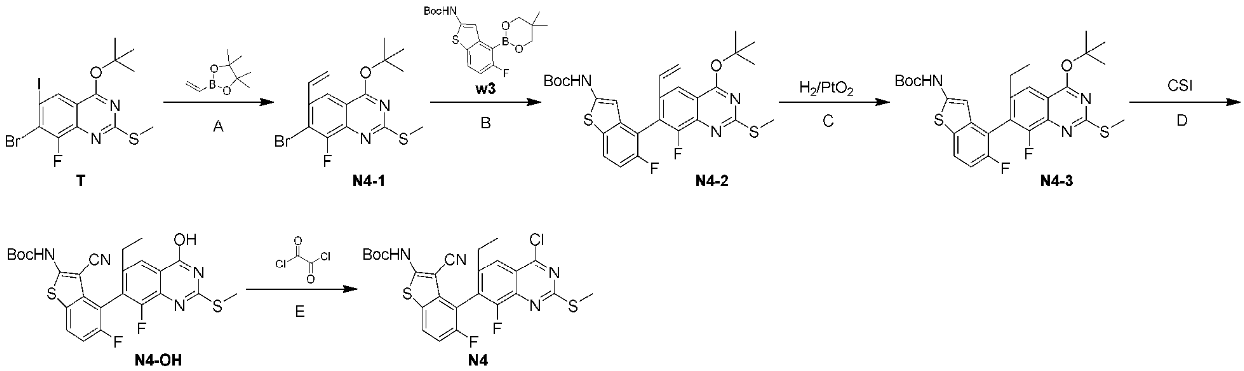


T N4-1 N4-2 N4-3

N4-OH N4

**Step A:** Compound **N4-1**

At room temperature, intermediate **T** (4.0 g, 8.5 mmol), vinylboronic acid pinacol ester (2.62 g, 17.02 mmol), Pd(dppf)$Cl_2$ (62.2 mg, 0.85 mol), and $K_2CO_3$ (3.52 g, 25.53 mmol) were added sequentially to a DMF (60 mL) solution. After $N_2$ replacement three times, the solution was warmed to 80°C and stirred for 1.5 h. The reaction completion was monitored by LCMS and TLC. The reaction solution was brought to room temperature, and the palladium catalyst was removed by diatomite filtration. The mother liquor was collected, and water (50 mL) was added. Extraction was performed using EA (50 mL×3). The collected organic phase was washed with saturated brine, dried over anhydrous $Na_2SO_4$, filtered, and the crude product obtained by concentrating the organic liquid was purified by FCC ($SiO_2$, THF/PE=0-3%) to give a pale yellow solid compound **N4-1** (1.74 g, yield 55 %). LCMS (m/z): 316.9 (M+H-56).

**Step B:** Compound **N4-2**

At room temperature, compound **N4-1** (1.74 g, 4.70 mmol) and intermediate w3. (2.67 g, 7.05 mmol), RuPhos Pd G4 (400 mg, 0.1mol), DIEA (3.03 mg, 23.50 mmol) were sequentially added to a solution of 1,4-dioxane (25 mL) and water (5 mL). After $N_2$ replacement three times, the mixture was warmed to 70°C and stirred for 2 h. The reaction completion was monitored by LCMS and TLC. The reaction solution was brought to room temperature, and the palladium catalyst was removed by diatomite filtration. The mother liquor was collected, and water (50 mL) was added. Extraction was performed with EA (50 mL×3). The collected organic phase was washed with saturated brine, dried over anhydrous $Na_2SO_4$, filtered, and the crude product obtained by concentrating the organic liquid was purified by FCC ($SiO_2$, EA/PE=0-20%) to obtain a brown solid compound **N4-2** (1.80 g, yield 69%). LCMS (m/z): 558.2 (M+H).

**Step C:** Compound **N4-3**

Compound **N4-2** (1.80 g, 3.12 mmol) and $PtO_2$ (71 mg) were added sequentially to a methanol (30 mL) solution at room temperature. After $H_2$ replacement three times, the mixture was stirred at room temperature for 16 h. The reaction completion was monitored by LCMS and TLC. The reaction solution was filtered through diatomite to remove the palladium catalyst. The mother liquor was collected, and the crude product obtained by concentrating the organic liquid was purified by FCC ($SiO_2$, EA/PE=0-11%) to obtain a white solid compound **N4-3** (1.30 g, yield 72%). LCMS (m/z): 560.2 (M+H).

**Step D:** Intermediate **N4-OH**

Under ice-salt bath conditions (-10°C), CSI (130 mL) was added to compound **N4-3** (1.30 g, 2.33 mmol) in MeCN solution (1.31 mg, 9.30 mmol). The reaction mixture was stirred at the same temperature for 1.0 h, and then DMF (20 mL) was added to the reaction solution. The resulting reaction solution was allowed to continue reacting at room temperature for another 1 hour. The reaction completion was monitored by TLC. The reaction solution was then poured into a semi-saturated solution of $NaHCO_3$ (50 mL), extracted with EA (50 mL×3), the organic phase was collected, washed with

saturated brine (30 mL), dried over anhydrous $Na_2SO_4$, filtered, and the organic phase was concentrated and purified by FCC ($SiO_2$, THF/PE=0-78%) to yield a yellow solid compound **$N_4$-OH**

**Step E:** Intermediate **N4**

Under ice bath conditions, DMF (28 mg, 0.38 mmol) was added to oxalyl chloride (144 mg, 1.13 mmol) in solution of DCM (5 mL), and the reaction was stirred for 0.5 h while maintaining the temperature. Then, compound **N4-4** (200 mg, 0.38 mmol) was added to the reaction solution. The resulting reaction solution was allowed to react for another hour at room temperature. The reaction completion was monitored by LCMS. The reaction solution was then poured into water (20 mL), extracted with DCM (20 mL×3), and the organic phase was collected. The organic phase was washed with saturated saline solution, dried over anhydrous $Na_2SO_4$, and the organic phase was concentrated and purified by FCC ($SiO_2$, THF/PE=0-15%) to yield a white solid intermediate **$N_4$** (150 mg, 73% yield). LCMS (m/z): 547.1 (M+H).

## Intermediate N5 and N5-OH

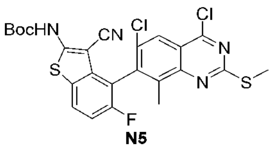
N5

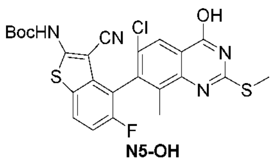
N5-OH

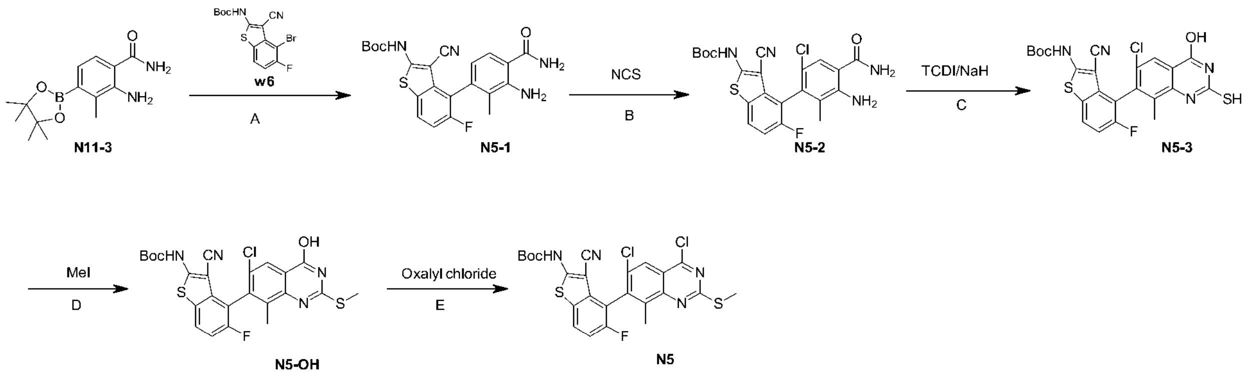


**Step A:** Compound **N5-1**

At room temperature, intermediate **w6** (2.00 g, 5.41 mmol), compound **N11-3** (1.79 g, 6.49 mmol), Pd-118 (353 mg, 0.541 mmol) and $K_3PO_4$ (3.44 g, 16.2 mmol) were added to a solution of 1,4-dioxane (30 mL) and water (6 mL). After $N_2$ replacement three times, the mixture was warmed to 80°C and stirred for 2 h. The reaction completion was monitored by LCMS and TLC. The reaction solution was brought to room temperature, and the palladium catalyst was removed by diatomite filtration. The mother liquor was collected, and water (50 mL) was added. Extraction was performed with EA (50 mL×3). The collected organic phase was washed with saturated brine and dried over anhydrous $Na_2SO_4$. After filtration and concentration, the crude product obtained by concentration was purified by FCC ($SiO_2$, THF/PE=0-57%) to obtain a gray solid compound **N5-1** (1.69 g, yield 71%). LCMS (m/z): 441.1 (M+H).

**Step B:** Compound **N5-2**

At room temperature, TFA (876 mg, 7.68 mmol) and NCS (615 mg, 4.61 mmol) were added sequentially to a solution of compound **N5-1** (1.69 g, 3.84 mmol) in acetonitrile (25 mL). The resulting reaction solution was warmed to 40°C and stirred for 1.5 h. The reaction completion was monitored by LCMS and TLC. The reaction solution was poured into a semi-saturated solution of $NaHCO_3$ (50 mL), extracted with EA (50 mL×3), and the organic phase was collected, washed with saturated saline solution, and dried over anhydrous $Na_2SO_4$. After filtration, the crude product obtained by concentrating the organic liquid was purified by FCC ($SiO_2$, THF/PE=0-58%) to give a yellow solid compound **N5-2** (1.13 g, yield 62%). LCMS (m/z): 475.1 (M+H).

**Step C:** Compound **N5-3**

At room temperature, sodium hydride (60% dispersed in mineral oil, 334 mg, 8.34 mmol) was added to a tetrahydrofuran (25 mL) solution of compound **N5-2** (1.13 g, 2.38 mmol). The resulting reaction solution was stirred at 40°C for 0.5 h, and TCDI (635 mg, 3.57 mmol) was added to the reaction solution. The temperature was warmed to 60°C and stirred for 16 h. The reaction completion was monitored by LCMS. The reaction solution was then returned to room temperature and poured into a semi-saturated $NH_4Cl$ (20 mL) solution. The pH was adjusted to ~5 with hydrochloric acid, and the solution was extracted with EA (50 mL×3). The organic phase was collected, washed with saturated saline solution, and dried over anhydrous $Na_2SO_4$. The solution was filtered and concentrated to obtain a brown solid compound **N5-3** (1.11 g, crude product). LCMS (m/z): 517.1 (M+H).

**Step D: Intermediate N5-OH**

At room temperature, compound **N5-3** (1.11 g), NaOH (344 mg, 8.59 mmol), and methyl iodide (458 mg, 3.23 mmol) were added sequentially to a solution of methanol (10 mL) and water (10 mL), and stirred at room temperature for 2 h. The reaction completion was monitored by LCMS. The reaction solution was adjusted to pH~5 with hydrochloric acid, extracted with EA (50 mL×3), and the organic phase was collected. The mixture was washed with saturated saline solution and dried over anhydrous $Na_2SO_4$. After filtration and concentration, the crude product obtained was purified by FCC ($SiO_2$, THF/PE=0-38%) to give a white solid **intermediate N5-OH** (266 mg). LCMS (m/z): 531.1 (M+H).

**Step E: Intermediate N5**

Under ice bath conditions, DMF (20.0 mg, 0.274 mmol) was added to a solution of oxalyl chloride (139 mg, 1.09 mmol) in DCM (5 mL), and the reaction was stirred for 0.5 h while maintaining the temperature. The **intermediate N5-OH** (145 mg, 0.274 mmol) was then added to the reaction solution. The resulting reaction solution was allowed to react for another 1 h at room temperature. The reaction completion was monitored by LCMS. The reaction solution was then poured into water (30 mL), extracted with DCM (30 mL×3), and the organic phase was collected, washed with saturated saline solution, and dried over anhydrous $Na_2SO_4$. The organic phase was concentrated and purified by FCC ($SiO_2$, THF/PE=0-12%) to yield a white solid **intermediate N5** (126 mg, yield 84%). LCMS (m/z): 548.9 (M+H).

## Intermediates N5-OH-A and N5-OH-B

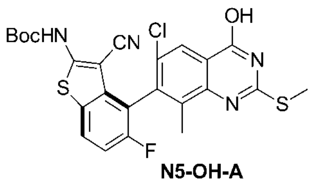

**N5-OH-A**

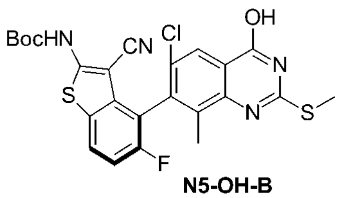

**N5-OH-B**

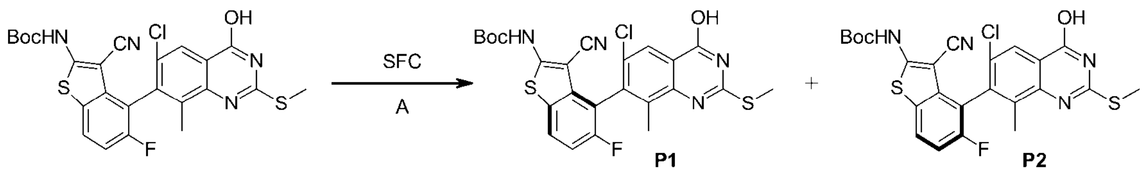


**P1** **P2**

**N5-OH** **N5-OH-A** **N5-OH-B**

Intermediate **N5-OH** (5 g) was resolved by SFC (Waters SFC 150, column: DAICELCHIRALPAK®OJ 250*30 mm 10 μm; mobile phase $CO_2$/MeOH (+0.1% 7.0 mol/1 $NH_3$-MeOH) = 80/20; flow rate: 140 mL/min) to yield the first eluted isomer 1, intermediate **N5-OH-A** (2 g, relatively short retention time). Chiral analysis was performed using SFC-N5-OH, Rt = 2.004 min. LCMS (m/z): 531.1 (M+H). Subsequently eluted isomer 2 was intermediate **N5-OH-B** (2 g, relatively long retention time). Chiral analysis method: SFC-N5-OH, Rt = 3.151 min. LCMS (m/z): 531.1 (M+H).

Chiral analysis method SFC-N5-OH: Waters UPCC, analytical column: DAICELCHIRALPAK® OJ, 100*3mm 3μm; mobile phase A: $CO_2$, mobile phase B: MeOH (+0.1% DEA); Flow rate: 1.5 mL/min; Column temperature: 35°C; Back pressure: 1800 psi; Gradient: 0-7.0 min; A/B=8.5/1.5.

## Intermediates N5A and N5B

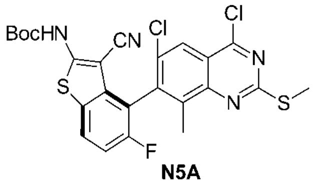

N5A

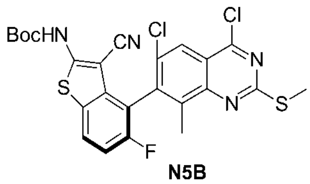

N5B

Intermediates **NSA** and **N5B** were prepared from intermediates **N5-OH-A** and **N5-OH-B** respectively, following the same synthesis method as intermediate **N5.** LCMS (m/z): 548.9 (M+H).

## Intermediate N6 and N6-OH

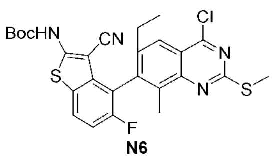

N6

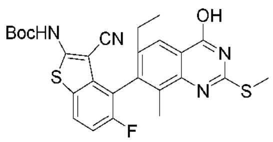

N6-OH

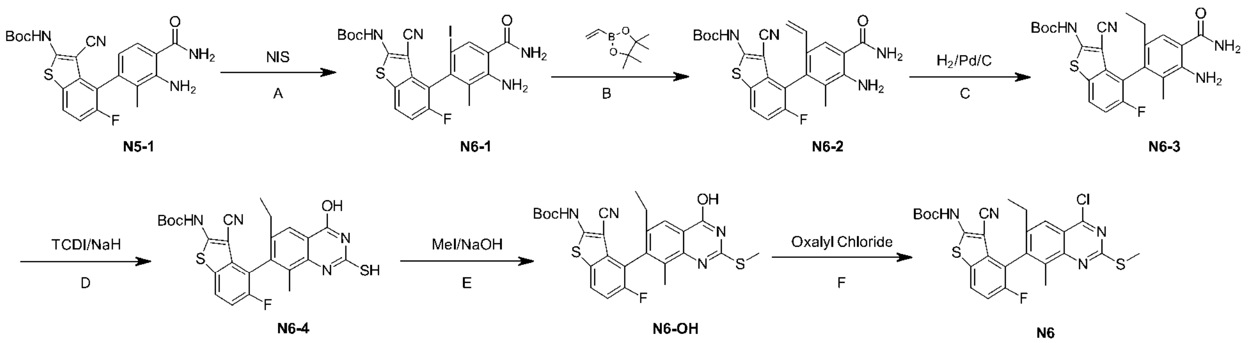


**Step A:** Compound **N6-1**

At room temperature, TFA (389 mg, 3.41 mmol) and NIS (1.53 g, 6.81 mmol) were added sequentially to a soluion of the compound **N5-1** (1.50 g, 3.41 mmol) in acetonitrile (25 mL), the resulting reaction solution was stirred overnight at room temperature. The reaction completion was monitored by LCMS and TLC. The reaction solution was then poured into a semi-saturated $Na_2SO_3$ (50 mL) solution. Extraction was performed using EA (100 mL×3), the organic phase was collected, washed with saturated saline solution and dried over anhydrous $Na_2SO_4$. The crude product obtained by concentrating the organic liquid was purified by FCC ($SiO_2$, EA/PE=0-50%) to give a brownish-gray solid compound **N6-1** (1.00 g, yield 52 %). LCMS (m/z): 566.9 (M+H).

**Step B:** Compound **N6-2**

At room temperature, Compound **N6-1** (1.00 g, 1.77 mmol), vinylboronic acid pinacol ester (816 mg, 5.30 mmol), Pd-118 (228 mg, 0.353 mmol), and $K_3PO_4$ (1.50 g, 7.06 mmol) were sequentially added to a solution of 1,4-dioxane (20 mL) and water (5 mL). After $N_2$ replacement three times, the mixture was heated to 90°C and stirred for 3 h. The reaction completion was monitored by LCMS. The reaction solution was then cooled to room temperature and filtered through diatomite to remove the palladium catalyst. The mother liquor was collected, and water (50 mL) was added. Extraction was performed using EA (50 mL×3). The collected organic phase was washed with saturated brine and dried over anhydrous $Na_2SO_4$. The crude product obtained by filtration and concentration of the organic liquid was successively purified by FCC ($SiO_2$, EA/PE=0-60%) and FCC (C18, ACN/(0.05%TFA/$H_2O$)=5-60%) to obtain a yellow solid compound **N6-2** (350 mg, yield 42%). LCMS (m/z): 467.1 (M+H).

**Step C:** Compound **N6-3**

At room temperature, compound **N6-2** (350 mg, 0.75 mmol) was dissolved in methanol (20 mL), and Pd/C (160 mg, 10 wt%, 0.15 mmol) was added, $H_2$ balloon was used for two replacements and the mixture was stirred at room

temperature for 3 h. The reaction completion was monitored by LCMS. A clear organic phase was obtained by filtering the reaction solution through a filter head. After complete concentration, a yellow solid compound **N6-3** (350 mg, 99 % yield) was obtained. LCMS (m/z): 469.1 (M+H).

**Step D:** Compound **N6-4**

At room temperature, sodium hydride (60% dispersed in mineral oil, 105 mg, 2.61 mmol) was added to a solution of compound **N6-3** (350 mg, 0.747 mmol) in tetrahydrofuran (12 mL) at room temperature. The resulting reaction solution was stirred at 40°C for 0.5 h. Then, N,N'-thiocarbonyldiimidazole (200 mg, 1.12 mmol) was added to the reaction solution, and the temperature was warmed to 60°C and stirred for 3 h. The reaction completion was monitored by LCMS. The reaction solution was then returned to room temperature and poured into a semi-saturated $NH_4Cl$ (20 mL) solution. The pH was adjusted to ~5 with hydrochloric acid, and the solution was extracted with EA (50 mL×3). The organic phase was collected, washed with saturated saline solution, and dried over anhydrous $Na_2SO_4$. The organic liquid was concentrated to give a brown solid compound **N6-4** (360 mg, crude product). LCMS (m/z): 511.0 (M+H).

**Step E: Intermediate N6-OH**

At room temperature, compound **N6-4** (360 mg), NaOH (113 mg, 2.82 mmol), and methyl iodide (150 mg, 1.06 mmol) were added sequentially to a solution of methanol (5 mL) and water (5 mL), and stirred at room temperature for 1 h. The reaction completion was monitored by LCMS. The pH of the reaction solution was adjusted to ~5 with hydrochloric acid. Extraction was performed using (50 mL×3). After collecting the organic phase, it was washed with saturated saline solution and dried over anhydrous $Na_2SO_4$. After filtration, the crude product obtained by concentrating the organic liquid was purified by FCC ($SiO_2$, EA/PE=0-40%) to give a white solid **intermediate N65-OH** (220 mg). LCMS (m/z): 525.2 (M+H).

**Step F: Intermediate N6**

Under ice bath conditions, DMF (15.3 mg, 0.210 mmol) was added to a solution of oxalyl chloride (110 mg, 0.944 mmol) in DCM (3 mL), and the reaction was stirred for 0.5 h while maintaining the temperature. Then, **intermediate N6-OH** (110 mg, 0.210 mmol) was added to the reaction solution. The resulting reaction solution was allowed to react for another 1 h at room temperature. The reaction completion was monitored by LCMS. The reaction solution was then poured into a $NaHCO_3$ aqueous solution (30 mL) and extracted with DCM (30 mL×3). The organic phase was collected, washed with saturated saline solution, and dried over anhydrous $Na_2SO_4$. The organic phase was concentrated and purified by FCC ($SiO_2$, EA/PE=0-12%) to obtain a white solid **intermediate N6** (110 mg, yield 96 %). LCMS (m/z): 543.0 (M+H).

## Intermediate N7 and N7-OH

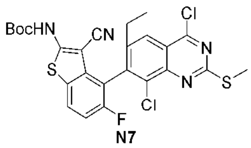

N7

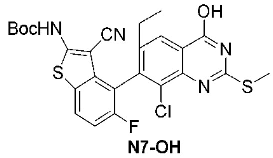

N7-OH

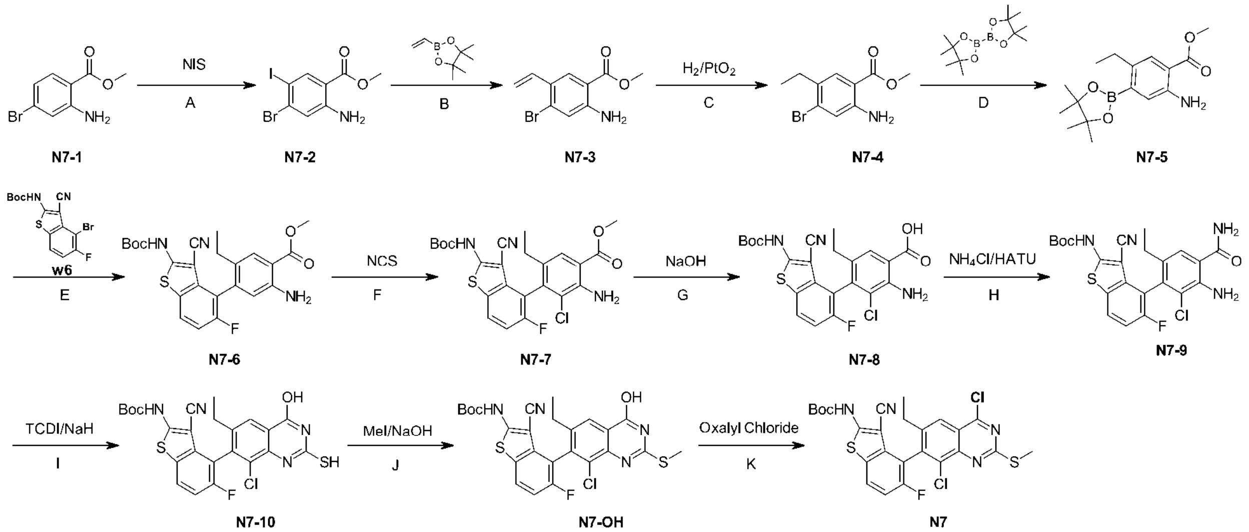


**Step A:** Compound **N7-2**

At room temperature, NIS (73.4 g, 326 mmol) was added to a mixed solution of compound **N7-1** (50.0 g, 217 mmol) and ACN (500 mL), and the mixture was stirred at room temperature for 72 h. The reaction completion was monitored by LCMS, and the reaction solution was concentrated and then dissolved using EA (2 L), then washed successively with water (500 mL×3) and saturated $Na_2S_2O_3$ (500 mL). The organic phase was collected, dried with anhydrous $Na_2SO_4$, filtered, and the organic phase was concentrated to obtain a brown solid product **N7-2** (70 g, yield 90 %). LCMS (m/z): 355.9 (M+ H) and 357.9 (M+H).

**Step B:** Compound **N7-3**

At room temperature, compound **N7-2** (10.0 g, 28.1 mmol), vinylboronic acid pinacol ester (8.43 g, 56.2 mmol), Pd(dppf)$Cl_2$ (2.05 g, 2.81 mmol), and $K_2CO_3$ (11.6 g, 84.3 mmol) were added sequentially to a DMF (150 mL) solution. After $N_2$ replacement three times, the mixture was warmed to 80°C and stirred for 2 h. The reaction completion was monitored by LCMS. After cooling to room temperature, the palladium catalyst was removed by diatomite filtration. The mother liquor was collected, and water (500 mL) was added. Extraction was performed using EA (150 mL×3). The collected organic phase was washed with saturated brine (300 mL). The crude product obtained by concentrating the organic phase was purified by FCC ($SiO_2$, DCM/PE=0-80%) to give a brown solid product **N7-3** (4.94 g, yield 69%). LCMS (m/z): 256.0 (M+H) and 258.0 (M+H).

**Step C:** Compound **N7-4**

At room temperature, $PtO_2$ (388 mg, 0.1 wt) was added to a mixed solution of compound **N7-3** (3.88 g, 15.2 mmol) and EtOH (50 mL). After $H_2$ replacement three times, the mixture was stirred at room temperature for 1.0 h under an $H_2$ (balloon) atmosphere. The reaction completion was monitored by LCMS. The reaction solution was filtered, and the crude product obtained by concentrating the organic phase was purified by FCC ($SiO_2$, EA/PE=0-30%) to yield a yellow solid product **N74** (2.7 g, yield 69%). LCMS (m/z): 258.0 (M+H) and 260.0 (M +H).

**Step D:** Compound **N7-5**

At room temperature, **compound N7-4** (2.70 g, 10.5 mmol), bis(pinacolato)diboron (5.31 g, 20.9 mmol), Pd(dppf)$Cl_2$ (765 mg, 1.05 mmol), and KOAc (3.08 g, 31.4 mmol) were added sequentially to 1,4-dioxane (100 mL).

After $N_2$ replacement three times, the mixture was warmed to 90°C and stirred for 3.0 h. The reaction completion was monitored by LCMS. The palladium catalyst was removed by diatomite filtration, and the crude product obtained after concentration of the mother liquor was purified by FCC ($SiO_2$, DCM/PE=0-80%) to yield a yellow solid product **N75** (3.2 g, 100 % yield). LCMS (m/z): 306.2 (M+H).

**Step E:** Compound **N7-6**

At room temperature, compound **N7-5** (2.30 g, 7.54 mmol), **intermediate w6** (5.59 g, 15.1 mmol), Pd(dtbpf)$Cl_2$ (487 mg, 0.754 mmol), and $K_3PO_4$ (4.79 g, 22.6 mmol) were added sequentially to a solution of 1,4-dioxane (45 mL) and water (9.0 mL). After $N_2$ replacement three times, the mixture was warmed to 110°C and stirred for 1.0 h. The reaction was monitored by LCMS until completion. After cooling to room temperature, the palladium catalyst was removed by diatomite filtration. The mother liquor was collected, and water (80 mL) was added. Extraction was performed using EA (50 mL×3). The collected organic phase was washed with saturated brine (90 mL). The crude product obtained by concentrating the organic phase was purified by FCC ($SiO_2$, EA/PE=0-80%), and then purified by pre-HPLC (C18, ACN/$H_2O$=10-100%) to give a white solid compound **N76** (1.76 g, yield 50 %). LCMS (m/z): 470.2 (M+H).

**Step F:** Compound **N7-7**

At room temperature, NCS (501 mg, 3.75 mmol) and TFA (427 mg, 3.75 mmol) were added sequentially to a mixed solution of compound **N7-6** (1.76 g, 3.75 mmol) and ACN (50 mL), and the mixture was warmed to 45°C and stirred for 4.0 h. The reaction completion was monitored by LCMS. The reaction solution was poured into saturated sodium thiosulfate (70 mL), extracted with EA (50 mL×3), and the organic phase was collected, washed with saturated brine (70 mL), and dried over anhydrous $Na_2SO_4$. After filtration, the organic phase was concentrated to give a yellow solid product **N7-7** (1.89 g, 100% yield). LCMS (m/z): 504.1 (M+H).

**Step G:** Compound **N7-8**

At room temperature, NaOH (7.50 mL, 15.0 mmol, 2 M) was added to a mixed solution of compound **N7-7** (1.89 g, 3.75 mmol) and MeOH (37.5 mL), and the mixture was heated to 60°C and stirred for 2 h. The reaction completion was monitored by LCMS. After cooling to room temperature, the reaction solution was concentrated to obtain a crude product, which was then added to water (70 mL). The pH was adjusted to approximately 3 using HCl (1N). Extraction was performed using EA (50 mL×3), the organic phase was collected, washed with saturated brine (70 mL), and dried over anhydrous $Na_2SO_4$. After filtration, the organic phase was concentrated to give a brown solid product **N78** (1.83 g, 100% yield). LCMS (m/z): 490.1 (M+H).

**Step H:** Compound **N7-9**

Compounds **N7-8** (1.83 g, 3.74 mmol), $NH_4Cl$ (1.00 g, 18.7 mmol), HATU (2.84 g, 7.47 mmol), and DIEA (3.85 g, 29.9 mmol) were added sequentially to a DMF (40 mL) solution at room temperature and stirred for 1.0 h at room temperature. The reaction completion was monitored by LCMS. The reaction solution was poured into water (200 mL) and extracted with EA (50 mL×3). The organic phase was collected, washed with saturated brine (80 mL), and dried over anhydrous $Na_2SO_4$. The crude product obtained after filtration and concentration of the organic phase was purified by FCC ($SiO_2$, EA/PE=0-80%) to give a yellow solid product **N7-9** (1.73 g, 95% yield). LCMS (m/z): 489.1 (M+H).

**Step I:** Compound **N7-10**

At room temperature, NaH (143 mg, 3.58 mmol) was added to a mixed solution of compound **N7-9** (700 mg, 1.43 mmol) and THF (20 mL), and the mixture was warmed to 40°C and stirred for 0.5 h. At 40°C, TCDI (383 mg, 2.15 mmol) was added to the reaction solution, and the mixture was warmed to 60°C and stirred for 2.0 h. The reaction completion was monitored by LCMS. After cooling to room temperature, the reaction solution was poured into a saturated ammonium chloride aqueous solution (50 mL) and then adjusted to pH~5 with HCl (1N), extracted by EA(30 mL×3), the organic phase was collected, washed with saturated brine (70 mL), and dried over anhydrous $Na_2SO_4$. After filtration, the organic phase was concentrated to give a brown solid product **N7-OH** (760 mg, 100% yield). LCMS (m/z): 531.1 (M+H).

**Step J: Intermediate N7-OH**

At room temperature, iodomethane (305 mg, 2.15 mmol), NaOH (229 mg, 5.72 mmol), and compound **N7-10** (760 mg, 1.43 mmol) were added sequentially to a mixed solution of MeOH (15 mL) and $H_2O$ (15 mL), and stirred at room

temperature for 1.0 h. The reaction completion was monitored by LCMS. The reaction solution was then poured into water (30 mL), and the pH was adjusted to 5 with HCl (1 M). Extraction was performed using EA (30 mL×3), the organic phase was collected, washed with saturated brine (70 mL), and dried over anhydrous $Na_2SO_4$. The organic phase was then concentrated after filtration, and the crude product was purified by FCC ($SiO_2$, EA/PE=0-50%) to yield a yellow solid **intermediate N7-OH** (610 mg, yield 78 %). LCMS (m/z): 545.1 (M+H).

**Step K: Intermediate N7**

Under ice-water bath conditions, DMF (20.1 mg, 0.275 mmol) was added to a mixed solution of oxalyl chloride (105 mg, 0.826 mmol) and DCM (3 mL), and stirred for 0.5 h under ice-water bath conditions. The **intermediate N7-OH** (150 mg, 0.275 mmol) was then added under ice-water bath conditions. A solution of DCM (2 mL) was added to the above reaction solution, and the mixture was stirred for 1.0 h after reaching room temperature. The reaction completion was monitored by LCMS. The reaction solution was poured into water (50 mL), extracted with EA (30 mL×3), and the organic phase was collected. The mixture was washed with saturated brine (70 mL) and dried over anhydrous $Na_2SO_4$. After filtration, the organic phase was concentrated, and the crude product was purified by FCC ($SiO_2$, THF/PE=0-20%) to obtain a yellow solid **intermediate N7** (150 mg, yield 97%). LCMS (m/z): 563.1 (M+H).

## Intermediate N8 and N8-OH

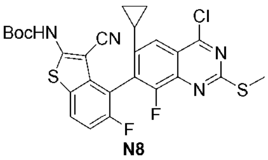
N8

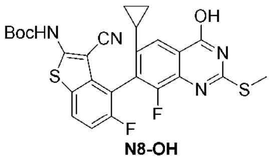
N8-OH

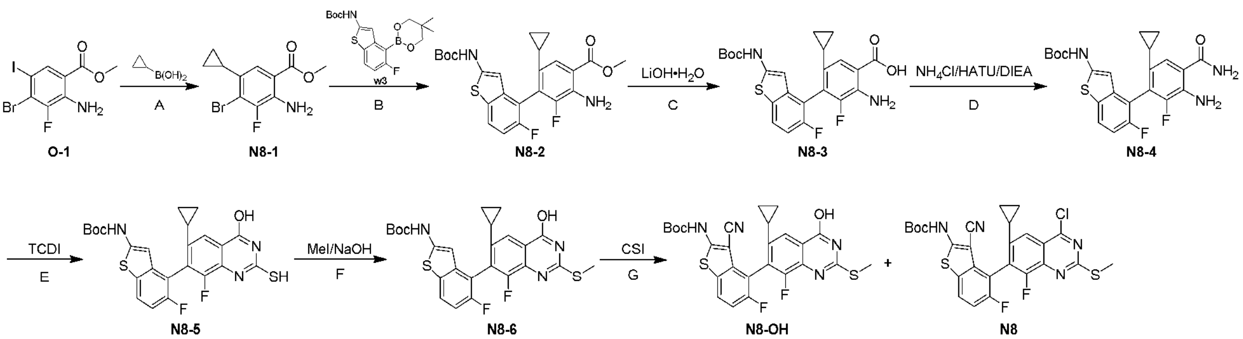


**Step A:** Compound **N8-1**

At room temperature, compound **O-1** (30 g, 80.4 mmol), cyclopropylboronic acid (21 g, 241.3 mmol), Pd(dppf)$Cl_2$ (5.88 g, 8.04 mmol) and $K_3PO_4$ (51 g, 241.3 mmol) were added sequentially to a solution of 1,4-dioxane (300 mL) and water (60 mL). After $N_2$ replacement three times, the mixture was warmed to 100°C and stirred for 36 h. The reaction completion was monitored by LCMS and TLC. The reaction solution was then cooled to room temperature, filtered through diatomite to remove the palladium catalyst, and the mother liquor was collected. Water (150 mL) was added, and the mixture was extracted with EA (150 mL×3). The collected organic phase was washed with saturated brine, dried over anhydrous $Na_2SO_4$, filtered, and the crude product obtained by concentrating the organic liquid was purified by FCC ($SiO_2$, EA/PE=0-16%) to give a yellow oily compound **N8-1** (11.4 g, yield 50%). LCMS (m/z): 228.1 (M+H).

**Step B:** Compound **N8-2**

At room temperature, compound **N8-1** (1.30 g, 4.48 mmol) and intermediate **w3** (2.55 g, 6.72 mmol), RuPhos Pd $G_4$ (381 mg, 0.45 mol), DIEA (2.89 g, 22.4 mmol) were sequentially added to a solution of 1,4-dioxane (25 mL) and water (5 mL). After $N_2$ replacement three times, the mixture was heated to 70°C and stirred for 2 h. The reaction completion was monitored by LCMS and TLC. The reaction solution was brought to room temperature, and the palladium catalyst was removed by diatomite filtration. The mother liquor was collected, and water (50 mL) was added. Extraction was performed using EA (50 mL×3). The collected organic phase was washed with saturated brine, dried over anhydrous $Na_2SO_4$, filtered, and the crude product obtained by concentrating the organic liquid was purified by FCC ($SiO_2$, EA/PE=0-16%) to give a brown solid compound **N8-2** (1.52 g, yield 72%). LCMS (m/z): 475.1 (M+H).

**Step C:** Compound **N8-3**

At room temperature, compound **N8-2** (1.52 g, 3.21 mmol) and lithium hydroxide monohydrate (1.35 g, 32.1 mmol) were added sequentially to a solution of methanol (15 mL) and water (15 mL), and the mixture was warmed to 60°C and stirred for 1 h. The reaction completion was monitored by LCMS and TLC. The pH of the reaction solution was adjusted to approximately 5 with diluted hydrochloric acid, and water (50 mL) was added. Extraction was performed using EA (50 mL×3). The collected organic phase was washed with saturated brine and dried over anhydrous $Na_2SO_4$. After filtration, the organic liquid was concentrated to obtain a brown solid compound **N8-3** (1.48 g, 100% yield). LCMS (m/z): 461.1 (M+H).

**Step D:** Compound **N8-4**

At room temperature, Compound **N8-3** (1.48 g, 3.22 mmol), $NH_4Cl$ (860 mg, 16.1 mmol), HATU (2.43 g, 6.44 mmol), and DIEA (3.32 g, 25.7 mmol) were added sequentially to DMF. The reaction mixture was placed in 20 mL of water. The resulting reaction solution was allowed to react for 3 hours at room temperature. The reaction completion was monitored by LCMS and TLC. The reaction solution was then poured into 300 mL of water and extracted with EA (50 mL×3). The collected organic phase was washed with saturated brine, dried over anhydrous $Na_2SO_4$, filtered, and the crude product obtained by concentrating the organic liquid was purified by FCC ($SiO_2$, EA/PE=0.59%) to give a white solid compound **N8-4** (1.34 g, yield 91%). LCMS (m/z): 460.1 (M+H).

**Step E:** Compound **N8-5**

Sodium hydride (60 wt %, 270 mg, 6.75 mmol) was added to a tetrahydrofuran (30 mL) solution of compound **N8-4** (1.24 g, 2.70 mmol). The resulting reaction solution was stirred at 40°C for 0.5 h. TCDI (721 mg, 4.05 mmol) was then added to the reaction solution, and the temperature was warmed to 60°C and stirred for 2 h. The reaction completion was monitored by LCMS. The reaction solution was then returned to room temperature and poured into a semi-saturated $NH_4Cl$ (30 mL) solution. The pH was adjusted to 5 with diluted hydrochloric acid. Extraction was performed using (50 mL×3). After collecting the organic phase, it was washed with saturated saline solution, dried over anhydrous $Na_2SO_4$, and the organic liquid was concentrated to give a brown solid compound **N8-5** (1.35 mg, crude product). LCMS (m/z): 502.1 (M+H).

**Step F:** Compound **N8-6**

At room temperature, Compound **N8-5** (1.35 g, 2.70 mmol), NaOH (432 mg, 10.8 mmol), and methyl iodide (575 mg, 4.05 mmol) were added sequentially to a solution of methanol (1.5 mL) and water (15 mL), and stirred for 2 h at room temperature. The reaction completion was monitored by LCMS. The pH of the reaction solution was adjusted to 5 with hydrochloric acid, and extracted with EA (50 mL×3). After the organic phase was collected, the mixture was washed with saturated saline solution, dried over anhydrous $Na_2SO_4$, filtered, and the crude product obtained by concentrating the organic liquid was purified by FCC ($SiO_2$, THF/PE=0-31%) to give a yellow solid compound **N8-6** (1.21 g, yield 87%). LCMS (m/z): 516.1 (M+H).

**Step F:** Intermediate **N8** and **N8-OH**

Under ice-salt bath (-10°C) conditions, CSI (2.19 g, 15.5 mmol) was added to a solution of Compound **N8-5** (2 g, 3.88 mmol) in MeCN (150 mL), the reaction mixture was stirred and kept at the same temperature for 1 h. DMF (20 mL) was then added to the reaction solution. The resulting reaction solution was allowed to react for another 2 h at room temperature. The reaction completion was monitored by TLC. The reaction solution was then poured into water (50 mL). Extraction performed using EA (50 mL×3), the organic phase was collected, washed with saturated brine (50 mL), and dried over anhydrous $Na_2SO_4$. The mixture was filtered, and the organic phase was concentrated and purified by FCC ($SiO_2$, THF/PE=0-38%) to yield a white solid intermediate **N8** (501 mg, 23% yield) and a pale yellow solid intermediate **N8-OH** (1.0 g, 48% yield). LCMS (m/z): 559.1 (M+H), 541.1 (M+H).

## Intermediate N9 and N9-OH

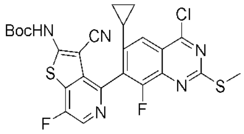

N9

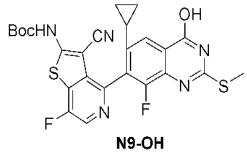

N9-OH

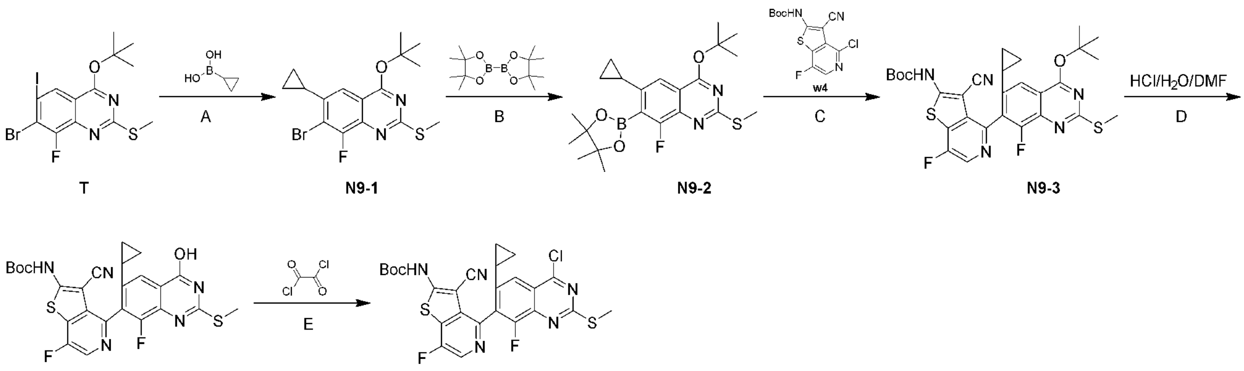


T N9-1 N9-2 N9-3

N9-OH N9

**Step A:** Compound **N9-1**

At room temperature, intermediate **T** (1.50 g, 3.18 mmol), cyclopropylboronic acid( 547 mg, 6.37 mmol), and Pd(dppf)$Cl_2$. 233 mg (0.318 mmol), and $K_3PO_4$ (2.02 g, 9.55 mmol) were sequentially added to a solution of 1,4-dioxane (45 mL) and water (9.0 mL). After $N_2$ replacement three times, the mixture was warmed to 100°C and stirred for 2h. The reaction completion was monitored by LCMS. The palladium catalyst was removed by diatomite filtration, and the mother liquor was collected. Water (50 mL) was added, and the mixture was extracted with EA (30 mL×3). The collected organic phase was washed with saturated brine (70 mL), filtered, and the organic phase was concentrated. The crude product was purified by FCC ($SiO_2$, EA/PE=0-10%) to yield a yellow solid compound **N9-1** (1.03 g, yield 85%). LCMS (m/z): 329.0 and 331.0 (M+H).

**Step B:** Compound **N9-2**

At room temperature, compound **N9-1** (900 mg, 2.34 mmol), bis(pinacolato)diboron (1.48 g, 5.84 mmol), $Pd(OAc)_2$ (52.9 mg, 0.234 mmol), BIDIME (154 mg, 0.467 mmol), and $K_3PO_4$ (1.49 g, 7.01 mmol) were sequentially added to 1,4-dioxane (20 mL). After $N_2$ replacement three times, the mixture was warmed to 110°C and stirred for 16 h. The reaction completion was monitored by LCMS. The palladium catalyst was removed by diatomite filtration. The mother liquor was collected, concentrated, and the crude product was purified by FCC ($SiO_2$, EA/PE=0-15%) to obtain a yellow solid compound **N9-2** (400 mg, yield 40 %). LCMS (m/z): 433.2 (M+H).

**Step C:** Compound **N9-3**

At room temperature, compound **N9-2** (400 mg, 0.925 mmol), intermediate **w4** (243 mg, 0.740 mmol), and Pd(dtppf)$Cl_2$ (59.7 mg, 0.0925 mmol), and $K_3PO_4$ (588 mg, 2.78 mmol) were sequentially added to a solution of 1,4-dioxane (10 mL) and water (2.0 mL). After $N_2$ replacement three times, the mixture was warmed to 110°C and stirred for 2 h. The reaction completion was monitored by LCMS. The palladium catalyst was removed by diatomite filtration, and the mother liquor was collected. Water (50 mL) was added, and the mixture was extracted with EA (30 mL×3). The collected organic phase was washed with saturated brine (70 mL), filtered, and concentrated. The crude product was purified by FCC ($SiO_2$, EA/PE=0-50%) to obtain a yellow solid product compound **N9-3** (200 mg, yield 36%). LCMS (m/z): 598.2 (M+H).

**Step D:** Intermediate **N9-OH**

At room temperature, hydrochloric acid (1 mol/L, 6.0 mL) was added to a mixture of compound **N9-3** (200 mg, 0.335 mmol) in DMF (10 mL) and stirred at room temperature for 2.0 h. The reaction completion was monitored by LCMS. The reaction solution was poured into water (50 mL), extracted with EA (30 mL×3), and the organic phase was collected. The mixture was washed with saturated brine (70 mL), dried over anhydrous $Na_2SO_4$, filtered, and the organic phase was

concentrated. The crude product was purified by FCC ($SiO_2$, EA/PE=0-80%) to yield a yellow solid intermediate **N9-OH** (90 mg, yield 50%). LCMS (m/z): 542.0 (M+H).

**Step E:** Intermediate **N9**

Under ice-water bath conditions, DMF (4.00 mg, 0.0554 mmol) was added to a mixture of oxalyl chloride (21.1 mg, 0.166 mmol) in DCM (3.0 mL) and stirred in an ice-water bath for 0.5 h. Under ice-water bath conditions, a mixture of intermediate **N9-OH** (30.0 mg, 0.0554 mmol) in DCM (1.0 mL) was added dropwise to the above reaction solution, and stirred for 1.0 h after returning to room temperature. The reaction completion was monitored by LCMS. After returning to room temperature, the reaction solution was quenched in 50 mL of water. Extraction was performed using EA (30 mL×3), the organic phase was collected, washed with saturated brine (70 mL), dried over anhydrous $Na_2SO_4$, filtered, and the organic phase was concentrated. The crude product was purified by FCC ($SiO_2$, THF/PE=0-15%) to yield an intermediate **N9** (25 mg, yield 81%) as a yellow solid. LCMS (m/z): 560.1 (M+H).

## Intermediate N10 and N10-OH

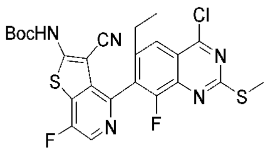

**N10**

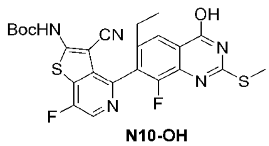

**N10-OH**

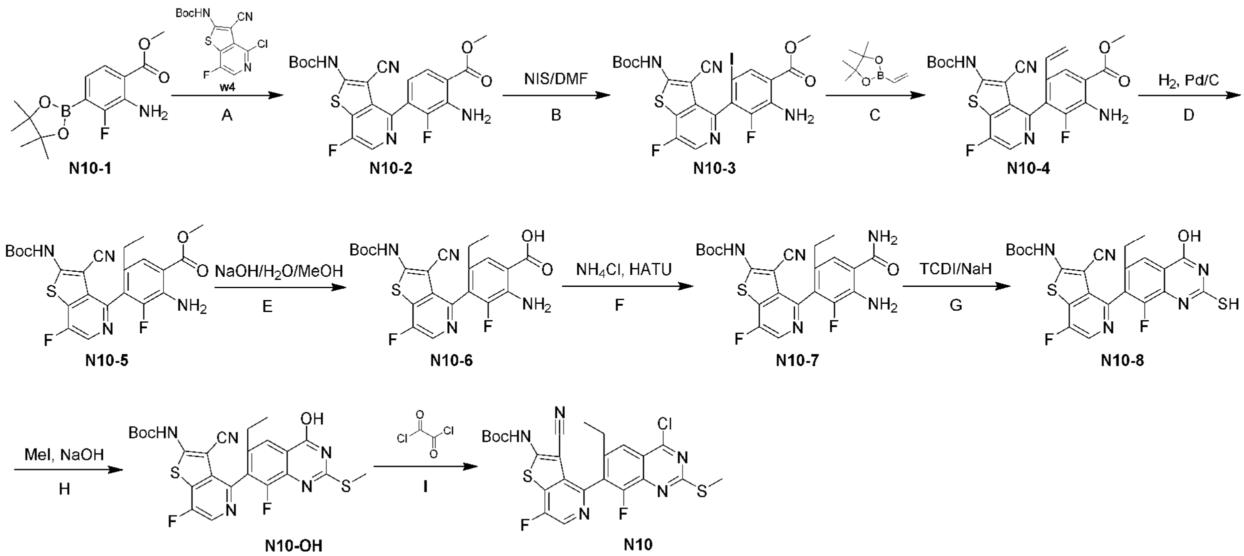


**Step A:** Compound **N10-2**

At room temperature, Compound **N10-1** (1.90 g, 6.44 mmol),Compound **w4** (2.32 g, 7.08 mmol), Pd (dtppf)$Cl_2$ (416 mg, 0.644 mmol), and $K_3PO_4$ (4.09 g, 19.3 mmol) were added sequentially to a solution of 1,4-dioxane (45 mL) and water (9.0 mL). After $N_2$ replacement three times, the mixture was warmed to 90°C and stirred for 2 h. The reaction completion was monitored by LCMS. After cooling to room temperature, the palladium catalyst was removed by diatomite filtration. The mother liquor was collected, and water (80 mL) was added. Extraction was performed using EA (50 mL×3). The collected organic phase was washed with saturated brine (90 mL). The crude product obtained by concentrating the organic phase was purified by FCC ($SiO_2$, EA/PE=0-40%) to give a yellow solid compound **N10-2** (2.24 g, yield 76%). LCMS (m/z): 461.0 (M+H).

**Step B:** Compound **N10-3**

At room temperature, NIS (4.32 g, 17.0 mmol) and $Ag_2SO_4$ (5.31 g, 17.0 mmol) were added sequentially to a mixed solution of compound **N10-2** (2.24 g, 4.86 mmol) and DMF (50 mL), and stirred at room temperature for 0.5 h. The reaction completion was monitored by LCMS. The reaction solution was filtered and poured into saturated sodium thiosulfate (100 mL). Extraction was performed using EA (30 mL×3), the organic phase was collected, washed with

saturated brine (70 mL), dried over anhydrous $Na_2SO_4$, filtered, and the organic phase was concentrated to give a yellow solid product, compound **N10-3** (2.31 g, yield 81%). LCMS (m/z): 587.0 (M+H).

**Step C:** Compound **N10-4**

At room temperature, Compound **N10-3** (2.05 g, 3.50 mmol), vinylboronic acid pinacol ester (1.08 g, 6.99 mmol), Pd(dtppf)$Cl_2$ (226 mg, 0.350 mmol), and $K_3PO_4$. (2.22 g, 10.5 mmol) were added sequentially to a solution of 1,4-dioxane (45 mL) and water (9.0 mL). After $N_2$ replacement three times, the mixture was warmed to 90°C and stirred for 2 h. The reaction completion was monitored by LCMS. After cooling to room temperature, the palladium catalyst was removed by diatomite filtration. The mother liquor was collected, and water (60 mL) was added. Extraction was performed using EA (30 mL×3). The collected organic phase was washed with saturated brine (70 mL). The crude product obtained by concentrating the organic phase was purified by FCC ($SiO_2$, EA/PE=0-40%) to give a yellow solid compound **N10-4** (1.28 g, yield 75%). LCMS (m/z): 487.1 (M+H).

**Step D:** Compound **N10-5**

At room temperature, Pd/C (10 wt%, 128 mg) was added to a mixed solution of compound **N10-4** (1.28 g, 2.63 mmol) and MeOH (50 mL). After $H_2$ replacement three times, and under a $H_2$ (balloon) atmosphere, the mixture was stirred at room temperature for 2.0 h. The reaction completion was monitored by LCMS. The reaction solution was filtered, and the organic phase was concentrated to obtain a brown solid compound **N10-5** (1.15 g, 90 % yield). LCMS (m/z): 489.0 (M+H).

**Step E:** Compound **N10-6**

At room temperature, NaOH (4.7 mL, 9.42 mmol, 2 M) was added to a mixed solution of compound **N10-5** (1.15 g, 2.35 mmol) and MeOH (25 mL), and the mixture was warmed to 60°C and stirred for 2 h. The reaction completion was monitored by LCMS. After cooling to room temperature, the reaction solution was concentrated to obtain a crude product, which was then added to water (50 mL) and adjusted to pH 3 with HCl (1 M). Extraction was performed using EA (30 mL×3). The organic phase was collected, washed with saturated brine (70 mL), dried over anhydrous $Na_2SO_4$, filtered, and the organic phase was concentrated to obtain a brown solid compound **N10-6** (1.03 g, yield 92 %). LCMS (m/z): 475.1 (M+H).

**Step F:** Compound **N10-7**

At room temperature, Compound **N10-6** (1.03 g, 2.17 mmol), $NH_4Cl$ (581 mg, 10.9 mmol), HATU (1.65 g, 4.34 mmol), and DIEA (2.24 g, 17.4 mmol) were added sequentially to a 20 mL DMF solution and stirred at room temperature for 1.0 h. The reaction completion was monitored by LCMS. The reaction solution was poured into 100 mL of water, extracted with EA (30 mL×3), and the organic phase was collected. The mixture was washed with saturated brine (70 mL), dried over anhydrous $Na_2SO_4$, filtered, and the organic phase was concentrated. The crude product was purified by FCC ($SiO_2$, EA/PE=0-80%) to yield a yellow solid compound **N10-7** (990 mg, 96% yield). LCMS (m/z): 474.1 (M+H).

**Step G:** Compound **N10-8**

At room temperature, NaH (116 mg, 2.50 mmol) was added to a mixed solution of compound **N10-7** (550 mg, 1.16 mmol) and THF (10 mL), and the mixture was warmed to 40°C and stirred for 0.5 h. At 40°C, TCDI (223 mg, 1.74 mmol) was added to the reaction solution, and the mixture was warmed to 60°C and stirred for 2.0 h. The reaction completion was monitored by LCMS. After cooling to room temperature, the reaction solution was poured into a saturated ammonium chloride aqueous solution (50 mL), and then the pH was adjusted to 5 with 1M hydrochloric acid. Extraction was performed using by (30 mL×3), the organic phase was collected, washed with saturated brine (70 mL), dried over anhydrous $Na_2SO_4$, filtered, and concentrated to give a brown solid compound **N10-8** (599 mg, 100% yield). LCMS (m/z): 516.1 (M+H).

**Step H:** Intermediate **N10-OH**

At room temperature, iodomethane (248 mg, 1.75 mmol), NaOH (186 mg, 4.66 mmol), and compound **N10-8** (600 mg, 1.16 mmol) were added sequentially to a mixed solution of MeOH (10 mL) and $H_2O$ (10 mL), and stirred at room temperature for 1.0 h. The reaction completion was monitored by LCMS. The reaction solution was then poured into water (30 mL), and the pH was adjusted to 5 with 1M hydrochloric acid. Extraction with EA was performed using (30 mL×3), the organic phase was collected, washed with saturated brine (70 mL), dried over anhydrous $Na_2SO_4$, filtered, and the organic phase was concentrated. The crude product was purified by FCC ($SiO_2$, EA/PE=0-50%) to obtain a yellow solid intermediate **N10-OH** (371 mg, yield 60%). LCMS (m/z): 530.2 (M+H).

**Step I:** Intermediate **N10**

Under ice-water bath conditions, DMF (20.7 mg, 0.283 mmol) was added to a mixed solution of oxaloyl chloride (108 mg, 0.849 mmol) and DCM (5 mL), and stirred for 0.5 h under ice-water bath conditions. A solution of intermediate **N10-OH** (150 mg, 0.283 mmol) in DCM (2 mL) was added to the above reaction solution under ice-water bath conditions, and the mixture was stirred for 1.0 h after being brought to room temperature. The reaction completion was monitored by LCMS, and the reaction solution was poured into water (50 mL). Extracted was performed using EA (30 mL×3), the organic phase was collected, washed with saturated brine (70 mL), dried over anhydrous $Na_2SO_4$, filtered, and the organic phase was concentrated. The crude product was purified by FCC ($SiO_2$, THF/PE=0-15%) to obtain a yellow solid intermediate **N10** (140 mg, yield 90%). LCMS (m/z): 548.1 (M+H).

## Intermediate N11 and N11-OH

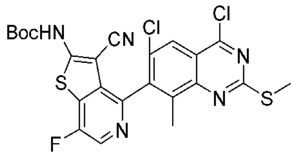

**N11**

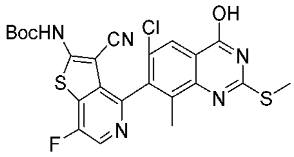

**N11-OH**

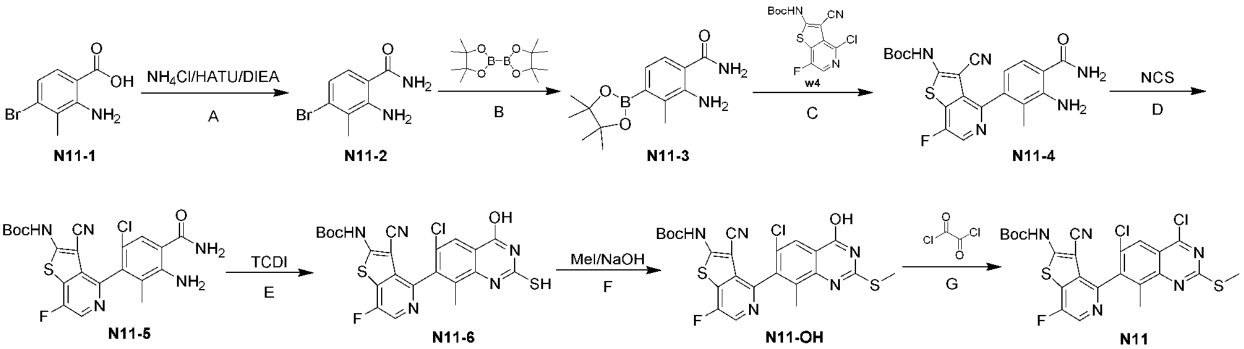


N11-1 N11-2 N11-3 N11-4 N11-5 N11-6 N11-OH N11

**Step A:** Compound **N11-2**

At room temperature, Compound **N11-1** (5.0 g, 21.8 mmol), $NH_4Cl$ (3.5 g, 65.4 mmol), HATU (16.6 g, 43.6 mmol), and DIEA (14.0 g, 109.0 mmol) were added sequentially to DMF (25 mL). The resulting reaction solution was allowed to react for another 3 hours at room temperature. The reaction completion was monitored by LCMS and TLC. The reaction solution was then poured into 300 mL of water, stirred at room temperature for 30 minutes, filtered, and the filter cake was dried in a vacuum drying oven to obtain a brown solid (24 g, yield 80%). LCMS (m/z): 231.1 (M+H).

**Step B:** Compound **N11-3**

At room temperature, compound **N11-2** (4.0 g, 17.5 mmol), bis(pinacolato)diboron (5.35 g, 21.1 mmol), Pd(dppf) $Cl_2$ (640 mg, 0.875 mmol), and potassium acetate (2.57 g, 26.3 mmol) were added sequentially to 1,4-dioxane (60 mL). After $N_2$ replacement three times, the mixture was warmed to 90°C and stirred for 2 h. The reaction completion was monitored by LCMS and TLC. The reaction solution was then returned to room temperature, and the palladium catalyst was removed by diatomite filtration. The crude product obtained from the concentrated mother liquor was purified by FCC ($SiO_2$, THF/PE=0-49%) to give a yellow solid compound **N11-3** (4.4 g, 91% yield). LCMS (m/z): 277.1 (M+H).

**Step C:** Compound **N11-4**

At room temperature, Compound **N11-3** (633 mg, 2.29 mmol) and **w4** (500mg, 1.53 mmol), Pd-118 (100 mg, 0.153 mmol), and $K_3PO_4$ (973 mg, 4.59 mmol) were sequentially added to a solution of 1,4-dioxane (10 mL) and water (2 mL). After $N_2$ replacement three times, the mixture was heated to 90°C and stirred for 3 h. The reaction completion was monitored by LCMS and TLC. The reaction solution was then brought to room temperature, filtered through diatomite to remove the palladium catalyst, and the mother liquor was collected. Water (30 mL) was added, and the mixture was extracted with EA (30 mL×3). The collected organic phase was washed with saturated brine, dried over anhydrous

$Na_2SO_4$, filtered, and the crude product obtained by concentrating the organic liquid was purified by FCC ($SiO_2$, THF/PE=0-62%) to give a yellow solid compound **N11-4** (630 mg, yield 93%). LCMS (m/z): 442.1 (M+H).

**Step D:** Compound **N11-5**

At room temperature, TFA (326 mg, 2.86 mmol), NCS (381 mg, 2.86 mmol) were added sequentially to a solution of compound **N11-4** (630 mg, 1.43 mmol) in acetonitrile (15 mL). The resulting reaction solution was allowed to react for another 16 h at room temperature. The reaction completion was monitored by LCMS and TLC. The reaction solution was concentrated, and the crude product was purified by FCC ($SiO_2$, THF/PE=0-55%) to give a yellow solid compound **N11-5** (630 mg, yield 59%). LCMS (m/z): 476.1 (M+H).

**Step E:** Compound **N11-6**

At room temperature, sodium hydride (60 wt%, 84 mg, 2.09 mmol) was added to a solution of compound **N11-5** (399 mg, 0.84 mmol) in tetrahydrofuran (10 mL). The resulting reaction solution was stirred at 40°C for 0.5 h, and then TCDI (224 g, 1.26 mmol) was added to the reaction solution. The temperature was warmed to 60°C and stirred for 2 h. The reaction completion was monitored by LCMS. The reaction solution was then returned to room temperature and poured into a semi-saturated $NH_4Cl$ (20 mL) solution. The pH was adjusted to 5 with hydrochloric acid. Extraction was performed using EA (30 mL×3), the organic phase was collected, washed with saturated saline solution, dried over anhydrous $Na_2SO_4$, and the organic liquid was concentrated to give a brown solid compound **N11-6** (430 mg, crude product). LCMS (m/z): 518.1 (M+H).

**Step F:** Intermediate **N11-OH**

At room temperature, compound **N11-6** (430 mg, 0.84 mmol), NaOH (134 mg, 3.36 mmol), and iodomethane (155 mg, 1.09 mmol) were added sequentially to a solution of methanol (5 mL) and water (5 mL), and stirred at room temperature for 2 h. The reaction completion was monitored by LCMS. The pH of the reaction solution was adjusted to 5 with hydrochloric acid. Extraction was performed using EA (30 mL×3), the organic phase was collected, washed with saturated saline solution, and dried over anhydrous $Na_2SO_4$. The mixture was filtered, the organic liquid was concentrated, and the crude product was purified by FCC ($SiO_2$, THF/PE=0-34%) to give a white solid intermediate **N11-OH** (275 mg, yield 62%). LCMS (m/z): 532.1 (M+H).

**Step G:** Intermediate **N11**

Under ice bath conditions, DMF (38 mg, 0.52 mmol) was added to a DCM (5 mL) solution of oxalyl chloride (197 mg, 1.55 mmol), and the reaction was stirred for 0.5 h while maintaining the temperature. The intermediate **N11-OH** (274 mg, 0.52 mmol) was then added to the reaction solution. The resulting reaction solution was allowed to react for another 1 h at room temperature. The reaction completion was monitored by LCMS. The reaction solution was then poured into water (30 mL), extracted with DCM (30 mL×3), and the organic phase was collected. The organic phase was washed with saturated saline solution, dried over anhydrous $Na_2SO_4$, concentrated, and the crude product was purified by FCC ($SiO_2$, THF/PE=0-13%) to obtain a white solid intermediate **N11** (203 mg, yield 71%). LCMS (m/z): 550.1 (M+H).

The following intermediates were prepared according to the above intermediate preparation scheme and appropriate variants.

| Number | Structural Formula | Characterization Data |
|---|---|---|
| **Intermediate N12-OH** | 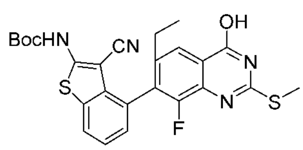 | LCMS (m/z): 511.1 (M+H). |
| **Intermediate N12** | 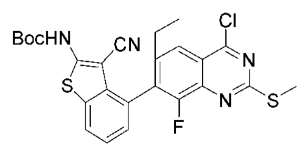 | LCMS (m/z): 529.1 (M+H). |

(continued)

| Number | Structural Formula | Characterization Data |
|---|---|---|
| **Intermediate N13-OH** | 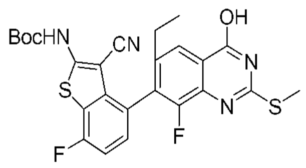 | LCMS (m/z): 529.2 (M+H). |
| **Intermediate N13** | 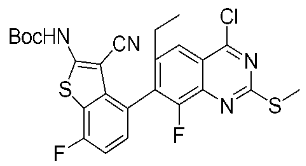 | LCMS (m/z): 547.1 (M+H). |
| **Intermediate N14-OH** | 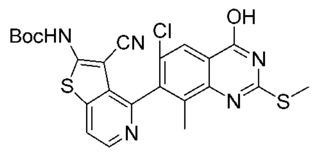 | LCMS (m/z): 514.1 (M+H). |
| **Intermediate N14** | 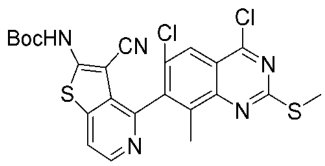 | LCMS (m/z): 532.0 (M+H). |
| **Intermediate N15-OH** | 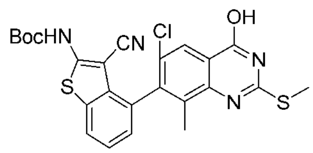 | LCMS (m/z): 513.1 (M+H). |
| **Intermediate N15** | 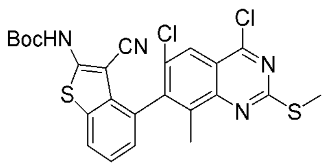 | LCMS (m/z): 531.0 (M+H). |
| **Intermediate N16-OH** | 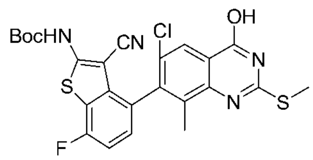 | LCMS (m/z): 531.1 (M+H). |
| **Intermediate N16** | 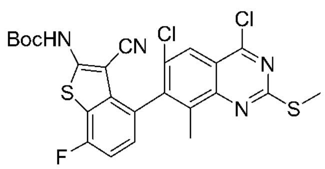 | LCMS (m/z): 549.0 (M+H). |
| **Intermediate N17-OH** | 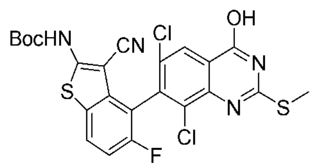 | LCMS (m/z): 551.0 (M+H). |

(continued)

| Number | Structural Formula | Characterization Data |
|---|---|---|
| **Intermediate N17** | 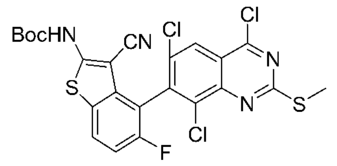 | LCMS (m/z): 569.0 (M+H). |

## Intermediate N18

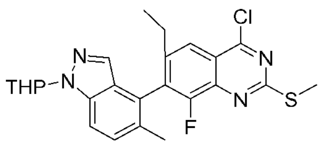

**N18**

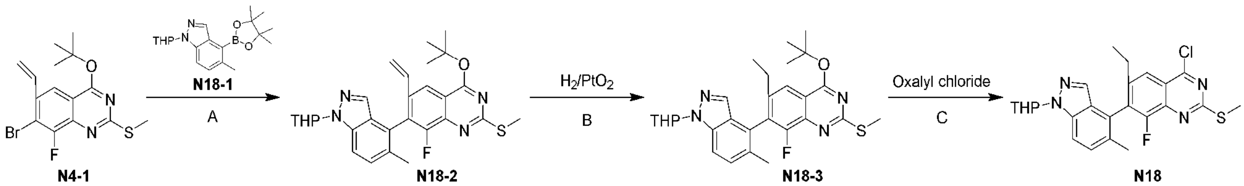


N4-1 N18-2 N18-3 N18

**Step A:** Compound **N18-2**

At room temperature, Compound **N18-1** (492 mg, 1.44 mmol), Compound **N4-1** (450 mg, 1.20 mmol), $Pd(PPh_3)_4$ (277 mg, 0.240 mol), and $K_3PO_4$ (763 mg, 3.60 mmol) were sequentially added to a solution of 1,4-dioxane (8 mL) and water (2 mL). After $N_2$ replacement three times, the mixture was warmed to 85°C and stirred for 16 h. The reaction completion was monitored by LCMS and TLC. The reaction solution was brought to room temperature, and the palladium catalyst was removed by diatomite filtration. The mother liquor was collected, and water (50 mL) was added. Extraction was performed using EA (50 mL×3). The collected organic phase was washed with saturated brine, dried over anhydrous $Na_2SO_4$, filtered, and the crude product obtained by concentrating the organic liquid was purified by FCC ($SiO_2$, EA/PE=0-10%) to obtain a white solid compound **N18-2** (203 g, yield 33%). LCMS (m/z): 507.1 (M+H).

**Step D:** Compound **N18-3**

At room temperature, Compound **N18-2** (203 mg) and $PtO_2$ (20 mg) were added sequentially to a methanol (4 mL) solution. After $H_2$ replacement three times, the mixture was stirred at room temperature for 4 h. The reaction completion was monitored by LCMS and TLC. The reaction solution was filtered through diatomite to remove the palladium catalyst. The mother liquor was collected, and the organic liquid was concentrated. The crude product was purified by FCC ($SiO_2$, EA/PE=0-11%) to obtain a white solid compound **N18-3** (175 mg, yield 86%). LCMS (m/z): 509.1 (M+H).

**Step E:** Intermediate **N18**

Under ice bath conditions, DMF (25 mg, 0.34 mmol) was added to a solution of oxalyl chloride (130 mg, 1.02 mmol) in DCM (4 mL), and the reaction was stirred for 0.5 h while maintaining the temperature. Compound **N18-3** (173 mg, 0.34 mmol) was then added to the reaction solution. The resulting reaction solution was allowed to react for another 1 h at room temperature. The reaction completion was monitored by LCMS. The reaction solution was then poured into water (30 mL), extracted with DCM (30 mL×3), and the organic phase was collected. The organic phase was washed with saturated saline solution, dried over anhydrous $Na_2SO_4$, and the organic phase was concentrated and purified by FCC ($SiO_2$, THF/PE=0-9%), obtained a white solid intermediate **N18** (126 mg, yield 79%). LCMS (m/z): 550.1 (M+H).

## Intermediates N19-OH, N19-OH-A and N19-OH-B

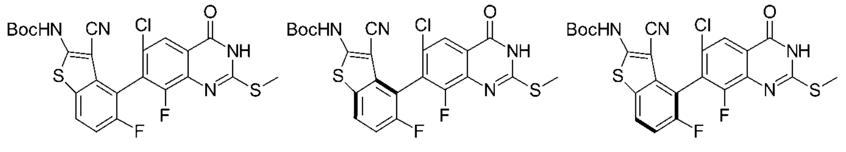

N19-OH N19-OH-A N19-OH-B

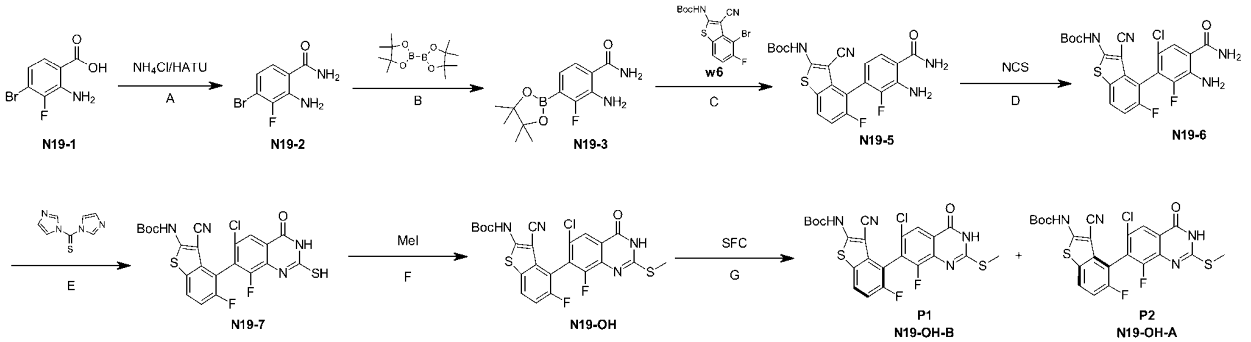


N19-1 N19-2 N19-3 N19-5 N19-6 N19-7 N19-OH P1 N19-OH-B + P2 N19-OH-A

**Step A:** Compound **N19-2**

At room temperature, 2-amino-3-fluoro-4-bromobenzoic acid (60.0 g, 256 mmol), $NH_4Cl$ (41.2 g, 769 mmol), HATU (146 g, 385 mmol), and DIEA (99.2 g, 769 mmol) were added sequentially to DMF (400 mL). The resulting reaction solution was allowed to react for another 3 h at room temperature. The reaction completion was monitored by LCMS and TLC. The reaction solution was poured into water (4 L), stirred at room temperature for 30 min, filtered, and the filter cake was dried in a vacuum drying oven to obtain a gray solid compound **N19-2** (50.8 g, yield 85%). LCMS (m/z): 233.1 (M+H).

**Step B:** Compound **N19-3**

At room temperature, Compound **N19-2** (22.1 g, 94.8 mmol), bis(pinacolato)diboron (28.9 g, 113.8 mmol), Pd(dppf)$Cl_2$ (3.47 g, 4.74 mmol), and potassium acetate (139 g, 142.2 mmol) were added sequentially to 1,4-dioxane (400 mL). After $N_2$ replacement three times, the mixture was warmed to 90°C and stirred for 4 h. The reaction completion was monitored by LCMS and TLC. The reaction solution was then returned to room temperature, and the palladium catalyst was removed by diatomite filtration. The crude compound **N19-3** obtained by concentrating the mother liquor was used directly in the next step. LCMS (m/z): 281.1 (M+H).

**Step C:** Compound **N19-5**

At room temperature, Compound **N19-3** (26.6 g, 94.8 mmol), intermediate **w6** (23.5 g, 63.5 mmol), Pd-118 (4.14 g, 6.35 mmol), and $K_3PO_4$ (40.4 g, 190.5 mmol) were added sequentially to a solution of 1,4-dioxane (400 mL) and water (80 mL). After $N_2$ replacement three times, the mixture was warmed to 90°C and stirred for 2 h. The reaction completion was monitored by LCMS and TLC. The reaction solution was brought to room temperature, and the palladium catalyst was removed by diatomite filtration. The mother liquor was collected, and water (300 mL) was added. Extraction was performed using EA (200 mL×3). The collected organic phase was washed with saturated brine, dried over anhydrous $Na_2SO_4$, filtered, and the crude product obtained by concentrating the organic liquid was purified by FCC ($SiO_2$, THF/PE=0-55%) to give a yellow solid compound **N19-5** (22.1 g, two-step yield 78%). LCMS (m/z): 445.3 (M+H).

**Step D:** Compound **N19-6**

At room temperature, NCS (7.97 g, 59.7 mmol) was added to a solution of compound **N19-5** (22.1 g, 49.8 mmol) in DMF (200 mL). The reaction solution was allowed to react for another 16 h at room temperature. The reaction completion was monitored by LCMS and TLC. The crude product obtained through concentration was purified by FCC ($SiO_2$, THF/PE=0-55%) to give a yellow solid compound **N19-6** (23.1 g, 97% yield). LCMS (m/z): 479.1 (M+H).

**Step E:** Compound **N19-7**

At room temperature, Sodium hydride (60 wt%, 7.73 g, 193.2 mmol) was added to a solution of compound **N19-6**

(23.1 g, 48.3 mmol) in tetrahydrofuran (400 mL). The resulting reaction solution was stirred at 40°C for 0.5 h. N,N'-thiocarbonyldiimidazole (10.3 g, 58.0 mmol) was added to the reaction solution, and the temperature was warmed to 60°C and stirred for 2 h. The reaction completion was monitored by LCMS. The reaction solution was returned to room temperature, the pH was adjusted to 3-5 with hydrochloric acid, and the solution was extracted with EA (150 mL×3). The organic phase was collected, washed with saturated saline solution, dried over anhydrous $Na_2SO_4$, and the organic liquid was concentrated to obtain a brown solid compound **N19-7** (26.5 g, crude product). LCMS (m/z): 521.1 (M +H).

**Step F:** Intermediate **N19-OH**

At room temperature, Compound **N19-7** (26.5 g, 51.0 mmol), NaOH (8.15 g, 20.4 mmol), and methyl iodide (21.7 g, 153 mmol) were added sequentially to a solution of methanol (200 mL) and water (200 mL), and stirred for 2 h at room temperature. The reaction completion was monitored by LCMS. The pH of the reaction solution was adjusted to 3-5 with hydrochloric acid, and the solution was extracted with EA (150 mL×3). The organic phase was collected, washed with saturated saline solution, and dried over anhydrous $Na_2SO_4$. After filtration and concentration, the crude product was purified by FCC ($SiO_2$, THF/PE=0-34%) to obtain a white solid intermediate **N19-OH** (19 g, two-step yield 74%). LCMS (m/z): 535.1 (M+H).

**Step G: Intermediates N19-OH-A and N19-OH-B**

The intermediate **N19-OH** (10g) obtained from step F was resolved by SFC (Waters SFC 150, separation column: DAICELCHIRALCEL® IC 250*50 mm 10 μm; mobile phase: $CO_2$/MeOH=55/45; flow rate: 150 mL/min) to obtain the first eluted isomer **P1,** which is the intermediate **N19-OH-B** (4.4g, yield 44%, relatively short retention time). Chirality analysis method SFC-N19, Rt = 2.501 min. LCMS (m/z): 535.1 (M+H). The subsequently eluted isomer **P2** was the intermediate **N19-OH-A** (4.6 g, yield 46%, relatively long retention time). Chirality analysis was performed using the SFC-N19 method, Rt = 3.208 min. LCMS (m/z): 535.1 (M+H).

Chiral analysis method SFC-N19: Waters UPCC (CA-352), analytical column: DAICELCHIRALPAK® IC, 100*3mm 3μm, mobile phase A: $CO_2$, mobile phase B: MeOH; flow rate: 1.5mL/min; column temperature: 35°C; back pressure: 1800 psi; gradient: 0-8 min A/B=70/30.

**Intermediate N19A**

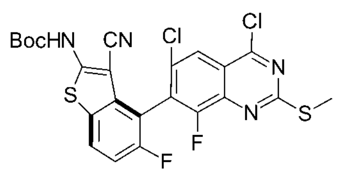

**N19A**

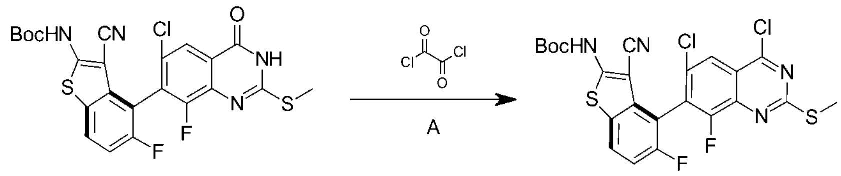


**N19-OH-A** **N19A**

**Step A:** Intermediate **N19A**

Under ice bath conditions, DMF (85 mg, 1.16 mmol) was added to a DCM (45 mL) solution of oxalyl chloride (2.21 g, 17.4 mmol), and the reaction was stirred for 0.5 h while maintaining the temperature. Intermediate **N19-OH-A** (3.1 g, 5.81 mmol) was then added to the reaction solution. The resulting reaction solution was allowed to react for another 1 h at room temperature. The reaction completion was monitored by LCMS. The reaction solution was then poured into water (50 mL), extracted with DCM (50 mL×3), and the organic phase was collected. The mixture was washed with saturated saline solution, dried over anhydrous $Na_2SO_4$, concentrated, and purified by FCC ($SiO_2$, THF/PE=0-15%) to obtain a white solid intermediate **N19A** (2.34 g, yield 73%). LCMS S (m/z): 553.0 (M+H).

**N19** and **N19B** were prepared using the same method.

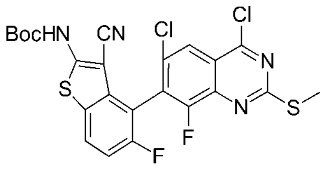

N19

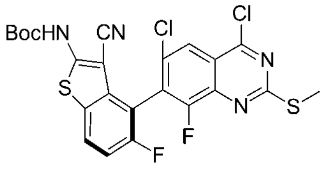

N19B

Intermediate **N19** was prepared from intermediate **N19-OH,** LCMS (m/z): 553.0 (M+H). Intermediate **N19B** was prepared from intermediate **N19-OH-B,** LCMS (m/z): 553.0 (M+H).

## Intermediate N20-OH

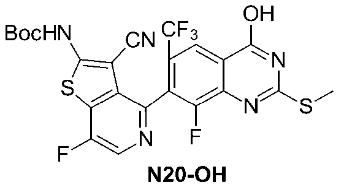

**N20-OH**

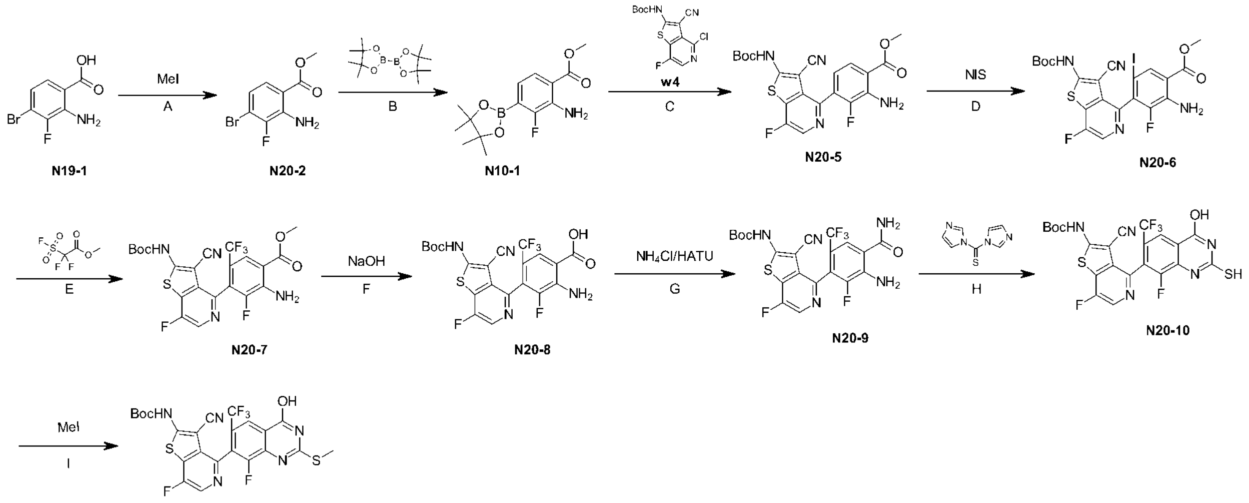


**Step A:** Compound **N20-2**

At room temperature, $Cs_2CO_3$ (52.2 g, 160 mmol) and iodomethane (18.2 g, 128 mmol) were added sequentially to a mixture of compound **N19-1** (25.0 g, 107 mmol) in DMF (200 mL) and stirred for 16 h at room temperature. After the reaction completion was monitored by LCMS, the reaction mixture was poured into water (800 mL), extracted with EA (150 mL×3), and the organic phase was collected. The mixture was washed with saturated brine (500 mL), dried over anhydrous $Na_2SO_4$, filtered, and the organic phase was concentrated. The crude product was slurried and purified by PE (150 mL), and the filter cake was dried under vacuum to give a white solid compound **N$_2$O-2** g, yield 65%). LCMS (m/z): 249.9 (M+H).

**Step B:** Compound **N10-1**

At room temperature, compound **N20-2** (8.00 g, 32.3 mmol), bis(pinacolato)diboron (9.83 g, 38.7 mmol), Pd(dppf)$Cl_2$ (1.18 g, 1.61 mmol), and KOAc (9.48 g, 96.8 mmol) were added sequentially to 1,4-dioxane (200 mL). After $N_2$ replacement three times, the mixture was warmed to 100°C and stirred for 4 h. The reaction completion was monitored by LCMS. The palladium catalyst was removed by diatomite filtration, and the mother liquor was collected. The crude product obtained after concentration was purified by FCC ($SiO_2$, EA/PE=0-25%) to obtain a white solid compound **N10-1** (9.5 g, crude product). LCMS (m/z): 214.1 (M+H).

**Step C:** Compound **N20-5**

At room temperature, intermediate **w4** (2.70 g, 8.24 mmol), compound **N10-1** (3.16 g, 10.7 mmol), Pd(dtbpf)$Cl_2$ (537 mg, 0.824 mmol), and $K_3PO_4$ (5.24 g, 24.7 mmol) were added sequentially to a solution of 1,4-dioxane (80 mL) and water (20 mL). After $N_2$ replacement three times, the mixture was warmed to 90°C and stirred for 2 h. The reaction completion was monitored by LCMS. The palladium catalyst was removed by diatomite filtration. The mother liquor was collected, and water (100 mL) was added. Extraction was performed using EA (50 mL×3). The collected organic phase was washed with saturated brine (100 mL), dried over anhydrous $Na_2SO_4$, filtered, and concentrated. The crude product was purified by FCC ($SiO_2$, EA/PE=0-50%) to obtain a yellow solid compound **N20-5** (3.8 g, 100% yield). LCMS (m/z): 461.1 (M+H).

**Step D:** Compound **N20-6**

At room temperature, $I_2$ (4.19 g, 16.5 mmol) and $Ag_2SO_4$ (5.15 g, 16.5 mmol) were added sequentially to a mixed solution of compound **N20-5** (3.80 g, 8.25 mmol) and DMF (80 mL), and stirred for 1 h at room temperature. The reaction completion was monitored by LCMS. The reaction solution was filtered and poured into saturated sodium thiosulfate (400 mL), extracted with EA (80 mL×3), and the organic phase was collected. The mixture was washed with saturated brine (200 mL) and dried over anhydrous $Na_2SO_4$. After filtration and concentration, the crude product was purified by FCC ($SiO_2$, EA/PE=0-50%) to obtain a brown solid compound **N20-6** (3.9 g, yield 81%). LCMS (m/z): 587.0 (M+H).

**Step E:** Compound **N20-7**

At room temperature, CuI (6.50 g, 34.1 mmol), TEA (3.45 g, 34.1 mmol), and methyl fluorosulfonyl difluoroacetate (6.55 g, 34.1 mmol) were added sequentially to a mixed solution of compound **N20-6** (2.00 g, 3.41 mmol) and NMP (40 mL). After $N_2$ replacement three times, the mixture was warmed to 90°C and stirred for 1 h. The reaction completionwas monitored by LCMS. The copper catalyst was removed by diatomite filtration, and the mother liquor was collected. Water (300 mL) was added, and the mixture was stirred at room temperature for 0.5 h. The crude filter cake obtained after filtration was purified by FCC ($SiO_2$, THF/PE=0-50%) to obtain a yellow solid compound **N20-7** (1.2 g, yield 67%). LCMS (m/z): 529.1 (M+H).

**Step F:** Compound **N20-8**

At room temperature, NaOH (3.60 mL, 7.19 mmol, 2 M) was added to a mixed solution of compound **N20-7** (950 mg, 1.80 mmol) and MeOH (18.0 mL), and the mixture was warmed to 60°C and stirred for 1 h. The reaction completion was monitored by LCMS. After cooling to room temperature, the reaction solution was concentrated to obtain a crude product, which was then added to water (50 mL) and adjusted to pH 3 with HCl (1 M). Extraction was performed using EA (30 mL×3). The organic phase was collected, washed with saturated brine (70 mL), dried over anhydrous $Na_2SO_4$, filtered, and the organic phase was concentrated to obtain a white solid compound **N20-8** (850 mg, yield 92%). LCMS (m/z): 515.1 (M+H).

**Step G:** Compound **N20-9**

At room temperature, Compound **N20-8** (850 mg, 1.65 mmol), $NH_4Cl$ (442 mg, 8.26 mmol), HATU (1.26 g, 3.30 mmol), and DIEA (1.71 g, 13.2 mmol) were added sequentially to a DMF (16 mL) solution and stirred at room temperature for 1 h. The reaction completion was monitored by LCMS. The reaction solution was poured into 100 mL of water, extracted with EA (30 mL×3), and the organic phase was collected. The mixture was washed with saturated brine (70 mL), dried over anhydrous $Na_2SO_4$, filtered, and the organic phase was concentrated. The crude product was purified by FCC ($SiO_2$, EA/PE=0-80%) to obtain a yellow solid compound **N20-9** (650 mg, yield 77%). LCMS (m/z): 514.1 (M+H).

**Step H:** Compound **N20-10**

At room temperature, NaH (127 mg, 3.16 mmol) was added to a mixed solution of compound **N20-9** (650 mg, 1.27 mmol) and THF (20 mL), and the mixture was heated to 40°C and stirred for 0.5 h. At 40°C, TCDI (338 mg, 1.90 mmol) was added to the reaction solution, and the mixture was warmed to 60°C and stirred for 2.0 h. The reaction completion was monitored by LCMS. After cooling to room temperature, the reaction solution was poured into a saturated ammonium chloride aqueous solution (50 mL), and the pH was adjusted to 5 with HCl (1 M). Extraction was performed using EA (30 mL×3). The organic phase was collected, washed with saturated brine (70 mL), dried over anhydrous $Na_2SO_4$, filtered, and concentrated to obtain a brown solid compound **N20-10** (703 mg, 100% yield). LCMS (m/z): 556.1 (M+H).

**Step I:** Intermediate **N20-OH**

At room temperature, iodomethane (270 mg, 1.90 mmol), NaOH (202 mg, 5.06 mmol), and compound **N20-10** (703 mg, 1.27 mmol) were sequentially added to a mixed solution of MeOH (10 mL) and $H_2O$ (10 mL), and stirred at room temperature for 1 h. After the reaction completion was monitored by LCMS, the reaction solution was poured into water (50 mL), and the pH was adjusted to 5 with HCl (1 M). Extraction was performed using EA (30 mL×3), and the organic phase was collected. The mixture was washed with saturated brine (70 mL), dried over anhydrous $Na_2SO_4$, filtered, and the organic phase was concentrated. The crude product was purified by FCC ($SiO_2$, EA/PE=0-60%) to obtain a yellow solid intermediate **N20-OH** (265 mg, yield 37%). LCMS (m/z): 570.1 (M+H).

## Intermediate N20

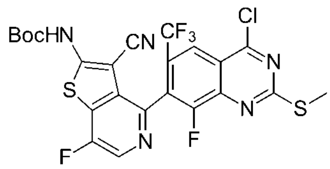

**N20**

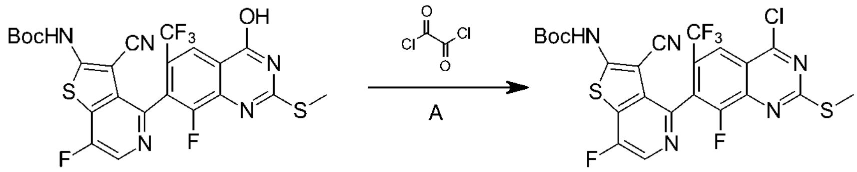


**N20-OH** **N20**

**Step A:** Intermediate **N20**

Under ice-water bath conditions, DMF (29.5 mg, 0.404 mmol) was added to a mixed solution of oxalyl chloride (230 mg, 1.21 mmol) and DCM (5 mL), and stirred for 0.5 h under ice-water bath conditions. Under ice-water bath conditions, a solution of intermediate **N20-OH** (230 mg, 0.404 mmol) in DCM (2 mL) was added to the above reaction solution, and the mixture was stirred for 2.0 h after reaching room temperature. The reaction was monitored by LCMS until completion. The reaction solution was poured into water (50 mL), extracted with EA (30 mL×3), and the organic phase was collected. The mixture was washed with saturated brine (70 mL), dried over anhydrous $Na_2SO_4$, filtered, and the organic phase was concentrated. The crude product was purified by FCC ($SiO_2$, THF/PE=0-20%) to obtain a yellow solid intermediate **N20** (210 mg, yield 88%). LCMS (m/z): 588.1 (M+H).

## Intermediate N21-OH

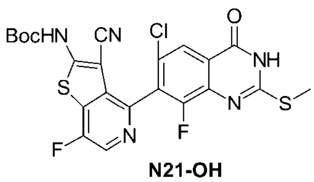

**N21-OH**

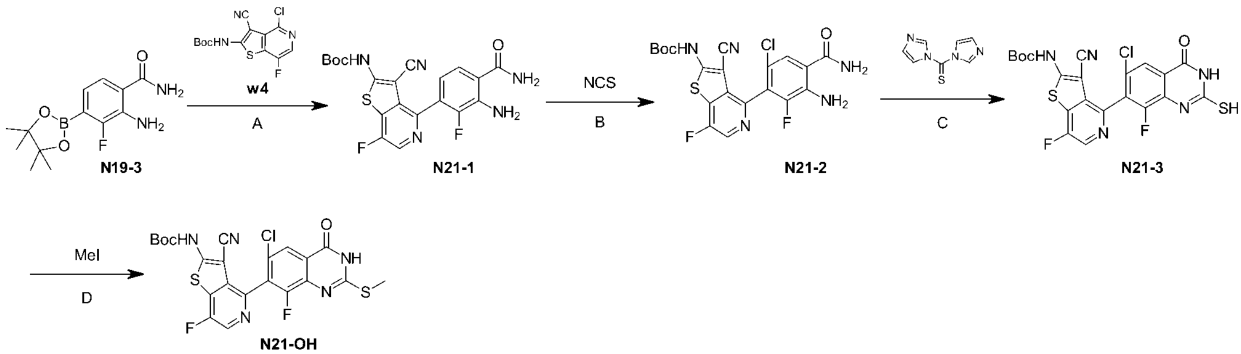


**N19-3** **N21-1** **N21-2** **N21-3** **N21-OH**

**Step A:** Compound **N21-1**

At room temperature, intermediate **w4** (4.0 g, 12.2 mmol) and Compound **N19-3** (4.45g, 15.9mmol), Pd-118 (797mg, 1.22mmol), $K_3PO_4$ (7.78 g, 36.7 mmol) were sequentially added to a solution of 1,4-dioxane (50 mL) and water (10 mL). After $N_2$ replacement three times, the mixture was heated to 80°C and stirred for 2 h. The reaction completion was monitored by LCMS and TLC. The reaction solution was brought to room temperature, and the palladium catalyst was removed by diatomite filtration. The mother liquor was collected, and water (50 mL) was added. Extraction was performed using EA (50 mL×3). The collected organic phase was washed with saturated brine, dried over anhydrous $Na_2SO_4$, filtered, and the organic liquid was concentrated. The crude product was purified by FCC ($SiO_2$, THF/PE=0-42%) to give a yellow solid compound **N21-1** (2.45 g, yield 45%). LCMS (m/z): 446.1 (M+H).

**Step B:** Compound **N21-2**

At room temperature, NCS (882 mg, 6.61 mmol) was added to a solution of Compound **N21-1** (2.45 g, 5.51 mmol) in DMF (25 mL). The resulting reaction solution was allowed to react for another 16 h at room temperature. The reaction completion was monitored by LCMS and TLC. The reaction solution was then added to water (50 mL), extracted with EA (50 mL×3), and the collected organic phase was washed with saturated brine, dried over anhydrous $Na_2SO_4$, filtered, and concentrated. The crude product was purified by FCC ($SiO_2$, THF/PE=0-53%) to give a yellow solid compound **N21-2** (1.7 g, yield 64%). LCMS (m/z): 480.1 (M+H).

**Step C:** Compound **N21-3**

At room temperature, sodium hydride (60 wt%, 568 mg, 14.2 mmol) was added to a solution of compound N21-2 (1.7 g, 3.55 mmol) in tetrahydrofuran (40 mL). The resulting reaction solution was stirred at 40°C for 0.5 h. N,N'-thiocarbonyldiimidazole (758 mg, 4.26 mmol) was then added to the reaction solution, and the temperature was raised to 60°C and stirred for 2 h. The reaction completion was monitored by LCMS. The reaction solution was then returned to room temperature, and the pH was adjusted to 4-5 with hydrochloric acid. Extraction was performed using EA (50 mL×3), the organic phase was collected, washed with saturated saline solution, dried over anhydrous $Na_2SO_4$, and the organic liquid was concentrated to give a yellow solid compound **N21-3** (1.75 g, crude product). LCMS (m/z): 522.1 (M+H).

**Step D:** Intermediate **N21-OH**

At room temperature, Compound **N21-3** (1.75 g), NaOH (568 mg, 14.2 mmol), and methyl iodide (1.51 g, 10.7 mmol) were added sequentially to a solution of methanol (10 mL) and water (10 mL), and stirred at room temperature for 2 h. The reaction completion was monitored by LCMS. The reaction solution was adjusted to pH 4-5 with hydrochloric acid, extracted with EA (50 mL×3), and the organic phase was collected. The mixture was washed with saturated saline solution, dried over anhydrous $Na_2SO_4$, filtered, and the organic liquid was concentrated. The crude product was purified by FCC ($SiO_2$, THF/PE=0-52%) to obtain a yellow solid intermediate **N21-OH** (1.3 g, two-step yield 68%). LCMS (m/z): 536.0 (M+H).

## Intermediate N21

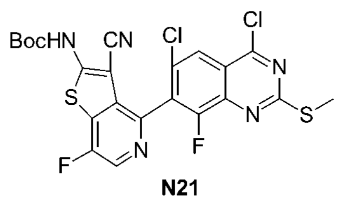

**N21**

The synthesis of intermediate **N21** was performed following that of intermediate **N20.** LCMS (m/z): 554 .0 (M+H).

## Intermediate N22

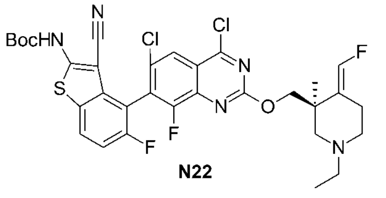

N22

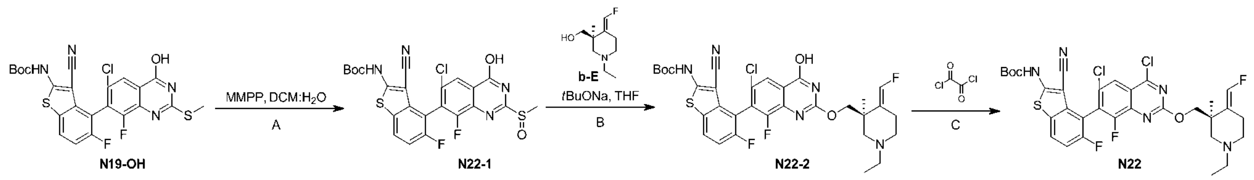


N19-OH N22-1 N22-2 N22

**Step A:** Compound **N22-1**

At room temperature, magnesium monoperoxyphthalate hexahydrate (2.77 g, 87 wt%, 5.61 mmol) was added to a mixed solution of intermediate **N19-OH** (1.00 g, 1.87 mmol) in dichloromethane (15 mL) and $H_2O$ (15 mL), and the mixture was stirred at room temperature for 2 h. The reaction completion was monitored by LCMS. Water (30 mL) was added, and the mixture was extracted with DCM (150 mL×3). The combined organic phases were washed with saturated brine, dried over anhydrous sodium sulfate, filtered, and concentrated to give a yellow solid compound **N22-1** (1.0 g, 97% yield). LCMS (m/z): 555.1 (M+H).

**Step B:** Compound **N22-2**

Under dry ice and ethanol bath conditions, NaOtBu (2M in THF, 4.5 mL, 9.05 mmol) was added dropwise to Compound **N22-1** (1.00 g, 1.81 mmol) and intermediate **b-E** (680 mg, 3.63 mmol) in THF (30mL). The mixture was reacted at this temperature for 0.5 hours, then warmed to room temperature and reacted overnight. The reaction completion was monitored by LCMS. The reaction solution was poured into a saturated $NH_4Cl$ aqueous solution (50mL), extracted with EA (50mL×3), and the collected organic phase was concentrated. The crude product was purified by FCC ($SiO_2$, EA/PE=0-80%) to obtain a yellow solid compound **N22-2** (1.0g, 82%). LCMS (m/z): 674.1 (M+H).

**Step C:** Intermediate **N22**

Under ice bath conditions, DMF (35 mg, 0.475 mmol) was added to a DCM (9 mL) solution of oxalyl chloride (271 mg, 2.14 mmol). The mixture was stirred at this temperature for 0.5 h, and compound **N22-2** (320 mg, 0.475 mmol) was added to the reaction solution. The resulting reaction solution was allowed to react for another 1 h at room temperature. The reaction completion was monitored by LCMS. The reaction solution was then poured into a $NaHCO_3$ aqueous solution (50 mL), extracted with DCM (50 mL×3), and the organic phase was collected. The mixture was washed with saturated saline solution, dried over anhydrous $Na_2SO_4$, and concentrated to give a yellow solid intermediate **N22** (280 mg, 85% yield). LCMS S (m/z): 692.1 (M+H).

## Intermediate N23-OH

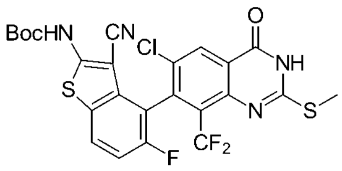

**N23-OH**

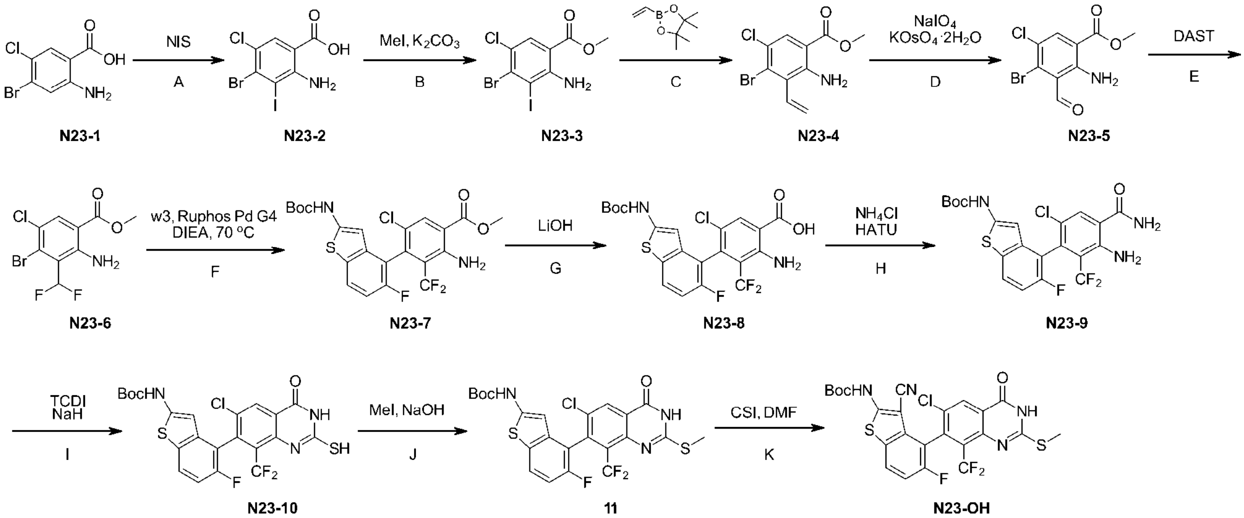


**N23-1** **N23-2** **N23-3** **N23-4** **N23-5** **N23-6** **N23-7** **N23-8** **N23-9** **N23-10** **11** **N23-OH**

**Step A:** Compound **N23-2**

At room temperature, *N*-iodosuccinimide (9.9 g, 43.9 mmol) and p-toluenesulfonic acid (688 mg, 3.99 mmol) were added to a solution of Compound 2-amino-4-bromo-5-chlorobenzoic acid (**N23-1,** 10.0 g, 39.9 mmol) in DMF (100 mL). The resulting reaction solution was warmed to 70°C and stirred for 12 h. After the reaction completion was monitored by LCMS, the reaction solution was poured into 300 mL of ice water and extracted with ethyl acetate. The organic phases were combined, washed with saturated brine, dried over anhydrous sodium sulfate, filtered, and concentrated to dryness. The crude product was purified by FCC ($SiO_2$, THF/PE=0-35%) to give a yellow solid compound **N23-2** (10.5 g, 70% yield). LCMS (m/z): 375.8 (M+H).

**Step B:** Compound **N23-3**

At room temperature, potassium carbonate (6.6 g, 47.8 mmol) and methyl iodide (5.1 g, 35.9 mmol) were added to a solution of compound **N23-2** (9.0 g, 23.9 mmol) in DMF (100 mL). The reaction was stirred for 2 h while maintaining the temperature. After the reaction completion was monitored by LCMS, the reaction solution was poured into 300 mL of ice water and extracted with ethyl acetate. The organic phases were combined, washed with saturated brine, dried over anhydrous sodium sulfate, filtered, and concentrated to dryness. The crude product was purified by FCC ($SiO_2$, EA/PE=0-15%) to give a yellow solid compound **N23-3** (8.1 g, yield 87%). LCMS (m/z): 392.0 (M+H).

**Step C:** Compound **N23-4**

Under $N_2$ conditions, to a solution of Compound **N23-3** (7.5 g, 19.2 mmol) in DMF, vinylboronic acid pinacol ester (5.9 g, 38.4 mmol), potassium carbonate (5.3 g, 38.4 mmol), and Pd(dppf)$Cl_2$ (1.4 g, 1.92 mmol) were added. The resulting reaction solution was stirred at 80°C for 2 h. After the reaction completion was monitored by LCMS, the reaction solution was filtered through diatomite, the filtrate was concentrated to dryness, and the crude product was purified by FCC ($SiO_2$, DCM/PE=0-15%) to give a yellow solid compound **N23-4** (2.9 g, yield 52%). $^1$H NMR (400 MHz, DMSO-$d_6$) δ 7.81 (s, 1H), 6.70 (s, 2H), 6.49 (dd, *J* = 18.0, 11.5 Hz, 1H), 5.75 (dd, *J* = 11.4, 1.4 Hz, 1H), 5.54 (dd, *J* = 18.0, 1.4 Hz, 1H), 3.82 (s, 3H). LCMS (m/z): 290.0 (M+H).

**Step D:** Compound **N23-5**

At room temperature, potassium osmium tetroxide monohydrate (316 mg, 1.0 mmol) and sodium periodate (8.5 g, 40.0 mmol) were added to a solution of compound **N23-4** (2.9 g, 10.0 mmol) in 1,4-dioxane (30 mL) and water (10 mL).

The reaction was stirred for 20 h while maintaining the temperature. After the reaction completion was monitored by LCMS, the reaction solution was filtered through diatomite and washed with EA (80 mL). The filtrate was washed with saturated brine, dried over anhydrous sodium sulfate, filtered and concentrated to dryness. The crude product was purified by FCC ($SiO_2$, DCM/PE=0-10%) to give a yellow solid compound **N23-5** (1.3 g, yield 45%). $^1H$ NMR (400 MHz, DMSO-$d_6$) δ 10.31 (s, 1H), 8.66 (s, 2H), 8.11 (s, 1H), 3.85 (s, 3H). LCMS (m/z): 291.9 (M+H).

**Step E:** Compound **N23-6**

Under nitrogen protection, diethylaminosulfur trifluoride (3.64 g, 22.4 mmol) was added to a solution of compound **N23-5** (1.1 g, 3.76 mmol) in dichloromethane (10 mL). The mixture was heated to 40°C and stirred for 6 h. After the reaction completion was monitored by LCMS, the reaction solution was poured into 30 mL of water and extracted with dichloromethane. The organic phases were combined, washed with saturated brine, dried over anhydrous sodium sulfate, filtered, and concentrated to dryness. The crude product was purified by FCC ($SiO_2$, DCM/PE=0-10%) to give a yellow solid product, compound **N23-6** (600 mg, yield 51%). $^1H$ NMR (400 MHz, DMSO-$d_6$) δ 8.01 (s, 1H), 7.43 (t, *J* = 52.1 Hz, 1H), 7.15 (s, 2H), 3.83 (s, 3H). LCMS (m/z): 314.0 (M+H).

**Step F:** Compound **N23-7**

Under $N_2$ conditions, intermediates **w3** (1.2 g, 3.1 mmol), DIPEA (1.35 g, 10.5 mmol), and RuphosPd-G4 (178 mg, 0.21 mmol) were added to a solution of compound **N23-6** (660 mg, 2.1 mmol) containing 1,4-dioxane (10 mL) and water (2 mL). The resulting reaction solution was stirred at 70°C for 2 h. After the reaction was completion as monitored by LCMS, the reaction solution was filtered through diatomite, the filtrate was concentrated to dryness, and the crude product was purified by FCC ($SiO_2$, THF/PE=0-15%) to give a yellow solid compound **N23-7** (315 mg, yield 30%). LCMS (m/z): 501.0 (M+H).

**Step G:** Compound **N23-8**

At room temperature, lithium hydroxide monohydrate (104 mg, 2.84 mmol) was added to a solution of compound **N23-7** (310 mg, 0.62 mmol) in anhydrous tetrahydrofuran (3 mL), methanol (1 mL), and water (1 mL). The reaction was stirred for 2 h while maintaining the temperature. After the reaction completion was monitored by LCMS, the reaction solution was poured into a 1 N hydrochloric acid aqueous solution (10 mL). The solution was then extracted with ethyl acetate. The organic phases were combined, washed with saturated brine, dried over anhydrous sodium sulfate, filtered, and concentrated to dryness to give a yellow solid crude product, compound **N23-8** (310 mg). LCMS (m/z): 487.0 (M+H).

**Step H:** Compound **N23-9**

Under nitrogen protection at 0°C, ammonium chloride (168 mg, 3.2 mmol), HATU (365 mg, 0.96 mmol), and DIPEA (412 mg, 3.2 mmol) were added to a solution of compound **N23-8** (310 mg) in anhydrous DMF (6 mL), and the mixture was stirred for 2 h while maintaining the temperature. After the reaction was completion as monitored by LCMS, the reaction solution was poured into 50 mL of ice water and then extracted with ethyl acetate. The organic phases were combined, washed with saturated brine, dried over anhydrous sodium sulfate, filtered, and concentrated to dryness. The crude product was purified by FCC ($SiO_2$, THF/PE=0-30%) to give a yellow solid compound **N23-9** (300 mg, 97% yield in two steps). LCMS (m/z): 486.3 (M+H).

**Step I:** Compound **N23-10**

Under nitrogen protection, NaH (99 mg, 2.48 mmol, 60 wt%) was added to a solution of compound **N23-9** (300 mg, 0.62 mmol) in anhydrous THF (3 mL). The resulting reaction solution was stirred at 40°C for 20 min. Then, N,N'-thiocarbonyldiimidazole (132 mg, 0.74 mmol) was added to the reaction solution, and the temperature was raised to 60°C and stirred for 2 h. After the reaction completion was monitored by LCMS, the reaction solution was poured into 20 mL of 0.5 N hydrochloric acid solution. The solution was then extracted with ethyl acetate. The combined organic phases were washed with saturated brine, dried over anhydrous sodium sulfate, filtered, and concentrated to dryness to obtain a yellow solid crude product, compound **N23-10** (300 mg). LCMS (m/z): 528.1 (M+H).

**Step J:** Compound **N23-11**

Under ice bath conditions, sodium hydroxide (114 mg, 2.84 mmol) and methyl iodide (121 mg, 0.85 mmol) were added to a solution of compound **N23-10** (300 mg, 0.57 mmol) in methanol (3 mL) and water (3 mL), and the mixture was

stirred for 1 h while maintaining the temperature. After the reaction completion was monitored by LCMS, the reaction solution was poured into a 0.5 N hydrochloric acid solution (20 mL) in water. The solution was then extracted with ethyl acetate. The combined organic phases were washed with saturated brine, dried over anhydrous sodium sulfate, filtered, and concentrated to dryness. The crude product was purified by FCC ($SiO_2$, THF/PE=0-20%) to give a yellow solid compound **N23-11** (167 mg, yield 54%). LCMS (m/z): 542.0 (M+H).

**Step K:** Intermediate **N23-OH**

Under nitrogen protection in an ice bath, chlorosulfonyl isocyanate (130 mg, 0.93 mmol) was added to a solution of compound **N23-11** (166 mg, 0.31 mmol) in acetonitrile (8 mL). The reaction was stirred for 0.5 h while maintaining the temperature, followed by the addition of DMF (1 mL). The resulting reaction solution was stirred for another 0.5 h at room temperature. After the reaction completion was monitored by LCMS, the reaction solution was poured into a saturated sodium bicarbonate aqueous solution (50 mL) and collected with ethyl acetate. The organic phases were combined, washed with saturated brine, dried over anhydrous sodium sulfate, filtered, and concentrated to dryness. The crude product was purified by FCC ($SiO_2$, THF/PE=0-20%) to give a yellow solid intermediate **N23-OH** (116 mg, yield 67%). LCMS (m/z): 567.0 (M+H).

## Intermediate N23

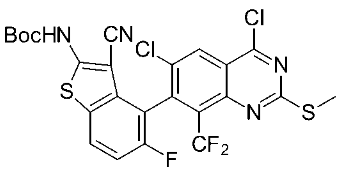

**N23**

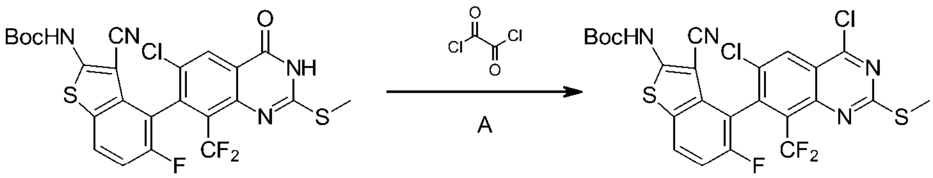


**N23-OH** **N23**

**Step A:** Intermediate **N23**

Under nitrogen protection at 0°C, DMF (3 mg, 0.04 mmol) was added to anhydrous dichloromethane (5 mL) containing oxalyl chloride (74 mg, 0.58 mmol). After stirring and maintaining the temperature for 0.5 h, intermediate **N23-OH** (110 mg, 0.19 mmol) was added to the reaction solution, and the reaction was continued at 0°C for 3 h. After the reaction completion was monitored by LCMS, the reaction solution was poured into 20 mL of saturated sodium bicarbonate solution and extracted with dichloromethane. The organic phases were combined, washed with saturated brine, dried over anhydrous sodium sulfate, filtered, and concentrated to dryness. The crude product was purified by FCC ($SiO_2$, EA/PE=0-15%) to obtain a yellow solid intermediate **N23** (80 mg, yield 70%). LCMS (m/z): 585.0 (M+H).

# Intermediate N24-OH and N24

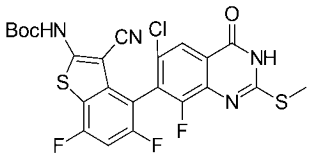
N24-OH

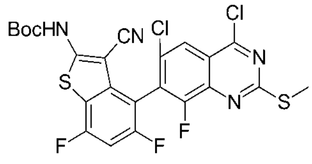
N-24

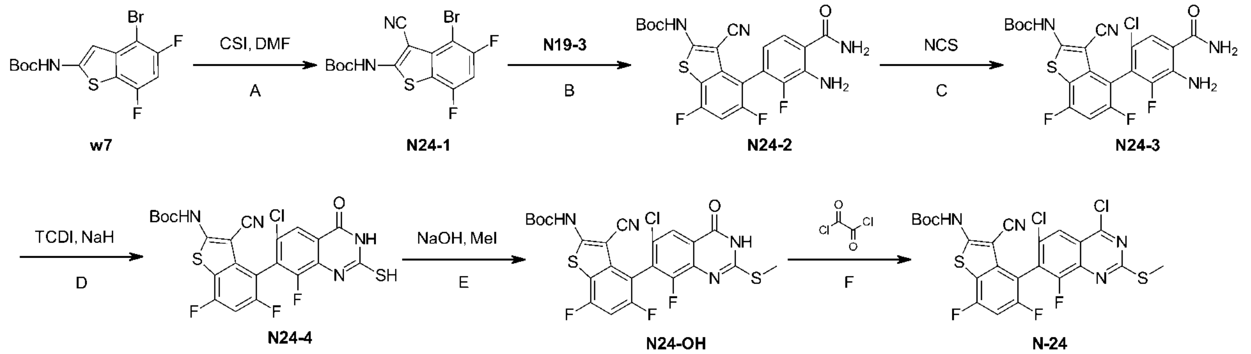

w7 N24-1 N24-2 N24-3 N24-4 N24-OH N-24

**Step A:** Compound **N24-1**

Under the protection of nitrogen in an ice bath, chlorosulfonyl isocyanate (1.29 g, 9.13 mmol) was added to intermediate **w7** (1.1 g, 2.97 mmol) in an acetonitrile (55 mL) solution. The mixture was stirred at the same temperature for 0.5 h, then DMF (2 mL) was added, and the reaction mixture was stirred at room temperature for another 0.5 h. After the reaction completion was monitored by LCMS, the reaction mixture was filtered, and the filter cake was dried to give a yellow solid compound **N24-1** (610 mg, yield 52%). LCMS (m/z): 332.9 (M-56).

**Step B:** Compound **N24-2**

Under $N_2$ conditions, Compound **N19-3** (667 mg, 2.51 mmol), potassium phosphate (1.06 g, 5.01 mmol), and Pd(dtbpf)$Cl_2$ (108 mg, 0.17 mmol) were added to a solution of compound **N24-1** (650 mg, 1.67 mmol) in 1,4-dioxane (10 mL) and water (2 mL). The resulting reaction solution was stirred at 100°C for 2 h. After the reaction completion was monitored by LCMS, the reaction solution was filtered through diatomite, the filtrate was concentrated to dryness, and the crude product was purified by FCC ($SiO_2$, THF/PE=0-35%) to yield a yellow solid compound **N24-2** (640 mg, yield 83%). LCMS (m/z): 643.1 (M+H).

**Step C:** Compound **N24-3**

At room temperature, *N*-chlorosuccinimide (203 mg, 1.52 mmol) was added to compound **N24-2** (640 mg, 1.38 mmol) in a solution of DMF (10 mL), and the reaction mixture was stirred at room temperature for 12 h. After the reaction completion was monitored by LCMS, the reaction mixture was poured into 50 mL of ice water and then extracted with ethyl acetate. The organic phases were combined, washed with saturated brine, dried over anhydrous sodium sulfate, filtered, and concentrated to dryness. The crude product was purified by FCC ($SiO_2$, THF/PE=0-35%) to obtain a yellow solid compound **N24-3** (635 mg, yield 93%). LCMS (m/z): 497.1 (M+H).

**Step D:** Compound **N24-4**

Under nitrogen protection, NaH (123 mg, 5.11 mmol, 60% purity) was added to compound **N24-3** (635 mg, 1.28 mmol) in anhydrous THF (10 mL) solution, stirred the resulting reaction solution at 40°C for 20 min. Then added *N,N'*-thiocarbonyldiimidazole (296 mg, 1.66 mmol) to the reaction solution, warmed to 60°C, and stirred for 2 h. After the reaction completion was monitored by LCMS, the reaction solution was poured into 10 mL of 0.5 N hydrochloric acid solution and 20 mL of water. Extracted with ethyl acetate. The organic phases were combined, washed with saturated brine, dried with anhydrous sodium sulfate, filtered, and concentrated to obtain a yellow solid crude product, compound **N24-4** (600 mg). LCMS (m/z): 539.2 (M+H).

**Step E:** Intermediate **N24-OH**

Under ice bath conditions, sodium hydroxide (256 mg, 6.39 mmol) and methyl iodide (272 mg, 1.92 mmol) were added to a methanol (5 mL) and water (5 mL) solution of compound **N24-4** (600 mg, 1.11 mmol), and the reaction was stirred for 0.5 h while maintaining the temperature. After the reaction completion was monitored by LCMS, the reaction solution was poured into a 0.5 N hydrochloric acid aqueous solution (20 mL). The solution was then extracted with ethyl acetate. The combined organic phases were washed with saturated brine, dried over anhydrous sodium sulfate, filtered, and concentrated to dryness. The crude product was purified by FCC ($SiO_2$, THF/PE=0-30%) to give a yellow solid intermediate **N24-OH** (420 mg, yield 59%). LCMS (m/z): 553.1 (M+H).

**Step F:** Intermediate **N24**

Under nitrogen protection at 0°C, DMF (11 mg, 0.15 mmol) was added to anhydrous oxalyl chloride (289 mg, 2.28 mmol) in dichloromethane (10 mL). After stirring and maintaining the temperature for 0.5 h, intermediate **N24-OH** (420 mg, 0.76 mmol) was added to the reaction solution, and the reaction continued at 0°C for 3 h. After the reaction completion was monitored by LCMS, the reaction solution was poured into 20 mL of saturated sodium bicarbonate solution and extracted with dichloromethane. The combined organic phases were washed with saturated brine, dried over anhydrous sodium sulfate, filtered, and concentrated to dryness. The crude product was purified by FCC ($SiO_2$, EA/PE=0-12%) to obtain a yellow solid intermediate **N24** (360 mg, yield 83%). LCMS (m/z): 571.0 (M+H).

## Intermediate N25-OH and N25

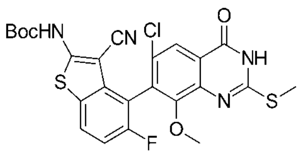

**N25-OH**

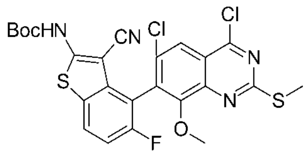

**N-25**

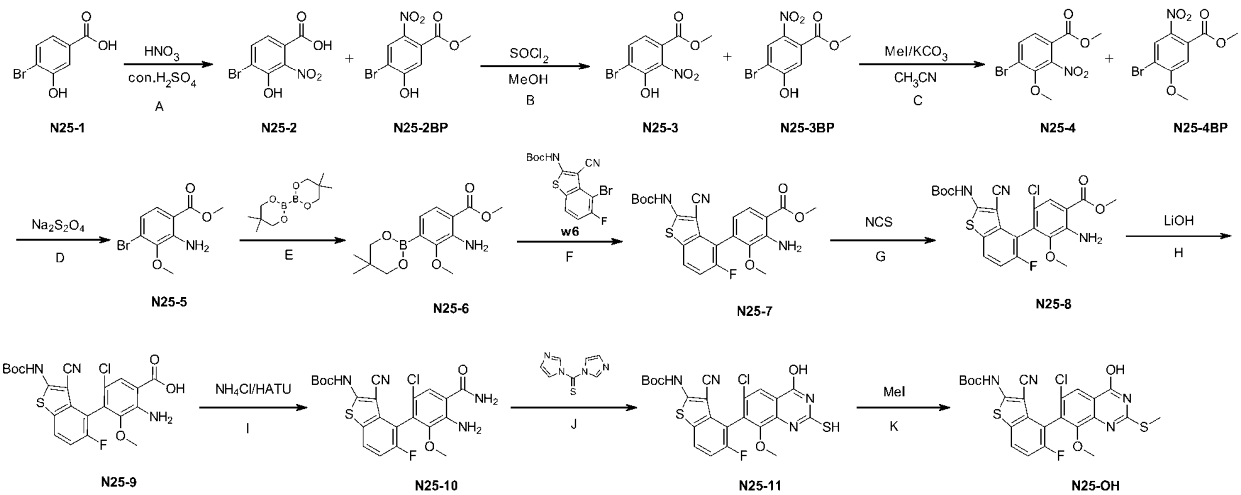


**Step A:** Compound **N25-2**

Compound **N25-1** (6.0 g, 27.6 mmol) was dissolved in concentrated sulfuric acid (138 mL) at room temperature and stirred for 30 min. Then, concentrated nitric acid (69 mL) was added to the reaction solution under ice-water bath conditions, and the mixture was stirred for 30 min while maintaining the temperature. The reaction solution was poured into ice water (1 L), extracted with EA (250 mL×3), and the organic phase was collected, washed with saturated brine (100 mL), and dried over anhydrous $Na_2SO_4$. The organic phase was concentrated to give a yellow solid compound **N25-2** (containing a portion of compound **N25-2BP**) (8.0 g).

**Step B:** Compound **N25-3**

Under ice-water bath conditions, $SOCl_2$ (9.08 mL, 0.77 mmol) was added to a mixed solution of compound **N25-2**

(8.0 g, crude product) and MeOH (110 mL). The mixture was heated to 70°C and stirred for 16 hours. The reaction completion was monitored by TLC. The reaction solution was concentrated, and the crude product was purified by FCC ($SiO_2$, EA/PE=5-30%) to give a yellow solid compound **N25-3** ($^1$H NMR showed 18% **N25-3BP)** (5.2 g, two-step yield 68%). $^1$H NMR (400 MHz, DMSO-$d_6$) δ 7.91 (d, *J* = 8.4 Hz, 1H), 7.43 (d, *J* = 8.4 Hz, 1H), 3.83 (s, 3H).

**Step C:** Compound **N25-4**

At room temperature, iodomethane (5.4 g, 37.8 mmol) was added to a mixed solution of compound **N25-3** (5.2 g, 18.9 mmol), $K_2CO_3$ (7.8 g, 56.7 mmol), and ACN (150 mL). The mixture was warmed to 50°C and stirred for 16 hours. The reaction completion was monitored by LCMS. The reaction solution was filtered and washed with EA (100 mL). The collected organic phase was concentrated, and the crude product was purified by FCC ($SiO_2$, DCM/PE=5-30%) to give a yellow solid compound **N25-4** (4.1 g, yield 75%) and a yellow solid **N25-4BP** (900mg, yield 16%).

**Step D:** Compound **N25-5**

At room temperature, $Na_2S_2O_4$ (2.4 g, 13.8 mmol) was dissolved in $H_2O$ (15 mL) and added to a mixed solution of compound **N25-4** (1.0 g, 3.46 mmol) and THF (15 mL). The mixture was stirred at room temperature for 16 hours, and the reaction completion was monitored by LCMS. The reaction solution was poured into $H_2O$ (20 mL), extracted with EA (20 mL×3), and the collected organic phase was washed with saturated NaCl aqueous solution (20 mL) and dried over anhydrous $Na_2SO_4$. The mixture was filtered, and the organic phase was concentrated to obtain a crude product, which was purified by FCC ($SiO_2$, EA/PE=5-30%) to give a yellow solid compound **N25-5** (500 mg, yield 56%). LCMS (m/z): 260.0 (M+H).

**Step E:** Compound **N25-6**

At room temperature, Pd(dppf)$Cl_2$ (141 mg, 0.2 mmol) was added to a mixed solution of compound **N25-5** (500 mg, 1.93 mmol), 2-(5,5-dimethyl-1,3,2-dioxaborolan-2-yl)-5,5-dimethyl-1,3,4-dioxaborborane (524 mg, 2.32 mmol), KOAc (284 mg, 2.90 mmol), and dioxane (10 mL). The mixture was substituted three times with a nitrogen balloon, heated to 90°C, and stirred for 2 hours. The reaction completion was monitored by LCMS. The reaction solution was poured into $H_2O$ (20 mL), extracted with EA (20 mL×3), and the collected organic phase was washed with saturated NaCl (20 mL) and dried over anhydrous $Na_2SO_4$. After filtration, the organic phase was concentrated to obtain a crude product, which was purified by FCC ($SiO_2$, EA/PE=5-30%) to give a yellow solid compound **N25-6** (350 mg, yield 62%). LCMS (m/z): 226.1 (M+H-68).

**Step F:** Compound **N25-7**

At room temperature, Pd-118 (77.9 mg, 0.12 mmol) was added to a mixed solution of compound **N25-6** (350 mg, 1.19 mmol), intermediate **w6** (530 mg, 1.43 mmol), $K_3PO_4$ (760 mg, 3.57 mmol) and dioxane (10 mL), $H_2O$ (3 mL). The mixture was substituted three times with a nitrogen balloon, heated to 90°C and stirred for 2 hours. The reaction completion was monitored by LCMS. The reaction solution was then poured into $H_2O$ (20 mL) and extracted with EA (20 mL×3). The collected organic phase was washed with saturated NaCl (20 mL) and dried over anhydrous $Na_2SO_4$. The organic phase was concentrated to obtain a crude product, which was purified by FCC ($SiO_2$, EA/PE=5-30%) to give a yellow solid compound **N25-7** (320 mg, yield 57%). LCMS (m/z): 472.1 (M+H).

**Step G:** Compound **N25-8**

At room temperature, NCS (109 mg, 0.82 mmol) was added to a mixed solution of compound **N25-7** (320 mg, 0.68 mmol) and DMF (3 mL). The mixture was stirred at room temperature for 16 hours. The reaction completion was monitored by LCMS. The reaction solution was poured into $H_2O$ (20 mL) and extracted with EA (10 mL×3). The collected organic phase was washed with saturated NaCl (20 mL) and dried over anhydrous $Na_2SO_4$. The organic phase was concentrated to obtain a crude product, which was purified by FCC ($SiO_2$, EA/PE=5-20%) to give a yellow oily compound **N25-8** (300 mg, yield 87%). LCMS (m/z): 506.0 (M+H).

**Step H:** Compound **N25-9**

At room temperature, LiOH (143 mg, 5.9 mmol) was added to a mixed solution of compound **N25-8** (300 mg, 0.59 mmol), MeOH (3 mL), $H_2O$ (3 mL), and THF (3 mL), stirred at room temperature for 16 hours. The reaction completion was monitored by LCMS. The reaction solution was poured into $H_2O$ (20 mL) and extracted with EA (10 mL×3). The collected

organic phase was washed with saturated NaCl (20 mL) and dried over anhydrous $Na_2SO_4$. The mixture was filtered, and the organic phase was concentrated to give a yellow oily compound **N25-9** (300 mg, crude). LCMS (m/z): 492.1 (M+H).

**Step I:** Compound **N25-10**

At room temperature, $NH_4Cl$ (163 mg, 3.05 mmol) was added to a mixed solution of compound **N25-9** (300 mg), DIEA (630 mg, 4.88 mmol), HATU (463 mg, 1.22 mmol), and DMF (5 mL). The mixture was stirred at room temperature for 3 hours. The reaction completion was monitored by LCMS. The reaction solution was poured into $H_2O$ (20 mL) and extracted with EA (10 mL×3). The collected organic phase was washed with saturated NaCl (20 mL) and dried over anhydrous $Na_2SO_4$. The organic phase was concentrated to obtain a crude product, which was separated by FCC ($SiO_2$, EA/PE=10-30%) to give a yellow solid compound **N25-10** (260 mg, two-step yield 87%). LCMS (m/z): 491.2 (M+H).

**Step J:** Compound **N25-11**

At room temperature, NaH (60wt%, 212mg, 3.18mmol) was added to a mixed solution of compound **N25-10** (260mg, 0.53mmol) and THF (5mL). The mixture was warmed to 40°C and stirred for 1 hour. TCDI (142mg, 0.80mmol) was added to the reaction mixture, and the mixture was warmed to 60°C and stirred for 2 hours. The reaction completion was monitored by LCMS. The pH was adjusted to ~4 with 1N diluted hydrochloric acid, and the mixture was extracted with EA (10mL×3). The collected organic phase was washed with saturated NaCl (20mL) and dried over anhydrous $Na_2SO_4$. The organic phase was concentrated to give a yellow oily compound **N25-11** (280mg, crude product). LCMS (m/z): 533.2 (M+H).

**Step K:** Compound **N25-OH**

Under ice-water bath conditions, NaOH (84.2 mg, 2.12 mmol) was added to a mixed solution of compound **N25-11** (280 mg, 0.53 mmol) and MeOH/$H_2O$ (3 mL/3 mL), then MeI (225 mg, 1.59 mmol) was added to the reaction solution, and the mixture was stirred at room temperature for 1 hour. The reaction completion was monitored by LCMS. The pH was adjusted to ~4 with 1N diluted hydrochloric acid, and the mixture was extracted with EA (10 mL×3). The collected organic phase was washed with saturated NaCl (20 mL) and dried over anhydrous $Na_2SO_4$. The organic phase was concentrated to obtain a crude product, which was purified by FCC ($SiO_2$, EA/PE=15-40%) to give a yellow solid compound **N25-OH** (160 mg, two-step yield 56%). LCMS (m/z): 547.1 (M+H).

The synthesis of intermediate **N25** was carried out by referring to the synthesis method of intermediate **N24** described above for intermediate **N25-OH.**

Following the above intermediate synthesis scheme, the following intermediates can be prepared:

| Number | Structural Formula | Number | Structural Formula |
|---|---|---|---|
| **Intermediate N26** | 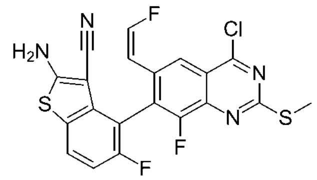 LCMS (m/z): 463.0 (M+H) | **Intermediate N27** | 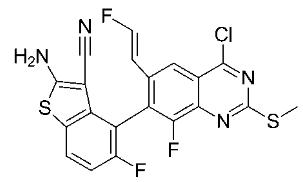 LCMS (m/z): 463.0 (M+H) |

**Example 1**

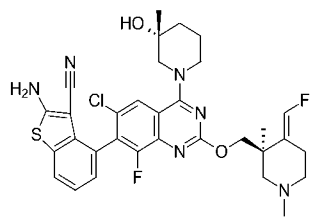

2-Amino-4-(6-chloro-8-fluoro-2-(((*S,E*)-4-(fluoromethylene)-1,3-dimethylpiperidine-3-yl)methoxy)-4-((*R*)-3-hydroxy-3-methylpiperidine-1-yl)quinazoline-7-yl)benzo[*b*]thiophene-3-carbonitrile

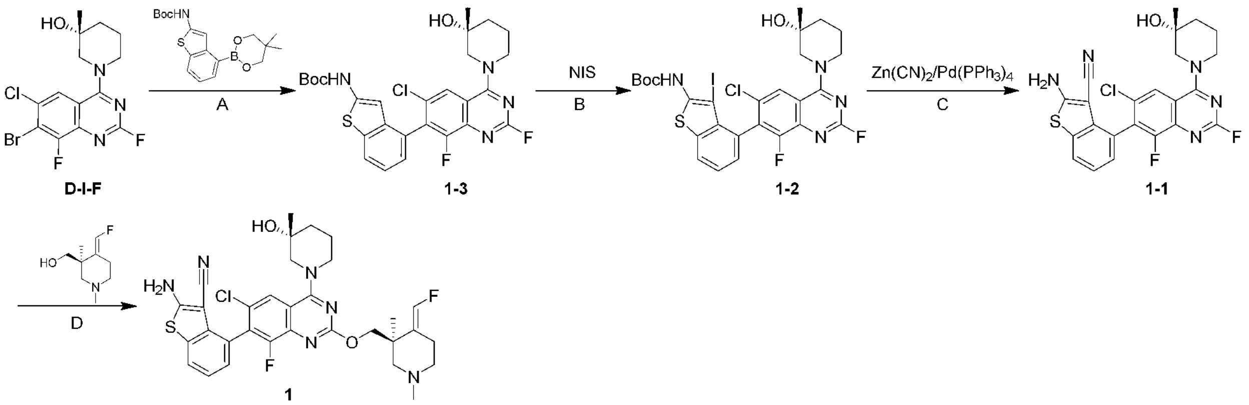


D-I-F 1-3 1-2 1-1

1

**Step A:** tert-Butyl (4-(6-chloro-2,8-difluoro-4-(*R*)-3-hydroxy-3-methylpiperidine-1-yl)quinazoline-7-yl)benzo[*b*]thiophene-2-yl)carbamate

At room temperature, a mixed solution of (*R*)-1-(7-bromo-6-chloro-2,8-difluoroquinazoline-4-yl)-3-methylpiperidine-3-ol (900 mg, 2.29 mmol), tert-butyl (4-(5,5-dimethyl-1,3,2-dioxaborolan-2-yl)benzo[b]thiophene-2-yl)carbamate (2.15 g, 5.96 mmol), $Pd(dppf)Cl_2$ (167 mg, 229 μmol), $K_3PO_4$ (1.49 g, 6.88 mmol), and 1,4-dioxane/$H_2O$ (V/V=4:1, 15 mL) was degassed three times with $N_2$ and heated to 110°C, and stirred for 16 h. The reaction completion was monitored by LCMS. After cooling, the reaction solution was poured into water (100 mL), extracted with EA (50 mL×3), and the collected organic phase was washed with saturated NaCl aqueous solution (20 mL). The crude product obtained by concentrating the organic liquid was purified by FCC ($SiO_2$, EtOH/EA=1:3)/PE=0-20%) to obtain a brown solid tert-butyl (4-(6-chloro-2,8-difluoro-4-(*R*)-3-hydroxy-3-methylpiperidine-1-yl)quinazoline-7-yl)benzo[*b*]thiophene-2-yl) carbamate (1.2 g, yield 93%). LCMS (m/z): 561.1 (M+H).

**Step B:** tert-butyl (4-(6-chloro-2,8-difluoro-4-((*R*)-3-hydroxy-3-methylpiperidine-1-yl)quinazoline-7-yl)-3-iodobenzo[*b*] thiophene-2-yl)carbamate

At room temperature, NIS (962 mg, 4.28 mmol) was added to a mixed solution of (4-(6-chloro-2,8-difluoro-4-(*R*)-3-hydroxy-3-methylpiperidine-1-yl)quinazoline-7-yl)benzo[*b*]thiophene-2-yl)carbamate (1.2 g, 2.14 mmol) and DMF (15 mL) and stirred for 2 h. The reaction completion was monitored by LCMS. The reaction solution was poured into water (100 mL) and extracted with EA (50 mL×3). The collected organic phase was washed with saturated NaCl aqueous solution (20 mL) and dried over anhydrous $Na_2SO_4$. The crude product obtained by concentration of the organic liquid was purified by FCC ($SiO_2$, EA/PE=30-100%) to obtain a yellow solid tert-butyl (4-(6-chloro-2,8-difluoro-4-((*R*)-3-hydroxy-3-methylpiperidine-1-yl)quinazoline-7-yl)-3-iodobenzo[*b*]thiophene-2-yl)carbamate (600 mg, yield 41%). LCMS (m/z): 687.0 (M+H).

**Step C:** 2-Amino-4-(6-chloro-2,8-difluoro-4-((*R*)-3-hydroxy-3-methylpiperidine-1-yl)quinazoline-7-yl)benzo[*b*]thiophene-3-carbonitrile

At room temperature, $Zn(CN)_2$ (1.03 g, 8.73 mmol) and $Pd(PPh_3)_4$ (90 mg, 87.3 μmol ) were added to a microwave tube containing a solution of tert-butyl (4-(6-chloro-2,8-difluoro-4-((*R*)-3-hydroxy-3-methylpiperidine-1-yl) quinazoline-7-yl)-3-iodobenzo[*b*]thiophene-2-yl)carbamate (600 mg, 873 μmol) in DMF(15 mL). The reaction solution was stirred in a microwave at 130°C for 2 h. The reaction completion was monitored by LCMS. The reaction solution was then poured into water (50 mL) and extracted by EA (50 mL×3). The organic phase was collected, washed with saturated NaCl aqueous solution (20 mL), dried over anhydrous $Na_2SO_4$, and concentrated. The organic phase was separated by FCC ($SiO_2$, EA/PE=20-60%) to obtain a yellow solid 2-amino-4-(6-chloro-2,8-difluoro-4-((*R*)-3-hydroxy-3-methylpiperidine-1-yl)quinazoline-7-yl)benzo[*b*]thiophene-3-carbonitrile (200 mg, yield 47%). LCMS (m/z): 486.1 (M+H).

**Step D:** 2-Amino-4-(6-chloro-8-fluoro-2-(((*S,E*)-4-(fluoromethylene)-1,3-dimethylpiperidine-3-yl)methoxy)-4-((*R*)-3-hydroxy-3-methylpiperidine-1-yl)quinazoline-7-yl)benzo[*b*]thiophene-3-carbonitrile

At 0°C, NaH (49 mg, 60 wt %, 1.23 mmol) was added to a THF (3 mL) solution containing (*S,E*)-(4-(fluoromethylene)-1,3-dimethylpiperidine-3-yl)methanol (71 mg, 412 μmol) and stirred for 30 minutes. Then, 2-amino-4-(6-chloro-2,8-difluoro-4-((*R*)-3-hydroxy-3-methylpiperidine-1-yl)quinazoline-7-yl)benzo[*b*]thiophene-3-carbonitrile (100 mg, 206 μmol) was added to the above reaction solution in one portion and stirred at 25°C for 1 h. The reaction completion was monitored by LCMS. The reaction solution was poured into a semi-saturated $NH_4Cl$ aqueous solution (20 mL), extracted with EA (20 mL×3), and the organic phase was collected. The organic phase was washed with a saturated NaCl aqueous solution (20 mL), dried over anhydrous $Na_2SO_4$, and concentrated. The organic phase was purified by Pre-HPLC (ACN/(10 mM $NH_4HCO_3$)=55-75%) to obtain a yellow solid 2-amino-4-(6-chloro-8-fluoro-2-(((*S,E*)-4-(fluoromethylene)-1,3-dimethylpiperidine-3-yl)methoxy)-4-((*R*)-3-hydroxy-3-methylpiperidine-1-yl)quinazoline-7-yl)benzo[b]thiophene-3-carbonitrile (6 mg, yield 5%). LCMS (m/z): 639.2 (M+H). $^1$H NMR (400 MHz, Methanol-$d_4$ ) δ 8.05 (d, *J* = 1.7 Hz, 1H), 7.69 - 7.63 (m, 1H), 7.27 - 7.22 (m, 1H), 7.18 - 7.14 (m, 1H), 6.78 - 6.54 (m, 1H), 4.56 - 4.51 (m, 1H), 4.46 - 4.40 (m, 1H), 4.25 - 4.16 (m, 1H), 4.06 - 3.99 (m, 1H), 3.57 - 3.50 (m, 1H), 3.50 - 3.39 (m, 1H), 2.93 - 2.84 (m, 1H), 2.81 - 2.71 (m, 1H), 2.69 - 2.61 (m, 1H), 2.38 - 2.22 (m, 4H), 2.20 - 1.98 (m, 3H), 1.87 - 1.71 (m, 3H), 1.26 (s, 3H), 1.21 (s, 3H). $^{19}$F NMR (376 MHz, Methanol-$d_4$) δ -123.94, -139.66.

## Example 2

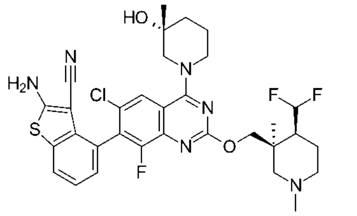

2-Amino-4-(6-chloro-2-(((3*S*,4*S*)-4-(difluoromethyl)-1,3-dimethylpiperidine-3-yl)methoxy)-8-fluoro-4-((*R*)-3-hydroxy-3-methylpiperidine-1-yl)quinazoline-7-yl)benzo[b]thiophene-3-carbonitrile

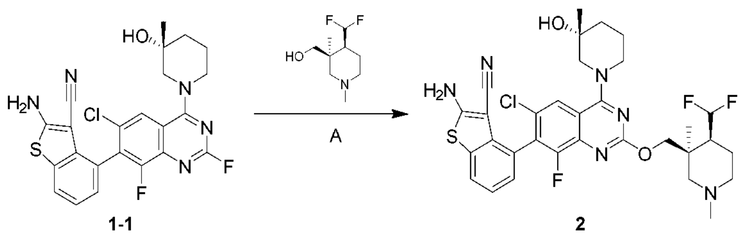


**1-1** **2**

**Step A:** 2-Amino-4-(6-chloro-2-(((3*S*,4*S*)-4-(difluoromethyl)-1,3-dimethylpiperidine-3-yl)methoxy)-8-fluoro-4-((*R*)-3-hydroxy-3-methylpiperidine-1-yl)quinazoline-7-yl)benzo[*b*]thiophene-3-carbonitrile

At 0°C, NaH (49 mg, 60 wt%, 1.23 mmol) was added to a mixed solution of ((3*S*,4*S*)-4-(difluoromethyl)-1,3-dimethylpiperidine-3-yl)methanol (79 mg, 412 μmol) and THF (3 mL) and stirred for 30 minutes. Then, 2-amino-4-(6-chloro-2,8-difluoro-4-((*R*)-3-hydroxy-3-methylpiperidine-1-yl)quinazoline-7-yl) benzo[*b*]thiophene-3-carbonitrile (100 mg, 206 μmol) was added to the above reaction solution in one portion and stirred at 25°C for 1 h. The reaction completion was monitored by LCMS. The reaction solution was poured into a semi-saturated $NH_4Cl$ aqueous solution (20 mL), extracted with EA (20 mL×3), and the organic phase was collected. The organic phase was washed with a saturated NaCl aqueous solution (20 mL), dried over anhydrous $Na_2SO_4$, and concentrated. The organic phase was purified by Pre-HPLC (ACN/(10 mM $NH_4HCO_3$)=55-75%) to obtain a yellow solid 2-amino-4-(6-chloro-2-(((3*S*,4*S*)-4-(difluoromethyl)-1,3-dimethylpiperidine-3-yl)methoxy)-8-fluoro-4-((*R*)-3-hydroxy-3-methylpiperidine-1-yl)quinazoline-7-yl)benzo[b]thiophene-3-carbonitrile (2 mg, 3.1 μmol, yield 2%). LCMS (m/z): 659.2 (M+H). $^1$H NMR (400 MHz, Methanol-$d_4$ ) δ 8.05 (d, *J* = 1.7 Hz, 1H), 7.68 - 7.65 (m, 1H), 7.27 - 7.22 (m, 1H), 7.18 - 7.14 (m, 1H), 6.38 - 6.04 (m, 1H), 4.53 - 4.47 (m, 1H), 4.27 - 4.17 (m, 1H), 4.08 - 4.01 (m, 1H), 3.58 - 3.51 (m, 1H), 3.49 - 3.41 (m, 1H), 3.14 - 3.04 (m, 1H), 2.99 - 2.91 (m, 1H), 2.23 (s, 3H), 2.19 - 2.09 (m, 1H), 2.06 - 1.94 (m, 2H), 1.87 - 1.69 (m, 7H), 1.25 (s, 3H), 1.21 (s, 3H). $^{19}$F NMR (376 MHz, Methanol- $d_4$ ) δ -118.49, -121.55, -123.98.

**Examples 3A and 3B**

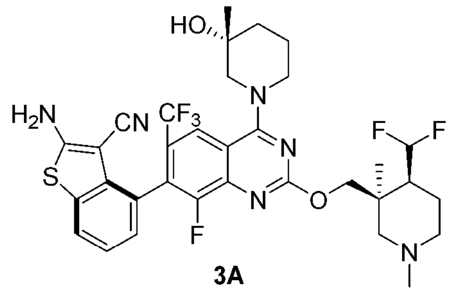

3A

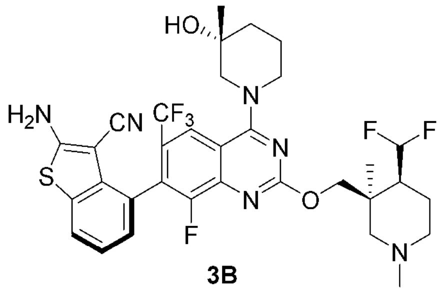

3B

tert-Butyl (3-cyano-4-(2,8-difluoro-4-((*R*)-3-hydroxy-3-methylpiperidine-1-yl)-6-(trifluoromethyl)quinazoline-7-yl)benzo[*b*]thiophene-2-yl)carbamate

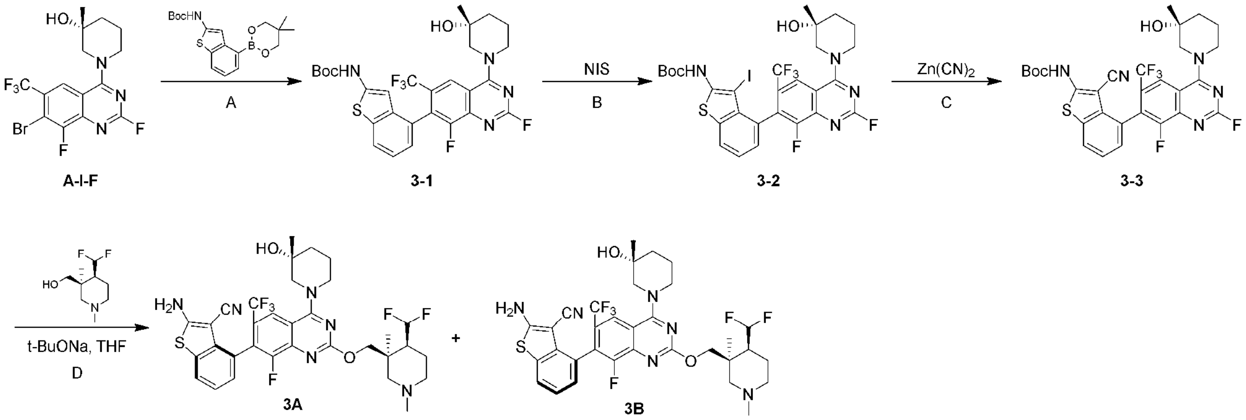


**Step A:** tert-Butyl (3-cyano-4-(2,8-difluoro-4-((*R*)-3-hydroxy-3-methylpiperidine-1-yl)-6-(trifluoromethyl)quinazoline-7-yl)benzo[*b*]thiophene-2-yl)carbamate

At room temperature, a mixed solution of tert-butyl (*R*)-1-(7-bromo-2,8-difluoro-6-(trifluoromethyl)quinazoline-4-yl)-3-methylpiperidine-3-ol (1 g, 2.35 mmol), [4-(5,5-dimethyl-1,3,2-dioxaborolan-2-yl)-1-benzothiophene-2-yl]carbamate (1.52 g, 3.52 mmol), Pd-117 (170 mg, 234 μmol), $Cs_2CO_3$ (2.3 g, 7.04 mmol), and 1,4-dioxane/$H_2O$ (20 mL, V/V=4:1) was degassed three times with $N_2$ and warmed to 80°C with stirring for 4 h. The reaction completion was monitored by LCMS. After cooling to room temperature, the reaction solution was poured into water (100 mL), extracted with EA (50 mL×3), and the collected organic phase was washed with saturated NaCl aqueous solution (20 mL). The organic solution was concentrated and purified by FCC ($SiO_2$, (EtOH/EA=1/3)/PE= 0 ~ 20%) to obtain a brown solid tert-butyl (3-cyano-4-(2,8-difluoro-4-((*R*)-3-hydroxy-3-methylpiperidine-1-yl)-6-(trifluoromethyl)quinazoline-7-yl)benzo[*b*]thiophene-2-yl) carbamate (970 mg, yield 69.3%). LCMS (m/z): 595.1 (M+H).

**Step B:** tert-Butyl (4-(2,8-difluoro-4-((*R*)-3-hydroxy-3-methylpiperidine-1-yl)-6-(trifluoromethyl)quinazoline-7-yl)-3-iodobenzo[*b*]thiophene-2-yl)carbamate

At room temperature, *N*-iodosuccinimide (440 mg, 1.96 mmol) was added to a mixture of tert-butyl (3-cyano-4-(2,8-difluoro-4-((*R*)-3-hydroxy-3-methylpiperidine-1-yl)-6-(trifluoromethyl)quinazoline-7-yl)benzo[*b*]thiophene-2-yl)carbamate (970 mg, 1.63 mmol) and DMF (8 mL) and stirred for 1 h. The reaction completion was monitored by LCMS. The reaction solution was poured into water (100 mL), and a gray solid precipitated out. The mixture was filtered, and the filter cake was washed with $H_2O$(100 mL). The filter cake was dissolved in EA (50 mL). The collected organic phase was washed with saturated NaCl (20 mL), dried over anhydrous $Na_2SO_4$, filtered, and concentrated to obtain a brown solid (1.07 g, yield 90.7%) tert-butyl 4-(2,8-difluoro-4-((*R*)-3-hydroxy-3-methylpiperidine-1-yl)-6-(trifluoromethyl)quinazoline-7-yl)-3-iodobenzo[*b*]thiophene-2-yl)carbamate. LCMS (m/z): 721.0 (M+H).

**Step C:** tert-butyl (3-cyano-4-(2,8-difluoro-4-((*R*)-3-hydroxy-3-methylpiperidine-1-yl)-6-(trifluoromethyl)quinazoline-7-yl)benzo[*b*]thiophene-2-yl)carbamate

At room temperature, $Zn(CN)_2$ (1.69 g, 14.4 mmol) and $Pd(PPh_3)_4$ (167 mg, 144 μmol) were added to a mixed solution of tert-butyl (4-(2,8-difluoro-4-((R)-3-hydroxy-3-methylpiperidin-1-yl)-6-(trifluoromethyl)quinazolin-7-yl)-3-iodobenzo[b]thiophen-2-yl)carbamate (1.04 g, 1.44 mmol) and DMF (15 mL). The reaction mixture was stirred at 105°C for 16 h, and the reaction completion was monitored by LCMS. The reaction mixture was then poured into water (50 mL) and extracted with EA (50 mL×3). The organic phase was collected and washed with saturated NaCl (20 mL) solution, dried with anhydrous $Na_2SO_4$, filtered, concentrated to obtain a crude product, which was purified by FCC ($SiO_2$, EA/PE=20-60%) to yield a yellow solid tert-butyl (3-cyano-4-(2,8-difluoro-4-((*R*)-3-hydroxy-3-methylpiperidine-1-yl)-6-(trifluoromethyl)quinazoline-7-yl)benzo[*b*]thiophene-2-yl)carbamate (410 mg, yield 54.7%). LCMS (m/z): 520.0 (M+H).

**Step D:** (*R*)-2-amino-4-(2-(((3*S*,4*S*)-4-(difluoromethyl)-1,3-dimethylpiperidine-3-yl)methoxy)-8-fluoro-4-((*R*)-3-hydroxy-3-methylpiperidine-1-yl)-6-(trifluoromethyl)quinazoline-7-yl)benzo[*b*]thiophene-3-carbonitrile (3A) and (*S*)-2-amino-4-(2-(((3*S*,4*S*)-4-(difluoromethyl)-1,3-dimethylpiperidine-3-yl)methoxy)-8-fluoro-4-((*R*)-3-hydroxy-3-methylpiperidine-1-yl)-6-(trifluoromethyl)quinazoline-7-yl)benzo[*b*]thiophene-3-carbonitrile (3B)

At -78°C, *t*-BuONa ( 4.9 mg, 2 M THF solution, 1.23 mmol) was added to a solution of ((3*S*,4*S*)-4-(difluoromethyl)-1,3-dimethylpiperidine-3-yl)methanol (7.9 mg, 412 μmol) and tert-butyl (3-cyano-4-(2,8-difluoro-4-((*R*)-3-hydroxy-3-methylpiperidine-1-yl)-6-(trifluoromethyl)quinazoline-7-yl)benzo[*b*]thiophene-2-yl)carbamate (120 mg, 206 μmol) in THF ( 5 mL) and stirred for 1 h. The reaction completion was monitored by LCMS. The reaction solution was poured into a semi-saturated $NH_4Cl$ (2.0 mL) and extracted by EA (2.0 mL × 3). The organic phase was collected and washed with saturated NaCl (20 mL) solution, dried with anhydrous $Na_2SO_4$, filtered, concentrated, and then separated by Pre-HPLC (ACN/(10 mM $NH_4HCO_3/H_2O$) 60%-80%) to obtain the first eluted yellow solid isomer 1, which was **Example 3A** (24 mg, retention time 11.31 min). LCMS (m/z): 693.3(M+H). $^1H$ NMR (400 MHz, $CD_3OD$) δ 8.39 (s, 1H), 7.72 - 7.62 (m, 1H), 7.28 - 7.08 (m, 2H), 6.46 - 5.83 (m, 1H), 4.66 - 4.56 (m, 1H), 4.55 - 4.48 (m, 1H), 4.41 - 4.29 (m, 1H), 4.19 - 4.02 (m, 1H), 3.65 - 3.52 (m, 1H), 3.51 - 3.35 (m, 1H), 3.07 (d, *J* = 11.8 Hz, 1H), 3.00 - 2.88 (m, 1H), 2.22 (s, 3H), 2.19 - 2.09 (m, 1H), 2.04 - 1.92 (m, 1H), 1.88 - 1.70 (m, 7H), 1.26 (s, 3H), 1.21 (s, 3H). $^{19}F$ NMR (376 MHz, $CD_3OD$) δ -59.78, -118.58, -121.62, -125.04. The subsequently eluted yellow solid isomer 2 was **Example 3B** (22 mg, retention time 13.0 min). LCMS (m/z): 693.3(M+H). $^1H$ NMR (400 MHz, $CD_3OD$) δ 8.33 (s, 1H), 7.75 - 7.59 (m, 1H), 7.30 - 7.11 (m, 2H), 6.42 - 5.99 (m, 1H), 4.66 - 4.47 (m, 2H), 4.44 - 4.26 (m, 1H), 4.18 - 4.02 (m, 1H), 3.64 - 3.53 (m, 1H), 3.51 - 3.36 (m, 1H), 3.17 - 3.03 (m, 1H), 3.02 - 2.87 (m, 1H), 2.22 (s, 3H), 2.20 - 2.09 (m, 1H), 2.08 - 1.91 (m, 1H), 1.88 - 1.66 (m, 7H), 1.26 (s, 3H), 1.21 (s, 3H). $^{19}F$ NMR (376 MHz, $CD_3OD$) δ -59.85, -118.70, - 121.55, -125.21.

**Examples 4A and 4B**

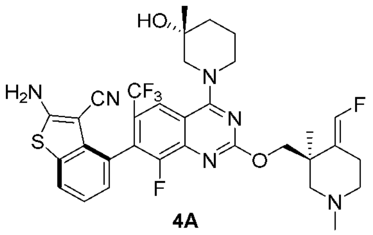

4A

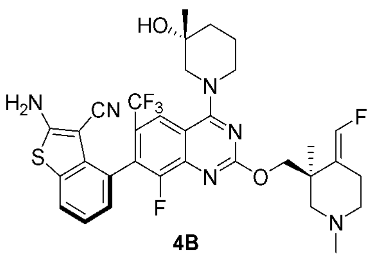

4B

(*R*)-2-amino-4-(8-fluoro-2-(((S,E)-4-(fluoromethylene)-1,3-dimethylpiperidine-3-yl)methoxy)-4-((*R*)-3-hydroxy-3-methylpiperidine-1-yl)-6-(trifluoromethyl)quinazoline-7-yl)benzo[b]thiophene-3-carbonitrile (**4A**) and (*R*)-2-amino-4-(8-fluoro-2-(((*S,E*)-4-(fluoromethylene)-1,3-dimethylpiperidine-3-yl)methoxy)-4-((*R*)-3-hydroxy-3-methylpiperidine-1-yl)-6-(trifluoromethyl)quinazoline-7-yl)benzo[*b*]thiophene-3-carbonitrile (**4B**)

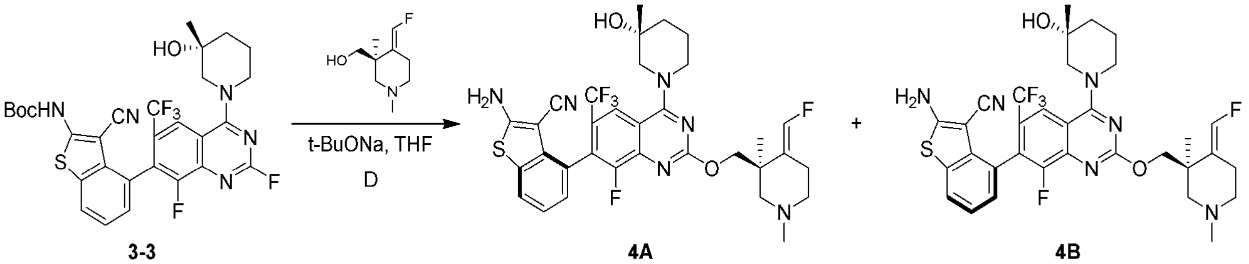


3-3 4A 4B

**Step A:** (*R*)-2-amino-4-(8-fluoro-2-(((*S,E*)-4-(fluoromethylene)-1,3-dimethylpiperidine-3-yl)methoxy)-4-((*R*)-3-hydroxy-3-methylpiperidine-1-yl)-6-(trifluoromethyl)quinazoline-7-yl)benzo[*b*]thiophene-3-carbonitrile (4A) and (*R*)-2-amino-4-(8-fluoro-2-(((*S,E*)-4-(fluoromethylene)-1,3-dimethylpiperidine-3-yl)methoxy)-4-((*R*)-3-hydroxy-3-methylpiperidine-1-yl)-6-(trifluoromethyl)quinazoline-7-yl)benzo[*b*]thiophene-3-carbonitrile (4B)

At -78°C, t-BuONa (49 mg, 2 M THF solution, 1.23 mmol) was added to a solution of (*S,E*)-(4-(fluoromethylene)-1,3-dimethylpiperidine-3-yl)methanol (60 mg, 412 μmol) and tert-butyl (3-cyano-4-(2,8-difluoro-4-((*R*)-3-hydroxy-3-methylpiperidine-1-yl)-6-(trifluoromethyl)quinazoline-7-yl)benzo[*b*]thiophene-2-yl)carbamate (120 mg, 206 μmol ) in THF (5 mL) and stirred for 1 h. The reaction completion was monitored by LCMS, and the reaction solution was poured into a semi-saturated $NH_4Cl$ solution (20 mL) and extracted by EA (20 mL×3). The organic phase was collected, washed with saturated NaCl (20 mL) solution, dried over anhydrous $Na_2SO_4$, filtered, concentrated, and then separated by Pre-HPLC (ACN/(10 mM $NH_4HCO_3/H_2O$)=60%-80%). The first eluted yellow solid isomer 1 was obtained as **Example 4A** (31 mg, retention time 11.8 min). LCMS (m/z): 673.3 (M+H). $^1$H NMR (400 MHz, $CD_3OD$ ) δ 8.40 (s, 1H), 7.74 - 7.62 (m, 1H), 7.30 - 7.14 (m, 2H), 6.86 - 6.49 (m, 1H), 4.64 - 4.53 (m, 1H), 4.49 - 4.38 (m, 1H), 4.37 - 4.23 (m, 1H), 4.18 - 4.00 (m, 1H), 3.62 - 3.52 (m, 1H), 3.50 - 3.38 (m, 1H), 2.90 - 2.83 (m, 1H), 2.80 - 2.71 (m, 1H), 2.69 - 2.59 (m, 1H), 2.37 - 2.26 (m, 1H), 2.24 (s, 3H), 2.19 - 2.09 (m, 1H), 2.08 - 2.00 (m, 1H), 1.99 - 1.92 (m, 1H), 1.88 - 1.71 (m, 3H), 1.27 (s, 3H), 1.20 (s, 3H). $^{19}$F NMR (376 MHz, $CD_3OD$) δ -59.78, -124.97, - 139.64. The subsequently eluted yellow solid isomer 2 was **Example 4B** (29 mg, retention time 13.6 min). LCMS (m/z): 673.3 (M+H). $^1$H NMR (400 MHz, $CD_3OD$) δ 8.34 (s, 1H), 7.73 - 7.61 (m, 1H), 7.32 - 7.15 (m, 2H), 6.85 - 6.48 (m, 1H), 4.68 - 4.54 (m, 1H), 4.49 - 4.39 (m, 1H), 4.38 - 4.25 (m, 1H), 4.16 - 3.99 (m, 1H), 3.61 - 3.50 (m, 1H), 3.47 - 3.36 (m, 1H), 2.92 - 2.83 (m, 1H), 2.80 - 2.71 (m, 1H), 2.69 - 2.60 (m, 1H), 2.39 - 2.10 (m, 5H), 2.07 - 1.90 (m, 2H), 1.88 - 1.69 (m, 3H), 1.27 (s, 3H), 1.20 (s, 3H). $^{19}$F NMR (376 MHz, $CD_3OD$) δ -59.83, -125.08, -139.62.

Following the synthetic method of the above-mentioned compounds, the following examples were also prepared and characterized:

## Examples 5, 5A, and 5B

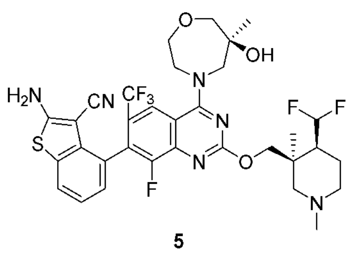
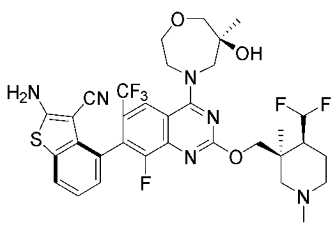
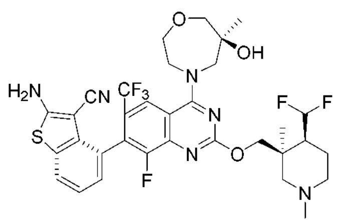

5 5A 5B

2-Amino-4-(2-((3*S*,4*S*)-4-(difluoromethyl)-1,3-dimethylpiperidine-3-yl)methoxy)-8-fluoro-4-((*S*)-6-hydroxy-6-methyl-1,4-oxazol-4-yl)-6-(trifluoromethyl)quinazoline-7-yl)benzothiophene-3-carbonitrile (**Example 5**), (*S*)-2-amino-4-(2-(((3*S*,4*S*)-4-(difluoromethyl)-1,3-dimethylpiperidine-3-yl)methoxy)-8-fluoro-4-((*S*)-6-hydroxy-6-methyl- 1,4-oxazol-4-yl)-6-(trifluoromethyl)quinazoline-7-yl)benzo[*b*]thiophene-3-carbonitrile (**Example 5A**), and (*R*)-2-amino-4-(2-(((3*S*,4*S*)-4-(difluoromethyl)-1,3-dimethylpiperidine-3-yl)methoxy)-8-fluoro-4-((*S*)-6-hydroxy-6-methyl-1,4-oxazol-4-yl)-6-(trifluoromethyl)quinazoline-7-yl)benzo[*b*]thiophene-3-carbonitrile (**Example 5B**)

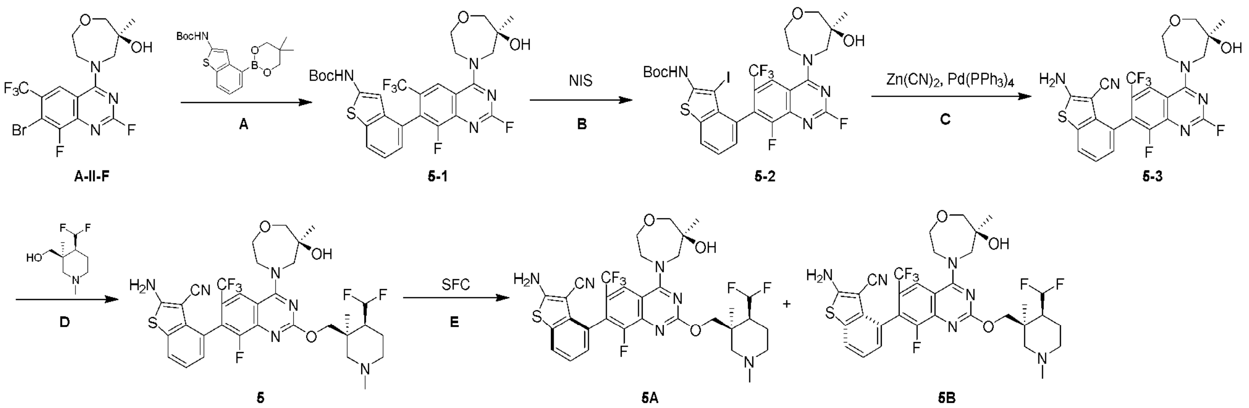


**Step A:** tert-Butyl (4-(2,8-difluoro-4-((*S*)-6-hydroxy-6-methyl-1,4-oxazol-4-yl)-6-(trifluoromethyl)quinazoline-7-yl)benzothiophene-2-yl)carbamate

At room temperature, (*S*)-4-(7-bromo-2,8-difluoro-6-(trifluoromethyl)quinazoline-4-yl)-6-methyl-1,4-oxazol-6-ol (1.00 g, 2.26 mmol), tert-butyl (4-(5,5-dimethyl-1,3,2-dioxaborahexane-2-yl)benzothiophene-2-yl)carbamate (1.63 g, 4.52 mmol), Pd-117 (162 mg, 0.0226 μmol), and $Cs_2CO_3$ (2.21 g, 6.78 mmol) were added sequentially to a solution of 1,4-dioxane (15 mL) and water (3 mL). After $N_2$ replacement three times, the solution was warmed to 80°C and stirred for 3 h. The reaction completion was monitored by LCMS and TLC. The reaction solution was brought to room temperature, and the palladium catalyst was removed by diatomite filtration. The mother liquor was collected, and water (100 mL) was added. Extraction was performed by EA (100 mL×3). The collected organic phase was washed with saturated brine (50 mL). The crude product obtained by concentrating the organic liquid was purified by FCC ($SiO_2$, EA/PE=0-40%) to obtain a yellow solid tert-butyl (4-(2,8-difluoro-4-((*S*)-6-hydroxy-6-methyl-1,4-oxazol-4-yl)-6-(trifluoromethyl)quinazoline-7-yl) benzothiophene-2-yl)carbamate (1.1 g, yield 80%). LCMS (m/z): 611.1 (M+H).

**Step B:** tert-Butyl (4-(2,8-difluoro-4-((*S*)-6-hydroxy-6-methyl-1,4-oxazol-4-yl)-6-(trifluoromethyl)quinazoline-7-yl)-3-iodobenzothiophene-2-yl)carbamate

At room temperature, NIS (407 mg, 1.81 mmol) was added to a mixed solution of tert-butyl (4-(2,8-difluoro-4-((*S*)-6-hydroxy-6-methyl-1,4-oxazol-4-yl)-6-(trifluoromethyl)quinazoline-7-yl)benzothiophene-2-yl)carbamate (920 mg, 1.51 mmol) and DMF (10 mL) and stirred for 0.5 h. After the reaction completion was monitored by LCMS, the reaction solution was poured into $H_2O$ (200 mL), flocculent matter was formed, filtered, and the filter cake was collected. The filter cake was then dissolved with EA (100 mL), extracted and washed with sodium thiosulfate aqueous solution (20 mL). The collected organic phase was washed with saturated brine (30 mL), dried over anhydrous $Na_2SO_4$, and concentrated to obtain a yellow solid tert-butyl (4-(2,8-difluoro-4-((*S*)-6-hydroxy-6-methyl-1,4-oxazol-4-yl)-6-(trifluoromethyl)quinazoline-7-yl)-3-iodobenzothiophene-2-yl)carbamate (940 mg, yield 85%). LCMS (m/z): 737.0 (M+H).

**Step C:** 2-Amino-4-(2,8-difluoro-4-((*S*)-6-hydroxy-6-methyl-1,4-oxazol-4-yl)-6-(trifluoromethyl)quinazoline-7-yl)benzothiophene-3-carbonitrile

At room temperature, tert-butyl (4-(2,8-difluoro-4-((*S*)-6-hydroxy-6-methyl-1,4-oxazol-4-yl)-6-(trifluoromethyl) quinazolin-7-yl)-3-iodobenzothiophen-2-yl)carbamate (880 mg, 1.19 mmol), $Zn(CN)_2$(701 mg, 5.97 mmol), and $Pd(PPh_3)_4$ (138 mg, 0.120 mmol) were added sequentially to DMF (15 mL). After $N_2$ replacement three times, the mixture was warmed to 105°C and stirred overnight. The reaction completion was monitored by LCMS and TLC. The reaction solution was brought to room temperature and then poured into $H_2O$ (200 mL). Flocculent matter was formed, filtered, and the filter cake was collected. The filter cake was then dissolved with EA (100 mL), and the organic liquid was concentrated. The crude product was purified by FCC ($SiO_2$, EA/PE=0-60%) to obtain a yellow solid 2-amino-4-(2,8-difluoro-4-((*S*)-6-hydroxy-6-methyl-1,4-oxazol-4-yl)-6-(trifluoromethyl)quinazoline-7-yl) benzothiophene-3-carbonitrile (400 mg, yield 63%). LCMS (m/z): 611.1 (M+H).

**Step D:** 2-Amino-4-(2-((3*S*,4*S*)-4-(difluoromethyl)-1,3-dimethylpiperidine-3-yl)methoxy)-8-fluoro-4-((*S*)-6-hydroxy-6-methyl-1,4-oxazol-4-yl)-6-(trifluoromethyl)quinazoline-7-yl)benzothiophene-3-carbonitrile (**Example 5**)

Under dry ice and ethanol bath conditions, *t*BuONa (0.7 mL, 2 M THF solution, 1.4 mmol) was added to a mixed solution of 2-amino-4-(2,8-difluoro-4-((*S*)-6-hydroxy-6-methyl-1,4-oxazol-4-yl)-6-(trifluoromethyl)quinazoline-7-yl)benzothiophene-3-carbonitrile (150 mg, 0.28 mmol), ((3S,4S)-4-(difluoromethyl)-1,3-dimethylpiperidine-3-yl)methanol (81 mg, 0.42 mmol ) and THF (10 mL) and stirred for 1.5 h. After the reaction completion was monitored by TLC and brought to room temperature, the solution was poured into a semi-saturated $NH_4Cl$ (30 mL) solution and extracted with EA (50 mL×3). The organic phase was collected, washed with saturated brine (30 mL), dried over anhydrous $Na_2SO_4$, and concentrated. The organic phase was then purified by FCC ($SiO_2$, (EA/EtOH=3/1)/PE=0-40%). The crude product was then purified by pre-HPLC (C18, ACN/(10mmol $NH_4HCO_3/H_2O$)=55-75%) to obtain a white solid 2-amino-4-(2-((3*S*,4*S*)-4-(difluoromethyl)-1,3-dimethylpiperidine-3-yl)methoxy)-8-fluoro-4-((*S*)-6-hydroxy-6-methyl-1,4-oxazol-4-yl)-6-(trifluoromethyl)quinazoline-7-yl)benzothiophene-3-carbonitrile (**Example 5**, 36 mg, yield 18%). LCMS (m/z): 709.3 (M+H). $^1$H NMR (400 MHz, DMSO-*d*$_6$) δ 9.03 - 8.81 (m, 1H), 7.99 - 7.71 (m, 3H), 7.31 - 7.11 (m, 2H), 6.52 - 6.10 (m, 1H), 5.48 - 5.25 (m, 1H), 4.45 (s, 2H), 4.36 - 4.21 (m, 1H), 4.20 - 4.00 (m, 2H), 3.99 - 3.82 (m, 2H), 3.79 - 3.67 (m, 1H), 3.63 - 3.53 (m, 2H), 2.88 - 2.76 (m, 2H), 2.20 - 2.05 (m, 3H), 1.91 - 1.52 (m, 5H), 1.19 - 1.12 (m, 3H), 1.11 (s, 3H). $^{19}$F NMR (376 MHz, DMSO-*d*$_6$) δ -56.79, -114.37 - -117.55 (m), -117.84 - -119.97 (m), -123.82.

**Step E:** (*S*)-2-amino-4-(2-(((3S,4S)-4-(difluoromethyl)-1,3-dimethylpiperidine-3-yl)methoxy)-8-fluoro-4-((*S*)-6-hydroxy-6-methyl-1,4-oxazol-4-yl)-6-(trifluoromethyl)quinazoline-7-yl)benzo[*b*]thiophene-3-carbonitrile (**Example 5A**) and (*R*)-2-amino-4-(2-(((3*S*,4*S*)-4-(difluoromethyl)-1,3-dimethylpiperidine-3-yl)methoxy)-8-fluoro-4-((*S*)-6-hydroxy-6-methyl-1,4-oxazol-4-yl)-6-(trifluoromethyl)quinazoline-7-yl)benzo[*b*]thiophene-3-carbonitrile (**Example 5B**)

The compound 2-amino-4-(2-((3*S*,4*S*)-4-(difluoromethyl)-1,3-dimethylpiperidine-3-yl)methoxy)-8-fluoro-4-((*S*)-6-hydroxy-6-methyl-1,4-oxazol-4-yl)-6-(trifluoromethyl)quinazoline-7-yl)benzothiophene-3-carbonitrile (30 mg) obtained from step D was resolved by SFC (Waters SFC 150, column: DAICELCHIRALCEL®IC 250*30 mm 10 μm; mobile phase $CO_2$/MeOH (+0.3% 7.0 mol/l $NH_3$-MeOH) = 82/1; flow rate: 130 mL/min) to yield the first eluted isomer 1, which was Example **5A** (7.6 mg, yield 25%, relatively short retention time). Chiral analysis method SFC-5, Rt = 2.223 min. LCMS (m/z): 709.3 (M+H). $^1$H NM R (400 MHz, $CDCl_3$) δ 8.28 (s, 1H), 7.63 (d, *J* = 7.7 Hz, 1H), 7.32 - 7.27 (m, 1H), 7.25 - 7.23 (m, 1H), 6.29 - 5.90 (m, 1H), 5.73 (s, 1H), 5.21 (s, 2H), 4.68 - 4.46 (m, 3H), 4.29 (d, *J* = 14.8 Hz, 1H), 3.98 - 3.86 (m, 1H), 3.83 - 3.73 (m, 2H), 3.66 (dd, *J* = 25.4, 13.7 Hz, 2H), 3.47 (d, *J* = 12.5 Hz, 1H), 3.01 - 2.81 (m, 2H), 2.18 (s, 3H), 1.93 - 1.80 (m, 2H), 1.77 - 1.67 (m, 3H), 1.36 (s, 3H), 1.18 (s, 3H). $^{19}$F NMR (376 MHz, $CDCl_3$) δ -58.60, -116.36, -119.40, -121.44. The subsequently eluted isomer 2 was Example **5B** (15 mg, yield 51%, relatively long retention time). Chiral analysis method SFC-5, Rt = 2.809 min. LCMS (m/z): 709.3 (M+H). $^1$H NM R (400 MHz, DMSO-*d*$_6$) δ 8.96 (s, 1H), 7.87 - 7.70 (m, 3H), 7.26 - 7.17 (m, 1H), 7.17 - 7.10 (m, 1H), 6.48 - 6.09 (m, 1H), 5.36 (s, 1H), 4.45 (s, 2H), 4.35 - 4.22 (m, 1H), 4.16 (d, *J* = 14.9 Hz, 1H), 4.08 - 3.99 (m, 1H), 3.98 - 3.89 (m, 1H), 3.88 - 3.82 (m, 1H), 3.78 - 3.65 (m, 1H), 3.63 - 3.50 (m, 2H), 2.91 - 2.76 (m, 2H), 2.11 (s, 3H), 1.87 - 1.55 (m, 5H), 1.16 (s, 3H), 1.11 (s, 3H). $^{19}$F NMR (376 MHz, DMSO-*d*$_6$) δ -56.74, -115.74, -118.87, -123.80.

Chiral analysis method SFC-5: Waters UPCC, analytical column: Daicel ChiralCEL®IC, 100*3mm 3μm; mobile phase A: $CO_2$. Phase B: MeOH (+0.1% DEA); Flow rate: 1.5 mL/min; Column temperature: 35°C; Back pressure: 1800 psi; Gradient: 0-8.0 min; A/B=75/25.

**Examples 6, 6A, and 6B**

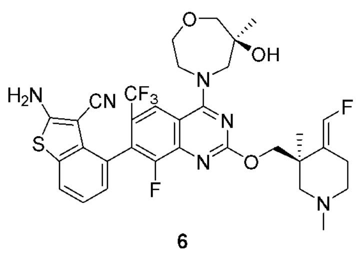

6

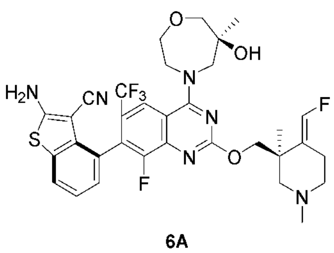

6A

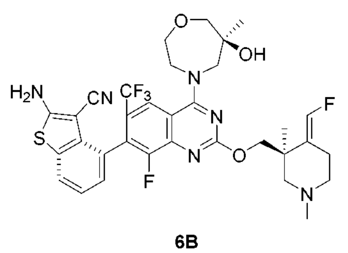

6B

2-Amino-4-(8-fluoro-2-(((*S*,*E*)-4-(fluoromethylene)-1,3-dimethylpiperidine-3-yl)methoxy)-4-((S)-6-hydroxy-6-methyl-1,4-oxazol-4-yl)-6-trifluoromethyl)quinazoline-7-yl)benzo[*b*]thiophene-3-carbonitrile (**Example 6**), (*S*)-2-amino-4-(8-fluoro-2-(((*S*,*E*)-4-(fluoromethylene)-1,3-dimethylpiperidine-3-yl)methoxy)-4-((*S*)-6-hydroxy-6-methyl-1,4-oxazol-4-yl)-6-(trifluoromethyl)quinazoline-7-yl)benzo[*b*]thiophene-3-carbonitrile (**Example 6A**), and (*R*)-2-amino-4-(8-fluoro-2-(((*S*,*E*)-4-(fluoromethylene)-1,3-dimethylpiperidine-3-yl)methoxy)-4-((*S*)-6-hydroxy-6-methyl-1,4-oxazol-4-yl)-6-(trifluoromethyl)quinazoline-7-yl)benzo[*b*]thiophene-3-carbonitrile (**Example 6B**)

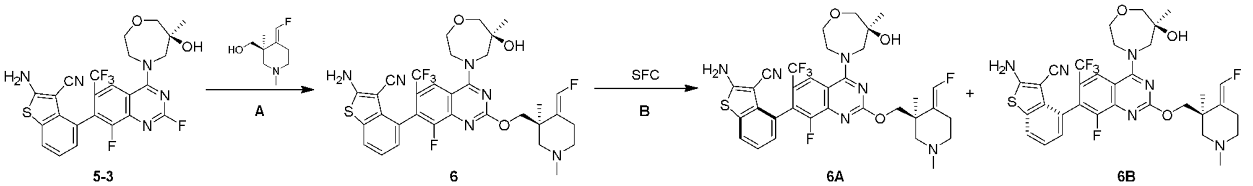


5-3 6 6A 6B

**Step A:** 2-Amino-4-(8-fluoro-2-(((*S,E*)-4-(fluoromethylene)-1,3-dimethylpiperidine-3-yl)methoxy)-4-((*S*)-6-hydroxy-6-methyl-1,4-oxazol-4-yl)-6-(trifluoromethyl)quinazoline-7-yl)benzothiophene-3-carbonitrile (**Example 6**)

Under dry ice and ethanol bath conditions, *t*BuONa (0.7 mL, 2 M THF solution, 1.4 mmol) was added to a mixed solution of 2-amino-4-(2,8-difluoro-4-((*S*)-6-hydroxy-6-methyl-1,4-oxazol-4-yl)-6-(trifluoromethyl)quinazoline-7-yl)benzothiophene-3-carbonitrile (150 mg, 0.28 mmol), (*S,E*)-(4-(fluoromethylene)-1,3-dimethylpiperidine-3-yl)methanol (73 mg, 0.42 mmol) and THF (10 mL) and stirred for 1.5 h. After the reaction completion was monitored by TLC and the reaction solution was brought back to room temperature, it was poured into a semi-saturated $NH_4Cl$ (30 mL) solution and extracted with EA (50 mL×3). The organic phase was collected, washed with saturated saline solution, dried over anhydrous $Na_2SO_4$, and concentrated. The organic phase was purified by FCC ($SiO_2$, (EA/EtOH=3/1)/PE=0-40%). The crude product was then purified by pre-HPLC (C18, ACN/(10 mmol $NH_4HCO_3/H_2O$)=55-75%) to obtain a white solid 2-amino-4-(8-fluoro-2-(((*S,E*)-4-(fluoromethylene)-1,3-dimethylpiperidine-3-yl)methoxy)-4-((*S*)-6-hydroxy-6-methyl-1,4-oxazol-4-yl)-6-(trifluoromethyl)quinazoline-7-yl)benzothiophene-3-carbonitrile (**Example 6,** 38 mg, yield 20%). LCMS (m/z): 689.3 (M+H). $^1$H NMR (400 MHz, DMSO-$d_6$) δ 9.06 - 8.85 (m, 1H), 8.00 - 7.64 (m, 3H), 7.30 - 7.09 (m, 2H), 6.88 - 6.54 (m, 1H), 5.47 - 5.29 (m, 1H), 4.69 - 4.57 (m, 1H), 4.35 - 4.22 (m, 2H), 4.20 - 3.89 (m, 4H), 3.87 - 3.78 (m, 1H), 3.76 - 3.65 (m, 1H), 3.63 - 3.54 (m, 2H), 2.71 - 2.67 (m, 2H), 2.27 - 2.19 (m, 1H), 2.14 (s, 3H), 1.93 - 1.79 (m, 2H), 1.16 (s, 3H), 1.11 (s, 3H). $^{19}$F NMR (376 MHz, DMSO-$d_6$) δ -56.74, -123.93, -138.77.

**Step B:** (*S*)-2-amino-4-(2-(((3*S*,4*S*)-4-(difluoromethyl)-1,3-dimethylpiperidine-3-yl)methoxy)-8-fluoro-4-((*S*)-6-hydroxy-6-methyl-1,4-oxazol-4-yl)-6-(trifluoromethyl)quinazoline-7-yl)benzo[*b*]thiophene-3-carbonitrile and (*R*)-2-amino-4-(2-(((3*S*,4*S*)-4-(difluoromethyl)-1,3-dimethylpiperidine-3-yl)methoxy)-8-fluoro-4-((*S*)-6-hydroxy-6-methyl-1,4-oxazol-4-yl)-6-(trifluoromethyl)quinazoline-7-yl)benzo[*b*]thiophene-3-carbonitrile

Compound 2-amino-4-(8-fluoro-2-(((*S,E*)-4-(fluoromethylene)-1,3-dimethylpiperidine-3-yl)methoxy)-4-((*S*)-6-hydroxy-6-methyl-1,4-oxazol-4-yl)-6-(trifluoromethyl)quinazoline-7-yl)benzothiophene-3-carbonitrile (30 mg) obtained from step A was resolved by SFC (Waters SFC 150, separation column: DAICELCHIRALCEL®IC 250*30 mm 10 μm; mobile phase $CO_2$/MeOH (+0.3% 7.0 mol/1 NH3-MeOH) = 75/25; flow rate: 100 mL/min), the first eluted isomer 1 was Example **6A** (4.6 mg, yield 15%, relatively short retention time). Chiral analysis method SFC- 6, Rt = 2.680. LCMS (m/z): 689.2 (M+H); the subsequently eluted isomer 2 was Example **6B** (18 mg, yield 61%, relatively long retention time). Chiral analysis method SFC-6, Rt = 3.589. LCMS (m/z): 689.2 (M+H). $^1$H NMR (400 MHz, DMSO-$d_6$) δ 8.99 (s, 1H), 7.78 (d, *J* = 6.2 Hz, 3H), 7.26 - 7.08 (m, 2H), 6.70 (d, *J* = 86.8 Hz, 1H), 5.36 (s, 1H), 4.63 (d, *J* = 10.6 Hz, 1H), 4.36 - 4.21 (m, 2H), 4.20 - 4.09 (m, 1H), 4.08 - 3.99 (m, 1H), 3.98 - 3.89 (m, 1H), 3.87 - 3.78 (m, 1H), 3.75 - 3.64 (m, 1H), 3.62 - 3.51 (m, 2H), 2.76 - 2.63 (m, 2H), 2.29 - 2.19 (m, 2H), 2.14 (s, 3H), 1.92 - 1.79 (m, 2H), 1.16 (s, 3H), 1.10 (s, 3H). $^{19}$F NMR (376 MHz, DMSO-$d_6$) δ -56.70, -123.94, -138.79.

Chiral analysis method SFC-6: Waters UPCC, analytical column: Daicel Chiral pak®IC, 100*3mm 3μm; mobile phase A: $CO_2$. Phase B: MeOH (+0.1% DEA); Flow rate: 1.5 mL/min; Column temperature: 35°C; Back pressure: 1800 psi; Gradient: 0-8.0 min; A/B=75/25.

**Example 7**

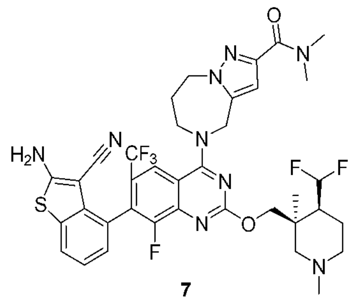

**7**

5-(7-(2-Amino-3-cyanobenzo[*b*]thiophene-4-yl)-2-(((3*S*,4*S*)-4-(difluoromethyl)-1,3-dimethylpiperidine-3-yl)methoxy)-8-fluoro-6-(trifluoromethyl)quinazoline-4-yl)-*N,N*-dimethyl-5,6,7,8-tetrahydro-4*H*-pyrazolo[1,5-*a*][1,4]diazepine-2-formamide

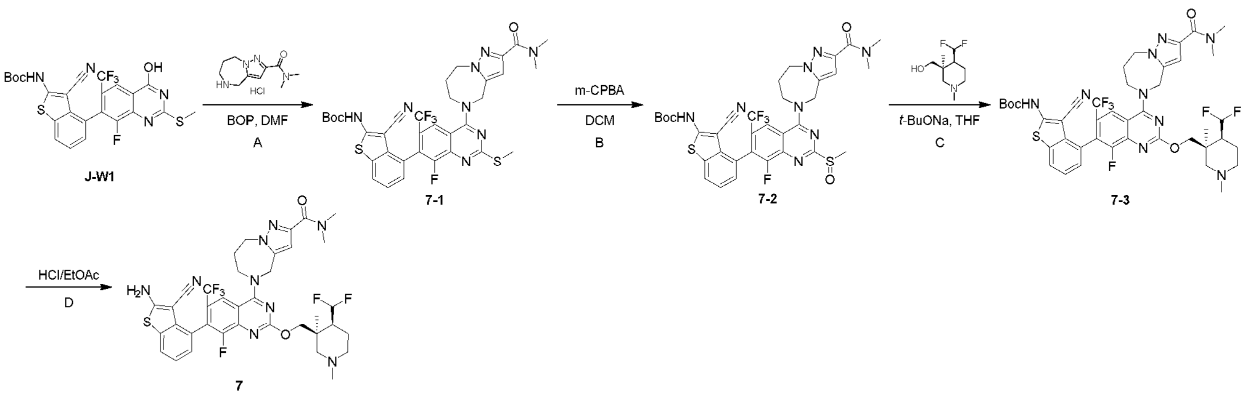


**Step A:** tert-Butyl (3-cyano-4-(4-(2-(dimethylcarbamoyl)-7,8-dihydro-4*H*-pyrazolo[1,5-*a*][1,4]diazepine-5(6*H*)-yl)-8-fluoro-2-(methylthio)-6-(trifluoromethyl)quinazoline-7-yl)benzo[*b*]thiophene-2-yl)carbamate

At room temperature, BOP (241 mg, 0.545 mmol) was added to a mixture of tert-butyl (3-cyano-4-(8-fluoro-4-hydroxy-2-(methylthio)-6-(trifluoromethyl)quinazoline-7-yl)benzo[*b*]thiophene-2-yl)carbamate (150 mg, 0.272 mmol), *N,N*-dimethyl-5,6,7,8-tetrahydro-4*H*-pyrazolo[1,5-*a*][1,4]diazepine-2-carboxamide hydrochloride (133 mg, 0.545 mmol), DIPEA (176 mg, 1.36 mmol), and DMF (3 mL). The resulting mixture was heated to 60°C and stirred overnight. After the reaction completion was monitored by LCMS, the reaction solution was poured into $H_2O$ (30 mL) and extracted with EtOAc (30 mL×3). The organic phases were combined, dried over anhydrous $Na_2SO_4$, and filtered. The filtrate was concentrated to dryness, and the crude product was purified by FCC ($SiO_2$, EA/PE=0-100%) to obtain a white solid tert-butyl 3-cyano-4-(4-(2-(dimethylcarbamoyl)-7,8-dihydro-4*H*-pyrazolo[1,5-*a*][1,4]diazepine-5(6H)-yl)-8-fluoro-2-(methylthio)-6-(trifluoromethyl)quinazoline-7-yl)benzo[*b*]thiophene-2-yl)carbamate (60 mg, yield 30%). LCMS (m/z): 309.1 (M+H).

**Step B:** tert-Butyl (3-cyano-4-(4-(2-(dimethylcarbamoyl)-7,8-dihydro-4*H*-pyrazolo[1,5-*a*][1,4]diazepine-5(6*H*)-yl)-8-fluoro-2-(methylsulfinyl)-6-(trifluoromethyl)quinazoline-7-yl)benzo[*b*]thiophene-2-yl)carbamate

At room temperature, tert-butyl (3-cyano-4-(4-(2-(dimethylcarbamoyl)-7,8-dihydro-4*H*-pyrazolo[1,5-*a*][1,4]diazepine-5(6*H*)-yl)-8-fluoro-2-(methylthio)-6-(trifluoromethyl)quinazoline-7-yl)benzo[*b*]thiophene-2-yl)carbamate (60 mg, 0.081 mmol) in dichloromethane (5 mL) was mixed with m-chloroperoxybenzoic acid (24.7 mg, 85% purity, 0.121 mmol), and the mixture was stirred for 1 h while maintaining the temperatur . After the reaction completion was monitored by LCMS, 10 mL of saturated sodium bicarbonate aqueous solution was added to the reaction mixture, followed by extraction with dichloromethane. The organic phases were combined, washed with saturated brine, dried over anhydrous sodium sulfate, filtered, concentrated, and dried to obtain a white solid tert-butyl (3-cyano-4-(4-(2-(dimethylcarbamoyl)-7,8-dihydro-4*H*-pyrazolo[1,5-*a*][1,4]diazepine-5(6*H*)-yl)-8-fluoro-2-(methylsulfinyl)-6-(trifluoromethyl)quinazoline-7-yl)benzo[*b*]thiophene-2-yl) carbamate (70 mg, crude product). LCMS (m/z): 757.1 (M+H).

**Step C:** tert-Butyl (3-cyano-4-(2-(((3*S*,4*S*)-4-(difluoromethyl)-1,3-dimethylpiperidine-3-yl)methoxy)-4-(2-(dimethylcarbamoyl)-7,8-dihydro-4*H*-pyrazolo[1,5-*a*][1,4]diazepine-5(6*H*)-yl)-8-fluoro-6-(trifluoromethyl)quinazoline-7-yl)benzo[*b*] thiophene-2-yl)carbamate

Under dry ice and ethanol bath conditions, *t*BuONa (0.23 mL, 2 M THF solution, 0.46 mmol) was added to a mixed solution of tert-butyl (3-cyano-4-(4-(2-(dimethylcarbamoyl)-7,8-dihydro-4*H*-pyrazolo[1,5-*a*][1,4]diazepine-5(6*H*)-yl)-8-fluoro-2-(methylsulfinyl)-6-(trifluoromethyl)quinazoline-7-yl)benzo[*b*]thiophene-2-yl)carbamate (70 mg, 0.092 mmol), ((3*S*,4*S*)-4-(difluoromethyl)-1,3-dimethylpiperidine-3-yl)methanol (26.8 mg, 0.139 mmol) and anhydrous THF (5 mL) and stirred for 1 h. The reaction completion was monitored by TLC. The reaction was then quenched with saturated $NH_4Cl$ (20 mL) solution, extracted with EA (30 mL×3), the organic phase was collected, washed with saturated brine, dried with anhydrous $Na_2SO_4$, and concentrated to obtain a white solid tert-butyl (3-cyano-4-(2-(((3*S*,4*S*)-4-(difluoromethyl)-1,3-dimethylpiperidine-3-yl)methoxy)-4-(2-(dimethylcarbamoyl)-7,8-dihydro-4*H*-pyrazolo[1,5-*a*][1,4]diazepine-5(6*H*)-yl)-8-fluoro-6-(trifluoromethyl)quinazoline-7-yl)benzo[*b*]thiophene-2-yl)carbamate (90 mg, crude product). LCMS (m/z): 886.2 (M+H).

**Step D:** 5-(7-(2-amino-3-cyanobenzo[*b*]thiophene-4-yl)-2-(((3*S*,4*S*)-4-(difluoromethyl)-1,3-dimethylpiperidine-3-yl) methoxy)-8-fluoro-6-(trifluoromethyl)quinazoline-4-yl)-*N,N*-dimethyl-5,6,7,8-tetrahydro-4*H*-pyrazolo[1,5-*a*][1,4]diazepine-2-formamide

At room temperature, 4 M HCl-ethyl acetate (10 mL, 40 mmol) was added to tert-butyl (3-cyano-4-(2(((3*S*,4*S*)-4-(difluoromethyl)-1,3-dimethylpiperidine-3-yl)methoxy)-4-(2-(dimethylcarbamoyl)-7,8-dihydro-4*H*-pyrazolo[1,5-*a*][1,4]diazepine-5(6*H*)-yl)-8-fluoro-6-(trifluoromethyl)quinazoline-7-yl)benzo[b]thiophene-2-yl)carbamate (90 mg, 0.102 mmol) and stirred at room temperature for 1 h. The reaction completion was monitored by LCMS. The acid solution was removed by concentration to obtain a crude product, which was then purified by pre-HPLC (C18, ACN/(10mmol $NH_4HCO_3/H_2O$) = 40-75%), lyophilized, and yielded a white solid product 5-(7-(2-amino-3-cyanobenzo [*b*]thiophene-4-yl)-2-(((3*S*,4*S*)-4-(difluoromethyl)-1,3-dimethylpiperidine-3-yl)methoxy)-8-fluoro-6-(trifluoromethyl)quinazoline-4-yl)-N,N-dimethyl-5,6,7,8-tetrahydro-4*H*-pyrazolo[1,5-*a*][1,4]diazepine-2-formamide (2.2 mg). LCMS (m/z): 786.3 (M+H).

**Examples 7A and 7B**

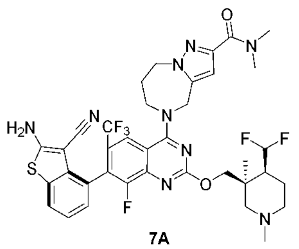

7A

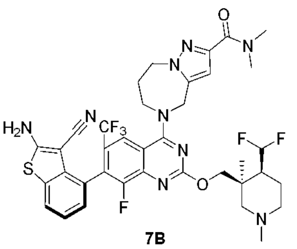

7B

Example 7 was resolved by pre-HPLC or chiral SFC under appropriate conditions to obtain Examples 7A and 7B.

**Example 8**

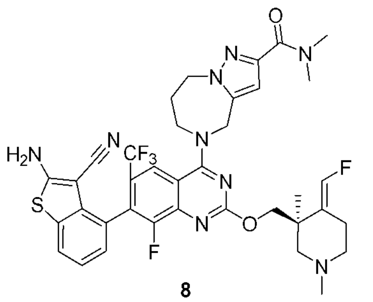

8

5-(7-(2-Amino-3-cyanobenzo[*b*]thiophene-4-yl)-8-fluoro-2-(((*S*,*E*)-4-(fluoromethylene)-1,3-dimethylpiperidine-3-yl)methoxy)-6-(trifluoromethyl)quinazoline-4-yl)-*N,N*-dimethyl-5,6,7,8-tetrahydro-4*H*-pyrazolo[1,5-*a*][1,4]diazepine-2-formamide

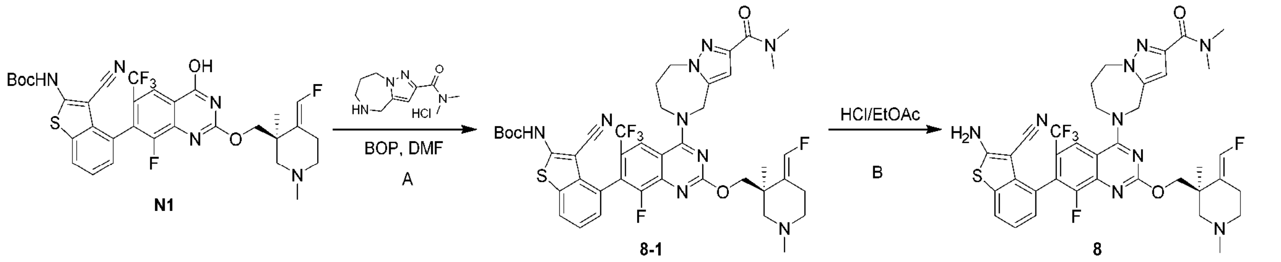


**Step A:** tert-Butyl (3-cyano-4-(4-(2-(dimethylcarbamoyl)-7,8-dihydro-4*H*-pyrazolo[1,5-*a*][1,4]diazepine-5(6*H*)-yl)-8-fluoro-2-(((*S*,*E*)-4-(fluoromethylene)-1,3-dimethylpiperidine-3-yl)methoxy)-6-(trifluoromethyl)quinazoline-7-yl)benzo[b]thiophene-2-yl)carbamate

At room temperature, BOP (78.8 mg, 0.178 mmol) was added to a mixture of tert-butyl (3-cyano-4-(8-fluoro-2-(((*S*,*E*)-4-(fluoromethylene)-1,3-dimethylpiperidine-3-yl)methoxy)-4-hydroxy-6-(trifluoromethyl)quinazoline-7-yl)benzo[*b*]thiophene-2-yl)carbamate (60 mg, 0.089 mmol), *N,N*-dimethyl-5,6,7,8-tetrahydro-4*H*-pyrazolo[1,5-*a*][1,4]diazepine-2-formamide hydrochloride (43.4 mg, 0.178 mmol), DIPEA (230 mg, 1.78 mmol), and DMF (2 mL), and the mixture was heated to 60°C and stirred overnight. After the reaction completion was monitored by LCMS, the reaction solution was poured into $H_2O$ (10 mL) and extracted with EtOAc (30 mL×3). The organic phases were combined, dried over anhydrous $Na_2SO_4$, and filtered. The filtrate was concentrated to dryness to give a white solid product tert-butyl (3-cyano-4-(4-(2-(dimethylcarbamoyl)-7,8-dihydro-4*H*-pyrazolo[1,5-*a*][1,4]diazepine-5(6*H*)-yl)-8-fluoro-2-(((*S*,*E*)-4-(fluoromethylene)-1,3-dimethylpiperidine-3-yl)methoxy)-6-(trifluoromethyl)quinazoline-7-yl)benzo[*b*]thiophene-2-yl)carbamate (90 mg, crude product). LCMS (m/z): 866.3 (M+H).

**Step B:** 5-(7-(2-Amino-3-cyanobenzo[*b*]thiophene-4-yl)-8-fluoro-2-(((*S*,*E*)-4-(fluoromethylene)-1,3-dimethylpiperidine-3-yl)methoxy)-6-(trifluoromethyl)quinazoline-4-yl)-*N,N*-dimethyl-5,6,7,8-tetrahydro-4*H*-pyrazolo[1,5-*a*][1,4]diazepine-2-formamide

At room temperature, 4 M HCl-ethyl acetate (10 mL, 40 mmol) was added to tert-butyl (3-cyano-4-(4-(2-(dimethylcarbamoyl)-7,8-dihydro-4*H*-pyrazolo[1,5-*a*][1,4]diazepine-5(6*H*)-yl)-8-fluoro-2-(((*S,E*)-4-(fluoromethylene)-1,3-dimethylpiperidine-3-yl)methoxy)-6-(trifluoromethyl)quinazoline-7-yl)benzo[b]thiophene-2-yl)carbamate (90 mg, 0.104 mmol) and stirred at room temperature for 1 h. The reaction completion was monitored by LCMS. The acid solution was removed by concentration to obtain a crude product, which was then purified by pre-HPLC (C18, ACN/(10mmol $NH_4HCO_3/H_2O$)=40-75%), lyophilized, and yielded a white solid product 5-(7-(2-amino-3-cyanobenzo[*b*]thiophene-4-yl)-8-fluoro-2-(((*S*,*E*)-4-(fluoromethylene)-1,3-dimethylpiperidine-3-yl)methoxy)-6-(trifluoromethyl)quinazoline-4-yl)-*N,N*-dimethyl-5,6,7,8-tetrahydro-4H-pyrazolo[1,5-*a*][1,4]diazepine-2-formamide) (3.0 mg, yield 4%). LCMS (m/z): 766.2 (M+H).

**Examples 8A and 8B**

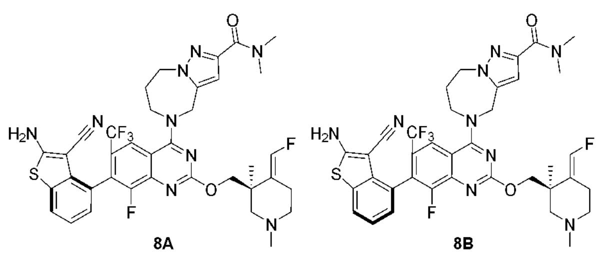

Example 8 was purified by Pre-HPLC (C18, ACN/(0.1%FA/$H_2O$) = 25-45%) to obtain the first eluted isomer 1 (retention time 10.7 min). This isomer was neutralized with ion exchange resin to obtain Example 8A. LCMS (m/z): 766.3

(M+H). $^1$H NMR (400 MHz, DMSO-$d_6$) δ 8.15 (s, 1H), 7.83 - 7.75 (m, 3H), 7.25 - 7.18 (m, 1H), 7.18 - 7.13 (m, 1H), 6.83 (s, 0.5H), 6.64 (s, 1H), 6.61 (s, 0.5H), 5.24 - 5.12 (m, 1H), 5.01 - 4.88 (m, 1H), 4.64 - 4.56 (m, 1H), 4.55 - 4.41 (m, 2H), 4.37 - 4.22 (m, 2H), 4.17 - 4.04 (m, 1H), 3.26 (s, 3H), 2.96 (s, 3H), 2.74 - 2.65 (m, 2H), 2.56 - 2.52 (m, 2H), 2.42 - 2.24 (m, 2H), 2.23 - 2.08 (m, 4H), 1.94 - 1.79 (m, 2H), 1.10 (s, 3H). $^{19}$F NMR (376 MHz, DMSO-$d_6$) δ -56.95, -123.21, -138.68; the subsequently eluted isomer 2 (retention time 11.6) was then neutralized with ion exchange resin to obtain Example 8B, LCMS (m/z): 766.3 (M+H).

Following the synthetic method described above, the following examples were prepared and characterized:

| | | |
|---|---|---|
| 9 | 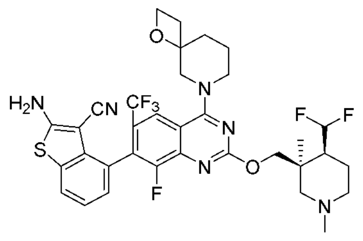 | LCMS (m/z): 705.2 (M+H) |
| 9A | 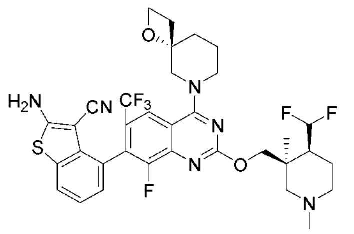 | LCMS (m/z): 705.3 (M+H). $^1$H NMR (400 MHz, DMSO-$d_6$) δ 8.66 - 8.38 (m, 1H), 7.85 - 7.73 (m, 3H), 7.26 - 7.14 (m, 2H), 6.31 (t, $J$ = 55.7 Hz, 1H), 4.51 - 4.39 (m, 4H), 4.22 - 3.71 (m, 2H), 2.87 - 2.78 (m, 1H), 2.46 - 2.30 (m, 3H), 2.11 (s, 3H), 1.91 - 1.55 (m, 7H), 1.29 - 1.15 (m, 4H), 1.12 (s, 3H). $^{19}$F NMR (376 MHz, DMSO-$d_6$) δ -57.28, -113.14 - -120.05, -122.46 --126.51. |
| 10 | 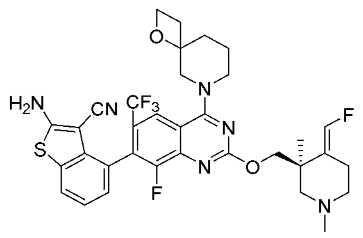 | LCMS (m/z): 685.2 (M+H) |
| 10A | 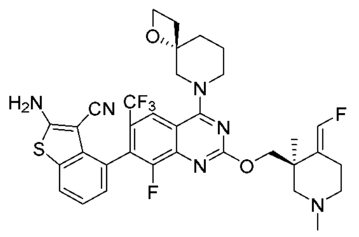 | LCMS (m/z): 685.3 (M+H). $^1$H NMR (400 MHz, DMSO-$d_6$) δ 8.72 - 8.33 (m, 1H), 7.93 - 7.68 (m, 3H), 7.27 - 7.15 (m, 2H), 6.72 (d, $J$ = 86.6 Hz, 1H), 4.69 - 4.58 (m, 1H), 4.50 - 4.40 (m, 2H), 4.34 - 3.98 (m, 2H), 3.72 - 3.45 (m, 2H), 2.78 - 2.62 (m, 2H), 2.42 - 2.32 (m, 2H), 2.21 - 2.09 (m, 7H), 1.90 - 1.64 (m, 5H), 1.11 (s, 3H). $^{19}$F NMR (376 MHz, DMSO-$d_6$) δ - 53.71 - -61.12 (m), -120.00 - -128.28 (m), - 138.69. |
| 10A-A | 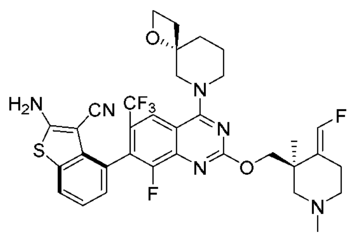 | LCMS (m/z): 685.0 (M+H) |
| 10A-B | 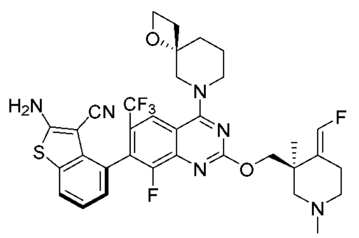 | LCMS (m/z): 685.0 (M+H) |

(continued)

| | | |
|---|---|---|
| 11 | 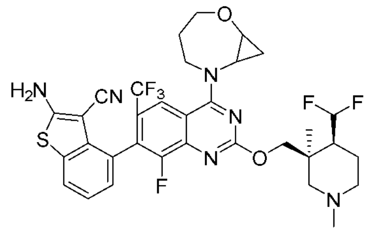 | LCMS (m/z): 691.2 (M+H) |
| 11A-A | 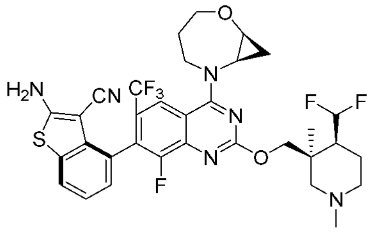 | LCMS (m/z): 691.3 (M+H). $^1$H NMR (400 MHz, Methanol-$d_4$) δ 8.64 (s, 1H), 7.73 - 7.56 (m, 1H), 7.34 - 7.09 (m, 2H), 6.44 - 5.93 (m, 1H), 4.73 - 4.67 (m, 1H), 4.65 - 4.43 (m, 3H), 4.02 - 3.95 (m, 1H), 3.89 - 3.76 (m, 2H), 3.70 - 3.56 (m, 1H), 3.42 - 3.36 (m, 1H), 3.19 - 3.04 (m, 1H), 2.98 - 2.90 (m, 1H), 2.55 - 2.39 (m, 1H), 2.30 - 2.11 (m, 3H), 2.04 - 1.62 (m, 6H), 1.24 - 1.17 (m, 3H), 0.81 - 0.67 (m, 1H). $^{19}$F NMR (376 MHz, Methanol-$d_4$) δ -59.81, -119.33, -121.60, -125.08. |
| 11A-B | 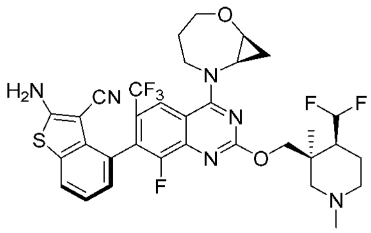 | LCMS (m/z): 691.3 (M+H). $^1$H NMR (400 MHz, Methanol-$d_4$) δ 8.62 (s, 1H), 7.76 - 7.58 (m, 1H), 7.31 - 7.11 (m, 2H), 6.46 - 6.02 (m, 1H), 4.79 - 4.68 (m, 1H), 4.64 - 4.51 (m, 3H), 4.03 - 3.92 (m, 1H), 3.85 - 3.73 (m, 2H), 3.71 - 3.57 (m, 1H), 3.38 - 3.32 (m, 1H), 3.16 - 3.07 (m, 1H), 3.01 - 2.89 (m, 1H), 2.53 - 2.37 (m, 1H), 2.26 - 2.13 (m, 3H), 2.10 - 1.96 (m, 1H), 1.91 - 1.65 (m, 5H), 1.24 - 1.17 (m, 3H), 0.96 - 0.79 (m, 1H). $^{19}$F NMR (376 MHz, Methanol-$d_4$) δ -59.74, -119.47, -121.22, -125.82. |
| 12 | 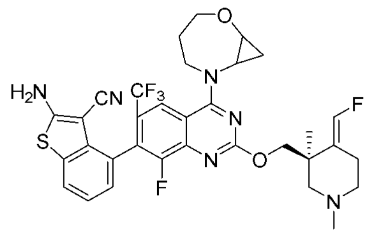 | LCMS (m/z): 671.2 (M+H) |
| 12A-A | 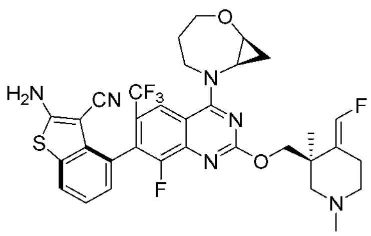 | LCMS (m/z): 671.3 (M+H). $^1$H NMR (400 MHz, Methanol-$d_4$) δ 8.64 (s, 1H), 7.73 - 7.55 (m, 1H), 7.35 - 7.13 (m, 2H), 6.87 - 6.71 (m, 0.5H), 6.63 - 6.49 (m, 0.5H), 4.74 - 4.64 (m, 1H), 4.63 - 4.40 (m, 3H), 4.04 - 3.93 (m, 1H), 3.90 - 3.82 (m, 1H), 3.81 - 3.75 (m, 1H), 3.70 - 3.56 (m, 1H), 3.41 - 3.35 (m, 1H), 2.91 - 2.85 (m, 1H), 2.77 - 2.71 (m, 1H), 2.68 - 2.61 (m, 1H), 2.51 - 2.36 (m, 1H), 2.34 - 2.26 (m, 1H), 2.24 (s, 3H), 2.09 - 2.01 (m, 1H), 2.00 - 1.95 (m, 1H), 1.95 - 1.79 (m, 1H), 1.21 (s, 3H), 0.78 - 0.65 (m, 1H). $^{19}$F NMR (376 MHz, Methanol-$d_4$) δ -59.80, -124.96, -139.63. |
| 12A-B | 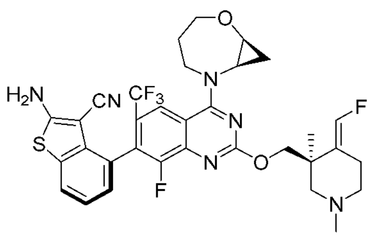 | LCMS (m/z): 671.3 (M+H). $^1$H NMR (400 MHz, Methanol-$d_4$) δ 8.61 (s, 1H), 7.71 - 7.57 (m, 1H), 7.36 - 7.12 (m, 2H), 6.84 - 6.72 (m, 0.5H), 6.59 - 6.51 (m, 0.5H), 4.74 - 4.65 (m, 1H), 4.63 - 4.57 (m, 1H), 4.50 (s, 2H), 4.02 - 3.94 (m, 1H), 3.86 - 3.76 (m, 1H), 3.75 - 3.70 (m, 1H), 3.69 - 3.59 (m, 1H), 3.37 - 3.32 (m, 1H), 2.93 - 2.87 (m, 1H), 2.82 - 2.71 (m, 1H), 2.71 - 2.59 (m, 1H), 2.53 - 2.36 (m, 1H), 2.35 - 2.28 (m, 1H), 2.25 (s, 3H), 2.14 - 2.04 (m, 1H), 2.03 - 1.96 (m, 1H), 1.92 - 1.84 (m, 1H), 1.21 (s, 3H), 0.95 - 0.82 (m, 1H). $^{19}$F NMR (376 MHz, Methanol-$d_4$) δ -59.72, -125.76, -139.25. |

(continued)

| | | |
|---|---|---|
| 13 | 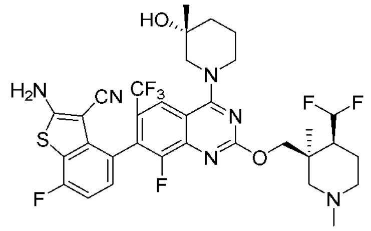 | LCMS (m/z): 711.2 (M+H) |
| 14 | 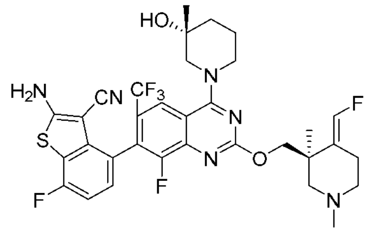 | LCMS (m/z): 691.2 (M+H) |
| 15 | 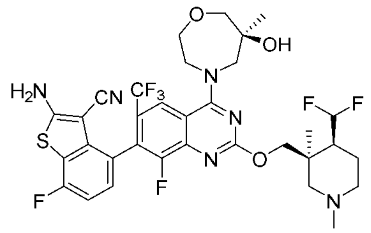 | LCMS (m/z): 727.2 (M+H) |
| 16 | 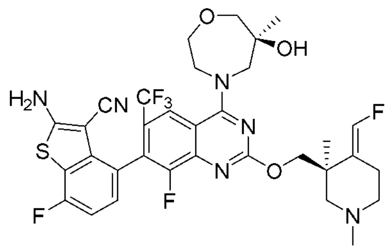 | LCMS (m/z): 707.2 (M+H). $^{1}$H NMR (400 MHz, DMSO-$d_6$ ) δ 9.08 - 8.88 (m, 1H), 8.10 (s, 2H), 7.32 - 7.21 (m, 1H), 7.20 - 7.10 (m, 1H), 6.88 - 6.59 (m, 1H), 5.43 - 5.24 (m, 1H), 4.71 - 4.57 (m, 1H), 4.36 - 4.24 (m, 2H), 4.21 - 4.02 (m, 2H), 4.01 - 3.81 (m, 2H), 3.76 - 3.68 (m, 1H), 3.65 - 3.55 (m, 2H), 2.32 - 2.18 (m, 1H), 2.16 (s, 3H), 1.99 - 1.79 (m, 2H), 1.21 - 1.04 (m, 7H). $^{19}$F NMR (376 MHz, DMSO-$d_6$) δ -56.74, -56.85, -116.63, -116.65, -123.77, -138.75. |
| 17 | 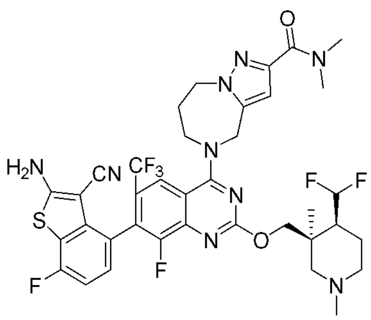 | LCMS (m/z): 804.3 (M+H) |
| 18 | 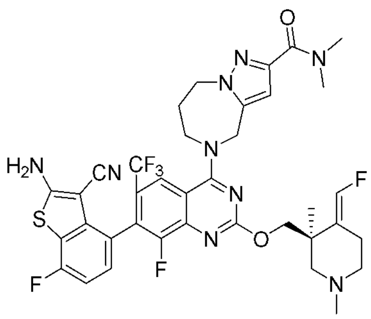 | LCMS (m/z): 784.3 (M+H) |

(continued)

| | | |
|---|---|---|
| 19 | 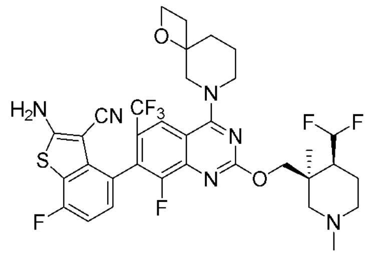 | LCMS (m/z): 723.2 (M+H) |
| 20 | 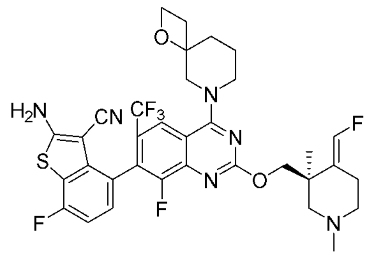 | LCMS (m/z): 703.2 (M+H) |
| 21 | 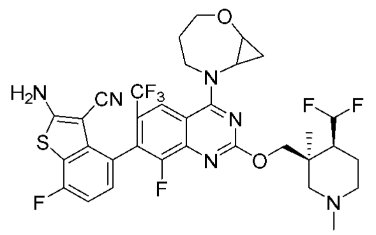 | LCMS (m/z): 709.2 (M+H) |
| 22 | 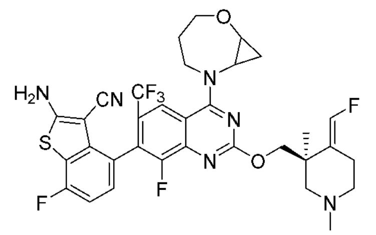 | LCMS (m/z): 689.2 (M+H) |
| 23 | 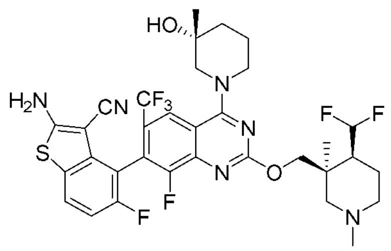 | LCMS (m/z): 711.2 (M+H) |
| 24 | 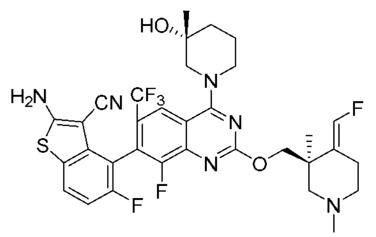 | LCMS (m/z): 691.2 (M+H) |

(continued)

| | | |
|---|---|---|
| 25 | 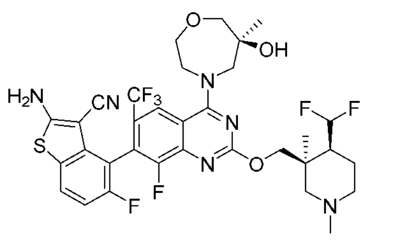 | LCMS (m/z): 727.2 (M+H) |
| 26 | 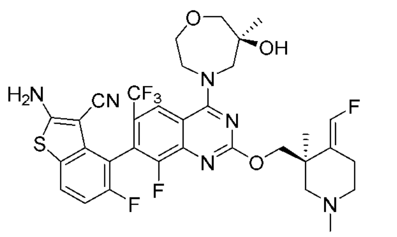 | LCMS (m/z): 707.2 (M+H) |
| 27 | 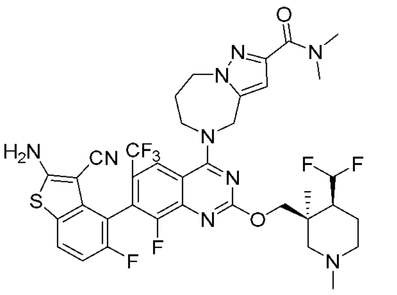 | LCMS (m/z): 804.3 (M+H) |
| 28 | 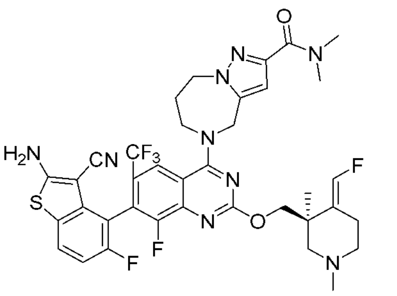 | LCMS (m/z): 784.3 (M+H) |
| 28A | 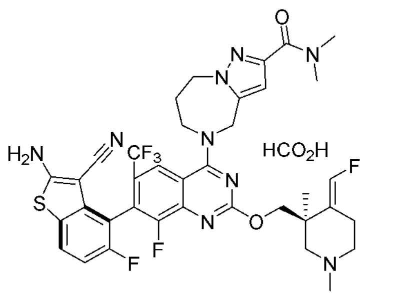 | LCMS (m/z): 784.3 (M+H). $^1$H NMR (400 MHz, DMSO-$d_6$) δ 8.30 - 8.15 (m, 2H), 8.07 - 7.92 (m, 2H), 7.90 - 7.76 (m, 1H), 7.20 - 7.05 (m, 1H), 6.91 - 6.80 (m, 0.5H), 6.66 (s, 1H), 6.64 - 6.60 (m, 0.5H), 5.30 - 5.14 (m, 1H), 5.00 - 4.90 (m, 1H), 4.68 - 4.58 (m, 1H), 4.55 - 4.46 (m, 2H), 4.39 - 4.25 (m, 2H), 4.17 - 4.07 (m, 1H), 3.27 (s, 3H), 2.96 (s, 3H), 2.78 - 2.63 (m, 2H), 2.55 - 2.52 (m, 1H), 2.42 - 2.27 (m, 2H), 2.21 - 2.09 (m, 4H), 1.95 - 1.81 (m, 2H), 1.11 (s, 3H). $^{19}$F NMR (376 MHz, DMSO-$d_6$) δ - 59.01, -117.68, -122.67, -138.63. |
| 28B | 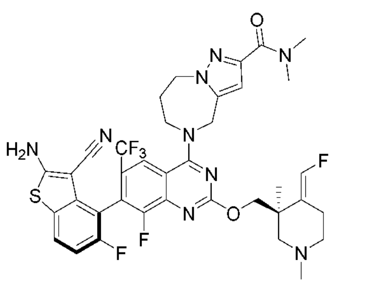 | LCMS (m/z): 784.3 (M+H). $^1$H NMR (400 MHz, DMSO-$d_6$) δ 8.21 (s, 1H), 7.98 (s, 2H), 7.87 (dd, *J* = 8.7, 4.9 Hz, 1H), 7.15 (t, *J* = 9.1 Hz, 1H), 6.88 - 6.55 (m, 2H), 5.20 (d, *J* = 16.4 Hz, 1H), 4.98 (d, *J* = 16.4 Hz, 1H), 4.65 (d, *J* = 10.6 Hz, 1H), 4.50 (m, 2H), 4.30 (m, 2H), 4.14 (m, 1H), 3.29 (s, 3H), 2.98 (s, 3H), 2.72 (d, J = 10.7 Hz, 2H), 2.55 (m, 1H), 2.36 (m, 2H), 2.18 (m, 4H), 1.96 - 1.81 (m, 2H), 1.11 (s, 3H). |

(continued)

| | | |
|---|---|---|
| 29 | 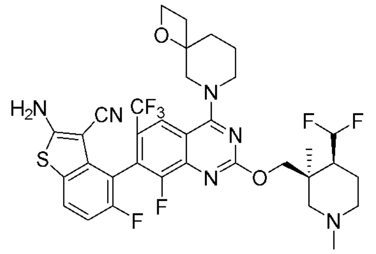 | LCMS (m/z): 723.2 (M+H) |
| 30 | 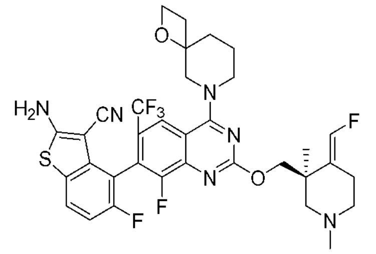 | LCMS (m/z): 703.2 (M+H) |
| 31 | 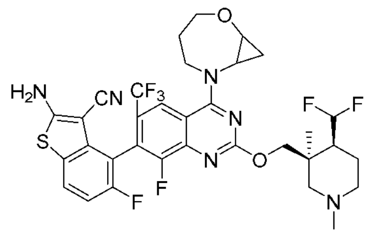 | LCMS (m/z): 709.2 (M+H) |
| 32 | 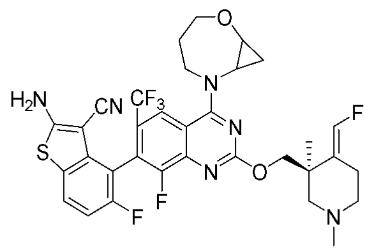 | LCMS (m/z): 689.2 (M+H) |
| 33 | 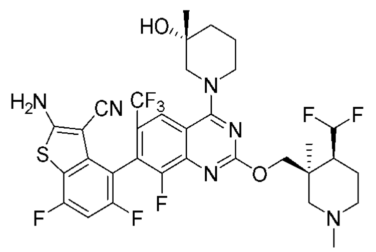 | LCMS (m/z): 729.2 (M+H) |
| 34 | 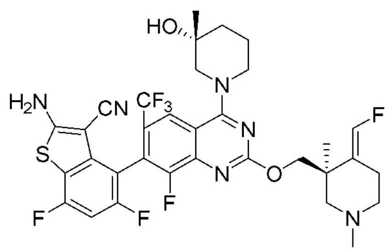 | LCMS (m/z): 709.2 (M+H) |

(continued)

| | | |
|---|---|---|
| 35 | 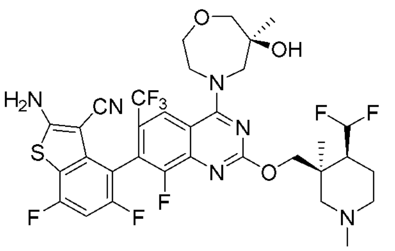 | LCMS (m/z): 745.2 (M+H) |
| 36 | 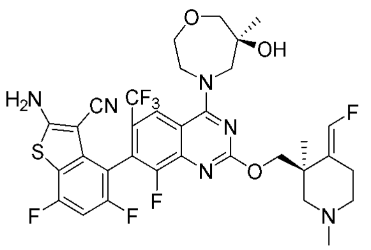 | LCMS (m/z): 725.2 (M+H) |
| 37 | 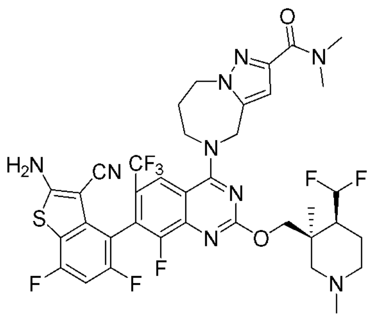 | LCMS (m/z): 822.3 (M+H) |
| 38 | 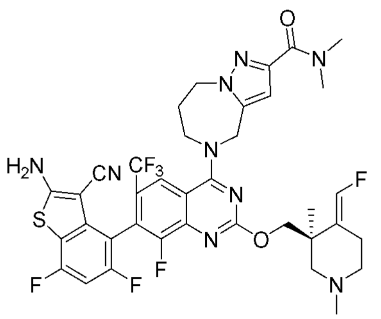 | LCMS (m/z): 802.2 (M+H) |
| 39 | 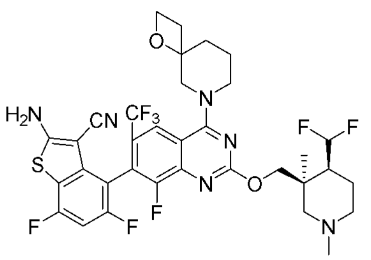 | LCMS (m/z): 741.2 (M+H) |
| 40 | 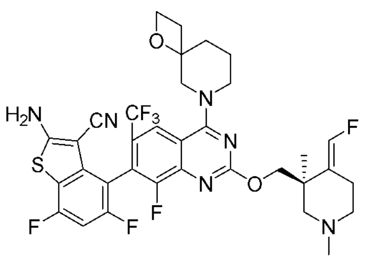 | LCMS (m/z): 721.2 (M+H) |

(continued)

| | | |
|---|---|---|
| 41 | 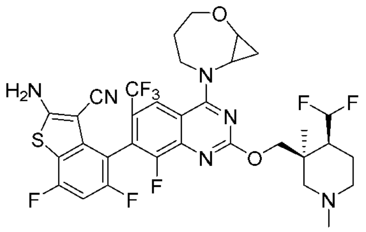 | LCMS (m/z): 727.2 (M+H) |
| 42 | 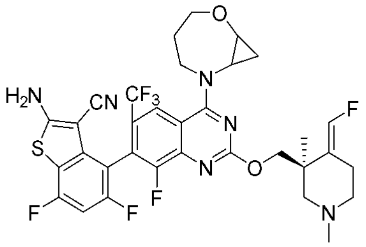 | LCMS (m/z): 707.2 (M+H) |
| 43 | 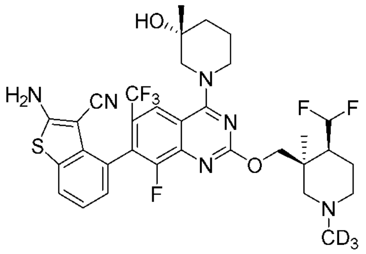 | LCMS (m/z): 696.3 (M+H) |
| 44 | 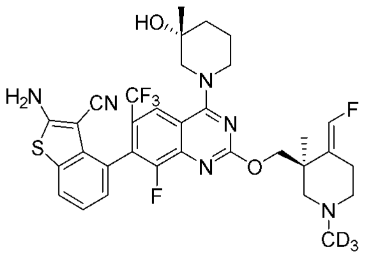 | LCMS (m/z): 676.3 (M+H) |
| 45 | 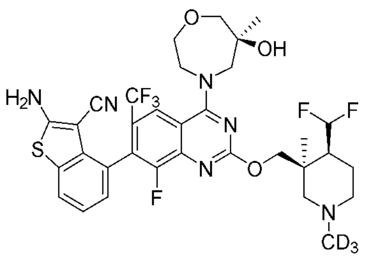 | LCMS (m/z): 712.3 (M+H) |
| 46 | 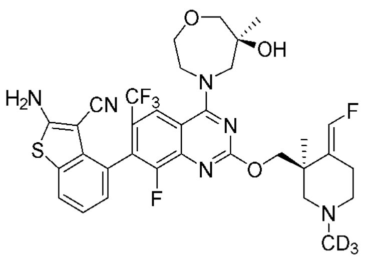 | LCMS (m/z): 692.2 (M+H) |

(continued)

| | | |
|---|---|---|
| 47 | 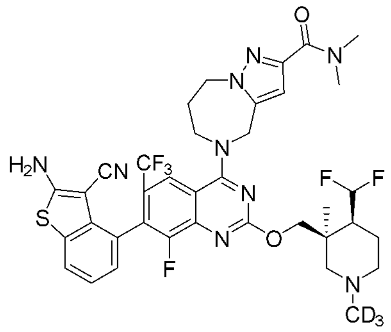 | LCMS (m/z): 789.3 (M+H) |
| 48 | 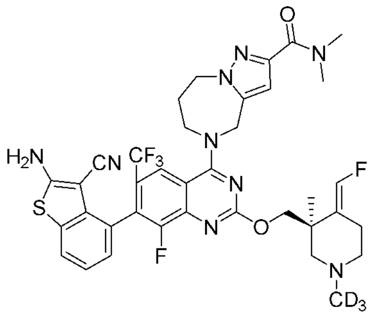 | LCMS (m/z): 769.3 (M+H) |
| 49 | 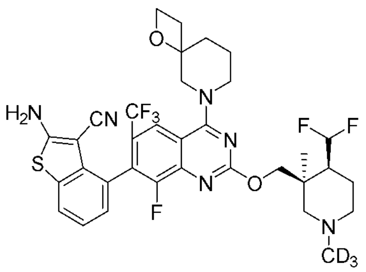 | LCMS (m/z): 708.3 (M+H) |
| 50 | 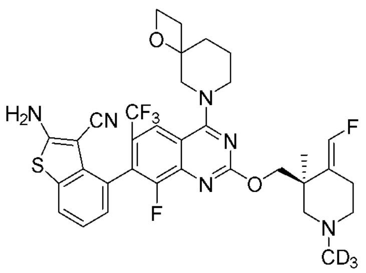 | LCMS (m/z): 688.3 (M+H) |
| 51 | 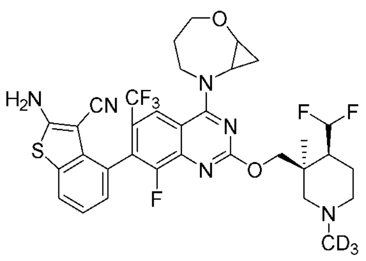 | LCMS (m/z): 694.2 (M+H) |

(continued)

| | | |
|---|---|---|
| 52 | 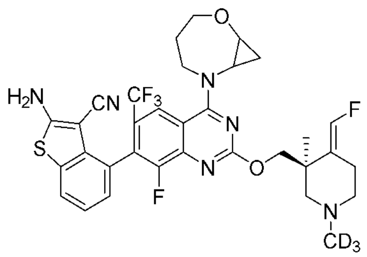 | LCMS (m/z): 674.2 (M+H) |
| 53 | 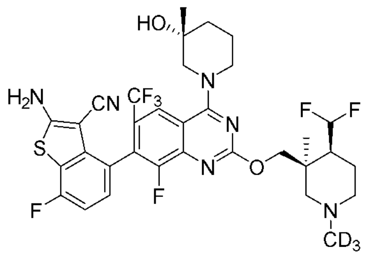 | LCMS (m/z): 714.3 (M+H) |
| 54 | 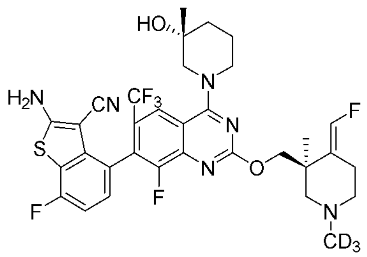 | LCMS (m/z): 694.2 (M+H) |
| 55 | 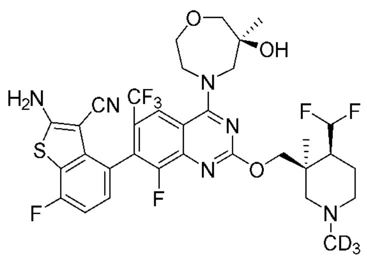 | LCMS (m/z): 730.2 (M+H) |
| 56 | 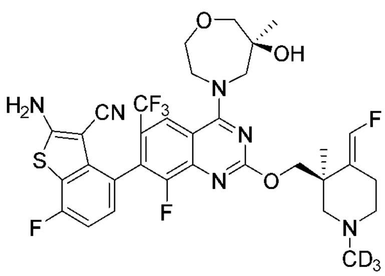 | LCMS (m/z): 710.2 (M+H) |
| 57 | 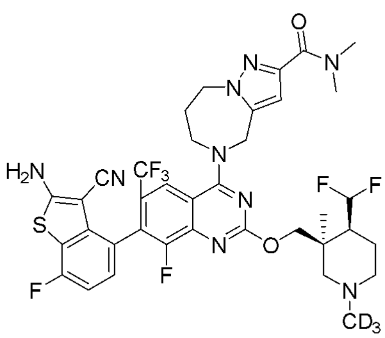 | LCMS (m/z): 807.3 (M+H) |

(continued)

| | | |
|---|---|---|
| 58 | 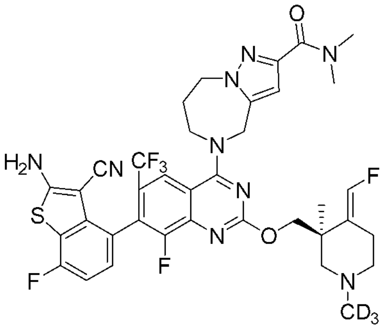 | LCMS (m/z): 787.3 (M+H) |
| 59 | 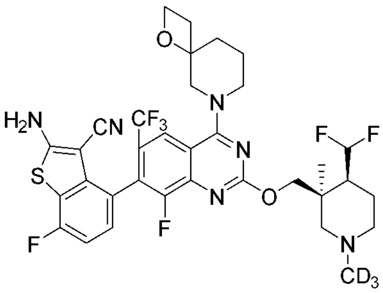 | LCMS (m/z): 726.3 (M+H) |
| 60 | 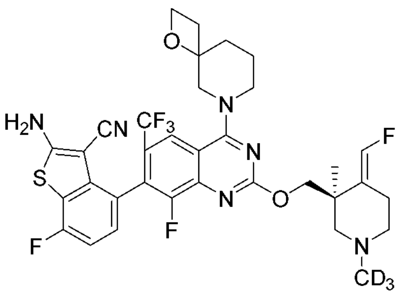 | LCMS (m/z): 706.2 (M+H) |
| 61 | 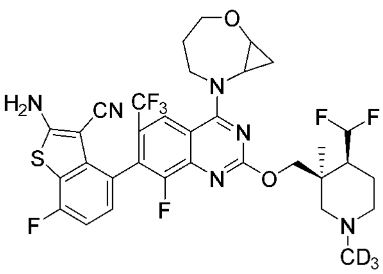 | LCMS (m/z): 712.2 (M+H) |
| 62 | 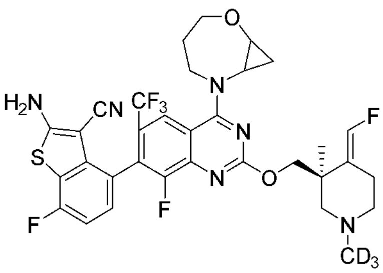 | LCMS (m/z): 692.2 (M+H) |

(continued)

| | | |
|---|---|---|
| 63 | 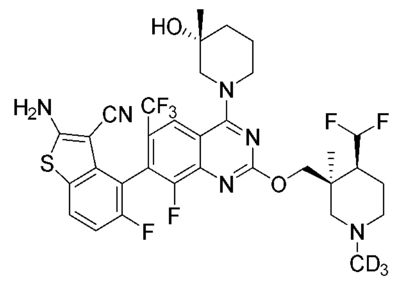 | LCMS (m/z): 714.3 (M+H) |
| 64 | 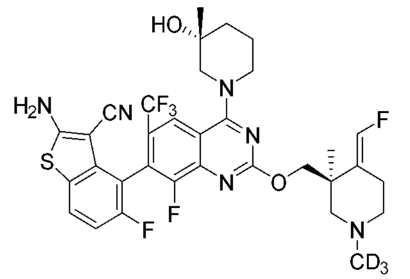 | LCMS (m/z): 694.2 (M+H) |
| 65 | 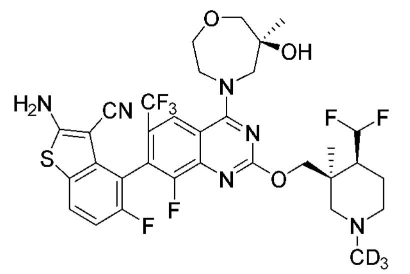 | LCMS (m/z): 730.2 (M+H) |
| 65A | 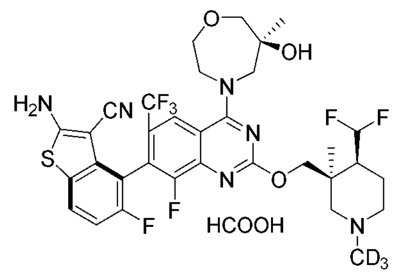 HCOOH | LCMS (m/z): 730.2 (M+H). $^{1}$H NMR (400 MHz, DMSO-$d_6$) δ 8.94 (s, 1H), 7.96 (s, 2H), 7.84 (dd, *J* = 8.7, 4.9 Hz, 1H), 7.18 - 7.08 (m, 1H), 6.47 - 6.14 (m, 1H), 5.34 (s, 1H), 4.51 - 4.42 (m, 2H), 4.31 - 4.22 (m, 1H), 4.14 - 4.03 (m, 2H), 4.00 - 3.89 (m, 2H), 3.78 - 3.69 (m, 1H), 3.61 - 3.56 (m, 2H), 2.89 - 2.78 (m, 2H), 1.89 - 1.55 (m, 5H), 1.13 (s, 3H), 1.11 (s, 3H). $^{19}$F NMR (376 MHz, DMSO-$d_6$) δ -58.91, - 115.82, -118.05, -118.85, -123.51. |
| 65B | 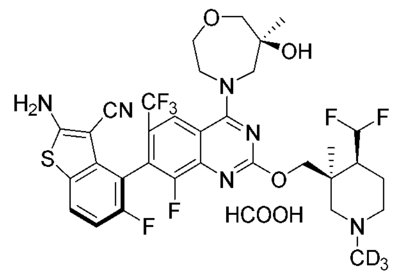 HCOOH | LCMS(m/z): 730.2 (M+H). $^{1}$H NMR (400 MHz, DMSO-$d_6$) δ 9.01 (s, 1H), 7.96 (s, 2H), 7.84 (dd, *J* = 8.7, 4.9 Hz, 1H), 7.16 - 7.08 (m, 1H), 6.46 - 6.13 (m, 1H), 5.37 (s, 1H), 4.51 - 4.42 (m, 2H), 4.34 - 4.25 (m, 1H), 4.22 - 4.15 (m, 1H), 4.08 - 4.00 (m, 1H), 3.98 - 3.90 (m, 1H), 3.88 - 3.81 (m, 1H), 3.76 - 3.68 (m, 1H), 3.61 - 3.56 (m, 2H), 2.89 - 2.79 (m, 2H), 1.90 - 1.56 (m, 5H), 1.16 (s, 3H), 1.11 (s, 3H). $^{19}$F NMR (376 MHz, DMSO-$d_6$) δ -58.83, -115.77, -117.66, -118.87, -123.16. |
| 66 | 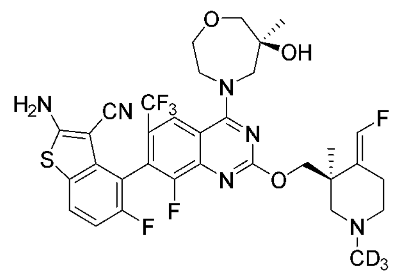 | LCMS (m/z): 710.2 (M+H) |

**Example 67**

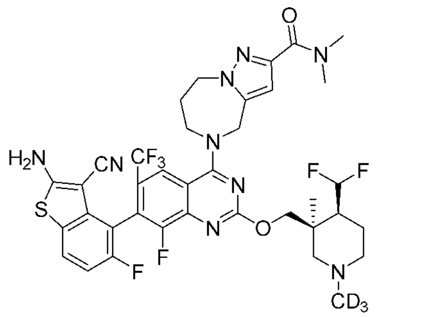

5-(7-(2-Amino-3-cyano-5-fluorobenzo[*b*]thiophene-4-yl)-2-(((3*S*,4*S*)-4-(difluoromethyl)-3-methyl-1-(methyl-$d_3$)piperidine-3-yl)methoxy)-8-fluoro-6-(trifluoromethyl)quinazoline-4-yl)-*N,N*-dimethyl-5,6,7,8-tetrahydro-4*H*-pyrazolo[1,5-*a*][1,4]diazepine-2-formamide

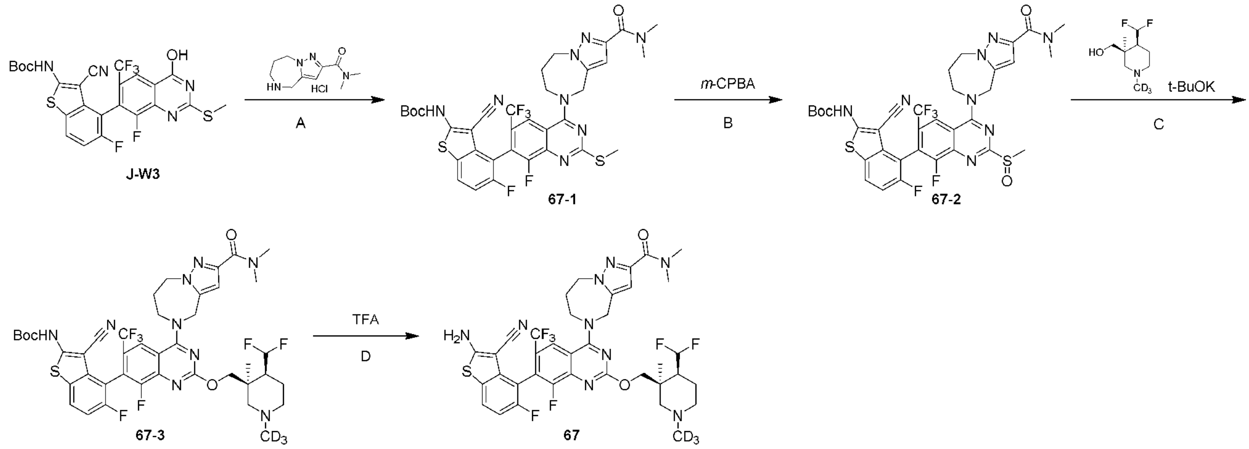


**Step A:** tert-Butyl (3-cyano-4-(4-(2-(dimethylcarbamoyl)-7,8-dihydro-4*H*-pyrazolo[1,5-*a*][1,4]diazepine-5(6*H*)-yl)-8-fluoro-2-(methylthio)-6-(trifluoromethyl)quinazoline-7-yl)-5-fluorobenzo[*b*]thiophene-2-yl)carbamate

At room temperature, tert-butyl (3-cyano-5-fluoro-4-(8-fluoro-4-hydroxy-2-(methylthio)-6-(trifluoromethyl)quinazolin-7-yl)benzo[b]thiophen-2-yl)carbamate (200 mg, 0.35 mmol), *N,N-d*imethyl-5,6,7,8-tetrahydro-4*H*-pyrazolo[1,5-*a*][1,4]diazepine-2-formamidehydrochloride (172 mg, 0.74 mmol), BOP (312 mg, 0.74 mmol), and DIEA (228 mg, 1.76 mmol) were sequentially added to DMF (5 mL). After $N_2$ replacement three times, the mixture was warmed to 50°C and stirred for 5 h. The reaction completion was monitored by LCMS and TLC. The system was cooled to room temperature, and the reaction solution was poured into $H_2O$ (50 mL), flocculent solid was formed, filtered, the filter cake was collected, dissolved in EA (50 mL), and then dried with anhydrous $Na_2SO_4$, filtered and concentrated to yield a yellow solid tert-butyl (3-cyano-4-(4-(2-(dimethylcarbamoyl)-7,8-dihydro-4*H*-pyrazolo[1,5-*a*][1,4]diazepine-5(6*H*)-yl)-8-fluoro-2-(methylthio)-6-(trifluoromethyl)quinazoline-7-yl)-5-fluorobenzo[*b*]thiophene-2-yl)carbamate (240 mg, 90% yield). LCMS (m/z): 758.9 (M+H).

**Step B:** tert-Butyl (3-cyano-4-(4-(2-(dimethylcarbamoyl)-7,8-dihydro-4*H*-pyrazolo[1,5-*a*][1,4]diazepine-5(6*H*)-yl)-8-fluoro-2-(methylsulfinyl)-6-(trifluoromethyl)quinazoline-7-yl)-5-fluorobenzo[*b*]thiophene-2-yl)carbamate

At room temperature, *m*-CPBA (85% w/w, 128 mg, 0.63 mmol) and tert-butyl (3-cyano-4-(4-(2-(dimethylcarbamoyl)-7,8-dihydro-4*H*-pyrazolo[1,5-*a*][1,4]diazepine-5(6*H*)-yl)-8-fluoro-2-(methylthio)-6-(trifluoromethyl)quinazoline-7-yl)-5-fluorobenzo[*b*]thiophene-2-yl)carbamate (240 mg, 0.32 mmol) were added to DCM (5 mL) and stirred at room temperature for 1 h. The reaction completion was monitored by LCMS and TLC, and the reaction solution was poured into saturated $NaHCO_3$ (10 mL) solution, and extracted with EA (10 mL×3). The organic phase was collected, washed with saturated brine (20 mL), and dried over anhydrous $Na_2SO_4$, filtered and concentrated to yield a product, which was a

yellow solid tert-butyl (3-cyano-4-(4-(2-(dimethylcarbamoyl)-7,8-dihydro-4*H*-pyrazolo[1,5-*a*][1,4]diazepine-5(6*H*)-yl)-8-fluoro-2-(methylsulfinyl)-6-(trifluoromethyl)quinazoline-7-yl)-5-fluorobenzo[*b*]thiophene-2-yl)carbamate (240 mg, 98% yield). LCMS (m/z): 774.9 (M+H).

**Step C:** tert-Butyl (3-cyano-4-(2-(((3*S*,4*S*)-4-(difluoromethyl)-3-methyl-1-(methyl-*d*$_3$)piperidine-3-yl)methoxy)-4-(2-(dimethylcarbamoyl)-7,8-dihydro-4*H*-pyrazolo[1,5-*a*][1,4]diazepine-5(6*H*)-yl)-8-fluoro-6-(trifluoromethyl)quinazoline-7-yl)-5-fluorobenzo[*b*]thiophene-2-yl)carbamate

Under dry ice and ethanol bath conditions, tBuOK (0.39 mL, 1 M THF solution, 0.39 mmol) was added to a mixed solution of tert-butyl (3-cyano-4-(4-(2-(dimethylcarbamoyl)-7,8-dihydro-4H-pyrazolo[1,5-*a*][1,4]diazepine-5(6*H*)-yl)-8-fluoro-2-(methylsulfinyl)-6-(trifluoromethyl)quinazoline-7-yl)-5-fluorobenzo[*b*]thiophene-2-yl)carbamate (100 mg, 0.13 mmol), ((3*S*,4*S*)-4-(difluoromethyl)-3-methyl-1-(methyl-*d*$_3$)piperidine-3-yl)methanol (38 mg, 0.19 mmol) and THF (5 mL) and stirred for 1 h. After the reaction completion was monitored by LCMS and brought to room temperature, the solution was poured into a semi-saturated $NH_4Cl$ (30 mL) solution and extracted with EA (10 mL×3). The organic phase was collected, washed with saturated brine (30 mL), and dried over anhydrous $Na_2SO_4$. After filtration and concentration, a yellow solid tert-butyl (3-cyano-4-(2-(((3*S*,4*S*)-4-(difluoromethyl)-3-methyl-1-(methyl-*d*$_3$)piperidine-3-yl)methoxy)-4-(2-(dimethylcarbamoyl)-7,8-dihydro-4*H*-pyrazolo[1,5-*a*][1,4]diazepine-5(6*H*)-yl)-8-fluoro-6-(trifluoromethyl)quinazoline-7-yl)-5-fluorobenzo[*b*]thiophene-2-yl)carbamate (100 mg, yield 85%) was obtained. LCMS (m/z): 907.0 (M+H).

**Step D:** 5-(7-(2-amino-3-cyano-5-fluorobenzo[*b*]thiophene-4-yl)-2-(((3*S*,4*S*)-4-(difluoromethyl)-3-methyl-1-(methyl-*d*$_3$) piperidine-3-yl)methoxy)-8-fluoro-6-(trifluoromethyl)quinazoline-4-yl)-*N,N*-dimethyl-5,6,7,8-tetrahydro-4*H*-pyrazolo [1,5-*a*][1,4]diazepine-2-formamide

At room temperature, tert-butyl (3-cyano-4-(2-(((3*S*,4*S*)-4-(difluoromethyl)-3-methyl-1-(methyl-*d*$_3$)piperidine-3-yl)methoxy)-4-(2-(dimethylcarbamoyl)-7,8-dihydro-4*H*-pyrazolo[1,5*a*][1,4]diazepine-5(6*H*)-yl)-8-fluoro-6-(trifluoromethyl)quinazoline-7-yl)-5-fluorobenzo[b]thiophene-2-yl)carbamate (100 mg, 0.11 mmol) was added to a mixed solution of DCM (2 mL) and TFA (2 mL) and stirred at room temperature for 1 h. The reaction completion was monitored by LCMS. The reaction solution was concentrated and poured into saturated $NaHCO_3$ (10 mL), and extracted with EA (10 mL×3). After the organic phase was collected, washed with saturated brine (20 mL) and dried with anhydrous $Na_2SO_4$. After filtration and concentration, the solution was purified by pre-HPLC (C18, ACN/(10mmol $NH_4HCO_3/H_2O$)=50-65%) to obtain a white solid 5-(7-(2-amino-3-cyano-5-fluorobenzo[*b*]thiophene-4-yl)-2-(((35,45)-4-(difluoromethyl)-3-methyl-1-(methyl-*d*$_3$)piperidine-3-yl)methoxy)-8-fluoro-6-(trifluoromethyl)quinazoline-4-yl)-*N,N*-dimethyl-5,6,7,8-tetrahydro-4*H*-pyrazolo[1,5-*a*][1,4]diazepine-2-formamide (5 mg, yield 5.6%). LCMS (m/z): 807.3 (M+H). $^1$H NMR (400 MHz, DMSO-*d*$_6$) δ 8.20 (s, 1H), 8.03 - 7.93 (m, 2H), 7.89 - 7.78 (m, 1H), 7.16 - 7.05 (m, 1H), 6.72 - 6.61 (m, 1H), 6.49 - 6.07 (m, 1H), 5.25 - 5.13 (m, 1H), 5.05 - 4.88 (m, 1H), 4.58 - 4.39 (m, 4H), 4.38 - 4.24 (m, 1H), 4.22 - 4.04 (m, 1H), 3.26 (s, 3H), 2.95 (s, 3H), 2.88 - 2.76 (m, 2H), 2.41 - 2.26 (m, 2H), 2.05 - 1.92 (m, 1H), 1.89 - 1.78 (m, 1H), 1.74 - 1.58 (m, 3H), 1.13 - 1.04 (m, 3H). $^{19}$F NMR (376 MHz, DMSO-*d*$_6$) δ -59.03, -115.65, -117.70, -118.82, - 122.57.

**Example 68**

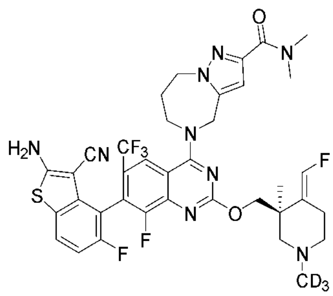

tert-Butyl (3-cyano-4-(4-(2-(dimethylcarbamoyl)-7,8-dihydro-4*H*-pyrazolo[1,5-*a*][1,4]diazepine-5(6*H*)-yl)-8-fluoro-2-(((*S,E*)-4-(fluoromethylene)-3-methyl-1-(methyl-*d*$_3$)piperidine-3-yl)methoxy)-6-(trifluoromethyl)quinazoline-7-yl)-5-fluorobenzo[*b*]thiophene-2-yl)carbamate

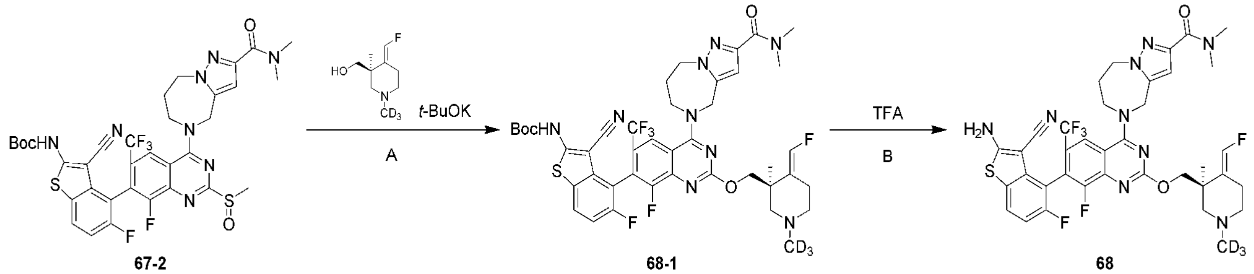


67-2 68-1 68

**Step A:** tert-butyl (3-cyano-4-(4-(2-(dimethylcarbamoyl)-7,8-dihydro-4*H*-pyrazolo[1,5-*a*][1,4]diazepine-5(6*H*)-yl)-8-fluoro-2-(((*S,E*)-4-(fluoromethylene)-3-methyl-1-(methyl-$d_3$)piperidine-3-yl)methoxy)-6-(trifluoromethyl)quinazoline-7-yl)-5-fluorobenzo[*b*]thiophene-2-yl)carbamate

Under dry ice and ethanol bath conditions, *t*BuOK (0.54 mL, 1 M THF solution, 0.54 mmol) was added to a mixed solution of tert-butyl (3-cyano-4-(4-(2-(dimethylcarbamoyl)-7,8-dihydro-4*H*-pyrazolo[1,5-*a*][1,4]diazepine-5(6*H*)-yl)-8-fluoro-2-(methylsulfinyl)-6-(trifluoromethyl)quinazoline-7-yl)-5-fluorobenzo[*b*]thiophene-2-yl)carbamate (140 mg, 0.18 mmol), (*S,E*)-(4-(fluoromethylene)-3-methyl-1-(methyl-$d_3$)piperidine-3-yl)methanol (48 mg, 0.27 mmol) and THF (5 mL) and stirred for 1 h. After the reaction completion was monitored by LCMS and brought to room temperature, the solution was poured into a semi-saturated $NH_4Cl$ (30 mL) solution and extracted with EA (10 mL×3). The organic phase was collected, washed with saturated brine (30 mL), and dried over anhydrous $Na_2SO_4$. After filtration and concentration, a yellow solid tert-butyl (3-cyano-4-(4-(2-(dimethylcarbamoyl)-7,8-dihydro-4*H*-pyrazolo[1,5-*a*][1,4]diazepine-5(6*H*)-yl)-8-fluoro-2-(((*S,E*)4-(fluoromethylene)-3-methyl-1-(methyl-$d_3$)piperidine-3-yl)methoxy)-6-(trifluoromethyl)quinazoline-7-yl)-5-fluorobenzo[*b*]thiophene-2-yl)carbamate (150 mg, yield 93%) was obtained. LCMS (m/z): 887.3 (M+H).

**Step B:** 5-(7-(2-amino-3-cyano-5-fluorobenzo[*b*]thiophene-4-yl)-8-fluoro-2-(((*S,E*)-4-(fluoromethylene)-3-methyl-1-(methyl-$d_3$)piperidine-3-yl)methoxy)-6-(trifluoromethyl)quinazoline-4-yl)-*N,N*-dimethyl-5,6,7,8-tetrahydro-4*H*-pyrazolo[1,5-*a*][1,4]diazepine-2-formamide

At room temperature, tert-butyl 3-cyano-4-(4-(2-(dimethylcarbamoyl)-7,8-dihydro-4*H*-pyrazolo[1,5-*a*][1,4]diazepine-5(6*H*)-yl)-8-fluoro-2-(((*S,E*)-4-(fluoromethylene)-3-methyl-1-(methyl-$d_3$)piperidine-3-yl)methoxy)-6-(trifluoromethyl)quinazoline-7-yl)-5-fluorobenzo[b]thiophene-2-yl)carbamate (150 mg, 0.17 mmol) was added to a mixed solution of DCM (2 mL) and TFA (2 mL), and stirred at room temperature for 1 h. The reaction completion was monitored by LCMS. The reaction solution was concentrated and poured into saturated $NaHCO_3$ (10 mL), extracted with EA (10 mL×3). The organic phase was collected, washed with saturated brine (20 mL), dried with anhydrous $Na_2SO_4$, and the organic phase was concentrated to obtain the crude product. Further purification by pre-HPLC (C18, ACN/(10mmol NH4$HCO_3$/$H_2O$) =50-65%) yielded a white solid 5-(7-(2-amino-3-cyano-5-fluorobenzo[b]thiophene-4-yl)-8-fluoro-2-(((*S,E*)-4-(fluoromethylene)-3-methyl-1-(methyl-$d_3$)piperidine-3-yl)methoxy)-6-(trifluoromethyl)quinazoline-4-yl)-*N,N*-dimethyl-5,6,7,8-tetrahydro-4*H*-pyrazolo[1,5-*a*][1,4]diazepine-2-formamide (8 mg, yield 6.4%). LCMS (m/z): 787.3 (M+H). $^1$H NMR (400 MHz, DMSO-$d_6$) δ 8.19 (s, 1H), 7.96 (s, 2H), 7.93 - 7.71 (m, 1H), 7.18 - 7.09 (m, 1H), 6.90 - 6.79 (m, 0.5H), 6.65 (s, 1H), 6.62 - 6.55 (m, 0.5H), 5.29 - 5.15 (m, 1H), 5.05 - 4.90 (m, 1H), 4.68 - 4.57 (m, 1H), 4.56 - 4.42 (m, 2H), 4.37 - 4.24 (m, 2H), 4.18 - 4.02 (m, 1H), 3.29 - 3.21 (m, 3H), 2.96 (s, 3H), 2.78 - 2.66 (m, 2H), 2.43 - 2.27 (m, 2H), 2.25 - 2.10 (m, 1H), 2.07 - 1.96 (m, 1H), 1.94 - 1.80 (m, 2H), 1.16 - 1.03 (m, 3H). $^{19}$F NMR (376 MHz, DMSO-$d_6$) δ -59.02, -117.67, -122.68, -138.71.

**Examples 68A and 68B**

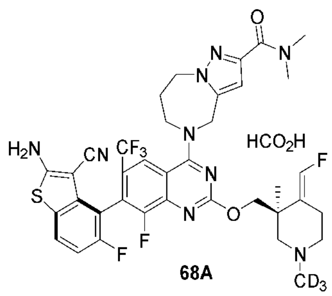

68A

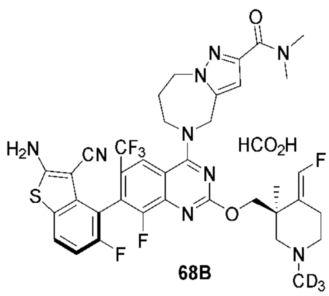

68B

Example 68 was resolved by pre-HPLC (C18, ACN/(0.1%FA/$H_2O$)=25-45%) to yield the first eluted isomer 1, which was Example 68A, with a relatively short retention time (Rt = 10.9 min, LCMS (m/z): 787.3 (M+H); and the subsequently eluted isomer 2, which was Example 68B, with a relatively long retention time (Rt = 11.8 min, LCM S (m/z): 787.3 (M+H).

| | | |
|---|---|---|
| 69 | 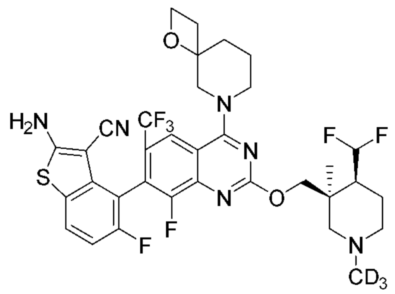 | LCMS (m/z): 726.3 (M+H) |
| 70 | 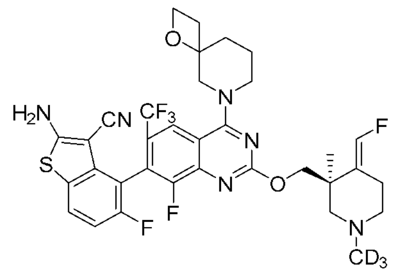 | LCMS (m/z): 706.2 (M+H) |
| 71 | 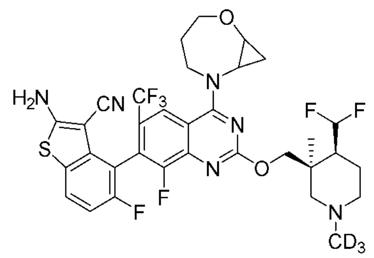 | LCMS (m/z): 712.2 (M+H) |
| 72 | 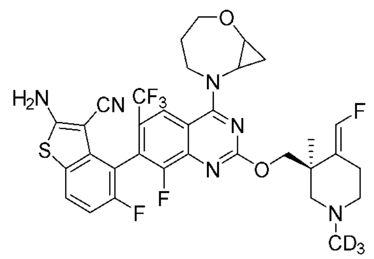 | LCMS (m/z): 692.2 (M+H) |
| 73 | 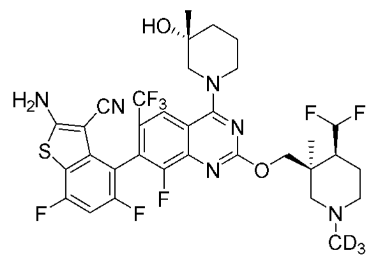 | LCMS (m/z): 732.2 (M+H) |

(continued)

| | | |
|---|---|---|
| 74 | 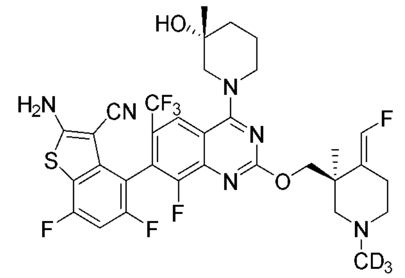 | LCMS (m/z): 712.2 (M+H) |
| 75 | 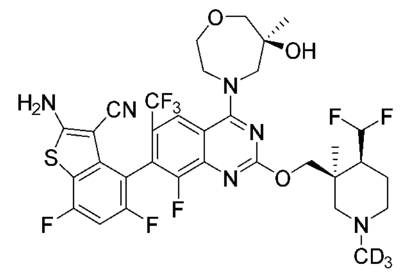 | LCMS (m/z): 748.2 (M+H) |
| 76 | 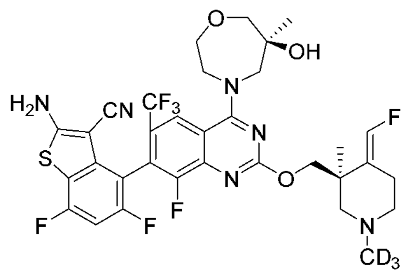 | LCMS (m/z): 728.2 (M+H) |
| 77 | 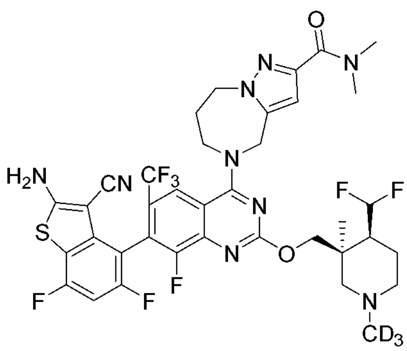 | LCMS (m/z): 825.3 (M+H) |
| 78 | 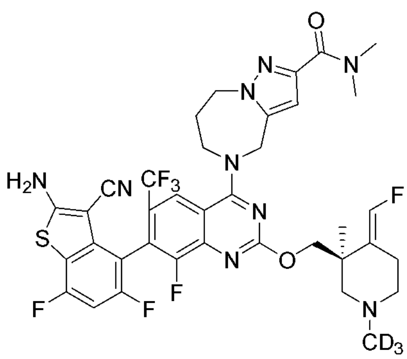 | LCMS (m/z): 805.3 (M+H) |

(continued)

| | | |
|---|---|---|
| 79 | 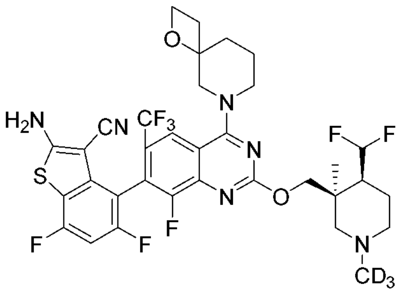 | LCMS (m/z): 744.2 (M+H) |
| 80 | 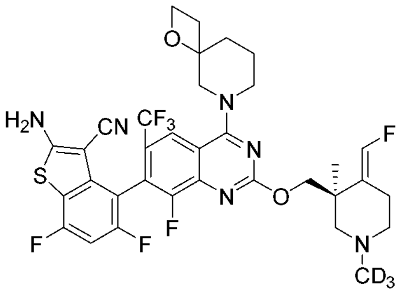 | LCMS (m/z): 724.2 (M+H) |
| 81 | 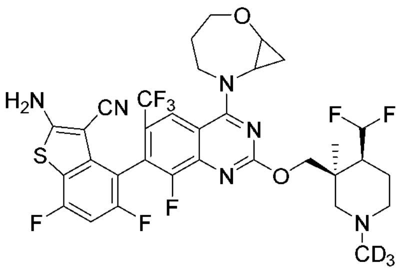 | LCMS (m/z): 730.2 (M+H) |
| 82 | 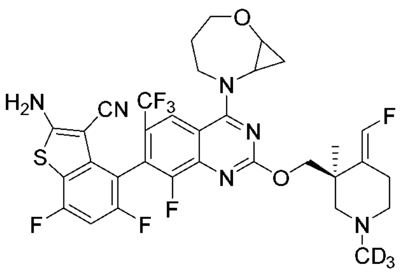 | LCMS (m/z): 710.2 (M+H) |

**Example 83**

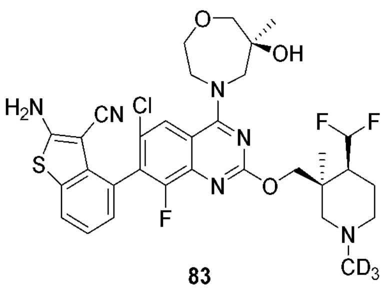

**83**

2-Amino-4-(6-chloro-2-(((3*S*,4*S*-4-(difluoromethyl)-3-methyl-1-(methyl-*d*$_3$)piperidine-3-yl)methoxy)-8-fluoro-4-((*S*)-6-hydroxy-6-methyl-1,4-oxazacycloheptane-4-yl)quinazoline-7-yl)benzo[b]thiophene-3-carbonitrile

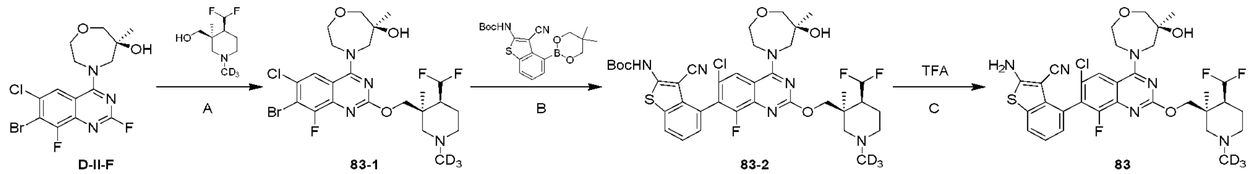


D-II-F 83-1 83-2 83

**Step A:** (*S*)-4-(7-Bromo-6-chloro-2-(((3*S*,4*S*)-4-(difluoromethyl)-3-methyl-1-(methyl-$d_3$)piperidine-3-yl)methoxy)-8-fluoroquinazoline-4-yl)-6-methyl-1,4-oxazacycloheptane-6-ol

At room temperature, NaH (73.4 mg, 1.84 mmol) was added to a mixed solution of ((3*S*,4*S*)-4-(difluoromethyl)-3-methyl-1-(methyl-$d_3$)piperidine-3-yl)methanol (144 mg, 0.734 mmol) and THF (10 mL) and stirred for 0.5 h. Then, (*S*)-4-(7-bromo-6-chloro-2,8-difluoro-4-quinazolinyl)-6-methyl-1,4-oxazacycloheptane-6-ol (250 mg, 0.612 mmol) was added to the reaction mixture and stirred for 1.0 h. The reaction completion was monitored by LCMS. The reaction mixture was poured into a saturated $NH_4Cl$ solution (50 mL) and extracted with EA (50 mL×3). The organic phase was collected, washed with saturated brine (70 mL), and dried over anhydrous $Na_2SO_4$. After filtration, the organic phase was concentrated and purified by FCC ($SiO_2$, EA/PE=0-80%) to give a brown solid product (*S*)-4-(7-bromo-6-chloro-2-(((3*S*,4*S*)-4-(difluoromethyl)-3-methyl-1-(methyl-$d_3$)piperidine-3-yl)methoxy)-8-fluoroquinazoline-4-yl)-6-methyl-1,4-oxazacycloheptane-6-ol (60 mg, yield 17%). LCMS (m/z): 584.1 (M+H) and 586.1 (M+H).

**Step B:** tert-Butyl (4-(6-chloro-2-(((3*S*,4*S*)-4-(difluoromethyl)-3-methyl-1-(methyl-$d_3$)piperidine-3-yl)methoxy)-8-fluoro-4-((*S*)-6-hydroxy-6-methyl-1,4-oxazacycloheptane-4-yl)quinazoline-7-yl)-3-cyanobenzo[*b*]thiophene-2-yl)carbamate

At room temperature, tert-butyl (*S*)-4-(7-bromo-6-chloro-2-(((3*S*,4*S*)-4-(difluoromethyl)-3-methyl-1-(methyl-$d_3$)piperidine-3-yl)methoxy)-8-fluoroquinazoline-4-yl)-6-methyl-1,4-oxazacycloheptane-6-ol (60.0 mg, 0.103 mmol), (3-cyano-4-(5,5-dimethyl-1,3,2-dioxaborolan-2-yl)benzo[*b*]thiophene-2-yl)carbamate (99.0 mg, 0.256 mmol), Pd-117 (7.35 mg, 0.010 μmol), and $Cs_2CO_3$ (100 mg, 0.308 mmol) were added sequentially to a toluene (5 mL) solution. After $N_2$ replacement three times, the mixture was warmed to 120°C and stirred for 2 hours. The reaction completion was monitored by LCMS. The reaction solution was then returned to room temperature, filtered through diatomite to remove the palladium catalyst, and the mother liquor was collected. The concentrated crude product was purified by FCC ($SiO_2$, EA/PE=0-90%) to obtain a brown solid tert-butyl (4-(6-chloro-2-(((3*S*,4*S*)-4-(difluoromethyl)-3-methyl-1-(methyl-$d_3$)piperidine-3-yl)methoxy)-8-fluoro-4-((*S*)-6-hydroxy-6-methyl-1,4-oxazacycloheptane-4-yl)quinazoline-7-yl)-3-cyanobenzo[*b*]thiophene-2-yl)carbamate (60 mg, yield 75%). LCMS (m/z): 778.3 (M+H).

**Step C:** 2-Amino-4-(6-chloro-2-(((3*S*,4*S*)-4-(difluoromethyl)-3-methyl-1-(methyl-$d_3$)piperidine-3-yl)methoxy)-8-fluoro-4-((*S*)-6-hydroxy-6-methyl-1,4-oxazacycloheptane-4-yl)quinazoline-7-yl)benzo[*b*]thiophene-3-carbonitrile

At room temperature, tert-butyl (4-(6-chloro-2-(((3*S*,4*S*)-4-(difluoromethyl)-3-methyl-1-(methyl-$d_3$)piperidine-3-yl)methoxy)-8-fluoro-4-((*S*)-6-hydroxy-6-methyl-1,4-oxazacycloheptane-4-yl)quinazoline-7-yl)-3-cyanobenzo[*b*]thiophene-2-yl)carbamate (60 mg, 0.077 mmol) was added to a mixed solution of TFA (1.0 mL) and DCM (1.0 mL) and stirred for 1 h . The reaction completion was monitored by LCMS. The reaction solution was concentrated and quenched by addition of saturated $NaHCO_3$ aqueous solution (50 mL), and extracted with EA (30 mL×3). The organic phase was collected, washed with saturated brine (70 mL), and dried over anhydrous $Na_2SO_4$. The crude product obtained by filtration and concentration of the organic phase was purified by pre-HPLC (C18, 0.1% $NH_4HCO_3$) to give a white solid 2-amino-4-(6-chloro-2-(((3*S*,4*S*)-4-(difluoromethyl)-3-methyl-1-(methyl-$d_3$)piperidine-3-yl)methoxy)-8-fluoro-4-((*S*)-6-hydroxy-6-methyl-1,4-oxazacycloheptane-4-yl)quinazoline-7-yl)benzo[*b*]thiophene-3-carbonitrile (13.6 mg, yield 26%). LCMS (m/z): 678.3 (M+H). $^1H$ NMR (400 MHz, DMSO-$d_6$) δ 8.59 - 8.39 (m, 1H), 7.92 - 7.74 (m, 3H), 7.29 - 7.21 (m, 1H), 7.18 (d, *J* = 7.5 Hz, 1H), 6.51 - 6.11 (m, 1H), 5.31 (d, *J* = 37.4 Hz, 1H), 4.51 - 4.36 (m, 2H), 4.33 - 4.19 (m, 1H), 4.18 - 4.06 (m, 1H), 4.03 - 3.80 (m, 3H), 3.80 - 3.68 (m, 1H), 3.55 (d, *J* = 5.5 Hz, 2H), 2.93 - 2.71 (m, 2H), 1.95 - 1.53 (m, 5H), 1.18 - 1.08 (m, 6H). $^{19}F$ NMR (376 MHz, DMSO-$d_6$) δ -115.24, -116.01, -118.50, -119.27, -122.73.

**Example 84**

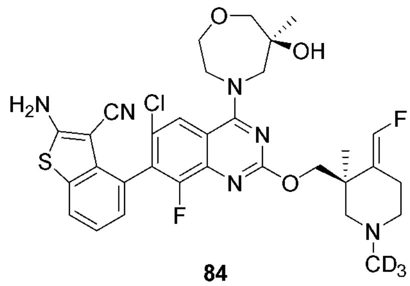

84

2-Amino-4-(6-chloro-8-fluoro-2-(((*S,E*)-4-(fluoromethylene)-3-methyl-1-(methyl-$d_3$)piperidine-3-yl)methoxy)-4-((*S*)-6-hydroxy-6-methyl-1,4-oxazacycloheptane-4-yl)quinazoline-7-yl)benzo[b]thiophene-3-carbonitrile

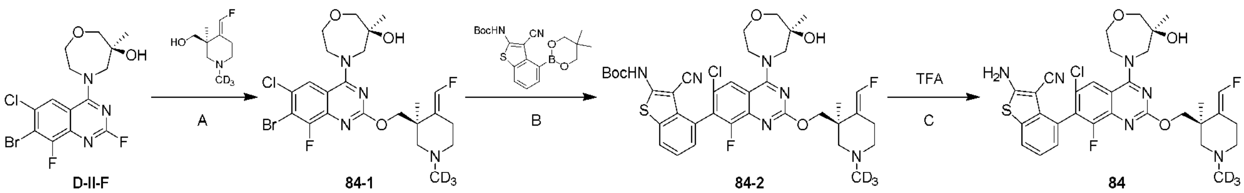


**Step A:** (*S*)-4-(7-bromo-6-chloro-8-fluoro-2-(((*S,E*)-4-(fluoromethylene)-3-methyl-1-(methyl-$d_3$)piperidine-3-yl)methoxy)quinazoline-4-yl)-6-methyl-1,4-oxazacycloheptane-6-ol

At room temperature, NaH (88.0 mg, 2.20 mmol) was added to a mixture of (*S,E*)-(4-(fluoromethylene)-3-methyl-1-(methyl-$d_3$)piperidine-3-yl)methanol (194 mg, 1.10 mmol) and THF (10 mL) and stirred for 0.5 h. Then, (*S*)-4-(7-bromo-6-chloro-2,8-difluoro-4-quinazolinyl)-6- methyl-1,4-oxazacycloheptane-6-ol (300 mg, 0.734 mmol) was added to the reaction mixture and stirred for 1.0 h. After the reaction completion was monitored by LCMS, the reaction solution was poured into a saturated solution of $NH_4Cl$ (50 mL), extracted with EA (50 mL×3), and the organic phase was collected. The mixture was then washed with saturated brine (70 mL), dried over anhydrous $Na_2SO_4$, and filtered. The crude product obtained after concentration of the organic phase was purified by FCC ($SiO_2$, EA/PE=0-80%) to give a brown solid product (S)-4-(7-bromo-6-chloro-8-fluoro-2-(((*S,E*)-4-(fluoromethylene)-3-methyl-1-(methyl-$d_3$)piperidine-3-yl)methoxy)quinazoline-4-yl)-6-methyl-1,4-oxazacycloheptane-6-ol (80 mg, yield 20%). LCMS (m/z): 564.0 (M+H) and 566.0 (M+H).

**Step B:** tert-Butyl (4-(6-chloro-8-fluoro-2-(((*S,E*)-4-(fluoromethylene)-3-methyl-1-(methyl-$d_3$)piperidine-3-yl)methoxy)-4-((*S*)-6-hydroxy-6-methyl-1,4-oxazacycloheptane-4-yl)quinazoline-7-yl)-3-cyanobenzo[*b*]thiophene-2-yl)carbamate

At room temperature, tert-butyl (*S*)-4-(7-bromo-6-chloro-8-fluoro-2-(((*S,E*)-4-(fluoromethylene)-3-methyl-1-(methyl-$d_3$)piperidine-3-yl)methoxy)quinazoline-4-yl)-6-methyl-1,4-oxazacycloheptane-6-ol (80.0 mg, 0.142 mmol), (3-cyano-4-(5,5-dimethyl-1,3,2-dioxaborolan-2-yl)benzo[*b*]thiophene-2-yl)carbamate (137 mg, 0.354 mmol), Pd-117 (10.1 mg, 0.014 μmol), and $Cs_2CO_3$ (138 mg, 0.425 mmol) were added sequentially to a 5 mL toluene solution. After $N_2$ replacement three times, the mixture was warmed to 120°C and stirred for 2 hours. The reaction completion was monitored by LCMS. The reaction solution was then returned to room temperature, filtered through diatomite to remove the palladium catalyst, and the mother liquor was collected. The concentrated crude product was purified by FCC ($SiO_2$, EA/PE=0-90%) to obtain a brown solid tert-butyl (4-(6-chloro-8-fluoro-2-(((*S,E*)-4-(fluoromethylene)-3-methyl-1-(methyl-$d_3$)piperidine-3-yl)methoxy)-4-((*S*)-6-hydroxy-6-methyl-1,4-oxazacycloheptane-4-yl)quinazoline-7-yl)-3-cyanobenzo[*b*]thiophene-2-yl)carbamate (60 mg, yield 56%). LCMS (m /z): 758.3 (M+H).

**Step C:** 2-Amino-4-(6-chloro-8-fluoro-2-(((*S,E*)-4-(fluoromethylene)-3-methyl-1-(methyl-$d_3$)piperidine-3-yl)methoxy)-4-((*S*)-6-hydroxy-6-methyl-1,4-oxazacycloheptane-4-yl)quinazoline-7-yl)benzo[*b*]thiophene-3-carbonitrile

At room temperature, tert-butyl (4-(6-chloro-8-fluoro-2-(((*S,E*)-4-(fluoromethylene)-3-methyl-1-(methyl-$d_3$)piperidine-3-yl)methoxy)-4-((*S*)-6-hydroxy-6-methyl-1,4-oxazacycloheptane-4-yl)quinazoline-7-yl)-3-cyanobenzo[*b*]thiophene-2-yl)carbamate (60 mg, 0.079 mmol) was added to a mixed solution of TFA (1.0 mL) and DCM (1.0 mL), and stirred for 1 h. The reaction completion was monitored by LCMS. The reaction solution was concentrated and quenched in saturated $NaHCO_3$ aqueous solution (50 mL). Extraction was performed using EA (30 mL×3). The organic phase was collected, washed with saturated brine (70 mL), and dried over anhydrous $Na_2SO_4$. After filtration, the organic phase was

concentrated, and then purified by pre-HPLC (C18, 0.1% $NH_4HCO_3$) to give a white solid 2-amino-4-(6-chloro-8-fluoro-2-(((*S,E*)-4-(fluoromethylene)-3-methyl-1-(methyl-*d*$_3$)piperidine-3-yl)methoxy)-4-((*S*)-6-hydroxy-6-methyl-1,4-oxazacycloheptane-4-yl)quinazoline-7-yl)benzo[*b*]thiophene-3-carbonitrile (5.0 mg, 10% yield). LCMS (m/z): 658.3 (M+H).

**Examples 85A and 85B**

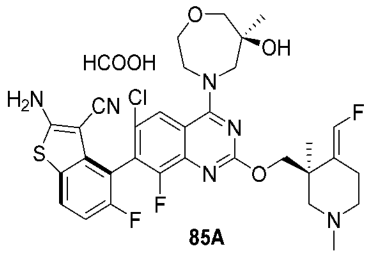

85A

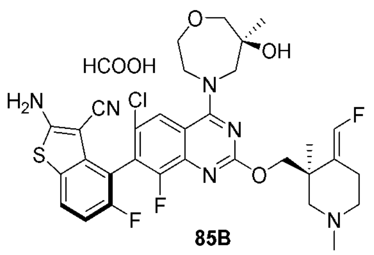

85B

(*Ra*)-2-amino-4-(6-chloro-8-fluoro-2-(((*S,E*)-4-(fluoromethylene)-1,3-dimethylpiperidine-3-yl)methoxy)-4-((*S*)-6-hydroxy-6-methyl-1,4-oxazacycloheptane-4-yl)quinazoline-7-yl)-5-fluorobenzo[*b*]thiophene-3-carbonitrile formate·formic acid salt (**85A**) and (*Sa*)-2-amino-4-(6-chloro-8-fluoro-2-(((*S,E*)-4-(fluoromethylene)-1,3-dimethylpiperidine-3-yl)methoxy)-4-((S)-6-hydroxy-6-methyl-1,4-oxazacycloheptane-4-yl)quinazoline-7-yl)-5-fluorobenzo[*b*]thiophene-3-carbonitrile formate·formic acid salt (**85B**)

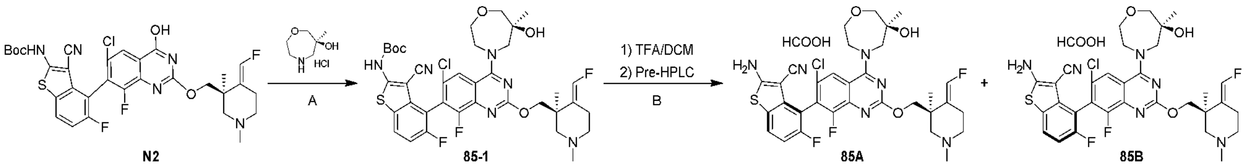


N2 85-1 85A 85B

**Step A:** tert-butyl (4-(6-chloro-8-fluoro-2-(((*S,E*)-4-(fluoromethylene)-1,3-dimethylpiperidine-3-yl)methoxy)-4-((*S*)-6-hydroxy-6-methyl-1,4-oxazacycloheptane-4-yl)quinazoline-7-yl)-3-cyano-5-fluorobenzo[*b*]thiophene-2-yl)carbamate

At room temperature, DIEA (176 mg, 1.36 mmol) was added to a mixture of tert-butyl (4-(6-chloro-8-fluoro-2-(((*S,E*)-4-(fluoromethylene)-1,3-dimethylpiperidine-3-yl)methoxy)-4-hydroxyquinazoline-7-yl)-3-cyano-5-fluorobenzo[*b*]thiophene-2-yl)carbamate (150 mg, 0.227 mmol), (*S*)-6-methyl-1,4-oxazacycloheptane-6-ol hydrochloride (75 mg, 0.46 mmol), BOP (201 mg, 0.455 mmol), and DMF (3 mL). The reaction mixture was warmed to 50°C and stirred overnight. The reaction completion was monitored by LCMS. The reaction solution was purified by FCC ($SiO_2$, EA/PE=0-100%) to obtain a yellow oily product tert-butyl (4-(6-chloro-8-fluoro-2-(((*S,E*)-4-(fluoromethylene)-1,3-dimethylpiperidine-3-yl) methoxy)-4-((S)-6-hydroxy-6-methyl-1,4-oxazacycloheptane-4-yl)quinazoline-7-yl)-3-cyano-5-fluorobenzo[b]thiophene-2-yl)carbamate (90 mg, yield 51%). LCMS (m/z): 773.0 (M+H).

**Step B:** (*Ra*)-2-amino-4-(6-chloro-8-fluoro-2-(((*S,E*)-4-(fluoromethylene)-1,3-dimethylpiperidine-3-yl)methoxy)-4-((*S*)-6-hydroxy-6-methyl-1,4-oxazacycloheptane-4-yl)quinazoline-7-yl)-5-fluorobenzo[*b*]thiophene-3-carbonitrile formate·formic acid salt (**85A**) and (S*a*)-2-amino-4-(6-chloro-8-fluoro-2-(((*S,E*)-4-(fluoromethylene)-1,3-dimethylpiperidine-3-yl)methoxy)-4-((S)-6-hydroxy-6-methyl-1,4-oxazacycloheptane-4-yl)quinazoline-7-yl)-5-fluorobenzo[*b*]thiophene-3-carbonitrile formate·formic acid salt (**85B**)

At room temperature, TFA/DCM (V/V=3:1, 10 mL) was added to tert-butyl (4-(6-chloro-8-fluoro-2-(((*S,E*)-4-(fluoromethylene)-1,3-dimethylpiperidine-3-yl)methoxy)-4-((S)-6-hydroxy-6-methyl-1,4-oxazacycloheptane-4-yl)quinazoline-7-yl)-3-cyano-5-fluorobenzo[*b*]thiophene-2-yl)carbamate (90 mg, 0.12 mmol) and stirred for 1 h. The reaction completion was monitored by LCMS. The crude product obtained after concentration was purified by pre-HPLC (C18, ACN/(0.1%FA/$H_2O$)=25-40%) to obtain the first eluted isomer 1, which was **Example 85A** (white solid, 8 mg, yield 20%, retention time 11.0 min). LCMS (m/z): 673.2 (M+H). $^1$H NMR (400 MHz, DMSO-*d*$_6$) δ 8.58 (s, 1H), 8.15 (s, 1H), 8.06 - 7.96 (m, 2H), 7.85 (dd, *J* = 8.7, 4.9 Hz, 1H), 7.20 - 7.10 (m, 1H), 6.95 - 6.57 (m, 1H), 5.41 - 5.21 (m, 1H), 4.57 (d, *J* = 10.6 Hz, 1H), 4.34 - 4.20 (m, 2H), 4.13 - 3.85 (m, 5H), 3.75 - 3.67 (m, 1H), 3.55 (s, 2H), 2.76 - 2.65 (m, 2H), 2.28 - 2.10 (m, 5H), 2.06 - 1.83 (m, 3H), 1.14 (s, 3H), 1.12 - 1.08 (m, 3H). $^{19}$F NMR (376 MHz, DMSO-*d*$_6$) δ -118.95, - 121.94, -138.68. The subsequently eluted isomer 2 was **Example 85B** (white solid, 8 mg, yield 20%, retention time 12.5 min). LCMS (m/z): 673.2

(M+H). $^1H$ NMR (400 MHz, DMSO-$d_6$) δ 8.65 (s, 1H), 8.14 (s, 1 H), 8.01 (s, 2H), 7.86 (dd, *J*= 8.8, 5.0 Hz, 1H), 7.21 - 7.08 (m, 1H), 6.98 - 6.62 (m, 1H), 5.35 (s, 1H), 4.62 - 4.54 (m, 1 H), 4.35 - 4.26 (m, 2H), 4.22 - 4.10 (m, 2 H), 3.99 - 3.92 (m, 2H), 3.80 (d, *J* = 14.8 Hz, 1H), 3.74 - 3.64 (m, 1H), 3.62 - 3.55 (m, 2H), 2.78 - 2.59 (m, 3 H), 2.47 - 2.16 (m, 5 H), 2.09 - 1.89 (m, 2 H), 1.23 - 1.13 (m, 6H).

**Examples 86, 86A and 86B**

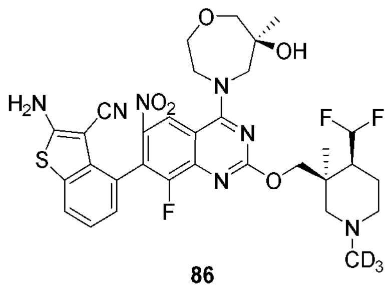
86

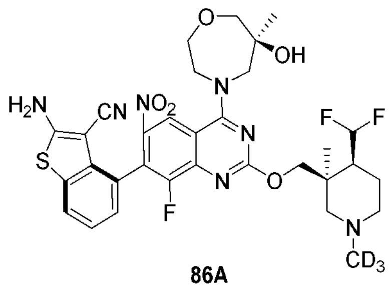
86A

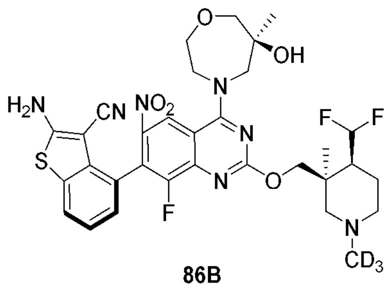
86B

4-(2-(((3*S*,4*S*)-4-(difluoromethyl)-1-((deuterated methyl)-3-methyl-3-piperidinyl)methoxy)-4-((S)-6-hydroxy-6-methyl-1,4-oxycyclopentane-4-yl)-8-fluoro-6-nitro-7-quinazolinyl)-2-amino-1-benzothiophene-3-carbonitrile (86), (*Ra*)-4-(2-(((3*S*,4*S*)-4-(difluoromethyl)-1-((deuterated methyl)-3-methyl-3-piperidinyl)methoxy)-4-((*S*)-6-hydroxy-6-methyl-1,4-oxycyclopentane-4-yl)-8-fluoro-6-nitro-7-quinazolinyl)-2-amino-1-benzothiophene-3-carbonitrile (**86A**) and (*Sa*)-4-(2-(((3*S*,4*S*)-4-(difluoromethyl)-1-((deuterated methyl)-3-methyl-3-piperidinyl)methoxy)-4-((*S*)-6-hydroxy-6-methyl-1,4-oxycycloheptane-4-yl)-8-fluoro-6-nitro-7-quinazolinyl)-2-amino-1-benzothiophene-3-carbonitrile (**86B**)

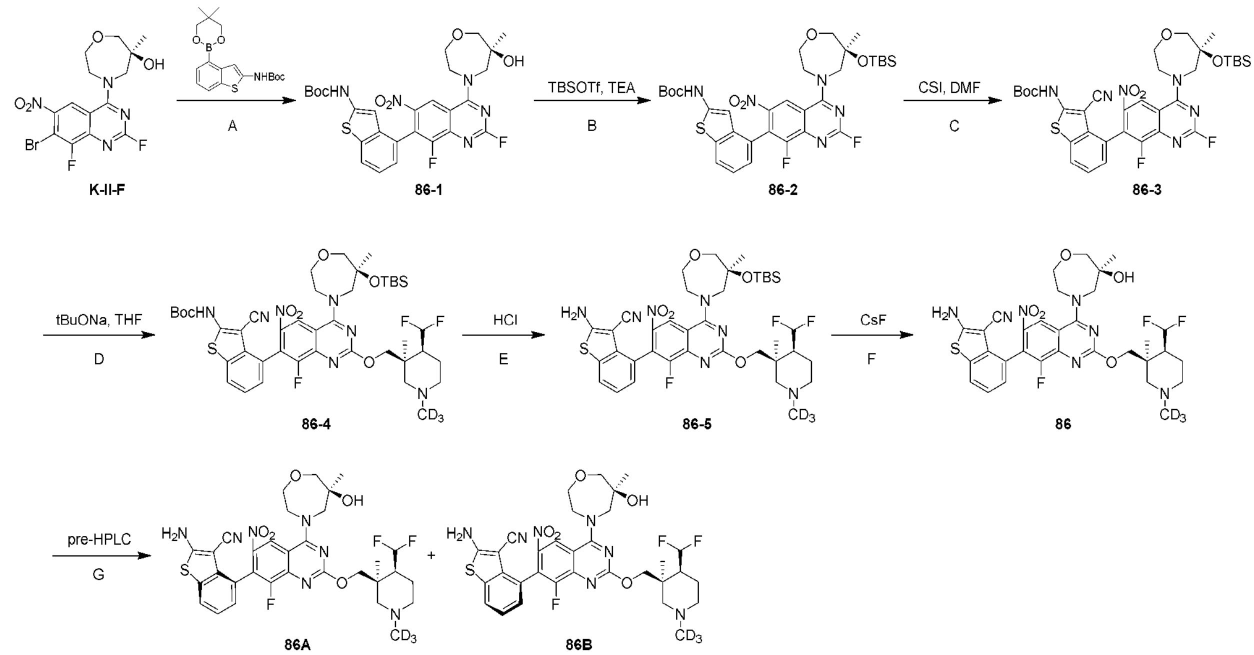


**Step A:** 4-(4-((*S*)-6-hydroxy-6-methyl-1,4-oxycycloheptane-4-yl)-2,8-difluoro-6-nitro-7-quinazolinyl)-2-((tert-butyl)(oxycarbonylamino))-1-benzothiophene

At room temperature, tert-butyl (*S*)-4-(7-bromo-2,8-difluoro-6-nitro-4-quinazolinyl)-6-methyl-1,4-oxazin-6-ol (700 mg, 1.67 mmol), (4-(5,5-dimethyl-1,3,2-dioxaborhexane-2-yl) benzothiophene-2-yl)carbamate (723 mg, 2.0 mmol), DPEphosPdCl$_2$ (119 mg, 0.16 μmol), and $K_3PO_4$ (845 mg, 3.99 mmol) were added sequentially was added to a solution of 1,4-dioxane (8 mL) and water (2 mL). After $N_2$ replacement three times, the mixture was warmed to 85°C and stirred for 2 h. The reaction completion was monitored by LCMS. The reaction solution was then returned to room temperature, filtered through diatomite, and the mother liquor was collected. Water (50 mL) was added, and the mixture was extracted with EA (50 mL×3). The organic phase was washed with saturated brine (30 mL), dried over anhydrous $Na_2SO_4$, and concen-

trated. The crude product was purified by FCC ($SiO_2$, EA/PE=0-60%) to give a yellow solid 4-(4-((*S*)-6-hydroxy-6-methyl-1,4-oxycyclopentane-4-yl)-2,8-difluoro-6-nitro-7-quinazolinyl)-2-((tert-butyl)(oxycarbonylamino))-1-benzothiophene (520 mg, yield 53%). LCMS (m/z): 588.0 (M+H).

**Step B:** 4-(4-((*S*)-6-((tert-butyl)bis(methyl)siloxy)-6-methyl-1,4-oxacyclopentane-4-yl)-2,8-difluoro-6-nitro-7-quinazolinyl)-2-((tert-butyl)(oxycarbonylamino))-1-benzothiophene

At room temperature, tert-butyl dimethoxy trifluoromethanesulfonate (945 mg, 3.57 mmol) was added to a solution of 4-(4-((*S*)-6-hydroxy-6-methyl-1,4-oxacyclopentane-4-yl)-2,8-difluoro-6-nitro-7-quinazolinyl)-2-((tert-butyl)(oxycarbonylamino))-1-benzothiophene (700 mg,1.19 mmol) in DCM (10 mL) and stirred for 20 h. After the reaction completion was monitored by LCMS, the reaction solution was poured into $H_2O$ (50 mL), extracted with EA (20 mL×3), and the organic phase was collected. The mixture was washed with saturated brine (10 mL), dried over anhydrous $Na_2SO_4$, and the organic phase was concentrated. The crude product was purified by FCC ($SiO_2$, EA/PE=0-15%) to obtain a yellow solid product 4-(4-((*S*)-6-((tert-butyl)bis(methyl)siloxy)-6-methyl-1,4-oxacyclopentane-4-yl)-2,8-difluoro-6-nitro-7-quinazolinyl)-2-((tert-butyl)(oxycarbonylamino))-1-benzothiophene (750 mg, yield 89%). LCMS (m/z): 702.3 (M+H).

**Step C:** 4-(4-((*S*)-6-((tert-butyl)bis(methyl)siloxy)-6-methyl-1,4-oxacyclopentane-4-yl)-2,8-difluoro-6-nitro-7-quinazolinyl)-2-((tert-butyl)(oxycarbonylamino))-1-benzothiophene-3-carbonitrile

Under ice bath conditions, chlorosulfonyl isocyanate (472 mg, 3.0 mmol) was added to a solution of 4-(4-((*S*)-6-((tert-butyl)bis(methyl)siloxy)-6-methyl-1,4-oxacyclopentane-4-yl)-2,8-difluoro-6-nitro-7-quinazolinyl)-2-((tert-butyl)(oxycarbonylamino))-1-benzothiophene (700 mg, 1.0 mmol) in acetonitrile (10 mL). After stirring for 1 h while maintaining the temperature, DMF (0.5 mL) was added, and the reaction continued for 0.5 h while maintaining the ice bath conditions. The reaction completion was monitored by LCMS. Saturated sodium bicarbonate solution (30 mL) was added to the reaction solution, and the mixture was extracted with EA (20 mL×3). The organic phase was collected, washed with saturated brine (10 mL), dried over anhydrous $Na_2SO_4$, and concentrated. The crude product was purified by FCC ($SiO_2$, EA/PE=0-20%) to obtain a yellow solid product 4-(4-((S)-6-((tert-butyl)bis(methyl)siloxy)-6-methyl-1,4-oxacyclopentane-4-yl)-2,8-difluoro-6-nitro-7-quinazolinyl)-2-((tert-butyl)(oxycarbonylamino))-1-benzothiophene-3-carbonitrile (180 mg, yield 25%). LCMS (m/z): 727.2 (M+H).

**Step D:** 4-(2-(((3*S*,4*S*)-4-(difluoromethyl)-1-((deuterated methyl)-3-methyl-3-piperidinyl)methoxy)-4-((*S*)-6-((tert-butyl)bis(methyl)siloxy)-6-methyl-1,4-oxacyclopentene-4-yl)-8-fluoro-6-nitro-7-quinazolinyl)-2-((tert-butyl)(oxycarbonylamino))-1-benzothiophene-3-carbonitrile

Under dry ice and ethanol bath conditions, tBuONa (0.24 mL, 1M THF solution, 0.22 mmol) was added to a solution of 4-(4-((*S*)-6-((tert-butyl)bis(methyl)siloxy)-6-methyl-1,4-oxacyclopentane-4-yl)-2,8-difluoro-6-nitro-7-quinazolinyl)-2-((tert-butyl)(oxycarbonylamino))-1-benzothiophene-3-carbonitrile (90 mg, 0.12 mmol) and ((3*S*,4*S*)-4-(difluoromethyl)-1,3-dimethylpiperidine-3-yl)methanol (36 mg, 0.18 mmol) in THF (2 mL) and stirred for 0.5 h. After the reaction completion was monitored by LCMS, a semi-saturated $NH_4Cl$ (30 mL) solution was added, and the mixture was extracted with EA (20 mL×2). The organic phase was collected, washed with saturated brine (30 mL), dried over anhydrous $Na_2SO_4$, and concentrated to obtain a yellow oily crude product 4-(2-(((3*S*,4*S*)-4-(difluoromethyl)-1-((deuterated methyl)-3-methyl-3-piperidinyl)methoxy)-4-((*S*)-6-((tert-butyl)bis(methyl)siloxy)-6-methyl-1,4-oxacyclopentene-4-yl)-8-fluoro-6-nitro-7-quinazolinyl)-2-((tert-butyl)(oxycarbonylamino))-1-benzothiophene-3-carbonitrile (95 mg, yield 85%). LCMS (m/z): 903.4 (M+H).

**Step E:** 4-(2-(((3*S*,4*S*)-4-(difluoromethyl)-1-((deuterated methyl)-3-methyl-3-piperidinyl)methoxy)-4-((*S*)-6-((tert-butyl)bis(methyl)siloxy)-6-methyl-1,4-oxacyclopentene-4-yl)-8-fluoro-6-nitro-7-quinazolinyl)-2-amino-1-benzothiophene-3-carbonitrile

At room temperature, TFA (0.5 mL) was added to a solution of compound 4-(2-(((3*S*,4*S*)-4-(difluoromethyl)-1-((deuterated methyl)-3-methyl-3-piperidinyl)methoxy)-4-((*S*)-6-((tert-butyl)bis(methyl)silyloxy)-6-methyl-1,4-oxacyclopentene-4-yl)-8-fluoro-6-nitro-7-quinazolinyl)-2-((tert-butyl)(oxycarbonylamino))-1-benzothiophene-3-carbonitrile (95 mg, 0.1 mmol) in DCM (1.5 mL). The mixture was stirred for 1 h while maintaining the temperature. After the reaction completion was monitored by LCMS, the reaction solution was concentrated to obtain a yellow oily crude product 4-(2-(((3*S*,4*S*)-4-(difluoromethyl)-1-((deuterated methyl)-3-methyl-3-piperidinyl)methoxy)-4-((*S*)-6-((tert-butyl)bis(methyl)siloxy)-6-methyl-1,4-oxacyclopentene-4-yl)-8-fluoro-6-nitro-7-quinazolinyl)-2-amino-1-benzothiophene-3-carbonitrile (120 mg). LCMS (m/z): 803.3 (M+H).

**Step F:** 4-(2-(((3*S*,4*S*)-4-(difluoromethyl)-1-((deuterated methyl)-3-methyl-3-piperidinyl)methoxy)-4-((*S*)-6-hydroxy-6-methyl-1,4-oxacyclopentene-4-yl)-8-fluoro-6-nitro-7-quinazolinyl)-2-amino-1-benzothiophene-3-carbonitrile (**86**)

At room temperature, CsF (454 mg, 3.0 mmol) was added to a solution of compound 4-(2-(((3*S*,4*S*)-4-(difluoromethyl)-1-((deuterated methyl)-3-methyl-3-piperidinyl)methoxy)-4-((S)-6-((tert-butyl)bis(methyl)siloxy)-6-methyl-1,4-oxacyclopentene-4-yl)-8-fluoro-6-nitro-7-quinazolinyl)-2-amino-1-benzothiophene-3-carbonitrile (120 mg, 0.15 mmol) in DMF (2.0 mL) and the mixture was warmed to 55°C and stirred for 6 h. After the reaction was completion monitored by LCMS, 0.5 mL of the reaction solution was purified by pre-HPLC (C18, ACN/(10 mmol $NH_4HCO_3/H_2O$)=40-80%), to yield a white solid product 4-(2-(((3S,4S)-4-(difluoromethyl)-1-((deuterated methyl)-3-methyl-3-piperidinyl)methoxy)-4-((S)-6-hydroxy-6-methyl-1,4-oxacyclopentene-4-yl)-8-fluoro-6-nitro-7-quinazolinyl)-2-amino-1-benzothiophene-3-carbonitrile (3 mg). LCMS (m/z): 689.2 (M+H). $^1$H NMR (400 MHz, DMSO-$d_6$) δ 9.53 - 9.21 (m, 1H), 7.84 (s, 2H), 7.77 (d, *J* = 7.7 Hz, 1H), 7.28 - 7.06 (m, 2H). 6.30 (t, *J* = 55.6 Hz, 1H), 5.42 - 5.23 (m, 1H), 4.54 - 4.41 (m, 2H), 4.37 - 4.01 (m, 4H), 4.00 - 3.88 (m, 2H), 3.82 - 3.72 (m, 1H), 3.61 - 3.55 (m, 2H), 2.91 - 2.74 (m, 2H), 1.89 - 1.74 (m, 2H), 1.68 - 1.57 (m, 2H), 1.23 (s, 3H), 1.11 (s, 3H). $^{19}$F NMR (376 MHz, DMSO-$d_6$) δ -115.05 - -119.89 (m), -121.71 - -123.20 (m).

**Step G:** (*Ra*)-4-(2-(((3*S*,4*S*)-4-(difluoromethyl)-1-((deuterated methyl)-3-methyl-3-piperidinyl)methoxy)-4-((*S*)-6-hydroxy-6-methyl-1,4-oxycycloheptane-4-yl)-8-fluoro-6-nitro-7-quinazolinyl)-2-amino-1-benzothiophene-3-carbonitrile (**86A**) and (*Sa*)-4-(2-(((3*S,4S*)-4-(difluoromethyl)-1-((deuterated methyl)-3-methyl-3-piperidinyl)methoxy)-4-((*S*)-6-hydroxy-6-methyl-1,4-oxycycloheptane-4-yl)-8-fluoro-6-nitro-7-quinazolinyl)-2-amino-1-benzothiophene-3-carbonitrile (**86B**)

The 1.5 mL reaction solution from step F was purified by pre-HPLC (C18, ACN/(0.1% FA /$H_2O$)=25-38%) to obtain the first eluted isomer 1, which was Example **86A** (white solid, 7.1mg, yield 6.9%, retention time 11.0min). LCMS (m/z): 689.2 (M+H). $^1$H NMR (400 MHz, DMSO-$d_6$) δ 9.44 - 9.25 (m, 1H), 8.20 (s, 1H), 7.85 (s, 1H), 7.77 (dd, *J* = 7.6, 1.6 Hz, 1H), 7.26 - 7.13 (m, 2H), 6.29 (t, *J* = 55.5 Hz, 1H), 5.35 (d, *J* = 33.2 Hz, 1H), 4.51 - 4.39 (m, 2H), 4.36 - 4.24 (m, 1H), 4.23 - 4.13 (m, 1H), 4.12 - 4.04 (m, 1H), 4.01 - 3.93 (m, 2H), 3.82 - 3.72 (m, 2H), 3.59 - 3.56 (m, 2H), 2.87 - 2.76 (m, 2H), 1.88 - 1.71 (m, 2H), 1.68 - 1.57 (m, 2H), 1.24 - 1.08 (m, 6H). $^{19}$F NMR (376 MHz, DMSO-$d_6$) δ -114.89 - -119.98 (m), -123.48 (d, *J*= 681.4 Hz); the subsequently eluted isomer 2 was Example **86B** (white solid, 5.7 mg, yield 5.5%, retention time 13.5 min). LCMS (m/z): 689.3 (M+H). $^1$H NMR (400 MHz, DMSO-$d_6$) δ 9.44 (s, 1H), 8.24 (s, 1H), 7.85 (s, 2H), 7.77 (d, *J* = 7.7 Hz, 1H), 7.22 - 7.15 (m, 1H), 7.11 (d, *J= 7.4* Hz, 1H), 6.29 *(t, J=*55.6 Hz, 1H), 5.38 (s, 1H), 4.53 - 4.42 (m, 2H), 4.32 - 4.19 (m, 2H), 4.11 - 4.04 (m, d6) 1H), 3.98 - 3.91 (m, 2H), 3.79 - 3.70 (m, 2H), 3.59 - 3.57 (m, 2H), 2.86 - 2.79 (m, 2H), 1.87 - 1.72 (m, 2H), 1.69 - 1.59 (m, 2H), 1.21 (s, 3H), 1.11 (s, 3H). $^{19}$F NMR (376 MHz, DMSO-$d_6$) δ -113.88 - -120.65 (m), -122.43.

**Examples 87, 87A and 87B**

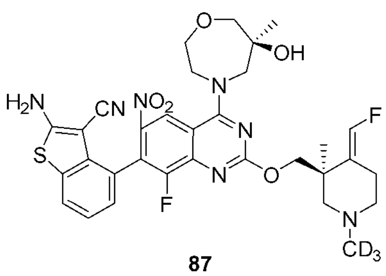

87

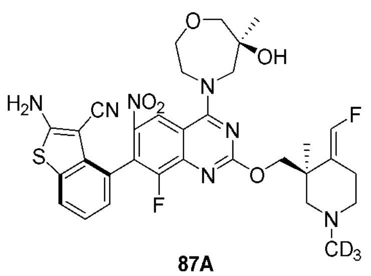

87A

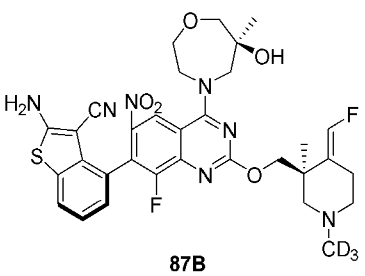

87B

4-(2-(((*S*)-(*E*)-4-fluoromethylene-1-((deuterated methyl)-3-methyl-3-piperidinyl)methoxy)-4-((*S*)-6-hydroxy-6-methyl-1,4-oxycycloheptane-4-yl)-8-fluoro-6-nitro-7-quinazolinyl)-2-amino-1-benzothiophene-**3**-carbonitrile(**87**), (*Ra*)-4-(2-(((*S*)-(E)-4-fluoromethylene-1-((deuterated methyl)-3-methyl-3-piperidinyl)methoxy)-4-((*S*)-6-hydroxy-6-methyl-1,4-oxycycloheptane-4-yl-8-fluoro-6-nitro-7-quinazolinyl)-2-amino-1-benzothiophene-3-carbonitrile(**87A**), and (*Sa*)-4-(2-(((*S*)-(E)-4-fluoromethylene-1-((deuterated methyl)-3-methyl-3-piperidinyl)methoxy)-4-((*S*)-6-hydroxy-6-methyl-1,4-oxacyclopentene-4-yl)-8-fluoro-6-nitro-7-quinazolinyl)-2-amino-1-benzothiophene-3-carbonitrile (**87B**)

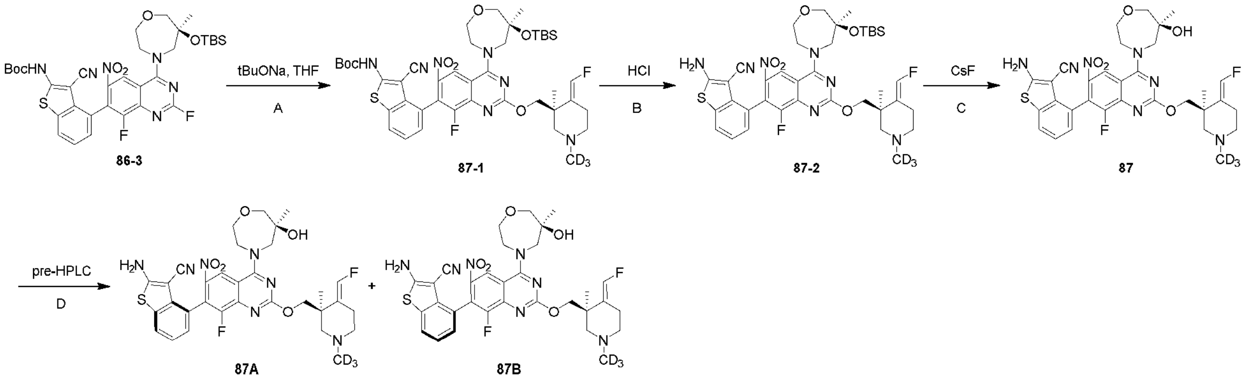


86-3 87-1 87-2 87

87A 87B

**Step A:** 4-(2-(((3*S*,4*S*)-(*E*)-4-fluoromethylene-1-((deuterated methyl)-3-methyl-3-piperidinyl)methoxy)-4-((*S*)-6-((tert-butyl)bis(methyl)silyloxy)-6-methyl-1,4-oxacyclopentene-4-yl)-8-fluoro-6-nitro-7-quinazolinyl)-2-((tert-butyl)(oxycarbonylamino))-1-benzothiophene-3-carbonitrile

Under dry ice and ethanol bath conditions (-70°C), *t*BuONa (0.22 mL, 1 M THF solution, 0.22 mmol) was added to a solution of 4-(4-((S)-6-((tert-butyl)bis(methyl)siloxy)-6-methyl-1,4-oxacyclopentane-4-yl)-2,8-difluoro-6-nitro-7-quinazolinyl)-2-((tert-butyl)(oxycarbonylamino))-1-benzothiophene-3-carbonitrile (80 mg, 0.11 mmol) and ((*S*)-(E)-4-fluoromethylene-1-((deuterated methyl)-3-methyl-3-piperidinyl)methanol (30 mg, 0.16 mmol) in THF (2 mL) and stirred for 0.5 h. After the reaction completion was monitored by LCMS, a semi-saturated $NH_4Cl$ solution (30 mL) was added, extracted with EA (20 mL×3), the organic phase was collected, washed with saturated brine (30 mL), dried over anhydrous $Na_2SO_4$, and concentrated to give a yellow oily crude product 4-(2-(((3*S*,4*S*)-(E)-4-fluoromethylene-1-((deuterated methyl)-3-methyl-3-piperidinyl)methoxy)-4-((*S*)-6-((tert-butyl)bis(methyl)silyloxy)-6-methyl-1,4-oxacyclopentene-4-yl)-8-fluoro-6-nitro-7-quinazolinyl)-2-((tert-butyl)(oxycarbonylamino))-1-benzothiophene-3-carbonitrile (82 mg, yield 84%). LCMS (m/z): 883.3 (M+H).

**Step B:** 4-(2-(((3*S*,4*S*)-(*E*)-4-fluoromethylene-1-((deuterated methyl)-3-methyl-3-piperidinyl)methoxy)-4-((*S*)-6-((tert-butyl)bis(methyl)silyloxy)-6-methyl-1,4-oxacyclopentene-4-yl)-8-fluoro-6-nitro-7-quinazolinyl)-2-amino-1-benzothiophene-3-carbonitrile

At room temperature, TFA (0.5 mL) was added to a solution of Compound 4-(2-(((3*S*,4*S*)-(*E*)-4-fluoromethylene-1-((deuterated methyl)-3-methyl-3-piperidinyl)methoxy)-4-((*S*)-6-((tert-butyl)bis(methyl)silyloxy)-6-methyl-1,4-oxacyclopentene-4-yl)-8-fluoro-6-nitro-7-quinazolinyl)-2-((tert-butyl)(oxycarbonylamino))-1-benzothiophene-3-carbonitrile(82 mg, 0.09 mmol) in DCM (1.5 mL) and the mixture was stirred for 1 h. After the reaction completion was monitored by LCMS, the reaction solution was concentrated to obtain a yellow oily crude product 4-(2-(((3*S*,4*S*)-(E)-4-fluoromethylene-1-((deuterated methyl)-3-methyl-3-piperidinyl)methoxy)-4-((*S*)-6-((tert-butyl)bis(methyl)silyloxy)-6-methyl-1,4-oxacyclopentene-4-yl)-8-fluoro-6-nitro-7-quinazolinyl)-2-amino-1-benzothiophene-3-carbonitrile (130 mg). LCMS (m/z): 783.3 (M+H).

**Step C:** 4-(2-(((*S*)-(E)-4-fluoromethylene-1-((deuterated methyl)-3-methyl-3-piperidinyl)methoxy)-4-((*S*)-6-hydroxy-6-methyl-1,4-oxacyclopentene-4-yl)-8-fluoro-6-nitro-7-quinazolinyl)-2-amino-1-benzothiophene-3-carbonitrile (**86**)

At room temperature, CsF (50.4 mg, 3.3 mmol) was added to a solution of compound 4-(2-(((3S,4S)-(E)-4-fluoromethylene-1-((deuterated methyl)-3-methyl-3-piperidinyl)methoxy)-4-((S)-6-((tert-butyl)bis(methyl)silyloxy)-6-methyl-1,4-oxacyclopentene-4-yl)-8-fluoro-6-nitro-7-quinazolinyl)-2-amino-1-benzothiophene-3-carbonitrile (130 mg, 0.16 mmol) in DMF (2.0 mL), and the mixture was warmed to 55°C and stirred for 6 h. After the reaction completion was monitored by LCMS, 0.5 mL of the reaction solution was purified by pre-HPLC (C18, ACN/(10 mmol $NH_4HCO_3/H_2O$) =40-80%) to yield a white solid product 4-(2-(((S)-(E)-4-fluoromethylene-1-((deuterated methyl)-3-methyl-3-piperidinyl)methoxy)-4-((S)-6-hydroxy-6-methyl-1,4-oxacyclopentene-4-yl)-8-fluoro-6-nitro-7-quinazolinyl)-2-amino-1-benzothiophene-3-carbonitrile (4 mg). LCMS (m/z): 669.3 (M+H). $^1H$ NMR (400 MHz, DMSO-$d_6$) δ 9.53 - 9.25 (m, 1H), 7.84 (s, 2H), 7.79 - 7.73 (m, 1H), 7.23 - 7.10 (m, 2H), 6.86 - 6.57 (m, 1H), 5.44 - 5.26 (m, 1H), 4.68 - 4.56 (m, 1H), 4.39 - 4.01 (m, 4H), 4.01 - 3.86 (m, 2H), 3.81 - 3.68 (m, 1H), 3.66 - 3.50 (m, 2H), 2.75 - 2.64 (m, 2H), 2.56 - 2.51 (m, 1H), 2.27 - 2.11 (m, 1H), 1.96 - 1.79 (m, 2H), 1.25 - 1.21 (m, 3H), 1.11 (s, 3H). $^{19}F$ NMR (376 MHz, DMSO-$d_6$) δ -120.72 - -125.05, -136.09 - -145.14.

**Step D:** (*Ra*)-4-(2-(((*S*)-(E)-4-fluoromethylene-1-((deuterated methyl)-3-methyl-3-piperidinyl)methoxy)-4-((*S*)-6-hydroxy-6-methyl-1,4-oxycycloheptane-4-yl)-8-fluoro-6-nitro-7-quinazolinyl)-2-amino-1-benzothiophene-3-carbonitrile (**87A**) and (*Sa*)-4-(2-(((*S*)-(E)-4-fluoromethylene-1-((deuterated methyl)-3-methyl-3-piperidinyl)methoxy)-4-((*S*)-6-hydroxy-6-methyl-1,4-oxycycloheptane-4-yl)-8-fluoro-6-nitro-7-quinazolinyl)-2-amino-1-benzothiophene-3-carbonitrile (**87B**)

The reaction solution obtained in step C was purified by pre-HPLC (C 18, ACN/(0.1% FA /$H_2O$)=25-45%) to obtain the first eluted isomer 1, which was Example 87A (white solid product, 6.3 mg, yield 5.6%, retention time 11.0 min). LCMS (m/z): 669.3 (M+H). $^1$H NMR (400 MHz, DMSO-$d_6$) δ 9.35 (s, 1H), 8.25 (s, 1H), 7.85 (s, 2H), 7.77 (d, *J* = 7.5 Hz, 1H), 7.24 - 7.13 (m, 2H), 6.72 (d, *J* = 86.7 Hz, 1H), 5.34 (s, 1H), 4.61 (d, *J* = 10.6 Hz, 1H), 4.33 (d, *J* = 10.4 Hz, 1H), 4.30 - 4.13 (m, 2H), 4.12 - 3.90 (m, 4H), 3.77 - 3.70 (m, 2H), 2.71 - 2.63 (m, 2H), 2.55 - 2.51 (m, 1H), 2.26 - 2.13 (m, 1H), 1.94 - 1.80 (m, 2H), 1.22 - 1.09 (m, 6H). $^{19}$F NMR (376 MHz, DMSO-$d_6$) δ -122.74, - 138.67. The subsequently eluted isomer 2 was Example 87B (white solid, 6.4 mg, yield 5.7%, retention time 12.3 min). LCMS (m/z): 669.3 (M+H). $^1$H NMR (400 MHz, DMSO-$d_6$) δ 9.47 (s, 1H), 8.25 (s, 1H), 7.86 (s, 2H), 7.77 (d, *J* = 7.7 Hz, 1H), 7.23 - 7.14 (m, 1H), 7.11 (d, *J* = 7.4 Hz, 1H), 6.71 (d, *J* = 86.3 Hz, 1H), 5.40 (s, 1H), 4.63 (d, *J* = 10.6 Hz, 1H), 4.32 (d, *J* = 10.7 Hz, 1H), 4.28 - 4.18 (m, 2H), 4.11 - 3.97 (m, 2H), 3.96 - 3.90 (m, 2H), 3.74 - 3.71 (m, 2H), 2.71 - 2.65 (m, 2H), 2.56 - 2.51 (m, 1H), 2.27 - 2.18 (m, 1H), 1.92 - 1.80 (m, 2H), 1.21 (s, 3H), 1.10 (s, 3H). $^{19}$F NMR (376 MHz, DMSO-$d_6$) δ -122.56, -138.69.

**Example 88**

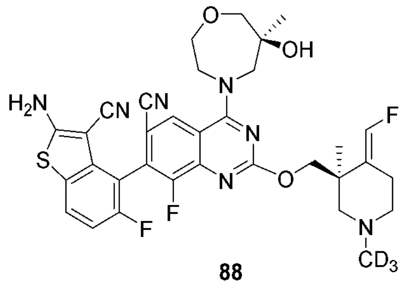

**88**

*7-(2-Amino-3-cyano-5-fluorobenzo[b]thiophene-4-yl)-8-fluoro-2-(((S,E)-4-(fluoromethylene)-3*-methyl-1-(methyl-*d*$_3$)piperidine-3-yl)methoxy)-4-((*S*)-6-hydroxy-6-methyl-1,4-oxazacycloheptane-4-yl)quinazoline-6-carbonitrile

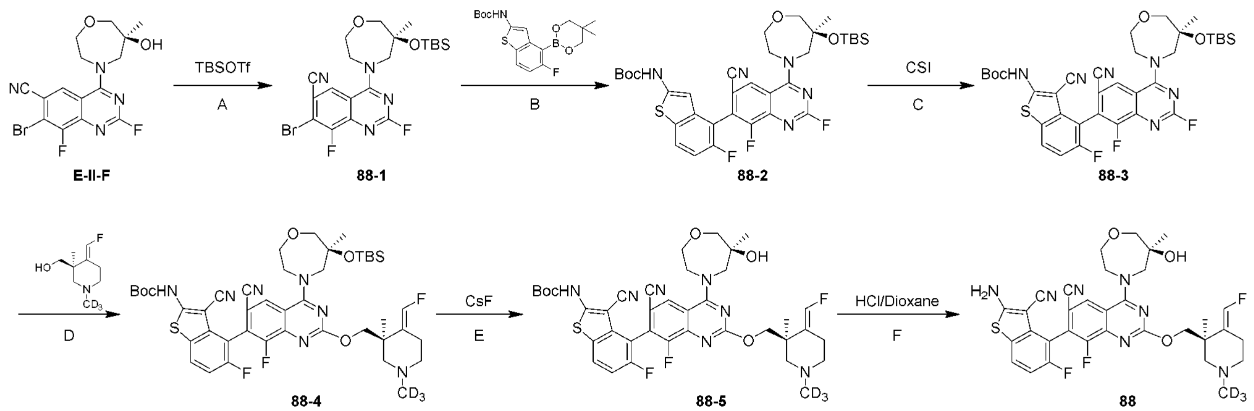

E-II-F
88-1
88-2
88-3
88-4
88-5
88

Step A: (*S*)-7-Bromo-4-(6-((tert-butyldimethylsilyl)oxy)-6-methyl-1,4-oxazacycloheptane-4-yl)-2,8-difluoroquinazoline-6-carbonitrile

Under ice bath conditions, (*S*)-7-bromo-2,8-difluoro-4-(6-hydroxy-6-methyl-1,4-oxazacycloheptane-4-yl)quinazoline-6-carbonitrile (1.7g, 4.25mmol), tert-butyl dimethoxytrifluoromethanesulfonate (2.25 g, 8.5 mmol), and TEA (1.7 g, 17 mmol) were added sequentially to DCM (50 mL), and the mixture was stirred at room temperature for 16 h. The reaction completion was monitored by LCMS, and then DCM (100 mL) was added. The solution was washed with saturated saline (30 mL×3). The organic phase was dried over anhydrous $Na_2SO_4$, filtered, and the crude product obtained by concentrating the organic liquid was purified by FCC ($SiO_2$, EA/PE=0-15%) to give a yellow solid (*S*)-7-bromo-4-(6-((tert-butyldimethylsilyl)oxy)-6-methyl-1,4-oxazacycloheptane-4-yl)-2,8-difluoroquinazoline-6-carbonitrile (2.3 g, yield 95%). LCMS (m/z): 513.1 (M+H).

**Step B:** tert-Butyl (4-(4-((*S*)-6-((tert-butyldimethylsilyl)oxy)-6-methyl-1,4-oxazacycloheptane-4-yl)-6-cyano-2,8-difluoroquinazoline-7-yl)-5-fluorobenzo[*b*]thiophene-2-yl)carbamate

At room temperature, (*S*)-7-bromo-4-(6-((tert-butyldimethylsilyl)oxy)-6-methyl-1,4-oxazacycloheptane-4-yl)-2,8-difluoroquinazoline-6-carbonitrile (1.10 g, 2.14 mmol), tert-butyl (4-(5,5-dimethyl-1,3,2-dioxaborolan-2-yl)-5-fluorobenzo[*b*]thiophene-2-yl)carbamate (1.0 g, 2.6 mmol), Ruphous-Pd-G4 (100 mg, 0.118 mmol), and DIEA (900 mg, 6.98 mmol) were added sequentially to a solution of 1,4-dioxane (15 mL) and water (5 mL). After $N_2$ replacement three times, the solution was warmed to 80°C and stirred for 2 h. The reaction completion was monitored by LCMS. The reaction solution was then brought to room temperature and the palladium catalyst was removed by diatomite filtration. The mother liquor was collected and water (80 mL) was added. Extraction was performed using EA (100 mL×2). The collected organic phase was washed with saturated brine (50 mL). The crude product obtained by concentrating the organic liquid was purified by FCC ($SiO_2$, EA/PE=0-70%) to give a yellow solid tert-butyl (4-(4-((*S*)-6-((tertbutyldimethylsilyl)oxy)-6-methyl-1,4-oxazacycloheptane-4-yl)-6-cyano-2,8-difluoroquinazoline-7-yl)-5-fluorobenzo[*b*]thiophene-2-yl)carbamate (1.2 g, yield 80%). LCMS (m/z): 700.2 (M+H).

**Step C:** tert-Butyl (4-(4-((*S*)-6-((tert-butyldimethylsilyl)oxy)-6-methyl-1,4-oxazacycloheptane-4-yl)-6-cyano-2,8-difluoroquinazoline-7-yl)-3-cyano-5-fluorobenzo[*b*]thiophene-2-yl)carbamate

Under ice-salt bath conditions, CSI (363 mg, 2.57 mmol) was slowly added dropwise to a mixed solution of tert-butyl (4-(4-((*S*)-6-((tert-butyldimethylsilyl)oxy)-6-methyl-1,4-oxazacycloheptane-4-yl)-6-cyano-2,8-difluoroquinazoline-7-yl)-5-fluorobenzo[*b*]thiophene-2-yl)carbamate (1.2 g, 1.71 mmol) and ACN (60 mL) and stirred for 1 h under ice-salt bath conditions. Then slowly added DMF (2 mL) dropwise and continued stirring for 1 h under ice-salt bath conditions. After the reaction completion was monitored by LCMS and brought to room temperature, the solution was poured into a 100 mL water solution and extracted with EA (100 mL×2). The organic phase was collected, washed with saturated brine (50 mL), dried over anhydrous $Na_2SO_4$, filtered, and concentrated to obtain a yellow solid tert-butyl (4-(4-((*S*)-6-((tert-butyldimethylsilyl)oxy)-6-methyl-1,4-oxazacycloheptane-4-yl)-6-cyano-2,8-difluoroquinazoline-7-yl)-3-cyano-5-fluorobenzo[*b*]thiophene-2-yl)carbamate (800 mg, yield 65%). LCMS (m/z): 725.2 (M+H).

**Step D:** tert-butyl (4-(4-((S)-6-((tert-butyldimethylsilyl)oxy)-6-methyl-1,4-oxazacycloheptane-4-yl)-6-cyano-8-fluoro-2-(((*S,E*)-4-(fluoromethylene)-3-methyl-1-(methyl-$d_3$)piperidine-3-yl)methoxy)quinazoline-7-yl)-3-cyano-5-fluorobenzo[b]thiophene-2-yl)carbamate

Under dry ice and ethanol bath conditions, tBuONa (0.5 mL, 2 M THF solution, 1.0 mmol ) was added to a mixed solution of tert-butyl (4-(4-((*S*)-6-((tert-butyldimethylsilyl)oxy)-6-methyl-1,4-oxazacycloheptane-4-yl)-6-cyano-2,8-difluoroquinazoline-7-yl)-3-cyano-5-fluorobenzo[b]thiophene-2-yl)carbamate (250 mg, 0.345 mmol), (*S,E*)-(4-(fluoromethylene)-3-methyl-1-(methyl-$d_3$)piperidine-3-yl)methanol (73 mg, 0.41 mmol) and THF (5 mL) and stirred for 1 h. The reaction completion was monitored by LCMS and TLC until it ended. After the reaction solution was brought back to room temperature, it was poured into a semi-saturated $NH_4Cl$ (30 mL) solution and extracted with EA (50 mL×3). The organic phase was collected, washed with saturated brine (30 mL), dried over anhydrous $Na_2SO_4$, and the organic phase was concentrated to obtain a yellow solid crude product tert-butyl (4-(4-((S)-6-((tert-butyldimethylsilyl)oxy)-6-methyl-1,4-oxazacycloheptane-4-yl)-6-cyano-8-fluoro-2-(((*S,E*)-4-(fluoromethylene)-3-methyl-1-(methyl-$d_3$)piperidine-3-yl)methoxy)quinazoline-7-yl)-3-cyano-5-fluorobenzo[*b*]thiophene-2-yl)carbamate (200 mg, yield 64%). LCMS (m/z): 881.2 (M+H).

**Step E:** tert-Butyl (3-cyano-4-(6-cyano-8-fluoro-2-(((*S,E*)-4-(fluoromethylene)-3-methyl-1-(methyl-$d_3$)piperidine-3-yl)methoxy)-4-((*S*)-6-hydroxy-6-methyl-1,4-oxazacycloheptane-4-yl)quinazoline-7-yl)-5-fluorobenzo[*b*]thiophene-2-yl)carbamate

At room temperature, tert-butyl (4-(4-((S)-6-((tert-butyldimethylsilyl)oxy)-6-methyl-1,4-oxazolidinyl-4-yl)-6-cyano-8-fluoro-2-(((*S,E*)-4-(fluoromethylene)-3-methyl-1-(methyl-$d_3$)piperidine-3-yl)methoxy)quinazoline-7-yl)-3-cyano-5-fluorobenzo[*b*]thiophene-2-yl)carbamate (200 mg, 0.244 mmol) and CsF (200 mg, 1.32 mmol) were added sequentially to a DMF (3 mL) solution and the mixture was warmed to 55°C and stirred for 16 h. After the reaction completion was monitored by LCMS and brought to room temperature, the reaction solution was poured into $H_2O$ (20 mL), extracted with EA (30 mL×3), and the organic phase was collected. The mixture was then washed with saturated brine (30 mL), dried over anhydrous $Na_2SO_4$, and the organic phase was concentrated to obtain a yellow solid crude product tert-butyl (3-cyano-4-(6-cyano-8-fluoro-2-(((*S,E*)-4-(fluoromethylene)-3-methyl-1-(methyl-$d_3$)piperidine-3-yl)methoxy)-4-((*S*)-6-hydroxy-6-methyl-1,4-oxazacycloheptane4-yl)quinazoline-7-yl)-5-fluorobenzo[*b*]thiophene-2-yl)carbamate (150 mg, yield 80 %). LCMS (m/z): 766.3 (M+H).

**Step F:** 7-(2-Amino-3-cyano-5-fluorobenzo[*b*]thiophene-4-yl)-8-fluoro-2-(((*S,E*)-4-(fluoromethylene)-3-methyl-1-(methyl-$d_3$)piperidine-3-yl)methoxy)-4-((*S*)-6-hydroxy-6-methyl-1,4-oxazacycloheptane-4-yl)quinazoline-6-carbonitrile

At room temperature, HCl (3 mL, 4 M, in 1,4-dioxane) was added to tert-butyl (3-cyano-4-(6-cyano-8-fluoro-2-(((*S,E*)-4-(fluoromethylene)-3-methyl-1-(methyl-$d_3$)piperidine-3-yl)methoxy)-4-((*S*)-6-hydroxy-6-methyl-1,4-oxazacycloheptane-4-yl)quinazoline-7-yl)-5-fluorobenzo[*b*]thiophene-2-yl)carbamate (150 mg, 0.195 mmol) and stirred for 1 h. The reaction completion was monitored by LCMS. The reaction solution was concentrated and quenched in saturated $NaHCO_3$ aqueous solution (20 mL). Extraction was performed by EA (20 mL×3). The organic phase was collected, washed with 30 mL of saturated brine, dried over anhydrous $Na_2SO_4$, and purified by pre-HPLC (C18, ACN/(10 mmol $NH_4HCO_3/H_2O$=55-75%) to obtain a white solid 7-(2-amino-3-cyano-5-fluorobenzo[b]thiophene-4-yl)-8-fluoro-2-(((*S,E*)-4-(fluoromethylene)-3-methyl-1-(methyl-$d_3$)piperidine-3-yl)methoxy)-4-((*S*)-6-hydroxy-6-methyl-1,4-oxazacycloheptane-4-yl)quinazoline-6-carbonitrile (19 mg, 15% yield). LCMS (m/z): 667.3 (M+H). $^1$H NMR (400 MHz, DMSO-$d_6$) δ 9.13 - 9.01 (m, 1H), 8.08 (s, 2H), 7.95 - 7.87 (m, 1H), 7.23 - 7.15 (m, 1H), 6.85 - 6.57 (m, 1H), 5.41 - 5.29 (m, 1H), 4.66 - 4.56 (m, 1H), 4.43 - 4.28 (m, 2H), 4.25 - 4.09 (m, 1H), 4.05 - 3.81 (m, 3H), 3.79 - 3.67 (m, 1H), 3.63 - 3.52 (m, 2H), 2.75 - 2.63 (m, 2H), 2.54 (s, 1H), 2.28 - 2.14 (m, 1H), 1.96 - 1.80 (m, 2H), 1.20 - 1.13 (m, 3H), 1.12 - 1.07 (m, 3H). $^{19}$F NMR (376 MHz, DMSO-$d_6$) δ -118.67 - -118.83, -122.96 - -123.28, -138.72.

**Example 89**

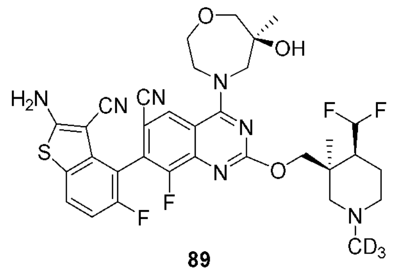

**89**

7-(2-Amino-3-cyano-5-fluorobenzo[*b*]thiophene-4-yl)-2-(((3*S*,4*S*)-4-(difluoromethyl)-3-methyl-1-(methyl-$d_3$)piperidine-3-yl)methoxy)-8-fluoro-4-((*S*)-6-hydroxy-6-methyl-1,4-oxazacycloheptane-4-yl)quinazoline-6-carbonitrile

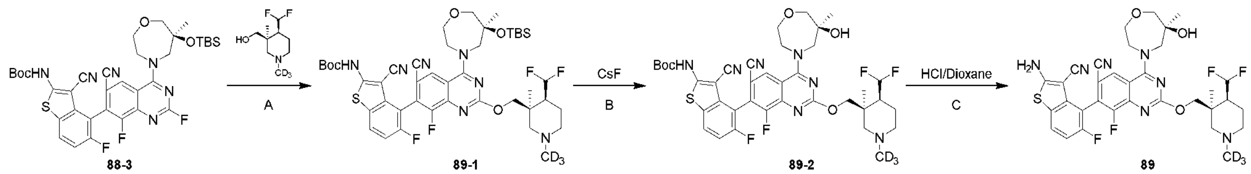


**Step A:** tert-Butyl (4-(4-((*S*)-6-((tert-butyldimethylsilyl)oxy)-6-methyl-1,4-oxazacycloheptane-4-yl)-6-cyano-2-(((3*S*,4*S*)-4-(difluoromethyl)-3-methyl-1-(methyl-$d_3$)piperidine-3-yl)methoxy)-8-fluoroquinazoline-7-yl)-3-cyano-5-fluorobenzo[*b*]thiophene-2-yl)carbamate

Under dry ice and ethanol bath conditions (-70°C), *t*BuONa (0.5 mL, 2 M THF solution, 1.0 mmol) was added to a mixture of tert-butyl (4-(4-((*S*)-6-((tert-butyldimethylsilyl)oxy)-6-methyl-1,4-oxazacycloheptane-4-yl)-6-cyano-2,8-difluoroquinazoline-7-yl)-3-cyano-5-fluorobenzo[b]thiophene-2-yl)carbamate (250 mg, 0.345 mmol), ((3S,4S)-4-(difluoromethyl)-3-methyl-1-(methyl-$d_3$)piperidine-3-yl)methanol (81 mg, 0.41 mmol) and THF (5 mL) and stirred for 1 h. The reaction completion was monitored by LCMS and TLC. After the reaction solution was brought back to room temperature, it was poured into a semi-saturated $NH_4Cl$ (30 mL) solution and extracted with EA (50 mL×3). The organic phase was collected, washed with saturated brine (30 mL), dried over anhydrous $Na_2SO_4$, and the organic phase was concentrated to obtain a yellow solid crude product tert-butyl (4-(4-((*S*)-6-((tert-butyldimethylsilyl)oxy)-6-methyl-1,4-oxazacycloheptane-4-yl)-6-cyano-2-(((3*S*,4*S*)-4-(difluoromethyl)-3-methyl-1-(methyl-*d*$_3$)piperidine-3-yl)methoxy)-8-fluoroquinazoline-7-yl)-3-cyano-5-fluorobenzo[*b*]thiophene-2-yl)carbamate (200 mg, yield 63%). LCMS (m/z): 902.2 (M+H).

**Step B:** tert-Butyl (3-cyano-4-(6-cyano-2-(((3S,4S)-4-(difluoromethyl)-3-methyl-1-(methyl-*d*$_3$)piperidine-3-yl)methoxy)-8-fluoro-4-((*S*)-6-hydroxy-6-methyl-1,4-oxazacycloheptane-4-yl)quinazoline-7-yl)-5-fluorobenzo[b]thiophene-2-yl) carbamate

At room temperature, tert-butyl (4-(4-((*S*)-6-((tert-butyldimethylsilyl)oxy)-6-methyl-1,4-oxazacycloheptane-4-yl)-6-cyano-2-(((3*S*,4*S*)-4-(difluoromethyl)-3-methyl-1-(methyl-*d*$_3$)piperidine-3-yl)methoxy)-8-fluoroquinazoline-7-yl)-3-cyano-5-fluorobenzo[*b*]thiophene-2-yl)carbamate (200 mg, 0.221 mmol) and CsF (200 mg, 1.32 mmol) were added sequentially to a DMF (3 mL) solution and the mixture was warmed to 55°C and stirred for 16 h. After the reaction completion was monitored by LCMS and brought to room temperature, the solution was poured into $H_2O$ (20 mL) and extracted with EA (30 mL×3). The organic phase was collected, washed with saturated brine (30 mL), dried over anhydrous $Na_2SO_4$, and concentrated to obtain a yellow solid crude product tert-butyl (3-cyano-4-(6-cyano-2-(((3*S*,4*S*)-4-(difluoromethyl)-3-methyl-1-(methyl-*d*$_3$)piperidine-3-yl)methoxy)-8-fluoro-4-((*S*)-6-hydroxy-6-methyl-1,4-oxazacycloheptane-4-yl)quinazoline-7-yl)-5-fluorobenzo[*b*]thiophene-2-yl)carbamate (140 mg, yield 81%). LCMS (m/z): 766.3 (M+H).

**Step C:** 7-(2-Amino-3-cyano-5-fluorobenzo[*b*]thiophene-4-yl)-2-(((3*S*,4*S*)-4-(difluoromethyl)-3-methyl-1-(methyl-*d*$_3$)piperidine-3-yl)methoxy)-8-fluoro-4-((*S*)-6-hydroxy-6-methyl-1,4-oxazacycloheptane-4-yl)quinazoline-6-carbonitrile

At room temperature, HCl (3 mL, 4 M 1,4-dioxane solution) was added to tert-butyl (3-cyano-4-(6-cyano-2-(((3*S*,4*S*)-4-(difluoromethyl)-3-methyl-1-(methyl-*d*$_3$)piperidine-3-yl)methoxy)-8-fluoro-4-((S)-6-hydroxy-6-methyl-1,4-oxazacycloheptane-4-yl)quinazoline-7-yl)-5- fluorobenzo[*b*]thiophene-2-yl)carbamate (140 mg, 0.178 mmol) and stirred for 1 h. The reaction completion was monitored by LCMS. The reaction solution was concentrated and quenched in 20 mL of saturated $NaHCO_3$ aqueous solution. Extraction was performed by EA (20 mL×3). The organic phase was collected, washed with 30 mL of saturated $Na_2SO_4$, and purified by pre-HPLC (C18, ACN/(10 mmol $NH_4HCO_3$/$H_2O$)=55-75%) to obtain a white solid 7-(2-amino-3-cyano-5-fluorobenzo[*b*]thiophene-4-yl)-2-(((3*S*,4*S*)-4-(difluoromethyl)-3-methyl-1-(methyl-*d*$_3$)piperidine-3-yl)methoxy)-8-fluoro-4-((*S*)-6-hydroxy-6-methyl-1,4-oxazacycloheptane-4-yl)quinazoline-6-carbonitrile (32 mg, yield 26%). LCMS (m/z): 687.3 (M+H). $^1$H NMR (400 MHz, DMSO-*d*$_6$) δ 9.11 - 8.97 (m, 1H), 8.08 (s, 2H), 7.95 - 7.86 (m, 1H), 7.24 - 7.11 (m, 1H), 6.49 - 6.13 (m, 1H), 5.44 - 5.23 (m, 1H), 4.51 - 4.42 (m, 2H), 4.42 - 4.27 (m, 1H), 4.23 - 4.07 (m, 1H), 4.03 - 3.84 (m, 3H), 3.81 - 3.70 (m, 1H), 3.62 - 3.51 (m, 2H), 2.89 - 2.76 (m, 2H), 2.54 (s, 1H), 1.85 - 1.61 (m, 4H), 1.20 - 1.07 (m, 6H). $^{19}$F NMR (376 MHz, DMSO-*d*$_6$) δ -115.11 - -116.62, -118.23 - -119.45, -122.60 - - 123.39.

**Example 90**

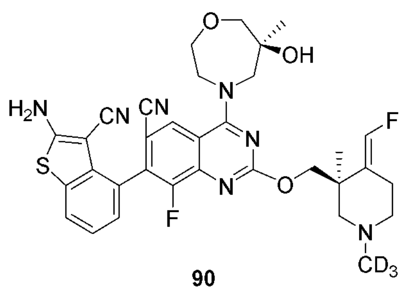

**90**

7-(2-Amino-3-cyanobenzo[*b*]thiophene-4-yl)-8-fluoro-2-(((*S*,*E*)-4-(fluoromethylene)-3-methyl-1-(methyl-*d*$_3$)piperidine-3-yl)methoxy)-4-((*S*)-6-hydroxy-6-methyl-1,4-oxazacycloheptane-4-yl)quinazoline-6-carbonitrile

Synthesis of Example 90 was carried out according to the scheme described in Example 88, except that in step B, tert-butyl (4-(5,5-dimethyl-1,3,2-dioxaborolan-2-yl)benzo[b]thiophene-2-yl)carbamate was used instead of tert-butyl (4-(5,5-dimethyl-1,3,2-dioxaborolan-2-yl)-5-fluorobenzo[b]thiophene-2-yl)carbamate. LCMS (m/z): 649.3 (M+H). $^1$H NMR (400 MHz, DMSO-*d*$_6$) δ 9.03 (d, J = 31.2 Hz, 1H), 7.91 (s, 2H), 7.85 (m, 1H), 7.33 - 7.20 (m, 2H), 6.89 - 6.54 (m, 1H), 5.37 (d, *J* = 29.3 Hz, 1H), 4.62 (dd, *J* = 16.0, 10.6 Hz, 1H), 4.32 (m, 2H), 4.15 (t, *J* = 14.5 Hz, 1H), 4.05 - 3.81 (m, 3H),

3.78 - 3.66 (m, 1H), 3.57 (d, *J* = 5.6 Hz, 2H), 2.68 (m, 2H), 2.21 (m, 1H), 1.87 (m, 2H), 1.17 (d, *J* = 9.0 Hz, 3H), 1.12 - 1.04 (s, 3H).

Using the above preparation method and appropriate separation techniques, the present invention also involved preparation of the following compounds.

| Example | Structural Formula | Characterization data |
|---|---|---|
| 91 | 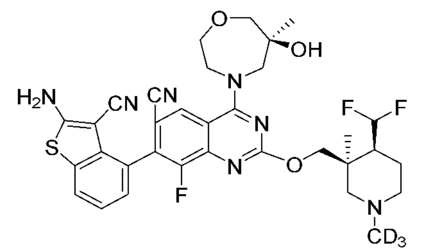 | LCMS (m/z): 669.3 (M+H). $^1$H NMR (400 MHz, DMSO-$d_6$) δ 9.00 (d, *J* = 27.7 Hz, 1H), 7.91 (s, 2H), 7.87 - 7.77 (m, 1H), 7.34 - 7.20 (m, 2H), 6.30 (t, *J* = 55.3 Hz, 1H), 5.34 (d, *J* = 29.0 Hz, 1H), 4.46 (s, 2H), 4.35 (m, 1H), 4.16 (t, *J* = 14.9 Hz, 1H), 4.05 - 3.84 (m, 3H), 3.75 (m, 1H), 3.56 (d, *J* = 6.2 Hz, 2H), 2.82 (m, 2H), 1.91 - 1.51 (m, 5H), 1.16 (d, *J* = 10.2 Hz, 3H), 1.11 (s, 3H) . |
| 92 | 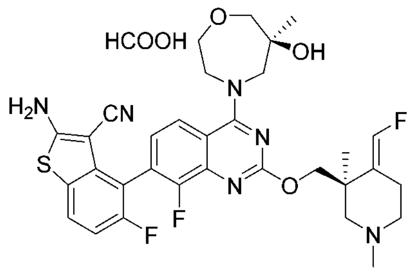 | LCMS (m/z): 746.3 (M+H). $^1$H NMR (400 MHz, DMSO-$d_6$) δ 8.30 (s, 1H), 8.17 - 8.10 (m, 1H), 7.92 - 7.85 (m, 2H), 7.79 - 7.70 (m, 1H), 7.61 (d, *J* = 10.9 Hz, 1H), 7.15 - 7.02 (m, 1H), 6.99 - 6.63 (m, 1H), 5.17 (d, *J* = 22.1 Hz, 1H), 4.75 - 4.47 (m, 2H), 4.42 - 4.28 (m, 1H), 4.26 - 4.09 (m, 2H), 3.99 - 3.76 (m, 4H), 3.57 - 3.47 (m, 2H), 2.94 - 2.59 (m, 5H), 2.44 - 1.82 (m, 5H), 1.21 - 1.12 (m, 3H), 1.10 - 0.96 (m, 3H). $^{19}$F NMR (376 MHz, DMSO-$d_6$) δ - 120.50, -126.94, -138.81. |

**Examples 93A and 93B**

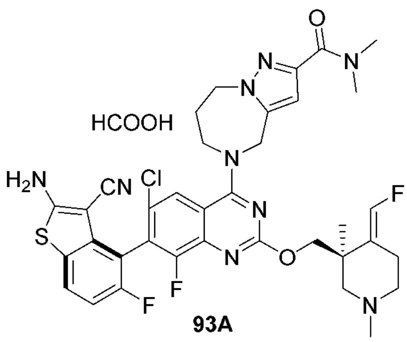
93A

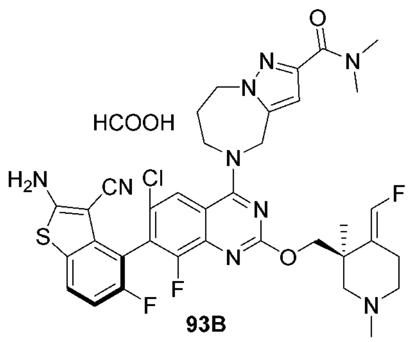
93B

(*Ra*)-5-(7-(2-amino-3-cyano-5-fluorobenzo[*b*]thiophene-4-yl)-6-chloro-8-fluoro-2-(((*S,E*)-4-(fluoromethylene)-1,3-dimethylpiperidine-3-yl)methoxy)quinazoline-4-yl)-*N,N*-dimethyl-5,6,7,8-tetrahydro-4*H*-pyrazolo[1,5-*a*][1,4]diazepine-2-formamide·formate **(93A)** and (*Sa*)-5-(7-(2-amino-3-cyano-5-fluorobenzo[b]thiophene-4-yl)-6-chloro-8-fluoro-2-(((*S,E*)-4-(fluoromethylene)-1,3-dimethylpiperidine-3-yl)methoxy)quinazoline-4-yl)-*N,N*-dimethyl-5,6,7,8-tetrahydro-4*H*-pyrazolo[1,5-*a*][1,4]diazepine-2-formamide·formate **(93B)**

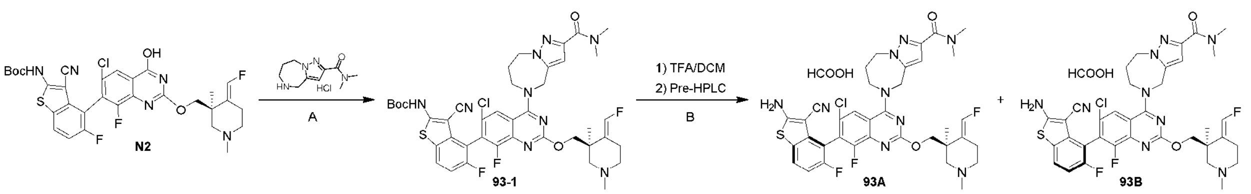

**Step A:** tert-Butyl *(*4-(6-chloro-4-(2-(dimethylcarbamoyl)-7,8-dihydro-4*H*-pyrazolo[1,5-*a*][1,4]diazepine-5(6*H*)-yl)-8-fluoro-2-(((*S,E*)-4-(fluoromethylene)-1,3-dimethylpiperidine-3-yl)methoxy)quinazoline-7-yl)-3-cyano-5-fluorobenzo[*b*] thiophene-2-yl) carbamate

At room temperature, DIEA (176 mg, 1.36 mmol) was added to a mixture of tert-butyl (4-(6-chloro-8-fluoro-2-(((*S,E*)-4-(fluoromethylene)-1,3-dimethylpiperidine-3-yl)methoxy)-4-hydroxyquinazoline-7-yl)-3-cyano-5-fluorobenzo[*b*]thiophene-2-yl)carbamate (150 mg, 0.227 mmol), *N,N*-dimethyl-5,6,7,8-tetrahydro-*4H*-pyrazolo[1,5-*a*][1,4]diazepine-2-formamide hydrochloride (111 mg, 0.455 mmol), BOP (201 mg, 0.455 mmol), and DMF (3 mL). The reaction mixture was warmed to 50°C and stirred overnight. The reaction completion was monitored by LCMS. The reaction solution was purified by FCC ($SiO_2$, MeOH/DCM= 0-10%) to obtain a yellow solid product tert-butyl (4-(6-chloro-4-(2-(dimethylcarbamoyl)-7,8-dihydro-4*H*-pyrazolo[1,5-*a*][1,4]diazepine-5(6*H*)-yl)-8-fluoro-2-(((*S,E*)-4-(fluoromethylene)-1,3-dimethylpiperidine-3-yl)methoxy)quinazoline-7-yl)-3-cyano-5-fluorobenzo[b]thiophene-2-yl)carbamate (110 mg, yield 57%). LCMS (m/z): 850.1 (M+H).

**Step B:** (*Ra*)-5-(7-(2-amino-3-cyano-5-fluorobenzo[*b*]thiophene-4-yl)-6-chloro-8-fluoro-2-(((*S,E*)-4-(fluoromethylene)-1,3-dimethylpiperidine-3-yl)methoxy)quinazoline-4-yl)-*N,N*-dimethyl-5,6,7,8-tetrahydro-4*H*-pyrazolo[1,5-*a*][1,4] diazepine-2-formamide·formate **(93A)** and (*Sa*)-5-(7-(2-amino-3-cyano-5-fluorobenzo[b]thiophene-4-yl)-6-chloro-8-fluoro-2-(((*S,E*)-4-(fluoromethylene)-1,3-dimethylpiperidine-3-yl)methoxy)quinazoline-4-yl)-*N,N*-dimethyl-5,6,7,8-tetrahydro-*4H*-pyrazolo[1,5-*a*][1,4]diazepine-2-formamide·formate **(93B)**

At room temperature, TFA:DCM=3:1 (10 mL) was added to tert-butyl (4-(6-chloro-4-(2-(dimethylcarbamoyl)-7,8-dihydro-4H-pyrazolo[1,5-*a*][1,4]diazepine-5(6*H*)-yl)-8-fluoro-2-(((*S,E*)-4-(fluoromethylene)-1,3-dimethylpiperidine-3-yl) methoxy)quinazoline-7-yl)-3-cyano-5-fluorobenzo[*b*]thiophene-2-yl)carbamate (110 mg, 0.13 mmol) and stirred for 1 h. The reaction completion was monitored by LCMS. The crude product obtained after concentration of the reaction solution was purified by pre-HPLC (C18, ACN/(0.1%FA/$H_2O$)=25-40%) to obtain the first eluted isomer 1, which was **Example 93A.** (white solid, 8 mg, yield 17%, retention time 14.2 min). LCMS (m/z): 750.2 (M+H). $^1H$ NMR (400 MHz, DMSO-$d_6$) δ 8.17 - 7.94 (m, 3H), 7.86 (dd, *J* = 8.7, 5.0 Hz, 1H), 7.22 - 7.10 (m, 1H), 7.02 - 6.55 (m, 2H), 5.22 - 4.89 (m, 2H), 4.63 - 4.42 (m, 3H), 4.38 - 4.08 (m, 3H), 3.54 - 3.42 (m, 4 H), 3.27 (s, 3H), 2.96 (s, 3H), 2.84 - 2.57 (m, 3H), 2.40 - 1.75 (m, 6H), 1.15 (s, 3H). $^{19}F$ NMR (376 MHz, DMSO-$d_6$) δ -118.82, -121.18, -138.75. The subsequently eluted isomer 2 was **Example 93B.** (white solid, 7 mg, yield 15%, retention time 15.6 min). LCMS (m/z): 750.2 (M+H). $^1H$ NMR (400 MHz, DMSO-$d_6$) δ 8.18 - 7.96 (m, 3H), 7.86 (dd, *J* = 8.7, 4.9 Hz, 1H), 7.22 - 7.10 (m, 1H), 6.91 - 6.58 (m, 2H), 5.20 - 4.88 (m, 2H), 4.63 - 4.41 (m, 3H), 4.35 - 4.06 (m, 3H), 3.51 - 3.33 (m, 4 H), 3.27 (s, 3H), 2.95 (s, 3H), 2.86 - 2.66 (m, 2H), 2.39 - 1.96 (m, 7H), 1.12 (s, 3H). $^{19}F$ NMR (376 MHz, DMSO-$d_6$) δ -118.77, -121.18, -138.75.

Examples 94A and 94B

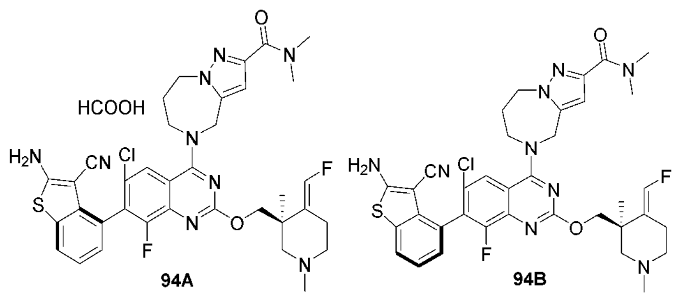

94A 94B

(*Sa*)-5-(7-(2-amino-3-cyanobenzo[*b*]thiophene-4-yl)-6-chloro-8-fluoro-2-(((*S,E*)-4-(fluoromethylene)-1,3-dimethylpiperidine-3-yl)methoxy)quinazoline-4-yl)-*N,N*-dimethyl-5,6,7,8-tetrahydro-4*H*-pyrazolo[1,5-*a*][1,4]diazepine-2-formamide·-formate **(94A)** and (*Ra*)-5-(7-(2-amino-3-cyanobenzo[*b*]thiophene-4-yl)-6-chloro-8-fluoro-2-(((*S,E*)-4-(fluoromethylene)-1,3-dimethylpiperidine-3-yl)methoxy)quinazoline-4-yl)-*N,N*-dimethyl-5,6,7,8-tetrahydro-*4H*-pyrazolo[1,5-*a*][1,4] diazepine-2-formamide **(94B)**

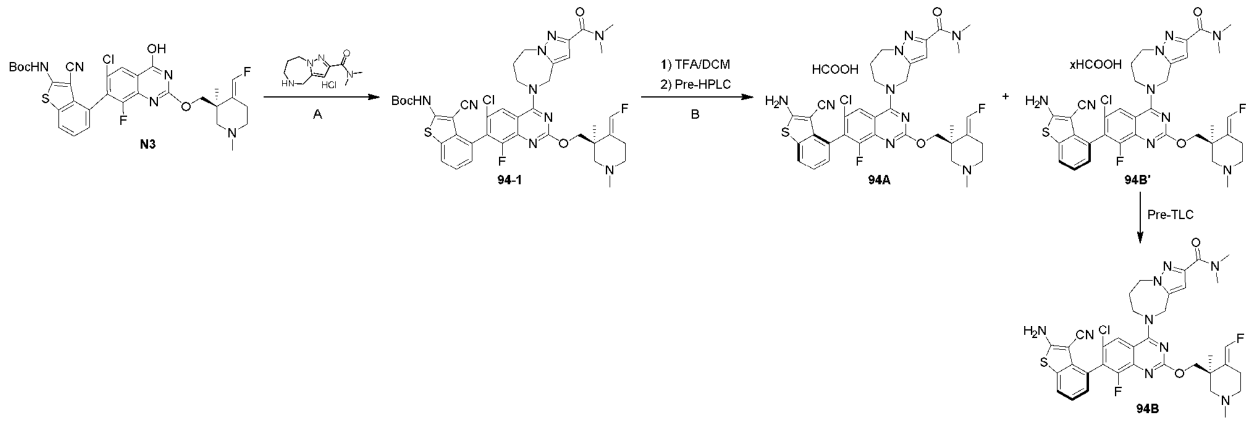

N3, 94-1, 94A, 94B', 94B

**Step A:** tert-Butyl (4-(6-chloro-4-(2-(dimethylcarbamoyl)-7,8-dihydro-4*H*-pyrazolo[1,5-*a*][1,4]diazepine-5(6*H*)-yl)-8-fluoro-2-(((*S,E*)-4-(fluoromethylene)-1,3-dimethylpiperidine-3-yl)methoxy)quinazoline-7-yl)-3-cyanobenzo[*b*]thiophene-2-yl)carbamate

At room temperature, tert-butyl (4-(6-chloro-8-fluoro-2-(((*S,E*)-4-(fluoromethylene)-1,3-dimethylpiperidin-3-yl) methoxy)-4-hydroxyquinazoline-7-yl)-3-cyanobenzo[*b*]thiophene-2-yl)carbamate (320 mg, 0.500 mmol), N,N-dimethyl-5,6,7,8-tetrahydro-4*H*-pyrazolo[1,5-*a*][1,4]diazepine-2-formamide hydrochloride (242 mg, 242 mmol), BOP (441 mg, 1.00 mmol), and DIEA (386 mg, 2.99 mmol) were added sequentially to a DMF (10 mL) solution and the mixture was warmed to 50°C and stirred for 16 h. After the reaction completion was monitored by LCMS, the reaction solution was poured into saturated sodium bicarbonate (100 mL) and extracted with EA (50 mL×3). The organic phase was collected, washed with saturated brine (70 mL), and dried over anhydrous $Na_2SO_4$. After filtration, the organic phase was concentrated and purified by FCC ($SiO_2$, MeOH/DCM=0-10%) to obtain a yellow solid product tert-Butyl (4-(6-chloro-4-(2-(dimethylcarbamoyl)-7,8-dihydro-4*H*-pyrazolo[1,5-*a*][1,4]diazepine-5(6*H*)-yl)-8-fluoro-2-(((*S,E*)-4-(fluoromethylene)-1,3-dimethylpiperidine-3-yl)methoxy)quinazoline-7-yl)-3-cyanobenzo[*b*]thiophene-2-yl)carbamate (400 mg, yield 96%). LCMS (m/z): 832.2 (M+H).

**Step B:** (*Sa*)-5-(7-(2-amino-3-cyanobenzo[*b*]thiophene-4-yl)-6-chloro-8-fluoro-2-(((*S,E*)-4-(fluoromethylene)-1,3-dimethylpiperidine-3-yl)methoxy)quinazoline-4-yl)-*N,N*-dimethyl-5,6,7,8-tetrahydro-4*H*-pyrazolo[1,5-*a*][1,4]diazepine-2-formamide·formate **(94A)** and (*Ra*)-5-(7-(2-amino-3-cyanobenzo[*b*]thiophene-4-yl)-6-chloro-8-fluoro-2-(((*S,E*)-4-(fluoromethylene)-1,3-dimethylpiperidine-3-yl)methoxy)quinazoline-4-yl)-*N,N*-dimethyl-5,6,7,8-tetrahydro-4*H* - pyrazolo [1,5-*a*][1,4]diazepine-2-formamide **(94B)**

At room temperature, tert-butyl (4-(6-chloro-4-(2-(dimethylcarbamoyl)-7,8-dihydro-4*H*-pyrazolo[1,5-*a*][1,4]diazepine-5(6*H*)-yl)-8-fluoro-2-(((*S,E*)-4-(fluoromethylene)-1,3-dimethylpiperidine-3-yl)methoxy)quinazoline-7-yl)-3-cyanobenzo[*b*]thiophene-2-yl)carbamate was added to a mixed solution of TFA (5.0 mL) and DCM (5.0 mL) and stirred for 1 h. After the reaction completion was monitored by LCMS, the reaction solution was concentrated and quenched in saturated $NaHCO_3$ aqueous solution (50 mL). Extraction was performed using EA (30 mL×3). The organic phase was collected, washed with saturated brine (70 mL), and dried over anhydrous $Na_2SO_4$. The mixture was filtered, and the crude product obtained by concentrating the organic phase was purified by pre-HPLC (C18, ACN/(10 mmol FA/$H_2O$)=26-36%) to obtain the first eluted isomer 1, which was **Example 94A** (white solid, 143 mg, yield 20%, retention time 9.5 min). LCMS (m/z): 732.3 (M+H). $^1$H NMR (400 MHz, DMSO-$d_6$) δ 8.16 (s, 1H), 7.99 - 7.91 (m, 1H), 7.84 (s, 2H), 7.82 - 7.76 (m, 1H), 7.29 - 7.21 (m, 1H), 7.20 - 7.12 (m, 1H), 6.86 - 6.77 (m, 0.5H), 6.64 (s, 1H), 6.61 (s, 0.5H), 5.19 - 5.02 (m, 1H), 5.01 - 4.84 (m, 1H), 4.64 - 4.53 (m, 1H), 4.52 - 4.41 (m, 2H), 4.31 - 4.18 (m, 2H), 4.16 - 4.00 (m, 1H), 3.26 (s, 3H), 2.95 (s, 3H), 2.74 - 2.64 (m, 2H), 2.55 - 2.51 (m, 1H), 2.37 - 2.24 (m, 2H), 2.20 - 2.16 (m, 1H), 2.15 (s, 3H), 1.93 - 1.79 (m, 2H), 1.10 (s, 3H). $^{19}$F NMR (376 MHz, DMSO-$d_6$) δ -122.20, -138.77; the subsequently eluted isomer 2 was compound **94B'** (140 mg, retention time 11.0 min). Compound **94B'** was freed with DIEA and further purified by pre-TLC (MeOH/EA=5%) to give a white solid. **Example 94B.** LCMS (m/z): 732.3 (M+H). $^1$H NMR (400 MHz, Methanol-$d_4$) δ 8.10 - 8.01 (m, 1H), 7.73 - 7.64 (m, 1H), 7.31 - 7.23 (m, 1H), 7.21 - 7.13 (m, 1H), 7.02 - 6.92 (m, 0.5H), 6.78 - 6.74 (m, 0.5H), 6.73 (s, 1H), 5.16 - 5.05 (m, 2H), 4.57 - 4.43 (m, 3H), 4.42 - 4.26 (m, 3H), 3.80 - 3.65 (m, 1H), 3.34 (s, 3H), 3.26 - 3.18 (m, 1H), 3.08 (s, 3H), 3.03 - 2.64 (m, 6H), 2.63 - 2.48 (m, 1H), 2.45 - 2.31 (m, 2H), 1.29 (s, 3H).

**Example 95**

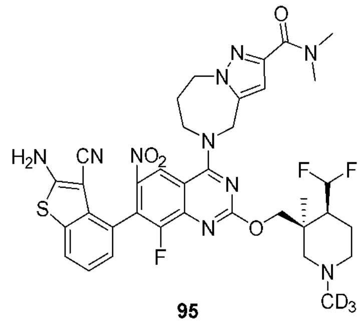

95

5-(7-(2-Amino-3-cyanobenzo[*b*]thiophene-4-yl)-2-(((3*S*,4*S*)-4-(difluoromethyl)-3-methyl-1-(methyl-$d_3$)piperidine-3-yl) methoxy)-8-fluoro-6-nitroquinazoline-4-yl)-*N,N*-dimethyl-5,6,7,8-tetrahydro-4*H*-pyrazolo[1,5-*a*][1,4]diazepine-2-formamide

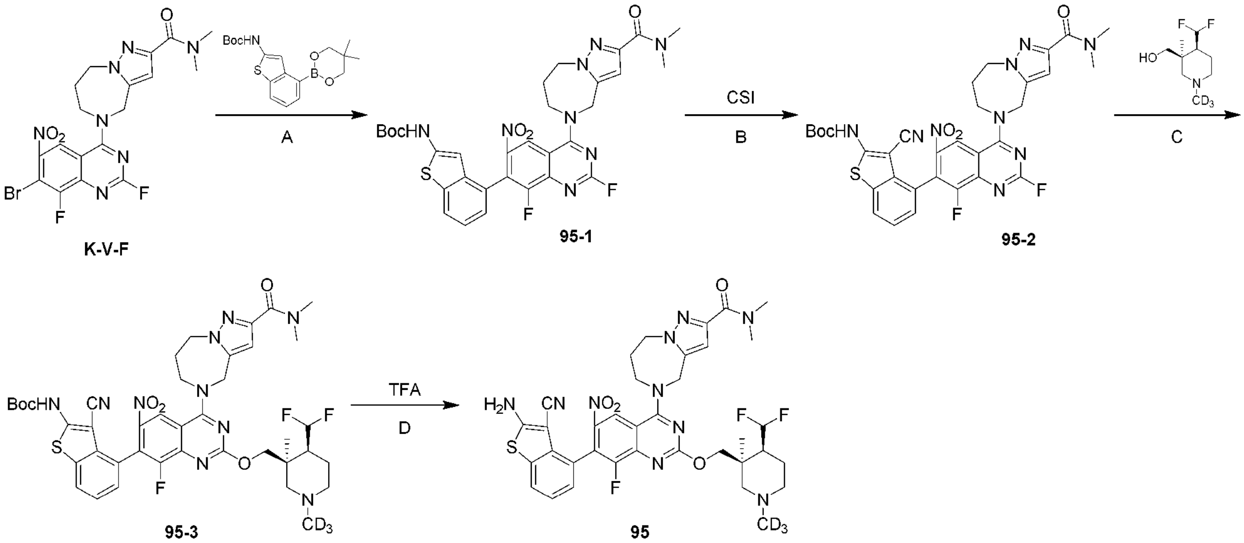


K-V-F 95-1 95-2 95-3 95

**Step A:** tert-Butyl (4-(4-(2-(dimethylcarbamoyl)-7,8-dihydro-4*H*-pyrazolo[1,5-*a*][1,4]diazepine-5(6*H*)-yl)-2,8-difluoro-6-nitroquinazoline-7-yl)benzo[*b*]thiophene-2-yl)carbamate

At room temperature, 5-(7-bromo-2,8-difluoro-6-nitroquinazoline-4-yl)-*N,N*-dimethyl-5,6,7,8-tetrahydro-4*H*-pyrazolo[1,5-*a*][1,4]diazepine-2-formamide (300 mg, 0.605 mmol), tert-butyl (4-(5,5-dimethyl-1,3,2-dioxaborolan-2-yl) benzo[b]thiophene-2-yl)carbamate (328 mg, 0.907 mmol), Pd-117 (43.3 mg, 0.0600 mmol), and $K_3PO_4$ (384 mg, 1.81 mmol) were added sequentially to a solution of 1,4-dioxane (10 mL) and water (2 mL). After replacement three times, the temperature was raised to 80°C and stirred for 1.5 hours. The reaction completion was monitored by LCMS, and the palladium catalyst was removed by diatomite filtration. The mother liquor was collected, water (50 mL) was added, and the mixture was extracted with EA (30 mL×3). The collected organic phase was washed with saturated brine (70 mL). The crude product obtained by concentrating the organic phase was purified by FCC ($SiO_2$, EA/PE=0-90%) to give a yellow solid tert-butyl (4-(4-(2-(dimethylcarbamoyl)-7,8-dihydro-4*H*-pyrazolo[1,5-*a*][1,4]diazepine-5(6*H*)-yl)-2,8-difluoro-6-nitroquinazoline-7-yl)benzo[*b*]thiophene-2-yl)carbamate(4-(5,5-dimethyl-1,3,2-dioxoborane-2-yl)-1-benzothiophene-2-yl)carbamate (300 mg, yield 75%). LCMS (m/z): 565.2 (M-Boc).

**Step B:** tert-Butyl (3-cyano-4-(4-(2-(dimethylcarbamoyl)-7,8-dihydro-4*H*-pyrazolo[1,5-*a*][1,4]diazepine-5(6*H*)-yl)-2,8-difluoro-6-nitroquinazoline-7-yl)benzo[*b*]thiophene-2-yl)carbamate

Under ice-water bath conditions, CSI (319 mg, 2.26 mmol) was added to a mixed solution of tert-butyl (4-(4-(2-(dimethylcarbamoyl)-7,8-dihydro-4*H*-pyrazolo[1,5-*a*][1,4]diazepine-5(6*H*)-yl)-2,8-difluoro-6-nitroquinazoline-7-yl)benzo[*b*]thiophene-2-yl)carbamate (300 mg, 0.450 mmol) and ACN (12 mL) and stirred at the same temperature for 1.0 h. DMF (3 mL) was added dropwise to the above reaction solution and stirring was continued for 1.0 h under ice-water bath conditions. After the reaction completion was monitored by LCMS and brought to room temperature, the solution was poured into a 50 mL water solution and extracted with EA (30 mL×3). The organic phase was collected, washed with saturated brine (70 mL), dried over anhydrous $Na_2SO_4$, filtered, and the organic phase was concentrated. The crude product was purified by FCC ($SiO_2$, EA/PE=0-90%) to obtain a yellow solid tert-butyl (3-cyano-4-(4-(2-(dimethylcarbamoyl)-7,8-dihydro-4*H*-pyrazolo[1,5-*a*][1,4]diazepine-5(6*H*)-yl)-2,8-difluoro-6-nitroquinazoline-7-yl)benzo[*b*]thiophene-2-yl)carbamate (220 mg, yield 71%). LCMS (m/z): 690.2 (M+H).

**Step C:** tert-butyl (3-cyano-4-(2-(((3*S*,4*S*)-4-(difluoromethyl)-3-methyl-1-(methyl-*d*$_3$)piperidine-3-yl)methoxy)-4-(2-(dimethylcarbamoyl)-7,8-dihydro-4*H*-pyrazolo[1,5-*a*][1,4]diazepine-5(6*H*)-yl)-8-fluoro-6-nitroquinazoline-7-yl)benzo[*b*] thiophene-2-yl)carbamate

Under dry ice and ethanol bath conditions, *t*BuONa (0.13 mL, 2 M THF solution, 0.26 mmol) was added to a mixed solution of tert-butyl (3-cyano-4-(4-(2-(dimethylcarbamoyl)-7,8-dihydro-4H-pyrazolo[1,5-*a*][1,4]diazepine-5(6*H*)-yl)-2,8-difluoro-6-nitroquinazoline-7-yl)benzo[*b*]thiophene-2-yl)carbamate (60 mg, 0.087 mmol), ((3*S*,4*S*)-4-(difluoromethyl)-3-methyl-1-(methyl-*d*$_3$)piperidine-3-yl)methanol (25 mg, 0.13 mmol) and THF (5 mL) and stirred for 1.0 h under dry ice and ethanol bath conditions. After the reaction solution was brought to room temperature, it was stirred for 1.0 h. The reaction completion was monitored by LCMS. The reaction solution was then poured into a saturated $NH_4Cl$ (50 mL) aqueous solution and extracted with EA (30 mL×3). After the organic phase was collected, washed with saturated brine (70 mL) and dried over anhydrous $Na_2SO_4$. After filtration and concentration of the organic phase, a yellow solid tert-butyl (3-cyano-4-(2-(((3S,4S)-4-(difluoromethyl)-3-methyl-1-(methyl-*d*$_3$)piperidine-3-yl)methoxy)-4-(2-(dimethylcarbamoyl)-7,8-dihydro-4*H*-pyrazolo[1,5-*a*][1,4]diazepine-5(6*H*)-yl)-8-fluoro-6-nitroquinazoline-7-yl)benzo[*b*]thiophene-2-yl)carbamate (80 mg, 95% yield) was obtained. LCMS (m/z): 866.3 (M+H).

**Step D:** 5-(7-(2-Amino-3-cyanobenzo[*b*]thiophene-4-yl)-2-(((3*S*,4*S*)-4-(difluoromethyl)-3-methyl-1-(methyl-*d*$_3$)piperidine-3-yl)methoxy)-8-fluoro-6-nitroquinazoline-4-yl)-*N,N*-dimethyl-5,6,7,8-tetrahydro-4*H*-pyrazolo[1,5-*a*][1,4]diazepine-2-formamide

At room temperature, tert-butyl (3-cyano-4-(2-(((3*S*,4*S*)-4-(difluoromethyl)-3-methyl-1-(methyl-*d*$_3$)piperidine-3-yl)methoxy)-4-(2-(dimethylcarbamoyl)-7,8-dihydro-4*H*-pyrazolo[1,5-*a*][1,4]diazepine-5(6*H*)-yl)-8-fluoro-6-nitroquinazoline-7-yl)benzo[*b*]thiophene-2-yl)carbamate (80mg, 0.092mmol) was added to a mixed solution of TFA (1.0 mL) and DCM (1.0 mL) and stirred for 1 h. After the reaction completion was monitored by LCMS, the reaction solution was concentrated and quenched in saturated $NaHCO_3$ aqueous solution (50 mL). Extraction was performed by EA (30 mL×3). The organic phase was collected, washed with saturated brine (70 mL), dried over anhydrous $Na_2SO_4$, and purified bypre-HPLC (C1 8, ACN/(10 mmol $NH_4HCO_3/H_2O$)=45-65%) to obtain a white solid 5-(7-(2-amino-3-cyanobenzo[*b*]thiophene-4-yl)-2-(((3*S*,4*S*)-4-(difluoromethyl)-3-methyl-1-(methyl-*d*$_3$)piperidine-3-yl)methoxy)-8-fluoro-6-nitroquinazoline-4-yl)-*N,N*-dimethyl-5,6,7,8-tetrahydro-4H-pyrazolo[1,5-*a*][1,4]diazepine-2-formamide (143 mg, 20% yield). LCMS (m/z): 766.3 (M+H). $^1$H NMR (400 MHz, DMSO-*d*$_6$) δ 8.68 (s, 1H), 7.85 (s, 2H), 7.80 - 7.76 (m, 1H), 7.23 - 7.18 (m, 1H), 7.17 - 7.09 (m, 1H), 6.71 (d, *J* = 4.8 Hz, 1H), 6.46 - 6.15 (m, 1H), 5.27 - 5.17 (m, 1H), 5.13 - 5.03 (m, 1H), 4.58 - 4.09 (m, 7H), 3.25 (s, 3H), 2.95 (s, 3H), 2.86 - 2.75 (m, 2H), 1.91 - 1.54 (m, 6H), 1.11 (s, 3H). $^{19}$F NMR (376 MHz, DMSO-*d*$_6$) δ -115.33, -115.38, -116.08, -116.13, -118.46, -118.49, -119.21, 119.24, 121.73, 121.76.

Using the above preparation methods and appropriate separation techniques, the present invention prepared the following compounds.

| Example | Structural Formula | Characterization Data |
|---|---|---|
| 96 | 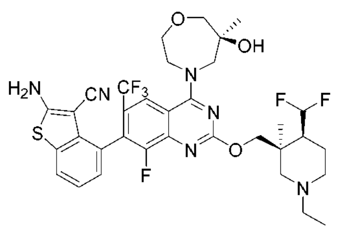 | LCMS(m/z): 723.3 (M+H) |

(continued)

| Example | Structural Formula | Characterization Data |
|---|---|---|
| 97 | 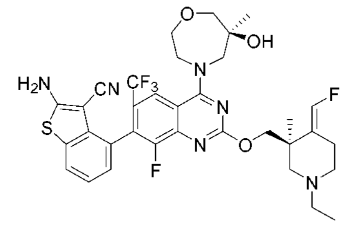 | LCMS(m/z): 703.2 (M+H) |
| 98 | 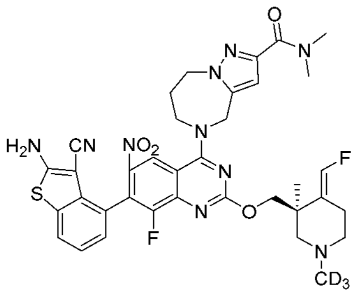 | LCMS(m/z): 746.3 (M+H). $^1$H NMR (400 MHz, DMSO-$d_6$) δ 8.66 (s, 1H), 7.86 (s, 2H), 7.78 (dd, $J$ = 7.9, 1.2 Hz, 1H), 7.23 - 7.17 (m, 1H), 7.16 - 7.10 (m, 1H), 6.83 (d, $J$ = 5.0 Hz, 0.5H), 6.74 (d, $J$ = 2.1 Hz, 1H), 6.61 (d, $J$ = 4.9 Hz, 0.5H), 5.28 - 5.15 (m, 1H), 5.12 - 5.00 (m, 1H), 4.68 - 4.59 (m, 1H), 4.58 - 4.45 (m, 2H), 4.40 - 4.15 (m, 3H), 3.25 (s, 3H), 2.96 (s, 3H), 2.76 - 2.65 (m, 2H), 2.56 - 2.54 (m, 1H), 2.44 -2.25 (m, 2H), 2.25 -2.09 (m, 1H), 1.93 -1.78 (m, 2H), 1.09 (d, $J$ = 4.1 Hz, 3H). $^{19}$F NMR (376 MHz, DMSO-$d_6$) δ -121.86, -138.63. |
| 99 | 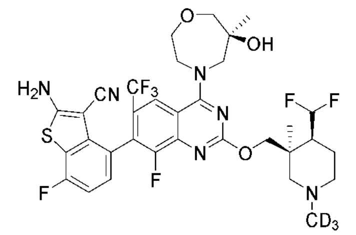 | LCMS(m/z): 730.2 (M+H) |
| 100 | 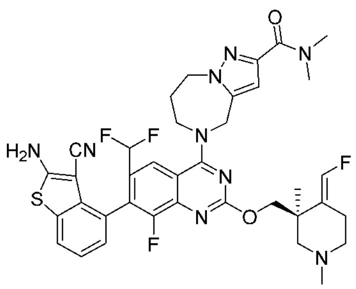 | LCMS(m/z): 748.3 (M+H). |
| 101 | 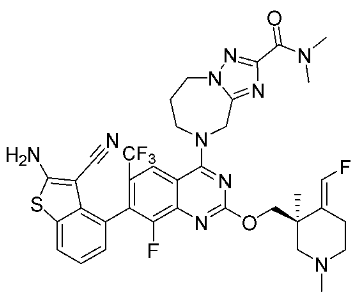 | LCMS(m/z): 767.1 (M+H). |
| 102 | 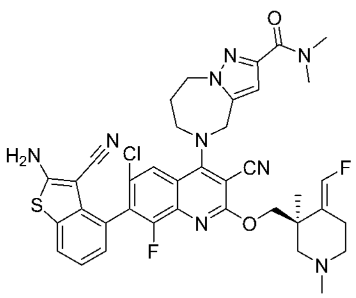 | LCMS(m/z): 756.3 (M+H) |

(continued)

| Example | Structural Formula | Characterization Data |
|---|---|---|
| 103 | 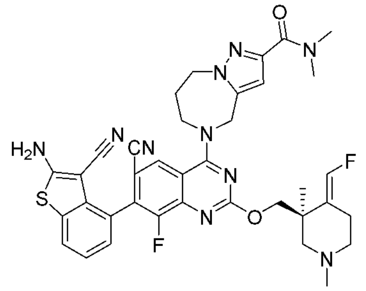 | LCMS(m/z): 723.3 (M+H) |
| 104 | 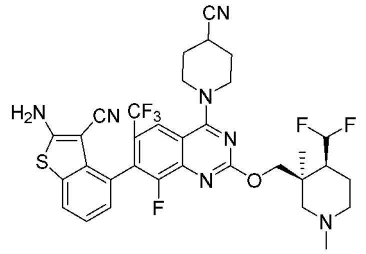 | LCMS (m/z): 668.3 (M+H). $^{1}$H NMR (400 MHz, DMSO-$d_6$) δ 8.04 (s, 1H), 7.86 - 7.75 (m, 3H), 7.26 - 7.14 (m, 2H), 6.86 - 6.58 (m, 1H), 4.66 - 4.56 (m, 1H), 4.35 - 4.27 (m, 1H), 4.10 - 3.95 (m, 2H), 3.76 - 3.58 (m, 2H), 3.29 - 3.22 (m, 1H), 2.73 -2.67 (m, 2H), 2.55 -2.52 (m, 1H), 2.23 - 2.08 (m, 6H), 2.04 - 1.92 (m, 2H), 1.92 - 1.79 (m, 2H), 1.11 (d, *J* = 1.7 Hz, 3H). $^{19}$F NMR (376 MHz, DMSO-$d_6$) δ - 57.34, -123.56, -138.70. |
| 105 | 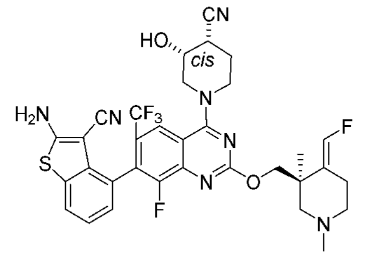 | LCMS (m/z): 684.3 (M+H). |
| 106 | 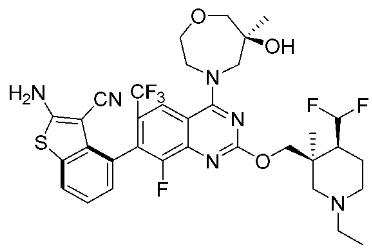 | LCMS (m/z): 723.3 (M+H). |
| 107 | 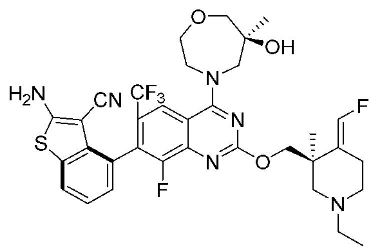 | LCMS (m/z): 703.3 (M+H). |
| 108 | 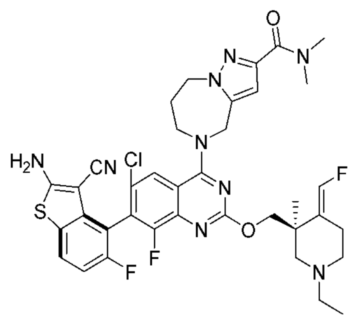 | LCMS (m/z): 764.3 (M+H). |

(continued)

| Example | Structural Formula | Characterization Data |
|---|---|---|
| 109 | 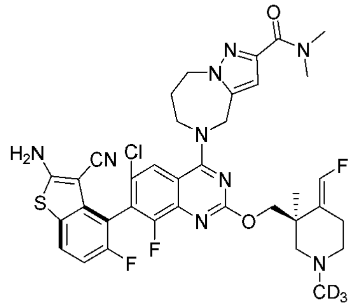 | LCMS (m/z): 753.3 (M+H). |
| 110 | 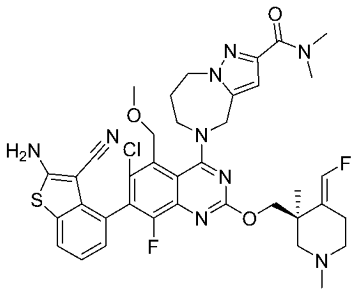 | LCMS (m/z): 776.3 (M+H). |
| 111 | 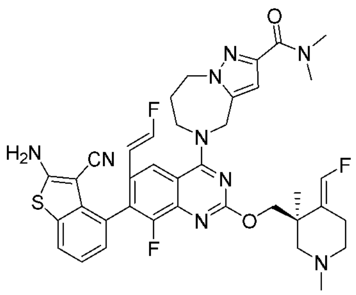 | LCMS (m/z): 742.3 (M+H). $^1$H NMR (400 MHz, DMSO-$d_6$) δ 8.17 (s, 0.5H), 7.82 - 7.78 (m, 3H), 7.45 (d, $J$ = 11.2 Hz, 0.5H), 7.28 - 7.21 (m, 1.5H), 7.13 - 7.09 (m, 1H), 7.01 - 6.80 (m, 1H), 6.62 - 6.57 (m, 1.5H), 5.93 (dd, $J_1$ = 20.4 Hz, $J_2$ = 11.2 Hz, 1H), 5.28 - 4.97 (m, 3H), 4.62 - 4.56 (m, 1H), 4.54 - 4.44 (m, 2H), 4.27 - 4.13 (m, 3H), 3.25 (s, 3H), 2.94 (s, 3H), 2.72 - 2.65 (m, 2H), 2.51 (m, 1H), 2.38 - 2.20 (m, 3H), 2.15 (s, 3H), 1.90 - 1.78 (m, 2H), 1.09 (t, $J$ = 3.6 Hz, 3H). |
| 112 | 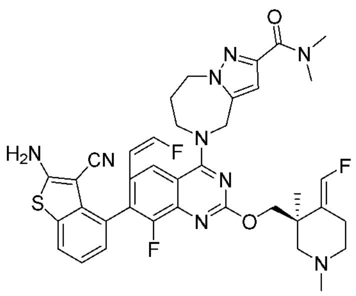 | LCMS (m/z): 742.3 (M+H). LCMS (m/z): 742.3 (M+H). $^1$H NMR (400 MHz, DMSO-$d_6$) δ 8.17 (s, 1H), 7.79 (*d, J*= 3.6 Hz, 3H), 7.24 (t, J= 7.6 Hz, 1H), 7.11 (d, $J$= 7.6 Hz, 1H), 6.89 (dd, $J_1$ = 83.6 Hz, $J_2$ = 5.6 Hz, 1H), 6.71 (dd, $J_1$ = 87.2 Hz, $J_2$ = 5.6 Hz, 1H), 6.57 (s, 1H), 5.23 (dd, $J_1$ = 44.8 Hz, $J_2$ = 4.8 Hz, 1H), 5.12 - 5.00 (m, 2H), 4.59 (t, $J$ = 11.2 Hz, 1H), 4.52 - 4.91 (m, 2H), 4.27 - 4.14 (m, 3H), 3.24 (s, 3H), 2.94 (s, 3H), 2.70 - 2.68 (m, 2H), 2.52 (m, 1H), 2.31 - 2.20 (m, 3H), 2.15 (s, 3H), 1.93 - 1.81 (m, 2H), 1.10 (d, $J$ = 3.2 Hz, 3H). |

**Examples 112A and 112B**

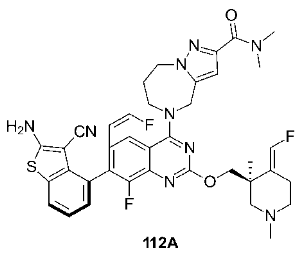

112A

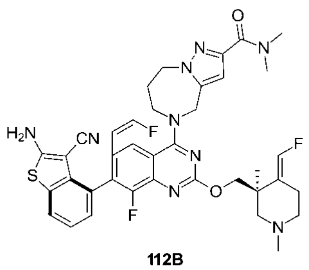

112B

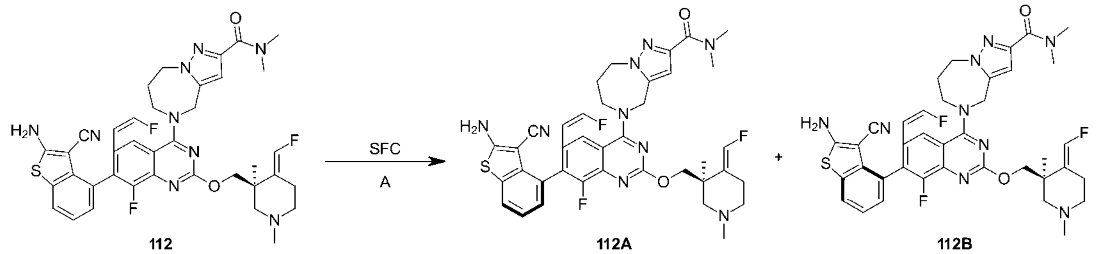

112 112A 112B

**Step A: Examples 112A and 112B**

Example 112 (85 mg) was resolved by chiral Waters SFC 150 (column: DAICELCHIRALPAK®AD, 250*30 mm, 10 μm; mobile phase: $CO_2$/EtOH (0.1% 7.0 mol/L Ammonia in MEOH)=60/40; flow rate: 120 mL/min) to yield the first eluted isomer 1, which was identified as **Example 112A** (32.7 mg, with a relatively short retention time). The chiral analysis method was SFC-112, Rt = 0.97 min. LCMS(m/z): 742.3 (M+H). $^1$H NMR (400 MHz, DMSO-$d_6$) δ 8.17 (s, 1H), 7.80 - 7.78 (m, 3H), 7.24 (t, $J$ = 7.6 Hz, 1H), 7.11 (dd, $J_1$ = 7.6 Hz, $J_2$ = 0.8 Hz, 1H), 6.89 (dd, $J_1$ = 83.6 Hz, $J_2$ = 5.6 Hz, 1H), 6.70 (d, $J$ = 86.8 Hz, 1H), 6.57 (s, 1H), 5.23 (dd, $J_1$ = 44.8 Hz, $J_2$ = 4.8 Hz, 1H), 5.07 (dd, $J_1$ = 25.2 Hz, $J_2$ = 16.0 Hz, 2H), 4.61 (d, $J$ = 10.8 Hz, 1H), 4.55 - 4.45 (m, 2H), 4.24 - 4.14 (m, 3H), 3.25 (s, 3H), 2.94 (s, 3H), 2.70 (d, $J$ = 10.8 Hz, 2H), 2.52 (m, 1H), 2.34 - 2.19 (m, 3H), 2.15 (s, 3H), 1.91 - 1.81 (m, 2H), 1.09 (s, 3H). $^{19}$F NMR (376 MHz, DMSO-$d_6$) δ - 122.79, -126.05, -138.84. The subsequently eluted isomer 2 was **Example 112B** (27.2 mg, relatively long retention time). Chiral analysis method SFC-112, Rt=1.90 min. LCMS(m/z): 742.3 (M+H). $^1$H NMR (400 MHz, DMSO-$d_6$) δ 8.17 (s, 1H), 7.80 - 7.78 (m, 3H), 7.24 (t, $J$ = 7.6 Hz, 1H), 7.11 (dd, $J_1$ = 7.2 Hz, $J_2$ = 0.8 Hz, 1H), 6.89 (dd, $J_1$ = 83.6 Hz, $J_2$ = 5.6 Hz, 1H), 6.72 (d, $J$ = 86.8 Hz, 1H), 6.57 (s, 1H), 5.23 (dd, $J_1$ = 44.8 Hz, $J_2$ = 5.2 Hz, 1H), 5.08 (dd, $J_1$ = 33.2 Hz, $J_2$ = 16.0 Hz, 2H), 4.58 (d, $J$ = 10.8 Hz, 1H), 4.52 - 4.49 (m, 2H), 4.25 (d, $J$ = 10.8 Hz, 1H), 4.22 - 4.12 (m, 2H), 3.24 (s, 3H), 2.94 (s, 3H), 2.68 (d, $J$ = 10.8 Hz, 2H), 2.53 (m, 1H), 2.30 - 2.17 (m, 3H), 2.15 (s, 3H), 1.93 - 1.83 (m, 2H), 1.10 (s, 3H). $^{19}$F NMR (376 MHz, DMSO-$d_6$) δ -122.78, -126.07, -138.80. Chiral analysis method SFC-112: Waters UPCC (CA-352), analytical column: DAICELCHIRALPAK®AD; mobile phase A: $CO_2$, mobile phase B: EtOH (0.1% DEA); Flow rate: 1.5 mL/min; Column temperature: 35°C; Back pressure: 1800 psi; Gradient: 0-8.0 min A/B=60/40.

| | | |
|---|---|---|
| 113 | 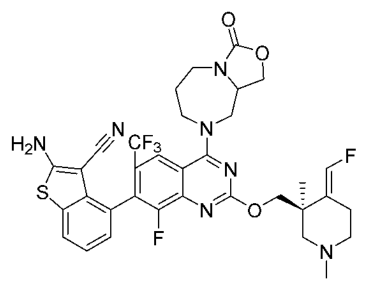 | LCMS (m/z): 714.2 (M+H). $^1$H NMR (400 MHz, DMSO-$d_6$) δ 8.17 (d, $J$ = 6.3 Hz, 1H), 7.86 - 7.76 (m, 3H), 7.27 - 7.15 (m, 2H), 6.73 (dd, $J$ = 86.8, 5.2 Hz, 1H), 4.71 - 4.57 (m, 1H), 4.49 - 3.89 (m, 9H), 3.83 - 3.74 (m, 1H), 3.21 - 3.10 (m, 1H), 2.76 - 2.64 (m, 2H), 2.57 - 2.56 (m, 1H), 2.20 - 2.13 (m, 5H), 1.97 - 1.83 (m, 2H), 1.14 (s, 3H). |
| 114 | 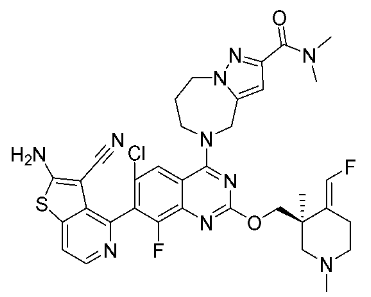 | LCMS (m/z): 733.2 (M+H). |

(continued)

| | | |
|---|---|---|
| 115 | 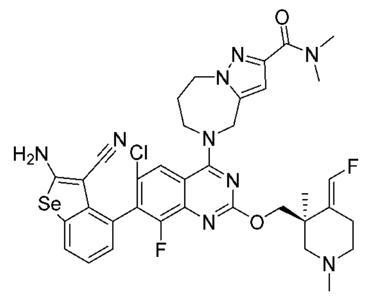 | LCMS (m/z): 780.2 (M+H). |
| 116 | 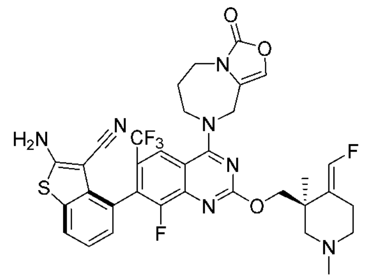 | LCMS (m/z): 712.2 (M+H). |
| 117 | 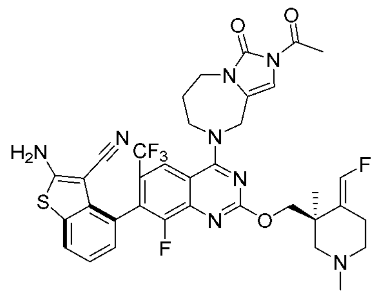 | LCMS (m/z): 753.2 (M+H). |
| 118 | 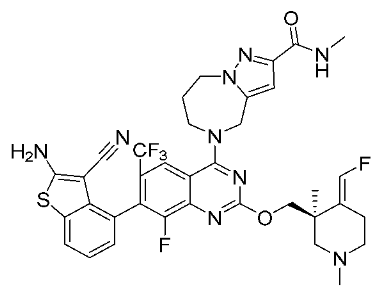 | LCMS (m/z): 752.3 (M+H). $^1$H NMR (400 MHz, DMSO-$d_6$) δ 8.13 (s, 1H), 8.08 - 7.99 (m, 1H), 7.85 - 7.74 (m, 3H), 7.27 - 7.12 (m, 2H), 6.86 - 6.57 (m, 2H), 5.22 - 5.10 (m, 1H), 5.02 - 4.90 (m, 1H), 4.68 - 4.58 (m, 1H), 4.55 - 4.41 (m, 2H), 4.36 - 4.21 (m, 2H), 4.16 - 4.01 (m, 1H), 2.78 - 2.63 $^{19}$F NMR MHz, DMSO-$d_6$) δ - 56.97, -123.20, -138.65. |
| 119 | 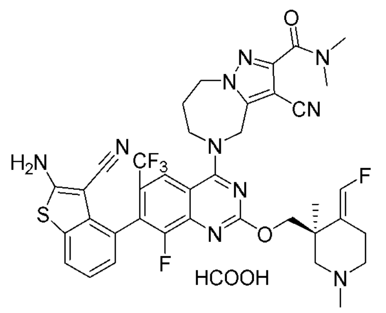 | LCMS (m/z): 776.3 (M+H). $^1$H NMR (400 MHz, DMSO-$d_6$) δ 8.19 (s, 1H), 8.17 - 8.13 (m, 1H), 7.87 - 7.71 (m, 3H), 7.25 - 7.19 (m, 1H), 7.18 - 7.10 (m, 1H), 6.66 (dd, $J$ = 86.6, 11.3 Hz, 1H), 5.24 - 5.05 (m, 2H), 4.54 - 4.37 (m, 3H), 4.37 - 4.28 (m, 2H), 4.26 - 4.19 (m, 1H), 3.19 (s, 3H), 3.00 (s, 3H), 2.78 - 2.71 (m, 1H), 2.70 - 2.62 (m, 1H), 2.49 - 2.40 (m, 2H), 2.39 - 2.29 (m, 1H), 2.19 - 2.02 (m, 4H), 1.90 - 1.82 (m, 1H), 1.78 (dd, $J$ = 11.3, 3.0 Hz, 1H), 1.07 (d, $J$ = 4.7 Hz, 3H). $^{19}$F NMR (376 MHz, DMSO-$d_6$) δ -57.23, -123.25, -138.50 - -138.75 (m). |

(continued)

| | | |
|---|---|---|
| 119A | 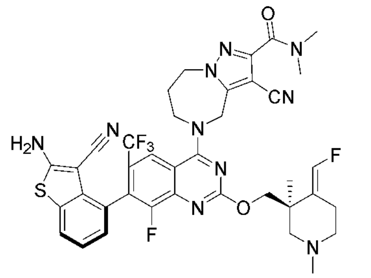 | LCMS (m/z): 791.3 (M+H). $^1$H NMR (400 MHz, DMSO-$d_6$) δ 8.15 (s, 1H), 7.84 - 7.79 (m, 2H), 7.78 - 7.75 (m, 1H), 7.27 - 7.19 (m, 1H), 7.18 - 7.11 (m, 1H), 6.77 - 6.51 (m, 1H), 5.24 - 5.05 (m, 2H), 4.52 - 4.38 (m, 3H), 4.37 - 4.26 (m, 2H), 4.25 - 4.17 (m, 1H), 3.19 (s, 3H), 3.00 (s, 3H), 2.77 - 2.70 (m, 1H), 2.69 - 2.63 (m, 1H), 2.47 - 2.27 (m, 3H), 2.20 - 2.14 (m, 1H), 2.12 (s, 3H), 1.89 - 1.81 (m, 1H), 1.81 - 1.75 (m, 1H), 1.07 (s, 3H). $^{19}$F NMR (376 MHz, DMSO-$d_6$) δ -57.24, -123.26, -138.68. |
| 119B | 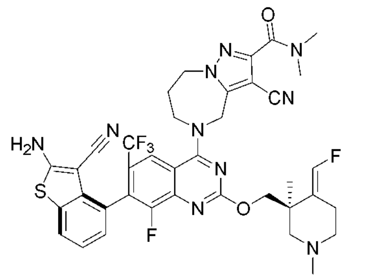 | LCMS(m/z): 791.2 (M+H). $^1$H NMR (400 MHz, DMSO-$d_6$) δ 8.16 (s, 1H), 7.80 (s, 2H), 7.78 (s, 1H), 7.26 - 7.18 (m, 1H), 7.18 - 7.11 (m, 1H), 6.78 (s, 0.5H), 6.57 (s, 0.5H), 5.15 (q, *J* = 16.3 Hz, 2H), 4.52 - 4.18 (m, 6H), 3.19 (s, 3H), 3.00 (s, 3H), 2.81 - 2.70 (m, 1H), 2.70 - 2.61 (m, 1H), 2.50 - 2.40 (m, 2H), 2.39 - 2.27 (m, 1H), 2.22 - 1.99 (m, 4H), 1.93 - 1.70 (m, 2H), 1.08 (s, 3H). $^{19}$F NMR (376 MHz, DMSO-$d_6$) δ -57.23, -123.24, -138.57. |
| 120 | 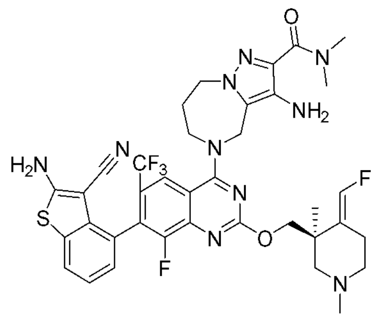 | LCMS (m/z): 781.3 (M+H). |
| 121 | 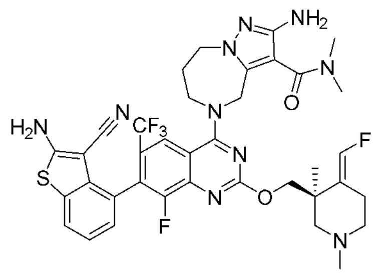 | LCMS (m/z): 781.3 (M+H). $^1$H NMR (400 MHz, DMSO-$d_6$) δ 8.10 (s, 1H), 7.88 -7.68 (m, 3H), 7.26 - 7.19 (m, 1H), 7.18 - 7.11 (m, 1H), 6.86 - 6.55 (m, 1H), 5.15 - 5.01 (m, 2H), 4.75 (s, 2H), 4.61 - 4.46 (m, 1H), 4.30 (dd, *J* = 10.7, 3.4 Hz, 1H), 4.24 - 3.97 (m, 4H), 2.86 (s, 6H), 2.73 - 2.61 (m, 2H), 2.48 - 2.44 (m, 1H), 2.40 - 2.16 (m, 3H), 2.13 (s, 3H), 1.94 - 1.79 (m, 2H), 1.16 - 1.03 (m, 3H). $^{19}$F NMR (376 MHz, DMSO-$d_6$) δ -57.25, -123.36, -138.75. |
| 122 | 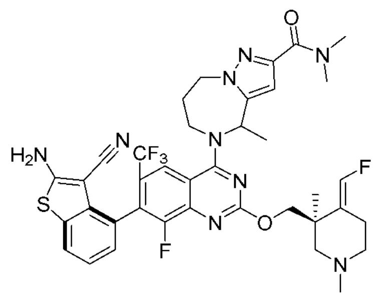 | LCMS (m/z): 780.3 (M+H). |

(continued)

| | | |
|---|---|---|
| 123 | 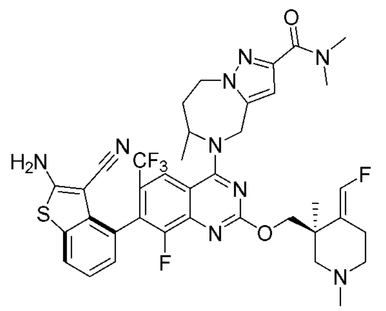 | LCMS (m/z): 780.3 (M+H). |
| 123A | 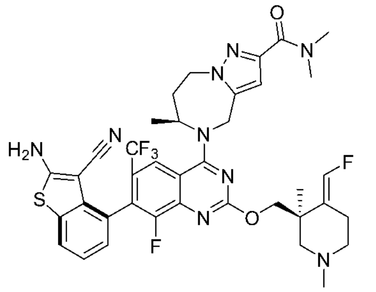 | $^1$H NMR (400 MHz, DMSO-$d_6$) δ 8.12 (s, 1H), 7.91 - 7.68 (m, 3H), 7.26 - 7.18 (m, 1H), 7.16 - 7.09 (m, 1H), 6.88 - 6.56 (m, 2H), 5.17 (d, *J* = 17.1 Hz, 1H), 4.98 - 4.76 (m, 2H), 4.59 - 4.39 (m, 2H), 4.34 - 4.10 (m, 2H), 3.25 (s, 3H), 2.96 (s, 3H), 2.73 - 2.60 (m, 2H), 2.48 - 2.28 (m, 3H), 2.15 (s, 3H), 2.13 - 2.04 (m, 1H), 1.98 - 1.75 (m, 2H), 1.55 (d, *J* = 6.1 Hz, 3H), 1.09 (s, 3H). $^{19}$F NMR (376 MHz, DMSO-$d_6$) δ -56.90, -123.09, -138.69. |
| 123B | 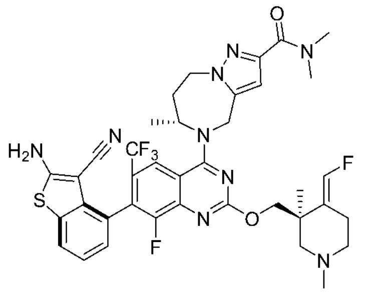 | LCMS(m/z): 780.1 (M+H). $^1$H NMR (400 MHz, DMSO-$d_6$) δ 8.12 (s, 1H), 7.85 - 7.75 (m, 3H), 7.24 - 7.18 (m, 1H), 7.18 - 7.13 (m, 1H), 6.80 (s, 0.5H), 6.61 (s, 1H), 6.58 (s, 0.5H), 5.07 - 4.96 (m, 1H), 4.96 - 4.86 (m, 1H), 4.85 - 4.72 (m, 1H), 4.59 - 4.51 (m, 1H), 4.51 - 4.41 (m, 1H), 4.25 - 4.12 (m, 1H), 4.09 - 3.92 (m, 1H), 3.25 (s, 3H), 2.96 (s, 3H), 2.74 - 2.61 (m, 2H), 2.49 - 2.40 (m, 2H), 2.40 - 2.27 (m, 1H), 2.26 - 2.17 (m, 1H), 2.15 (s, 3H), 1.95 - 1.86 (m, 1H), 1.86 - 1.78 (m, 1H), 1.58 (d, *J* = 6.3 Hz, 3H), 1.06 (s, 3H). $^{19}$F NMR (376 MHz, DMSO-$d_6$) δ -56.89, -123.19, -138.79. |
| 124 | 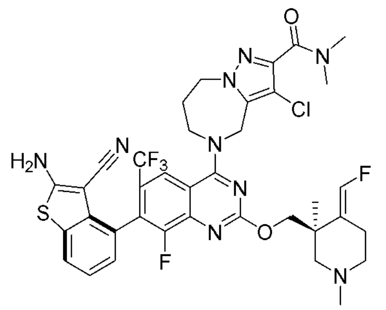 | LCMS (m/z): 800.2 (M+H). $^1$H NMR (400 MHz, DMSO-$d_6$) δ 8.06 (s, 1H), 7.84 - 7.73 (m, 3H), 7.25 - 7.18 (m, 1H), 7.18 - 7.13 (m, 1H), 6.81 (s, 0.5H), 6.59 (s, 0.5H), 5.18 - 5.07 (m, 1H), 5.04 - 4.94 (m, 1H), 4.52 - 4.35 (m, 3H), 4.35 - 4.27 (m, 1H), 4.27 - 4.17 (m, 2H), 3.01 (s, 3H), 2.98 (s, 3H), 2.78 - 2.63 (m, 2H), 2.48 - 2.31 (m, 3H), 2.26 - 2.01 (m, 4H), 1.97 - 1.77 (m, 2H), 1.09 (s, 3H). $^{19}$F NMR (377 MHz, DMSO-$d_6$) δ -57.24, -123.31, -138.69. |
| 125 | 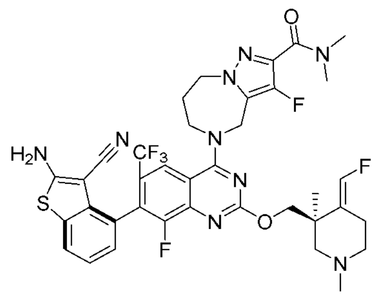 | LCMS (m/z): 784.3 (M+H). |

(continued)

| | | |
|---|---|---|
| 126 | 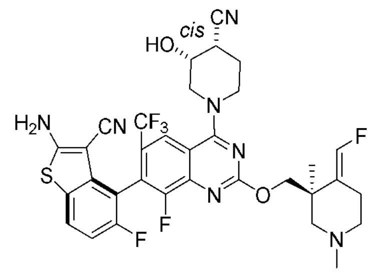 | LCMS (m/z): 702.2 (M+H). |
| 126A | 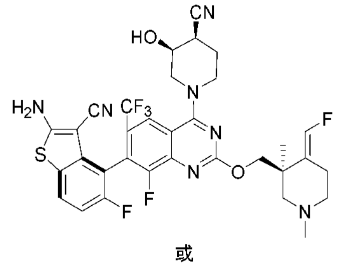<br>或<br>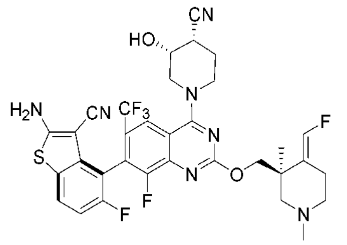<br>Prepared from intermediate n-7A | LCMS (m/z): 702.2 (M+H). $^1$H NMR (400 MHz, DMSO-$d_6$) δ 8.15 (s, 1H), 7.96 (s, 2H), 7.89 - 7.79 (m, 1H), 7.19 - 7.09 (m, 1H), 6.87 - 6.76 (m, 0.5H), 6.66 - 6.58 (m, 0.5H), 6.05 - 5.96 (m, 1H), 4.70 - 4.50 (m, 1H), 4.40 - 4.27 (m, 2H), 4.15 - 4.02 (m, 1H), 4.00 - 3.91 (m, 1H), 3.63 - 3.51 (m, 1H), 3.28 - 3.17 (m, 1H), 3.04 - 2.93 (m, 1H), 2.76 - 2.66 (m, 2H), 2.57 - 2.52 (m, 1H), 2.29 - 2.19 (m, 2H), 2.14 (s, 3H), 2.05 - 1.78 (m, 3H), 1.11 (s, 3H). $^{19}$F NMR (376 MHz, DMSO-$d_6$) δ -59.34, -117.73, - 122.92, -138.70. |
| 126B | 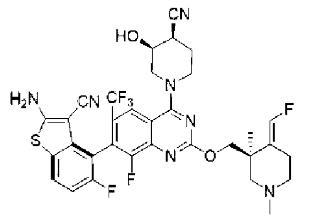<br>or<br>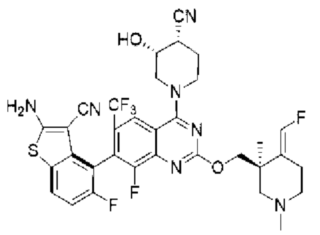<br>Prepared from intermediate **n-7B** | LCMS (m/z): 702.2 (M+H). $^1$H NMR (400 MHz, DMSO-$d_6$) δ 8.22 (s, 1H), 7.96 (s, 2H), 7.85 (dd, $J$ = 8.7, 5.0 Hz, 1H), 7.25 - 7.06 (m, 1H), 6.89 - 6.55 (m, 1H), 6.01 (d, $J$ = 5.3 Hz, 1H), 4.62 (d, $J$ = 10.6 Hz, 1H), 4.32 (d, $J$ = 10.6 Hz, 1H), 4.26 - 4.03 (m, 2H), 4.00 - 3.83 (m, 1H), 3.52 - 3.39 (m, 1H), 3.31 - 3.28 (m, 1H), 3.05 - 2.91 (m, 1H), 2.77 - 2.65 (m, 2H), 2.55 - 2.52 (m, 1H), 2.29 - 2.17 (m, 2H), 2.14 (s, 3H), 2.04 - 1.77 (m, 3H), 1.11 (s, 3H). $^{19}$F NMR (376 MHz, DMSO-$d_6$) δ -59.33, -117.75, - 123.02, -138.71. |
| 127 | 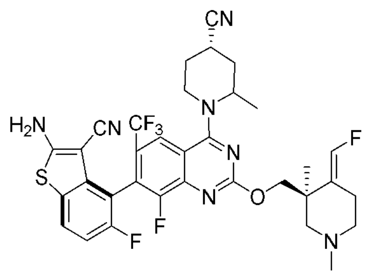 | LCMS (m/z): 700.2 (M+H). |

(continued)

| | | |
|---|---|---|
| 128 | 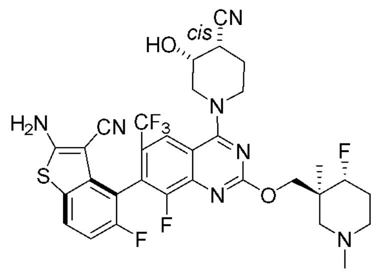 | LCMS (m/z): 690.2 (M+H). |
| 129 | 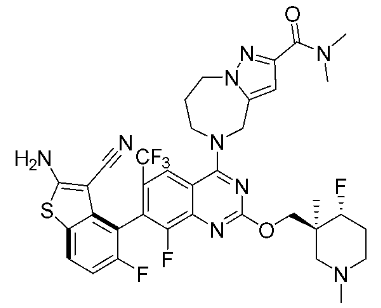 | LCMS (m/z): 772.3 (M+H). |
| 130 | 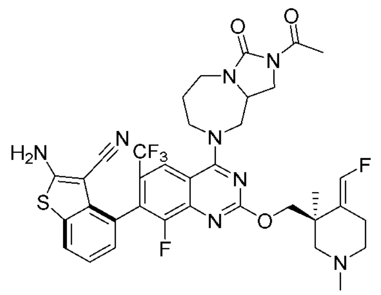 | LCMS (m/z): 755.2 (M+H). |
| 131 | 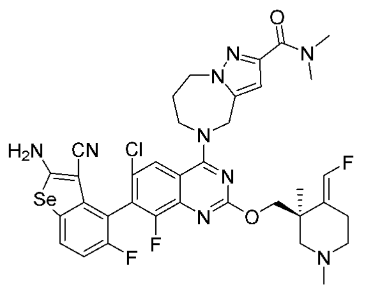 | LCMS (m/z): 798.2 (M+H). |
| 132 | 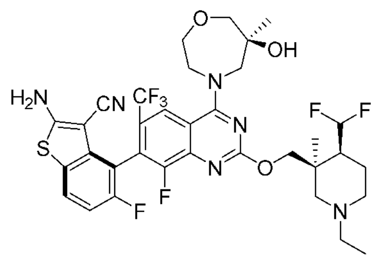 | LCMS (m/z): 741.2 (M+H). |
| 133 | 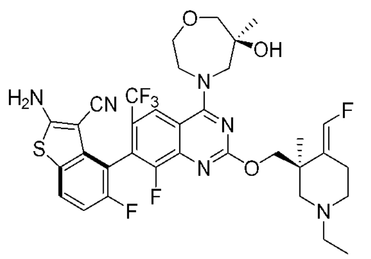 | LCMS (m/z): 721.2 (M+H). |

(continued)

| | | |
|---|---|---|
| 134 | 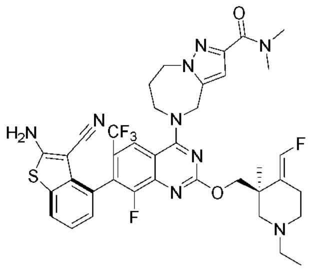 | LCMS (m/z): 780.3 (M+H). |
| 135 | 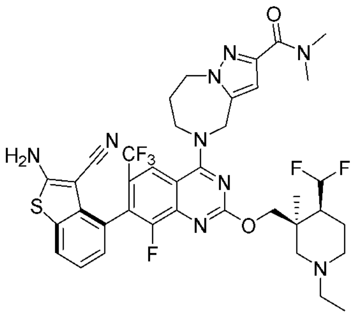 | LCMS (m/z): 800.3 (M+H). |
| 136 | 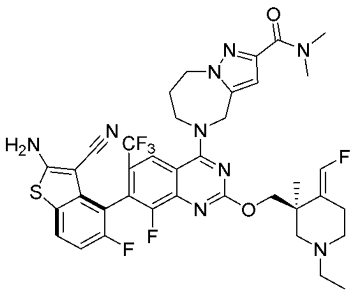 | LCMS (m/z): 798.3 (M+H). |
| 137 | 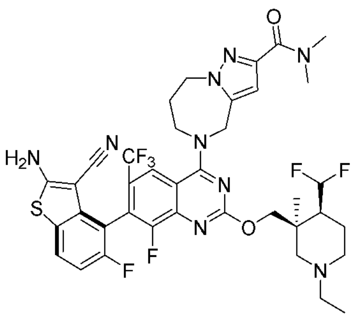 | LCMS (m/z): 818.3 (M+H). |
| 138 | 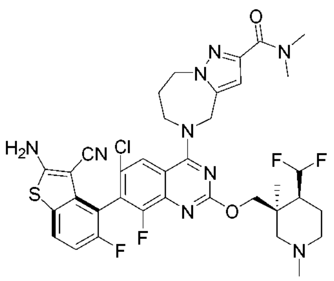 | LCMS (m/z): 770.2 (M+H). |

(continued)

| | | |
|---|---|---|
| 139 | 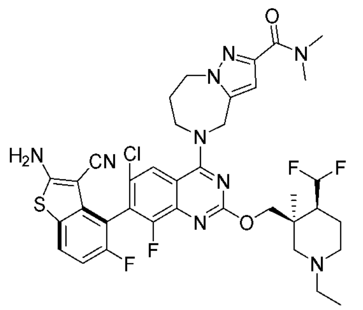 | LCMS (m/z): 784.3 (M+H). |
| 140 | 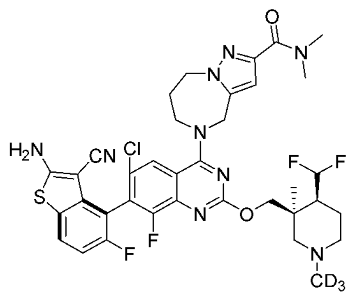 | LCMS (m/z): 773.3 (M+H). |
| 141 | 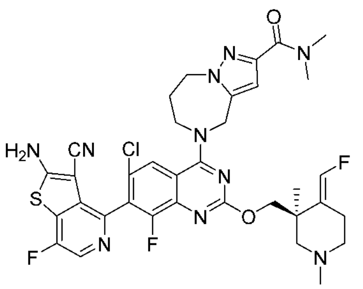 | LCMS (m/z): 751.2 (M+H). |
| 142 | 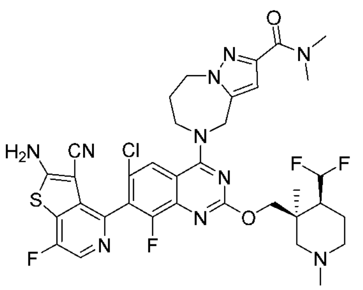 | LCMS (m/z): 771.2 (M+H). |
| 143 | 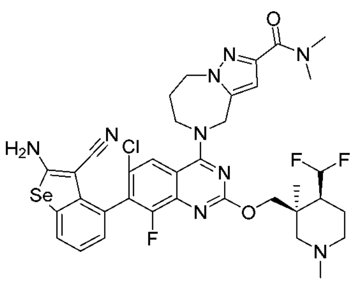 | LCMS (m/z): 800.2 (M+H). |

(continued)

| | | |
|---|---|---|
| 144 | 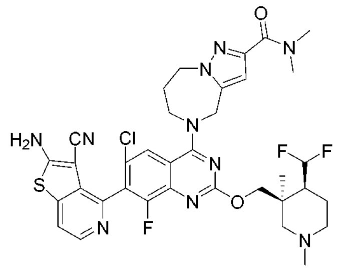 | LCMS (m/z): 753.2 (M+H). |
| 145 | 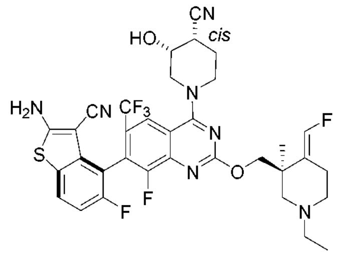 | LCMS (m/z): 716.2 (M+H). |
| 146 | 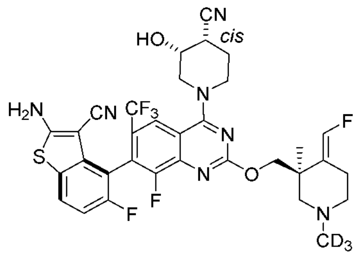 | LCMS (m/z): 705.2 (M+H). |
| 147 | 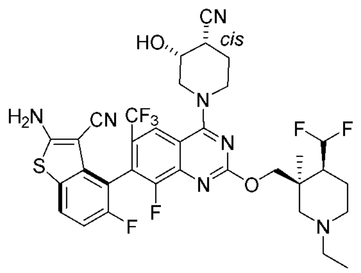 | LCMS (m/z): 736.2 (M+H). |
| 148 | 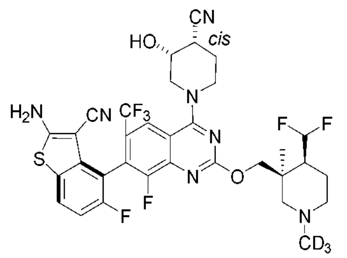 | LCMS (m/z): 725.2 (M+H). |
| 149 | 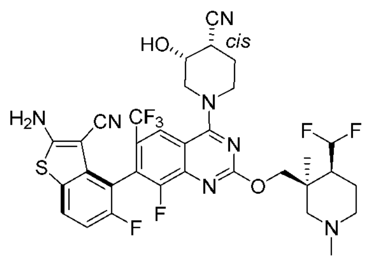 | LCMS (m/z): 722.2 (M+H). |

(continued)

| | | |
|---|---|---|
| 150 | 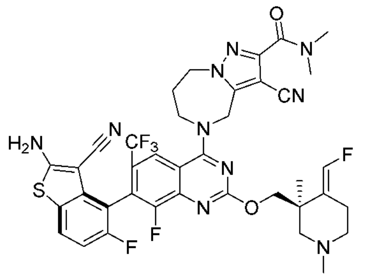 | LCMS (m/z): 809.3 (M+H). $^1$H NMR (400 MHz, DMSO-$d_6$) δ 8.20 (s, 1H), 7.96 (s, 2H), 7.84 (dd, *J* = 8.7, 4.9 Hz, 1H), 7.16 - 7.09 (m, 1H), 6.79 - 6.56 (m, 1H), 5.27 - 5.16 (m, 1H), 5.13 - 5.05 (m, 1H), 4.49 - 4.40 (m, 2H), 4.39 - 4.27 (m, 3H), 4.27 - 4.21 (m, 1H), 3.19 (s, 3H), 3.00 (s, 3H), 2.78 - 2.70 (m, 1H), 2.70 - 2.62 (m, 1H), 2.49 - 2.41 (m, 2H), 2.40 - 2.31 (m, 1H), 2.12 (s, 3H), 2.09 - 2.03 (m, 1H), 1.89 - 1.81 (m, 1H), 1.80 - 1.74 (m, 1H), 1.08 (s, 3H). $^{19}$F NMR (376 MHz, DMSO-$d_6$) δ -59.29, -117.62, -122.71, -138.58. |
| 151 | 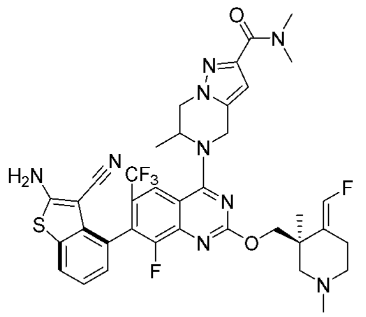 | LCMS (m/z): 766.3 (M+H). |
| 152 | 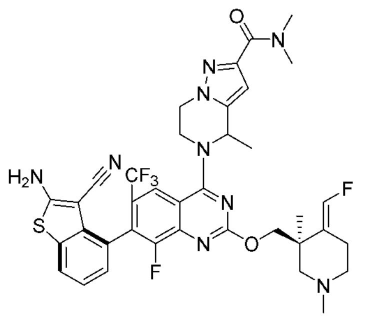 | LCMS (m/z): 766.3 (M+H). |
| 153 | 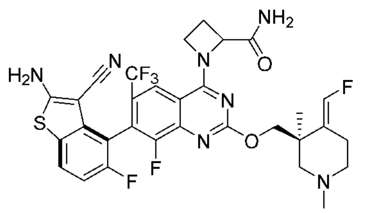 | LCMS (m/z): 676.2 (M+H). |
| 153A | 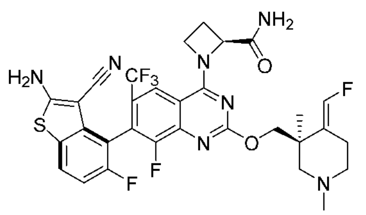 | LCMS (m/z): 676.2 (M+H). |
| 153B | 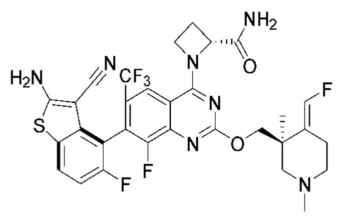 | LCMS (m/z): 676.2 (M+H). |

(continued)

| | | |
|---|---|---|
| 154 | 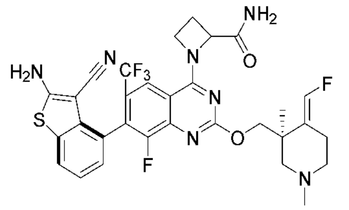 | LCMS (m/z): 658.2 (M+H). |
| 154A | 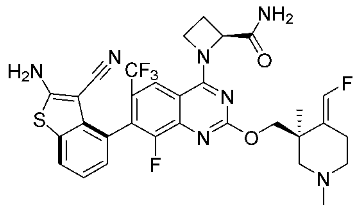 | LCMS (m/z): 658.2 (M+H). |
| 154B | 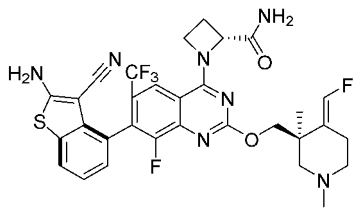 | LCMS (m/z): 658.2 (M+H). |
| *155* | 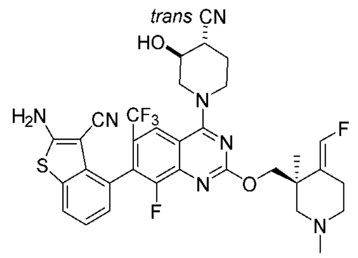 | LCMS (m/z): 684.3 (M+H). |
| 156 | 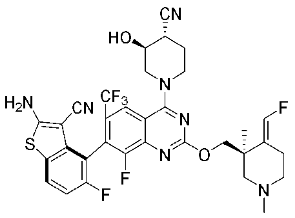 or 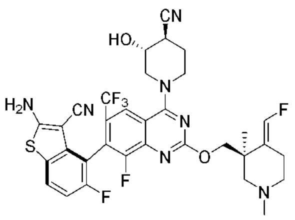 Prepared from intermediate **n-6B** | LCMS (m/z): 702.2 (M+H). $^{1}$H NMR (400 MHz, DMSO-$d_6$) $\delta$ 8.16 (s, 1H), 7.96 (s, 2H), 7.85 (dd, *J* = 8.7, 4.9 Hz, 1H), 7.20 - 7.06 (m, 1H), 6.88 - 6.53 (m, 1H), 6.01 (d, *J* = 5.2 Hz, 1H), 4.61 (d, *J* = 10.6 Hz, 1H), 4.42 - 4.25 (m, 2H), 4.16 - 3.91 (m, 2H), 3.67 - 3.43 (m, 1H), 3.24 (dd, *J* = 13.2, 8.9 Hz, 1H), 3.04 - 2.89 $^{19}$F NMR (376 MHz, DMSO-$d_6$) $\delta$ -59.34 (d, *J* = 6.7 Hz), -115.60 - -118.99 (m), -122.92, -138.67 (d, *J* = 19.8 Hz). |

(continued)

| | | |
|---|---|---|
| 157 | 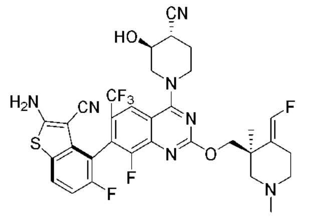<br>or<br>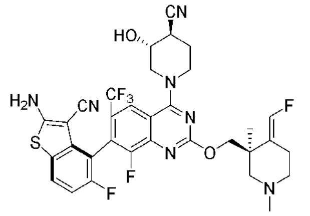<br>Prepared from intermediate **n-6A** | LCMS (m/z): 702.2 (M+H). $^1$H NMR (400 MHz, DMSO-$d_6$) δ 8.28 (s, 1H), 8.02 (s, 2H), 7.96 - 7.84 (m, 1H), 7.29 - 7.14 (m, 1H), 6.96 - 6.84 (m, 0.5H), 6.74 - 6.62 (m, 0.5H), 6.08 (d, J = 5.3 Hz, 1H), 4.74 - 4.61 (m, 1H), 4.44 - 4.35 (m, 1H), 4.33 - 4.25 (m, 1H), 4.22 - 4.13 (m, 1H), 4.06 - 3.93 (m, 1H), 3.58 - 3.48 (m, 1H), 3.39 - 3.33 (m, 1H), 3.10 - 3.00 (m, 1H), 2.85 - 2.70 (m, 2H), 2.64 - 2.59 (m, 1H), 2.34 - 2.26 (m, 2H), 2.21 (s, 3H), 2.11 - 1.86 (m, 3H), 1.18 (s, 3H). $^{19}$F NMR (376 MHz, DMSO-$d_6$) δ - 59.34, -117.75, -123.03, -138.68. |
| 158 | 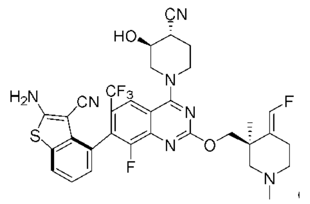<br>or<br>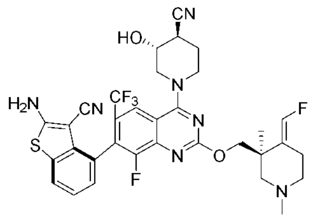<br>Prepared from intermediate **n-6A** | LCMS (m/z): 702.2 (M+H). $^1$H NMR (400 MHz, DMSO-$d_6$) δ 8.12 (s, 1H), 7.90 - 7.70 (m, 3H), 7.30 - 7.08 (m, 2H), 6.89 - 6.80 (m, 0.5H), 6.69 - 6.60 (m, 0.5H), 6.11 - 5.96 (m, 1H), 4.72 - 4.57 (m, 1H), 4.42 - 4.25 (m, 2H), 4.13 - 4.01 (m, 1H), 3.99 - 3.89 (m, 1H), 3.64 - 3.49 (m, 1H), 3.28 - 3.17 (m, 1H), 2.78 - 2.64 (m, 2H), 2.57 - 2.52 (m, 1H), 2.28 - 2.09 (m, 5H), 2.04 - 1.80 (m, 3H), 1.12 (s, 3H). $^{19}$F NMR (376 MHz, DMSO-$d_6$) δ -57.28, -123.48, -138.65. |
| 159 | 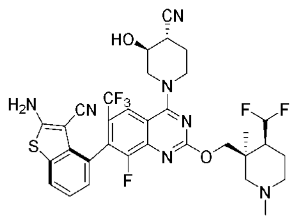<br>or<br>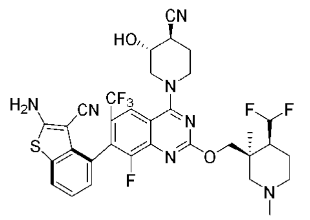<br>Prepared from intermediate **n-6A** | LCMS (m/z): 704.2 (M+H). $^1$H NMR (400 MHz, DMSO-$d_6$) δ 8.13 (s, 1H), 7.89 - 7.73 (m, 3H), 7.32 - 7.10 (m, 2H), 6.52 - 6.14 (m, 1H), 6.02 (d, *J* = 5.5 Hz, 1H), 4.47 (s, 2H), 4.37 - 4.20 (m, 1H), 4.14 - 4.04 (m, 1H), 4.00 - 3.89 (m, 1H), 3.63 - 3.50 (m, 1H), 3.30 - 3.21 (m, 1H), 3.07 - 2.95 (m, 1H), 2.90 - 2.75 (m, 2H), 2.30 - 2.21 (m, 1H), 2.11 (s, 3H), 2.05 - 1.93 (m, 1H), 1.90 - 1.56 (m, 5H), 1.13 (s, 3H). $^{19}$F NMR (376 MHz, DMSO-$d_6$) δ -57.29, -115.77, -118.85, -123.35. |

(continued)

| | | |
|---|---|---|
| 160 | 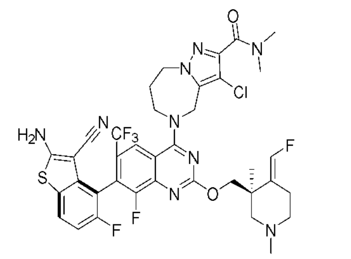 | LCMS (m/z): 818.2 (M+H). $^1$H NMR (400 MHz, DMSO-$d_6$) δ 8.11 (s, 1H), 7.96 (s, 2H), 7.84 (dd, J = 8.7, 4.9 Hz, 1H), 7.17 - 7.07 (m, 1H), 6.91 - 6.44 (m, 1H), 5.22 - 4.92 (m, 2H), 4.56 - 4.15 (m, 6H), 2.99 (d, J = 13.1 Hz, 6H), 2.73 - 2.60 (m, 2H), 2.54 - 2.51 (m, 1H), 2.44 - 2.29 (m, 2H), 2.20 - 2.05 (m, 4H), 2.04 - 1.77 (m, 2H), 1.08 (s, 3H). $^{19}$F NMR (377 MHz, DMSO-$d_6$) δ -59.30, -117.73, -122.83, -138.68. |

**Examples 161A and 161B**

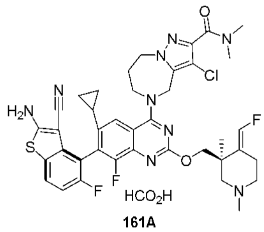

161A

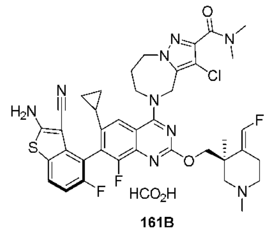

161B

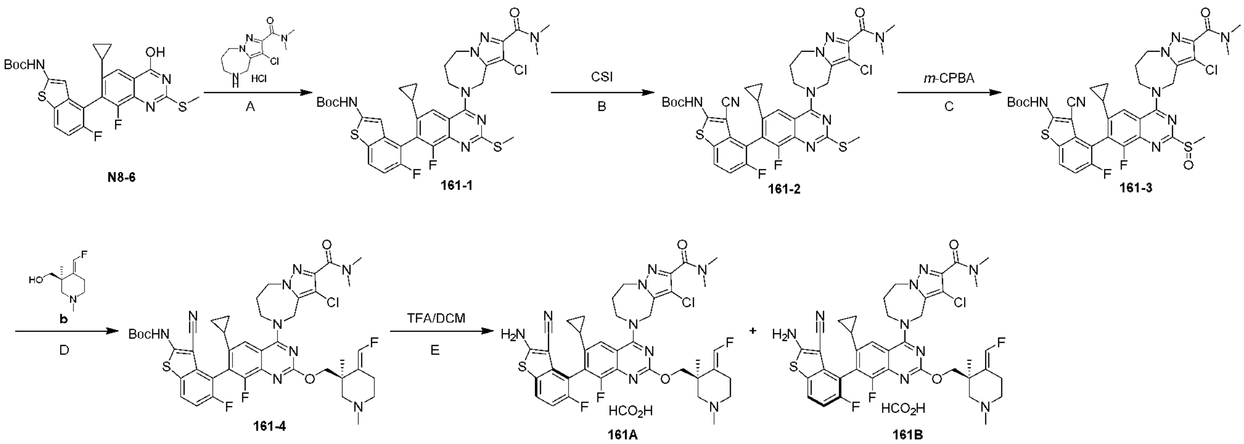


**Step G:** Compound **161-1**

At room temperature, Compound **N8-6** (200 mg, 0.388 mmol), 3-chloro-*N,N*-dimethyl-5,6,7,8-tetrahydro-4*H*-pyrazolo[1,5-*a*][1,4]diazepine-2-formamide·formate (CAS: 2791277-01-3, 129 mg, 0.466 mmol), BOP (246 mg, 0.582 mmol), and DBU (295 mg, 1.94 mmol) were sequentially added to DMF (5 mL). The resulting reaction solution was reacted at 55°C for 1 h. The reaction completion was monitored by LCMS and TLC. The reaction solution was then poured into water (30 mL), extracted with EA (30 mL×3), the organic phase was collected, washed with saturated brine (30 mL), and dried over anhydrous $Na_2SO_4$. The mixture was filtered, and the organic phase was concentrated and purified by FCC ($SiO_2$, EA/PE=0-82%) to yield a yellow solid compound **161-1** (175 mg, yield 61%). LCMS (m/z): 740.2 (M+H).

**Step H:** Compound **161-2**

Under an ice-salt bath (-10°C), CSI (118 mg, 0.839 mmol) was added to a solution of compound **161-1** (155 mg, 0.210 mmol) in MeCN (15 mL). The reaction mixture was stirred at the same temperature for 1 h, and then 5 mL of DMF was added to the reaction solution. The resulting reaction solution was allowed to continue reacting at room temperature for another 1 h. The reaction completion was monitored by TLC, and then the reaction solution was poured into a semi-saturated solution of $NaHCO_3$ (30 mL), extracted with EA (30 mL×3), the organic phase was collected, washed with saturated brine (30 mL), dried over anhydrous $Na_2SO_4$, filtered, and the organic phase was concentrated and purified by

FCC ($SiO_2$, THF/PE 0-68%) to yield a white solid compound **161-2** (166 mg, 100% yield). LCMS (m/z): 765.0 (M+H).

**Step I:** Compound **161-3**

At room temperature, *m*-CPBA (53 mg, 0.26 mmol) was added to a solution of compound **161-2** (170 mg, 0.23 mmol) in DCM (3 mL), and stirred at room temperature for 1 h. After the reaction completion was monitored by LCMS, the reaction solution was quenched with $NaHCO_3$ (30 mL) solution, extracted with DCM (30 mL×3), the organic phase was collected, washed with saturated saline solution, and dried over anhydrous $Na_2SO_4$, concentrated to yield a yellow solid compound **161-3** (177 mg, crude), which was used directly in the next step. LCMS (m/z): 781.1 (M+H).

**Step J:** Compound **161-3**

Under dry ice ethanol bath conditions (-70°C), *t*BuONa (0.23 mL, 2 M in THF, 0.45 mmol) was added to a solution of compound **161-3** (87 mg, 0.11 mmol), intermediate b (29 mg, 0.17 mmol) and THF (3 mL). The resulting reaction solution was allowed to react for another 1 h at room temperature. The reaction completion was monitored by TLC, the reaction solution was poured into a semi-saturated $NH_4Cl$ solution, extracted with EA (30 mL×3), the organic phase was collected, washed with saturated saline solution, and dried over anhydrous $Na_2SO_4$. The organic phase was concentrated and purified by FCC ($SiO_2$, MeOH/DCM=0-4%) to yield a brown solid compound **161-4** (90 mg, 91% yield). LCMS (m/z): 890.1 (M+H).

**Step K: Examples 161A and 161B**

A mixture of compound **161-4** (90 mg) and TFA/DCM (V/V=1:1, 5 mL) was stirred at room temperature for 1 h. After the reaction completion was detected by LCMS, the mixture was concentrated and saturated sodium bicarbonate aqueous solution (30 mL) was added, followed by extraction using EA (30 mL×2). The extract was collected, washed with saline (30 mL), dried over anhydrous $Na_2SO_4$, and the organic phase was concentrated and purified by pre-HPLC (C18, ACN/(10 mmol FA/$H_2O$)=35-45%) to obtain a white solid isomer 1, which was Example **161A** ($Rt_1$=7.0 min) (13.1 mg, yield 15%). LCMS (m/z): 790.2 (M+H). $^1$H NMR (400 MHz, DMSO-*d*$_6$) δ 8.38 (s, 1H), 7.90 (s, 2H), 7.81 (dd, *J* = 8.7, 5.0 Hz, 1H), 7.28 (s, 1H), 7.18 - 7.08 (m, 1H), 6.83 - 6.53 (m, 1H), 5.15 - 4.86 (m, 2H), 4.47 - 4.38 (m, 3H), 4.28 - 4.13 (m, 3H), 3.02 (s, 3H), 2.97 (s, 3H), 2.71 - 2.65 (m, 2H), 2.58 - 2.51 (m, 1H), 2.38 - 2.28 (m, 2H), 2.19 - 2.09 (m, 4H), 1.95 - 1.78 (m, 2H), 1.55 - 1.47 (m, 1H), 1.08 (s, 3H), 0.69 - 0.59 (m, 4H). $^{19}$F NMR (376 MHz, DMSO-*d*$_6$) δ - 118.66, -126.21, -138.90; obtained a white solid isomer 2, which was Example **161B** ($Rt_2$=9.0 min) (14.8 mg, yield 17 %). LCMS (m/z): 790.2 (M+H). $^1$H NMR (400 MHz, DMSO-*d*$_6$) δ 8.30 (s, 1H), 7.89 (s, 2H), 7.81 (dd, *J* = 8.7, 4.9 Hz, 1H), 7.28 (s, 1H), 7.18 - 7.09 (m, 1H), 6.83 - 6.53 (m, 1H), 5.09 - 4.94 (m, 2H), 4.54 - 4.39 (m, 3H), 4.26 - 4.10 (m, 3H), 3.02 (s, 3H), 2.97 (s, 3H), 2.72 - 2.65 (m, 2H), 2.49 - 2.46 (m, 1H), 2.40 - 2.27 (m, 2H), 2.24 - 2.15 (m, 1H), 2.13 (s, 3H), 1.93 - 1.77 (m, 2H), 1.57 - 1.46 (m, 1H), 1.07 (s, 3H), 0.73 - 0.55 (m, 4H). $^{19}$F NMR (376 MHz, DMSO-*d*$_6$) δ - 118.63, -126.20, -138.94.

**Examples 162A and 162B**

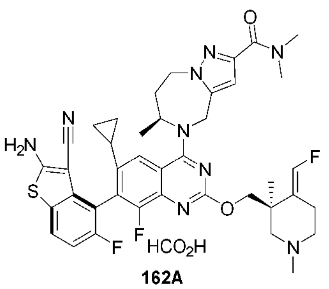

162A

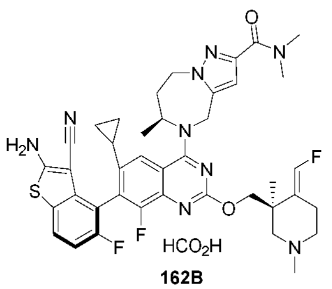

162B

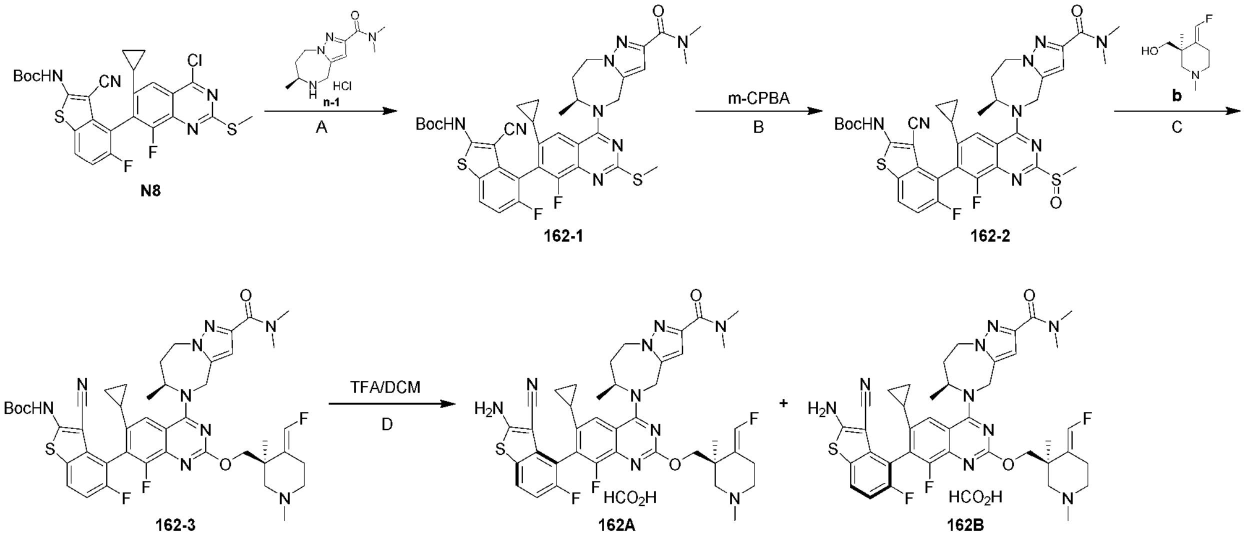


N8, 162-1, 162-2, 162-3, 162A, 162B

**Step A:** Compound **162-1**

At room temperature, Intermediates **N8** (100 mg, 0.18 mmol) and **n-1** (60 mg, 0.234 mmol) and DIEA (116 mg, 0.90 mmol) were sequentially added to DMF (3 mL). The resulting reaction solution was reacted at 35°C for 16 h. The reaction completion was monitored by LCMS and TLC. The reaction solution was poured into 30 mL of water, extracted with EA (30 mL×3), and the organic phase was collected. The organic phase was washed with saturated brine (30 mL) and dried over anhydrous $Na_2SO_4$. After filtration, the organic phase was concentrated and purified by FCC ($SiO_2$, EA/PE=0-20%) to give a yellow solid compound **162-1** (80 mg, yield 60%). LCMS (m/z): 745.3 (M+H).

**Step B:** Compound **162-2**

At room temperature, *m*-CPBA (26 mg, 0.13 mmol) was added to a solution of compound **162-1** (80 mg, 0.11 mmol) in DCM (3 mL), and stirred at room temperature for 1 h. After reaction completion was monitored by LCMS, the reaction solution was quenched by pouring it into a solution of $NaHCO_3$ (30 mL), and extracted with DCM (30 mL×3), the organic phase was collected, washed with saturated saline solution, and dried over anhydrous $Na_2SO_4$, concentrated to yield a yellow solid compound **162-2** (80 mg, crude), which was used directly in the next step. LCMS (m/z): 761.3 (M+H).

**Step C:** Compound **162-3**

Under dry ice-ethanol bath conditions (-70°C), *t*BuONa (0.21 mL, 2 M in THF, 0.42 mmol) was added to a solution of compound **162-2** (80 mg, 0.11 mmol), Intermediate **b** (27 mg, 0.16 mmol), and THF (3 mL). The resulting reaction solution was allowed to react for another 1 h at room temperature. The reaction completion was monitored by TLC, the solution was poured into a semi-saturated $NH_4Cl$ solution (30 mL), and extracted with EA (30 mL×3), the organic phase was collected, washed with saturated saline solution, and dried over anhydrous $Na_2SO_4$. The organic phase was concentrated and purified by FCC ($SiO_2$, MeOH/DCM=0-4%) to yield a brown solid compound **162-3** (81 mg, 88 % yield). LCMS (m/z): 870.3 (M+H).

**Step D: Examples 162A and 162B**

A mixture of compound **162-3** (81 mg) and TFA/DCM (V/V=1:1, 5 mL) was stirred at room temperature for 1 h. The reaction completion was monitored by LCMS. After concentration, saturated sodium bicarbonate aqueous solution (30 mL) was added, and the mixture was extracted with EA (30 mL×2). The extract was collected, washed with brine (30 mL), dried over anhydrous $Na_2SO_4$, and the organic phase was concentrated and purified by pre-HPLC (C18, ACN/(10 mmol FA/$H_2O$)=35-45%) to obtain a white solid isomer 1, which was **Example 162A** ($Rt_1$=5.8 min) (34.2 mg, yield 45%). LCMS (m/z): 770.3 (M+H). $^1H$ NMR (400 MHz, DMSO-$d_6$) δ 8.32 (s, 1H), 7.89 (s, 2H), 7.83 - 7.78 (m, 1H), 7.30 (s, 1H), 7.18 - 7.09 (m, 1H), 6.82 - 6.54 (m, 2H), 5.11 (d, *J* = 17.0 Hz, 1H), 4.87 - 4.70 (m, 2H), 4.50 - 4.33 (m, 2H), 4.25 - 4.07 (m, 2H), 3.27 (s, 3H), 2.96 (s, 3H), 2.69 - 2.59 (m, 2H), 2.47 - 2.38 (m, 2.33-2.24 (m, 1H), 2.14 (s, 3H), 2.13-2.03 (m, 1H), 1.96-1.79 (m, 2H), 1.59-1.49 (m, 4H), 1.07 (s, 3H), 0.77-0.45 (m, 4H). $^{19}F$ NMR (376 MHz, DMSO-$d_6$) δ -118.45, -125.73, - 138.95; obtained a white solid isomer 2, which was **Example 162B** ($Rt_2$=7.8 min) (12.5 mg, yield 16 %). LCMS (m/z): 770.3 (M+H). $^1H$ NMR

(400 MHz, DMSO-$d_6$) δ 8.36 (s, 1H), 7.90 (s, 2H), 7.83 - 7.77 (m, 1H), 7.28 (s, 1H), 7.16 - 7.08 (m, 1H), 6.82 - 6.55 (m, 2H), 4.99 (d, $J$ = 16.9 Hz, 1H), 4.85 - 4.71 (m, 2H), 4.50 - 4.41 (m, 2H), 4.23 (d, $J$ = 10.6 Hz, 1H), 4.07 - 3.93 (m, 1H), 3.26 (s, 3H), 2.96 (s, 3H), 2.69 - 2.63 (m, 2H), 2.47 - 2.34 (m, 2H), 2.31 - 2.15 (m, 2H), 2.14 (s, 3H), 1.94 - 1.78 (m, 2H), 1.56 (d, $J$ = 6.3 Hz, 3H), 1.52 - 1.42 (m, 1H), 1.07 (s, 3H), 0.72 - 0.42 (m, 4H). $^{19}$F NMR (376 MHz, DMSO-$d_6$) δ -118.79, -126.47, -138.96.

**Examples 163A and 163B**

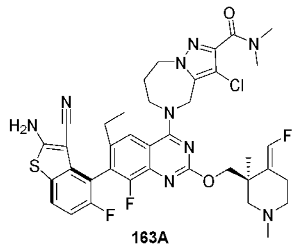

163A

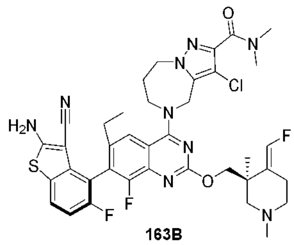

163B

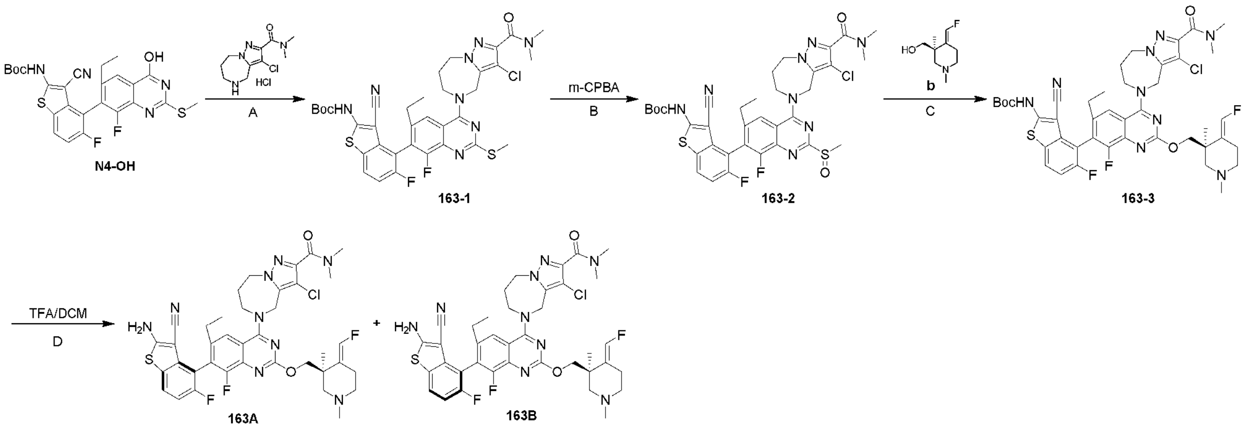


**Step A:** Compound **163-1**

At room temperature, intermediate **N4-OH** (200 mg, 0.38 mmol), 3-chloro-*N,N*-dimethyl-5,6,7,8-tetrahydro-4*H*-pyrazolo[1,5-*a*][1,4]diazepine-2-formamide-hydrochloride (CAS: 2791277-01-3, 127 mg, 0.45 mmol), BOP (241 mg, 0.57 mmol), and DBU (288 mg, 1.89 mmol) were sequentially added to DMF (5 mL). The resulting reaction solution was stirred at 55°C for 1 h. The reaction completion was monitored by LCMS and TLC. The reaction solution was poured into water (30 mL), extracted with EA (30 mL×3), the organic phase was collected, washed with saturated brine (30 mL), and dried over anhydrous $Na_2SO_4$. After filtration, the organic phase was concentrated and purified by FCC ($SiO_2$, THF/PE=0-64%) to yield a yellow solid compound **163-1** (180 mg, yield 63%). LCMS (m/z): 753.1 (M+H).

**Steps B-C:** The synthesis of compound **163-3** was carried out in accordance with the relevant steps in **Examples 162A/162B.**

**Step D: Examples 163A and 163B**

At room temperature, a mixture of compound **163-3** (200 mg) and DCM/TFA (V/V=1:1, 8 mL) was stirred for 1 h. After the reaction completion was monitored by LCMS, the mixture was concentrated and saturated sodium bicarbonate aqueous solution (50 mL) was added. Then, it was extracted with EA (50 mL×2) and the extract was collected. The extract was washed with brine (50 mL), dried over anhydrous $Na_2SO_4$, and the organic phase was concentrated and purified by pre-HPLC (C18, ACN/(10 mmol FA/$H_2O$)=25-45%) to obtain a white solid isomer 1, which was Example **163A.** ($Rt_1$=11.5 min) (26.5 mg, yield 15%). LCMS (m/z): 778.2 (M+H). $^1$H NMR (400 MHz, DMSO-$d_6$) δ 7.90 (s, 2H), 7.82 (dd, $J$ = 8.7, 5.0 Hz, 1H), 7.59 (s, 1H), 7.17 - 7.09 (m, 1H), 6.83 - 6.55 (m, 1H), 5.10 - 4.93 (m, 2H), 4.49 - 4.41 (m, 3H), 4.29 - 4.15 (m, 3H), 3.01 (s, 3H), 2.97 (s, 3H), 2.71 - 2.65 (m, 2H), 2.49 - 2.46 (m, 1H), 2.45 - 2.28 (m, 4H), 2.13 (s, 3H), 1.95 - 1.79 (m, 2H), 1.09 (s, 3H), 0.97 (t, $J$ = 7.5 Hz, 3H). $^{19}$F NMR (376 MHz, DMSO-$d_6$) δ -118.75, -125.74, -138.91; obtained a white solid isomer 2, which was Example **163B.** ($Rt_2$=12.7 min) (20.7 mg, yield 12%). LCMS (m/z): 778.2 (M+H). $^1$H NMR (400 MHz, DMSO-$d_6$)

δ 7.90 (s, 2H), 7.82 (dd, *J* = 8.7, 5.0 Hz, 1H), 7.58 (s, 1H), 7.17 - 7.09 (m, 1H), 6.82 - 6.54 (m, 1H), 5.09 - 4.94 (m, 2H), 4.54 - 4.41 (m, 3H), 4.27 - 4.13 (m, 3H), 3.01 (s, 3H), 2.97 (s, 3H), 2.72 - 2.66 (m, 2H), 2.46 (s, 1H), 2.45 - 2.26 (m, 4H), 2.25 - 2.16 (m, 1H), 2.14 (s, 3H), 1.84 (s, 2H), 1.08 (s, 3H), 0.97 (t, *J* = 7.5 Hz, 3H). $^{19}F$ NMR (376 MHz, DMSO-$d_6$) δ -118.72, -118.74, -125.74, -138.93.

**Examples 164A and 164B**

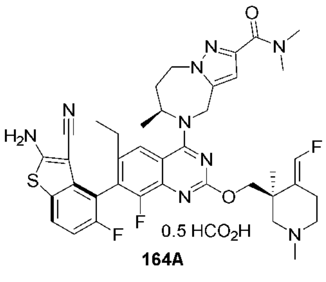
0.5 $HCO_2H$
**164A**

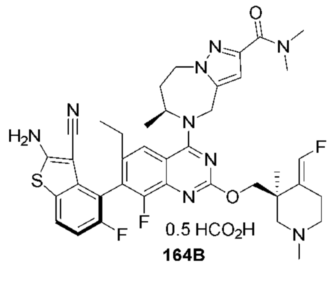
0.5 $HCO_2H$
**164B**

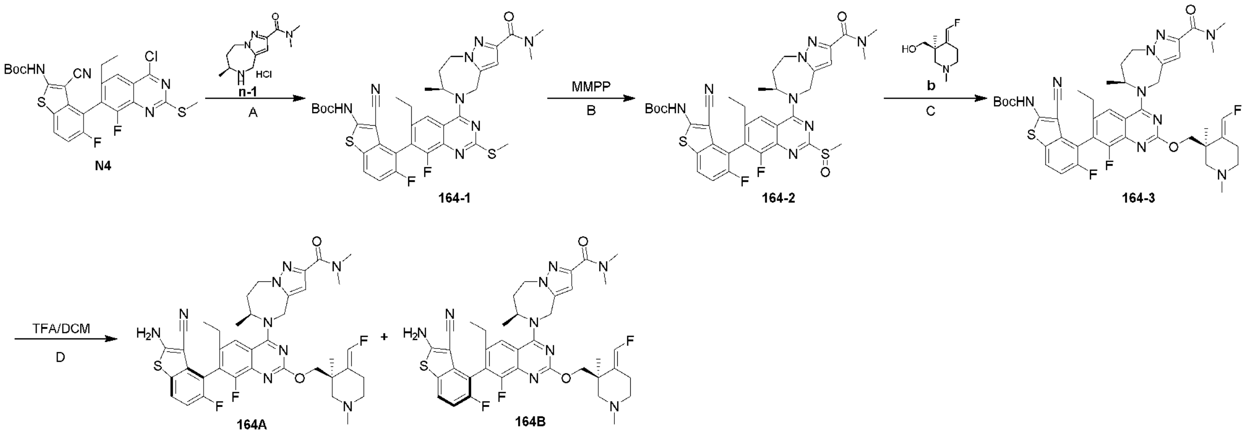


**Step A:** Compound **164-1**

At room temperature, DIPEA (277 mg, 2.74 mmol) was added to a mixture of intermediate **N4** (150 mg, 0.274 mmol), **n-1** (122 mg, 0.548 mmol), and DMF (3 mL), and stirred overnight at room temperature. The reaction completion was monitored by LCMS. The reaction solution was then poured into a saturated $NH_4Cl$ aqueous solution (30 mL), and extracted with EA (30 mL×3). The organic phase was collected, washed with saturated NaCl solution (30 mL), and dried over anhydrous $Na_2SO_4$. The mixture was filtered, and the crude product was concentrated and purified by FCC ($SiO_2$, EA/PE=0-90%) to give a white solid compound **164-1** (150 mg, yield 75%). LCMS (m/z): 733.1 (M+H).

**Step B:** Compound **164-2**

At room temperature, MMPP (85wt%, 357 mg, 0.614 mmol) was added to a mixed solution of compound **164-1** (150 mg, 0.205 mmol) in dichloromethane (5 mL) and $H_2O$ (5 mL), and the reaction was stirred for 2 h. After the reaction completion was monitored by LCMS, water (30 mL) was added, and the mixture was extracted with dichloromethane (50 mL×3). The organic phases were combined, washed with saturated brine, dried over anhydrous sodium sulfate, filtered, and concentrated to give the crude product compound **164-2.** (150 mg, 98%). LCMS (m/z): 749.1 (M+H).

**Step C:** Compound **164-3**

Under dry ice and ethanol bath conditions, NaO*t*Bu (2 M THF solution, 0.501 mL, 0.1 mmol) was added dropwise to solution containing compound **164-2** (150 mg, 0.2 mmol) and intermediate **b** (69 mg, 0.401 mmol) in tetrahydrofuran (5 mL), and reacted at this temperature for 0.5 hours. The temperature was warmed to room temperature for 1 hour. After the reaction completion was monitored by LCMS, the reaction solution was poured into a saturated $NH_4Cl$ aqueous solution (30 mL), and extracted with EA (30 mL×3). The organic phase was collected, washed with saturated NaCl solution (30

mL), dried over anhydrous $Na_2SO_4$, filtered, and concentrated to give crude product compound **164-3** (160 mg, yield 93%). LCMS (m /z): 858.1 (M+H).

**Step D: Examples 164A and 164B**

At room temperature, trifluoroacetic acid (10 mL) was added to compound **164-3** (150 mg, 0.175 mmol) and stirred for 1 hour. After the reaction completion was monitored by LCMS, the mixture was concentrated under reduced pressure, neutralized with saturated sodium bicarbonate solution, extracted with ethyl acetate (40 mL×3), concentrated under reduced pressure, and the crude product was purified by pre-HPLC (C18, ACN/(10 mmol FA/$H_2O$)=35-45%) to obtain a white powder isomer 1, which was **Example 164A** ($Rt_1$=11 min) (53 mg, yield 80%) LCMS(m/z): 758.1 (M+H). $^1H$ NMR (400 MHz, DMSO-$d_6$) δ 8.22 (s, 0.5 H), 7.90 (s, 2H), 7.82 (dd, *J* = 8.7, 4.9 Hz, 1H), 7.65 (s, 1H), 7.18 - 7.08 (m, 1H), 6.80 (s, 0.5 H), 6.67 (s, 1H), 6.58 (s, 0.5 H), 5.15 (d, *J* = 17.1 Hz, 1H), 4.94 - 4.72 (m, 2H), 4.51 - 4.35 (m, 2H), 4.29 - 4.09 (m, 2H), 3.25 (s, 3H), 2.96 (s, 3H), 2.70 - 2.61 (m, 2H), 2.48 - 2.25 (m, 5H ), 2.21 - 2.08 (m, 4H), 1.95 - 1.87 (m, 1H), 1.83 (d, *J* = 11.3 Hz, 1H), 1.57 (d, *J* = 6.2 Hz, 3H), 1.08 (s, 3H), 0.99 (t, *J* = 7.5 Hz, 3H). $^{19}F$ NMR (376 MHz, DMSO-$d_6$) δ -118.58, -125.47, -138.94; and obtained a white solid isomer 2, which was **Example 164B** ($Rt_2$=14 min) (15 mg, yield 23%). LCMS(m/z): 758.1 (M+H). $^1H$ NMR (400 MHz, DMSO-$d_6$) δ 8.36 (s, 0.5 H), 7.98 (s, 2 H), 7.87 (dd, *J* = 8.7, 5.0 Hz, 1H), 7.69 (s, 1H), 7.24 - 7.12 (m, 1H), 6.86 (s, 0.5 H), 6.73 - 6.66 (m, 1H), 6.64 (s, 0.5 H), 5.15 - 5.04 (m, 1H), 5.00 - 4.82 (m, 2 H), 4.60 - 4.45 (m, 2H), 4.37 - 4.25 (m, 1H), 4.18 - 4.00 (m, 1H), 3.30 (s, 3 H), 3.01 (s, 3 H), 2.76 - 2.66 (m, 2 H), 2.54 - 2.26 (m, 5 H), 2.24 - 2.17 (m, 4H), 2.01 - 1.91 (m, 1H), 1.88 (d, *J* = 11.2 Hz, 1H), 1.66 - 1.60 (m, 3 H), 1.14 (s, 3H), 1.02 (t, *J* = 7.5 Hz, 3H). $^{19}F$ NMR (376 MHz, DMSO-$d_6$) δ -118.96, -126.07, -138.93.

Following the above synthetic schemes and appropriate variants, the following examples were prepared and characterized:

| Example | Structural Formula | Characterization Data |
|---|---|---|
| 165A | 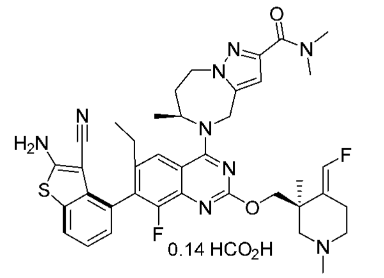 | LCMS (m/z): 740.3 (M+H). $^1H$ NMR (400 MHz, DMSO-$d_6$) δ 8.20 (s, 0.14H), δ 7.77 (m, 3H), 7.61 (s, 1H), 7.23 (t, *J* = 7.7 Hz, 1H), 7.08 (dd, *J* = 7.5, 1.1 Hz, 1H), 6.82 (s, 0.5H), 6.65 (s, 1H), 6.61 (s, 0.5H), 5.12 (d, *J* = 16.9 Hz, 1H), 4.85 (m, 1H), 4.78 (d, *J* = 16.9 Hz, 1H), 4.45 (dd, *J* = 13.9, 7.0 Hz, 1H), 4.38 (d, *J* = 10.7 Hz, 1H), 4.23 (d, *J* = 10.7 Hz, 1H), 4.12 (t, *J* = 12.7 Hz, 1H), 3.25 (s, 3H), 2.96 (s, 3H), 2.71 (s, 2H), 2.47 - 2.26 (m, 5H), 2.20 (m, 4H), 1.97 (m, 2H), 1.58 (d, *J* = 6.2 Hz, 3H), 1.09 (s, 3H), 0.96 (t, *J* = 7.5 Hz, 3H). $^{19}F$ NMR (376 MHz, DMSO-$d_6$) δ -126.38, -138.97. |
| 165B | 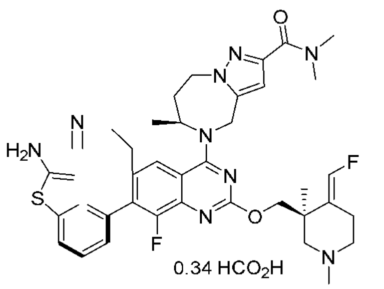 | LCMS (m/z): 740.3 (M+H). $^1H$ NMR (400 MHz, DMSO-$d_6$) δ 8.20 (s, 0.34H), 7.84 - 7.72 (m, 3H), 7.61 (s, 1H), 7.22 (t, *J* = 7.7 Hz, 1H), 7.12 (dd, *J* = 7.5, 1.2 Hz, 1H), 6.79 (s, 0.5H), 6.63 (s, 1H), 6.58 (s, 0.5H), 5.06 (d, *J* = 17.0 Hz, 1H), 4.83 (m, 2H), 4.53 - 4.40 (m, 2H), 4.23 (d, *J* = 10.6 Hz, 1H), 4.02 (t, *J* = 12.4 Hz, 1H), 3.24 (s, 3H), 2.95 (s, 3H), 2.66 (m, 2H), 2.48 - 2.34 (m, 3H), 2.27 (m, 2H), 2.20 - 2.15 (m, 1H), 2.14 (s, 3H), 1.89 (m, 1H), 1.82 (d, *J* = 11.3 Hz, 1H), 1.56 (d, *J* = 6.3 Hz, 3H), 1.07 (s, 3H), 0.94 (t, *J* = 7.5 Hz, 3H). $^{19}F$ NMR (376 MHz, DMSO-$d_6$) δ -126.80, - 138.96. |
| 166A | 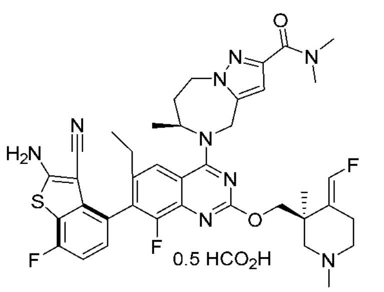 | LCMS (m/z): 758.3 (M+H). $^1H$ NMR (400 MHz, DMSO-$d_6$) δ 8.22 (s, 0.5 H), 8.04 (s, 2H), 7.61 (s, 1H), 7.15 (s, 1H), 7.13 (d, *J* = 2.8 Hz, 1H), 6.69 (d, *J* = 86.8 Hz, 1H), 6.65 (s, 1H), 5.11 (d, *J* = 17.2 Hz, 1H), 4.89 - 4.82 (m, 1H), 4.77 (d, *J* = 16.8 Hz, 1H), 4.48 - 4.39 (m, 2H), 4.22 (d, *J* = 10.8 Hz, 1H), 4.12 (t, *J* = 12.4 Hz, 1H), 3.24 (s, 3H), 2.95 (s, 3H), 2.64 (d, *J* = 10.8 Hz, 2H), 2.48 - 2.24 (m, 5H), 2.14 (s, 3H), 2.03 - 1.86 (m, 2H), 1.82 (d, *J* = 11.2 Hz, 1H), 1.57 (d, *J* = 6.0 Hz, 3H), 1.07 (s, 3H), 0.96 (t, *J* = 7.6 Hz, 3H). $^{19}F$ NMR (376 MHz, DMSO-$d_6$) δ -117.25, -126.14, -138.93. |

(continued)

| Example | Structural Formula | Characterization Data |
|---|---|---|
| 166B | 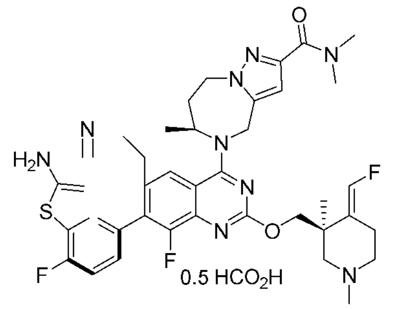 | LCMS (m/z): 758.3 (M+H). $^1$H NMR (400 MHz, DMSO-$d_6$) δ 8.24 (s, 0.5 H), 8.04 (s, 2H), 7.61 (s, 1H), 7.21 - 7.10 (m, 2H), 6.68 (d, $J$ = 86.8 Hz, 1H), 6.63 (s, 1H), 5.06 (d, $J$ = 17.2 Hz, 1H), 4.86 - 4.79 (m, 2H), 4.49 - 4.44 (m, 2H), 4.23 (d, $J$ = 10.4 Hz, 1H), 4.01 (t, $J$ = 12.0 Hz, 1H), 3.24 (s, 3H), 2.95 (s, 3H), 2.68 - 2.63 (m, 2H), 2.46 - 2.24 (m, 5H), 2.14 (s, 3H), 2.02 - 1.95 (m, 1H), 1.91 - 1.85 (m, 1H), 1.81 (d, $J$ = 11.2 Hz, 1H), 1.55 (d, $J$ = 6.0 Hz, 3H), 1.07 (s, 3H), 0.95 (t, $J$ = 7.6 Hz, 3H). $^{19}$F NMR (376 MHz, DMSO-$d_6$) δ -117.30, - 126.54, -138.95. |
| 167A | 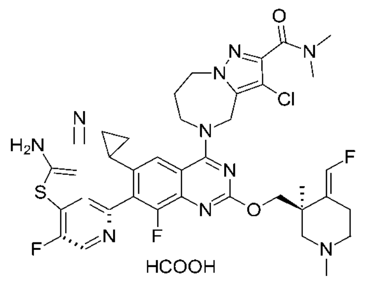 | LCMS (m/z): 791.2 (M+H). $^1$H NMR (400 MHz, DMSO-$d_6$) δ 8.44 (s, 1H), 8.34 (s, 2H), 8.20 (s, 1H), 7.28 (s, 1H), 6.83 - 6.52 (m, 1H), 5.09 - 4.93 (m, 2H), 4.50 - 4.37 (m, 3H), 4.31 - 4.21 (m, 1H), 4.19 - 4.11 (m, 2H), 3.07 - 2.91 (m, 6H), 2.73 - 2.62 (m, 2H), 2.53 (d, $J$ = 3.2 Hz, 1H), 2.44 - 2.28 (m, 2H), 2.20 - 2.07 (m, 4H), 1.94 - 1.85 (m, 1H), 1.81 (d, $J$ = 11.3 Hz, 1H), 1.61 - 1.48 (m, 1H), 1.08 (s, 3H), 0.75 - 0.63 (m, 2H), 0.61 - 0.49 (m, 1H), 0.48 - 0.36 (m, 1H). $^{19}$F NMR (376 MHz, DMSO-$d_6$) δ -73.43, -128.67, -132.82, -138.88. |
| 167B | 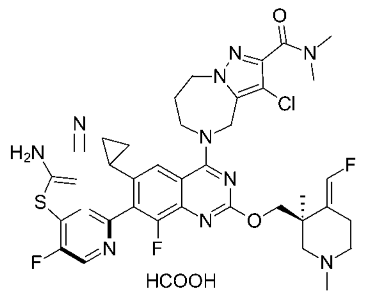 | LCMS (m/z): 791.3 (M+H). $^1$H NMR (400 MHz, DMSO-$d_6$) δ 8.44 (s, 1H), 8.35 (s, 2H), 8.28 (s, 1H), 7.28 (s, 1H), 6.80 - 6.51 (m, 1H), 5.11 - 4.92 (m, 2H), 4.52 (d, $J$ = 10.6 Hz, 1H), 4.48 - 4.37 (m, 2H), 4.25 - 4.08 (m, 3H), 3.06 - 2.94 (m, 6H), 2.73 - 2.62 (m, 2H), 2.49 - 2.45 (m, 1H), 2.43 - 2.27 (m, 2H), 2.25 - 2.15 (m, 1H), 2.13 (s, 3H), 1.93 - 1.84 (m, 1H), 1.83 - 1.76 (m, 1H), 1.60 - 1.48 (m, 1H), 1.07 (s, 3H), 0.77 - 0.62 (m, 2H), 0.61 - 0.49 (m, 1H), 0.48 - 0.36 (m, 1H). $^{19}$F NMR (376 MHz, DMSO-$d_6$) δ -73.42, -128.68, - 132.81, -138.92. |
| 168A | 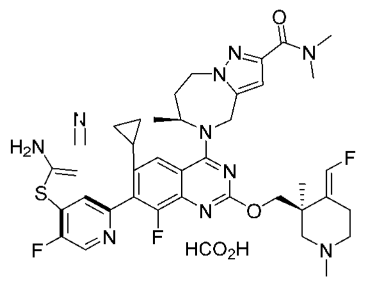 | LCMS (m/z): 7 71 .3 (M+H). |
| 168B | 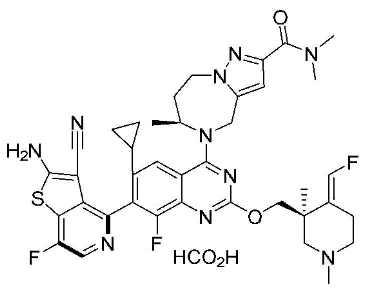 | LCMS (m/z): 7 71 .3 (M+H). |

(continued)

| Example | Structural Formula | Characterization Data |
|---|---|---|
| 169A | 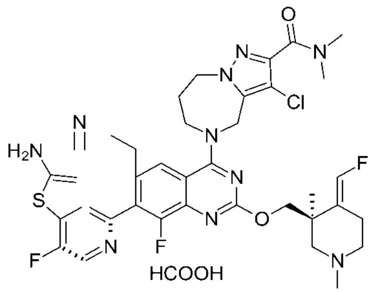 | LCMS (m/z): 779.3 (M+H). $^{1}$H NMR (400 MHz, DMSO-$d_6$) δ 8.43 (s, 1H), 8.42 - 8.35 (m, 3H), 7.57 (s, 1H), 6.81 - 6.78 (m, 0.5H), 6.60 - 6.56 (m, 0.5H), 5.03 (q, 2H), 4.51 - 4.39 (m, 3H), 4.28 - 4.22 (m, 1H), 4.21 - 4.12 (m, 2H), 3.05 - 2.94 (m, 6H), 2.74 - 2.62 (m, 2H), 2.48 - 2.27 (m, 5H), 2.19 - 2.08 (m, 4H), 1.94 - 1.84 (m, 1H), 1.84 - 1.78 (m, 1H), 1.09 (s, 3H), 0.93 (t, *J* = 7.5 Hz, 3H). $^{19}$F NMR (376 MHz, DMSO-$d_6$) δ - 73.41, -128.08, -132.50, -138.88. |
| 169B | 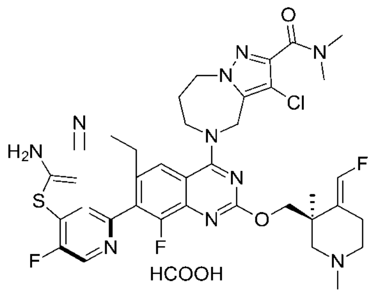 | LCMS (m/z): 779.3 (M+H). $^{1}$H NMR (400 MHz, DMSO-$d_6$) δ 8.43 (s, 1H), 8.42 - 8.33 (m, 3H), 7.56 (s, 1H), 6.80 - 6.75 (m, 0.5H), 6.59 - 6.52 (m, 0.5H), 5.04 - 4.98 (m, 2H), 4.54 (d, *J* = 10.6 Hz, 1H), 4.49 - 4.40 (m, 2H), 4.25 - 4.13 (m, 3H), 3.05 - 2.94 (m, 6H), 2.73 - 2.64 (m, 2H), 2.49 - 2.29 (m, 5H), 2.25 - 2.17 (m, 1H), 2.13 (s, 3H), 1.91 - 1.84 (m, 1H), 1.83 - 1.77 (m, 1H), 1.08 (s, 3H), 0.93 (t, *J* = 7.5 Hz, 3H). $^{19}$F NMR (376 MHz, DMSO-$d_6$) δ -73.41, -128.09, -132.50, - 138.91. |
| 170A | 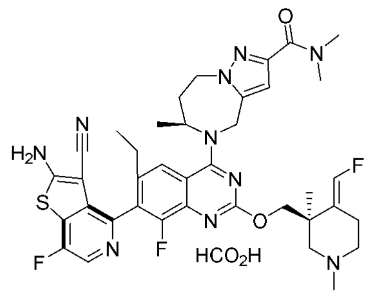 | LCMS (m/z): 759.3 (M+H). $^{1}$H NMR (400 MHz, DMSO-$d_6$) δ 8.42 (s, 1H), 8.36 (s, 2H), 8.25 (s, 1H), 7.63 (s, 1H), 6.82 - 6.78 (m, 0.5H), 6.66 (s, 1H), 6.61 - 6.54 (m, 0.5H), 5.12 (d, *J* = 17.0 Hz, 1H), 4.93 - 4.71 (m, 2H), 4.51 - 4.37 (m, 2H), 4.22 (d, *J*= 10.6 Hz, 1H), 4.19 - 4.09 (m, 1H), 3.24 (s, 3H), 2.95 (s, 3H), 2.70 - 2.61 (m, 2H), 2.48 - 2.23 (m, 5H), 2.19 - 2.05 (m, 4H), 1.96 - 1.86 (m, 1H), 1.83 (d, *J* = 11.3 Hz, 1H), 1.57 (d, *J* = 6.2 Hz, 3H), 1.08 (s, 3H), 0.94 (t, *J* = 7.5 Hz, 3H) . $^{19}$F NMR (376 MHz, DMSO-$d_6$) δ -128.13 -132.57, - 132.58 -138.93. |
| 170B | 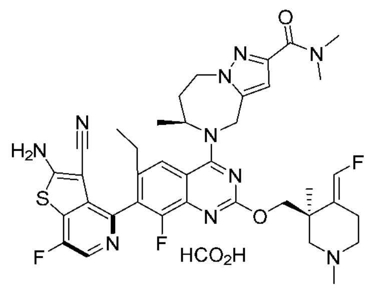 | LCMS (m/z): 759.3 (M+H). $^{1}$H NMR (400 MHz, DMSO-$d_6$) δ 8.48 - 8.20 (m, 4H), 7.64 (s, 1H), 6.83 - 6.75 (m, 0.5H), 6.64 (s, 1H), 6.59 - 6.54 (m, 0.5H), 5.15 - 5.01 (m, 1H), 4.92 - 4.74 (m, 2H), 4.55 - 4.39 (m, 2H), 4.27 - 4.17 (m, 1H), 4.12 - 3.97 (m, 1H), 3.24 (s, 3H), 2.95 (s, 3H), 2.71 - 2.62 (m, 2H), 2.48 - 2.23 (m, 5H), 2.22 - 2.16 (m, 1H), 2.14 (s, 3H), 2.05 - 1.94 (m, 1H), 1.82 (d, *J* = 11.3 Hz, 1H), 1.56 (d, *J* = 6.2 Hz, 3H), 1.08 (s, 3H), 0.95 (t, *J* = 7.5 Hz, 3H). $^{19}$F NMR (376 MHz, DMSO-$d_6$) δ -128.05 -132.56, -138.93. |
| 171A | 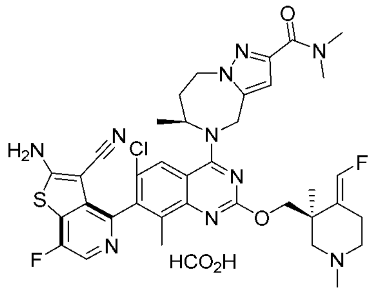 | LCMS(m/z): 761.2 (M+H). $^{1}$H NMR (400 MHz, DMSO-$d_6$) δ 8.40 (s, 1H), 8.37 - 8.28 (m, 2H), 7.87 (s, 1H), 6.83 - 6.57 (m, 2H), 5.07 (d, *J* = 17.1 Hz, 1H), 4.90 - 4.72 (m, 2H), 4.53 - 4.41 (m, 2H), 4.29 (d, *J* = 10.7 Hz, 1H), 4.17 (t, 1H), 3.25 (s, 3H), 2.95 (s, 3H), 2.69 - 2.60 (m, 2H), 2.49 - 2.21 (m, 4H), 2.21 - 2.08 (m, 7H), 1.97 - 1.81 (m, 2H), 1.53 (d, *J* = 6.2 Hz, 3H), 1.09 (s, 3H). $^{19}$F NMR (376 MHz, DMSO-$d_6$) δ - 132.82, -138.94. |

(continued)

| Example | Structural Formula | Characterization Data |
|---|---|---|
| 171B | 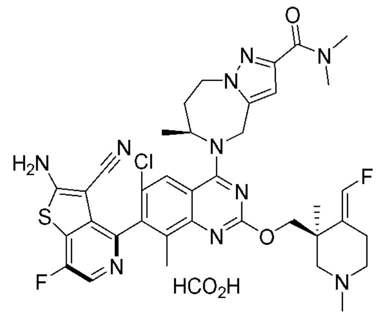 | LCMS(m/z): 761.2 (M+H). $^1$H NMR (400 MHz, DMSO-$d_6$) δ 8.42 (s, 1H), 8.37 (s, 3H), 7.86 (s, 1H), 6.70 (d, *J* = 88.3 Hz, 2H), 4.95 (d, *J* = 16.9 Hz, 1H), 4.87 - 4.74 (m, 2H), 4.54 - 4.43 (m, 2H), 4.30 (d, *J* = 10.6 Hz, 1H), 4.06 (t, *J* = 12.3 Hz, 1H), 3.24 (s, 3H), 2.95 (s, 3H), 2.70 - 2.60 (m, 2H), 2.49 - 2.23 (m, 4H), 2.20 - 2.11 (m, 7H), 1.96 - 1.79 (m, 2H), 1.54 (d, *J* = 6.3 Hz, 3H), 1.09 (s, 3H). $^{19}$F NMR (376 MHz, DMSO-$d_6$) δ -132.84, -138.91. |
| 172A | 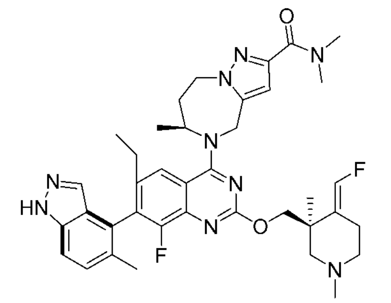 | LCMS(m/z): 698.4 (M+H). $^1$H NMR (400 MHz, DMSO-$d_6$) δ 13.14 (s, 1H), 7.75 (s, 1H), 7.55 (d, *J* = 8.5 Hz, 1H), 7.41 - 7.36 (m, 2H), 6.83 - 6.54 (m, 2H), 5.17 (d, *J* = 16.9 Hz, 1H), 4.94 - 4.78 (m, 2H), 4.53 - 4.41 (m, 2H), 4.31 - 4.06 (m, 3H), 3.25 (s, 3H), 2.95 (s, 3H), 2.83 - 2.80 (m, 1H), 2.69 - 2.62 (m, 3H), 2.48 - 2.28 (m, 6H), 2.26 - 2.10 (m, 7H), 1.96 - 1.78 (m, 1H), 1.64 - 1.53 (m, 3H), 0.88 (t, *J* = 7.5 Hz, 3H). $^{19}$F NMR (376 MHz, DMSO-$d_6$) δ -126.86, -139.04. |
| 172B | 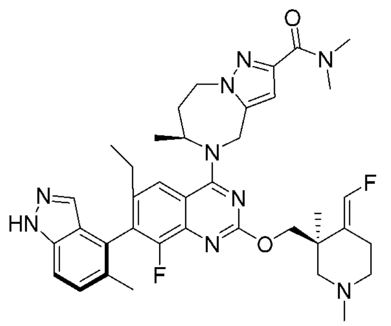 | LCMS(m/z): 698.4 (M+H). $^1$H NMR (400 MHz, DMSO-$d_6$) δ 13.14 (s, 1H), 7.75 (s, 1H), 7.55 (d, *J* = 8.5 Hz, 1H), 7.41 - 7.36 (m, 2H), 6.83 - 6.54 (m, 2H), 5.17 (d, *J* = 16.9 Hz, 1H), 4.94 - 4.78 (m, 2H), 4.53 - 4.41 (m, 2H), 4.31 - 4.06 (m, 3H), 3.25 (s, 3H), 2.95 (s, 3H), 2.83 - 2.80 (m, 1H), 2.69 - 2.62 (m, 3H), 2.48 - 2.28 (m, 6H), 2.26 - 2.10 (m, 7H), 1.96 - 1.78 (m, 1H), 1.64 - 1.53 (m, 3H), 0.88 (t, *J* = 7.5 Hz, 3H). $^{19}$F NMR (376 MHz, DMSO-$d_6$) δ -126.86, -139.04. |
| 173A | 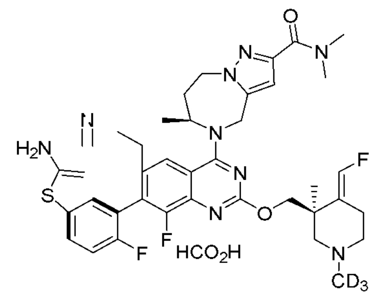 | LCMS(m/z): (M+H) 761.3. $^1$H NMR (400 MHz, DMSO-$d_6$) δ 8.34 (s, 1H), 7.91 (s, 2H), 7.82 (dd, *J* = 8.8, 4.9 Hz, 1H), 7.65 (s, 1H), 7.17 - 7.09 (m, 1H), 6.82 - 6.53 (m, 2H), 5.14 (d, *J* = 17.1 Hz, 1H), 4.93 - 4.71 (m, 2H), 4.50 - 4.34 (m, 2H), 4.26 - 4.10 (m, 2H), 3.24 (s, 3H), 2.95 (s, 3H), 2.67 - 2.61 (m, 2H), 2.46 - 2.27 (m, 5H), 2.18 - 2.09 (m, 1H), 1.95 - 1.86 (m, 1H), 1.82 (d, *J* = 11.2 Hz, 1H), 1.56 (d, *J* = 6.2 Hz, 3H), 1.08 (s, 3H), 0.98 (t, *J* = 7.5 Hz, 3H). $^{19}$F NMR (376 MHz, DMSO-$d_6$) δ -118.58, -125.49, -138.97. |
| 173B | 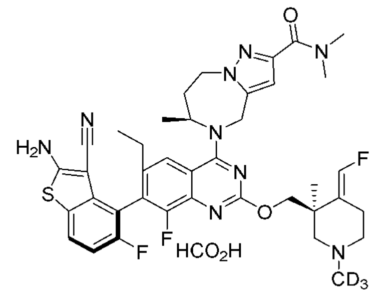 | LCMS (m/z): (M+H) 761.3. |

(continued)

| Example | Structural Formula | Characterization Data |
|---|---|---|
| 174A | 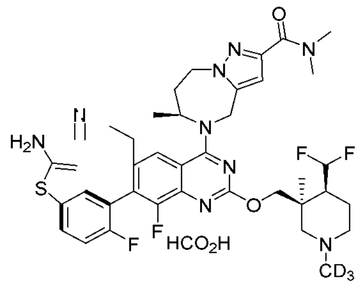 | LCMS(m/z): (M+H) 781.1. $^1$H NMR (400 MHz, DMSO-$d_6$) δ 8.31 (s, 1H), 7.92 (s, 2H), 7.82 (dd, $J$ = 8.7, 5.0 Hz, 1H), 7.65 (s, 1H), 7.17 -7.08 (m, 1H), 6.69 (s, 1H), 6.47 - 6.11 (m, 1H), 5.19 - 5.10 (m, 1H), 4.93 - 4.82 (m, 1H), 4.82 - 4.73 (m, 1H), 4.53 - 4.41 (m, 1H), 4.41 - 4.30 (m, 2H), 4.25 - 4.09 (m, 1H), 3.25 (s, 3H), 2.96 (s, 3H), 2.87 - 2.75 (m, 2H), 2.48 - 2.38 (m, 2H), 2.38 - 2.26 (m, 2H), 2.06 - 1.59 (m, 5H), 1.56 (d, $J$ = 6.2 Hz, 3H), 1.09 (s, 3H), 0.97 (t, $J$ = 7.5 Hz, 3H). $^{19}$F NMR (376 MHz, DMSO-$d_6$) δ -115.57, -118.61, - 118.73, -125.36. |
| 174B | 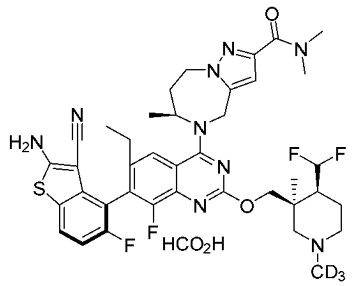 | LCMS(m/z): (M+H) 781.1. |
| 175A | 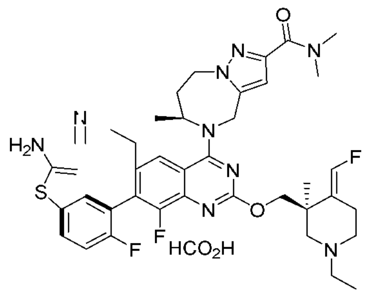 | LCMS (m/z): 772.4 (M+H). $^1$H NMR (400 MHz, DMSO-$d_6$) δ 8.31 (s, 1H), 7.91 (s, 2H), 7.81 (dd, $J$ = 8.7, 4.9 Hz, 1H), 7.64 (s, 1H), 7.17 -7.07 (m, 1H), 6.83 - 6.53 (m, 2H), 5.22 - 5.08 (m, 1H), 4.92 - 4.82 (m, 1H), 4.82 - 4.72 (m, 1H), 4.51 - 4.38 (m, 2H), 4.29 - 4.09 (m, 2H), 3.24 (s, 3H), 2.95 (s, 3H), 2.78 - 2.66 (m, 2H), 2.48 - 2.38 (m, 3H), 2.37 - 2.24 (m, 4H), 2.19 - 2.07 (m, 1H), 2.01 - 1.91 (m, 1H), 1.87 - 1.80 (m, 1H), 1.55 (d, $J$ = 6.2 Hz, 3H), 1.08 (s, 3H), 0.97 (t, $J$ = 7.3 Hz, 6H). $^{19}$F NMR (376 MHz, DMSO-$d_6$) δ -118.59, - 125.44, -139.28. |
| 175B | 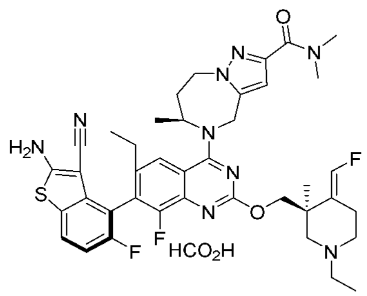 | LCMS (m/z): 772.3 (M+H). $^1$H NMR (400 MHz, DMSO-$d_6$) δ 8.40 (s, 2H), 7.93 (s, 2H), 7.81 (dd, $J$ = 8.6, 4.9 Hz, 1H), 7.63 (s, 1H), 7.17 -7.08 (m, 1H), 6.83 - 6.53 (m, 2H), 5.11 - 4.98 (m, 1H), 4.89 - 4.76 (m, 2H), 4.53 - 4.38 (m, 2H), 4.29 - 4.19 (m, 1H), 4.12 - 3.97 (m, 1H), 3.24 (s, 3H), 2.95 (s, 3H), 2.79 - 2.71 (m, 2H), 2.45 - 2.38 (m, 2H), 2.35 - 2.22 (m, 5H), 2.20 - 2.09 (m, 1H), 1.98 - 1.89 (m, 1H), 1.86 - 1.79 (m, 1H), 1.56 (d, $J$ = 6.3 Hz, 3H), 1.08 (s, 3H), 1.00 - 0.93 (m, 6H). $^{19}$F NMR (376 MHz, DMSO-$d_6$) δ -118.96, - 126.01, -139.28. |
| 176A | 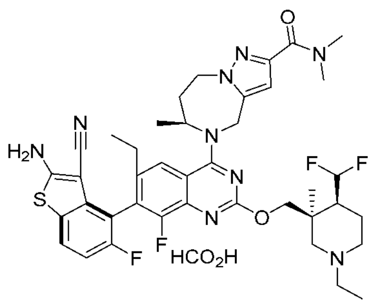 | LCMS(m/z): 792.3 (M+H). $^1$H NMR (400 MHz, DMSO-$d_6$) δ 8.30 (s, 1H), 7.91 (s, 2H), 7.82 (dd, $J$ = 8.7, 4.9 Hz, 1H), 7.65 (s, 1H), 7.17 -7.08 (m, 1H), 6.69 (s, 1H), 6.46 - 6.10 (m, 1H), 5.20 - 5.08 (m, 1H), 4.94 - 4.82 (m, 1H), 4.82 - 4.73 (m, 1H), 4.53 - 4.38 (m, 2H), 4.37 - 4.28 (m, 1H), 4.25 - 4.11 (m, 1H), 3.24 (s, 3H), 2.95 (s, 3H), 2.93 - 2.85 (m, 2H), 2.47 - 2.37 (m, 2H), 2.37 - 2.18 (m, 4H), 1.92 - 1.83 (m, 1H), 1.82 - 1.74 (m, 1H), 1.73 - 1.65 (m, 1H), 1.65 - 1.58 (m, 2H), 1.54 (d, $J$ = 6.2 Hz, 1H), 1.10 (s, 3H), 0.97 (t,$J$ = 7.5 Hz, 3H), 0.91 (t, $J$ = 7.1 Hz, 3H). $^{19}$F NMR (376 MHz, DMSO-$d_6$) δ -115.62, -118.62, -118.86, -125.30. |

(continued)

| Example | Structural Formula | Characterization Data |
|---|---|---|
| 176B | 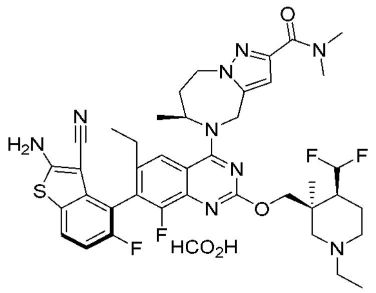 | LCMS(m/z): 792.3 (M+H). |
| 177A | 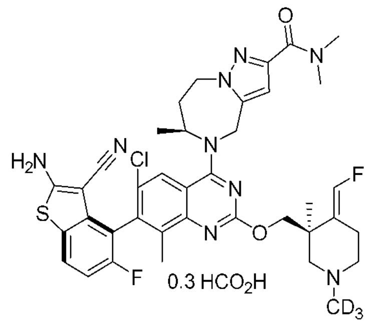 | LCMS (m/z): 763.3 (M+H). $^1H$ NMR (400 MHz, DMSO-$d_6$) δ 7.99 - 7.73 (m, 4H), 7.17 - 7.03 (m, 1H), 6.85 - 6.55 (m, 2H), 5.10 (d, *J* = 17.1 Hz, 1H), 4.90 - 4.70 (m, 2H), 4.53 - 4.40 (m, 2H), 4.30 (d, *J* = 10.6 Hz, 1H), 4.18 (t, *J* = 12.4 Hz, 1H), 3.25 (s, 3H), 2.95 (s, 3H), 2.68 - 2.60 (m, 2H), 2.48 - 2.35 (m, 2H), 2.33 - 2.25 (m, 1H), 2.22 (s, 3H), 2.19 - 2.10 (m, 1H), 1.96 - 1.88 (m, 1H), 1.84 (d, *J* = *11.3* Hz, 1H), 1.52 (d, *J* = 6.3 Hz, 3H), 1.09 (s, 3H). $^{19}F$ NMR (376 MHz, DMSO-$d_6$) δ - 119.48, -138.97. |
| 177B | 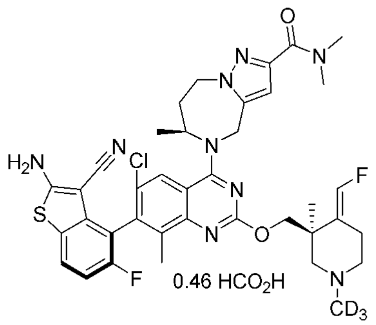 | LCMS (m/z): 763.3 (M+H). $^1H$ NMR (400 MHz, DMSO-$d_6$) δ 7.89 (d, *J* = 8.6 Hz, 3H), 7.79 (dd, *J* = 8.7, 4.9 Hz, 1H), 7.16 - 7.08 (m, 1H), 6.71 (d, *J* = 86.2 Hz, 2H), 4.96 (d, *J* = 17.0 Hz, 1H), 4.85 - 4.75 (m, 2H), 4.53 - 4.41 (m, 2H), 4.31 (d, *J* = 10.6 Hz, 1H), 4.11 - 3.96 (m, 1H), 3.24 (s, 3H), 2.95 (s, 3H), 2.68 - 2.63 (m, 2H), 2.48 - 2.34 (m, 2H), 2.34 - 2.26 (m, 1H), 2.23 (s, 3H), 2.19 - 2.11 (m, 1H), 1.96 - 1.87 (m, 1H), 1.83 (d, *J* = 11.3 Hz, 1H), 1.54 (d, *J* = 6.3 Hz, 3H), 1.09 (s, 3H). $^{19}F$ NMR (376 MHz, DMSO-$d_6$) δ -119.88, -138.96. |
| 178A | 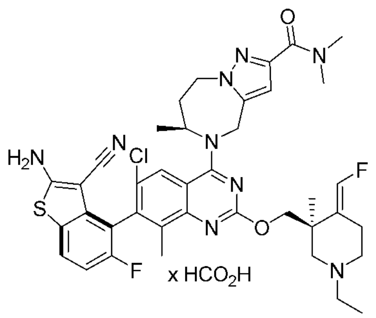 | LCMS (m/z): 774.3 (M+H). $^1H$ NMR (400 MHz, DMSO-$d_6$) δ 8.38 (s, 1H), 7.94 - 7.84 (m, 3H), 7.79 (dd, *J* = 8.7, 5.0 Hz, 1H), 7.16 - 7.06 (m, 1H), 6.84 - 6.56 (m, 2H), 5.10 (d, *J* = 17.1 Hz, 1H), 4.90 - 4.72 (m, 2H), 4.55 - 4.39 (m, 2H), 4.32 (d, *J* = 10.6 Hz, 1H), 4.26 - 4.14 (m, 1H), 3.25 (s, 3H), 2.95 (s, 3H), 2.79 - 2.69 (m, 2H), 2.48 - 2.35 (m, 2H), 2.30 (ddd, *J* = 13.0, 5.7, 3.9 Hz, 3H), 2.21 (s, 3H), 2.19 - 2.10 (m, 1H), 2.02 - 1.93 (m, 1H), 1.85 (d, *J* = 11.2 Hz, 1H), 1.51 (d, *J* = 6.2 Hz, 3H), 1.10 (s, 3H), 0.97 (t, *J* = 7.1 Hz, 3H). $^{19}F$ NMR (376 MHz, DMSO-$d_6$) δ -119.50, -139.26. |
| 178B | 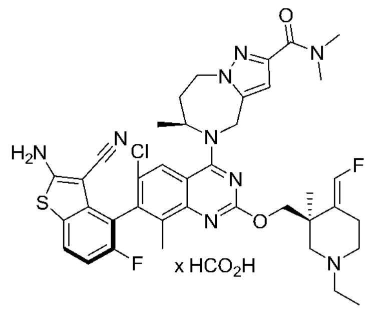 | LCMS (m/z): 774.3 (M+H) $^1H$ NMR (400 MHz, DMSO-$d_6$) δ 8.42 (s, 1H), 8.01 - 7.84 (m, 3H), 7.79 (dd, *J* = 8.7, 4.9 Hz, 1H), 7.17 - 7.03 (m, 1H), 6.86 - 6.53 (m, 2H), 5.03 - 4.90 (m, 1H), 4.88 - 4.71 (m, 2H), 4.47 (dd, *J* = 19.2, 9.0 Hz, 2H), 4.32 (d, *J* = 10.7 Hz, 1H), 4.10 - 3.94 (m, 1H), 3.24 (s, 3H), 2.95 (s, 3H), 2.75 (d, *J* = 11.0 Hz, 2H), 2.42 - 2.35 (m, 2H), 2.34 - 2.26 (m, 3H), 2.22 (s, 3H), 2.18 - 2.10 (m, 1H), 2.02 - 1.90 (m, 1H), 1.84 (d, *J* = 11.2 Hz, 1H), 1.54 (d, *J* = 6.3 Hz, 3H), 1.09 (s, 3H), 0.97 (t, *J* = 7.1 Hz, 3H). $^{19}F$ NMR (376 MHz, DMSO-$d_6$) δ -119.89, -139.26. |

(continued)

| Example | Structural Formula | Characterization Data |
|---|---|---|
| 179A | 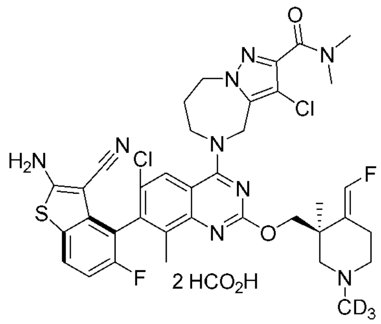 | LCMS (m/z): 783.2 (M+H). $^1$H NMR (400 MHz, DMSO-$d_6$) δ 8.41 (s, 2H), 7.89 (s, 2H), 7.84 - 7.75 (m, 2H), 7.16 - 7.07 (m, 1H), 6.70 (d, *J* = 86.9 Hz, 1H), 5.03 - 4.84 (m, 2H), 4.48 - 4.28 (m, 4H), 4.19 - 4.09 (m, 2H), 3.01 (s, 3H), 2.97 (s, 3H), 2.70 - 2.63 (m, 2H), 2.35 - 2.27 (m, 2H), 2.24 (s, 3H), 2.21 - 2.11 (m, 1H), 1.96 - 1.78 (m, 2H), 1.09 (s, 3H). $^{19}$F NMR (376 MHz, DMSO-$d_6$) δ -119.71, -138.95. |
| 179B | 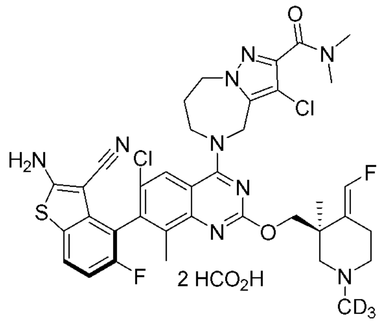 | LCMS (m/z): 783.2 (M+H). $^1$H NMR (400 MHz, DMSO-$d_6$) δ 8.40 (s, 2H), 7.89 (s, 2H), 7.85 - 7.75 (m, 2H), 7.17 - 7.07 (m, 1H), 6.69 (d, *J* = 86.7 Hz, 1H), 5.00 - 4.86 (m, 2H), 4.51 - 4.36 (m, 3H), 4.31 (d, *J* = 10.6 Hz, 1H), 4.20 - 4.07 (m, 2H), 3.01 (s, 3H), 2.97 (s, 3H), 2.70 - 2.63 (m, 2H), 2.36 - 2.28 (m, 2H), 2.24 (s, 3H), 2.21 - 2.12 (m, 1H), 1.95 - 1.86 (m, 1H), 1.83 (d, *J* = 11.3 Hz, 1H), 1.09 (s, 3H). $^{19}$F NMR (376 MHz, DMSO-$d_6$) δ -119.70, -138.96. |
| 180 | 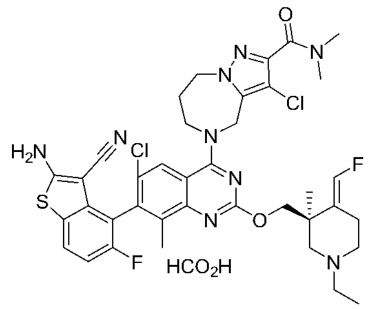 | LCMS(m/z): 794.2 (M+H). $^1$H NMR (400 MHz, DMSO-$d_6$) δ 8.34 (s, 1H), 7.88 (s, 2H), 7.84 - 7.76 (m, 2H), 7.17 - 7.06 (m, 1H), 6.68 (d, *J* = 87.2 Hz, 1H), 5.04 - 4.88 (m, 2H), 4.53 - 4.29 (m, 4H), 4.21 - 4.06 (m, 2H), 3.24 - 3.09 (m, 1H), 3.01 (s, 3H), 2.97 (s, 3H), 2.78 - 2.70 (m, 2H), 2.40 - 2.26 (m, 4H), 2.24 (s, 3H), 2.18 (s, 1H), 2.00 - 1.91 (m, 1H), 1.87 (d, *J* = 11.2 Hz, 1H), 1.10 (d, *J* = 3.7 Hz, 3H), 0.95 (t, *J* = 7.2, 1.9 Hz, 3H). $^{19}$F NMR (376 MHz, DMSO-$d_6$) δ -119.71, - 139.29. |
| 180A | 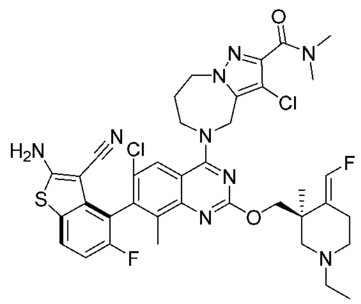 | LCMS(m/z): 7.94.2 (M+H) |
| 180B | 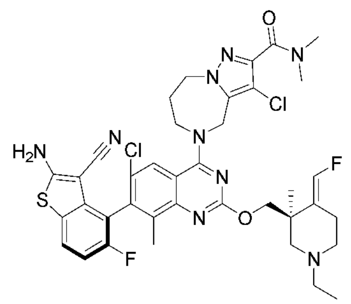 | LCMS(m/z): 7.94.2 (M+H) |

(continued)

| Example | Structural Formula | Characterization Data |
|---|---|---|
| 181A | 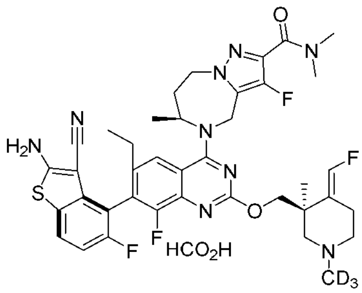 | LCMS (m/z): 779.1 (M+H). $^1$H NMR (400 MHz, DMSO-$d_6$) δ 8.14 (s, 1H), 7.92 (s, 2H), 7.82 (dd, $J$ = 8.7, 5.0 Hz, 1H), 7.58 (s, 1H), 7.18 -7.10 (m, 1H), 6.86 - 6.51 (m, 1H), 5.11 - 4.98 (m, 1H), 4.92 - 4.80 (m, 1H), 4.80 - 4.71 (m, 1H), 4.41 - 4.25 (m, 2H), 4.26 - 4.10 (m, 1H), 4.10 - 3.98 (m, 1H), 3.13 (s, 3H), 2.97 (s, 3H), 2.48 - 2.30 (m, 6H), 2.28 - 2.03 (m, 1H), 2.01 - 1.72 (m, 1H), 1.63 (d, $J$ = 6.2 Hz, 3H), 1.08 (s, 3H), 1.02 (t, $J$ = 7.5 Hz, 3H). $^{19}$F NMR (376 MHz, DMSO-$d_6$) δ -118.60, -125.34, -138.96, -171.06. |
| 181B | 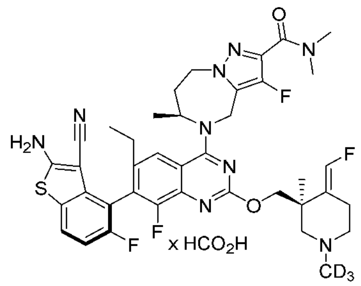 | LCMS (m/z): 779.2 (M+H). |
| 182A | 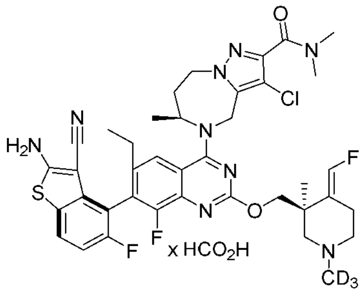 | LCMS (m/z): 795.3 (M+H). |
| 182B | 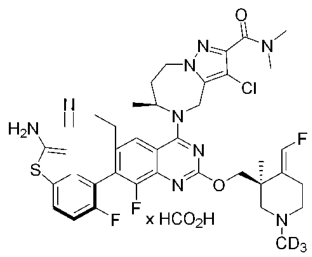 | LCMS (m/z): 7.95.3 (M+H). |
| 183A | 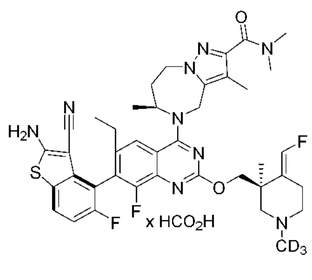 | LCMS (m/z): 775.3 (M+H). $^1$H NMR (400 MHz, DMSO-$d_6$) δ 8.27 (s, 0.5H), 7.92 (s, 2H), 7.85 - 7.76 (m, 1H), 7.51 (s, 1H), 7.20 - 7.08 (m, 1H), 6.85 - 6.51 (m, 1H), 5.09 - 4.95 (m, 1H), 4.90 - 4.79 (m, 1H), 4.71 - 4.60 (m, 1H), 4.45 - 4.28 (m, 2H), 4.24 - 4.14 (m, 1H), 4.09 - 3.95 (m, 1H), 3.09 (s, 3H), 2.95 (s, 3H), 2.66 (d, $J$ = 10.8 Hz, 2H), 2.44 - 2.21 (m, 5H), 2.15 (s, 3H), 2.10 - 2.02 (m, 1H), 1.93 - 1.85 (m, 1H), 1.83 - 1.71 (m, 1H), 1.69 - 1.55 (m, 3H), 1.07 (s, 3H), 1.00 - 0.91 (m, 3H). $^{19}$F NMR (376 MHz, DMSO-$d_6$) δ - 118.55, -125.35, -138.90. |

(continued)

| Example | Structural Formula | Characterization Data |
|---|---|---|
| 183B | 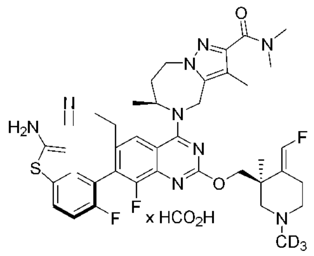 | LCMS (m/z): 775.3 (M+H). |
| 184A | 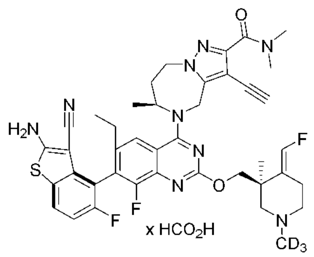 | LCMS (m/z): 785.3 (M+H). |
| 184B | 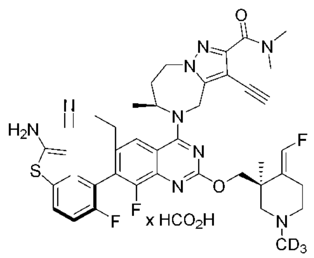 | LCMS (m/z): 785.3 (M+H). |
| 185A | 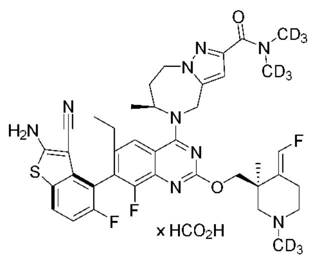 | LCMS(m/z): 767.4 (M+H). $^{1}$H NMR (400 MHz, DMSO-$d_6$) δ 8.37 (s, 1H), 7.92 (s, 2H), 7.85 -7.78 (m, 1H), 7.65 (s, 1H), 7.17 - 7.08 (m, 1H), 6.83 - 6.55 (m, 2H), 5.22 - 5.06 (m, 1H), 4.93 - 4.82 (m, 1H), 4.81 - 4.72 (m, 1H), 4.52 - 4.35 (m, 2H), 4.27 - 4.20 (m, 1H), 4.19 - 4.07 (m, 1H), 2.71 - 2.59 (m, 2H), 2.47 - 2.24 (m, 5H), 2.19 - 2.06 (m, 1H), 1.95 - 1.86 (m, 1H), 1.85 - 1.78 (m, 1H), 1.56 (d, *J* = 6.2 Hz, 3H), 1.08 (s, 3H), 0.98 (t, *J* = 7.5 Hz, 3H). $^{19}$F NMR (376 MHz, DMSO-$d_6$) δ -118.58, -125.49, -138.97. |
| 185B | 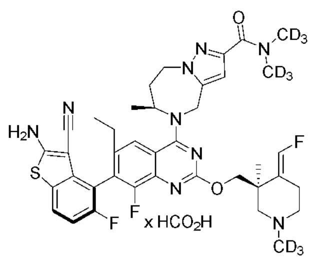 | LCMS(m/z): 767.4 (M+H). |
| 186 | 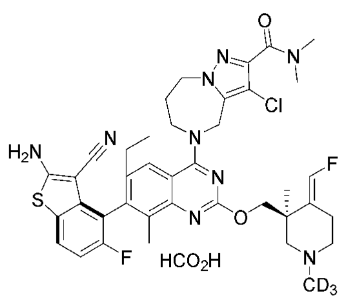 | LCMS(m/z): 777.3 (M+H). $^{1}$H NMR (400 MHz, DMSO-$d_6$) δ 8.35 (s, 1H), 7.96 - 7.70 (m, 3H), 7.57 (s, 1H), 7.18 - 7.06 (m, 1H), 6.70 (d, *J* = 86.8 Hz, 1H), 5.09 - 4.83 (m, 2H), 4.55 - 4.36 (m, 3H), 4.35 - 4.24 (m, 1H), 4.22 - 4.03 (m, 2H), 3.02 (s, 3H), 2.98 (s, 3H), 2.73 - 2.60 (m, 2H), 2.42 - 2.27 (m, 4H), 2.23 - 2.13 (m, 4H), 2.10 - 1.73 (m, 3H), 1.10 (s, 3H), 0.96 (t, *J* = 7.5 Hz, 3H). $^{19}$F NMR (376 MHz, DMSO-$d_6$) δ - 119.38, -139.06. |

(continued)

| Example | Structural Formula | Characterization Data |
|---|---|---|
| 187 | 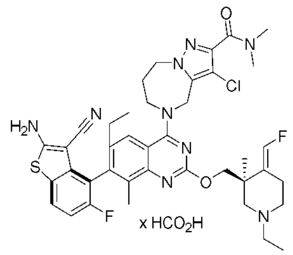 | LCMS(m/z): 777.3 (M+H). |
| 188 | 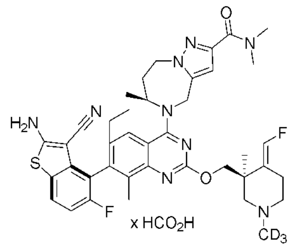 | LCMS(m/z): 757.3 (M+H). $^1$H NMR (400 MHz, DMSO-$d_6$) δ 8.16 (s, 0.3H), 7.81 (s, 2H), 7.77 (dd, *J* = 8.7, 5.0 Hz, 1H), 7.65 (s, 1H), 7.14 -7.06 (m, 1H), 6.80 (s, 0.5H), 6.63 (s, 1H), 6.59 (s, 0.5H), 5.09 (d, *J* = 16.8 Hz, 1H), 4.90 - 4.79 (m, 1H), 4.77 - 4.66 (m, 1H), 4.53 - 4.34 (m, 2H), 4.31 - 4.22 (m, 1H), 4.17 - 4.04 (m, 1H), 3.25 (s, 3H), 2.95 (s, 3H), 2.68 - 2.59 (m, 2H), 2.44 - 2.21 (m, 5H), 2.19 - 2.08 (m, 4H), 2.01 - 1.90 (m, 1H), 1.85 (d, *J* = 11.2 Hz, 1H), 1.56 (d, *J* = 6.2 Hz, 3H), 1.08 (s, 3H), 0.97 (t, *J* = 7.5 Hz, 3H). $^{19}$F NMR (376 MHz, DMSO-$d_6$) δ -119.15, -139.06. |
| 189 | 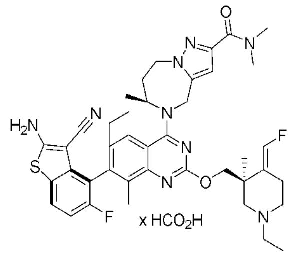 | LCMS(m/z): 768. 3 (M+H). $^1$H NMR (400 MHz, DMSO-$d_6$) δ 8.27 (s, 1H), 7.91 - 7.71 (m, 3H), 7.65 (s, 1H), 7.19 - 7.02 (m, 1H), 6.80 (s, 0.5H), 6.69 - 6.54 (m, 1.5H), 5.10 (d, *J* = 16.9 Hz, 1H), 4.93 - 4.68 (m, 2H), 4.52 - 4.35 (m, 2H), 4.32 - 4.24 (m, 1H), 4.20 - 4.05 (m, 1H), 3.24 (s, 3H), 2.95 (s, 3H), 2.76 - 2.63 (m, 2H), 2.43 - 2.21 (m, 7H), 2.18 - 2.09 (m, 4H), 2.04 - 1.92 (m, 1H), 1.86 (d, *J* = 11.3 Hz, 1H), 1.55 (d, *J* = 6.3 Hz, 3H), 1.09 (s, 3H), 1.02 - 0.88 (m, 6H). $^{19}$F NMR (376 MHz, DMSO-$d_6$) δ -119.16, -139.41. |
| 190 | 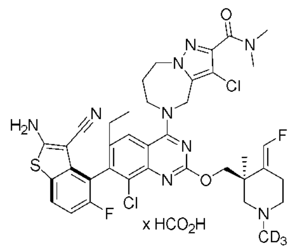 | LCMS(m/z): 797.2 (M+H). $^1$H NMR (400 MHz, DMSO-$d_6$) δ 8.36 (s, 2H), 7.88 (s, 2H), 7.82 - 7.76 (m, 1H), 7.72 (s, 1H), 7.17 - 7.06 (m, 1H), 6.84 - 6.77 (m, 0.5H), 6.63 - 6.51 (m, 0.5H), 5.01 (q, *J* = 16.3 Hz, 2H), 4.51 - 4.34 (m, 3H), 4.29 (d, *J* = 10.6 Hz, 1H), 4.24 - 4.09 (m, 2H), 2.99 (d, *J* = 16.1 Hz, 6H), 2.73 - 2.62 (m, 2H), 2.48 - 2.24 (m, 5H), 2.22 - 2.11 (m, 1H), 1.96 - 1.86 (m, 1H), 1.82 (d, *J* = 11.3 Hz, 1H), 1.09 (s, 3H), 0.98 (t, *J* = 7.5 Hz, 3H). $^{19}$F NMR (376 MHz, DMSO-$d_6$) δ -119.35 -138.93. |
| 191 | 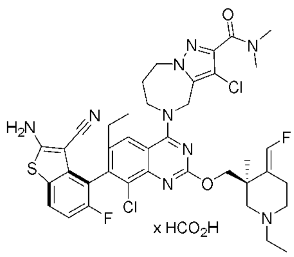 | LCMS(m/z): 808.2 (M+H). |
| 192 | 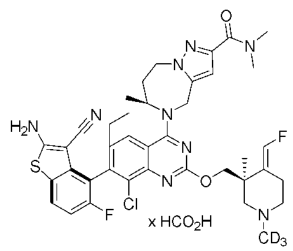 | LCMS(m/z): 777.3 (M+H). $^1$H NMR (400 MHz, DMSO-$d_6$) δ 8.32 (s, 1H), 7.87 (s, 2H), 7.83 - 7.73 (m, 2H), 7.11 (t, *J* = 9.1 Hz, 1H), 6.84 - 6.77 (m, 0.5H), 6.66 (s, 1H), 6.61 - 6.55 (m, 0.5H), 5.20 - 5.06 (m, 1 H), 4.92 - 4.69 (m, 2H), 4.52 - 4.37 (m, 2H), 4.31 - 4.22 (m, 1H), 4.20 -4.08 (m, 1H), 3.24 (s, 3H), 2.95 (s, 3H), 2.69 - 2.60 (m, 2H), 2.47 - 2.24 (m, 5H), 2.20 - 2.08 (m, 1H), 1.98 - 1.86 (m, 1H), 1.83 (d, *J* = 11.2 Hz, 1H), 1.57 (d, *J* = 6.2 Hz, 3H), 1.08 (s, 3H), 0.99 (t, *J* = 7.5 Hz, 3H). $^{19}$F NMR (376 MHz, DMSO-$d_6$) δ -119.23, -138.98. |

(continued)

| Example | Structural Formula | Characterization Data |
|---|---|---|
| 193 | 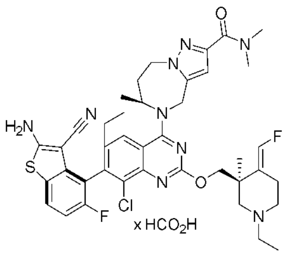 | LCMS(m/z): 788.3 (M+H). $^1$H NMR (400 MHz, DMSO-$d_6$) δ 8.40 (s, 1H), 7.87 (s, 2H), 7.82 - 7.74 (m, 2H), 7.11 (t, $J$ = 9.1 Hz, 1H), 6.86 - 6.76 (m, 0.5H), 6.72 - 6.63 (m, 1H), 6.59 (s, 0.5H), 5.21 - 5.05 (m, 1H), 4.94 - 4.72 (m, 2H), 4.54 - 4.38 (m, 2H), 4.34 - 4.23 (m, 1H), 4.22 - 4.07 (m, 1H), 3.24 (s, 3H), 2.95 (s, 3H), 2.79 - 2.68 (m, 2H), 2.48 - 2.36 (m, 3H), 2.35 - 2.23 (m, 4H), 2.20 - 2.09 (m, 1H), 2.03 - 1.92 (m, 1H), 1.86 (d, $J$ = 11.2 Hz, 1H), 1.56 (d, $J$ = 6.2 Hz, 3H), 1.09 (s, 3H), 1.03 - 0.91 (m, 6H). $^{19}$F NMR (376 MHz, DMSO-$d_6$) δ -73.42, -119.23, -139.30. |
| 194 | 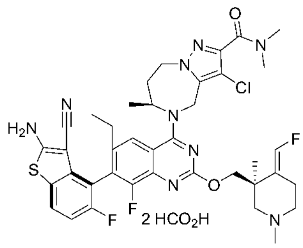 | LCMS(m/z): 792.3 (M+H). $^1$H NMR (400 MHz, DMSO-$d_6$) δ 8.41 (s, 2H), 7.92 (s, 2H), 7.82 (dd, $J$ = 8.7, 5.1 Hz, 1H), 7.49 (s, 1H), 7.17 - 7.09 (m, 1H), 6.66 (d, $J$ = 86.8 Hz, 1H), 5.04 - 4.92 (m, 1H), 4.88 - 4.71 (m, 2H), 4.40 - 4.30 (m, 1H), 4.28 - 4.15 (m, 2H), 4.12 - 4.05 (m, 1H), 3.02 (s, 3H), 2.98 (s, 3H), 2.66 - 2.60 (m, 2H), 2.47 - 2.28 (m, 5H), 2.21 - 2.05 (m, 4H), 1.95 - 1.84 (m, 1H), 1.84 - 1.73 (m, 1H), 1.62 (d, $J$ = 6.3 Hz, 3H), 1.06 (s, 3H), 1.01 - 0.94 (m, 3H). $^{19}$F NMR (376 MHz, DMSO-$d_6$) δ -118.54, -125.26, -138.92. |
| 195 | 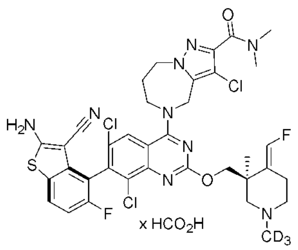 | LCMS(m/z): 803.1 (M+H). $^1$H NMR (400 MHz, DMSO-$d_6$) δ 8.25 (s, 1H), 7.97 (d, $J$ = 17.1 Hz, 3H), 7.86 - 7.77 (m, 1H), 7.18 - 7.09 (m, 1H), 6.84 - 6.56 (m, 1H), 5.10 - 4.86 (m, 2H), 4.51 - 4.27 (m, 4H), 4.19 (t, $J$ = 5.7 Hz, 2H), 3.01 (s, 3H), 2.97 (s, 3H), 2.72 - 2.65 (m, 2H), 2.38 - 2.31 (m, 2H), 2.20 - 2.11 (m, 1H), 1.94 - 1.85 (m, 1H), 1.81 (d, $J$= 11.3 Hz, 1H), 1.09 (s, 3H). $^{19}$F NMR (376 MHz, DMSO-$d_6$) δ -119.67, - 138.82. |
| 196 | 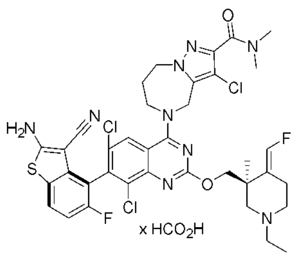 | LCMS(m/z): 814.2 (M+H). |
| 197 | 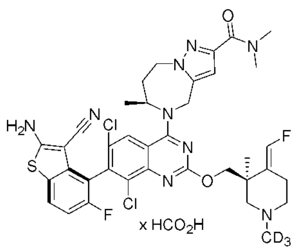 | LCMS(m/z): 783.2 (M+H). |
| 198 | 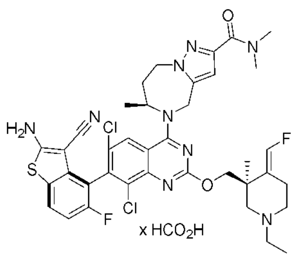 | LCMS(m/z): 794.2 (M+H). |

(continued)

| Example | Structural Formula | Characterization Data |
|---|---|---|
| 199 | 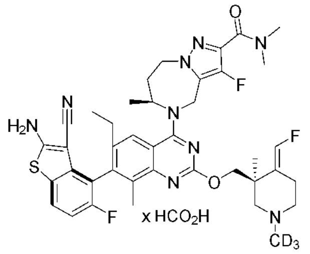 | LCMS(m/z): 775.3 (M+H). |
| 200 | 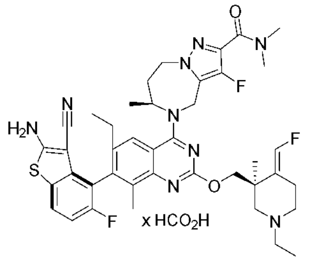 | LCMS(m/z): 786.3 (M+H). |
| 201 | 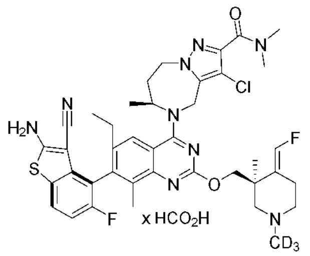 | LCMS(m/z): 791.3 (M+H). |
| 202 | 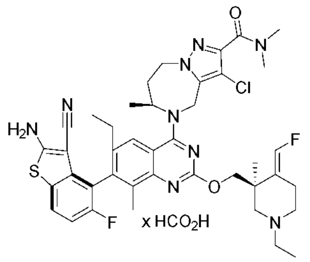 | LCMS(m/z): 802.3 (M+H). |
| 203 | 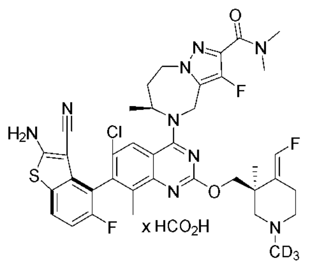 | LCMS(m/z): 781.3 (M+H). |
| 204 | 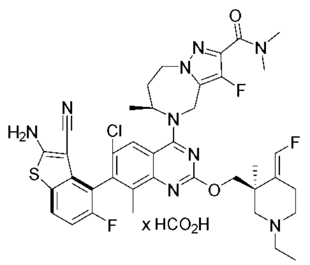 | LCMS(m/z): 792.3 (M+H). |

(continued)

| Example | Structural Formula | Characterization Data |
|---|---|---|
| 205 | 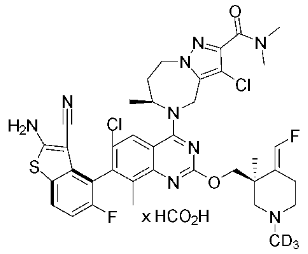 x HCO2H | LCMS(m/z): 797.2 (M+H). |
| 206 | 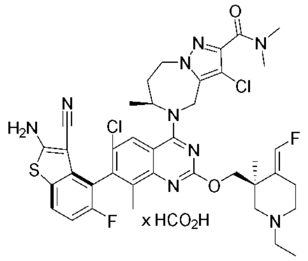 x HCO2H | LCMS(m/z): 808.2 (M+H). |
| 207 | 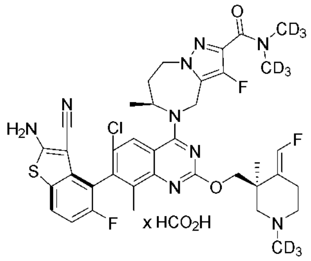 x HCO2H | LCMS(m/z): 787.3 (M+H). |
| 208 | 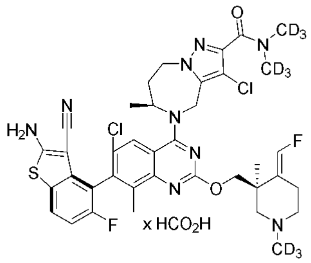 x HCO2H | LCMS(m/z): 803.3 (M+H). |
| 209 | 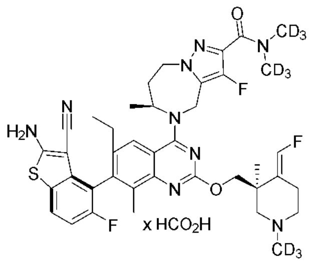 x HCO2H | LCMS(m/z): 781.4 (M+H). |
| 210 | 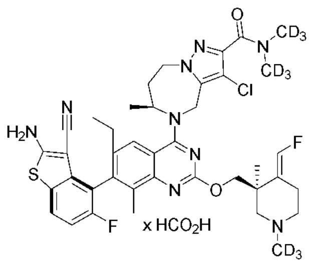 x HCO2H | LCMS(m/z): 797.3 (M+H). |

(continued)

| Example | Structural Formula | Characterization Data |
|---|---|---|
| 211 | 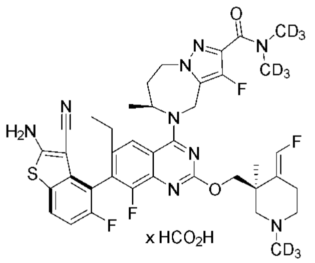 | LCMS(m/z): 785.3 (M+H). |
| 212 | 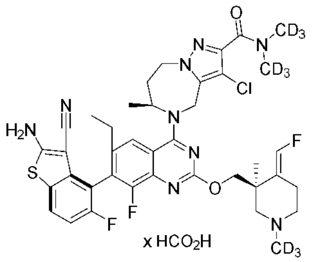 | LCMS(m/z): 801.3 (M+H). |

**Examples 213A and 214B**

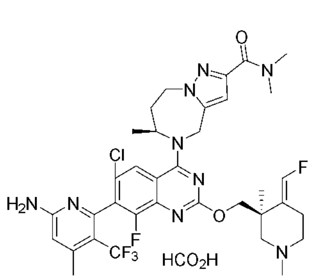

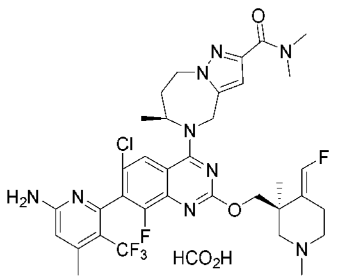

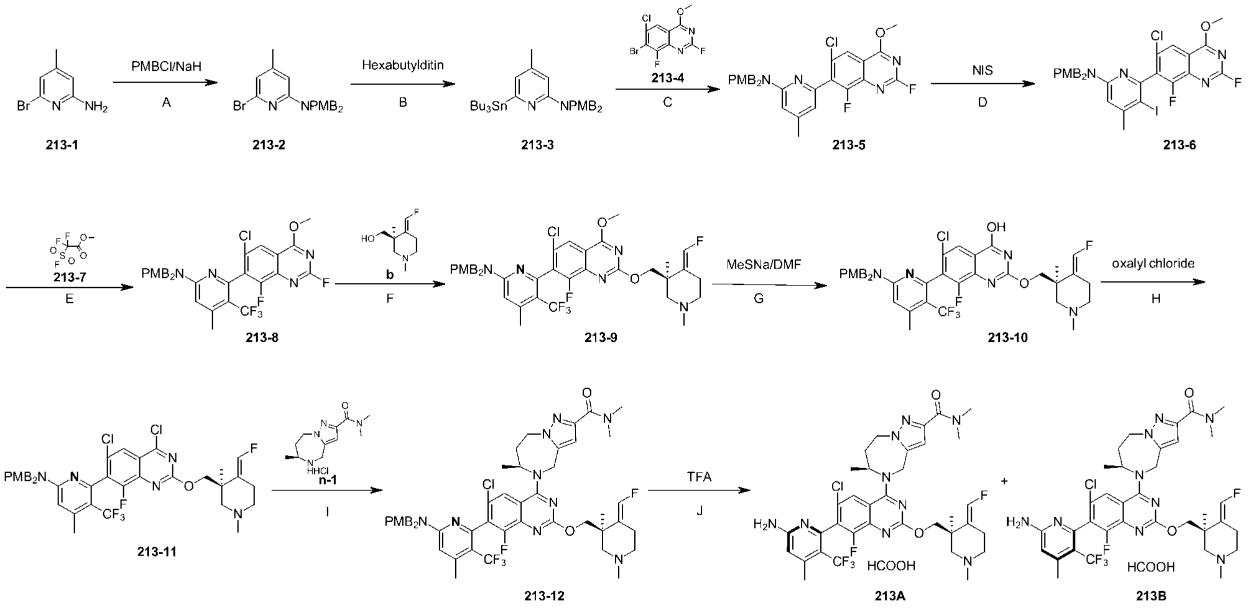

**Step A:** Compound **213-2**

Under ice-water bath conditions, NaH (3.21 g, 80.2 mmol) was added to a mixed solution of compound **213-1** (5.00 g, 26.7 mmol) and DMF (100 mL), and the mixture was warmed to room temperature and stirred for 1.0 h. Under ice-water bath conditions, PMBCl (9.21 g, 58.8 mmol) was added to the reaction solution, and the mixture was warmed to room temperature and stirred for 2.0 h. The reaction completion was monitored by LCMS, and the reaction solution was poured into a saturated ammonium chloride aqueous solution (500 mL) and extracted with EA (100 mL×3). After the organic phase was collected, washed successively with 5% LiCl aqueous solution (300 mL) and saturated brine (300 mL), and dried over anhydrous $Na_2SO_4$. After filtration, the organic phase was concentrated, and the crude product was purified by FCC ($SiO_2$, EA/PE=0-30%) to yield a yellow solid compound **213** (10.9 g, 96% yield). LCMS (m/z): 427.1 and 429.1 (M+H).

**Step B:** Compound **213-3**

At room temperature, Compound **213-2** (3.00 g, 7.02 mmol), hexa-n-butylditin (4.89 g, 8.42 mmol), $Pd_2(dba)_3$ (643 mg, 0.702 mmol), $P(Cy)_3$ (394 mg, 1.40 mmol), and LiCl (1.49 g, 35.1 mmol) were added sequentially to a solution of 1,4-dioxane (60 mL). After $N_2$ replacement three times, the mixture was warmed to 110°C and stirred for 16 h. The reaction completion was monitored by LCMS. The palladium catalyst was removed by diatomite filtration. The organic phase was concentrated after filtration, and the crude product was purified by FCC ($SiO_2$ (EA/PE=0-5%) to yield a yellow oily product **213-3** (4.5 g, 100% yield). LCMS (m/z): 639.3 (M+H).

**Step C:** Compound **213-5**

At room temperature, compounds **213-3** (3.71 g, 5.83 mmol), **213-4** (1.20 g, 3.88 mmol), Pd(PPh3)$_4$ (897 mg, 0.777 mmol), CuI (222 mg, 1.17 mmol, and LiCl (408 mg, 9.71 mmol) were sequentially added to a solution of 1,4-dioxane (100 mL). After $N_2$ replacement three times, the mixture was heated to 110°C and stirred for 16 h. The reaction completion was monitored by LCMS, and the palladium catalyst was removed by diatomite filtration. The organic phase was concentrated after filtration, and the crude product was purified by FCC ($SiO_2$ (EA/PE=0-25%) to yield a yellow solid compound **213-5** (800 mg, yield 36%). LCMS (m/z): 577.2 (M+H).

**Step D:** Compound **213-6**

At room temperature, NIS (1.56 g, 6.93 mmol), TsOH (11.9 mg, 0.0690 mmol), and DMF (20 mL) were added sequentially to a mixed solution of compound **213-5** (800 mg, 1.39 mmol) and stirred at room temperature for 6.0 h. The reaction completion was monitored by LCMS. The reaction solution was poured into saturated sodium thiosulfate (120 mL), extracted with EA (30 mL×3), and the organic phase was collected. The mixture was washed with saturated brine (70 mL) and dried over anhydrous $Na_2SO_4$. After filtration, the organic phase was concentrated, and the crude product was purified by FCC ( $SiO_2$ (EA/PE=0-35%) to yield a yellow solid compound **213-6** (830 mg, 85% yield). LCMS (m/z): 703.1 (M+H).

**Step E:** Compound **213-8**

At room temperature, compound **213-7** (5.47 g, 28.5 mmol) and CuI (2.60 g, 13.7 mmol) were added sequentially to a mixed solution of compound **213-6** (800 mg, 1.14 mmol) and DMA (30 mL), and the mixture was warmed to 90°C and stirred for 16.0 h. The reaction completion was monitored by LCMS. The reaction solution was diluted with EA (30 mL) and filtered. The filtrate was poured into water (150 mL) and extracted with EA (50 mL×3). After the organic phase was collected, washed with saturated brine (100 mL) and dried over anhydrous $Na_2SO_4$. After filtration, the organic phase was concentrated, and the crude product was purified by FCC ($SiO_2$, EA/PE=0-20%) to yield a yellow oily compound **213-8** (550 mg, 75% yield). LCMS (m/z): 645.2 (M+H).

**Step F:** Compound **213-9**

At room temperature, NaH (55.8 mg, 1.40 mmol) was added to a mixed solution of **Intermediate b** (121 mg, 0.698 mmol) and THF (10 mL), and was stirred at room temperature for 0.5 h. Then, at room temperature, Compound **213-8** (300mg, 0.465mmol) was added to the reaction solution, and was stirred at room temperature for 1.5 h. The reaction completion was monitored by LCMS, and the reaction mixture was poured into a saturated ammonium chloride aqueous solution (600 mL), and extracted with EA (30 mL×3). After the organic phase was collected, washed with saturated brine (70 mL) and dried over anhydrous $Na_2SO_4$. After filtration, the organic phase was concentrated, and the crude product was purified by FCC ($SiO_2$, EA/PE=0-60%) to yield a yellow solid compound **213-9** (150 mg, yield 40%). LCMS (m/z): 798.3

(M+H).

**Step G:** Compound **213-10**

At room temperature, sodium methanethiol (263 mg, 3.76 mmol) was added to a mixed solution of compound **213-9** (150 mg, 0.188 mmol) and DMF (2 mL), warmed to 90°C, and stirred for 2.0 h. The reaction completion was monitored by LCMS. The reaction solution was then poured into water (50 mL) and adjusted to pH=5 with 1 M hydrochloric acid. Extracted with EA (30 mL×3). After the organic phase was collected, washed successively with saturated sodium bicarbonate (50 mL) and saturated brine (70 mL), and dried over anhydrous $Na_2SO_4$. After filtration, the organic phase was concentrated, and the crude product was purified by FCC ($SiO_2$, EA/PE=0-90%) to yield a yellow solid compound **213-10** (140 mg, 95% yield). LCMS (m/z): 784.3 (M+H).

**Step H:** Compound **213-11**

Under ice-water bath conditions, DMF (13.0 mg, 0.179 mmol) was added to a mixed solution of oxalyl chloride (68.0 mg, 0.536 mmol) and DCM (3 mL), and stirred for 0.5 h under ice-water bath conditions. A DCM (2 mL) solution of compound **213-10** (140 mg, 0.179 mmol) was added to the above reaction solution under ice-water bath conditions, and stirred for 1.0 h after warming to room temperature. The reaction completion was monitored by LCMS, and the reaction solution was poured into water (50 mL), extracted with EA (30 mL×3). The organic phase was collected, washed with saturated brine (70 mL), and dried over anhydrous $Na_2SO_4$. After filtration, the organic phase was concentrated, and the crude product was purified by FCC ($SiO_2$, EA/PE=0-50%) to yield a brown solid compound **213-11** (100 mg, 70% yield). LCMS (m/z): 802.0 (M+H).

**Step I:** Compound **213-12**

At room temperature, Compound **213-11** (100 mg, 0.125 mmol), **Intermediate n-1** (64.2 mg, 0.249 mmol), and DIEA (80.5 mg, 0.623 mmol) were added sequentially to DMF (2.0 mL), and the mixture was warmed to 35°C and stirred for 24.0 h. The reaction completion was monitored by LCMS. After the reaction solution was returned to room temperature, it was quenched in water (50 mL), and extracted with EA (30 mL×3). The organic phase was collected, washed with saturated brine (70 mL), and dried over anhydrous $Na_2SO_4$. After filtration, the organic phase was concentrated, and the crude product was purified by FCC ($SiO_2$, EA/PE=0-80%) to yield a yellow solid compound 213-12 (40 mg, yield 33%). LCMS (m/z): 802.0 (M+H).

**Step J: Examples 213A and 213B**

At room temperature, Compound **213-12** (40 mg, 0.040 mmol) was added to TFA. The mixture was warmed to 50°C and stirred for 4.0 h. The reaction completion was monitored by LCMS. After concentration, saturated sodium bicarbonate aqueous solution (50 mL) was added to quench the reaction, and then extracted with EA (30 mL×3). After the organic phase was collected, it was washed with brine (70 mL) and dried over anhydrous $Na_2SO_4$. After filtration, the organic phase was concentrated, and the crude product was purified by pre-HPLC (C18, ACN/(10 mmol FA/$H_2O$) =30-45%) to obtain a white solid **Example 213A** (1.13 mg, yield 4.0%), RT=8.0 min, LCMS (m/z): 748.3 (M+H); and to obtain a white solid **Example 213B** (2.49 mg, yield 8.0%), RT=10.5 min, LCMS (m/z): 748.3 (M+H).

The following compounds were prepared and characterized in accordance with the above-described examples and appropriate variants.

| | | |
|---|---|---|
| 214 | 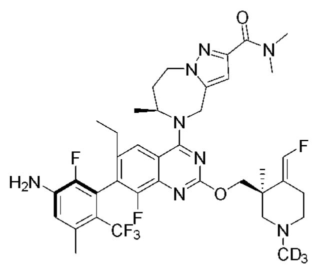 | LCMS(m/z): 762.3 (M+H). |

(continued)

| | | |
|---|---|---|
| 215 | 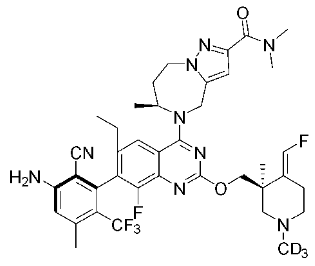 | LCMS(m/z): 769.3 (M+H). |
| 216 | 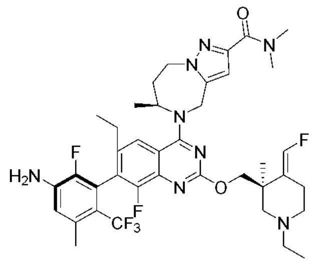 | LCMS(m/z): 773.3 (M+H). |
| 217 | 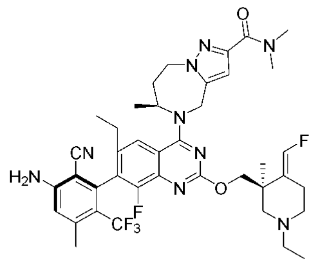 | LCMS(m/z): 780.3 (M+H). |
| 218 | 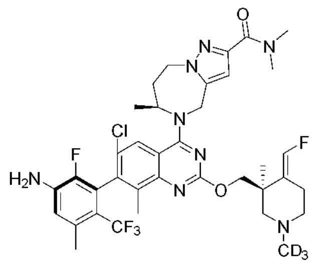 | LCMS(m/z): 764.3 (M+H). |
| 219 | 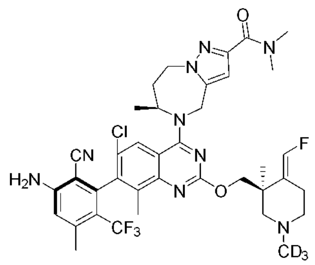 | LCMS(m/z): 771.3 (M+H). |
| 220 | 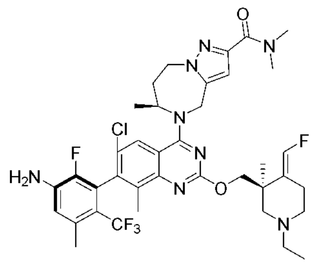 | LCMS(m/z): 775.3 (M+H). |

(continued)

| | | |
|---|---|---|
| 221 | 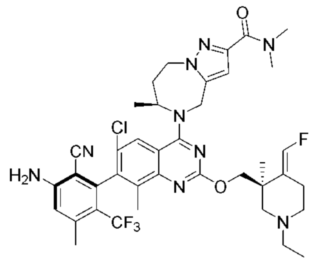 | LCMS(m/z): 782.3 (M+H). $^{1}$H NMR (400 MHz, DMSO-$d_6$) $\delta$ 8.41 (s, 2.5H), 7.95 (s, 1H), 7.31 - 7.18 (m, 0.5H), 6.90 - 6.79 (m, 3H), 6.67 (s, 1H), 6.63 - 6.57 (m, 0.5H), 5.08 (d, $J$ = 17.3 Hz, 1H), 4.87 - 4.72 (m, 2H), 4.53 - 4.40 (m, 2H), 4.33 (d, $J$ = 10.7 Hz, 1H), 4.25 - 4.10 (m, 1H), 3.25 (s, 3H), 2.96 (s, 3H), 2.78 - 2.70 (m, 2H), 2.47 - 2.37 (m, 4H), 2.35 - 2.24 (m, 4H), 2.22 - 2.09 (m, 3H), 2.06 - 1.92 (m, 2H), 1.85 (d, $J$ = 11.3 Hz, 1H), 1.51 (d, $J$ = 6.3 Hz, 3H), 1.10 (s, 3H), 0.97 (t, $J$ = 7.1 Hz, 3H). $^{19}$F NMR (376 MHz, DMSO-$d_6$) $\delta$ - 53.48, -139.24. |
| 222 | 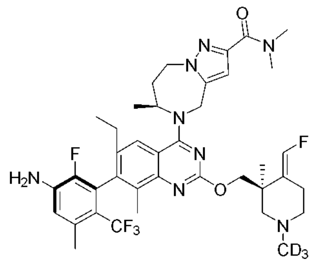 | LCMS(m/z): 758.3 (M+H). |
| 223 | 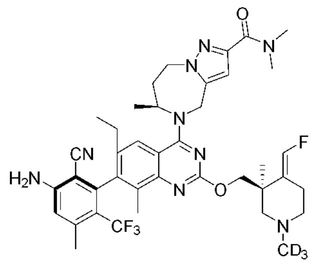 | LCMS(m/z): 765.4 (M+H). |
| 224 | 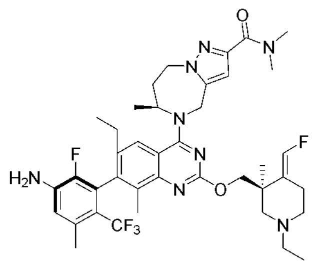 | LCMS(m/z): 769.3 (M+H). |
| 225 | 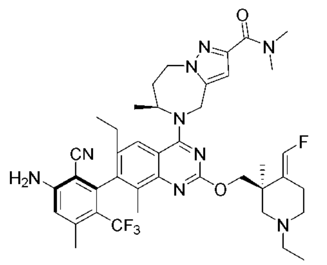 | LCMS(m/z): 776.3 (M+H). |

**Examples 226A and 226B**

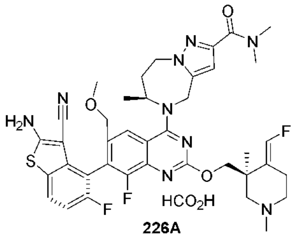

HCO$_2$H

226A

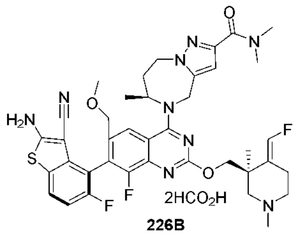

2HCO$_2$H

226B

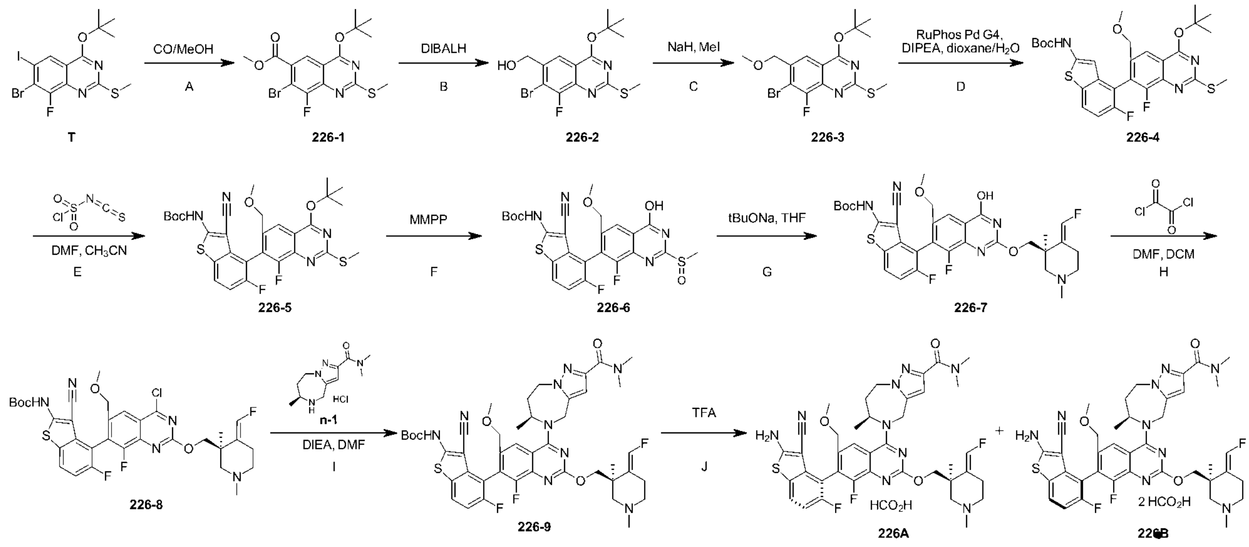


**Step A:** Compound **226-1**

At room temperature, Pd(dppf)Cl$_2$ (776 mg, 1.05 mmol) and triethylamine (3.2 g, 30.5 mmol) were added to a solution of **Intermediate T** (5.0 g, 10.5 mmol) in a methanol (60 mL). The solution was replaced three times with carbon monoxide (15 psi), and the resulting reaction mixture was warmed to 80°C and stirred for 24 h. After the reaction was completed, the solution was filtered through diatomite, and the filtrate was concentrated to dryness. The crude product was purified by FCC (SiO$_2$, PE/DCM=0-10%) to give a yellow solid compound **226-1** (2.8 g, yield 65%). LCMS (m/z): 403.9 (M+H).

**Step B:** Compound **226-2**

Under nitrogen protection in an ice bath, 1N of diisobutylaluminum hydride (17.3 mL, 17.3 mmol) was added to a solution of compound **226-1** (2.8 g, 6.9 mmol) in dichloromethane (30 mL), and the mixture was stirred for 3 h while maintaining the temperature. After the reaction completion was monitored by LCMS, sodium sulfate decahydrate was added to the reaction solution until no more bubbles were generated. The reaction solution was filtered and concentrated to dryness. The crude product was purified by FCC (SiO$_2$, EA/PE=0-15%) to give a yellow solid compound **226-2** (2.2 g, yield 85%). LCMS (m/z): 374.9 (M+H).

**Step C:** Compound **226-3**

Under ice bath conditions, sodium hydride (352 mg, 8.8 mmol) was added to a solution of Compound **226-2** (2.2 g, 5.9 mmol) in anhydrous DMF (30 mL). The mixture was stirred for 0.5 h while maintaining the temperature, and then iodomethane (1.2 g, 8.8 mmol) was added to the reaction solution. The resulting mixture was stirred for another 1 h at room temperature. After the reaction completion was monitored by LCMS, the reaction solution was poured into 50 mL of saturated ammonium chloride aqueous solution. The solution was then extracted with ethyl acetate. The combined organic phases were washed with saturated brine, dried over anhydrous sodium sulfate, filtered, and concentrated to dryness. The crude product was purified by FCC (SiO$_2$, EA/PE=0.5%) to give a yellow solid compound **226-3** (1.93 g, 85% yield). LCMS (m/z): 391.0 (M+H).

**Step D:** Compound **226-4**

Under $N_2$ conditions, **intermediate w3** (585 mg, 1.5 mmol), DIPEA (664 mg, 5.0 mmol), and RuphosPd-G4 (85 mg, 0.1 mmol) were added to a solution of compound 226-3 (400 mg, 1.0 mmol) in 1,4-dioxane (5 mL) and water (1 mL). The resulting reaction solution was stirred at 70°C for 2 h. After the reaction completion was monitored by LCMS, the reaction solution was filtered through diatomite, the filtrate was concentrated to dryness, and the crude product was purified by FCC ($SiO_2$, EA/PE=0-10%) to give a yellow solid compound **226-4** (360 mg, yield 60%). LCMS (m/z): 576.1 (M+H).

**Step E:** Compound **226-5**

Under nitrogen protection in an ice bath, chlorosulfonyl isocyanate (293 mg, 1.86 mmol) was added to a solution of compound **226-4** (360 mg, 0.62 mmol) in acetonitrile (26 mL). The reaction mixture was stirred for 0.5 h while maintaining the temperature, followed by the addition of 1 mL of LDMF. The resulting reaction mixture was stirred for another 0.5 h at room temperature. After the reaction completion was monitored by LCMS, 50 mL of saturated sodium bicarbonate aqueous solution was added to the reaction mixture, and the solution was extracted with ethyl acetate. The organic phases were combined, washed with saturated brine, dried over anhydrous sodium sulfate, filtered, and concentrated to dryness. The crude product was purified by FCC ($SiO_2$, EA/PE=0-10%) to give a yellow solid compound **226-5** (290 mg, yield 77%). LCMS (m/z): 601.1 (M+H).

**Step F:** Compound **226-6**

At room temperature, a solution of magnesium monoperoxyphthalate hexahydrate (MMPP, 864 mg, 1.45 mmol) in water (2 mL) was added to a solution of compound **226-5** (290 mg, 0.48 mmol) in dichloromethane (5 mL), and the mixture was stirred for 3 h while maintaining the temperature. After the reaction completion was monitored by LCMS, 30 mL of water was added to the reaction mixture, and the mixture was extracted with dichloromethane (30 mLx2). The organic phases were combined, washed with saturated brine, dried over anhydrous sodium sulfate, filtered, and concentrated to dryness to give a yellow solid crude product, compound **226-6** (310 mg). LCMS (m/z): 681.0 (M+H).

**Step G:** Compound **226-7**

Under nitrogen protection at -70°C, 1N sodium tert-butoxide THF solution (1.6 mL, 1.6 mmol) was added to a solution of compound **226-6** (310 mg, 0.55 mmol) and ((S)-(E)-4-fluoromethylene-1-methyl-3-methyl-3-piperidinyl) methanol **(intermediate b,** 191 mg, 1.1 mmol) in anhydrous tetrahydrofuran (6 mL). The reaction mixture was allowed to react at -70°C up to room temperature for 2 h. After the reaction completion was monitored by LCMS, the mixture was poured into 30 mL of saturated ammonium chloride solution and extracted with ethyl acetate. The combined organic phases were washed with saturated brine, dried over anhydrous sodium sulfate, filtered, and concentrated to dryness. The crude product was purified by FCC (THF/PE=0-70%) to give a yellow solid compound **226-7** (140 mg, yield 38%). LCMS (m/z): 670.1 (M+H).

**Step H:** Compound **226-8**

Under nitrogen protection at 0°C, DMF (3 mg, 0.04 mmol) was added to a solution of oxalyl chloride (80 mg, 0.63 mmol) in anhydrous dichloromethane (5 mL). After stirring and maintaining the temperature for 0.5 h, compound **226-7** (140 mg, 0.21 mmol) was added to the reaction solution, and the reaction solution was further reacted at 0°C for 1 h. After the reaction completion was monitored by LCMS, the reaction solution was poured into 20 mL of saturated sodium bicarbonate solution and extracted with dichloromethane. The combined organic phases were washed with saturated brine, dried over anhydrous sodium sulfate, filtered, and concentrated to dryness to obtain a yellow solid crude product, compound **226-8** (150 mg). LCMS (m/z): 688.0 (M+H).

**Step I:** Compound **226-9**

Under nitrogen protection, DIPEA (142 mg, 1.1 mmol) was added to a solution of Compound **226-8** (150 mg, 0.22 mmol) and **Intermediate n-1** (97 mg, 0.44 mmol) in anhydrous DMF (3 mL). The resulting reaction solution was stirred at 70°C for 16 h. After the reaction completion was monitored by LCMS, the reaction solution was poured into 30 mL of water. The mixture was then extracted with ethyl acetate. The combined organic phases were washed with saturated brine, dried over anhydrous sodium sulfate, filtered, and concentrated to dryness. The crude product was purified by pre-TLC (MeOH/DCM=10%) to give a yellow solid compound **226-9** (40 mg, yield 21%). LCMS (m/z): 875.1 (M+H).

**Step J: Examples 226A and 226B**

At room temperature, 1 mL of trifluoroacetic acid was added to a solution of Compound **226-9** (40 mg, 0.04 mmol) in dichloromethane (1 mL), and the mixture was stirred for 1 h while maintaining the temperature. After the reaction completion was monitored by LCMS, the reaction solution was purified by pre-HPLC (C18, CAN/(0.1%FA/$H_2O$)=25-40%), and the first eluted white solid was **Example 226A** (5.1 mg, yield 15%, Rt=8.5 min). $^1H$ NMR (400 MHz, DMSO-$d_6$) δ 8.30 (s, 1H), 7.94 - 7.86 (m, 3H), 7.82 (dd, *J* = 8.7, 4.9 Hz, 1H), 7.16 - 7.07 (m, 1H), 6.87 - 6.55 (m, 2H), 5.19 - 5.08 (m, 1H), 4.96 - 4.85 (m, 1H), 4.84 - 4.74 (m, 1H), 4.54 - 4.38 (m, 2H), 4.27 - 4.09 (m, 4H), 3.27 (s, 3H), 3.16 (s, 3H), 2.96 (s, 3H), 2.69 - 2.63 (m, 2H), 2.46 - 2.24 (m, 3H), 2.17 - 2.10 (m, 4H), 1.95 -1.80 (m, 2H), 1.53 (d, *J* = 6.2 Hz, 3H), 1.09 (s, 3H). $^{19}F$ NMR (376 MHz, DMSO-$d_6$) δ -118.74, -125.91, -138.91. LCMS (m/z): 774.1 (M+H); and the second eluted white solid was **Example 226B** (1.3 mg, yield 4%, Rt=10.3 min). $^1H$ NMR (400 MHz, DMSO-$d_6$) δ 8.36 (s, 2H), 7.94 (s, 2H), 7.86 (s, 1H), 7.81 (dd, *J* = 8.7, 4.9 Hz, 1H), 7.16 - 7.07 (m, 1H), 6.84 - 6.55 (m, 2H), 5.09 - 4.97 (m, 1H), 4.93 - 4.79 (m, 2H), 4.54 - 4.42 (m, 2H), 4.30 - 4.00 (m, 4H), 3.26 (s, 3H), 3.17 (s, 3H), 2.95 (s, 3H), 2.70 - 2.63 (m, 2H), 2.47 - 2.23 (m, 3H), 2.20 - 2.12 (m, 4H), 1.95 - 1.77 (m, 2H), 1.53 (d, *J* = 6.3 Hz, 3H), 1.08 (s, 3H). $^{19}F$ NMR (376 MHz, DMSO-$d_6$) δ -118.94, -126.40, -138.93. LCMS (m/z): 774.1 (M+H).

The following compounds were prepared and characterized in accordance with the above-described examples and appropriate variants.

| | | |
|---|---|---|
| 227A | 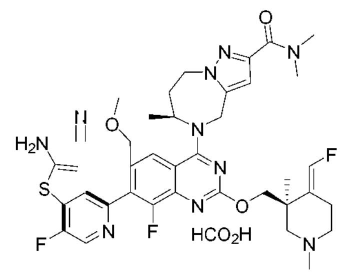 | LCMS (m/z): 775.3 (M+H). $^1H$ NMR (400 MHz, DMSO-$d_6$) δ 8.49 - 8.16 (m, 4H), 7.87 (s, 1H), 6.84 - 6.55 (m, 2H), 5.17 - 5.08 (m, 1H), 4.93 - 4.87 (m, 1H), 4.84 - 4.76 (m, 1H), 4.50 - 4.43 (m, 2H), 4.28 - 4.17 (m, 4H), 3.26 (s, 3H), 3.06 (s, 3H), 2.95 (s, 3H), 2.69 - 2.63 (m, 2H), 2.40 - 2.28 (m, 2H), 2.18 - 2.09 (m, 4H), 2.01 - 1.81 (m, 3H), 1.53 (d, *J* = 6.2 Hz, 3H), 1.09 (s, 3H). $^{19}F$ NMR (376 MHz, DMSO-$d_6$) δ -128.22, -132.54, -138.88. |
| 227B | 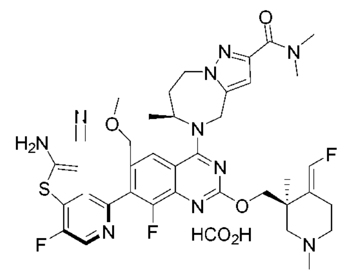 | LCMS (m/z): 775.3 (M+H). |

**Examples 228A and 228B**

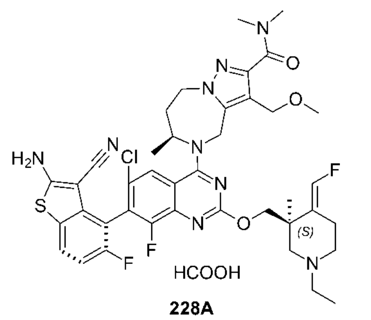

228A

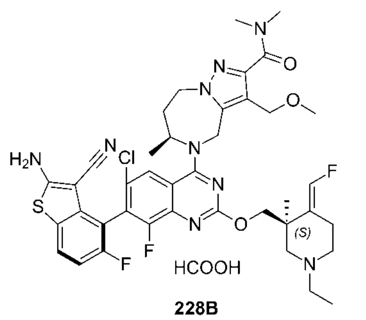

228B

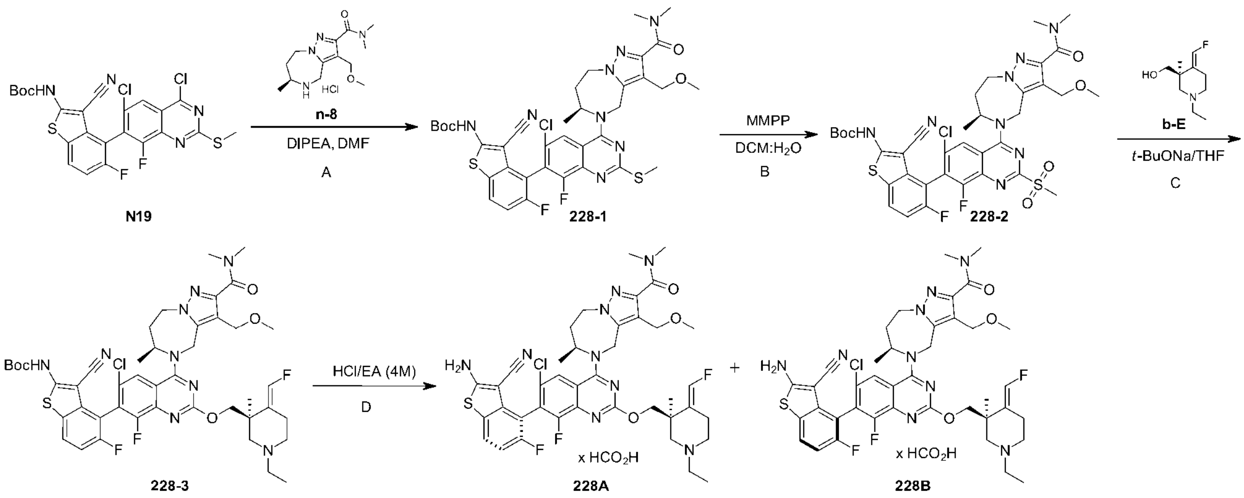

N19, 228-1, 228-2, 228-3, 228A, 228B

Step A: Compound 228-1

At room temperature, intermediate n-8 (163 mg, 0.54 mmol), N19 (150 mg, 0.27 mmol), and DIEA (350 mg, 2.7 mmol) were added sequentially to DMF (4 mL). After $N_2$ replacement three times, the mixture was heated to 50°C and stirred for 16 hours. The reaction completion was monitored by LCMS and TLC. The reaction solution was then returned to room temperature, and water (30 mL) was added. Extraction was performed using EA (30 mL×3). The collected organic phase was washed with saturated brine and dried over anhydrous $Na_2SO_4$. After filtration and concentration, the crude product obtained was purified by FCC ($SiO_2$, EA/PE=0-100%) to give a pale yellow solid compound 228-1 (100 mg). LCMS (m/z): 783.2 (M+H).

Step B: Compound 228-2

At room temperature, magnesium monoperoxyphthalate hexahydrate (316 mg, 0.64 mmol) was added to a mixed solution of compound **228-1** (100 mg, 0.13 mmol) and DCM/$H_2O$ (V/V=1:1, 5 mL) and stirred for 16 h. After the reaction completion was monitored by LCMS, the reaction solution was quenched with $NaHCO_3$ (30 mL) solution, extracted with DCM (30 mL×3), and the organic phase was collected, washed with saturated saline solution, and dried over anhydrous $Na_2SO_4$, concentrated to yield a yellow solid compound **228-2** (110 mg, crude product), which was used directly in the next step. LCMS (m/z): 815.2 (M+H).

**Step C:** Compound **228-3**

Under dry ice and ethanol bath conditions, *t*-BuONa (0.33 mL, 2 M in THF, 0.65 mmol) was added to a mixed solution of compound **228-2** (110 mg), intermediate **bE** (50 mg, 0.26 mmol), and THF (4 mL), and the mixture was stirred for 10 min while maintaining the temperature. The mixture was then warmed to room temperature and stirred for 30 min. The reaction completion was monitored by LCMS. The reaction solution was poured into a semi-saturated aqueous solution of $NH_4Cl$ (20 mL), extracted with EA (30 mL×3), and the organic phase was collected. The mixture was washed with saturated saline solution and dried over anhydrous $Na_2SO_4$. The solution was filtered and concentrated to obtain a pale yellow solid, compound **228-3** (120 mg, crude product). LCMS (m/z): 922.3 (M+H).

**Step D: Examples 228A** and **228B**

At room temperature, 4M HCl-ethyl acetate (5 mL, 20 mmol) was added to compound **228-3** (120 mg), and the mixture was stirred at room temperature for 1 h. The reaction completion was monitored by LCMS. After concentration, saturated sodium bicarbonate aqueous solution (20 mL) was added, followed by extraction with EA (30 mL×2). The extract was collected, washed with brine (40 mL), and dried over anhydrous $Na_2SO_4$. After filtration, the organic phase was concentrated and purified by pre-HPLC (C18, ACN/(10 mmol FA/$H_2O$)=30-38%) to obtain the first eluted white solid isomer 1, which was Example **228A** (21.10 mg, Rt=8.75 min). The second eluted white solid isomer 2 was **Example 228B** (10.05 mg, Rt=11.88 min). LCMS (m/z): 822.3 (M+H). $^1$H NMR (400 MHz, DMSO-$d_6$) δ 8.39 (s, 1.54H), 8.02 (s, 2H), 7.85 (dd, *J* = 8.8, 4.8 Hz, 1H), 768 (s, 1H), 7.14 (t, *J* =9.2Hz, 1H), 6.80 (s, 0.5H), 6.58 (s, 0.5H), 4.98 (d, *J* = 16.8 Hz, 1H), 4.81-4.76 (m,2H), 4.54 (s, 2H), 4.48-4.43 (m,2H), 4.29 (d, *J* =10.4 Hz, 1H), 4.12-4.05 (m, 1H), 3.23 (s, 3H), 3.06 (s, 3H), 2.95 (s, 3H), 2.78-2.75 (m, 2H), 2.56-2.48 (m, 1H), 2.42-2.26 (m, 6H), 1.95-1.89 (m, 1H), 1.83 (d, *J* =11.2 Hz, 1H), 1.55 (d, *J* =6.0 Hz, 3H),

1.09 (s, 3H), 0.96 (t, $J$ =7.2 Hz, 3H). $^{19}$F NMR (376 MHz, DMSO-$d_6$) δ -119.08, -121.45, -139.18.

Example **228A** was further purified by SFC (Waters SFC 150, separation column: DAICELCHIRALPAK®IH, 250*30mm 10μm, mobile phase: $CO_2$/MeOH (+0.2% 7.0mol/L $NH_3$-MeOH)=75/25; flow rate: 140mL/min) to remove a small amount of non-isomer impurities, yielded a white solid, Example **228A** (21.10mg). Chirality analysis was performed using SFC-228, Rt=2.202. LCMS (m/z): 822.3 (M+H). $^1$H NMR (400 MHz, DMSO-$d_6$) δ 7.99 (s, 2H), 7.85 (dd, $J$ = 8.8,4.8 Hz, 1H), 7.74 (s, 1H), 7.14 (t, $J$ =9.2Hz, 1H), 6.80 (s, 0.5H), 6.58 (s, 0.5H), 5.12 (d, $J$ = 16.8 Hz, 1H), 4.86-4.80 (m,2H), 4.72 (d, $J$ =16.0Hz, 1H), 4.62-4.52 (m, 2H), 4.40-4.35 (m, 1H), 4.72 (d, $J$ =10.4Hz, 1H), 4.16-4.08 (m,1H), 3.23 (s, 3H), 3.07 (s, 3H), 2.96 (s, 3H), 2.79-2.73 (m, 2H), 2.54-2.48 (m, 1H), 2.35-2.27 (m, 4H), $^{19}$F NMR(376 MHz, DMSO-$d_6$) δ -118.67, -120.61, -139.16.

Chiral analysis method SFC-228: Waters UPCC (CA-352), analytical column: DAICELCHIRALPAK®IH, 100*3mm 3μm; mobile phase A: $CO_2$. Phase B: MeOH (0.1% DEA); Flow rate: 1.5 mL/min; Column temperature: 35°C; Back pressure: 1800 psi; Gradient: 0-8.0 min A/B=75/25.

**Examples 229A and 229B**

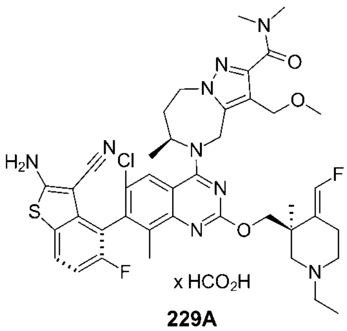

x $HCO_2H$

**229A**

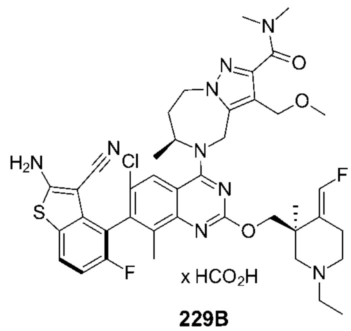

x $HCO_2H$

**229B**

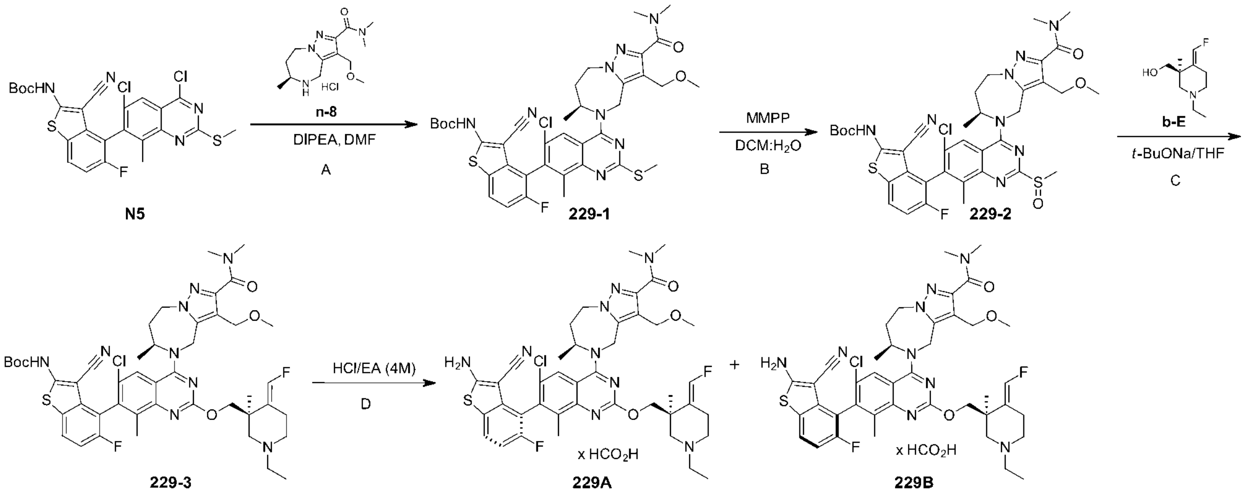


**Step A:** Compound **229-1**

At room temperature, intermediate **N5** (183 mg, 0.33 mmol), **n-8** (106 mg, 0.40 mmol), and DIEA (340 mg, 2.64 mmol) were added sequentially to DMF (3 mL). The resulting reaction solution was reacted at 60°C for 16 h. The reaction completion was monitored by LCMS and TLC. The reaction solution was poured into water (30 mL), extracted with EA (30 mL×3). The organic phase was collected, washed with saturated brine (30 mL), dried over anhydrous $Na_2SO_4$, filtered, and concentrated. The organic phase was purified by FCC ($SiO_2$, EA/PE=0.63%) to obtain a yellow solid compound **229-1** (200 mg, yield 77%). LCMS (m/z): 779.3 (M+H).

**Step B:** Compound **229-2**

At room temperature, magnesium monoperoxyphthalate hexahydrate (584 mg, 1.03 mmol) was added to a

mixed solution of compound **229-1** (200 mg, 0.257 mmol) in DCM (4 mL) and water (4 mL), and stirred for 1 h at room temperature. After the reaction completion was monitored by LCMS, the reaction solution was quenched in (30 mL) solution, extracted with DCM (30 mL×3). The organic phase was collected, washed with saturated saline solution, dried over anhydrous $Na_2SO_4$, and concentrated to obtain a yellow solid compound **229-2** (250 mg, crude product), which was directly used in the next step. LCMS (m/z): 795.3 (M+H).

**Step C:** Compound **229-3**

Under dry ice and ethanol bath conditions, tBuONa (0.63 mL, 2 M in THF, 1.26 mmol) was added to a solution of compound **229-2** (250 mg, 0.315 mmol), intermediate **bE** (88 mg, 0.472 mmol), and THF (4 mL). The resulting reaction solution was allowed to react for 1 h at room temperature. The reaction completion was monitored by TLC, the reaction solution was poured into a semi-saturated solution of $NH_4Cl$ (30 mL), and extracted with EA (30 mL×3). The organic phase was collected, washed with saturated saline solution, and dried over anhydrous $Na_2SO_4$ to obtain a yellow solid compound **229-3** (203 mg, 86% yield in two steps), which was used directly in the next step. LCMS (m/z): 918.3 (M+H).

**Step D:** Examples **229A** and **229B**

At room temperature, a mixture of compound **229-3** (203 mg) and HCl-EA (4N, 5 mL) was stirred for 1 h. After the reaction completion was detected by LCMS, the mixture was concentrated and saturated sodium bicarbonate aqueous solution (30 mL) was added. The mixture was then extracted with EA (30 mL×2). The extract was collected, washed with brine (30 mL), dried over anhydrous $Na_2SO_4$, and the organic phase was concentrated and purified by pre-HPLC (C18, ACN/(10 mmol FA/$H_2O$)=30-37%). The first eluted white solid isomer 1 was Example **229A** 15-1 (47.1 mg, Rt=9.47 min). The second eluted white solid isomer 2 was Example **229B** (27.9 mg, Rt=12.18 min). LCMS (m/z): 818.3 (M+H). $^1$H NMR (400 MHz, DMSO-$d_6$) δ 8.35 (s, 1H), 7.90 (s, 2H), 7.79 (dd, *J* = 8.7, 5.0 Hz, 1H), 7.65 (s, 1H), 7.16 - 7.08 (m, 1H), 6.86 - 6.58 (m, 1H), 5.00 - 4.90 (m, 1H), 4.83 - 4.69 (m, 2H), 4.57 - 4.32 (m, 5H), 4.11 - 3.99 (m, 1H), 3.22 (s, 3H), 3.05 (s, 3H), 2.95 (s, 3H), 2.75 (d, *J* = 10.6 Hz, 2H), 2.59 - 2.52 (m, 1H), 2.42 - 2.25 (m, 4H), 2.23 (s, 3H), 2.20 - 2.11 (m, 1H), 2.00 - 1.93 (m, 1H), 1.90 - 1.83 (m, 1H), 1.56 (d, *J* = 6.4 Hz, 3H), 1.10 (s, 3H), 0.96 (*t*, *J* = 7.1 Hz, 3H). $^{19}$F NMR (376 MHz, DMSO-$d_6$) δ -120.08, -139.30.

Example **229A** (44 mg) was further purified by SFC (Waters SFC 150, column: DAICELCHIRALCEL®IH 250*30 mm 10 μm; mobile phase: $CO_2$/MeOH (+0.1% 7.0 mol/L $NH_3$-MeOH)=70/30; flow rate: 140 mL/min) to remove a small amount of non-isomer impurities, yielded a white solid, Example **229A** (27.8 mg). Chiral analysis method SFC-229, Rt=2.059 min. LCMS(m/z): 818.3 (M+H). $^1$H NMR (400 MHz, DMSO-$d_6$) δ 7.87 (s, 2H), 7.79 (dd, *J* = 8.7, 4.9 Hz, 1H), 7.73 (s, 1H), 7.14 - 7.08 (m, 1H), 6.83 - 6.54 (m, 1H), 5.08 (d, *J* = 16.9 Hz, 1H), 4.85 - 4.75 (m, 1H), 4.72 - 4.57 (m, 2H), 4.53 (d, *J* = 11.8 Hz, 1H), 4.49 - 4.23 (m, 3H), 4.12 (t, *J* = 12.2 Hz, 1H), 3.29 (s, 2H), 3.22 (s, 3H), 3.06 (s, 3H), 2.96 (s, 3H), 2.74 (d, *J* = 10.8 Hz, 2H), 2.42 - 2.25 (m, 4H), 2.23 (s, 3H), 2.19 - 2.08 (m, 1H), 2.01 - 1.91 (m, 1H), 1.85 (d, *J* = 11.3 Hz, 1H), 1.58 (d, *J* = 6.3 Hz, 3H), 1.09 (s, 3H), 0.95 (t, *J* = 7.1 Hz, 3H). $^{19}$F NMR (376 MHz, DMSO-$d_6$) δ -119.52, - 139.29.

Chiral analysis method SFC-229: Waters UPCC (CA-352), analytical column: DAICELCHIRALPAK®IH, 100*3mm 3μm, mobile phase A: $CO_2$, mobile phase B: MeOH (0.1%DEA); flow rate: 1.5mL/min; column temperature: 35°C; back pressure: 1800 psi; gradient: 0-8 min A/B=75/25.

**Examples 230A and 230B**

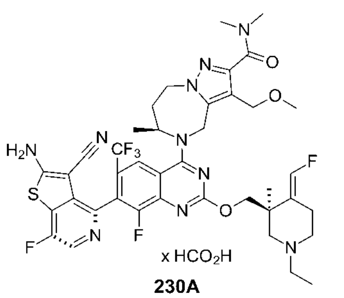

x $HCO_2H$

**230A**

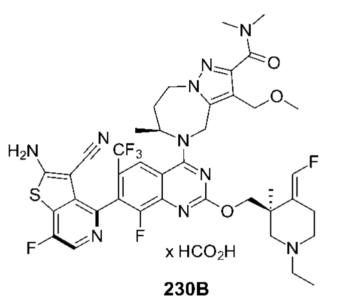

x $HCO_2H$

**230B**

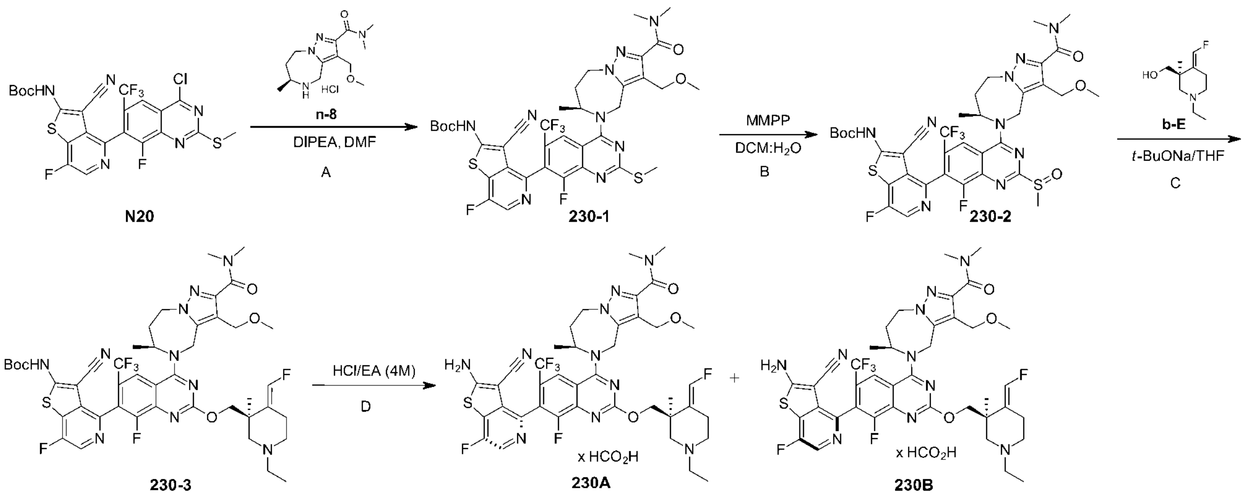

N20, 230-1, 230-2, 230-3, 230A, 230B

Synthesis of Examples **230A and 230B** was carried out according to the above method. The crude product obtained in step D was purified by pre-HPLC (C18, ACN/(10mmol FA/$H_2O$)=29-34%) to obtain the first eluted white solid isomer 1, which was Example **230A** (19.6mg, Rt=9.1min). LCMS (m/z): 857.3 (M+H). The second eluted white solid isomer 2, which was Example **230B** (20.2mg, Rt=10.7min). LCMS (m/z): 857.3 (M+H). $^1$H NMR (400 MHz, DMSO-$d_6$) δ 8.46 (s, 2H), 8.43 (s, 1H), 8.35 (s, 1H), 7.96 (s, 1H), 6.84 - 6.75 (m, 0.5H), 6.62 - 6.53 (m, 0.5H), 5.11 - 4.96 (m, 1H), 4.91 - 4.61 (m, 2H), 4.58 - 4.35 (m, 4H), 4.29 (d, *J*= 10.5 Hz, 1H), 4.17 - 3.81 (m, 1H), 3.22 (s, 3H), 3.06 (s, 3H), 2.96 (s, 3H), 2.85 - 2.72 (m, 2H), 2.59 - 2.51 (m, 1H), 2.44 - 2.22 (m, 3H), 2.19 - 2.09 (m, 1H), 2.04 - 1.94 (m, 1H), 1.94 - 1.85 (m, 1H), 1.81 (d, *J* = 11.3 Hz, 1H), 1.62 (d, *J* = 6.3 Hz, 3H), 1.08 (s, 3H), 0.94 (t, *J* = 7.1 Hz, 3H). $^{19}$F NMR (376 MHz, DMSO-$d_6$) δ -57.47, -125.18, -131.61, - 139.02.

Example **230A** was further purified by SFC (Waters SFC 150, separation column: DAICELCHIRALPAK®IH, 250*30mm 10 μm, mobile phase: $CO_2$/MeOH (+0.1% 7.0mol/L $NH_3$-MeOH=80:20) to remove a small amount of non-isomer impurities, yielded Example **230A** (8.9 mg). Chiral analysis was performed using SFC-230, with Rt=3.103 min. LCMS (m/z): 857.3 (M+H). $^1$H NMR (400 MHz, DMSO-$d_6$) δ 8.42 (s, 3H), 8.00 (s, 1H), 6.84 - 6.75 (m, 0.5H), 6.61 - 6.54 (m, 0.5H), 5.12 (d, *J* = 16.8 Hz, 1H), 4.89 - 4.69 (m, 2H), 4.51 (s, 2H), 4.45 - 4.32 (m, 2H), 4.31 - 4.24 (m, 1H), 4.20 - 4.06 (m, 1H), 3.24 (s, 3H), 3.06 (s, 3H), 2.96 (s, 3H), 2.78 (t, *J* = 12.0 Hz, 2H), 2.55 - 2.51 (m, 1H), 2.42 - 2.35 (m, 1H), 2.34 - 2.31 (m, 1H), 2.31 - 2.28 (m, 1H), 2.19 - 2.03 (m, 1H), 1.95 - 1.87 (m, 1H), 1.82 (d, *J* = 11.3 Hz, 1H), 1.79 - 1.73 (m, 1H), 1.63 (d, *J* = 6.2 Hz, 3H), 1.08 (s, 3H), 0.95 (t, *J* = 7.1 Hz, 3H). $^{19}$F NMR (376 MHz, DMSO-$d_6$) δ -57.39, -124.97, -131.67, -139.03.
Chiral analysis method SFC-230: Waters UPCC (CA-352), analytical column: DAICELCHIRALPAK®IH, 100*3mm 3μm, mobile phase A: $CO_2$, mobile phase B: MeOH(0.1%DEA); flow rate: 1.5mL/min; column temperature: 35°C; back pressure: 1800 psi; gradient: 0-8 min, A/B=8.5/1.5.

**Examples 231A and 231B**

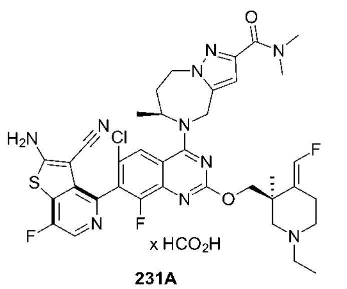

231A

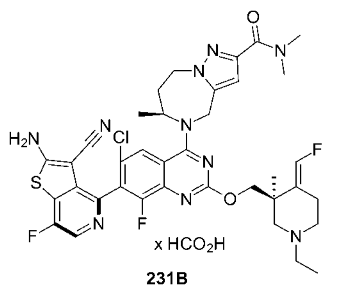

231B

Synthesis of Examples **231A** and **231B** was carried out starting from intermediate **N21** according to the method described above. The crude product obtained in step D was purified by pre-HPLC (C18, ACN/(10mmol FA/$H_2O$) =20%-35%) to obtain the active first eluted white solid isomer 1, which was Example **231A** (20.5 mg, yield 12%, Rt=10.0 min). LCMS (m/z): 779.3 (M+H). $^1$H NMR (400 MHz, DMSO-$d_6$) δ 8.57 - 8.29 (m, 4H), 7.87 (s, 1H), 6.84 - 6.56 (m, 2H), 5.13 (d, *J*= 17.2 Hz, 1H), 4.92 - 4.75 (m, 2H), 4.53 - 4.40 (m, 2H), 4.32 - 4.13 (m, 2H), 3.25 (s, 3H), 2.95 (s, 3H), 2.81 - 2.71 (m, 2H), 2.44 - 2.37 (m, 1H), 2.37 - 2.22 (m, 3H), 2.17 - 2.05 (m, 1H), 1.99 - 1.89 (m, 1H), 1.84 (d, *J* = 11.2 Hz, 1H),

1.53 (d, $J$ = 6.2 Hz, 3H), 1.09 (s, 3H), 0.97 (t, $J$ = 7.1 Hz, 3H). $^{19}$F NMR (376 MHz, DMSO-$d_6$) δ -123.76, -131.51, -139.13. The second eluted white solid isomer 2 was Example **231B** (17.1 mg, yield 10%, Rt=11.7 min). LCMS (m/z): 779.3 (M+H). $^1$H NMR (400 MHz, DMSO-$d_6$) δ8.63 - 8.28 (m, 4H), 7.91 (s, 1H), 6.84 - 6.52 (m, 2H), 5.02 (d, $J$ = 17.2 Hz, 1H), 4.94 - 4.76 (m, 2H), 4.58 - 4.41 (m, 2H), 4.26 (d, $J$ = 10.6 Hz, 1H), 4.20 - 4.03 (m, 1H), 3.24 (s, 3H), 2.95 (s, 3H), 2.80 - 2.74 (m, 2H), 2.44 - 2.38 (m, 1H), 2.35 - 2.23 (m, 3H), 2.22 - 2.09 (m, 1H), 1.99 - 1.88 (m, 1H), 1.82 (d, $J$ = 11.2 Hz, 1H), 1.53 (d, $J$ = 6.3 Hz, 3H), 1.09 (s, 3H), 0.97 (t, $J$ = 7.1 Hz, 3H). $^{19}$F NMR (376 MHz, DMSO-$d_6$) δ -123.68, -131.48, -139.11.

**Example 232A**

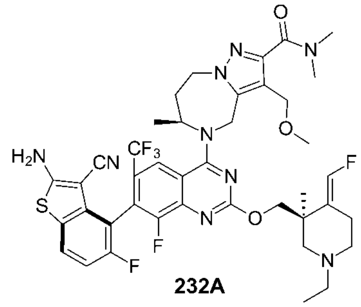

232A

Example **232A** was prepared from intermediate **J-W3A-Cl** according to the method described above. LCMS (m/z): 856.3 (M+H). $^1$H NMR (400 MHz, DMSO-$d_6$) δ 8.04 (s, 1H), 7.95 (s, 2H), 7.84 (dd, $J$ = 8.7, 4.9 Hz, 1H), 7.12 (t, $J$ = 9.1 Hz, 1H), 6.80 (s, 0.5H), 6.58 (s, 0.5H), 5.14 (d, $J$ = 16.6 Hz, 1H), 4.92 - 4.67 (m, 2H), 4.62 - 4.45 (m, 2H), 4.44 - 4.32 (m, 2H), 4.31 - 4.23 (m, 1H), 4.20 - 4.06 (m, 1H), 3.24 (s, 3H), 3.06 (s, 3H), 2.96 (s, 3H), 2.85 - 2.70 (m, 2H), 2.59 - 2.53 (m, 1H), 2.44 - 2.24 (m, 4H), 2.17 - 2.02 (m, 1H), 1.99 - 1.77 (m, 2H), 1.64 (d, $J$ = 6.2 Hz, 3H), 1.08 (s, 3H), 0.95 (t, $J$ = 7.1 Hz, 3H). $^{19}$F NMR (376 MHz, DMSO-$d_6$) δ -59.39, -117.50, -122.29, -139.05.

**Examples 233A and 233B**

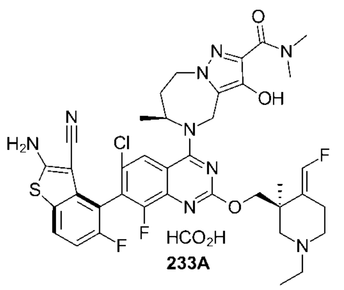

233A

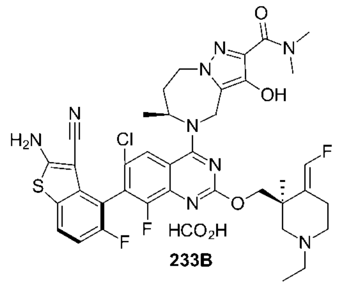

233B

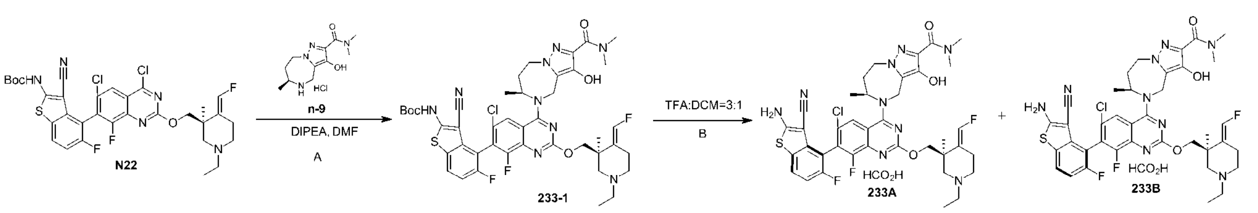


**Step A:** Compound **233-1**

At room temperature, DIPEA (280 mg, 2.17 mmol) was added to a mixture of intermediate **N22** (150 mg, 0.217 mmol), **n-8** (152 mg, 0.433 mmol), and THF/DMF (V/V=1:4, 5 mL), and stirred at 65°C for 4 h. The reaction completion was monitored by LCMS. The reaction mixture was then poured into a saturated $NH_4Cl$ aqueous solution (30 mL) and extracted with EA (30 mL×3). The crude product obtained by organic phase concentration was collected and purified by FCC ($SiO_2$,

EA/PE=0-100%) to obtain a yellow solid compound **233-1** (150 mg, crude product). LCMS (m/z): 894.2 (M+H).

**Step B:** Examples **233A and 233B**

At room temperature, TFA/DCM (V/V=3:1, 20 mL) was added to compound **233-1** (150 mg) and stirred at room temperature for 1 h. The reaction completion was monitored by LCMS. The solution was concentrated to remove acid, diluted with EA (50 mL), neutralized with saturated sodium bicarbonate solution, extracted with EA (50 mL×3), concentrated under reduced pressure, and the crude product was purified by pre-HPLC (C18, ACN/(10 mmol FA/$H_2O$)=30-40%) to obtain the first eluted white powder isomer 1, which was Example **233A** (30 mg, yield 68%, Rt=11.1 min). LCMS (m/z): 794.1 (M+H). $^1$H NMR (400 MHz, DMSO-$d_6$) δ 8.35 (s, 1H), 7.98 (s, 2H), 7.85 (dd, *J* = 8.7, 4.9 Hz, 1H), 7.80 (s, 1H), 7.26 - 7.07 (m, 1H), 6.69 (d, *J* = 86.9 Hz, 1H), 5.01 (d, *J* = 17.1 Hz, 1H), 4.90 - 4.79 (m, 1H), 4.68 (d, *J* = 17.1 Hz, 1H), 4.48 (d, *J* = 10.7 Hz, 1H), 4.42 - 4.34 (m, 1H), 4.29 (d, *J* = 10.6 Hz, 1H), 4.24 - 4.09 (m, 1H), 3.25 (s, 3H), 2.99 (s, 3H), 2.81 - 2.70 (m, 2H), 2.41 - 2.22 (m, 4H), 2.17 - 1.79 (m, 5H), 1.50 (d, *J* = 6.2 Hz, 3H), 1.10 (s, 3H), 0.98 (t, *J* = 7.1 Hz, 3H). $^{19}$F NMR (376 MHz, DMSO-$d_6$) δ -118.73, -120.86, -139.11. The second eluted white solid isomer 2 was Example **233B** (11 mg, yield 25%, Rt=13.8 min). LCMS(m/z): 794.3 (M+H). $^1$H NMR (400 MHz, DMSO-$d_6$) δ 8.39 (s, 1H), 8.02 (s, 2H), 7.89 - 7.77 (m, 2H), 7.13 (t, *J* = 9.1 Hz, 1H), 6.80 (s, 0.5H), 6.58 (s, 0.5H), 4.99 - 4.90 (m, 1H), 4.90 - 4.80 (m, 1H), 4.79 - 4.69 (m, 1H), 4.56 - 4.45 (m, 1H), 4.45 - 4.34 (m, 1H), 4.34 - 4.25 (m, 1H), 4.14 - 3.99 (m, 1H), 3.29 (s, 3H), 2.99 (s, 3H), 2.82 - 2.72 (m, 2H), 2.46 - 2.36 (m, 1H), 2.36 - 2.22 (m, 3H), 2.21 - 2.09 (m, 1H), 2.06 - 1.89 (m, 2H), 1.88 - 1.79 (m, 1H), 1.51 (d, *J* = 6.3 Hz, 3H), 1.10 (s, 3H), 0.98 (t, *J* = 7.1 Hz, 3H). $^{19}$F NMR (376 MHz, DMSO-$d_6$) δ -118.90, -121.00, -139.14.

**Examples 234A and 234B**

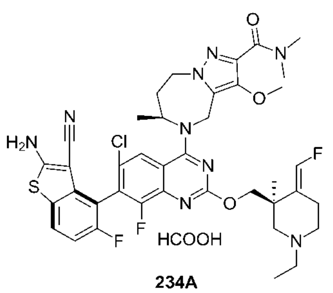

234A

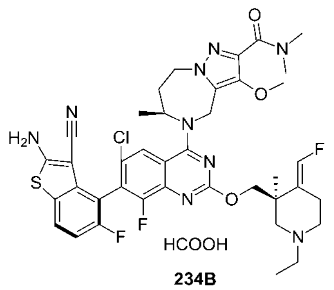

234B

Examples **234A** and **234B** were synthesized according to Examples **233A** and **233B.** The crude product obtained in step B was purified by pre-HPLC (C18, ACN/(10mmol FA/$H_2O$)=30-35%) to obtain the first eluted white powder isomer 1, which was Example **234A** (32mg, yield 72%, Rt=10.8min). LCMS (m/z): 808.2 (M+H). $^1$H NMR (400 MHz, DMSO-$d_6$) δ 8.34 (s, 1H), 7.99 (s, 2H), 7.89 - 7.72 (m, 2H), 7.23 - 7.07 (m, 1H), 6.64 (d, *J* = 86.9 Hz, 1H), 5.02 (d, *J* = 16.9 Hz, 1H), 4.91 - 4.78 (m, 1H), 4.70 (d, *J* = 16.9 Hz, 1H), 4.45 (d, *J* = 10.5 Hz, 1H), 4.36 - 4.22 (m, 2H), 4.16 - 4.03 (m, 1H), 3.86 (s, 3H), 3.02 (s, 3H), 2.97 (s, 3H), 2.79 - 2.65 (m, 2H), 2.43 - 2.23 (m, 4H), 2.18 - 1.88 (m, 3H), 1.83 (d, *J* = 11.2 Hz, 1H), 1.54 (d, *J* = 6.2 Hz, 3H), 1.07 (s, 3H), 0.94 (t, *J* = 7.1 Hz, 3H). $^{19}$F NMR (376 MHz, DMSO-$d_6$) δ -118.64, -120.63, -139.24. The second eluted white powder isomer 2 was Example **234B** (14 mg, yield 31%, Rt=14.8 min). LCMS (m/z): 808.3 (M+H). $^1$H NMR (400 MHz, DMSO-$d_6$) δ 8.40 (s, 1H), 8.03 (s, 2H), 7.91 - 7.75 (m, 2H), 7.20 - 7.04 (m, 1H), 6.65 (d, *J* = 86.9 Hz, 1H), 5.01 - 4.86 (m, 1H), 4.84 - 4.69 (m, 2H), 4.48 (d, *J* = 10.6 Hz, 1H), 4.33 (dd, *J* = 13.7, 8.4 Hz, 2H), 4.04 - 3.90 (m, 1H), 3.83 (s, 3H), 3.01 (s, 3H), 2.96 (s, 3H), 2.80 - 2.68 (m, 2H), 2.47 - 1.78 (m, 8H), 1.55 (d, *J*= 6.2 Hz, 3H), 1.08 (s, 3H), 0.95 (t, *J* = 7.1 Hz, 3H). $^{19}$F NMR (376 MHz, DMSO-$d_6$) δ -118.91, -121.03, -139.28.

**Example 235A**

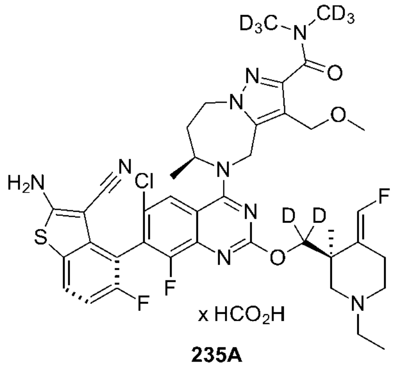

x $HCO_2H$

**235A**

Example **235A** was prepared from intermediate **N19A, n-8-d6,** and **bE-d2** according to the synthesis method described in the above examples. LCMS (m/z): 830.3 (M+H). $^1$H NMR (400 MHz, DMSO-$d_6$) δ 8.17 (s, 0.6H), 7.99 (s, 2H), 7.85 (dd, $J$ = 8.7, 5.0 Hz, 1H), 7.74 (s, 1H), 7.14 (t, $J$ = 9.1 Hz, 1H), 6.80 (s, 0.5H), 6.58 (s, 0.5H), 5.18 - 5.06 (m, 1H), 4.90 - 4.78 (m, 1H), 4.78 - 4.67 (m, 1H), 4.67 - 4.50 (m, 2H), 4.50 - 4.35 (m, 1H), 4.22 - 4.06 (m, 1H), 3.24 (s, 3H), 2.84 - 2.69 (m, 2H), 2.57 - 2.53 (m, 1H), 2.43 - 2.25 (m, 4H), 2.19 - 2.05 (m, 1H), 1.98 - 1.87 (m, 1H), 1.87 - 1.79 (m, 1H), 1.58 (d, J = 6.2 Hz, 3H), 1.08 (s, 3H), 0.95 (t, $J$ = 7.1 Hz, 3H). $^{19}$F NMR (376 MHz, DMSO-$d_6$) δ - 118.68, -120.62, -139.14.

The following compounds of the present invention were prepared by referring to the above-described synthetic schemes or similar variants thereof.

| Example | Structural Formula | Characterization Data |
|---|---|---|
| 236A | 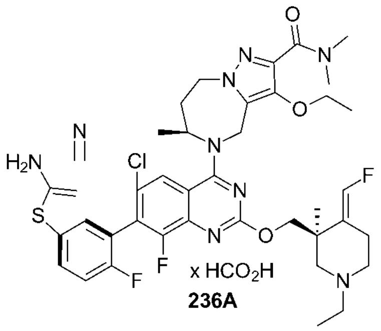 x $HCO_2H$ 236A | LCMS (m/z): 822.3 (M+H). |
| 236B | 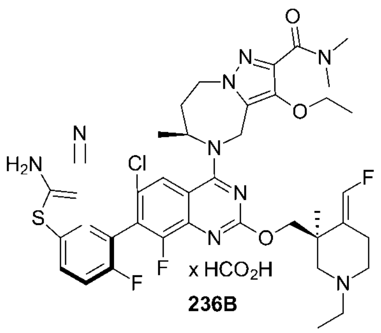 x $HCO_2H$ 236B | LCMS (m/z): 822.3 (M+H). |
| 237A | 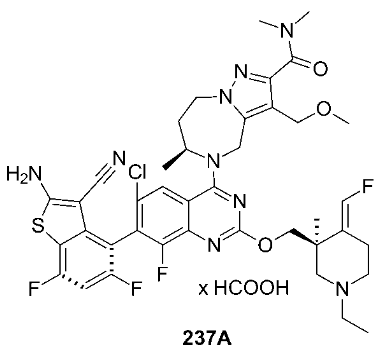 x HCOOH 237A | LCMS(m/z): 840.3 (M+H). $^1$H NMR (400 MHz, DMSO-$d_6$) δ 8.28 (s, 2H), 8.24 (s, 1H), 7.74 (s, 1H), 7.35 - 7.25 (m, 1H), 6.68 (d, $J$ = 86.9 Hz, 1H), 5.18 - 5.06 (m, 1H), 4.87 - 4.77 (m, 1H), 4.77 - 4.67 (m, 1H), 4.63 - 4.51 (m, 2H), 4.46 - 4.34 (m, 2H), 4.28 - 4.22 (m, 1H), 4.18 - 4.09 (m, 1H), 3.23 (s, 3H), 3.06 (s, 3H), 2.95 (s, 3H), 2.81 - 2.71 (m, 2H), 2.56 - 2.51 (m, 1H), 2.39 - 2.23 (m, 4H), 2.16 - 2.05 (m, 1H), 1.97 - 1.86 (m, 1H), 1.86 - 1.78 (m, 1H), 1.58 (d, $J$ = 6.3 Hz, 3H), 1.08 (s, 3H), 0.95 (t, $J$ = 7.1 Hz, 3H). $^{19}$F NMR (376 MHz, DMSO-$d_6$) δ -111.87 - -112.03 (m), -113.57 - -113.73 (m), -120.40, - 139.14. |

(continued)

| Example | Structural Formula | Characterization Data |
|---|---|---|
| 237B | 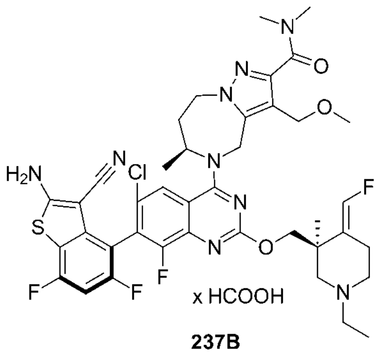 x HCOOH **237B** | LCMS(m/z): 840.3 (M+H). $^1$H NMR (400 MHz, DMSO-$d_6$) δ 8.32 (s, 2H), 8.26 (s, 1H), 7.68 (s, 1H), 7.36 - 7.24 (m, 1H), 6.68 (dd, *J* = 86.9, 1.6 Hz, 1H), 5.05 - 4.93 (m, 1H), 4.86 - 4.72 (m, 2H), 4.54 (s, 2H), 4.50 - 4.40 (m, 2H), 4.34 - 4.25 (m, 1H), 4.17 - 4.01 (m, 1H), 3.23 (s, 3H), 3.05 (s, 3H), 2.95 (s, 3H), 2.82 - 2.72 (m, 2H), 2.56 - 2.51 (m, 1H), 2.45 - 2.22 (m, 4H), 2.21 - 2.09 (m, 1H), 1.96 - 1.87 (m, 1H), 1.87 - 1.78 (m, 1H), 1.55 (d, *J* = 6.2 Hz, 3H), 1.09 (s, 3H), 0.95 (t, *J* = 7.1 Hz, 3H). $^{19}$F NMR (376 MHz, DMSO-$d_6$) δ -111.86 - -112.05 (m), -114.03, - 121.21, -139.15. |
| 238A | 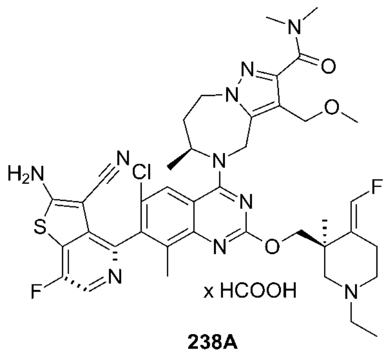 x HCOOH **238A** | LCMS(m/z): 819.3 (M+H). $^1$H NMR (400 MHz, DMSO-$d_6$) δ 8.41 (s, 1H), 8.34 (d, *J* = 6.9 Hz, 2H), 7.67 (s, 1H), 6.83 - 6.56 (m, 1H), 5.05 (d, *J* = 16.9 Hz, 1H), 4.87 - 4.75 (m, 1H), 4.70 (d, *J* = 17.0 Hz, 1H), 4.62 - 4.51 (m, 2H), 4.48 - 4.34 (m, 2H), 4.31 (d, *J* = 10.5 Hz, 1H), 4.19 - 4.03 (m, 1H), 3.22 (s, 3H), 3.06 (s, 3H), 2.96 (s, 3H), 2.74 (d, *J* = 10.9 Hz, 2H), 2.57 - 2.52 (m, 1H), 2.40 - 2.24 (m, 4H), 2.16 (s, 3H), 2.15 - 2.08 (m, 1H), 2.02 - 1.90 (m, 1H), 1.85 (d, *J* = 11.3 Hz, 1H), 1.57 (d, *J* = 6.3 Hz, 3H), 1.10 (s, 3H), 0.95 (t, *J* = 7.1 Hz, 3H). $^{19}$F NMR (376 MHz, DMSO-$d_6$) δ -132.80, -139.27. |
| 238B | 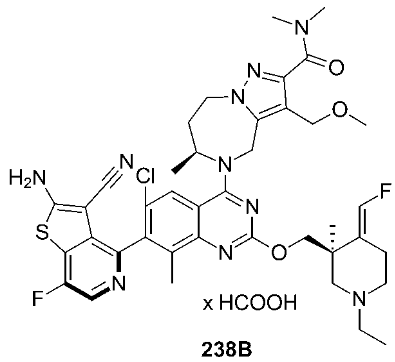 x HCOOH **238B** | LCMS(m/z): 819.3 (M+H). $^1$H NMR (400 MHz, DMSO-$d_6$) δ 8.42 (s, 1H), 8.35 (s, 2H), 7.62 (s, 1H), 6.84 - 6.55 (m, 1H), 4.93 (d, *J* = 17.1 Hz, 1H), 4.83 - 4.69 (m, 2H), 4.55 - 4.40 (m, 4H), 4.34 (d, *J* = 10.6 Hz, 1H), 4.15 - 4.01 (m, 1H), 3.22 (s, 3H), 3.05 (s, 3H), 2.95 (s, 3H), 2.79 - 2.71 (m, 2H), 2.56 - 2.51 (m, 1H), 2.43 - 2.34 (m, 1H), 2.33 - 2.24 (m, 3H), 2.20 - 2.11 (m, 4H), 2.00 - 1.90 (m, 1H), 1.85 (d, *J* = 11.3 Hz, 1H), 1.55 (d, *J* = 6.3 Hz, 3H), 1.10 (s, 3H), 0.95 (t, *J* = 7.1 Hz, 3H). $^{19}$F NMR (376 MHz, DMSO-$d_6$) δ -132.84, -139.25. |
| 239A | 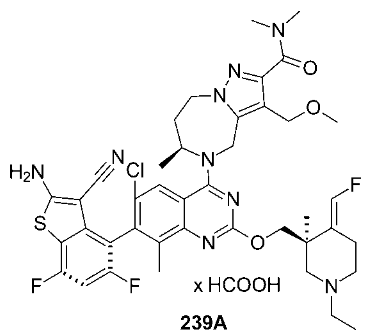 x HCOOH **239A** | LCMS(m/z): 836.3 (M+H). |

(continued)

| Example | Structural Formula | Characterization Data |
|---|---|---|
| 239B | 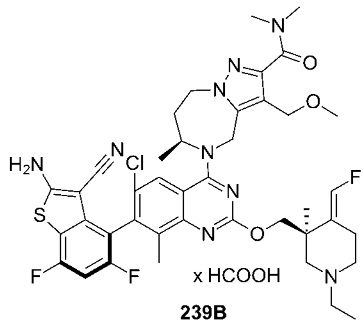 x HCOOH 239B | LCMS(m/z): 836.3 (M+H). |
| 240A | 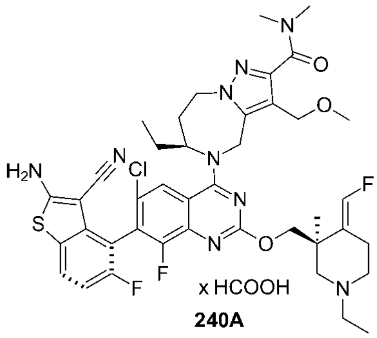 x HCOOH 240A | LCMS(m/z): 836.3 (M+H). $^1$H NMR (400 MHz, DMSO-$d_6$) δ 7.98 (s, 2H),7.91 (s, 1H), 7.85 (dd, *J* = 8.8,5.2 Hz, 1H),7.73 (s, 1H), 7.14 (t, *J* =9.2Hz, 1H), 6.80 (s, 0.5H), 6.58 (s, 0.5H), 5.22(d, *J* =16.8 Hz, 1H), 4.70-4.37 (m,6H), 4.25(d, *J* =10.4 Hz, 1H), 4.17-4.10 (m, 1H), 3.23(s, 3H), 3.06 (s, 3H), 2.95 (s, 3H), 2.80-2.73 (m, 2H), 2.55-2.48 (m, 2H), 2.33-2.26 (m, 3H), 2.14-2.00(m, 3H), 1.99-1.88(m, 1H), 1.82(d, *J* =11.2 Hz, 1H),1.10-1.06(m, 6H), 0.95 (t, *J* =7.2 Hz, 3H). $^{19}$F NMR (376 MHz, DMSO-$d_6$) δ - 118.66, -120.60, -139.17 |
| 240B | 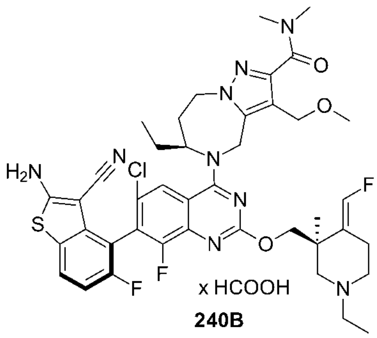 x HCOOH 240B | LCMS(m/z): 836.3 (M+H). |
| 241A | 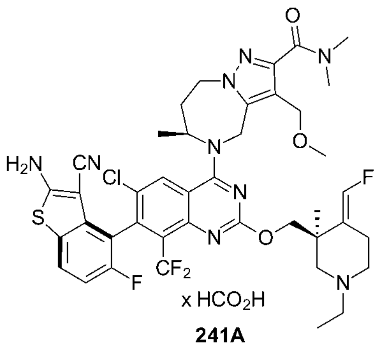 x $HCO_2H$ 241A | LCMS (m/z): 854.3 (M+H). $^1$H NMR (400 MHz, DMSO-$d_6$) δ 8.01 (s, 1H), 7.82 (d, *J* = 4.8 Hz, 2H), 7.81 (t, *J* = 56.0 Hz, 1H), 7.77 (dd, *J* = 8.7, 4.9 Hz, 1H), 7.10 - 7.01 (m, 1H), 6.71 (d, *J* = 86.8 Hz, 1H), 5.17 - 5.05 (m, 1H), 4.90 - 4.77 (m, 1H), 4.77 - 4.69 (m, 1H), 4.65 - 4.49 (m, 2H), 4.49 - 4.27 (m, 3H), 4.22 - 4.08 (m, 1H), 3.31 (s, 2H), 3.22 (s, 3H), 3.06 (s, 3H), 2.95 (s, 3H), 2.76 - 2.66 (m, 2H), 2.37 - 2.15 (m, 4H), 2.04 - 1.92 (m, 1H), 1.91 - 1.83 (m, 1H), 1.58 (d, *J* = 6.2 Hz, 3H), 1.09 (s, 3H), 0.94 (t, *J*= 7.1 Hz, 3H). $^{19}$F NMR (376 MHz, DMSO-$d_6$) δ - 113.35 - -114.49, -117.52 - -118.48, -139.27. |

(continued)

| Example | Structural Formula | Characterization Data |
|---|---|---|
| 241B | 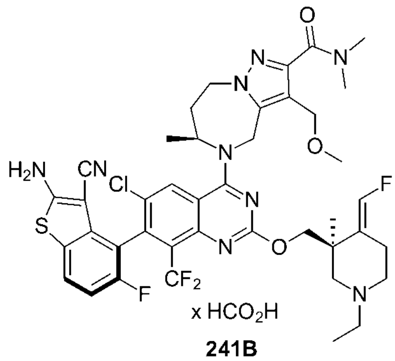 x $HCO_2H$ **241B** | LCMS (m/z): 854.3 (M+H). |
| 242A | 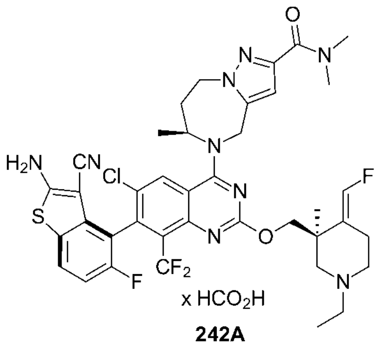 x $HCO_2H$ **242A** | LCMS (m/z): 810.3 (M+H). |
| 242B | 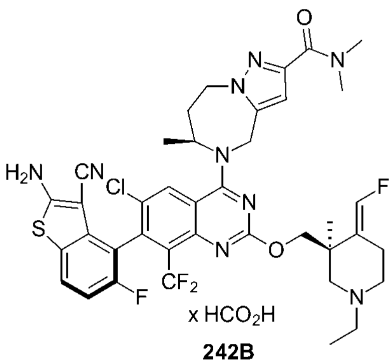 x $HCO_2H$ **242B** | LCMS (m/z): 810.3 (M+H). |

**Example 243A**

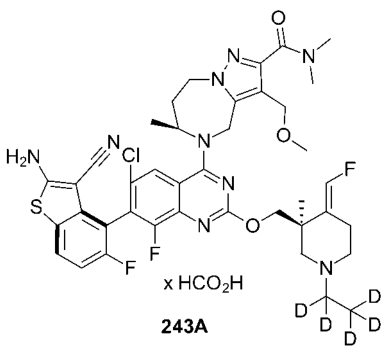

x $HCO_2H$

**243A**

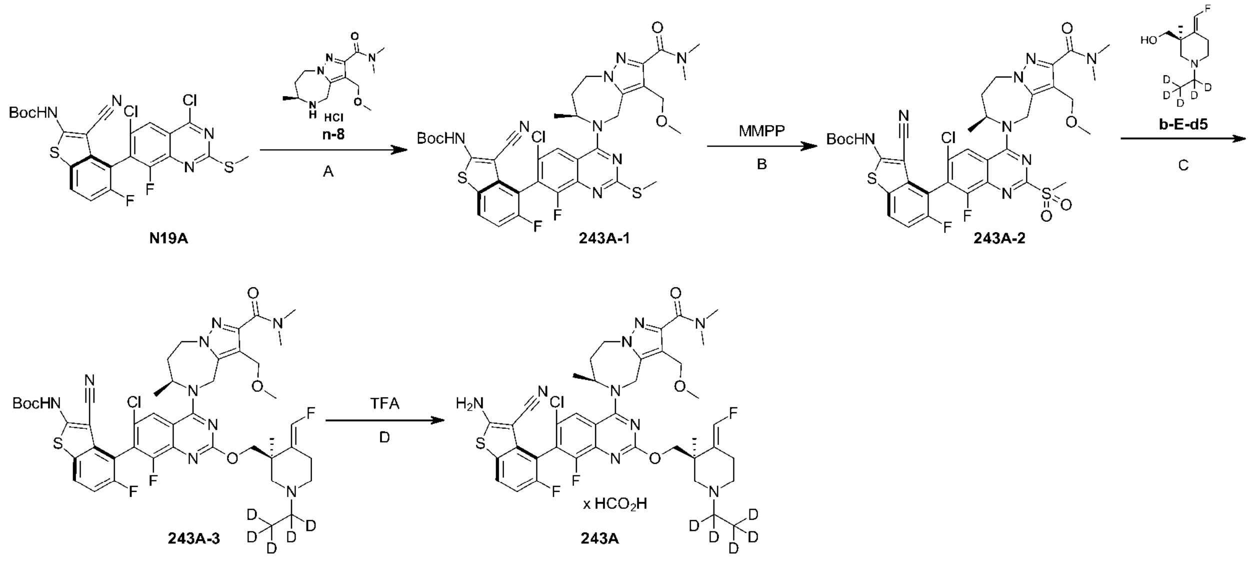


**Step A:** Compound **243A-1**

At room temperature, intermediate **N19A** (2.00 g, 3.61 mmol), **n-8** (1.31 g, 4.34 mmol), and DIEA (4.67 g, 36.1 mmol) were added sequentially to DMF (40 mL). The resulting reaction solution was reacted overnight at 60°C. The reaction completion was monitored by LCMS. The reaction solution was poured into 5% LiCl aqueous solution (200 mL), extracted with EtOAc (150 mL×3), and the organic phase was collected, washed with saturated brine (50 mL), dried over anhydrous $Na_2SO_4$, filtered, and concentrated. The organic phase was purified by FCC ($SiO_2$, THF/PE=0-100%) to obtain a brown solid compound **243A-1** (2.32 g, yield 82%). LCMS (m/z): 783.4 (M+H). (Page:0202-HFL-112)

**Step B:** Compound **243A-2**

At room temperature, magnesium monoperoxyphthalate hexahydrate (9.15 g, 80wt%, 14.8 mmol) was added to a mixed solution of compound **243A-1** (2.32 g, 2.96 mmol) in toluene (50 mL) and water (50 mL) and stirred overnight at room temperature. After the reaction completion was monitored by LCMS, the reaction solution was extracted with EtOAc (50 mL×3), the organic phase was collected, washed with saturated saline solution, dried over anhydrous $Na_2SO_4$, filtered, and the organic phase was concentrated and purified by FCC ($SiO_2$, THF/PE=0-100%) to give a white solid compound **243A-2** (2.05 g, yield 85%). LCMS (m/z): 815.3 (M+H).

**Step C:** Compound **243A-3**

Under dry ice and ethanol bath conditions, *t*-BuONa (2.9 mL, 2 M in THF, 5.9 mmol) was added to a mixed solution of compound **243A-2** (1.20 g, 1.47 mmol), intermediate **bE-d5** (425 mg, 2.21 mmol), and THF (50 mL), and stirred for 0.5 h under dry ice and ethanol bath conditions. The mixture was warmed to room temperature and stirred for 1.0 h. The reaction completion was monitored by LCMS. The reaction solution was quenched in a saturated solution of $NH_4Cl$ (80 mL), extracted with EtOAc (50 mL×3), and the organic phase was collected. The mixture was washed with saturated brine (100 mL), dried over anhydrous $Na_2SO_4$, filtered, and the organic phase was concentrated and purified by FCC ($SiO_2$, THF/PE=0-70%) to obtain a yellow solid compound **243A-3** (1.1 g, yield 81%). LCMS (m/z): 927.3 (M+H).

**Step D:** Example **243A**

At room temperature, Compound **243A-3** (1.10 g, 1.19 mmol) was added to a mixture of DCM/TFA (V/V=1:1, 20 mL) and stirred for 1 h. The reaction completion was monitored by LCMS. After concentration, the mixture was quenched with saturated sodium bicarbonate aqueous solution (80 mL), then extracted with EtOAc (50 mL×3). The organic phase was collected, washed with brine (100 mL), dried over anhydrous $Na_2SO_4$, filtered, and concentrated to obtain a yellow solid (1.0 g, crude product). The crude product (250 mg) was purified by pre-HPLC (C18, ACN/(10 mmol FA/$H_2O$) =28-35%, Rt=9.4 min) to obtain a white solid, Example **243A** (113.6 mg, yield 45%). LCMS (m/z): 827.3 (M+H). $^1H$ NMR (400 MHz, DMSO-$d_6$) δ 8.16 (s, 0.66H), 7.99 (s, 2H), 7.89 - 7.82 (m, 1H), 7.74 (s, 1H), 7.20 - 7.10 (m, 1H), 6.83 - 6.78 (m, 0.5H), 6.61 - 6.55 (m, 0.5H), 5.13 (d, *J* = 16.9 Hz, 1H), 4.91 - 4.78 (m, 1H), 4.72 (d, *J* = 17.1 Hz, 1H), 4.66 - 4.51 (m, 2H), 4.49 - 4.33 (m, 2H), 4.29 - 4.21 (m, 1H), 4.21 - 4.07 (m, 1H), 3.26 - 3.23 (m, 3H), 3.07 (s, 3H), 2.96 (s, 3H), 2.82 - 2.71 (m, 2H), 2.58 -

2.53 (m, 1H), 2.41 - 2.31 (m, 2H), 2.18 - 2.05 (m, 1H), 1.97 - 1.88 (m, 1H), 1.83 (d, J = 11.1 Hz, 1H), 1.59 (s, 3H), 1.08 (s, 3H). $^{19}$F NMR (376 MHz, DMSO-$d_6$) δ -118.68, -120.62, - 139.17.

**Example 243B**

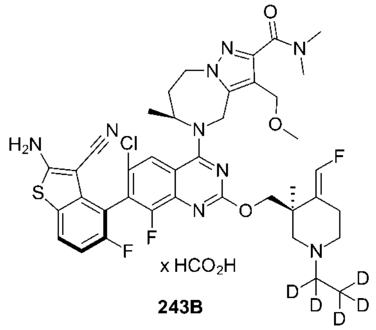

x HCO$_2$H

**243B**

Example 243B follows the above synthesis scheme, using intermediate N19B instead of N19A for preparation. LCMS (m/z): 827.3 (M+H).

The following compounds of the present invention were prepared by referring to the above-described synthetic scheme or similar variants thereof.

| Example | Structural Formula | Characterization Data |
|---|---|---|
| 244A | 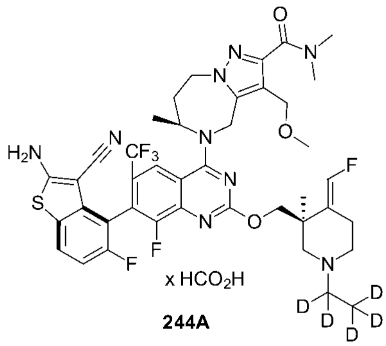 x HCO$_2$H **244A** | LCMS (m/z): 861.3 (M+H). $^1$H NMR (400 MHz, DMSO-$d_6$) δ 8.05 (s, 1H), 7.96 (s, 2H), 7.89 - 7.79 (m, 1H), 7.19 - 7.07 (m, 1H), 6.80 (s, 0.5H), 6.58 (s, 0.5H), 5.24 - 5.07 (m, 1H), 4.90 - 4.67 (m, 2H), 4.62 - 4.47 (m, 2H), 4.46 - 4.32 (m, 2H), 4.31 - 4.21 (m, 1H), 4.20 - 4.04 (m, 1H), 3.24 (s, 3H), 3.07 (s, 3H), 2.96 (s, 3H), 2.85 - 2.71 (m, 2H), 2.60 - 2.53 (m, 1H), 2.44 - 2.30 (m, 2H), 2.18 - 2.03 (m, 1H), 1.97 - 1.87 (m, 1H), 1.82 (d, *J* = 11.3 Hz, 1H), 1.64 (d, *J* = 6.1 Hz, 3H), 1.08 (s, 3H). $^{19}$F NMR (376 MHz, DMSO-$d_6$) δ -59.40, -117.49, -122.29, -139.05. |
| 244B | 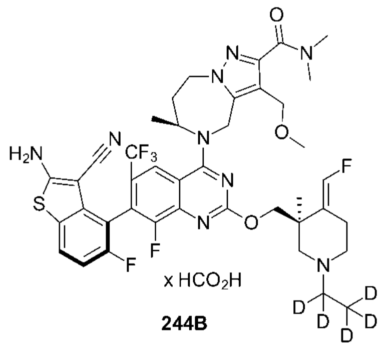 x HCO$_2$H **244B** | LCMS (m/z): 861.3 (M+H). |

(continued)

| Example | Structural Formula | Characterization Data |
|---|---|---|
| 245A | 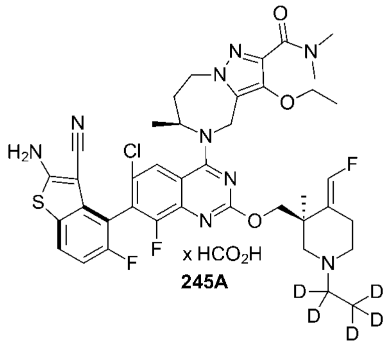 x $HCO_2H$ **245A** | LCMS (m/z): 827.3 (M+H). |
| 245B | 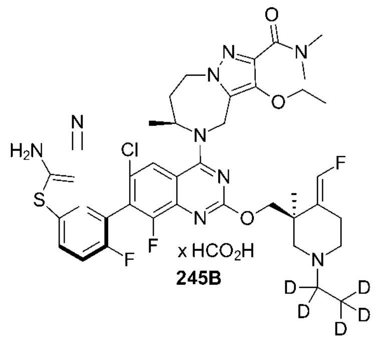 x $HCO_2H$ **245B** | LCMS (m/z): 827.3 (M+H). |
| 246A | 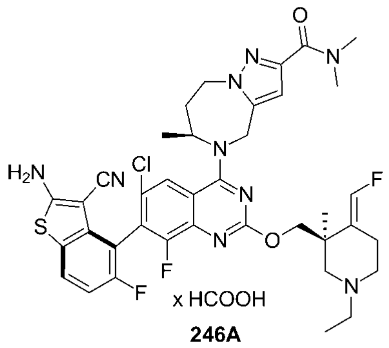 x HCOOH **246A** | LCMS (m/z): 778.3 (M+H). $^1$H NMR (400 MHz, DMSO-$d_6$) δ 8.14 (s, 0.36H), 7.99 (s, 2H), 7.95 - 7.89 (m, 1H), 7.89 - 7.82 (m, 1H), 7.15 (t, *J* = 9.1 Hz, 1H), 6.86 - 6.77 (m, 0.5H), 6.69 (s, 1H), 6.63 - 6.56 (m, 0.5H), 5.16 (d, *J*= 17.2 Hz, 1H), 4.95 - 4.74 (m, 2H), 4.57 - 4.38 (m, 2H), 4.35 - 4.13 (m, 2H), 3.25 (s, 3H), 2.96 (s, 3H), 2.85 - 2.71 (m, 2H), 2.47 - 2.25 (m, 4H), 2.22 - 2.07 (m, 1H), 2.06 - 1.95 (m, 1H), 1.94 - 1.80 (m, 1H), 1.53 (d, *J* = 6.3 Hz, 3H), 1.10 (s, 3H), 0.99 (t, *J* = 7.1 Hz, 3H). $^{19}$F NMR (376 MHz, DMSO-$d_6$) δ -118.67, -120.84, -138.95. |
| 246B | 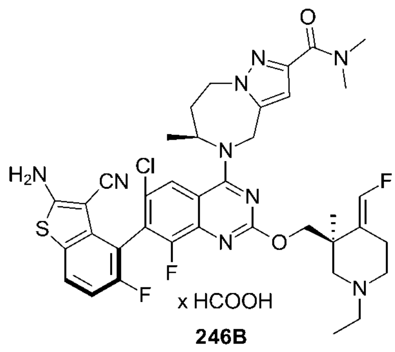 x HCOOH **246B** | LCMS(m/z):778.3 (M+H). $^1$H NMR (400 MHz, DMSO-$d_6$) δ 8.24 (s, 0.3H), 8.09 (s, 2H), 7.98 (s, 1H), 7.94 - 7.86 (m, 1H), 7.20 (t, *J* = 9.1 Hz, 1H), 6.89 - 6.83 (m, 0.5H), 6.71 (s, 1H), 6.66 - 6.60 (m, 0.5H), 5.11 - 4.93 (m, 1H), 4.92 - 4.84 (m, 1H), 4.65 - 4.45 (m, 2H), 4.39 - 4.28 (m, 1H), 4.24 - 4.07 (m, 1H), 3.30 (s, 3H), 3.01 (s, 3H), 2.90 - 2.77 (m, 2H), 2.55 - 2.29 (m, 5H), 2.27 - 2.15 (m, 1H), 2.09 - 1.95 (m, 1H), 1.89 (d, *J* = 11.2 Hz, 1H), 1.59 (d, *J* = 6.3 Hz, 3H), 1.15 (s, 3H), 1.03 (t, *J* = 7.1 Hz, 3H). $^{19}$F NMR (376 MHz, DMSO-$d_6$) δ -118.83, -121.13, -139.12. |

(continued)

| Example | Structural Formula | Characterization Data |
|---|---|---|
| 247A | 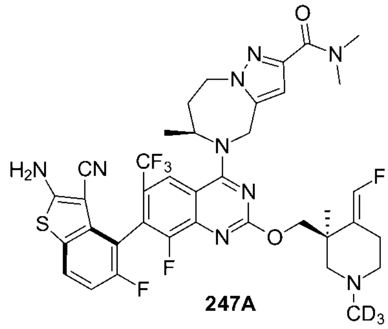 247A | LCMS (m/z): 801.3 (M+H). $^{1}$H NMR (400 MHz, DMSO-$d_6$) δ 8.16 (s, 1H), 7.95 (s, 2H), 7.89 - 7.74 (m, 1H), 7.21 - 7.06 (m, 1H), 6.88 - 6.51 (m, 2H), 5.28 - 5.11 (m, 1H), 4.96 - 4.76 (m, 2H), 4.58 - 4.40 (m, 2H), 4.36 - 4.11 (m, 2H), 3.25 (s, 3H), 2.96 (s, 3H), 2.76 - 2.61 (m, 2H), 2.48 - 2.24 (m, 3H), 2.20 - 2.05 (m, 1H), 1.99 - 1.76 (m, 2H), 1.54 (d, *J* = 6.1 Hz, 3H), 1.09 (s, 3H). $^{19}$F NMR (376 MHz, Chloroform-d) δ -54.22, -112.63, - 117.62, -133.95. |
| 247B | 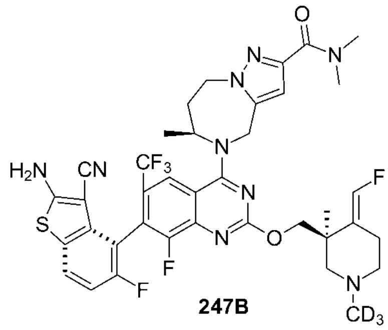 247B | LCMS (m/z): 801.3 (M+H). |
| 248A | 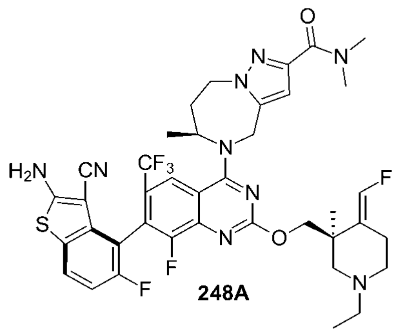 248A | LCMS (m/z): 812.3 (M+H). $^{1}$H NMR (400 MHz, DMSO-$d_6$) δ 8.15 (s, 1H), 7.94 (s, 2H), 7.84 (dd, *J* = 8.7, 4.9 Hz, 1H), 7.12 (t, *J* = 9.1 Hz, 1H), 6.82 (s, 0.5H), 6.67 (s, 1H), 6.60 (s, 0.5H), 5.20 (d, *J* = 17.3 Hz, 1H), 4.94 - 4.77 (m, 1H), 4.57 - 4.41 (m, 2H), 4.37 - 4.17 (m, 2H), 3.25 (s, 3H), 2.96 (s, 3H), 2.84 - 2.69 (m, 2H), 2.47 - 2.22 (m, 4H), 2.18 - 2.07 (m, 1H), 2.05 - 1.89 (m, 2H), 1.84 (d, *J* = 11.3 Hz, 1H), 1.53 (d, *J* = 6.2 Hz, 3H), 1.10 (s, 3H), 0.98 (t, *J* = 7.1 Hz, 3H). $^{19}$F NMR (376 MHz, DMSO-$d_6$) δ -58.94, -117.43, -122.33, - 139.03. |
| 248B | 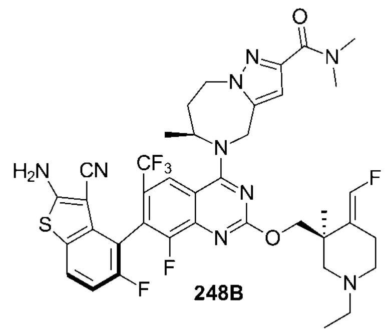 248B | LCMS (m/z): 812.3 (M+H). |

(continued)

| Example | Structural Formula | Characterization Data |
|---|---|---|
| 251A | 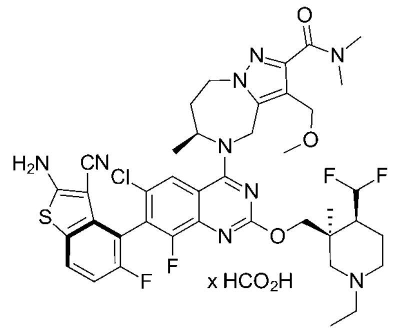 | LCMS(m/z): 842.3 (M+H). $^1$H NMR (400 MHz, DMSO-$d_6$) δ 8.33 (s, 0.63H), 7.98 (s, 2H), 7.85 (dd, $J$ = 8.7, 4.9 Hz, 1H), 7.73 (s, 1H), 7.14 (t, $J$ = 9.1 Hz, 1H), 6.45 - 6.09 (m, 1H), 5.19 - 5.07 (m, 1H), 4.91-4.78 (m, 1H), 4.78 -4.67 (m, 1H), 4.66 - 4.52 (m, 2H), 4.49 - 4.38 (m, 2H), 4.36 - 4.28 (m, 1H), 4.26 - 4.11 (m, 1H), 3.23 (s, 3H), 3.07 (s, 3H), 2.96 (s, 3H), 2.93 - 2.82 (m, 2H), 2.45 - 2.28 (m, 2H), 2.28 - 2.17 (m, 2H), 1.92 - 1.59 (m, 5H), 1.56 (d, $J$ = 6.3 Hz, 3H), 1.11 (s, 3H), 0.87 (t, $J$ = 7.1 Hz, 3H). $^{19}$F NMR (376 MHz, DMSO-$d_6$) δ -115.62, -118.72, -118.92, -120.52. |
| 252 | 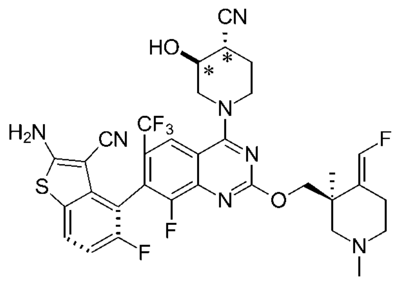 | LCMS(m/z): 702.2 (M+H). |
| 253 | 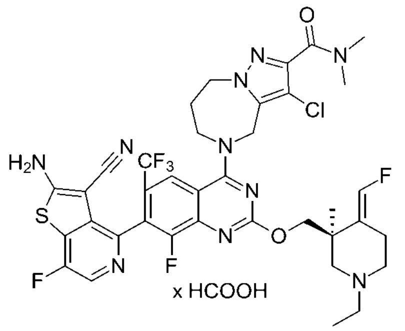 | LCMS(m/z): 833.2 (M+H). $^1$H NMR (400 MHz, DMSO-$d_6$) δ 8.48 - 8.41 (m, 3H), 8.39 (s, 1.5H), 8.08 (s, 1H), 6.82 - 6.75 (m, 0.5H), 6.60 - 6.53 (m, 0.5H), 5.17 - 4.95 (m, 1H), 4.60 - 4.36 (m, 3H), 4.36 - 4.13 (m, 3H), 3.01 (s, 3H), 2.97 (s, 3H), 2.81 - 2.74 (m, 2H), 2.58 - 2.52 (m, 1H), 2.45 - 2.36 (m, 2H), 2.34 - 2.26 (m, 3H), 2.20 - 2.07 (m, 1H), 1.97 - 1.87 (m, 1H), 1.84 (d, $J$ = 11.3 Hz, 1H), 1.09 (d, $J$ = 4.6 Hz, 3H), 0.96 (t, $J$ = 7.1, 3H). $^{19}$F NMR (376 MHz, DMSO-$d_6$) δ - 57.28, -73.40, -131.68, -138.65. |
| 284A | 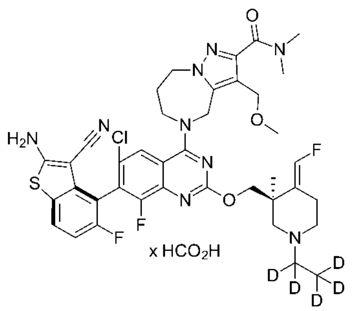 | LCMS (m/z): 813.3 (M+H). |
| 285A | 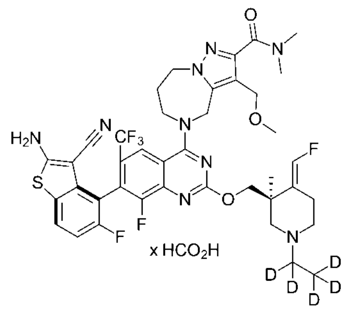 | LCMS (m/z): 847.3 (M+H). |

(continued)

| Example | Structural Formula | Characterization Data |
|---|---|---|
| 286A | 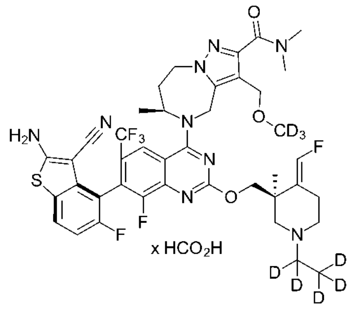 | LCMS (m/z): 864.3 (M+H). |
| 287A | 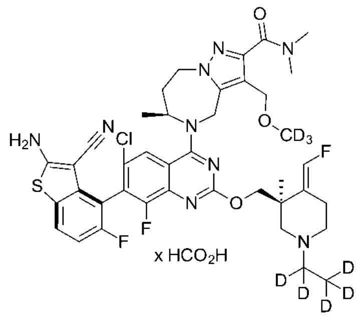 | LCMS (m/z): 830.3 (M+H). |
| 288A | 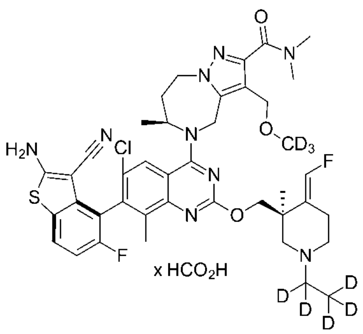 | LCMS (m/z): 826.3 (M+H). |

**Examples 249A and 249B**

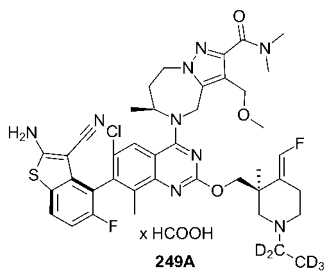

249A

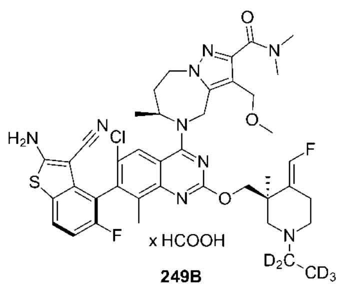

249B

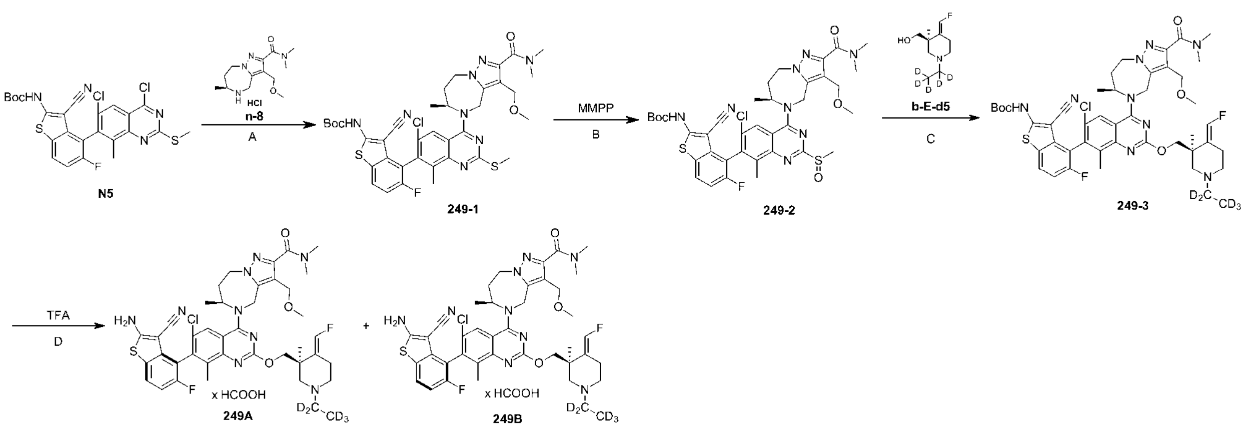

N5 249-1 249-2 249-3
249A + 249B

**Step A:** Compound **249-1**

At room temperature, intermediate **N5** (400 mg, 0.73 mmol), **n-8** (265 mg, 0.88 mmol), and DIEA (753 mg, 5.84 mmol) were added sequentially to DMF (6 mL). The resulting reaction solution was reacted at 60°C for 16 h. The reaction completion was monitored by LCMS and TLC. The reaction solution was poured into water (30 mL), extracted with EA (30 mL×3). The organic phase was collected, washed with saturated brine (30 mL), and dried over anhydrous $Na_2SO_4$. After filtration, the organic phase was concentrated and purified by FCC ($SiO_2$, EA/PE=0-63%) to obtain a yellow solid compound **249-1** (405 mg, yield 71%). LCMS (m/z): 779.2 (M+H).

**Step B:** Compound **249-2**

At room temperature, magnesium monoperoxyphthalate hexahydrate (1.16 mg, 2.04 mmol) was added to a mixed solution of compound **249-1** (405 mg, 0.51 mmol) in DCM (10 mL) and water (10 mL), and stirred for 1 h at room temperature. After the reaction completion was monitored by LCMS, the reaction solution was quenched in aqueous solution (30 mL), and extracted with DCM (30 mL×3). The organic phase was collected, washed with saturated saline solution, and dried over anhydrous $Na_2SO_4$. After filtration, the solution was concentrated to obtain a yellow solid compound **249-2** (410 mg, crude product), which was used directly in the next step. LCMS (m/z): 795.3 (M+H).

**Step** C: Compound **249-3**

Under dry ice and ethanol bath conditions, tBuONa (0.76 mL, 2 M in THF, 1.51 mmol) was added to a solution of compound **249-2** (300 mg, 0.378 mmol), intermediate **bE-d5** (109 mg, 0.567 mmol), and THF (8 mL). The resulting reaction solution was allowed to react for 1 h at room temperature. After reaction completion was monitored by TLC, the reaction solution was poured into a semi-saturated $NH_4Cl$ aqueous solution (30 mL) and extracted with EA (30 mL×3). The organic phase was collected, washed with saturated saline solution, and dried over anhydrous $Na_2SO_4$. After filtration, the organic phase was concentrated and purified by FCC ($SiO_2$, EA/PE=0-57%) to obtain a yellow solid compound **249-3** (258 mg, two-step yield 54%), which was used directly in the next step. LCMS (m/z): 923.4 (M+H).

**Step D:** Examples **249A** and **249B**

At room temperature, Compound **249-3** (258 mg) was added to a mixed solution of TFA (3 mL) and DCM (3 mL). The resulting reaction solution was allowed to react for another 1 h at room temperature. After the reaction completion was monitored by LCMS, the solution was concentrated and saturated sodium bicarbonate aqueous solution (30 mL) was added, followed by extraction with EA (30 mL×2). The extract was collected, washed with brine (30 mL), and dried over anhydrous $Na_2SO_4$. After filtration, the organic phase was concentrated and purified by pre-HPLC (C18, ACN/(10 mmol FA/$H_2O$)=30-35%) to obtain the first eluted white solid isomer 1, which was Example **249A** (83.3 mg, $Rt_1$=10.4 min, yield 35%). LCMS (m/z): 823.3 (M+H). $^1$H NMR (400 MHz, DMSO-*d*$_6$) δ 8.36 (s, 1H), 7.87 (s, 2H), 7.79 (dd, *J* = 8.7, 5.0 Hz, 1H), 7.73 (s, 1H), 7.16 - 7.05 (m, 1H), 6.84 - 6.53 (m, 1H), 5.08 (d, *J* = 16.9 Hz, 1H), 4.85 - 4.74 (m, 1H), 4.68 (d, *J= 16.9* Hz, 1H), 4.61 (d, *J* = 11.8 Hz, 1H), 4.56 - 4.49 (m, 1H), 4.49 - 4.40 (m, 1H), 4.39 - 4.34 (m, 1H), 4.31 (d, *J*= 10.5 Hz, 1H), 4.17 - 4.04 (m, 1H), 3.23 (s, 3H), 3.06 (s, 3H), 2.96 (s, 3H), 2.78 - 2.69 (m, 2H), 2.56 - 2.51 (m, 1H), 2.41 - 2.26 (m, 2H), 2.23 (s, 3H), 2.18 - 2.08 (m, 1H), 2.01 - 1.91 (m, 1H), 1.89 - 1.80 (m, 1H), 1.58 (d, *J* = 6.3 Hz, 3H), 1.09 (s, 3H). $^{19}$F NMR (376 MHz, DMSO-*d*$_6$) δ -119.52, -139.30. The second eluted white solid isomer 2 was Example **249B.** (39.8 mg, $Rt_2$=13.2 min, yield 16%). LCMS(m/z): 823.3 (M+H). $^1$H NMR (400 MHz, DMSO-*d*$_6$) δ 8.36 (s, 1H), 7.90 (s, 2H), 7.79 (dd, *J* = 8.7, 4.9 Hz, 1H), 7.65 (s,

1H), 7.17 - 7.07 (m, 1H), 6.84 - 6.57 (m, 1H), 5.02 - 4.87 (m, 1H), 4.83 - 4.68 (m, 2H), 4.58 - 4.30 (m, 5H), 4.04 (t, *J* = 12.5 Hz, 1H), 3.22 (s, 3H), 3.05 (s, 3H), 2.95 (s, 3H), 2.78 - 2.70 (m, 2H), 2.58 - 2.51 (m, 1H), 2.42 - 2.32 (m, 1H), 2.31 - 2.24 (m, 1H), 2.23 (s, 3H), 2.20 - 2.08 (m, 1H), 2.01 - 1.90 (m, 1H), 1.86 (d, *J* = 11.3 Hz, 1H), 1.56 (d, *J* = 6.3 Hz, 3H), 1.10 (s, 3H). $^{19}$F NMR (376 MHz, DMSO-$d_6$) δ -120.08, -139.32.

The following compounds of the present invention were prepared by referring to the synthetic schemes of the above examples or similar variants. It should be noted that the final diastereomeric mixture prepared from the axially chiral racemic intermediate can be separated by conventional preparative HPLC or by SFC.

| Example | Structural Formula | Characterization Data |
|---|---|---|
| 250A | 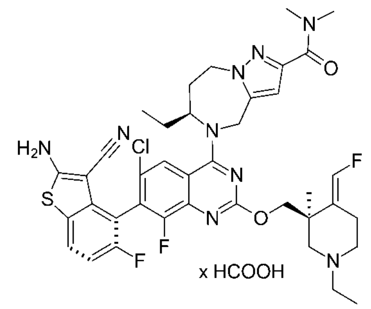 | LCMS (m/z): 792.3 (M+H). $^1$H NMR (400 MHz, DMSO-$d_6$) δ 8.14 (s, 0.74H), 7.97 (s, 2H), 7.91 (s, 1H), 7.85 (dd, *J* = 8.4,4.8 Hz, 1H), 7.14 (t, *J* =9.2Hz, 1H), 6.83 (s, 0.5H), 6.66 (s, 1H), 6.61 (s, 0.5H), 5.23(d, *J* =17.2 Hz, 1H), 4.78 (d, *J* =17.2 Hz, 1H), 4.71-4.66 (m,1H), 4.54-4.48 (m, 2H), 4.28-4.17 (m, 2H), 3.24 (s, 3H), 2.95 (s, 3H), 2.82-2.77 (m, 2H), 2.59-2.48 (m, 2H), 2.36-2.23 (m, 3H), 2.16-1.92 (m, 5H), 1.10-1.06 (m, 6H), 0.99 (t, *J* =7.2 Hz, 3H). $^{19}$F NMR (376 MHz, DMSO-$d_6$) δ -118.69, -120.86, - 139.16. |
| 250B | 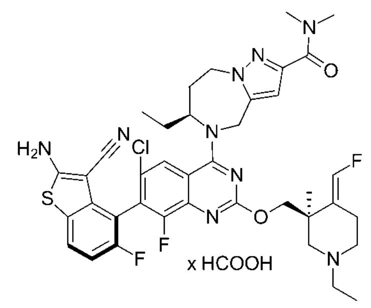 | LCMS (m/z): 792.3 (M+H). $^1$H NMR (400 MHz, DMSO-$d_6$) δ 8.30 (s, 0.61H), 8.01 (s, 2H), 7.92 (s, 1H), 7.85 (dd, *J* = 8.8, 5.2 Hz, 1H), 7.14 (t, *J* =9.2 Hz, 1H), 6.80 (s, 0.5H), 6.64 (s, 1H), 6.59 (s, 0.5H), 5.09 (d, *J* = 17.2 Hz, 1H), 4.85 (d, *J* =17.2 Hz, 1H), 4.71-4.60 (m,1H), 4.55-4.48 (m, 2H), 4.25 (d, *J* =10.4 Hz, 1H), 4.12-4.05 (m, 1H), 3.23 (s, 3H), 2.95 (s, 3H), 2.81-2.74 (m, 2H), 2.56-2.48 (m, 2H), 2.34-2.31 (m, 3H), 2.14-2.04 (m, 2H), 1.95-1.88 (m, 2H), 1.82 (d, *J* =11.2 Hz, 1H), 1.09-1.05 (m, 6H), 0.97 (t, *J* =7.2 Hz, 3H). $^{19}$F NMR (376 MHz, DMSO-$d_6$) δ - 118.23, -121.0, -139.13. |
| 254A | 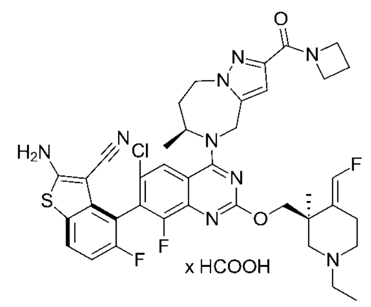 | LCMS (m/z): 790.2 (M+H). $^1$H NMR (400 MHz, DMSO-$d_6$) δ 8.42 (s, 1.2H), 7.99 (s, 2H), 7.89 (s, 1H), 7.85 (dd, *J* = 8.8,5.2 Hz, 1H), 7.14 (t, J=9.2Hz, 1H), 6.82 (s, 0.5H), 6.75(s, 1H), 6.60(s, 0.5H), 5.16(d, *J* = 17.2 Hz, 1H), 4.87-4.76 (m, 2H), 4.45 (t, *J* =8.0Hz, 4H), 4.29-4.19 (m, 2H), 3.99 (t, *J* = 7.6Hz, 2H), 2.79-2.71 (m, 2H), 2.43-2.10 (m, 8H), 1.97-1.92(m, 1H), 1.84 (d, *J* = 11.2 Hz, 1H), 1.51(d, *J=6.0* Hz, 3H), 1.09 (s, 3H), 0.97 (t, *J* =7.2 Hz, 3H). $^{19}$F NMR (376 MHz, DMSO-$d_6$) δ -118.67, -120.83, - 139.10. |
| 254B | 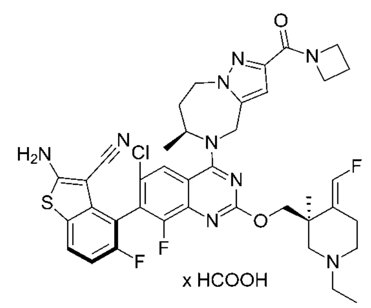 | LCMS (m/z): 790.2 (M+H). $^1$H NMR (400 MHz, DMSO-$d_6$) δ 8.40 (s, 1.8 H), 8.02(s, 2H), 7.89 (s, 1H), 7.85 (dd, *J* = 8.8, 4.8 Hz, 1H), 7.14 (t, J=9.2Hz, 1H), 6.81 (s, 0.5H), 6.72 (s, 1H), 6.59 (s, 0.5H), 5.00 (d, *J* =17.2 Hz, 1H), 4.88-4.78 (m,2H), 4.52-4.42 (m, 4H), 4.27 (d, *J* =10.8 Hz, 1H), 4.14-4.07 (m, 1H), 3.99 (t, *J* =7.6Hz, 2H), 2.81-2.73 (m, 2H), 2.43-2.13 (m, 8H), 1.96-1.90 (m, 1H), 1.83 (d, *J* = 11.2 Hz, 1H), 1.51 (d, *J* =6.0 Hz, 3H), 1.09 (s, 3H), 0.97 (t, *J* =7.2 Hz, 3H). $^{19}$F NMR (376 MHz, DMSO-$d_6$) δ -118.83, -121.11, -139.12. |

(continued)

| Example | Structural Formula | Characterization Data |
|---|---|---|
| 255A | 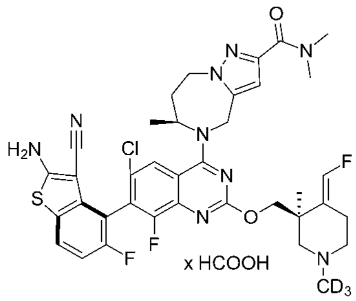 | LCMS (m/z): 767.3 (M+H). $^1$H NMR (400 MHz, DMSO-$d_6$) δ 7.98 (s, 2H), 7.91 (d, *J* = 1.5 Hz, 1H), 7.85 (dd, *J* = 8.7, 5.0 Hz, 1H), 7.19 - 7.10 (m, 1H), 6.84 - 6.56 (m, 2H), 5.23 - 5.09 (m, 1H), 4.94 - 4.73 (m, 2H), 4.52 - 4.38 (m, 2H), 4.32 - 4.14 (m, 2H), 3.25 (s, 3H), 2.95 (s, 3H), 2.72 - 2.62 (m, 2H), 2.47 - 2.22 (m, 3H), 2.20 - 2.05 (m, 1H), 1.95 - 1.77 (m, 2H), 1.53 (d, *J* = 6.2 Hz, 3H), 1.08 (s, 3H). $^{19}$F NMR (376 MHz, DMSO-$d_6$) δ -118.65, -120.87, -138.84. |
| 255B | 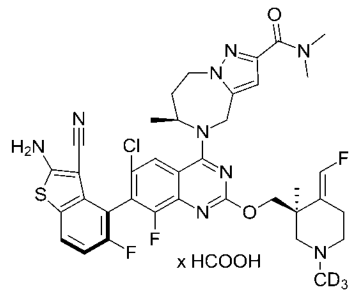 | LCMS (m/z): 767.3 (M+H). $^1$H NMR (400 MHz, DMSO-$d_6$) δ 8.31 (s, 1.5H), 8.03 (s, 2H), 7.92 (s, 1H), 7.85 (dd, *J* = 8.7, 4.9 Hz, 1H), 7.20 - 7.08 (m, 1H), 6.85 - 6.55 (m, 2H), 5.07 - 4.96 (m, 1H), 4.93 - 4.73 (m, 2H), 4.56 - 4.42 (m, 2H), 4.32 - 4.21 (m, 1H), 4.18 - 4.01 (m, 1H), 3.24 (s, 3H), 2.95 (s, 3H), 2.73 - 2.60 (m, 2H), 2.43 - 2.09 (m, 4H), 1.96 - 1.78 (m, 2H), 1.53 (d, *J* = 6.2 Hz, 3H), 1.08 (s, 3H). $^{19}$F NMR (376 MHz, DMSO-$d_6$) δ -118.83, -121.17, - 138.85. |
| 256A | 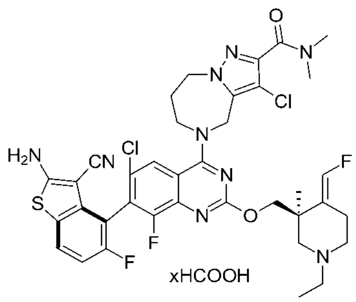 | LCMS (m/z): 798.2 (M+H). $^1$H NMR (400 MHz, DMSO-$d_6$) δ 8.00 (s, 2H), 7.90 - 7.81 (m, 2H), 7.22 - 7.07 (m, 1H), 6.79 (s, 0.5H), 6.58 (s, 0.5H), 5.13 - 5.04 (m, 1H), 5.00 - 4.87 (m, 1H), 4.49 - 4.36 (m, 3H), 4.32 - 4.26 (m, 1H), 4.22 - 4.12 (m, 2H), 3.01 (s, 3H), 2.97 (s, 3H), 2.81 - 2.72 (m, 2H), 2.56 - 2.52 (m, 1H), 2.40 - 2.24 (m, 4H), 2.20 - 2.09 (m, 1H), 2.01 - 1.89 (m, 1H), 1.85 (d, *J*= 11.2 Hz, 1H), 1.09 (s, 3H), 1.01 - 0.91 (m, 3H). $^{19}$F NMR (376 MHz, DMSO-$d_6$) δ -73.46, -118.83, -121.05, -139.09. |
| 256B | 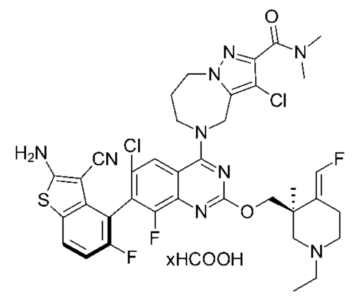 | LCMS (m/z): 798.2 (M+H). |
| 257A | 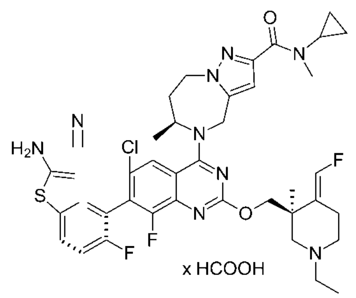 | LCMS(m/z): 804.3 (M+H). $^1$H NMR (400 MHz, DMSO-$d_6$) δ 8.39 (s, 1.73H), 7.98 (s, 2H), 7.95 (s, 1H), 7.84 (dd, *J* = 8.8,5.2 Hz, 1H), 7.14 (t, J=9.2Hz, 1H), 6.81 (s, 0.5H), 6.65 (s, 1H), 6.59 (s, 0.5H), 5.15 (d, *J* =17.2 Hz, 1H), 4.89-4.79 (m, 2H), 4.51-4.40 (m, 2H), 4.31-4.19 (m, 2H), 2.97 (s, 4H), 2.77-2.73 (m, 2H), 2.46-2.25 (m, 5H), 2.14-2.10 (m, 1H), 1.98-191 (m, 1H), 1.84 (d, *J*= 11.2 Hz, 1H), 1.51 (d, *J* = 6.0 Hz, 3H), 1.10 (s, 3H), 0.97 (t, *J* =6.8 Hz, 3H), 0.63-0.50 (m, 4H). $^{19}$F NMR (376 MHz, DMSO-$d_6$) δ -118.71, -120.86, -139.13. |

(continued)

| Example | Structural Formula | Characterization Data |
|---|---|---|
| 257B | 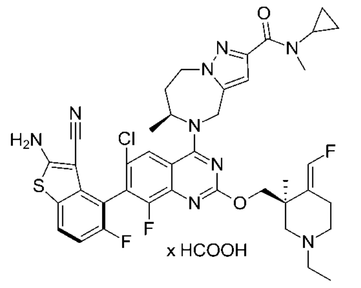 x HCOOH | LCMS(m/z): 804.3 (M+H). $^1$H NMR (400 MHz, DMSO-$d_6$) δ 8.38 (s, 1.87H), 8.02 (s, 2H), 7.94 (s, 1H), 7.85 (dd, *J* = 8.8, 5.2 Hz, 1H), 7.13 (t, J=9.2Hz, 1H), 6.80 (s, 0.5H), 6.61 (s, 1H), 6.58 (s, 0.5H), 5.01-4.80 (m, 3H), 4.54-4.50 (m, 2H), 4.28 (d, *J* = 10.4 Hz, 1H), 4.15-4.09 (m, 1H), 2.97 (s, 4H), 2.79-2.74 (m, 2H), 2.45-2.25 (m, 5H), 2.17-2.13 (m, 1H), 1.97-190 (m, 1H), 1.84 (d, *J* = 11.2 Hz, 1H), 1.52 (d, *J* = 6.4 Hz, 3H), 1.09 (s, 3H), 0.97 (t, *J* =7.2 Hz, 3H), 0.63-0.50 (m, 4H). $^{19}$F NMR (376 MHz, DMSO-$d_6$) δ -118.83, -121.08, -139.15. |
| 258A | 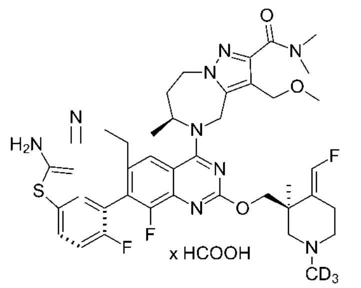 x HCOOH | LCMS (m/z): 805.4 (M+H). $^1$H NMR (400 MHz, DMSO-$d_6$) δ 8.36 (s, 1.0H), 7.92 (s, 2H), 7.81 (dd, *J* = 8.8,4.8 Hz, 1H), 7.56 (s, 1H), 7.13 (t, *J* =8.8 Hz, 1H), 6.78 (s, 0.5H), 6.57 (s, 0.5H), 5.09 (d, *J* =16.4 Hz, 1H), 4.87-4.82 (m, 1H), 4.69-4.59 (m, 2H), 4.51 (d, *J* = 11.2 Hz, 1H), 4.40-4.38 (m, 1H), 4.28 (d, *J*= 10.4 Hz, 1H), 4.15 (d, *J* = 10.4 Hz, 1H), 4.02-3.97 (m, 1H), 3.24 (s, 3H), 3.05 (s, 3H), 2.95 (s, 3H), 2.69-2.65 (m, 2H), 2.45-2.40 (m, 2H), 2.34-2.27 (m, 2H), 2.12-2.05 (m, 1H), 1.90-1.80 (m, 1H), 1.79 (d, *J* = 11.6 Hz, 1H), 1.67 (d, *J* = 6.4 Hz, 3H), 1.06 (s, 3H), 1.01 (t, *J* =7.6 Hz, 3H). $^{19}$F NMR (376 MHz, DMSO-$d_6$) δ -118.63, -125.36, -138.94. |
| 258B | 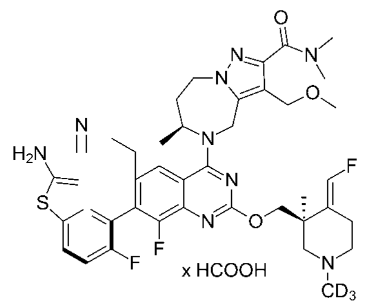 x HCOOH | LCMS (m/z): 805.3 (M+H). $^1$H NMR (400 MHz, DMSO-$d_6$) δ 8.38 (s, 1.66H), 7.93 (s, 2H), 7.81 (dd, *J*= 8.4,4.0 Hz, 1H), 7.48 (s, 1H), 7.13 (t, *J* =8.8 Hz, 1H), 6.79 (s, 0.5H), 6.57 (s, 0.5H), 5.03 (d, *J* =16.8 Hz, 1H), 4.81-4.75 (m, 1H), 4.70 (d, *J* =16.4 Hz, 1H), 4.54-4.41 (m, 3H), 4.36 (d, *J* = 10.4 Hz, 1H), 4.20 (d, *J* = 10.4 Hz, 1H), 3.97-3.86 (m, 1H), 3.23 (s, 3H), 3.05 (s, 3H), 2.95 (s, 3H), 2.69-2.65 (m, 2H), 2.45-2.40 (m, 1H), 2.34-2.25 (m, 3H), 2.18-2.11 (m, 1H), 1.90-1.83 (m, 1H), 1.80 (d, *J* = 11.2 Hz, 1H), 1.63 (d, *J* = 6.4 Hz, 3H), 1.07 (s, 3H), 0.93 (t, *J* =7.6 Hz, 3H). $^{19}$F NMR (376 MHz, DMSO-$d_6$) δ -119.03, -126.33, -139.00 |
| 259A | 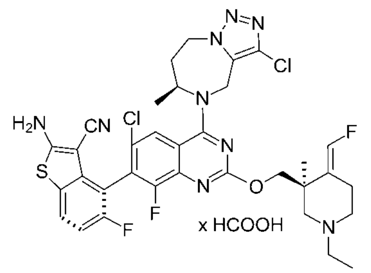 x HCOOH | LCMS(m/z): 742.2 (M+H). $^1$H NMR (400 MHz, DMSO-$d_6$) δ 8.13 (s, 0.43H), 7.99 (s, 2H), 7.86 (dd, *J*= 8.7, 4.9 Hz, 1H), 7.71 (s, 1H), 7.15 (t, *J*= 9.2 Hz, 1H), 6.82 (m, 1H), 5.01 (d, *J* = 16.5 Hz, 1H), 4.83 (d, *J* = 16.5 Hz, 2H), 4.69 (m, 1H), 4.40 - 4.02 (m, 3H), 2.81 (m, 2H), 2.46 - 1.72 (m, 8H), 1.62 (d, *J*= 6.2 Hz, 3H), 1.11 (m, 6H). |
| 259B | 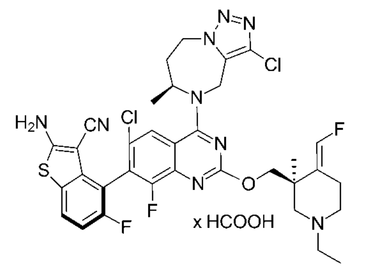 x HCOOH | LCMS(m/z): 742.2 (M+H). |

(continued)

| Example | Structural Formula | Characterization Data |
|---|---|---|
| 260A | 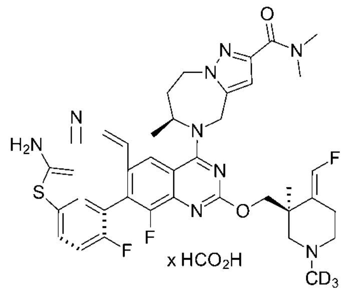 | LCMS(m/z): 759.3 (M+H). |
| 260B | 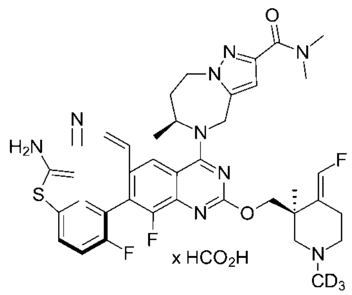 | LCMS(m/z): 759.3 (M+H). |
| 261A | 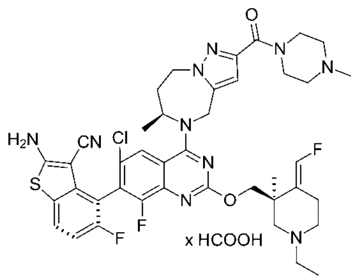 | LCMS(m/z): 833.2 (M+H). $^1$H NMR (400 MHz, DMSO-$d_6$) δ 8.13 (s, 0.45H), 7.98 (s, 2H), 7.90 (s, 1H), 7.85 (m, 1H), 7.14 (t, *J*= 9.1 Hz, 1H), 6.83 (s, 1.5H), 6.71 (s, 1H), 6.61 (s, 0.5H), 5.17 (d, *J* = 17.1 Hz, 1H), 4.93 - 4.74 (m, 2H), 4.46 (m, 2H), 4.35 - 4.11 (m, 2H), 3.75 (m, 8H), 2.78 (s, 1H), 2.47 - 1.84 (m, 12H), 1.53 (d, *J*= 6.2 Hz, 3H), 1.11 (s, 3H), 1.00 (m, 3H). |
| 261B | 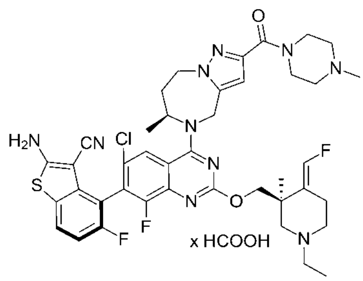 | LCMS(m/z): 833.2 (M+H). |
| 262A | 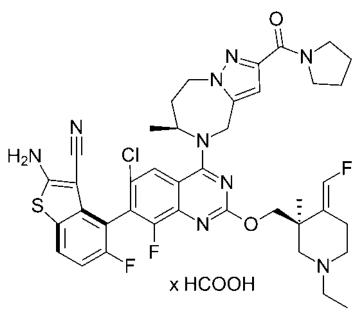 | LCMS(m/z): 804.3 (M+H). $^1$H NMR (400 MHz, DMSO-$d_6$) δ 8.38 (s, 1H), 7.98 (s, 2H), 7.91 (s, 1H), 7.85 (dd, J = 8.7, 5.0 Hz, 1H), 7.14 (t, J = 9.2 Hz, 1H), 6.81 (s, 0.5H), 6.78 (s, 1H), 6.59 (s, 0.5H), 5.22 - 5.10 (m, 1H), 4.93 - 4.73 (m, 2H), 4.54 - 4.39 (m, 2H), 4.34 - 4.17 (m, 2H), 3.86 - 3.74 (m, 2H), 3.47 - 3.43 (m, 2H), 2.80 - 2.70 (m, 2H), 2.49 - 2.24 (m, 5H), 2.19 - 2.06 (m, 1H), 2.00 - 1.91 (m, 1H), 1.91 - 1.74 (m, 5H), 1.51 (d, *J* = 6.2 Hz, 3H), 1.09 (s, 3H), 0.97 (t, J = 7.1 Hz, 3H). $^{19}$F NMR (376 MHz, DMSO-$d_6$) δ -118.68, -120.84, -139.13. |

(continued)

| Example | Structural Formula | Characterization Data |
|---|---|---|
| 262B | 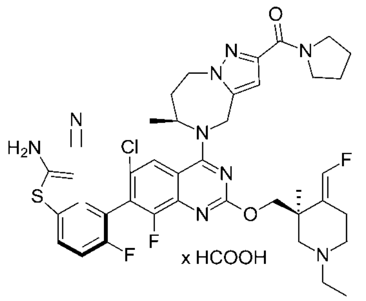 | LCMS(m/z): 804.3 (M+H). |
| 263A | 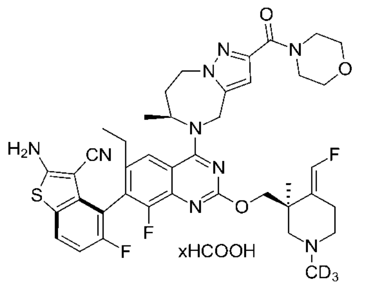 | LCMS (m/z): 803.3 (M+H). 1H NMR (400 MHz, DMSO-$d_6$) δ 9.46 (s, 0.5H), 8.03 - 7.79 (m, 3H), 7.66 (s, 1H), 7.20 - 7.08 (m, 1H), 6.97 - 6.44 (m, 2H), 5.25 - 5.06 (m, 1H), 4.94 - 4.73 (m, 2H), 4.54 - 4.41 (m, 1H), 4.38 - 4.19 (m, 2H), 4.14 - 4.05 (m, 1H), 4.04 - 3.89 (m, 2H), 3.76 - 3.53 (m, 7H), 3.49 - 3.34 (m, 2H), 2.99 - 2.83 (m, 2H), 2.45 - 2.21 (m, 5H), 1.59 (s, 3H), 1.24 - 1.09 (m, 3H), 1.06 - 0.91 (m, 3H). 19F NMR (376 MHz, DMSO-$d_6$) δ -73.45, -118.60, -125.57. |
| 263B | 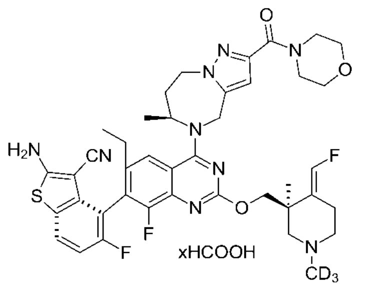 | LCMS (m/z): 803.3 (M+H). |
| 264A | 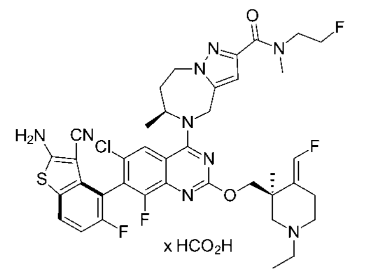 | LCMS(m/z): 810.3 (M+H). 1H NMR (400 MHz, DMSO-$d_6$) δ 8.33 (s, 2H), 7.98 (s, 2H), 7.93 - 7.89 (m, 1H), 7.88 - 7.81 (m, 1H), 7.18 - 7.11 (m, 1H), 6.83 - 6.56 (m, 2H), 5.23 - 5.12 (m, 1H), 4.92 - 4.75 (m, 2H), 4.71 - 4.13 (m, 7H), 3.00 (s, 3H), 2.80 - 2.70 (m, 2H), 2.43 - 2.23 (m, 5H), 2.21 - 1.78 (m, 4H), 1.57 - 1.46 (m, 3H), 1.09 (s, 3H), 0.97 (t, $J$ = 7.1 Hz, 3H). |
| 264B | 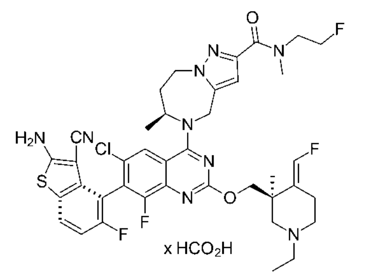 | LCMS(m/z): 810.3 (M+H). |

(continued)

| Example | Structural Formula | Characterization Data |
|---|---|---|
| 265A | 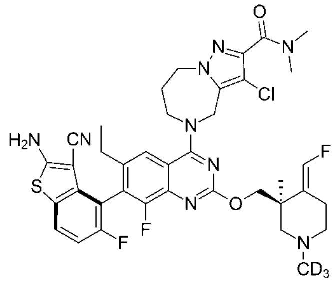 | LCMS(m/z): 781.3 (M+H). $^1$H NMR (400 MHz, DMSO-$d_6$) δ 7.92 (s, 2H), 7.85 - 7.78 (m, 1H), 7.58 (s, 1H), 7.18 - 7.08 (m, 1H), 6.83 - 6.55 (m, 1H), 5.10 - 4.92 (m, 2H), 4.50 - 4.39 (m, 3H), 4.29 - 4.22 (m, 1H), 4.21 - 4.12 (m, 2H), 3.01 (s, 3H), 2.97 (s, 3H), 2.72 - 2.62 (m, 2H), 2.48 - 2.25 (m, 5H), 2.20 - 2.08 (m, 1H), 1.95 - 1.74 (m, 2H), 1.09 (s, 3H), 0.96 (t, *J* = 7.5 Hz, 3H). $^{19}$F NMR (376 MHz, DMSO-$d_6$) δ -118.75, -125.75, -138.93. |
| 266A | 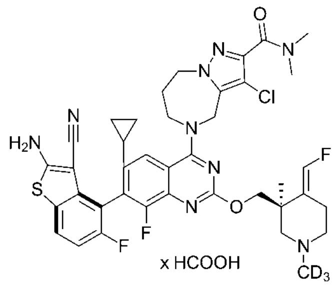 | LCMS(m/z): 793.3 (M+H). $^1$H NMR (400 MHz, DMSO-$d_6$) δ 7.89 (s, 2H), 7.81 (dd, *J* = 8.7, 4.9 Hz, 1H), 7.28 (s, 1H), 7.18 - 7.10 (m, 1H), 6.84 - 6.54 (m, 1H), 5.11 - 4.91 (m, 2H), 4.48 - 4.39 (m, 3H), 4.24 (d, *J* = 10.6 Hz, 1H), 4.21 - 4.06 (m, 2H), 3.02 (s, 3H), 2.97 (s, 3H), 2.69 - 2.64 (m, 2H), 2.54 - 2.52 (m, 1H), 2.37 - 2.29 (m, 2H), 2.14 (s, 1H), 1.94 - 1.76 (m, 2H), 1.57 - 1.46 (m, 1H), 1.08 (s, 3H), 0.70 - 0.57 (m, 4H). $^{19}$F NMR (376 MHz, DMSO-$d_6$) δ -118.65, -126.21, -138.92. |
| 266B | 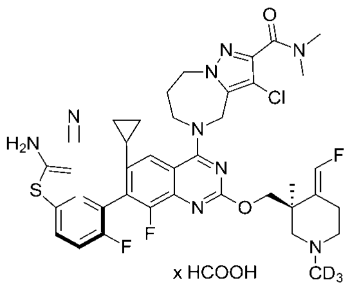 | LCMS(m/z): 793.3 (M+H). $^1$H NMR (400 MHz, DMSO-$d_6$) δ 8.31 (s, 1H), 7.89 (s, 2H), 7.81 (dd, *J* = 8.7, 4.9 Hz, 1H), 7.28 (s, 1H), 7.19 - 7.09 (m, 1H), 6.82 - 6.53 (m, 1H), 5.12 - 4.94 (m, 2H), 4.56 - 4.36 (m, 3H), 4.27 - 4.07 (m, 3H), 3.02 (s, 3H), 2.97 (s, 3H), 2.71 - 2.65 (m, 2H), 2.47 - 2.24 (m, 3H), 2.23 - 2.12 (m, 1H), 1.94 - 1.77 (m, 2H), 1.58 - 1.47 (m, 1H), 1.07 (s, 3H), 0.74 - 0.52 (m, 4H). $^{19}$F NMR (376 MHz, DMSO-$d_6$) δ -118.63, -126.21, -138.96. |
| 267A | 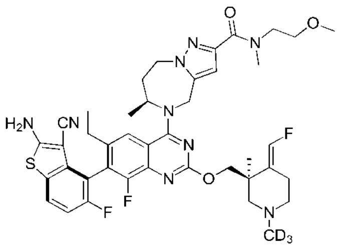 | LCMS(m/z): 805.4 (M+H). $^1$H NMR (400 MHz, DMSO-$d_6$) δ 8.14 (s, 1H), 7.91 (s, 2H), 7.85 - 7.79 (m, 1H), 7.66 (s, 1H), 7.16 - 7.10 (m, 1H), 6.75 - 6.50 (m, 2H), 5.19 - 5.10 (m, 1H), 4.92 - 4.82 (m, 1H), 4.82 - 4.75 (m, 1H), 4.52 - 4.40 (m, 1H), 4.40 - 4.24 (m, 2H), 4.19 - 4.02 (m, 1H), 3.94 - 3.81 (m, 1H), 3.61 - 3.56 (m, 1H), 3.53 - 3.50 (m, 2H), 3.31 - 3.28 (m, 3H), 3.28 - 3.23 (m, 3H), 3.18 (s, 2H), 2.97 (s, 2H), 2.48 - 2.23 (m, 7H), 1.63 - 1.52 (m, 3H), 1.12 (s, 3H), 0.99 (t, *J*= 7.5 Hz, 3H). $^{19}$F NMR (376 MHz, DMSO-$d_6$) δ -73.48, -118.59, -125.52. |
| 268A | 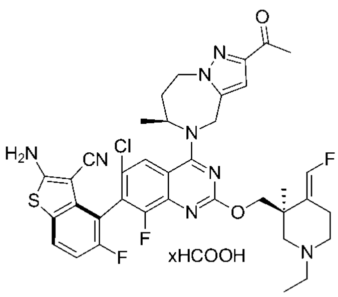 | LCMS (m/z): 749.3 (M+H). $^1$H NMR (400 MHz, $CD_3OD$) δ 8.03 - 7.92 (m, 1H), 7.73 - 7.60 (m, 1H), 7.03 (t, *J*= 9.1 Hz, 1H), 6.86 (s, 1H), 6.79 (s, 0.5H), 6.58 (s, 0.5H), 5.28 - 5.13 (m, 1H), 5.04 - 4.92 (m, 1H), 4.83 - 4.72 (m, 1H), 4.67 - 4.53 (m, 2H), 4.48 - 4.34 (m, 1H), 4.27 - 4.18 (m, 1H), 4.16 - 4.05 (m, 1H), 3.15 - 2.92 (m, 2H), 2.76 - 2.66 (m, 1H), 2.65 - 2.55 (m, 2H), 2.50 (s, 3H), 2.44 - 2.28 (m, 3H), 2.25 - 2.14 (m, 1H), 1.68 (d, 3H), 1.23 - 1.08 (m, 6H). $^{19}$F NMR (376 MHz, $CD_3OD$) δ -76.94, - 120.33, -121.99. |

(continued)

| Example | Structural Formula | Characterization Data |
|---|---|---|
| 268B | 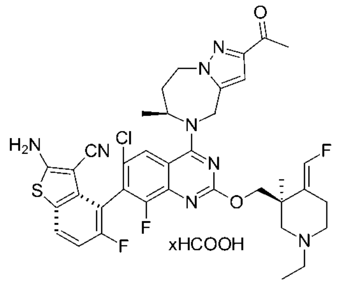 | LCMS (m/z): 749.3 (M+H). |
| 269A | 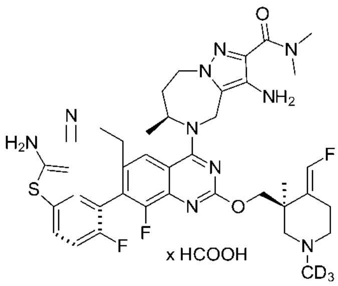 | LCMS (m/z): 776.3 (M+H). $^1$H NMR (400 MHz, DMSO-$d_6$) δ 8.31 (s, 1H), 7.91 (s, 2H), 7.81 (dd, *J* = 8.7, 4.9 Hz, 1H), 7.51 (s, 1H), 7.17 - 7.06 (m, 1H), 6.69 (d, *J* = 86.9 Hz, 1H), 4.98 - 4.84 (m, 2H), 4.62 - 4.51 (m, 1H), 4.49 - 4.39 (m, 2H), 4.30 - 4.20 (m, 1H), 4.17 - 4.07 (m, 1H), 3.31 - 3.20 (m, 3H), 3.02 - 2.88 (m, 3H), 2.72 - 2.63 (m, 2H), 2.57 - 2.53 (m, 2H), 2.39 - 2.09 (m, 5H), 2.05 - 1.72 (m, 3H), 1.58 (d, *J* = 6.1 Hz, 3H), 1.10 (s, 3H), 0.92 (t, *J* = 7.5 Hz, 3H). $^{19}$F NMR (376 MHz, DMSO-$d_6$) δ -118.45, - 125.62, -138.90. |
| 269B | 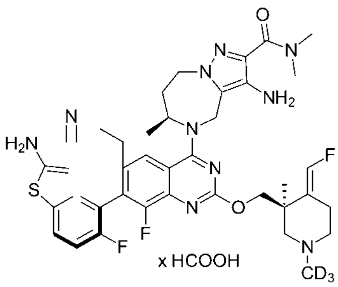 | LCMS (m/z): 776.3 (M+H). $^1$H NMR (400 MHz, DMSO-$d_6$) δ 8.36 (s, 1H), 7.92 (s, 2H), 7.81 (dd, *J* = 8.7, 4.9 Hz, 1H), 7.52 (s, 1H), 7.18 - 7.06 (m, 1H), 6.69 (d, *J* = 86.9 Hz, 1H), 4.96 - 4.80 (m, 2H), 4.63 - 4.55 (m, 1H), 4.52 - 4.41 (m, 2H), 4.27 (d,*J* = 10.6 Hz, 1H), 4.08 - 3.96 (m, 1H), 3.30 - 3.23 (m, 3H), 3.00 - 2.88 (m, 3H), 2.72 - 2.62 (m, 2H), 2.50 - 2.44 (m, 2H), 2.39 - 2.11 (m, 5H), 2.04 - 1.77 (m, 3H), 1.59 (d, *J* = 6.3 Hz, 3H), 1.09 (s, 3H), 0.92 (t, *J*= 7.5 Hz, 3H). $^{19}$F NMR (376 MHz, DMSO-$d_6$) δ -118.90, -126.03, -138.96. |
| 270A | 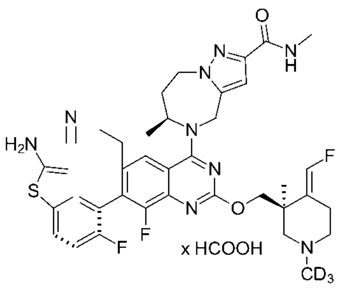 | LCMS(m/z): 747.3 (M+H). $^1$H NMR (400 MHz, DMSO-$d_6$) δ 8.14 (s, 0.14H), 8.04 (m, 1H), 7.91 (s, 2H), 7.83 (dd, *J* = 8.7, 5.0 Hz, 1H), 7.66 (s, 1H), 7.13 (t, *J* = 9.1 Hz, 1H), 6.97 - 6.42 (m, 2H), 5.13 (m, 1H), 4.80 (m, 2H), 4.27 (m, 4H), 3.51 (m, 2H), 2.90 (m, 2H), 2.72 (d, *J* = 4.7 Hz, 3H), 2.48 - 2.19 (m, 6H), 1.60 (s, 3H), 1.30 (s, 1H), 1.11 (s, 2H), 1.01 (t, *J*= 7.5 Hz, 3H). |
| 270B | 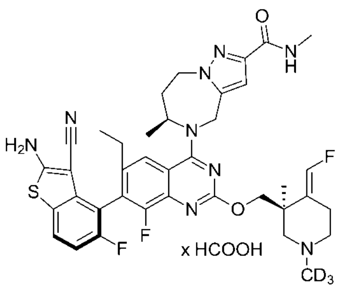 | LCMS(m/z): 747.3 (M+H). |

(continued)

| Example | Structural Formula | Characterization Data |
|---|---|---|
| 271A | 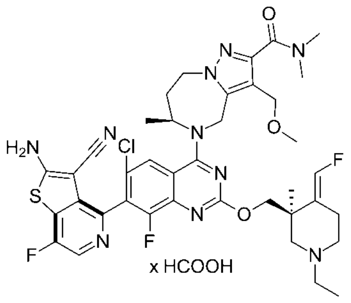 | LCMS(m/z): 823.3 (M+H). |
| 271B | 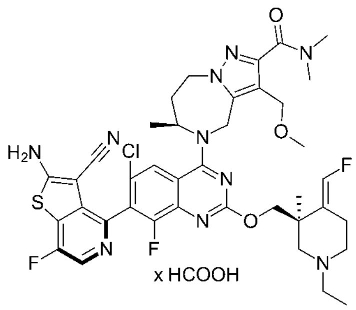 | LCMS(m/z): 823.3 (M+H). |
| 272A | 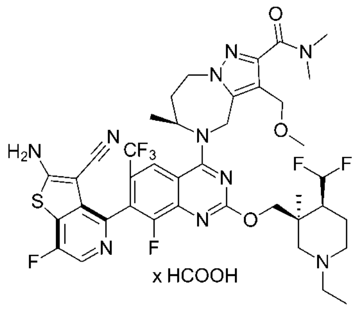 | LCMS(m/z): 877.3 (M+H). $^1$H NMR (400 MHz, DMSO-$d_6$) δ 8.63 - 8.43 (m, 2H), 8.42 (s, 1H), 8.41 (s, 1H), 7.98 (s, 1H), 6.46 - 6.09 (m, 1H), 5.12 (d, *J* = 17.0 Hz, 1H), 4.88 - 4.80 (m, 1H), 4.77 (d, *J* = 17.1 Hz, 1H), 4.58 - 4.38 (m, 4H), 4.37 - 4.31 (m, 1H), 4.28 - 4.09 (m, 1H), 3.23 (s, 3H), 3.07 (s, 3H), 2.96 (s, 3H), 2.94 - 2.81 (m, 2H), 2.46 - 2.29 (m, 2H), 2.23 (q, *J*= 7.0 Hz, 2H), 2.05 - 1.84 (m, 1H), 1.83 - 1.72 (m, 1H), 1.69 - 1.55 (m, 6H), 1.11 (s, 3H), 0.86 (t, *J* = 7.1 Hz, 3H). $^{19}$F NMR (376 MHz, DMSO-$d_6$) δ -57.35, -115.24, -116.04, -118.61, -119.36, - 125.03, -131.71. |
| 272B | 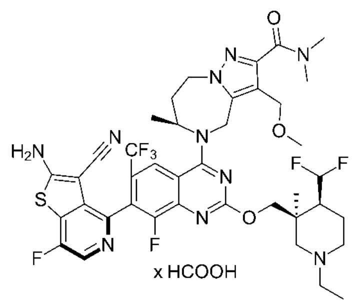 | LCMS(m/z): 877.3 (M+H). $^1$H NMR (400 MHz, DMSO-$d_6$) δ 8.63 - 8.43 (m, 3H), 8.43 (s, 1H), 7.93 (s, 1H), 6.46 - 6.07 (m, 1H), 5.06 - 4.91 (m, 1H), 4.88 - 4.79 (m, 1H), 4.79 - 4.68 (m, 1H), 4.55 - 4.40 (m, 4H), 4.40 - 4.32 (m, 1H), 4.22 - 4.01 (m, 1H), 3.21 (s, 3H), 3.07 (s, 3H), 2.96 (s, 3H), 2.94 - 2.82 (m, 2H), 2.47 - 2.39 (m, 1H), 2.37 - 2.28 (m, 1H), 2.24 (q, *J* = 6.6, 5.9 Hz, 2H), 1.94 - 1.83 (m, 1H), 1.82 - 1.71 (m, 1H), 1.70 - 1.49 (m, 6H), 1.11 (s, 3H), 0.86 (t, *J* = 7.1 Hz, 3H). $^{19}$F NMR (376 MHz, DMSO-$d_6$) δ -57.45, -115.30, -116.21, -118.63, - 119.38, -125.03, -131.69. |
| 273A | 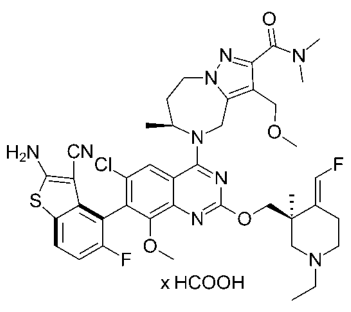 | LCMS (m/z): 834.3 (M+H). $^1$H NMR (400 MHz, DMSO-$d_6$) δ 8.37 (s, 1H), 7.86 (s, 2H), 7.76 (dd, J = 8.7, 5.0 Hz, 1H), 7.62 (s, 1H), 7.16 - 7.03 (m, 1H), 6.81 (s, 0.5H), 6.59 (s, 0.5H), 5.16 - 5.01 (m, 1H), 4.88 - 4.75 (m, 1H), 4.73 - 4.59 (m, 2H), 4.57 - 4.50 (m, 1H), 4.49 - 4.40 (m, 1H), 4.34 (s, 2H), 4.18 - 4.06 (m, 1H), 3.84 (s, 3H), 3.23 (s, 3H), 3.06 (s, 3H), 2.96 (s, 3H), 2.81 - 2.69 (m, 2H), 2.58 - 2.52 (m, 1H), 2.38 - 2.22 (m, 4H), 2.18 - 2.06 (m, 1H), 1.99 - 1.89 (m, 1H), 1.82 (d, *J* = 11.3 Hz, 1H), 1.58 (d, *J* = 6.3 Hz, 3H), 1.10 (s, 3H), 0.92 (t, *J* = 7.1 Hz, 3H). $^{19}$F NMR (376 MHz, DMSO-$d_6$) δ -118.90, -139.18. |

(continued)

| Example | Structural Formula | Characterization Data |
|---|---|---|
| 273B | 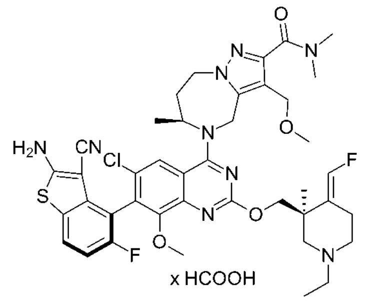 | LCMS (m/z): 834.3 (M+H). |
| 274A | 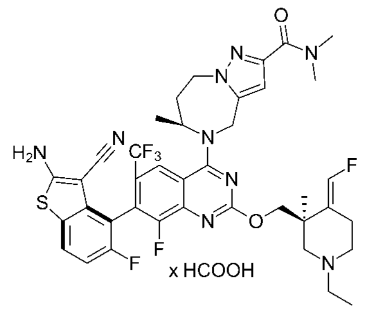 | LCMS (m/z): 821.0 (M+H). $^1$H NMR (400 MHz, DMSO-$d_6$) δ 8.33 (s, 1H), 8.14 (s, 1H), 7.81 *(dd, J*= 8.7, 5.0 Hz, 1H), 7.08 - 6.99 (m, 1H), 6.81 - 6.47 (m, 4H), 5.27 - 5.14 (m, 1H), 4.96 - 4.78 (m, 2H), 4.67 - 4.52 (m, 1H), 4.51 - 4.38 (m, 1H), 4.33 - 4.16 (m, 2H), 3.24 (s, 3H), 2.95 (s, 3H), 2.81 - 2.71 (m, 2H), 2.46 - 2.23 (m, 5H), 2.21 - 2.08 (m, 1H), 1.98 - 1.87 (m, 1H), 1.86 - 1.78 (m, 1H), 1.53 (d, *J* = 6.2 Hz, 3H), 1.09 (s, 3H), 0.96 (t, *J* = 7.1 Hz, 3H). $^{19}$F NMR (376 MHz, DMSO-$d_6$) δ -58.76, -117.72 - - 118.05, -121.48, -139.03. |
| 274B | 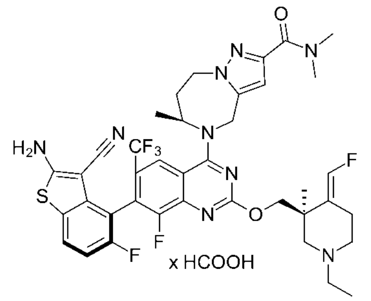 | LCMS (m/z): 821.0 (M+H). $^1$H NMR (400 MHz, DMSO-$d_6$) δ 8.37 (s, 1H), 8.15 (s, 1H), 7.80 *(dd, J*= 8.6, 5.0 Hz, 1H), 7.07 - 6.93 (m, 1H), 6.87 - 6.53 (m, 4H), 5.23 - 5.11 (m, 1H), 4.95 - 4.79 (m, 2H), 4.59 - 4.39 (m, 2H), 4.33 - 4.15 (m, 2H), 3.23 (s, 3H), 2.95 (s, 3H), 2.84 - 2.70 (m, 2H), 2.47 - 2.24 (m, 5H), 2.19 - 2.09 (m, 1H), 1.98 - 1.87 (m, 1H), 1.87 - 1.79 (m, 1H), 1.54 (d, *J* = 6.1 Hz, 3H), 1.09 (s, 3H), 0.97 (t, *J* = 7.1 Hz, 3H). $^{19}$F NMR (376 MHz, DMSO-$d_6$) δ -58.79, -118.22 - -118.53, -121.89, - 139.11. |
| 275A | 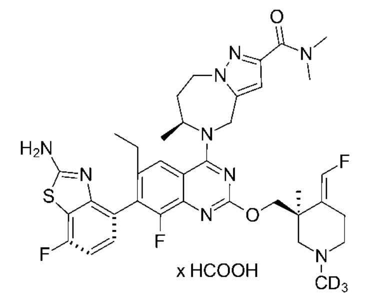 | LCMS (m/z): 737.4 (M+H). $^1$H NMR (400 MHz, $CD_3OD$) δ 8.53 (s, 0.4 H), 7.76 (s, 1H), 7.17 *(dd, J*= 8.4, 5.4 Hz, 1H), 7.05 - 6.96 (m, 1H), 6.91 (s, 0.5H), 6.72 (s, 1H), 6.70 (s, 0.5H), 5.30 - 5.17 (m, 1H), 5.07 - 4.94 (m, 1H), 4.85 - 4.76 (m, 1H), 4.53 (dd, *J* = 14.4, 6.8 Hz, 1H), 4.45 (d, *J* = 10.7 Hz, 1H), 4.12 (d, *J* = 10.7 Hz, 1H), 4.06 - 3.88 (m, 1H), 3.35 (s, 3H), 3.31 - 3.14 (m, 2H), 3.11 (s, 3H), 2.90 - 2.76 (m, 1H), 2.68 - 2.33 (m, 7H), 1.75 (d, *J* = 6.3 Hz, 3H), 1.23 (s, 3H), 1.08 (t, *J* = 7.5 Hz, 3H). $^{19}$F NMR (376 MHz, $CD_3OD$) δ -115.60, -127.87, -137.05. |
| 275B | 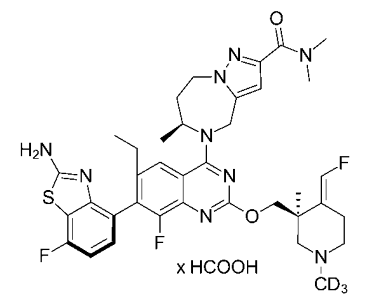 | LCMS (m/z): 737.4 (M+H). $^1$H NMR (400 MHz, $CD_3OD$) δ 8.53 (s, 0.5H), 7.74 (s, 1H), 7.19 (dd, *J* = 8.4, 5.5 Hz, 1H), 7.08 - 6.95 (m, 1H), 6.88 (s,0.5H), 6.71 (s, 1H), 6.67 (s, 0.5H), 5.25 (d, *J* = 16.7 Hz, 1H), 5.06 - 4.95 (m, 1H), 4.82 (d, *J*= 16.7 Hz, 1H), 4.54 *(dd, J*= 14.3, 6.7 Hz, 1H), 4.44 (d, *J* = 10.7 Hz, 1H), 4.14 (d, *J*= 10.7 Hz, 1H), 4.05 - 3.91 (m, 1H), 3.36 (s, 3H), 3.25 - 3.16 (m, 1H), 3.15 - 3.04 (m, 4H), 2.86 - 2.75 (m, 1H), 2.68 - 2.32 (m, 7H), 1.75 (d, *J* = 6.3 Hz, 3H), 1.22 (s, 3H), 1.05 (t, *J* = 7.5 Hz, 3H). $^{19}$F NMR (376 MHz, $CD_3OD$) δ -115.65, - 128.31, -137.51. |

(continued)

| Example | Structural Formula | Characterization Data |
|---|---|---|
| 276A | 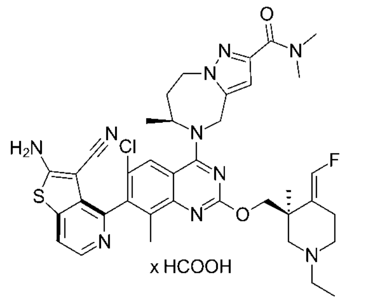 | LCMS(m/z): 757.3 (M+H). $^1$H NMR (400 MHz, DMSO-$d_6$) δ 8.44 - 8.31 (m, 3H), 8.05 (s, 2H), 7.89 - 7.81 (m, 2H), 6.90 - 6.53 (m, 2H), 5.07 (d, *J*= 17.1 Hz, 1H), 4.88 - 4.74 (m, 2H), 4.58 - 4.39 (m, 2H), 4.31 (d, J = 10.6 Hz, 1H), 4.25 - 4.07 (m, 1H), 3.25 (s, 3H), 2.95 (s, 3H), 2.79 - 2.69 (m, 2H), 2.42 - 2.39 (m, 1H), 2.34 - 2.24 (m, 4H), 2.16 - 2.09 (m, 4H), 2.00 - 1.94 (m, 1H), 1.85 (d, *J* = 11.2 Hz, 1H), 1.53 (d, *J* = 6.2 Hz, 3H), 1.10 (s, 3H), 0.97 (t, *J*= 7.1 Hz, 3H). $^{19}$F NMR (376 MHz, DMSO-$d_6$) δ -139.26. |
| 276B | 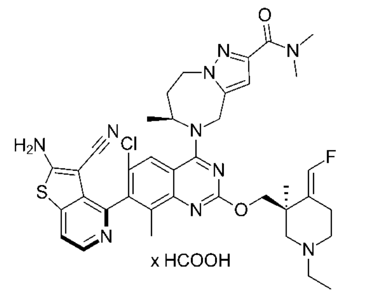 | LCMS(m/z): 757.3 (M+H). $^1$H NMR (400 MHz, DMSO-$d_6$) δ 8.44 - 8.33 (m, 4H), 8.06 (s, 2H), 7.84 (d, *J* = 5.8 Hz, 2H), 6.83 - 6.56 (m, 2H), 4.95 (d, *J* = 17.0 Hz, 1H), 4.87 - 4.74 (m, 2H), 4.54 - 4.43 (m, 2H), 4.31 (d, *J*= 10.6 Hz, 1H), 4.16 - 4.00 (m, 1H), 3.24 (s, 3H), 2.95 (s, 3H), 2.75 (d, *J* = 11.0 Hz, 2H), 2.39 - 2.24 (m, 4H), 2.19 - 2.10 (m, 4H), 2.00 - 1.91 (m, 1H), 1.84 (d, *J* = 11.3 Hz, 1H), 1.54 (d, *J* = 6.3 Hz, 3H), 1.09 (s, 3H), 0.97 (t, *J* = 7.1 Hz, 3H). $^{19}$F NMR (376 MHz, DMSO-$d_6$) δ -139.24. |
| 277A | 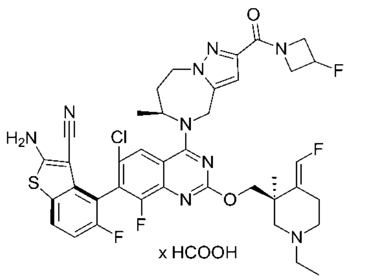 | LCMS(m/z): 808.3 (M+H). $^1$H NMR (400 MHz, DMSO-$d_6$) δ 8.31 (s, 1H), 7.99 (s, 2H), 7.92 - 7.75 (m, 2H), 7.24 - 7.06 (m, 1H), 6.88 - 6.50 (m, 2H), 5.43 (d, *J* = 57.7 Hz, 1H), 5.24 - 5.06 (m, 1H), 4.96 - 4.64 (m, 3H), 4.57 - 4.41 (m, 3H), 4.40 - 4.16 (m, 3H), 4.03 (dd, *J* = 25.2, 11.8 Hz, 1H), 2.82 - 2.68 (m, 2H), 2.47 - 2.37 (m, 2H), 2.36 - 2.23 (m, 3H), 2.19 - 2.06 (m, 1H), 2.01 - 1.90 (m, 1H), 1.84 (d, *J* = 11.1 Hz, 1H), 1.59 - 1.44 (m, 3H), 1.09 (s, 3H), 0.97 (t, *J* = 7.1 Hz, 3H). $^{19}$F NMR (376 MHz, DMSO-$d_6$) δ -118.68, -120.81, -139.10, -179.89. |
| 277B | 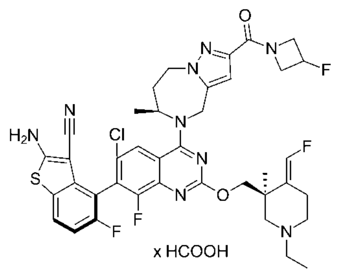 | LCMS(m/z): 808.2 (M+H). |
| 278A | 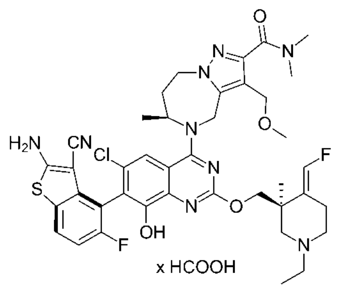 | LCMS (m/z): 820 .3 (M+H). |

(continued)

| Example | Structural Formula | Characterization Data |
|---|---|---|
| 283A | 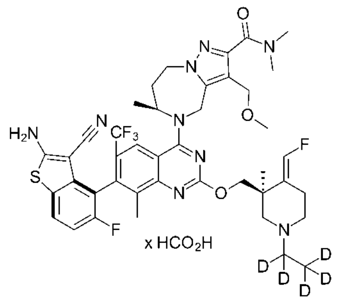 | LCMS (m/z): 857 .3 (M+H). |
| 283B | 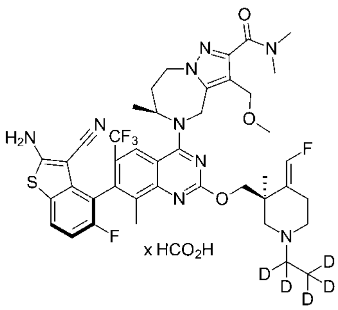 | LCMS (m/z): 857 .3 (M+H). |

**Example 279A**

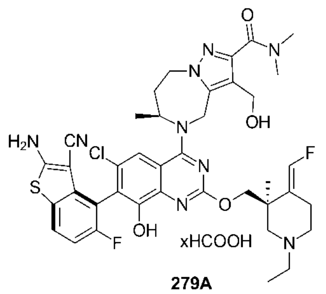

**279A**

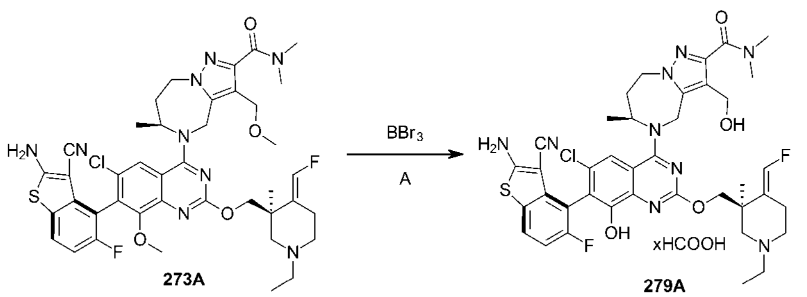


**273A** **279A**

**Step A:** Example **279A**

Under ice-water bath conditions, $BBr_3$ (2M, 0.5mL) was added to a mixed solution of Example **273A** (20mg, 0.024mmol) and DCM (1mL), and the mixture was stirred for 20 minutes while maintaining the temperature. The reaction completion was monitored by LCMS. The reaction solution was poured into saturated $NaHCO_3$ (10mL) and extracted with DCM (10mL×3). The collected organic phase was washed with saturated NaCl aqueous solution (50mL) and dried over anhydrous $Na_2SO_4$. After filtration and concentration, the crude product obtained was purified by prep-HPLC (C18, ACN/(10mmol FA/$H_2O$)=25-40%) to obtain a white solid, Example **279A** (3mg, Rt=11.3min, yield 16%). LCMS (m/z):

806.3 (M+H). $^1$H NMR (400 MHz, DMSO-$d_6$) δ 8.43 (s, 2H), 7.89 - 7.71 (m, 3H), 7.35 (s, 1H), 7.11 - 7.04 (m, 1H), 6.82 (s, 0.5H), 6.60 (s, 0.5H), 5.16 (d, $J$ = 17.2 Hz, 1H), 4.95 - 4.79 (m, 1H), 4.71 - 4.58 (m, 3H), 4.51 - 4.38 (m, 2H), 4.30 - 4.20 (m, 1H), 4.17 - 4.06 (m, 1H), 3.12 (s, 3H), 2.97 (s, 3H), 2.71 - 2.61 (m, 2H), 2.47 - 2.35 (m, 2H), 2.32 - 2.16 (m, 5H), 2.08 - 1.98 (m, 1H), 1.93 (d, $J$ = 11.2 Hz, 1H), 1.56 (d, $J$ = 6.3 Hz, 3H), 1.10 (s, 3H), 0.95 (t, J = 7.2 Hz, 3H). $^{19}$F NMR (376 MHz, DMSO-$d_6$) δ -118.74, -139.63.

The following compounds were prepared according to the above examples or appropriate variants:

| Example | Structural Formula | Characterization Data |
|---|---|---|
| 280A | 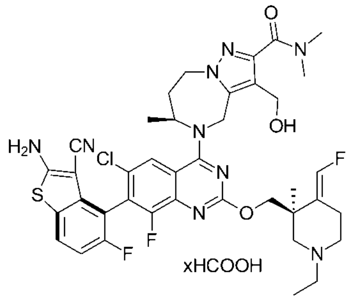 | LCMS (m/z): 808.3 (M+H). |
| 281A | 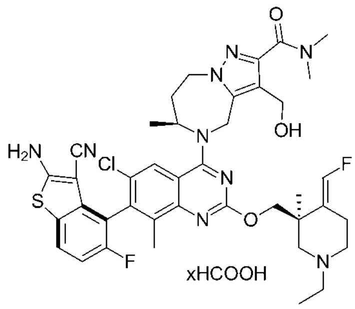 | LCMS (m/z): 804.3 (M+H). |
| 282A | 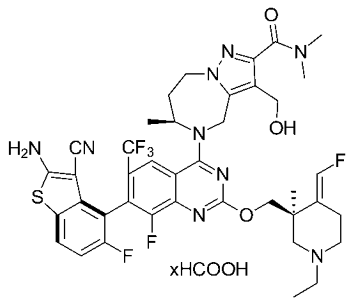 | LCMS (m/z): 842.3 (M+H). |

**Examples of Activity**

**Example 1: Proliferation inhibition effect of the compounds of the present invention on NCI-H727 cells with KRAS G12V mutation**

This experiment evaluated and verified the proliferation inhibitory activity of the representative compound of the present invention against KRAS G12V mutant NCI-H727 cells.

Method A:

NCI-H727 cells (Nanjing Kebai Biotechnology Co., Ltd., catalog number CBP60182, adherent, culture medium RPMI-1640+10%FBS (GIBCO, Cat#10091-148)) were cultured at 37°C, 5% $CO_2$, and 95% humidity.

3D Cell Viability Assay: Cells in the logarithmic growth phase were harvested and counted using a platelet counter. Cell viability was assessed using the trypan blue exclusion method to ensure a viability of over 90%. The 3D cell culture medium was prepared by adding 1% MC (methylcellulose, Sigma, Cat#M0512) to RPMI-1640 medium and 10% FBS. The cell concentration was adjusted to approximately 14815 cells/mL using the 3D medium, and the methylcellulose (MC) content was set to 0.65%. Then, 135 μL of cell suspension was added to each 96-well clear flat-bottomed, black-walled plate (Greiner, Cat# 655096). The cells in the 96-well plates were cultured overnight at 37°C and 5% $CO_2$.

Drug preparation for $IC_{50}$ assay: A 10-fold drug solution was prepared using culture medium. 10 μL of the drug solution was added to each well of a 96-well plate seeded with cells to achieve a working concentration of up to 10 μM, with a 3× dilution, and 9 concentrations. 2 replicate wells for each drug concentration were set. The cells in the drug-treated 96-well plates were cultured at 37°C and 5% $CO_2$ for additional 7 days, then CTG assay (CellTiter-Glo® Luminescent Cell Viability Assay (Promega, Cat#G7573)) were performed.

The cell plate was equilibrated to room temperature for 30 minutes and the CTG reagent was thawed. 75 μL of CTG solution was added to each well for detection, and the plate was shaken on an orbital shaker for 5 minutes to lyse the cells. The cell plate was placed at room temperature for 25 minutes to stabilize the cryo-luminescence signal and the cryo-luminescence value was read (EnVision® multi-plate reader, PerkinElmer#2105).

Data was analyzed using GraphPad Prism software. The dose-response-inhibition equation was used to fit the data to obtain the dose-response curve, and the $IC_{50}$ value was calculated accordingly.

Cell viability (%) = (Lum $_{\text{test drug}}$ - Lum $_{\text{culture medium control}}$)/(Lum $_{\text{cell control}}$ - Lum culture medium control) × 100%.

Representative compounds of the present invention showed satisfactory antiproliferative activity against KRAS G12V-mutant NCI-H727 human lung cancer cells, with an $IC_{50}$ range of <1 μM, preferably <0.1 μM.

Method B:

NCI-H727 cells (Nanjing Kebai Biotechnology Co., Ltd., catalog number CBP60182, adherent, culture medium RPMI-1640+10%FBS (GIBCO, Cat#10091-148)) were cultured at 37°C, 5% $CO_2$, and 95% humidity.

3D cell viability assay: Cells in the logarithmic growth phase were harvested and counted using a platelet counter. Cell viability was assessed using the trypan blue exclusion method to ensure a viability of over 90%. The 3D cell culture medium was prepared by adding 1% MC (methylcellulose, Sigma, Cat#M0512) to RPMI-1640 medium and 10% FBS. The cell concentration was adjusted to approximately 25,000 cells/mL using the 3D medium, and the methylcellulose (MC) content was set to 0.65%. Then, 80 μL of cell suspension was added to each 96-well clear, flat-bottomed, black-walled plate (Greiner, Cat# 655096). The cells in the 96-well plates were cultured overnight at 37°C and 5% $CO_2$.

Drug preparation for $IC_{50}$ assay: A 5-fold drug solution was prepared using culture medium. 20 μL of the drug solution was added to each well of a 96-well plate seeded with cells to achieve a working concentration of up to 10 μM, with a 3× dilution, and 9 concentrations. 2 replicate wells for each drug concentration were set. The cells in the drug-treated 96-well plates were cultured at 37°C and 5% $CO_2$ for additional 5 days, then CTG assay (CellTiter-Glo® Luminescent Cell Viability Assay (Promega, Cat#G7573)) were performed.

The cell plate was equilibrated to room temperature for 30 minutes and the CTG reagent was thawed. 50 μL of CTG solution was added to each well for detection and the plate was shaken on an orbital shaker for 5 minutes to lyse the cells. The cell plate was then placed at room temperature for 25 minutes to stabilize the cryo-luminescence signal, and the cryo-luminescence value was read (EnVision® multi-plate reader, PerkinElmer#2105).

Data was analyzed using GraphPad Prism software. The dose-response-inhibition equation was used to fit the data to obtain the dose-response curve, and the IC50 value (nM) was calculated accordingly.

Cell viability (%) = (Lum $_{\text{test drug}}$ - Lum $_{\text{culture medium control}}$)/(Lum $_{\text{cell control}}$ - Lum culture medium control) × 100%.

Compounds of the present invention exhibit satisfactory antiproliferative activity against KRAS G12V-mutant NCI-H727 human lung cancer cells, with an IC50 range of <1 μM, preferably <0.1 μM, for example 0.01 nM-100 nM, more preferably 0.01 nM-50 nM. The IC50 values (Method B) of representative compounds are shown in the table below:

| Example | NCI-H727 $IC_{50}$/nM | Example | NCI-H727 $IC_{50}$/nM | Example | NCI-H727 $IC_{50}$/nM |
|---|---|---|---|---|---|
| 3A | 40.7 | 120 | 29.8 | 230A | 2.2 |
| 4A | 66.9 | 121 | 438.8 | 231A | 4.4 |
| 5A | <1.5 | 123A | <1.5 | 232A | <1.5 |
| 6A | 1.6 | 124 | 2.2 | 233A | <1.5 |
| 7 | 13.1 | 126A | 77.0 | 234A | <1.5 |
| 8 | 21.3 | 126B | 56.0 | 235A | <1.5 |
| 8A | 4.4 | 150 | <1.5 | 236A | 1.5 |

(continued)

| Example | NCI-H727 $IC_{50}$/nM | Example | NCI-H727 $IC_{50}$/nM | Example | NCI-H727 $IC_{50}$/nM |
|---|---|---|---|---|---|
| 9A | 701.7 | 155 | 21.0 | 237A | <1.5 |
| 10A | 2120.7 | 156 | 69.2 | 238A | 6.5 |
| 10A-A | 888.3 | 157 | 57.9 | 240A | <1.5 |
| 11A-A | 127.8 | 158 | 7.9 | 241A | 6.5 |
| 12A-A | 261.9 | 159 | 4.3 | 242A | 6.3 |
| 12A-B | 3215.5 | 160 | <1.5 | 243A | <1.5 |
| 16 | 5 | 161 | 8.5 | 246A | <1.5 |
| 28A | 1.8 | 161A | 4.3 | 247A | <1.5 |
| 55 | 2.4 | 162A | 2.1 | 248A | <1.5 |
| 65A | <1.5 | 163A | 3.3 | 249A | <1.5 |
| 65B | 39.3 | 164A | <1.5 | 250A | <1.5 |
| 67 | 6.3 | 165A | 3.6 | 251A | <1.5 |
| 68 | 6.4 | 166A | 7.4 | 252 | 2.1 |
| 68A | 2.4 | 167A | 96.7 | 253 | 8.7 |
| 83 | 20.2 | 168A | 85.0 | 254A | <1.5 |
| 84 | 77.7 | 169A | 59.9 | 255A | <1.5 |
| 85A | 17.4 | 170A | 81.9 | 256A | <1.5 |
| 86 | 4.5 | 171A | 4.1 | 257A | <1.5 |
| 86A | 1.9 | 172A | 419.3 | 258A | <1.5 |
| 87 | 9 | 173A | <1.5 | 259A | 1.7 |
| 87A | 3.8 | 174A | 2.5 | 260A | 1.8 |
| 88 | 23.3 | 175A | <1.5 | 261A | 1.9 |
| 89 | 12.4 | 176A | <1.5 | 262A | 2.4 |
| 90 | 58.3 | 178A | <1.5 | 263A | 2.5 |
| 91 | 48.5 | 180 | 11.3 | 264A | 2.8 |
| 92 | 1693.5 | 182A | <1.5 | 265A | 2.8 |
| 93A | 4.1 | 183A | <1.5 | 266A | 3.0 |
| 93B | 191.3 | 184A | 4.3 | 267A | 3.9 |
| 94A | 9 | 185A | <1.5 | 268A | 4.0 |
| 95 | 36.1 | 186 | 5.4 | 269A | 4.6 |
| 98 | 27.1 | 188 | 3.1 | 270A | 4.9 |
| 99 | 2.4 | 189 | 2.6 | 271A | 8.7 |
| 100 | 47.7 | 190 | 3.2 | 272A | 10.4 |
| 102 | 4273.8 | 192 | 2.0 | 273A | 26.6 |
| 104 | 47.7 | 193 | 2.1 | 274A | 28.5 |
| 105 | 26.9 | 194 | <1.5 | 275A | 91.1 |
| 111 | 54.7 | 195 | 2.5 | 276A | 33.4 |
| 112 | 20.2 | 197 | 2.1 | 277A | 1.8 |
| 112B | 11.0 | 213A | 43.1 | | |

(continued)

| Example | NCI-H727 $IC_{50}$/nM | Example | NCI-H727 $IC_{50}$/nM | Example | NCI-H727 $IC_{50}$/nM |
|---|---|---|---|---|---|
| 113 | 168.0 | 221 | 71.8 | | |
| 118 | 35.5 | 226A | 39.8 | | |
| 119 | 18.3 | 228A | <1.5 | | |
| 119B | 6.9 | 229A | <1.5 | | |

**Example 2: Proliferation inhibition effect of the compounds of the present invention on AGS cells with KRAS G12D mutation**

This experiment evaluated and verified the proliferation inhibitory activity of the representative compound of the present invention against KRAS G12D mutant AGS cells.

AGS cells (Nanjing Kebai Biotechnology Co., Ltd., catalog number CBP60476, adherent, culture medium (F12K Nutrient Mixture+10% FBS (GIBCO, Cat#10091-148) were cultured at 37°C, 5% $CO_2$, and 95% humidity at a density of 1500 cells/well. Cells in the logarithmic growth phase were harvested and counted using a Countstar automated cell counter based on the classic trypan blue staining method to ensure cell viability was above 90%. The cell concentration was adjusted, and 80 μL of cell suspension was added to each 96-well clear flat-bottomed, black-walled plate (Greiner, Cat# 655090). The cells in the 96-well plates were then cultured at 37°C and 5% $CO_2$.

Drug preparation for IC50 assay: A 5-fold drug solution was prepared using culture medium. 20 μL of the drug solution was added to each well of a 96-well plate seeded with cells to achieve a working concentration of up to 10 μM, with a 3 × dilution, and a total of 9 concentrations. 2 replicate wells for each drug concentration were set. The cells in the drug-treated 96-well plates were cultured at 37°C and 5% $CO_2$ for additional 3 days and then CTG assay were performed.

The cell plate was equilibrated to room temperature for 30 minutes and the CTG reagent (CellTiter-Glo® Luminescent Cell Viability Assay (Promega, Cat# G7573)) was thawed. 50 μL of CTG solution was added to each well and the plate was shaken on an orbital shaker for 2 minutes to lyse the cells. The cell plate was placed at room temperature for 10 minutes to stabilize the cryo-luminescence signal and the cryo-luminescence value was read (EnVision® Multifunctional microplate reader, PerkinElmer #2105).

Data was analyzed using GraphPad Prism software. The dose-response-inhibition equation was used to fit the data to obtain the dose-response curve, and the IC50 value (nM) was calculated accordingly.

Cell viability (%) = (Lum $_{\text{test drug}}$ - Lum $_{\text{culture medium control}}$)/(Lum $_{\text{cell control}}$ - Lum culture medium control) × 100%.

The results showed that the compounds of the present invention exhibited satisfactory antiproliferative activity against KRAS G12D-mutant AGS human gastric adenocarcinoma cells, with an IC50 range of <1000 nM, preferably <100 nM, for example 0.01 nM-100 nM, more preferably 0.01 nM-50 nM. The IC50 values of representative compounds are shown in the table below:

| Example | AGS $IC_{50}$/nM | Example | AGS $IC_{50}$/nM | Example | AGS $IC_{50}$/nM |
|---|---|---|---|---|---|
| 3A | 112.1 | 150 | <1.5 | 235A | <1.5 |
| 4A | 148.4 | 159 | 87.4 | 236A | 2.7 |
| 5A | 1.6 | 160 | <1.5 | 237A | <1.5 |
| 6A | 4.7 | 161 | 5.6 | 238A | 11.5 |
| 7 | 6.7 | 161A | 2.4 | 240A | 2.5 |
| 8 | 8.1 | 161B | 33.5 | 241A | 8.3 |
| 8A | 1.5 | 162A | <1.5 | 242A | 5.4 |
| 9A | 1044.2 | 163A | <1.52 | 243A | <1.5 |
| 10A | 2409.7 | 164A | <1.5 | 246A | <1.5 |
| 10A-A | 1383.7 | 165A | 3.2 | 247A | <1.5 |
| 11A-A | 389.9 | 166A | 4.9 | 248A | <1.5 |

(continued)

| Example | AGS $IC_{50}$/nM | Example | AGS $IC_{50}$/nM | Example | AGS $IC_{50}$/nM |
|---|---|---|---|---|---|
| 12A-A | 790.4 | 167A | 21.7 | 249A | 2.3 |
| 12A-B | 886.2 | 168A | 42.1 | 250A | <1.5 |
| 16 | 13.2 | 169A | 14.6 | 251A | 1.8 |
| 28A | <1.5 | 170A | 33.2 | 252 | 41.5 |
| 55 | 13.05 | 171A | 4.7 | 253 | 2.5 |
| 65A | 1.5 | 172A | 198.9 | 254A | <1.5 |
| 67 | 1.75 | 173A | <1.5 | 255A | <1.5 |
| 68 | 1.9 | 174A | <1.5 | 256A | 1.5 |
| 68A | <1.5 | 175A | <1.5 | 257A | 1.5 |
| 83 | 38.2 | 176A | <1.5 | 258A | 1.9 |
| 84 | 59 | 177A | <1.5 | 259A | 3.1 |
| 85A | 22.7 | 178A | 1.9 | 260A | <1.5 |
| 86 | 8.7 | 179A | 4.2 | 261A | <1.5 |
| 86A | 4.9 | 180 | 10.6 | 262A | 2.9 |
| 91 | 84.7 | 181A | <1.5 | 263A | 2.5 |
| 92 | 987.1 | 182A | 2.0 | 264A | 2.3 |
| 93A | <1.5 | 183A | <1.5 | 265A | <1.5 |
| 93B | 177.8 | 184A | 3.4 | 266A | <1.5 |
| 94A | 3.3 | 185A | <1.5 | 267A | 4.4 |
| 95 | 10.5 | 186 | 4.4 | 268A | 3.5 |
| 98 | 15.8 | 188 | 2.9 | 269A | 2.8 |
| 99 | 13.05 | 189 | 2.0 | 270A | 3.6 |
| 100 | 20 | 190 | <1.5 | 271A | 9.3 |
| 101 | 100.1 | 192 | <1.5 | 272A | 12.5 |
| 103 | 301.45 | 193 | <1.5 | 273A | 40.6 |
| 104 | 368.9 | 194 | <1.5 | 274A | 14.9 |
| 105 | 338.3 | 195 | 2.5 | 275A | 56.4 |
| 111 | 25.6 | 197 | 3.0 | 276A | 18.5 |
| 112 | 9.9 | 213A | 49.6 | 277A | <1.5 |
| 112B | 5.3 | 221 | 102.6 | | |
| 113 | 583.9 | 226A | 19.7 | | |
| 113 | 583.9 | 227A | 462.0 | | |
| 118 | 12.8 | 228A | <1.5 | | |
| 119 | 2.4 | 229A | 1.7 | | |
| 119B | <1.5 | 230A | 1.7 | | |
| 120 | 10.3 | 231A | 2.5 | | |
| 121 | 178.7 | 232A | <1.5 | | |
| 123A | <1.5 | 233A | <1.5 | | |
| 124 | <1.5 | 234A | <1.5 | | |

**Example 3: Pharmacokinetic properties of the compounds of the present invention in mice**

The pharmacokinetic properties of some compounds of the present invention were evaluated using a mouse pharmacokinetic experiment.

Male CD-1 mice, aged 6-8 weeks, were purchased from Zhejiang Vital River Laboratory Animal Co., Ltd.

For the IV dosing group, the compounds were formulated in 20% Captisol (sulfobutyl β-cyclodextrin) in sodium acetate buffer (10 mM sodium acetate, pH adjusted to 4.0 with acetic acid) or a mixture of 5% DMSO and 95% (20% Captisol (sulfobutyl β-cyclodextrin)) to a final concentration of 0.6 mg/mL or 0.2 mg/mL for each compound. The drug formulation was administered to CD-1 mice via tail vein injection at a dose of 5 mL/kg. Blood samples were collected from the submandibular vein or other suitable methods at 5 min, 15 min, 30 min, 1 h, 2 h, 3 h, 4 h, 6 h, and 8 h post-administration. Blood samples were centrifuged at low temperature for 6 minutes, and plasma was collected and stored at -80°C for analysis. For the PO administration group, compounds were formulated in 0.5% Tween 80 and 99.5% (0.5%MC (400cp)) vehicle or 0.5%MC (400cp) vehicle to a final concentration of 1 mg/mL or 2 mg/mL for each compound. The drug formulation was administered to CD-1 mice via gavage at a dose of 10 mL/kg. Blood samples were collected from the submandibular vein or other suitable means at 15 min, 30 min, 1 h, 2 h, 4 h, 6 h, 8 h, and 24 h after administration. The blood samples were centrifuged at low temperature for 5 minutes, and the plasma was collected and stored at -80°C for analysis.

10 μL of plasma sample was taken, 200 μL of methanol containing an internal standard as a precipitant was added to precipitate plasma proteins, the compounds to be tested in the plasma was extracted, centrifuged at low temperature, and 10 μL of the supernatant was injected. The drug concentration in blood plasma was analyzed by LC-MS/MS. The pharmacokinetic parameters were calculated using Winnonlin based on the blood drug concentration data at different time points.

| Example | Route of administration/dosage | $AUC_{0-8}$ (ng.hr/mL) | Formulation |
|---|---|---|---|
| 178A | PO/10mpk | 1193 | 0.5%MC (400cp) |
| 228A | PO/20mpk | 4127 | 0.5%MC (400cp) |
| 229A | PO/20mpk | 4491 | 0.5%MC (400cp) |
| 232A | PO/20mpk | 1522 | 0.5%MC (400cp) |
| 233A | PO/20mpk | 822 | 0.5%MC (400cp) |
| 235A | PO/20mpk | 2990 | 0.5%MC (400cp) |
| 236A | PO/20mpk | 2823 | 0.5%MC (400cp) |
| 237A | PO/20mpk | 3911 | 0.5%MC (400cp) |
| 240A | PO/20mpk | 2918 | 0.5%MC (400cp) |
| 241A | PO/20mpk | 1661 | 0.5%MC (400cp) |
| 243A | PO/20mpk | 9962 | 0.5%MC (400cp) |
| 246A | PO/20mpk | 1043 | 0.5%MC (400cp) |
| 249A | PO/20mpk | 5365 | 0.5%MC (400cp) |
| 251A | PO/20mpk | 1961 | 0.5%MC (400cp) |
| 256A | PO/20mpk | 1380 | 0.5%MC (400cp) |
| 258A | PO/20mpk | 1317 | 0.5%MC (400cp) |
| 268A | PO/10mpk | 907 | 0.5%MC (400cp) |

Experimental results show that, in pharmacokinetic evaluation in mice, the compounds of the present invention, such as the compounds in the examples, exhibit good pharmacokinetic properties.

**Example 4: The inhibition assay of the compounds of the present invention on cytochrome P450**

This experiment evaluates the inhibitory effect of the compounds of the present invention on the cytochrome P450.

The following reagents were used in this experiment: human liver microsomes (Corning, catalog number 452161); reduced nicotinamide adenine dinucleotide phosphate (NADPH, MCE, catalog number HY-F0003/CS-4998); phenacetin, diclofenac, α-naphthoflavone, omeprazole, and ketoconazole were purchased from TCI; S- mephenytoin and testosterone were purchased from CAYMAN; midazolam was purchased from Bioreclamation IVT; quinidine was purchased from Damas-beta; sulfaphenazole was purchased from MCE; and bufuralol was purchased from TRC.

A 100mM potassium phosphate buffer (K-buffer) was prepared using potassium dihydrogen phosphate and dipotassium hydrogen phosphate, adjusted the pH to 7.4, and then prepared a 0.1M potassium phosphate buffer (K-buffer).

Preparation of 400× test compounds and positive control inhibitors: 8 μL of 10 mM test compound stock solution was dissolved in 12 μL acetonitrile. Test compound solutions were prepared; 12 μL of 1 mM α-naphthoflavone, 10 μL of 40 mM sulfaphenazole, 10 μL of 10 mM quinidine, and 8 μL of DMSO solution were mixed to prepare a mixed solution of CYP1A2, CYP2C9, and CYP2D6 inhibitors; 8 μL of 2.5 mM ketoconazole DMSO solution was dissolved in 12 μL acetonitrile to prepare CYP3A4 inhibitor solutions; 8 μL of 100 mM omeprazole DMSO solution were dissolved in 12 μL acetonitrile to prepare CYP2C19 inhibitor solutions.

66.7 mg NADPH to 10 mL of 0.1 M K-buffer (pH 7.4) was added to prepare a 4×NADPH potassium phosphate solution. Different substrates were prepared into solutions required for 4-fold concentration determination with 10 mL of 0.1 M K-buffer according to the concentration requirements to obtain a 4× substrate potassium phosphate solution. 10 μL of 20 mg/mL human liver microsomes was added to 990 μL of K-buffer to prepare a 0.2 mg/mL human liver microsome (HLM) solution, and store on ice until needed.

600 μL of 0.2 mg/mL HLM was added to a 96- well plate, followed by 3 μL of a 400-fold dilution of the test compound solution; 200 μL of 0.2 mg/mL HLM was added to the 96- well plate, followed by 1 μL of diluted positive control inhibitor solution. Aliquot 30 μL of the compound-human liver microsome mixture into the 96-well plate and then 15 μL of the substrate solution was added. The above-obtained solution and the prepared NADPH solution were preheat at 37°C for 5 min. 15 μL of the preheated NADPH solution was added to the reaction plate, mixed well, and the reaction was started. The reaction plate was incubated at 37°C. The 3A4 reaction lasted for 5 min; 1A2, 2C9, 2D6 reactions lasted for 10 min; and 2C19 for 45 min. After the reaction was completed, 120 μL of acetonitrile containing the internal standard was added to terminate the reaction. The sample was vortexed for 10 min, centrifuged at 5594 g for 15 min, and the prepared sample was sent for LC-MS/MS analysis.

Experimental results show that, at the tested concentrations, the compounds of the present invention, such as those in the examples, do not significantly inhibit key CYP subtypes of drug metabolism, demonstrating better drug-drug interaction safety.

The experimental results showed that at the tested concentrations, the compounds of the present invention, such as the compounds of the examples, had no significant inhibitory effect on the key CYP subtypes related to drug metabolism, indicating better safety in drug-drug interactions.

### Example 5: Proliferation inhibition effect of the compounds of the present invention on NCI-H358 cells with KRAS G12C mutation

This experiment evaluated and verified the proliferation inhibitory activity of the representative compound of the present invention against KRAS G12C mutant NCI-H358 cells.

NCI-H358 cells (Nanjing Kebai Biotechnology Co., Ltd., catalog number CBP60136, adherent, culture medium RPMI-1640+10%FBS (GIBCO, Cat#10091-148)) were cultured at 37°C, 5% $CO_2$, and 95% humidity.

3D cell viability assay: Cells in the logarithmic growth phase were harvested and counted using a platelet counter. Cell viability was assessed using the trypan blue exclusion method to ensure a viability of over 90%. The 3D cell complete culture medium was prepared by adding 1%MC (Sigma, Cat#M0512) to RPMI-1640 medium and 10% FBS. The cell concentration was adjusted to 12,500 cells/mL using the 3D medium, and the MC content was adjusted to 0.65%. Then, 80 μL of cell suspension was added to each 96-well clear flat-bottomed, black-walled plate (Greiner, Cat# 655096). The cells in the 96- well plates were cultured overnight at 37°C and 5% $CO_2$.

Drug preparation for IC50 assay: A 5-fold drug solution was prepared using culture medium. 20 μL of the drug solution was added to each well of a 96-well plate seeded with cells to achieve a working concentration of up to 10 μM, with a 3× dilution, and 9 concentrations. 2 replicate wells for each drug concentration were set. The cells in the drug-treated 96-well plates were cultured at 37°C and 5% $CO_2$ for additional 5 days, then CTG assay (CellTiter-Glo® Luminescent Cell Viability Assay (Promega, Cat#G7573)) were performed.

The cell plate was equilibrated to room temperature for 30 minutes and the CTG reagent was thawed. 50 μL of CTG solution was added to each well for detection, and the plate was shaken on an orbital shaker for 5 minutes to lyse the cells. The cell plate was placed at room temperature for 25 minutes to stabilize the cryo-luminescence signal and the cryo-luminescence value was read (EnVision® multi-plate reader, PerkinElmer#2105).

Data was analyzed using GraphPad Prism software. The dose-response-inhibition equation was used to fit the data to obtain the dose-response curve, and the IC50 value (nM) was calculated accordingly.

Cell viability (%) = (Lum $_{test\ drug}$ - Lum $_{culture\ medium\ control}$)/(Lum $_{cell\ control}$-Lum culture medium control) × 100%.

Representative compounds of the present invention showed satisfactory antiproliferative activity against KRAS G12C-mutant NCI-H358 human lung cancer cells, with an IC50 range of <1000 nM, preferably <100 nM.

**Example 6: Proliferation inhibition effect of the compounds of the present invention on 7 tumor cell lines**

This experiment evaluated and verified the proliferation inhibitory activity of the representative compounds of the present invention against the following 6 KRAS-related tumor cell lines.

| KRAS | Cell lines | Cell growth characteristics | Complete culture medium |
|---|---|---|---|
| G12V | NCI-H441 | Adherent | RPMI-1640 + 10% FBS |
| G12V | Capan-2 | Adherent | RPMI-1640 + 10% FBS |
| G12S | A549 | Adherent | DMEM + 10% FBS |
| G13D | HCT116 | Adherent | RPMI-1640 + 10% FBS |
| Q61H | NCI-H460 | Adherent | RPMI-1640 + 10% FBS |
| WT, Amplification | EBC-1 | Adherent | RPMI-1640 + 10% FBS |
| WT | A375 | Adherent | DMEM + 10% FBS |

The following materials, reagents, and instruments were used in this experiment: 6 cell lines were obtained from Kangyuan Bochuang Biotech (Beijing) Co., Ltd. (NCI-H441, KC-0510; Capan-2, KC-0185; A549, KC-0284; HCT116, KC-0281; NCI-H460, KC-0512; EBC-1, KC-0195; A375, KC-0158); RPMI-1640 (Hyclone, SH30809.01); fetal bovine serum FBS (GIBCO, 10099-141); methylcellulose (SIGMA, 9004-67-5); phosphate-buffered saline PBS (Solarbio, P1020-500); CellCounting-Lite 2.0 Luminescent Cell Viability Assay (Nanjing Vazyme, DD1101-04); 96-well transparent flat-bottom, black-walled plate (Thermo, 165305); multi-functional microplate reader (BMG LABTECH, CLARIOstar® Plus); $CO_2$ incubator (Thermo Scientific, Model 3100 Series).

Experimental methods:

Sterile 1% methylcellulose 3D medium was prepared in advance. Cells in the logarithmic growth phase were harvested and counted using a platelet counter. Cell viability was assessed using the trypan blue exclusion method to ensure a viability of over 90%. The cell concentrations of NCI-H441, A549, HCT116, NCI-H460, EBC-1, and A375 were adjusted to a final methylcellulose concentration of 0.65%, mixed well, and allow to stand. Once no visible gas remains in the cell suspension, 180 μL of cell suspension was added to each well of a 96-well plate, for a total of 2500 cells. the capan-2 cell concentration was adjusted using complete medium. 180 μL of cell suspension was added to each well of a 96-well plate, for a total of 3000 cells. The cells in the 96-well plates were cultured overnight at 37°C, 5% $CO_2$, and 95% humidity.

Drug preparation for IC50 assay: First, 10 mM DMSO stock solutions for each compound were prepared. For the first dilution, DMSO was used as a solvent, with a 3.16-fold dilution, and 9 concentrations, for the preparation of different concentrations of each compound in DMSO solutions. For the second dilution, 10-fold dilutions of each compound were prepared by a 1:100 dilution using complete culture medium as the solvent. Finally, 20 μL of each compound dilution was added to each well of a 96-well plate inoculated with cells. The final highest drug concentration is 10 μM, with 9 concentrations, a 3.16-fold dilution, and three replicates for each concentration.

Cells in 96-well plates with pre-treated cells were cultured at 37°C and 5% $CO_2$ for 144 hours, followed by CTG analysis. The CTG reagent was thawed and the cell plate was equilibrated to room temperature for 30 minutes. An equal volume of CTG solution was added to each well. The plate was shaken on an orbital shaker for 5 minutes to lyse the cells. The cell plate was then placed at room temperature for 20 minutes to stabilize the cryo-luminescence signal. The cryo-luminescence values were read, and data was collected.

Data was analyzed using GraphPad Prism 7.0 software. A nonlinear S-curve regression was used to fit the data to obtain the dose-response curve, and the IC50 value was calculated accordingly.

Cell viability (%) = (Lum $_{test\ drug}$ - Lum $_{culture\ medium\ control}$)/(Lum solvent control - Lum culture medium control) × 100%

The representative compounds of the present invention showed satisfactory antiproliferative activity against KRAS mutant cells and wild-type amplified cells in Example 5, with IC50 values in the range of <1000 nM, preferably <500 nM.

**Example 7: Antitumor effect of the compounds of the present invention in BALB/c nude mouse animal model with subcutaneous xenografts of human colon cancer cells GP2d with KRAS-G12D mutation**

GP2d cells were purchased from Nanjing Kebai Biotechnology Co., Ltd. Female BALB/c nude mice were purchased from Jiangsu Jicui Yaokang Biotechnology Co., Ltd. GP2d cells were cultured in DMEM and 10% FBS. Cells in the logarithmic growth phase were collected and resuspended in PBS to an appropriate concentration for subcutaneous tumor inoculation in mice. Experimental mice were subcutaneously inoculated in the right posterior back with GP2d cells at a dose of $1\times 10^7$/mouse, resuspended in a 1:1 mixture of PBS and matrix gel (inoculation volume 0.1 mL). When the average tumor volume reached ~150 $mm^3$, mice were randomly assigned to groups based on tumor size and body weight for drug administration. Drug administration began immediately after grouping, and the day of administration was considered day 0. The drug was administered to the mice twice daily by gavage at doses of 10 mg/kg, 30 mg/kg, or a solvent control (0.5% Tween 80+99.5% of (0.5%MC (400 cp)). During the experiment, mouse body weight and tumor size were measured twice weekly.

Formula for calculating tumor size: Tumor volume ($mm^3$)=0.5×(tumor long diameter×tumor short diameter$^2$).

Relative tumor inhibition rate (TGI)(%): TGI%=[1-(mean tumor volume at the end of treatment - mean tumor volume at the beginning of treatment)/(mean tumor volume at the end of treatment in solvent control group - mean tumor volume at the beginning of treatment in solvent control group)] × 100%.

Representative compounds of the present invention can significantly inhibit the growth of GP2d tumors.

**Example 8: Proliferation inhibition effect of the compound of the present invention on A549 cells with KRAS G12S mutation**

This experiment evaluated and verified the proliferation inhibitory activity of the representative compound of the present invention against the KRAS G12S mutant A549 cells.

A549 cells (Nanjing Kebai Biotechnology Co., Ltd., catalog number CBP60084, adherent, culture medium (F12K Nutrient Mixture+10%FBS (GIBCO, Cat#10091-148) at 37°C, 5%$CO_2$, and 95% humidity.

3D Cell Viability Assay: Cells in the logarithmic growth phase were harvested and counted using a platelet counter. Cell viability was assessed using the trypan blue exclusion method to ensure a viability of over 90%. The 3D cell culture medium was prepared by adding 1% MC (methylcellulose, Sigma, Cat#M0512) F-12K medium and 10% FBS. The cell concentration was adjusted to approximately 7407 cells/mL using the 3D medium, and the methylcellulose (MC) content was set to 0.65%. Then, 135 μL of cell suspension was added to each 96-well clear, flat-bottomed, black-walled plate (Greiner, Cat#655096). The cells in the 96-well plates were cultured overnight at 37°C with 5% $CO_2$.

Drug preparation for IC50 assay: A 10-fold drug solution was prepared using culture medium. 10 μL of the drug solution was added to each well of a 96-well plate seeded with cells to achieve a working concentration of up to 10 μM, with a 3× dilution, and 9 concentrations. 2 replicate wells for each drug concentration were set. The cells in the drug-treated 96-well plates were cultured at 37°C and 5% $CO^2$ for additional5 days, then CTG assay (CellTiter-Glo® Luminescent Cell Viability Assay (Promega, Cat#G7573)) were performed.

The cell plate was equilibrated to room temperature for 30 minutes and the CTG reagent was thawed. 75 μL of CTG solution was added to each well for detection, and the plate was shaken on an orbital shaker for 5 minutes to lyse the cells. The cell plate was placed at room temperature for 25 minutes to stabilize the cryo-luminescence signal and the cryo-luminescence value was read (EnVision® multi-plate reader, PerkinElmer#2105).

Data was analyzed using GraphPad Prism software. The dose-response-inhibition equation was used to fit the data to obtain the dose-response curve, and the IC50 value (nM) was calculated accordingly.

Cell viability (%) = ($Lum_{\text{test drug}}$ -$Lum_{\text{culture medium control}}$)/(Lum solvent control -Lum culture medium control) × 100%

Representative compounds of the present invention showed satisfactory antiproliferative activity against KRAS mutant cells and wild-type amplified cells in Example 5, with IC50 values in the range of <1000 nM, preferably <500 nM, for example 0.01 nM-100 nM, more preferably 0.01 nM-50 nM.

| Example | A549 $IC_{50}$/nM |
|---|---|
| 5A | 16.5 |
| 6A | 35.1 |
| 8A | 100.6 |
| 28A | 31.2 |
| 55 | 40.7 |
| 65A | 26.1 |
| 68A | 41.8 |
| 93A | 32.6 |
| 123A | 33.8 |
| 124 | 23.9 |
| 150 | 40.3 |
| 158 | 38.4 |
| 159 | 46.9 |
| 160 | 11.1 |
| 162A | 82.4 |
| 163A | 86.9 |
| 164A | 48.0 |
| 173A | 43.5 |
| 174A | 51.9 |
| 175A | 23.3 |
| 176A | 42.6 |

**Example 9: Antitumor effect of the compounds of the present invention in a subcutaneous xenograft NU/NU mouse model of human lung cancer cells NCI-H441 with KRAS-G12V mutation**

NCI-H441 cells were purchased from ATCC (catalog number: HTB-174). Male NU/NU mice were purchased from Jiangsu Jicui Yaokang Biotechnology Co., Ltd.

NCI-H441 cells were cultured in RPMI-1640 medium containing 10% FBS. Cells in the logarithmic growth phase were collected and resuspended in PBS to an appropriate concentration for subcutaneous tumor inoculation in mice. Experimental mice were subcutaneously inoculated in the right posterior back at a seeding density of $1\times10^6$ cells/mouse+Matrigel, with a seeding volume of 0.1 mL. When the average tumor volume reached ~150 $mm^3$, mice were randomly assigned to groups based on tumor size and body weight for drug administration. Drug administration began immediately after grouping, and the day of administration was considered day 0. The drug was administered to the mice twice daily via gavage at doses of 10 mpk, 20 mpk, 30 mpk, or a solvent control (0.5% Tween 80+99.5% of 0.5% MC (400 cp) or 0.5% MC (400 cp)). During the experiment, mouse body weight and tumor size were measured twice weekly. Tumor size was calculated using the formula: Tumor volume ($mm^3$)=0.5$\times$(Tumor long diameter $\times$ Tumor short diameter$^2$). Relative tumor inhibition rate (TGI) (%): TGI%=[1-(Average tumor volume at the end of treatment in a given treatment group - Average tumor volume at the start of treatment in the treatment group) / (Average tumor volume at the end of treatment in the solvent control group - Average tumor volume at the start of treatment in the solvent control group)] $\times$ 100%.

The compounds of the present invention can significantly inhibit the growth of NCI-H441 tumors, for example, at a dose of ≥10 mpk with a TGI% ≥50%.

| Group | Dosage | Administration method and frequency | Tumor volume ($mm^3$) (day 16) | TGI% |
|---|---|---|---|---|
| Solvent control group (0.5% MC (400 cp)) | / | PO, BID×17 | 575 | / |
| Example 175A | 20 mpk | PO, BID×17 | 266 | 73 |

(continued)

| Group | Dosage | Administration method and frequency | Tumor volume ($mm^3$) (day 16) | TGI% |
|---|---|---|---|---|
| Example 255A | 20 mpk | PO, BID×17 | 259 | 74 |
| Example 266A | 20 mpk | PO, BID×17 | 307 | 63 |
| Example 265A | 20 mpk | PO, BID×17 | 330 | 57 |
| Example 247A | 20 mpk | PO, BID×17 | 162 | 97 |

**Example 10: In vivo pharmacodynamic study of the compounds of the present invention in a subcutaneous xenograft NOD/SCID mouse model of human pancreatic cancer cells HPAC**

The following materials were used in this experiment: NOD/SCID mice, 6-8 weeks old, female, Jiangsu Jicui Yaokang Biotechnology Co., Ltd.; HPAC cells (carrying KRas G12D mutant protein, ATCC); Captisol (Wuhan Fengyao Tonghui Chemical Products Co., Ltd., Cas#182410-00-0); sodium acetate (aladdin) MC (sigma, 9004-67-5).

The experiment was conducted as follows: 6- to 8- week-old female NOD/SCID mice were subcutaneously inoculated with $1\times10^7$ HPAC cells in their right posterior back, with an inoculation volume of 0.1 mL. When the tumor reached an average volume of 150 $mm^3$, mice were randomly assigned to groups based on tumor size and body weight for drug administration. The day of drug administration was considered day 0. The test compound was administered by gavage to four mice in each group, while the control group received an equal volume of the solvent via gavage.

During the experiment, tumor volume and body weight were measured twice a week. Tumor volume was calculated using the formula: Tumor volume ($mm^3$)=0.5×(tumor long diameter×tumor short diameter$^2$). The mean tumor inhibition rate (TGI%) was calculated as follows: [(average tumor volume in the control group-average initial tumor volume in the control group)-(average tumor volume in the treatment group - average initial tumor volume in the treatment group)] / (average tumor volume in the control group - average initial tumor volume in the control group).

Experimental results showed that the representative compounds of the present invention exhibited excellent target-related tumor suppressor activity in a human pancreatic cancer HPAC model. Meanwhile, there were no significant changes in the body weight of mice in any group.

| Group | Dosage | Administration method and frequency | Tumor volume ($mm^3$) (day 17) | TGI (%) |
|---|---|---|---|---|
| Solvent control group (0.5% MC (400 cp)) | / | PO, BID×17 | 583 | / |
| Example 175A | 20 mpk | PO, BID×17 | 128 | 106 |
| Example 255A | 20 mpk | PO, BID×17 | 170 | 96 |
| Example 266A | 20 mpk | PO, BID×17 | 239 | 80 |
| Example 265A | 20 mpk | PO, BID×17 | 237 | 80 |
| Example 247A | 20 mpk | PO, BID×17 | 161 | 98 |

**Example 11: Proliferation inhibition effect of the compounds of the present invention on Ba/F3-engineered cells with KRAS mutation**

This experiment evaluated the proliferation inhibitory activity of the compounds of the present invention against Ba/F3-KRAS mutant cell lines by using the CellCounting-Lite®2.0 method.

The following materials, reagents, and instruments were used in this experiment: Cells were all obtained from Kangyuan Bochuang Biotechnology (Beijing) Co., Ltd. RPMI-1640 (Hyclone, SH30809.01); fetal bovine serum (FBS) was used. (GIBCO, 10099-141); Phosphate-buffered saline (PBS) (Solarbio, P1020-500); CellCounting-Lite 2.0 Luminescent Cell Viability Assay (Vazyme, DD1101-02); 96-well plate (Absin, abs7016); Multifunctional microplate reader (BMG LABTECH, CLARIOstar® Plus); $CO_2$ incubator (Thermo Scientific, Model 3100 Series).

Experimental methods:

All cell lines were cultured in complete medium at 37°C and 5% $CO_2$. Cells in the logarithmic growth phase were harvested and counted using a platelet counter. Cell viability was assessed using the trypan blue exclusion method to

ensure a viability of over 90%. Cell density was adjusted using complete medium (RPMI 1640+10% FBS) and then seeded into 96-well cell culture plates at 90 μL per well, for a total of 3000 cells. The cells in the 96-well plates were cultured at 37°C and 5% $CO_2$. A 10-fold drug solution was prepared using complete medium, and then 10 μL of each serially diluted compound was transferred to the corresponding wells of the 96-well cell culture plate, with two replicates for each drug concentration. The cells in the drug-treated 96-well plates were cultured at 37°C and 5% $CO_2$ for 72 h, followed by CellCounting-Lite 2.0 analysis.

The CellCounting-Lite 2.0 Luminescent Cell Viability Assay reagent was thawed and the cell plate was equilibrated to room temperature for 30 minutes. An equal volume of CellCounting-Lite 2.0 Luminescent Cell Viability Assay solution was added to each well and the plate was shaken on an orbital shaker for 5 minutes to lyse the cells. The cell plate was placed at room temperature for 20 minutes to stabilize the cryo-luminescence signal. The cryo-luminescence values were read and the data were collected.

Data Analysis:

Data was analyzed using GraphPad Prism 7.0 software. A nonlinear S-curve regression was used to fit the data to derive the dose-response curve, and the IC50 value was calculated from this curve.

Cell viability (%) = (Lum $_{\text{test drug}}$-Lum $_{\text{culture medium control}}$)/(Lum solvent $_{\text{control}}$-Lum culture medium control) × 100%.
Cell proliferation inhibition rate (%) = (1-cell viability rate) × 100%.

Experiments have shown that that the compounds of the present invention exhibit excellent anti-proliferation inhibitory activity against a series of KRAS mutant cell lines. The test results of the representative compound in Example 228A are shown below.

| Cell Number | Cell Name | Inhibition rate% @1uM | Inhibition rate% @0.05uM |
|---|---|---|---|
| KC-2619 | Ba/F3-KRAS-G12C-Y96S | 74.9 | 45.4 |
| KC-2604 | Ba/F3-KRAS-G12C-R68M | 96.6 | 91.6 |
| KC-2611 | Ba/F3-KRAS-G12C-Q99L | 98.1 | 93.9 |
| KC-2618 | Ba/F3-KRAS-G12C-H95R | 98.6 | 71.9 |
| KC-3683 | Ba/F3-KRAS-G12C-H95L | 100.0 | 99.7 |
| KC-2610 | Ba/F3-KRAS-G12C-A59T | 95.1 | 79.8 |
| KC-2605 | Ba/F3-KRAS-G12C-A59S | 97.4 | 83.3 |
| KC-1259 | Ba/F3-KRAS-G12D | 99.8 | 99.5 |
| KC-1261 | Ba/F3-KRAS-G 12V | 100.0 | 99.9 |
| KC-2894 | Ba/F3-KRAS-G12S | 98.8 | 96.4 |
| KC-2026 | Ba/F3-KRAS-G12R | 97.6 | 85.7 |
| KC-1436 | Ba/F3-KRAS-G13D | 98.2 | 88.1 |
| KC-2620 | Ba/F3-KRAS-Q61H | 99.6 | 99.1 |

It should be understood that various changes and modifications to the currently preferred examples described herein will be apparent to those skilled in the art. These changes and modifications can be made without departing from the spirit and scope of the subject matter of the present invention and without diminishing its intended advantages. Therefore, such changes and modifications are intended to be covered by the claims in the appendix.

All publicly available publications cited in this specification are incorporated herein by reference.

## Claims

1. A compound of Formula (II), a stereoisomer, tautomer, stable isotopic variant, pharmaceutically acceptable salt or solvate thereof,

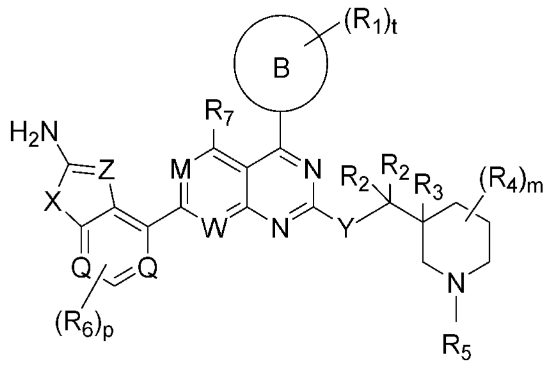

(II)

wherein:

M is selected from N and $CR_8$;
W is selected from N and $CR_9$;
Z is selected from N and $CR_{10}$;
X is selected from S or Se;
each Q is independently selected from C-$R_6$ and N;
Y is selected from O, S, Se, and N-$R_a$;
B is selected from 4-7 membered monocyclic heterocycloalkyl containing 1 to 3 heteroatoms independently selected from N, O, P, S and Se, and 6-12 membered polycyclic heterocyclic alkyl containing 1 to 4 heteroatoms independently selected from N, O, P, S and Se, provided that the ring atom attached to the pyrimidine ring portion of the molecule is a nitrogen heteroatom;
$R_1$ is selected from H, halogen, -CN, -OH, $-NH_2$, $-B(OH)_2$, oxo, $-C_{1-6}$ alkyl, $-C_{2-6}$ alkenyl, $-C_{2-6}$ alkynyl, $-OC_{1-6}$ alkyl, -C(O)-$C_{1-6}$ alkyl, $-C(O)N(R_b)_2$, wherein the $C_{1-6}$ alkyl present is optionally substituted by halogen, deuterium, OH or $-OC_{1-6}$ alkyl;
$R_2$ is each independently selected from H, D, $-C_{1-6}$ alkyl and $-(CH_2)_n-C_{3-6}$ cycloalkyl, or two $R_2$ together with the carbon atom to which they are attached form a $C_{3-4}$ cycloalkyl, wherein the $C_{1-6}$ alkyl, $C_{3-6}$ cycloalkyl or $C_{3-4}$ cycloalkyl is each independently and optionally substituted by halogen, D, $C_{1-6}$ alkyl optionally substituted by a halogen or D, or $-O-C_{1-6}$ alkyl optionally substituted by halogen or D;
$R_3$ is selected from $-C_{1-6}$ alkyl and $-(CH_2)_n-C_{3-6}$ cycloalkyl, each independently and optionally substituted by halogen, D, $C_{1-6}$ alkyl optionally substituted by halogen or D, or $-OC_{1-6}$ alkyl optionally substituted by halogen or D;
$R_4$ is selected from H, D, halogen, -CN, -OH, $-C_{1-6}$ alkyl, $-C_{2-6}$ alkenyl, $-C_{2-6}$ alkynyl, $-NH_2$, $-NH-C_{1-6}$ alkyl, $-N(C_{1-6}$ alkyl$)_2$, $-OC_{1-6}$ alkyl, $-OC_{3-6}$ cycloalkyl, and $-(CH_2)_n-C_{3-6}$ cycloalkyl, wherein the - $C_{1-6}$ alkyl, $-C_{2-6}$ alkenyl, $-C_{2-6}$ alkynyl or $C_{3-6}$ cycloalkyl present is each independently and optionally substituted by D, halogen, CN or $-OC_{1-6}$ alkyl, or
two $R_4$ attached to the same carbon atom form $=C(R_c)_2$, or two $R_4$ attached to the same ring carbon atom together with the ring carbon atom to which they are attached form a spiro $C_{3-6}$ cycloalkyl or a spiro 4-7 membered heterocycloalkyl, wherein each $R_c$ is independently selected from H, halogen and $-C_{1-6}$ alkyl optionally substituted by halogen, and the spiro $C_{3-6}$ cycloalkyl or spiro 4-7 membered heterocycloalkyl is optionally substituted by halogen and $-C_{1-6}$ alkyl optionally substituted by halogen;
$R_5$ is selected from H, $-C_{1-6}$ alkyl, $-C_{2-6}$ alkenyl, $-C_{2-6}$ alkynyl, and $-(CH_2)_n-C_{3-6}$ cycloalkyl, wherein the $-C_{1-6}$ alkyl, $-C_{2-6}$ alkenyl, $-C_{2-6}$ alkynyl, or $C_{3-6}$ cycloalkyl is each independently and optionally substituted by D, halogen, CN, $-OC_{1-6}$ alkyl, or $-O-CON(R_b)_2$;
$R_6$ and $R_7$ are each independently selected from H, D, halogen, CN, $-C_{1-6}$ alkyl, $-C_{2-6}$ alkenyl, - $C_{2-6}$ alkynyl, $-OC_{1-6}$ alkyl, $-SC_{1-6}$ alkyl and $-Se-C_{1-6}$ alkyl, wherein the $-C_{1-6}$ alkyl, $-C_{2-6}$ alkenyl, and $-C_{2-6}$ alkynyl present are each independently and optionally substituted by halogen, $-OC_{1-6}$ alkyl or D;
$R_8$ is selected from H, D, halogen, CN, $NO_2$, $-C_{1-6}$ alkyl, $-C_{2-6}$ alkenyl, $-C_{2-6}$ alkynyl and $-OR_b$, wherein the $-C_{1-6}$ alkyl, $-C_{2-6}$ alkenyl, and $-C_{2-6}$ alkynyl are each independently and optionally substituted by halogen, CN or $-OC_{1-6}$ alkyl; or $R_8$ is $-(CH_2)_n-C_{3-6}$ cycloalkyl, wherein the $C_{3-6}$ cycloalkyl is optionally substituted by halogen or CN;
$R_{10}$ is selected from H, D, halogen, CN, $NO_2$, $-C_{1-6}$ alkyl, $-C_{2-6}$ alkenyl, $-C_{2-6}$ alkynyl and $-OR_b$, wherein the $-C_{1-6}$ alkyl, $-C_{2-6}$ alkenyl, and $-C_{2-6}$ alkynyl are each independently and optionally substituted by halogen or CN;
$R_9$ is selected from H, halogen, CN, $-C_{1-6}$ alkyl, $-C_{2-6}$ alkenyl, $-C_{2-6}$ alkynyl, $-OR_b$, and $-O-CON(R_b)_2$, wherein the $-C_{1-6}$ alkyl, $-C_{2-6}$ alkenyl, and $-C_{2-6}$ alkynyl are each independently and optionally substituted by halogen or CN;
$R_a$ is selected from H, and $-C_{1-6}$ alkyl optionally substituted by halogen;
$R_b$ is selected from H, $-C_{1-6}$ alkyl optionally substituted by halogen, -OH, $-OC_{1-6}$ alkyl or deuterium, and $-C_{3-6}$

cycloalkyl, or wherein two $R_b$ attached to the same N atom together with the N atom to which they are attached form a 4-7 membered heterocycloalkyl optionally substituted by halogen or $-C_{1-6}$ alkyl;
n and p are each independently an integer from 0 to 3; and
m and t are each independently an integer from 0 to 8.

**2.** The compound of Formula (I), a stereoisomer, tautomer, stable isotopic variant, pharmaceutically acceptable salt or solvate thereof,

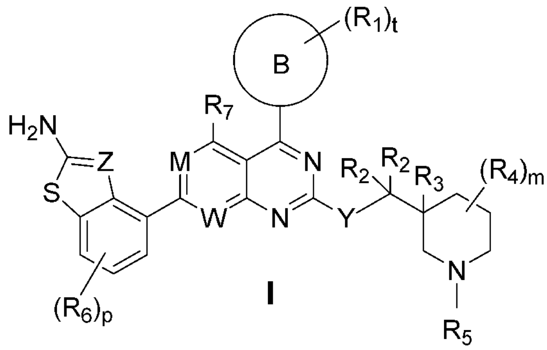

(I)

wherein:

M is selected from N and $CR_8$;
W is selected from N and $CR_9$;
Z is selected from N and $CR_{10}$;
Y is selected from O, S, Se, and $NR_a$;
B is selected from 5-7 membered monocyclic heterocycloalkyl containing 1 to 3 heteroatoms independently selected from N, O, P, S and Se, and 6-12 membered polycyclic heterocycloalkyl containing 1 to 4 heteroatoms independently selected from N, O, P, S and Se, provided that the ring atom attached to the pyrimidine ring portion of the molecule is a nitrogen heteroatom;
$R_1$ is selected from H, halogen, -CN, -OH, $-B(OH)_2$, $-C_{1-6}$ alkyl, $-OC_{1-6}$ alkyl, $-C(O)N(R_b)_2$, wherein the $C_{1-6}$ alkyl present is optionally substituted by halogen;
$R_2$ is each independently selected from H, D, $-C_{1-6}$ alkyl and $-(CH_2)_n-C_{3-6}$ cycloalkyl, or two $R_2$ together with the carbon atom to which they are attached form a $C_{3-4}$ cycloalkyl, wherein the $C_{1-6}$ alkyl, $C_{3-6}$ cycloalkyl or $C_{3-4}$ cycloalkyl is each independently and optionally substituted by halogen, D, $C_{1-6}$ alkyl optionally substituted by halogen or D, or $-OC_{1-6}$ alkyl optionally substituted by halogen or D;
$R_3$ is selected from $-C_{1-6}$ alkyl and $-(CH_2)_n-C_{3-6}$ cycloalkyl, each independently and optionally substituted by halogen, D, $C_{1-6}$ alkyl optionally substituted by halogen or D, or $-OC_{1-6}$ alkyl optionally substituted by halogen or D;
$R_4$ is selected from H, D, halogen, -CN, -OH, $-C_{1-6}$ alkyl, $-C_{2-6}$ alkenyl, $-C_{2-6}$ alkynyl, $-NH_2$, $-NH-C_{1-6}$ alkyl, $-N(C_{1-6}$ alkyl$)_2$, $-OC_{1-6}$ alkyl, $-OC_{3-6}$ cycloalkyl, and $-(CH_2)_n-C_{3-6}$ cycloalkyl, wherein the - $C_{1-6}$ alkyl, $-C_{2-6}$ alkenyl, $-C_{2-6}$ alkynyl or $C_{3-6}$ cycloalkyl present is each independently and optionally substituted by D, halogen, CN or $-OC_{1-6}$ alkyl, or
two $R_4$ attached to the same ring carbon atom form $=C(R_c)_2$, or two $R_4$ attached to the same ring carbon atom together with the ring carbon atom to which they are attached form a spiro $C_{3-6}$ cycloalkyl or a spiro 4-7 membered heterocycloalkyl, wherein each $R_c$ is independently selected from H, halogen and $-C_{1-6}$ alkyl optionally substituted by halogen, and the spiro $C_{3-6}$ cycloalkyl or spiro 4-7 membered heterocyclic alkyl is optionally substituted by halogen and $-C_{1-6}$ alkyl optionally substituted by halogen;
$R_5$ is selected from H, $-C_{1-6}$ alkyl, $-C_{2-6}$ alkenyl, $-C_{2-6}$ alkynyl, and $-(CH_2)_n-C_{3-6}$ cycloalkyl, wherein the $-C_{1-6}$ alkyl, $-C_{2-6}$ alkenyl, $-C_{2-6}$ alkynyl, or $C_{3-6}$ cycloalkyl is each independently and optionally substituted by D, halogen, CN, $-OC_{1-6}$ alkyl, or $-O-CON(R_b)_2$;
$R_6$ and $R_7$ are each independently selected from H, D, halogen, CN, $-C_{1-6}$ alkyl, $-C_{2-6}$ alkenyl, - $C_{2-6}$ alkynyl, $-OC_{1-6}$ alkyl, $-SC_{1-6}$ alkyl and $-Se-C_{1-6}$ alkyl, wherein each $-C_{1-6}$ alkyl, $-C_{2-6}$ alkenyl, and $-C_{2-6}$ alkynyl present is independently and optionally substituted by halogen;
$R_8$ and $R_{10}$ are each independently selected from H, D, halogen, CN, $-C_{1-6}$ alkyl, $-C_{2-6}$ alkenyl, - $C_{2-6}$ alkynyl and $-OR_b$, wherein the $-C_{1-6}$ alkyl, $-C_{2-6}$ alkenyl, and $-C_{2-6}$ alkynyl are each independently and optionally substituted by halogen or CN;
$R_9$ is selected from H, halogen, CN, $-C_{1-6}$ alkyl, $-C_{2-6}$ alkenyl, $-C_{2-6}$ alkynyl, $-OR_b$, and $-O-CON(R_b)_2$, wherein the

$-C_{1-6}$ alkyl, $-C_{2-6}$ alkenyl, and $-C_{2-6}$ alkynyl are each independently and optionally substituted by halogen or CN;
$R_a$ and $R_b$ are each independently selected from H and $-C_{1-6}$ alkyl optionally substituted by halogen;
n and p are each independently an integer from 0 to 3; and
m and t are each independently an integer from 0 to 8.

**3.** The compound of Claims 1 or 2, a stereoisomer, tautomer, stable isotopic variant, pharmaceutically acceptable salt or solvate thereof, wherein Z is C-CN.

**4.** The compound of any one of Claims 1 to 3, a stereoisomer, tautomer, stable isotopic variant, pharmaceutically acceptable salt or solvate thereof, wherein the benzo fused ring fragment containing Z is

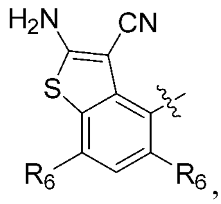,

wherein $R_6$ is each independently selected from H and halogen, wherein the halogen is preferably F.

**5.** The compound of any one of Claims 1 to 4, a stereoisomer, tautomer, stable isotopic variant, pharmaceutically acceptable salt or solvate thereof, wherein M is a C-halogen-substituted $C_{1-6}$ alkyl, C-halogen-substituted $C_{2-6}$ alkenyl, C-halogen, C-CN, or C-$NO_2$, preferably M is a C-halogen-substituted $C_{1-6}$ alkyl or C-halogen.

**6.** The compound of any one of Claims 1 to 5, a stereoisomer, tautomer, stable isotopic variant, pharmaceutically acceptable salt or solvate thereof, wherein W is selected from N, C-halogen or CO-optionally halogen-substituted $C_{1-3}$ alkyl; or W is selected from C-halogen or C-$C_{1-3}$ alkyl.

**7.** The compound of any one of Claims 1 to 6, a stereoisomer, tautomer, stable isotopic variant, pharmaceutically acceptable salt or solvate thereof, wherein B is a 5-7 membered monocyclic heterocycloalkyl containing 1 nitrogen atom, 5-7 membered monocyclic heterocycloalkyl containing 1 nitrogen atom and 1 O atom, 8-10 membered spiro heterocycloalkyl or 8-10 fused heterocycloalkyl containing 1-3 nitrogen atoms and 0-1 O atom, each optionally substituted by 0-3 groups independently selected from -OH, $NH_2$, CN, halogen, $-C_{1-6}$ alkyl, -CO-$C_{1-6}$ alkyl and $-CON(R_b)_2$.

**8.** The compound of Claim 7, a stereoisomer, tautomer, stable isotopic variant, pharmaceutically acceptable salt or solvate thereof, wherein B is selected from

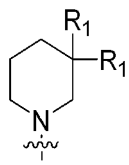, 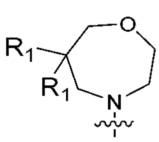,

wherein $R_1$ is each independently selected from -OH and $-C_{1-6}$ alkyl, optionally substituted by halogen; or B is selected from

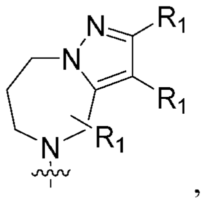, 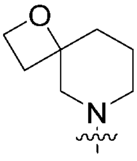, 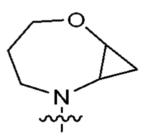,

wherein the $R_1$ adjacent to the nitrogen atom on the five-membered ring is selected from $-COC_{1-6}$ alkyl, $-CONH_2$, $-CONHC_{1-6}$ alkyl and $-CON(C_{1-6}$ alkyl$)_2$, and the remaining $R_1$ are selected from H, $NH_2$, CN, halogen and $-C_{1-6}$ alkyl;

or B is

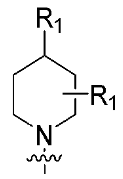

,

wherein one $R_1$ is selected from halogen, CN, halogen-substituted $-C_{1-6}$ alkyl, preferably at the para position of the ring N atom, and the other $R_1$ is selected from H, -OH, optionally halogen-substituted $-C_{1-6}$ alkyl or optionally halogen-substituted $-OC_{1-6}$ alkyl.

**9.** The compound of any one of Claims 1 to 6, a stereoisomer, tautomer, stable isotopic variant, pharmaceutically acceptable salt or solvate thereof, wherein B is

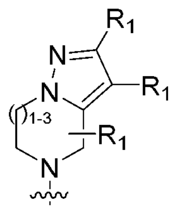

,

for example

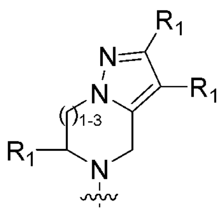

,

wherein

$R_1$ adjacent to the nitrogen atom on the five-membered ring is selected from $-C(O)-C_{1-6}$ alkyl and - $C(O)N(R_b)_2$, preferably $-C(O)N(R_b)_2$, each $R_b$ is independently H, $-C_{1-6}$ alkyl optionally substituted by one or more halogen, deuterium, or $-O-C_{1-6}$ alkyl, or $-C_{3-6}$ cycloalkyl, preferably each $R_b$ is independently H or $-C_{1-3}$ alkyl optionally substituted by one or more deuterium, most preferably each $R_b$ is $-C_{1-3}$ alkyl independently substituted with one or more deuterium, for example $R_1$ is $-C(O)CH_3$, $-C(O)N(CH_3)(CH_2CH_2F)$, $-C(O)N(CH_3)_2$, $-C(O)N(CH_3)$(cyclopropyl), $-C(O)NH(CH_3)$, - $C(O)N(CD_3)_2$, $-C(O)N(CH_3)(CH_2CH_2OCH_3)$; or the two $R_b$ together with the nitrogen atom they are attached to form a 4-7 membered heterocycloalkyl optionally substituted by halogen or $-C_{1-6}$ alkyl, for example azetidinyl, 3-F-azetidinyl, morpholinyl, pyrrolidinyl, piperazinyl, 4-methylpiperazinyl,

$R_1$ at the meta position of the nitrogen atom on the five-membered ring is selected from H, halogen, CN, -OH, $-NH_2$, $-C_{1-6}$ alkyl, $-C_{2-6}$ alkenyl, $-C_{2-6}$ alkynyl, $-OC_{1-6}$ alkyl, and $-C_{1-6}$ alkyl-$OC_{1-6}$ alkyl, preferably selected from **H,** halogen, -CN, -OH, $NH_2$, $-C_{1-3}$ alkyl, $-C_{2-4}$ alkenyl, $-C_{2-4}$ alkynyl, $-OC_{1-3}$ alkyl, and $-C_{1-3}$ alkyl-$OC_{1-3}$ alkyl, more preferably selected from $-C_{1-3}$ alkyl $-OC_{1-3}$ alkyl, wherein the alkyl present is optionally substituted by one or more halogen, OH, or deuterium, preferably with one or more deuterium, the $R_1$ at this position is, for example, but not limited to, H, F, Cl, -OH, $-NH_2$, - CN, $-OCH_3$, $-CH_3$, -ethynyl, $-CH_2F$, $-CHF_2$, $-O-CD_3$, $-CH_2OH$, $-CH_2-CH_3$, $-O-CH_2-CH_3$, $-CH_2-O-CH_3$, $-CD_2-O-CD_3$, $-CH_2-O-CD_3$, $-CH_2-OCH_2CH_3$,

the $R_1$ at the ortho position of nitrogen via which B is connected to the rest of the molecule, is selected from H or $-C_{1-6}$ alkyl, preferably H or $-C_{1-3}$ alkyl, for example H, $-CH_3$ or $-CH_2CH_3$;

preferably, B is selected from

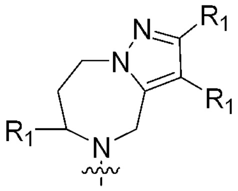, 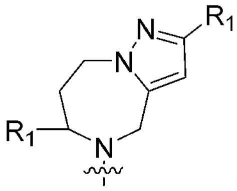, 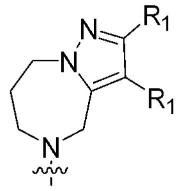, 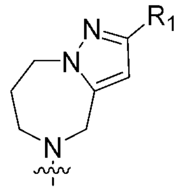,

wherein $R_1$ is as defined in this claim, provided that each of it is not H.

**10.** The compound of any one of Claims 1 to 9, a stereoisomer, tautomer, stable isotopic variant, pharmaceutically acceptable salt or solvate thereof, wherein Y is O.

**11.** The compound of any one of Claims 1 to 10, a stereoisomer, tautomer, stable isotopic variant, pharmaceutically acceptable salt or solvate thereof, wherein each $R_2$ is independently H or D.

**12.** The compound of any one of Claims 1 to 11, a stereoisomer, tautomer, stable isotopic variant, pharmaceutically acceptable salt or solvate thereof, wherein $R_3$ is a -$C_{1-6}$ alkyl.

**13.** The compound of any one of Claims 1 to 12, a stereoisomer, tautomer, stable isotopic variant, pharmaceutically acceptable salt or solvate thereof, wherein $R_4$ is selected from halogen, -O-$C_{1-6}$ alkyl, and -$C_{1-6}$ alkyl, wherein each $C_{1-6}$ alkyl present is independently and optionally substituted by halogen, or

two $R_4$ attached to the same carbon atom form =$C(R_c)_2$, wherein each $R_c$ is independently selected from H, halogen and -$C_{1-6}$ alkyl optionally substituted by halogen, or
two $R_4$ attached to the same ring carbon atom together with the ring carbon atom to which they are attached form a spiro $C_{3-6}$ cycloalkyl, and the spiro $C_{3-6}$ cycloalkyl is optionally substituted by halogen and -$C_{1-6}$ alkyl optionally substituted by halogen;
preferably, $R_4$ substitution occurs at the para position of the ring N atom.

**14.** The compound of any one of Claims 1 to 13, a stereoisomer, tautomer, stable isotopic variant, pharmaceutically acceptable salt or solvate thereof, wherein $R_5$ is selected from -$C_{1-6}$ alkyl, - deuterated $C_{1-6}$ alkyl and -$C_{3-6}$ cycloalkyl, preferably selected from -$C_{1-3}$ alkyl and -deuterated $C_{1-3}$ alkyl.

**15.** The compound of any one of Claims 1 to 14, a stereoisomer, tautomer, stable isotopic variant, pharmaceutically acceptable salt or solvate thereof, wherein the structural fragment

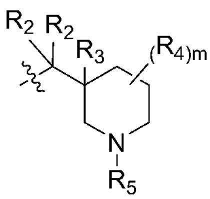

is

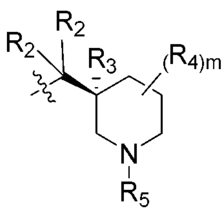,

preferably

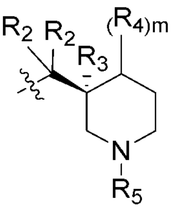,

wherein $R_2$ is each independently selected from H and D; $R_3$ is selected from $-C_{1-3}$ alkyl; m is 1 or 2; $R_4$ is halogen, optionally halogen-substituted $-C_{1-3}$ alkyl, or two $R_4$ attached to the same carbon atom to form $=C(R_c)_2$, wherein each $R_c$ is independently selected from H, halogen, and optionally halogen-substituted $-C_{1-3}$ alkyl, or two $R_4$ attached to the same ring carbon atom together with the ring carbon atom to which they are attached form an optionally halogen-substituted spiro $C_{3-4}$ cycloalkyl, $R_5$ is a $-C_{1-3}$ alkyl or $-C_{1-3}$ alkyl substituted by one or more deuterium.

**16.** The compound of Claim 15, a stereoisomer, tautomer, stable isotopic variant, pharmaceutically acceptable salt or solvate thereof, wherein the structural fragment

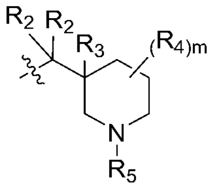

is selected from

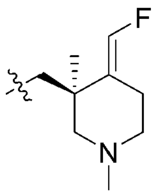, 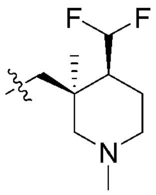, 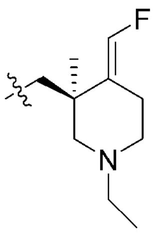, 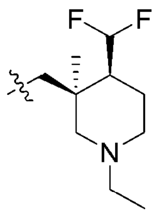, 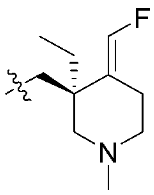, 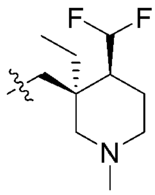,

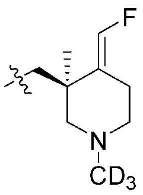, 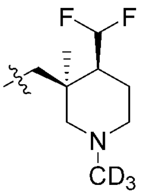, 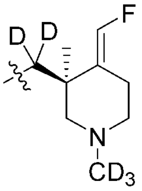, 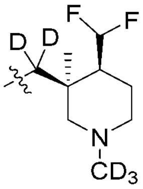, 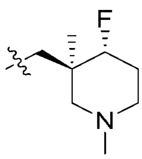, 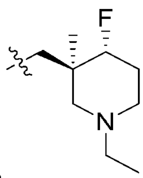, 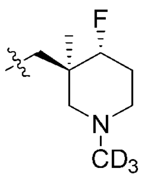, 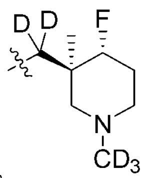,

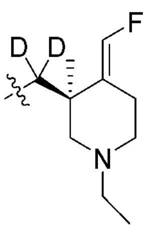, 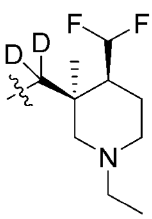, 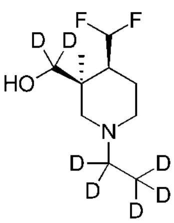, 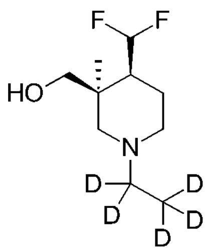, 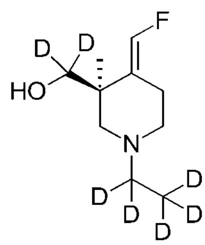.

**17.** The compound of Claim 1 or 2, a stereoisomer, tautomer, stable isotopic variant, pharmaceutically acceptable salt or solvate thereof, having the following sub-formula:

| | |
|---|---|
| 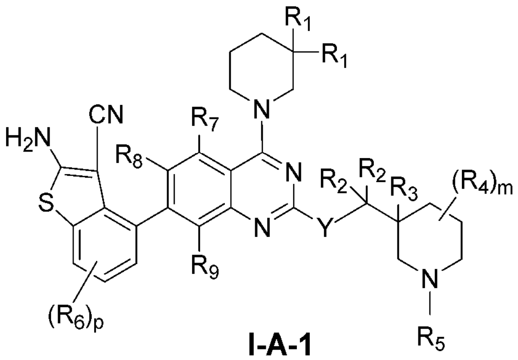 I-A-1 | 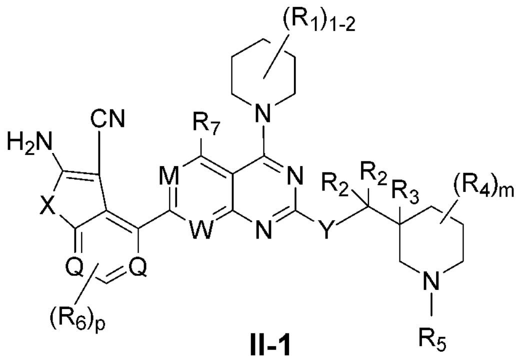 II-1 |
| 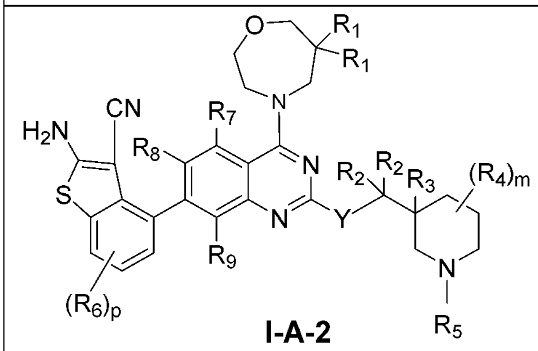 I-A-2 | 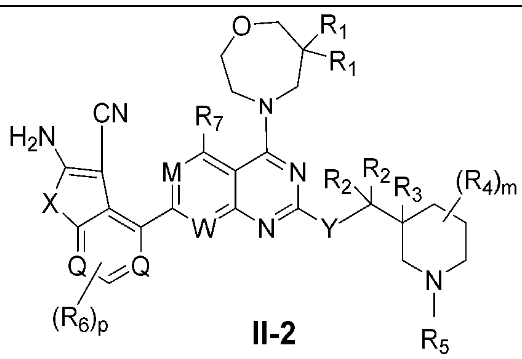 II-2 |
| 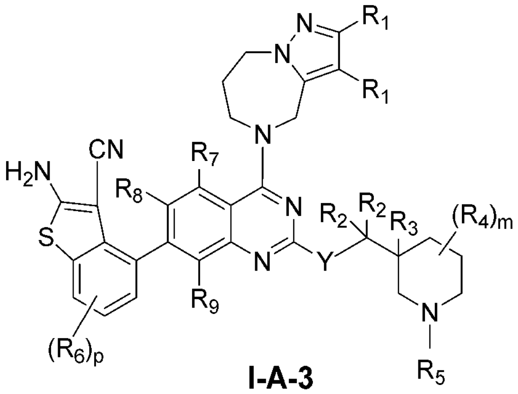 I-A-3 | 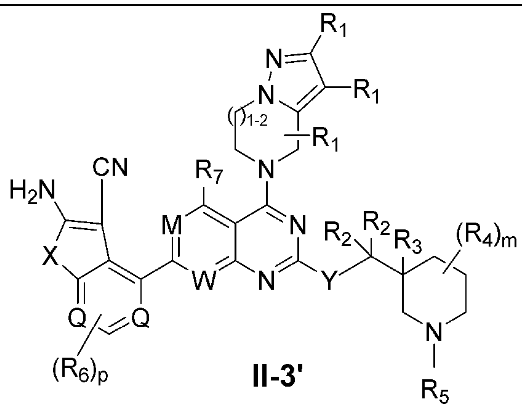 II-3' |

wherein each group has the meaning as defined in any one of Claims 1 to 15, preferably
X is selected from S and Se;
each Q is independently selected from C-$R_6$ and N;
$R_6$ is each independently selected from H and halogen;
the benzo-fused ring segment containing Z is preferably

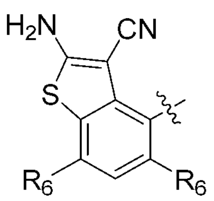,

wherein $R_6$ is each independently selected from H and halogen, wherein the halogen is preferably F;
M is selected from N and C-$R_8$;
W is selected from N and C-$R_9$;
$R_7$ is H;

$R_8$ is $C_{1-3}$ alkyl substituted by halogen (preferably F), $C_{2-4}$ alkenyl substituted by halogen (preferably F), CN, $NO_2$, or halogen (preferably Cl); or $R_8$ is $-C_{1-6}$ alkyl-O-$C_{1-6}$ alkyl, preferably $-C_{1-3}$ alkyl-O-$C_{1-3}$ alkyl;
$R_9$ is selected from F, Cl and -O-optionally halogen-substituted $C_{1-3}$ alkyl;
for Formulae I-A-1, I-A-2, and II-2, one of $R_1$ is -OH and the other is a $-C_{1-3}$ alkyl;
for Formula II-1, one $R_1$ is selected from halogen, CN, halogen-substituted $-C_{1-6}$ alkyl, preferably located at the para position of the ring N atom, and the other is selected from H, -OH, optionally halogen-substituted $-C_{1-6}$ alkyl or $-OC_{1-6}$ alkyl;
for Formulae I-A-3 and II-3', the $R_1$ adjacent to the nitrogen atom on the five-membered ring is selected from $-COC_{1-6}$ alkyl, $-CONH_2$, $-CONHC_{1-6}$ alkyl and $-CON(C_{1-6}$ alkyl$)_2$, and the remaining $R_1$ is selected from H, $NH_2$, CN, halogen and $-C_{1-6}$ alkyl;
Y is O;
$R_2$ is each independently selected from H and D;
$R_3$ is a $-C_{1-3}$ alkyl;
$R_4$ is selected from halogen, -O-$C_{1-3}$ alkyl, and $-C_{1-3}$ alkyl, wherein the $C_{1-3}$ alkyl present is each independently and optionally substituted by halogen, or
two $R_4$ attached to the same carbon atom form a $=C(R_c)_2$, wherein each $R_c$ is independently selected from H, halogen, optionally halogen-substituted $-C_{1-3}$ alkyl, or
two $R_4$ attached to the same ring carbon atom together with the ring carbon atom to which they are attached form a spiro $C_{3-4}$ cycloalkyl, and the spiro $C_{3-4}$ cycloalkyl is optionally substituted by halogen and optionally halogen-substituted $-C_{1-3}$ alkyl;
$R_5$ is selected from $-C_{1-3}$ alkyl and -deuterated $C_{1-3}$ alkyl;
m is an integer from 1 to 2, and p is an integer from 0 to 2;
preferably, $R_4$ is located at the para position of the ring nitrogen atom; preferably, the structural segment

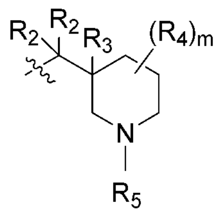 is 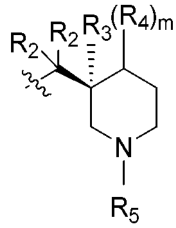 ;

more preferably, the structural fragment

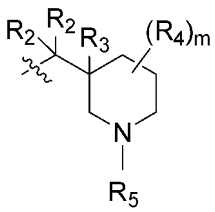

is selected from

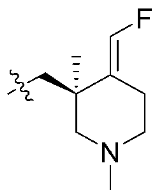 , 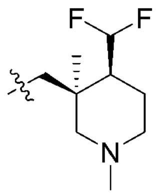 ,

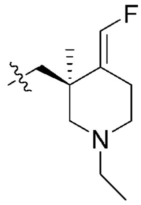, 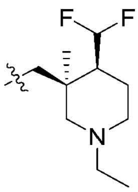, 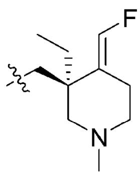, 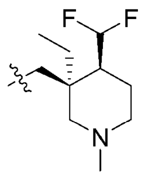, 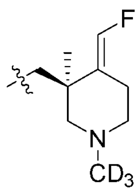, 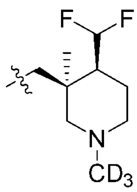, 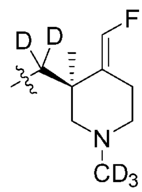,

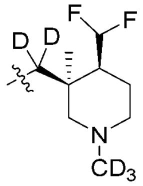, 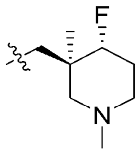, 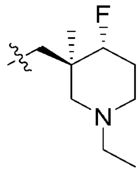, 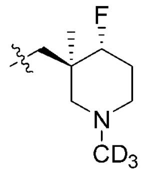, 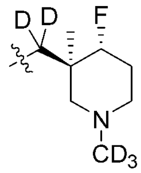, 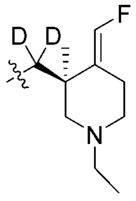, 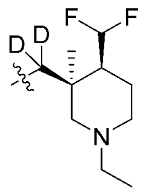,

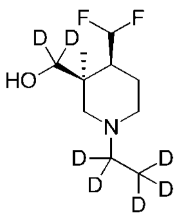, 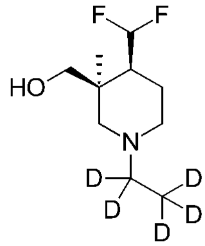, 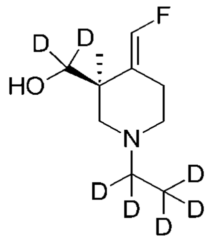.

**18.** The compound of Claim 1, a stereoisomer, tautomer, stable isotopic variant, pharmaceutically acceptable salt or solvate thereof, having the following general formula:

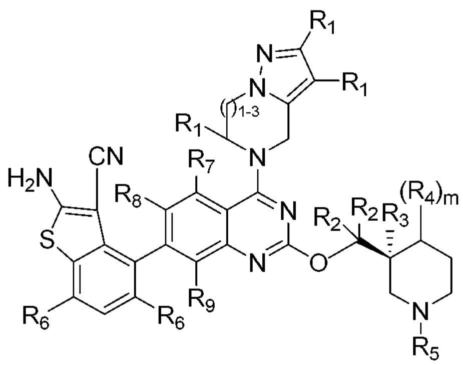

wherein,
the fused heterocyclic fragment carrying $R_1$ is

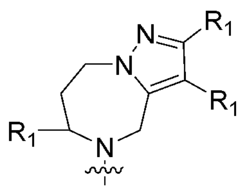

, wherein

$R_1$ adjacent to the nitrogen atom on the five-membered ring is selected from -C(O)-$C_{1-6}$ alkyl and - C(O)N($R_b$)$_2$, preferably -C(O)N($R_b$)$_2$, each $R_b$ is independently H, -$C_{1-6}$ alkyl optionally substituted by one or more halogen, deuterium or -$C_{3-6}$ cycloalkyl, preferably each $R_b$ is independently H or -$C_{1-3}$ alkyl optionally substituted by one or more deuterium, most preferably each $R_b$ is independently - $C_{1-3}$ alkyl optionally substituted by one or more

deuterium, for example $R_1$ is $-C(O)CH_3$, $-C(O)N(CH_3)(CH_2CH_2F)$, $-C(O)N(CH_3)_2$, $-C(O)N(CH_3)$(cyclopropyl), $-C(O)NH(CH_3)$, $-C(O)N(CD_3)_2$, $-C(O)N(CH_3)(CH_2CH_2OCH_3)$; or the two $R_b$ together with the nitrogen atom to which they are attached form a 4-7 membered heterocycloalkyl optionally substituted by halogen or $-C_{1-6}$ alkyl, for example azetidinyl, 3-F-azetidinyl, morpholinyl, pyrrolidinyl, piperazinyl, 4-methylpiperazinyl;

$R_1$ at the meta position of the nitrogen atom on the five-membered ring is selected from H, halogen, CN, -OH, $-NH_2$, $-C_{1-6}$ alkyl, $-C_{2-6}$ alkenyl, $-C_{2-6}$ alkynyl, $-OC_{1-6}$ alkyl, and $-C_{1-6}$ alkyl-O-$C_{1-6}$ alkyl, preferably selected from H, halogen, -CN, -OH, $NH_2$, $-C_{1-3}$ alkyl, $-C_{2-4}$ alkynyl, $-C_{2-4}$ alkenyl, $-OC_{1-3}$ alkyl and $-C_{1-3}$ alkyl-O-$C_{1-3}$ alkyl, more preferably selected from $-C_{1-3}$ alkyl-O-$C_{1-3}$ alkyl, wherein the alkyl present is optionally substituted by one or more halogen, OH, or deuterium, preferably by one or more deuterium, the $R_1$ at this position is, for example, but is not limited to, H, F, Cl, -OH, $-NH_2$, -CN, $-OCH_3$, $-CH_3$, -ethynyl, $-CH_2F$, $-CHF_2$, $-O-CD_3$ , $-CH_2OH$, $-CH_2-CH_3$, $-O-CH_2-CH_3$, $-CH_2-O-CH_3$, $-CD_2-O-CD_3$, $-CH_2-O-CD_3$, $-CH_2-O-CH_2CH_3$,

the $R_1$ at the ortho position of nitrogen via which this fragment is connected to the rest of the molecule is selected from H or $-C_{1-6}$ alkyl, preferably H or $-C_{1-3}$ alkyl, for example H, $-CH_3$ or $-CH_2CH_3$;

specifically, the fragment can be

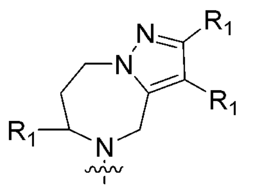
,
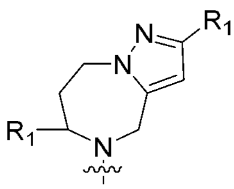
,
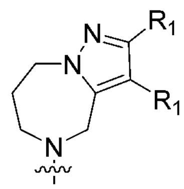
,
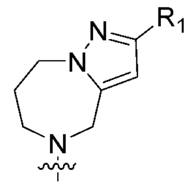
,

wherein $R_1$ is defined as described here, provided that each is not H;

$R_6$ is each independently selected from H and halogen (preferably F); the fragment carrying $R_6$ is preferably

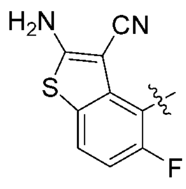
;

M is C-$R_8$;

W is C-$R_9$;

$R_7$ is H;

$R_8$ is halogen (preferably Cl), $C_{1-3}$ alkyl optionally substituted by halogen (preferably F) (e.g., $CF_3$), $-C_{2-4}$ alkenyl optionally substituted by halogen (preferably F), CN, $NO_2$, $-C_{1-6}$ alkyl-O-$C_{1-6}$ alkyl (preferably $-C_{1-3}$ alkyl-O-$C_{1-3}$ alkyl, e.g., $-CH_2-O-CH_3$); preferably halogen or $C_{1-3}$ alkyl substituted by halogen, such as Cl and $CF_3$;

$R_9$ is selected from halogen, $-C_{1-3}$ alkyl, -OH and -O-$C_{1-3}$ alkyl optionally substituted by halogen; preferably selected from halogen and $-C_{1-3}$ alkyl; for example, F, Cl, $CH_3$ and $-O-CH_3$;

Y is O;

$R_2$ is each independently selected from H and D;

$R_3$ is $-C_{1-3}$ alkyl;

$R_4$ is $-C_{1-3}$ alkyl substituted by halogen, or two $R_4$ attached to the same carbon atom form $=C(R_c)_2$, wherein each $R_c$ is independently selected from H and halogen;

$R_5$ is selected from $-C_{1-3}$ alkyl and -deuterated $C_{1-3}$ alkyl;

m is an integer from 1 to 2.

**19.** A compound is selected from the compound of the Examples, or a pharmaceutically acceptable salt or solvate thereof.

**20.** A pharmaceutical composition comprising a compound according to any one of Claims 1 to 19, a stereoisomer, tautomer, stable isotopic variant, pharmaceutically acceptable salt or solvate thereof, and a pharmaceutically acceptable excipient.

**21.** The compound according to any one of Claims 1 to 19, or a pharmaceutically acceptable salt or solvate thereof, or the pharmaceutical composition according to Claim 20, for use as a medicament for the treatment and/or prevention of diseases mediated by RAS mutations and RAS amplification.

**22.** Use of the compound according to any one of Claims 1 to 19, or a pharmaceutically acceptable salt or solvate thereof, or the pharmaceutical composition according to Claim 20, in the preparation of a medicament for preventing or treating diseases mediated by RAS mutations and RAS amplification.

**23.** The use according to Claim 22, wherein the diseases mediated by RAS mutations and RAS amplification are selected from: pancreatic cancer, lung cancer, lung adenocarcinoma, bone cancer, skin cancer, head and neck cancer, cutaneous or intraocular melanoma, uterine cancer, ovarian cancer, rectal cancer, anal region cancer, gastric cancer, colon cancer, breast cancer, fallopian tube cancer, endometrial cancer, cervical cancer, vaginal cancer, vulvar cancer, Hodgkin's disease, esophageal cancer, small intestinal cancer, endocrine system cancer, thyroid cancer, parathyroid cancer, adrenal cancer, soft tissue sarcoma, urethral cancer, penile cancer, prostate cancer, chronic or acute leukemia, lymphocytic lymphoma, bladder cancer, kidney or ureteral cancer, renal cell carcinoma, renal pelvis cancer, central nervous system (CNS) tumors, primary CNS lymphoma, spinal tumors, brainstem glioma, or pituitary adenoma.

**24.** The use according to Claim 22, wherein the diseases mediated by RAS mutations and RAS amplification are selected from pancreatic cancer, colon cancer, rectal cancer, lung adenocarcinoma, lung cancer, cholangiocarcinoma, endometrial cancer, ovarian cancer, leukemia; most preferably selected from pancreatic cancer, colon cancer, rectal cancer, lung adenocarcinoma, cholangiocarcinoma.

**25.** A method for treating and/or preventing diseases mediated by RAS proteins, especially KRAS mutant proteins and KRAS amplification, comprising administering a therapeutically effective amount of the compound according to any one of Claims 1 to 19, or a pharmaceutically acceptable salt or solvate thereof, or the pharmaceutical composition according to Claim 20, to a subject in need thereof.

**26.** The method according to Claim 25, wherein the diseases mediated by RAS mutations and RAS amplification are selected from pancreatic cancer, colon cancer, rectal cancer, lung adenocarcinoma, lung cancer, cholangiocarcinoma, endometrial cancer, ovarian cancer, leukemia; most preferably selected from pancreatic cancer, colon cancer, rectal cancer, lung adenocarcinoma, cholangiocarcinoma.

**INTERNATIONAL SEARCH REPORT**

International application No.
**PCT/CN2024/117451**

**A. CLASSIFICATION OF SUBJECT MATTER**

C07D409/14(2006.01)i; A61K31/513(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: C07D; A61K; A61P

Documentation searched other than minimum documentation to the extent that such documents are included in the fields searched

Electronic data base consulted during the international search (name of data base and, where practicable, search terms used)

CNTXT, STN, WPABS, 结构式, structural formula, RAS, 苯并噻吩, 哌啶, benzothiophene, piperidine

**C. DOCUMENTS CONSIDERED TO BE RELEVANT**

| Category* | Citation of document, with indication, where appropriate, of the relevant passages | Relevant to claim No. |
|---|---|---|
| PX | WO 2024067714 A1 (TYLIGAND BIOSCIENCE (SHANGHAI) LIMITED) 04 April 2024 (2024-04-04)<br>claims 1-23, and embodiments 190-200 | 1, 3-26 |
| PA | CN 117327103 A (SUZHOU ZELGEN BIOPHARMACEUTICALS CO., LTD. et al.) 02 January 2024 (2024-01-02)<br>entire document | 1-26 |
| X | WO 2022148422 A1 (BEIGENE, LTD. et al.) 14 July 2022 (2022-07-14)<br>claims 1-42, and description, table 6 | 1-26 |
| A | WO 2022177917 A2 (THERAS, INC. et al.) 25 August 2022 (2022-08-25)<br>entire document | 1-26 |
| A | WO 2023072188 A1 (BETTA PHARMACEUTICALS CO., LTD.) 04 May 2023 (2023-05-04)<br>entire document | 1-26 |
| A | WO 2022173870 A1 (KUMQUAT BIOSCIENCES INC.) 18 August 2022 (2022-08-18)<br>entire document | 1-26 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **28 November 2024** | **04 December 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

INTERNATIONAL SEARCH REPORT

International application No.
PCT/CN2024/117451

C. DOCUMENTS CONSIDERED TO BE RELEVANT

| Category* | Citation of document, with indication, where appropriate, of the relevant passages | Relevant to claim No. |
|---|---|---|
| A | CN 115836055 A (INVENTISBIO CO., LTD.) 21 March 2023 (2023-03-21)<br>entire document | 1-26 |
| A | WO 2023098832 A1 (3D MEDICINES (SHANGHAI) CO., LTD.) 08 June 2023 (2023-06-08)<br>entire document | 1-26 |
| A | WO 2023004102 A2 (THERAS, INC. et al.) 26 January 2023 (2023-01-26)<br>entire document | 1-26 |
| A | WO 2023141300 A1 (KUMQUAT BIOSCIENCES INC.) 27 July 2023 (2023-07-27)<br>entire document | 1-26 |

Form PCT/ISA/210 (second sheet) (July 2022)

**INTERNATIONAL SEARCH REPORT**

International application No. **PCT/CN2024/117451**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **25-26**
because they relate to subject matter not required to be searched by this Authority, namely:

   Claims 25-26 relate to a method for treating a disease. The present search is carried out on the basis of the use of a compound in the preparation of a drug.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/117451**

| Patent document cited in search report | Publication date (day/month/year) | Patent family member(s) | Publication date (day/month/year) |
|---|---|---|---|
| WO 2024067714 A1 | 04 April 2024 | TW 202430162 A | 01 August 2024 |
| CN 117327103 A | 02 January 2024 | WO 2024002373 A1 | 04 January 2024 |
| WO 2022148422 A1 | 14 July 2022 | US 2024140957 A1 | 02 May 2024 |
| WO 2022177917 A2 | 25 August 2022 | KR 20240002245 A | 04 January 2024 |
| | | JP 2024508755 A | 28 February 2024 |
| | | US 2024246954 A1 | 25 July 2024 |
| | | AU 2022224511 A1 | 10 August 2023 |
| | | WO 2022177917 A3 | 22 September 2022 |
| | | EP 4294799 A2 | 27 December 2023 |
| | | IL 304986 A | 01 October 2023 |
| | | MX 2023008463 A | 07 December 2023 |
| | | BR 112023016299 A2 | 28 November 2023 |
| | | TW 202245779 A | 01 December 2022 |
| | | CA 3209083 A1 | 25 August 2022 |
| WO 2023072188 A1 | 04 May 2023 | None | |
| WO 2022173870 A1 | 18 August 2022 | EP 4291199 A1 | 20 December 2023 |
| | | CA 3207854 A1 | 18 August 2022 |
| | | US 2024254129 A1 | 01 August 2024 |
| | | JP 2024506329 A | 13 February 2024 |
| | | AU 2022220678 A1 | 21 September 2023 |
| CN 115836055 A | 21 March 2023 | US 2023242544 A1 | 03 August 2023 |
| | | WO 2022002102 A1 | 06 January 2022 |
| | | JP 2023531269 A | 21 July 2023 |
| | | EP 4175947 A1 | 10 May 2023 |
| | | EP 4175947 A4 | 03 July 2024 |
| WO 2023098832 A1 | 08 June 2023 | None | |
| WO 2023004102 A2 | 26 January 2023 | CO 2024000588 A2 | 18 April 2024 |
| | | TW 202315610 A | 16 April 2023 |
| | | IL 310291 A | 01 March 2024 |
| | | CA 3227138 A1 | 26 January 2023 |
| | | WO 2023004102 A3 | 02 March 2023 |
| | | AU 2022315228 A1 | 08 February 2024 |
| | | US 2024300964 A1 | 12 September 2024 |
| | | EP 4373822 A2 | 29 May 2024 |
| | | MX 2024001108 A | 05 April 2024 |
| | | PE 20241063 A1 | 13 May 2024 |
| | | CL 2024000201 A1 | 14 June 2024 |
| | | KR 20240052096 A | 22 April 2024 |
| | | JP 2024526949 A | 19 July 2024 |
| WO 2023141300 A1 | 27 July 2023 | None | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2023111641130
- CN 2024102175790
- CN 2024106366320
- CN 2024111178533
- CN 2024112398386
- WO 2022095960 A
- WO 2024067714 A
- DD 110102


### Non-patent literature cited in the description


- **ALAMGEER et al.** *Current Opin Pharmacol.*, 2013, vol. 13, 394-401
- **ZHI TAN et al.** *Mini-Reviews in Medicinal Chemistry*, 2016, vol. 16, 345-357
- **CHANG, E.H. et al.** *Proceedings of the National Academy of Sciences of the United States of America*, 1982, vol. 79 (16), 4848-4852
- **MCCORMICK, F. et al.** *Clinical Cancer Research*, 2015, vol. 21 (8), 1797-1801
- McGraw-Hill Dictionary of Chemical Chemistry Terms. McGraw-Hill Book Company, 1984
- **ELIEL, E.** ; **WILEN, S.** Stereochemistry of Organic Compounds. John Wiley & Sons, Inc., 1994
- **ANSEL, HOWARD C. et al.** Ansel's Pharmaceutical Dosage Forms and Drug Delivery Systems.. Lippincott, Williams & Wilkins, 2004
- **SMITH M.B.** March's Advanced Organic Chemistry: Reactions, Mechanisms, and Structure. Wiley, 2013
- **E. L. ELIEL** ; **S. H. WILEN** ; **L. N. MANDER'S**. Stereochemistry of Organic Compounds. Wiley-Interscience, 1994
- **T. GREENE** ; **P. WUTS**. Protective Organic Groups in Synthesis. John Wiley & Sons, 1999
- *CHEMICAL ABSTRACTS*, 193274-53-2
- *CHEMICAL ABSTRACTS*, 2791277-01-3